# EUROPEAN PATENT SPECIFICATION

(11) **EP 3 768 684 B1**
(45) Date of publication and mention of the grant of the patent: **22.02.2023**
(21) Application number: 19711920.9
(22) Date of filing: 22.03.2019
(51) Int. Cl.: C07D 498/04, A61P 25/00, A61P 25/28, A61P 29/00, A61P 35/00, A61P 25/06, A61P 25/08, A61K 31/5375

(54) **OXAZINE MONOACYLGLYCEROL LIPASE (MAGL) INHIBITORS**
OXAZIN MONOACYLGLYCEROL LIPASE (MAGL) INHIBITOREN
INHIBITEURS OXAZINE DE MONOACYLGLYCEROL LIPASE (MAGL)

(30) Priority: 22.03.2018 EP 18163273; 18.02.2019 WO PCT/CN2019/075372
(43) Date of publication of application: 27.01.2021
(73) Proprietor: F. Hoffmann-La Roche AG, 4070 Basel (CH)
(72) Inventor: BELL, Charles, Stockport Cheshire SK7 2EA (GB); BENZ, Joerg, 4070 Basel (CH); GOBBI, Luca, 4070 Basel (CH); GRETHER, Uwe, 4070 Basel (CH); GROEBKE ZBINDEN, Katrin, 4070 Basel (CH); HANSEN, Dennis Jul, 2970 Hørsholm (DK); HORNSPERGER, Benoit, 4070 Basel (CH); KOCER, Buelent, 4070 Basel (CH); KROLL, Carsten, 4070 Basel (CH); KUHN, Bernd, 4070 Basel (CH); O`HARA, Fionn, 4070 Basel (CH); RICHTER, Hans, 4070 Basel (CH); RITTER, Martin, 4070 Basel (CH); TSUCHIYA, Satoshi, Tokyo, 103--8324 (JP); CHEN, Rui, Hubei, 430075 (CN)
(74) Representative: Neuhaus, Christian Michel
(86) International application number: PCT/EP2019/057174
(87) International publication number: WO 2019/180185

(56) References cited:
- US-A1- 2015 018 335
- SCALVINI LAURA ET AL: "Monoglyceride lipase: Structure and inhibitors", CHEMISTRY AND PHYSICS OF LIPIDS, LIMERICK, IR, vol. 197, 26 July 2015 (2015-07-26), pages 13-24, XP029445673, ISSN: 0009-3084, DOI: 10.1016/J.CHEMPHYSLIP.2015.07.011
- MULVIHILL MELINDA M ET AL: "Therapeutic potential of monoacylglycerol lipase inhibitors", LIFE SCIENCES, PERGAMON PRESS, OXFORD, GB, vol. 92, no. 8, 8 November 2012 (2012-11-08), pages 492-497, XP028986718, ISSN: 0024-3205, DOI: 10.1016/J.LFS.2012.10.025

## Description

### Field of the Invention

The present invention relates to organic compounds useful for therapy or prophylaxis in a mammal, and in particular to monoacylglycerol lipase (MAGL) inhibitors for the treatment or prophylaxis of neuroinflammation, neurodegenerative diseases, pain, cancer, mental disorders, multiple sclerosis, Alzheimer's disease, Parkinson's disease, amyotrophic lateral sclerosis, traumatic brain injury, neurotoxicity, stroke, epilepsy, anxiety, migraine and/or depression in a mammal.

### Background of the Invention

Endocannabinoids (ECs) are signaling lipids that exert their biological actions by interacting with cannabinoid receptors (CBRs), CB1 and CB2. They modulate multiple physiological processes including neuroinflammation, neurodegeneration and tissue regeneration (Iannotti, F.A., et al., Progress in lipid research 2016, 62, 107-28.). In the brain, the main endocannabinoid, 2-arachidonoylglycerol (2-AG), is produced by diacyglycerol lipases (DAGL) and hydrolyzed by the monoacylglycerol lipase, MAGL. MAGL hydrolyses 85% of 2-AG; the remaining 15% being hydrolysed by ABHD6 and ABDH12 (Nomura, D.K., et al., Science 2011, 334, 809.). MAGL is expressed throughout the brain and in most brain cell types, including neurons, astrocytes, oligodendrocytes and microglia cells (Chanda, P.K., et al., Molecular pharmacology 2010, 78, 996; Viader, A., et al., Cell reports 2015, 12, 798.). 2-AG hydrolysis results in the formation of arachidonic acid (AA), the precursor of prostaglandins (PGs) and leukotrienes (LTs). Oxidative metabolism of AA is increased in inflamed tissues. There are two principal enzyme pathways of arachidonic acid oxygenation involved in inflammatory processes, the cyclooxygenase which produces PGs and the 5-lipoxygenase which produces LTs. Of the various cyclooxygenase products formed during inflammation, PGE2 is one of the most important. These products have been detected at sites of inflammation, e.g. in the cerebrospinal fluid of patients suffering from neurodegenerative disorders and are believed to contribute to inflammatory response and disease progression. Mice lacking MAGL (Mgll-/-) exhibit dramatically reduced 2-AG hydrolase activity and elevated 2-AG levels in the nervous system while other arachidonoyl-containing phospho- and neutral lipid species including anandamide (AEA), as well as other free fatty acids, are unaltered. Conversely, levels of AA and AA-derived prostaglandins and other eicosanoids, including prostaglandin E2 (PGE2), D2 (PGD2), F2 (PGF2), and thromboxane B2 (TXB2), are strongly decreased. Phospholipase A₂ (PLA₂) enzymes have been viewed as the principal source of AA, but cPLA₂-deficient mice have unaltered AA levels in their brain, reinforcing the key role of MAGL in the brain for AA production and regulation of the brain inflammatory process.

Neuroinflammation is a common pathological change characteristic of diseases of the brain including, but not restricted to, neurodegenerative diseases (e.g. multiple sclerosis, Alzheimer's disease, Parkinson disease, amyotrophic lateral sclerosis, traumatic brain injury, neurotoxicity, stroke, epilepsy and mental disorders such as anxiety and migraine). In the brain, production of eicosanoids and prostaglandins controls the neuroinflammation process. The pro-inflammatory agent lipopolysaccharide (LPS) produces a robust, time-dependent increase in brain eicosanoids that is markedly blunted in Mgll-/- mice. LPS treatment also induces a widespread elevation in pro-inflammatory cytokines including interleukin-1-a (IL-1-a), IL-lb, IL-6, and tumor necrosis factor-a (TNF-a) that is prevented in Mgll-/- mice.

Neuroinflammation is characterized by the activation of the innate immune cells of the central nervous system, the microglia and the astrocytes. It has been reported that antiinflammatory drugs can suppress in preclinical models the activation of glia cells and the progression of disease including Alzheimer's disease and mutiple sclerosis (Lleo A., Cell Mol Life Sci. 2007, 64, 1403.). Importantly, genetic and/or pharmacological disruption of MAGL activity also blocks LPS-induced activation of microglial cells in the brain (Nomura, D.K., et al., Science 2011, 334, 809.).

In addition, genetic and/or pharmacological disruption of MAGL activity was shown to be protective in several animal models of neurodegeneration including, but not restricted to, Alzheimer's disease, Parkinson's disease and multiple sclerosis. For example, an irreversible MAGL inhibitor has been widely used in preclinical models of neuroinflammation and neurodegeneration (Long, J.Z., et al., Nature chemical biology 2009, 5, 37.). Systemic injection of such inhibitor recapitulates the Mgll-/- mice phenotype in the brain, including an increase in 2-AG levels, a reduction in AA levels and related eicosanoids production, as well as the prevention of cytokines production and microglia activation following LPS-induced neuroinflammation (Nomura, D.K., et al., Science 2011, 334, 809.), altogether confirming that MAGL is a druggable target.

Consecutive to the genetic and/or pharmacological disruption of MAGL activity, the endogenous levels of the MAGL natural substrate in the brain, 2-AG, are increased. 2-AG has been reported to show beneficial effects on pain with, for example, anti-nociceptive effects in mice (Ignatowska-Jankowska B. et al., J. Pharmacol. Exp. Ther. 2015, 353, 424.) and on mental disorders, such as depression in chronic stress models (Zhong P. et al., Neuropsychopharmacology 2014, 39, 1763.).

Furthermore, oligodendrocytes (OLs), the myelinating cells of the central nervous system, and their precursors (OPCs) express the cannabinoid receptor 2 (CB2) on their membrane. 2-AG is the endogenous ligand of CB1 and CB2 receptors. It has been reported that both cannabinoids and pharmacological inhibition of MAGL attenuate OLs's and OPCs's vulnerability to excitotoxic insults and therefore may be neuroprotective (Bernal-Chico, A., et al., Glia 2015, 63, 163.). Additionally, pharmacological inhibition of MAGL increases the number of myelinating OLs in the brain of mice, suggesting that MAGL inhibition may promote differentiation of OPCs in myelinating OLs in vivo (Alpar, A., et al., Nature communications 2014, 5, 4421.). Inhibition of MAGL was also shown to promote remyelination and functional recovery in a mouse model of progressive multiple sclerosis (Feliu A. et al., Journal of Neuroscience 2017, 37 (35), 8385.).

Finally, in recent years, metabolism is talked highly important in cancer research, especially the lipid metabolism. Researchers believe that the de novo fatty acid synthesis plays an important role in tumor development. Many studies illustrated that endocannabinoids have anti-tumorigenic actions, including anti-proliferation, apoptosis induction and anti-metastatic effects. MAGL as an important decomposing enzyme for both lipid metabolism and the endocannabinoids system, additionally as a part of a gene expression signature, contributes to different aspects of tumourigenesis (Qin, H., et al., Cell Biochem. Biophys. 2014, 70, 33*;* Nomura DK et al., Cell 2009, 140(1), 49-61; Nomura DK et al., Chem. Biol. 2011, 18(7), 846-856).

In conclusion, suppressing the action and/or the activation of MAGL is a promising new therapeutic strategy for the treatment or prevention of neuroinflammation, neurodegenerative diseases, pain, cancer and mental disorders. Furthermore, suppressing the action and/or the activation of MAGL is a promising new therapeutic strategy for providing neuroprotection and myelin regeneration. Accordingly, there is a high unmet medical need for new MAGL inhibitors.

### Summary of the Invention

In a first aspect, the present invention provides compounds of Formula (Ic) 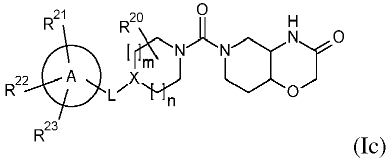 wherein A, L, X, m, n, and R²⁰ to R²³ are as defined herein.

In a further aspect, the present invention provides a process of manufacturing the compounds of formula (Ic) as described herein, comprising:
reacting 4a,5,6,7,8,8a-hexahydro-4H-pyrido[4,3-b][1,4]oxazin-3-one (1), 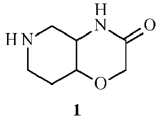
with a heterocyclic amine **2a,** wherein A, L, X, m, n, and R²⁰ to R²³ are as defined herein 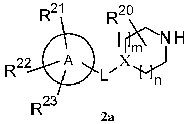
in the presence of a base and a urea forming reagent, to form said compounds of formula (Ic).

In a further aspect, the present invention provides a compound of formula (Ic) as described herein, when manufactured according to the processes described herein.

In a further aspect, the present invention provides a compound of formula (Ic) as described herein, for use as therapeutically active substance.

In a further aspect, the present invention provides a pharmaceutical composition comprising a compound of formula (Ic) as described herein and a therapeutically inert carrier.

In a further aspect, the present invention provides the use of a compound of formula (Ic) as described herein for inhibiting monoacylglycerol lipase (MAGL) in a mammal.

In a further aspect, the present invention provides the use of a compound of formula (Ic) as described herein for the treatment or prophylaxis of neuroinflammation, neurodegenerative diseases, pain, cancer and/or mental disorders in a mammal.

In a further aspect, the present invention provides the use of a compound of formula (Ic) as described herein for the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

In a further aspect, the present invention provides a compound of formula (Ic) as described herein for use in a method of inhibiting monoacylglycerol lipase in a mammal.

In a further aspect, the present invention provides a compound of formula (Ic) as described herein for use in the treatment or prophylaxis of neuroinflammation, neurodegenerative diseases, pain, cancer and/or mental disorders in a mammal.

In a further aspect, the present invention provides a compound of formula (Ic) as described herein, for use in the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

In a further aspect, the present invention provides the use of a compound of formula (Ic) as described herein for the preparation of a medicament for inhibiting monoacylglycerol lipase in a mammal.

In a further aspect, the present invention provides the use of a compound of formula (Ic) as described herein for the preparation of a medicament for the treatment or prophylaxis of neuroinflammation, neurodegenerative diseases, pain, cancer and/or mental disorders in a mammal.

In a further aspect, the present invention provides the use of a compound of formula (Ic) as described herein for the preparation of a medicament for the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

### Detailed Description of the Invention

### Definitions

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "alkyl" refers to a mono- or multivalent, e.g., a mono- or bivalent, linear or branched saturated hydrocarbon group of 1 to 12 carbon atoms. In some preferred embodiments, the alkyl group contains 1 to 6 carbon atoms, e.g., 1, 2, 3, 4, 5, or 6 carbon atoms. In other embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, iso-butyl, sec-butyl, tert-butyl, and 2,2-dimethylpropyl. A particularly preferred, yet non-limiting example of alkyl is methyl. An alkyl group may be substituted. Thus, the term "substituted alkyl" refers to an alkyl group wherein at least one of the hydrogen atoms of the alkyl group has been replaced by a substituent as described herein, preferably by a substituent selected from halogen, hydroxy, alkoxy, arylalkoxy, trialkylsilyloxy, substituted or unsubstituted cycloalkyl and substituted or unsubstituted heterocyclyl. Most preferably, "substituted alkyl" refers to an alkyl group wherein 1, 2 or 3 of the hydrogen atoms of the alkyl group have been replaced by a substituent selected from halogen, hydroxy, alkoxy, arylalkoxy, trialkylsilyloxy, substituted or unsubstituted cycloalkyl and substituted or unsubstituted heterocyclyl. Particular, yet non-limiting examples of substituted alkyl are 2-hydroxyethyl, 2-methoxyethyl, hydroxymethyl, methoxymethyl, trifluoromethyl, oxetan-3-yl-methyl, (1-tert-butoxycarbonylazetidin-3-yl)methyl, cyclopropylmethyl, 1-(chloromethyl)-2-hydroxy-ethyl, 2-[tert-butyl(dimethyl)silyl]oxyethyl and benzyloxymethyl.

The term "alkoxy" refers to an alkyl group, as previously defined, attached to the parent molecular moiety via an oxygen atom. Unless otherwise specified, the alkoxy group contains 1 to 12 carbon atoms. In some preferred embodiments, the alkoxy group contains 1 to 6 carbon atoms. In other embodiments, the alkoxy group contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "halogen" or "halo" refers to fluoro (F), chloro (Cl), bromo (Br), or iodo (I). Preferably, the term "halogen" or "halo" refers to fluoro (F), chloro (Cl) or bromo (Br). Particularly preferred, yet non-limiting examples of "halogen" or "halo" are fluoro (F) and chloro (Cl).

The term "cycloalkyl" as used herein refers to a saturated or partly unsaturated monocyclic or bicyclic hydrocarbon group of 3 to 10 ring carbon atoms. In some preferred embodiments, the cycloalkyl group is a saturated monocyclic hydrocarbon group of 3 to 8 ring carbon atoms. "Bicyclic cycloalkyl" refers to cycloalkyl moieties consisting of two saturated carbocycles having two carbon atoms in common, i.e., the bridge separating the two rings is either a single bond or a chain of one or two ring atoms, and to spirocyclic moieties, i.e., the two rings are connected via one common ring atom. Preferably, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl and cycloheptyl.

The term "cycloalkyloxy" refers to a group cycloalkyl-O-, i.e. a cycloalkyl group substituted with an oxy group and attached to the parent molecular moiety via said oxy group.

The term "heterocyclyl" as used herein refers to a saturated or partly unsaturated mono- or bicyclic, preferably monocyclic ring system of 3 to 10 ring atoms, preferably 3 to 8 ring atoms, wherein 1, 2, or 3 of said ring atoms are heteroatoms selected from N, O and S, the remaining ring atoms being carbon. Preferably, 1 to 2 of said ring atoms are selected from N and O, the remaining ring atoms being carbon. "Bicyclic heterocyclyl" refers to heterocyclic moieties consisting of two cycles having two ring atoms in common, i.e., the bridge separating the two rings is either a single bond or a chain of one or two ring atoms, and to spirocyclic moieties, i.e., the two rings are connected via one common ring atom. Some non-limiting examples of monocyclic heterocyclyl groups include azetidin-3-yl, azetidin-2-yl, oxetan-3-yl, oxetan-2-yl, 2-oxopyrrolidin-1-yl, 2-oxopyrrolidin-3-yl, 5-oxopyrrolidin-2-yl, 5-oxopyrrolidin-3-yl, 2-oxo-1-piperidyl, 2-oxo-3-piperidyl, 2-oxo-4-piperidyl, 6-oxo-2-piperidyl, 6-oxo-3-piperidyl, 1-piperidinyl, 2-piperidinyl, 3-piperidinyl, 4-piperidinyl, morpholino, morpholin-2-yl and morpholin-3-yl. A heterocyclyl group may be substituted. Thus, the term "substituted heterocyclyl" refers to a heterocyclyl group wherein at least one of the hydrogen atoms of the heterocyclyl group has been replaced by a substituent as described herein, preferably by a substituent selected from substituted or unsubstituted alkyl, halogen and alkoxy, wherein said substituted alkyl is substituted with 1-3 substituents selected from hydroxy, halogen, alkoxy, arylalkoxy and cycloalkyl. Most preferably, "substituted heterocyclyl" refers to a heterocyclyl group wherein 1-2 of the hydrogen atoms of the heterocyclyl group have been replaced by a substituent selected from substituted or unsubstituted alkyl, halogen and alkoxy, wherein said substituted alkyl is substituted with 1-3 substituents selected from hydroxy, halogen, alkoxy, arylalkoxy and cycloalkyl. Particular, yet non-limiting examples of substituted heterocyclyl are 2-methyl-5-oxo-pyrrolidin-1-yl, 4,4-difluoro-1-piperidyl, 1-tert-butoxycarbonylazetidin-3-yl and 1-tert-butoxycarbonylazetidin-2-yl.

The term "heterocyclyloxy" refers to a group heterocyclyl-O-, i.e. a heterocyclyl group substituted with an oxy group and attached to the parent molecular moiety via said oxy group. An non-limiting example of a heterocyclyloxy group is oxetanyloxy, such as oxetan-3-yloxy.

The term "aryl" refers to a monocyclic, bicyclic, or tricyclic carbocyclic ring system having a total of 6 to 14 ring members, preferably, 6 to 12 ring members, and more preferably 6 to 10 ring members, and wherein at least one ring in the system is aromatic. Some non-limiting examples of aryl include phenyl and 9H-fluorenyl (e.g. 9H-fluoren-9-yl). A particularly preferred, yet non-limiting example of aryl is phenyl. An aryl group may be substituted. Thus, the term "substituted aryl" refers to an aryl group wherein at least one of the hydrogen atoms of the aryl group has been replaced by a substituent as described herein, for example by a substituent selected from halogen, cyano, alkoxy, haloalkoxy, substituted or unsubstituted alkyl, substituted or unsubstituted cycloalkyl, substituted or unsubstituted cycloalkylalkyl, substituted or unsubstituted cycloalkyloxy, substituted or unsubstituted cycloalkyloxyalkyl, substituted or unsubstituted cycloalkylalkoxy, substituted or unsubstituted heterocyclyl, substituted or unsubstituted heterocyclyloxy, substituted or unsubstituted aryl and substituted or unsubstituted aryloxy. Preferably, the term "substituted aryl" refers to an aryl group wherein at least one of the hydrogen atoms of the aryl group has been replaced by a substituent selected from halogen and haloalkyl. Most preferably, "substituted aryl" refers to an aryl group wherein 1-3 of the hydrogen atoms of the aryl group have been replaced by a substituent selected from halogen and haloalkyl. Particular, yet non-limiting examples of substituted aryl are 4-fluorophenyl, 4-chlorophenyl, 2-chloro-4-fluoro-phenyl, 4-(trifluoromethyl)phenyl and 3,4-difluorophenyl.

The term "heteroaryl" refers to a mono- or multivalent, monocyclic, bicyclic or tricyclic, preferably bicyclic ring system having a total of 5 to 14 ring members, preferably, 5 to 12 ring members, and more preferably 5 to 10 ring members, wherein at least one ring in the system is aromatic, and at least one ring in the system contains one or more heteroatoms. Preferably, "heteroaryl" refers to a 5-10 membered heteroaryl comprising 1, 2, 3 or 4 heteroatoms independently selected from O, S and N. Most preferably, "heteroaryl" refers to a 5-10 membered heteroaryl comprising 1 to 2 heteroatoms independently selected from O and N. Some non-limiting examples of heteroaryl include spiro[cyclopropane-1,3'-indoline] (e.g., spiro[cyclopropane-1,3'-indoline]-1'-yl), 2-pyridyl, 3-pyridyl, 4-pyridyl, indol-1-yl, 1H-indol-2-yl, 1H-indol-3-yl, 1H-indol-4-yl, 1H-indol-5-yl, 1H-indol-6-yl, 1H-indol-7-yl, 1,2-benzoxazol-3-yl, 1,2-benzoxazol-4-yl, 1,2-benzoxazol-5-yl, 1,2-benzoxazol-6-yl, 1,2-benzoxazol-7-yl, 1H-indazol-3-yl, 1H-indazol-4-yl, 1H-indazol-5-yl, 1H-indazol-6-yl, 1H-indazol-7-yl, pyrazol-1-yl, 1H-pyrazol-3-yl, 1H-pyrazol-4-yl, 1H-pyrazol-5-yl, imidazol-1-yl, 1H-imidazol-2-yl, 1H-imidazol-4-yl, 1H-imidazol-5-yl, oxazol-2-yl, oxazol-4-yl and oxazol-5-yl. A particularly preferred, yet non-limiting example of heteroaryl is indolyl, in particular 1H-indol-3-yl. A heteroaryl group may be substituted. Thus, the term "substituted heteroaryl" refers to a heteroaryl group wherein at least one of the hydrogen atoms of the heteroaryl group has been replaced by a substituent as described herein, preferably by a substituent selected from halogen, substituted or unsubstituted alkyl, cycloalkyl, heterocyclyl and heterocyclyl substituted with alkoxycarbonyl, wherein substituted alkyl is substituted with 1-3 substituents selected from halogen, hydroxy, heterocyclyl, trialkylsilyloxy, cycloalkyl and heterocyclyl substituted with alkoxycarbonyl. Most preferably, "substituted heteroaryl" refers to a heteroaryl group wherein 1-2 of the hydrogen atoms of the heteroaryl group have been replaced by a substituent selected from halogen, substituted or unsubstituted alkyl, cycloalkyl, heterocyclyl and heterocyclyl substituted with alkoxycarbonyl, wherein substituted alkyl is substituted with 1-3 substituents selected from halogen, hydroxy, heterocyclyl, trialkylsilyloxy, cycloalkyl and heterocyclyl substituted with alkoxycarbonyl. Particular, yet non-limiting examples of substituted heteroaryl are 5-methyl-1,2,4-oxadiazol-3-yl, 5-fluoro-1-methyl-indol-3-yl, 5-chloro-1-methyl-indol-3-yl, 5-chloro-1-cyclopropyl-indol-3-yl, 5-chloro-1-oxetanyl-indol-3-yl, 5-chloro-1-(oxetan-3-ylmethyl)indol-3-yl, 5-chloro-1-(2-hydroxyethyl)indol-3-yl, 1-(1-tert-butoxycarbonylazetidin-3-yl)-5-chloro-indol-3-yl, 1-[(1-tert-butoxycarbonylazetidin-3-yl)methyl]-5-chloro-indol-3-yl, 5-(trifluoromethyl)-2-pyridyl, 5-(trifluoromethyl)-3-pyridyl, 4-(trifluoromethyl)imidazol-1-yl, 4-(trifluoromethyl)pyrazol-1-yl, 4-tert-butylpyrazol-1-yl, 4-tert-butyloxazol-2-yl, 5-chloro-1-(cyclopropylmethyl)indol-3-yl, 6-fluoro-1H-indol-3-yl, 5-fluoro-1,2-benzoxazol-3-yl, 5-chloro-1H-indol-3-yl, 1-methylindazol-5-yl, 5-chloro-1-[1-(chloromethyl)-2-hydroxy-ethyl]indol-3-yl and 1-[2-[tert-butyl(dimethyl)silyl]oxyethyl]-5-chloro-indol-3-yl.

The term "hydroxy" refers to an -OH group.

The term "cyano" refers to a -CN (nitrile) group.

The term "cycloalkylalkyl" refers to an alkyl group, wherein at least one of the hydrogen atoms of the alkyl group has been replaced by a cycloalkyl group. Preferably, "cycloalkylalkyl" refers to an alkyl group wherein 1, 2 or 3 hydrogen atoms, most preferably 1 hydrogen atom of the alkyl group have been replaced by a cycloalkyl group. A particularly preferred, yet non-limiting example of cycloalkylalkyl is cyclopropylmethyl.

The term "haloalkyl" refers to an alkyl group, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. Particularly preferred, yet non-limiting examples of haloalkyl are trifluoromethyl and trifluoroethyl.

The term "haloalkoxy" refers to an alkoxy group, wherein at least one of the hydrogen atoms of the alkoxy group has been replaced by a halogen atom, preferably fluoro. Preferably, "haloalkoxy" refers to an alkoxy group wherein 1, 2 or 3 hydrogen atoms of the alkoxy group have been replaced by a halogen atom, most preferably fluoro. A particularly preferred, yet non-limiting example of haloalkoxy is trifluoromethoxy (-OCF₃).

The term "cycloalkylalkoxy" refers to an alkoxy group, wherein at least one of the hydrogen atoms of the alkoxy group has been replaced by a cycloalkyl group. Preferably, "cycloalkylalkoxy" refers to an alkoxy group wherein 1, 2 or 3 hydrogen atoms, most preferably 1 hydrogen atom of the alkoxy group have been replaced by a cycloalkyl group. A particularly preferred, yet non-limiting example of cycloalkylalkoxy is cyclopropylmethoxy.

The term "cycloalkyloxyalkyl" refers to an alkyl group, wherein at least one of the hydrogen atoms of the alkyl group has been replaced by a cycloalkyloxy group as defined herein. Preferably, "cycloalkyloxyalkyl" refers to an alkyl group wherein 1, 2 or 3 hydrogen atoms, most preferably 1 hydrogen atom of the alkyl group have been replaced by a cycloalkyloxy group. A particularly preferred, yet non-limiting example of cycloalkyloxyalkyl is cyclopropoxymethyl.

The term "hydroxyalkyl" refers to an alkyl group, wherein at least one of the hydrogen atoms of the alkyl group has been replaced by a hydroxy group. Preferably, "hydroxyalkyl" refers to an alkyl group wherein 1, 2 or 3 hydrogen atoms, most preferably 1 hydrogen atom of the alkyl group have been replaced by a hydroxy group. Preferred, yet non-limiting examples of hydroxyalkyl are hydroxymethyl and hydroxyethyl (e.g. 2-hydroxyethyl). A particularly preferred, yet non-limiting example of hydroxyalkyl is hydroxymethyl.

The term "arylalkoxy" refers to an alkoxy group, wherein at least one of the hydrogen atoms of the alkoxy group has been replaced by an aryl group. Preferably, "arylalkoxy" refers to an alkoxy group wherein 1, 2 or 3 hydrogen atoms, most preferably 1 hydrogen atom of the alkoxy group have been replaced by an aryl group. A particularly preferred, yet non-limiting example of arylalkoxy is benzyloxy.

The term "arylalkoxyalkyl" refers to an alkyl group, wherein at least one of the hydrogen atoms of the alkyl group has been replaced by an arylalkoxy group as defined herein. Preferably, "arylalkoxyalkyl" refers to an alkyl group wherein 1, 2 or 3 hydrogen atoms, most preferably 1 hydrogen atom of the alkyl group have been replaced by an arylalkoxy group. A particularly preferred, yet non-limiting example of arylalkoxyalkyl is benzyloxymethyl.

The term "alkoxyalkyl" refers to an alkyl group, wherein at least one of the hydrogen atoms of the alkyl group has been replaced by an alkoxy group. Preferably, "alkoxyalkyl" refers to an alkyl group wherein 1, 2 or 3 hydrogen atoms, most preferably 1 hydrogen atom of the alkyl group have been replaced by an alkoxy group. A particularly preferred, yet non-limiting example of alkoxyalkyl is 2-methoxyethyl.

The term "alkoxycarbonyl" refers to a group alkyl-O-C(O)- (i.e. an alkylester).

The term "trialkylsilyloxy" refers to a group (alkyl)₃Si-O-. A particularly preferred, yet non-limiting example of trialkylsilyloxy is [tert-butyl(dimethyl)silyl]oxy.

The term "haloaryl" refers to an aryl group, wherein at least one of the hydrogen atoms of the aryl group has been replaced by a halogen atom. Preferably, "haloaryl" refers to an aryl group wherein 1, 2 or 3 hydrogen atoms, more preferably 1 or 2 hydrogen atoms, most preferably 1 hydrogen atom of the aryl group have been replaced by a halogen atom. A particularly preferred, yet non-limiting example of haloaryl is fluorophenyl, in particular 4-fluorophenyl.

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are hydrochloride salts.

The term "pharmaceutically acceptable ester" refers to esters that hydrolyze in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Representative examples of particular esters include, but are not limited to, formates, acetates, propionates, butyrates, acrylates and ethylsuccinates. Examples of pharmaceutically acceptable prodrug types are described in Higuchi and Stella, Pro-drugs as Novel Delivery Systems, Vol. 14 of the A.C.S. Symposium Series, and in Roche, ed., Bioreversible Carriers in Drug Design, American Pharmaceutical Association and Pergamon Press, 1987.

The term "protective group" (PG) denotes the group which selectively blocks a reactive site in a multifunctional compound such that a chemical reaction can be carried out selectively at another unprotected reactive site in the meaning conventionally associated with it in synthetic chemistry. Protective groups can be removed at the appropriate point. Exemplary protective groups are amino-protective groups, carboxy-protective groups or hydroxy-protective groups. Particular protective groups are the tert-butoxycarbonyl (Boc), benzyloxycarbonyl (Cbz), fluorenylmethoxycarbonyl (Fmoc) and benzyl (Bn). Further particular protective groups are the tert-butoxycarbonyl (Boc) and the fluorenylmethoxycarbonyl (Fmoc). More particular protective group is the tert-butoxycarbonyl (Boc). Exemplary protective groups and their application in organic synthesis are described, for example, in "Protective Groups in Organic Chemistry" by T. W. Greene and P. G. M. Wutts, 5th Ed., 2014, John Wiley & Sons, N.Y.

The term "urea forming reagent" refers to a chemical compound that is able to render a first amine to a species that will react with a second amine, thereby forming an urea derivative. Non-limiting examples of a urea forming reagent include bis(trichloromethyl) carbonate, phosgene, trichloromethyl chloroformate, (4-nitrophenyl)carbonate and 1,1'-carbonyldiimidazole. The urea forming reagents described in G. Sartori et al., Green Chemistry 2000, 2, 140 are incorporated herein by reference.

The compounds of formula (I) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates.

According to the Cahn-Ingold-Prelog Convention, the asymmetric carbon atom can be of the "R" or "S" configuration.

The abbreviation "MAGL" refers to the enzyme monoacylglycerol lipase. The terms "MAGL" and "monoacylglycerol lipase" are used herein interchangeably.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prophylaxis" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

The term "neuroinflammation" as used herein relates to acute and chronic inflammation of the nervous tissue, which is the main tissue component of the two parts of the nervous system; the brain and spinal cord of the central nervous system (CNS), and the branching peripheral nerves of the peripheral nervous system (PNS). Chronic neuroinflammation is associated with neurodegenerative diseases such as Alzheimer's disease, Parkinson's disease and multiple sclerosis. Acute neuroinflammation usually follows injury to the central nervous system immediately, e.g., as a result of traumatic brain injury (TBI).

The term "traumatic brain injury" ("TBI", also known as "intracranial injury"), relates to damage to the brain resulting from external mechanical force, such as rapid acceleration or deceleration, impact, blast waves, or penetration by a projectile.

The term "neurodegenerative diseases" relates to diseases that are related to the progressive loss of structure or function of neurons, including death of neurons. Examples of neurodegenerative diseases include, but are not limited to, multiple sclerosis, Alzheimer's disease, Parkinson's disease and amyotrophic lateral sclerosis.

The term "mental disorders" (also called mental illnesses or psychiatric disorders) relates to behavioral or mental patterns that may cause suffering or a poor ability to function in life. Such features may be persistent, relapsing and remitting, or occur as a single episode. Examples of mental disorders include, but are not limited to, anxiety and depression.

The term "pain" relates to an unpleasant sensory and emotional experience associated with actual or potential tissue damage. Examples of pain include, but are not limited to, nociceptive pain, chronic pain (including idiopathic pain), neuropathic pain including chemotherapy induced neuropathy, phantom pain and phsychogenic pain. A particular example of pain is neuropathic pain, which is caused by damage or disease affecting any part of the nervous system involved in bodily feelings (i.e., the somatosensory system). In one embodiment, "pain" is neuropathic pain resulting from amputation or thoracotomy. In one embodiment, "pain" is chemotherapy induced neuropathy.

The term "neurotoxicity" relates to toxicity in the nervous system. It occurs when exposure to natural or artificial toxic substances (neurotoxins) alter the normal activity of the nervous system in such a way as to cause damage to nervous tissue. Examples of neurotoxicity include, but are not limited to, neurotoxicity resulting from exposure to substances used in chemotherapy, radiation treatment, drug therapies, drug abuse, and organ transplants, as well as exposure to heavy metals, certain foods and food additives, pesticides, industrial and/or cleaning solvents, cosmetics, and some naturally occurring substances.

The term "cancer" refers to a disease characterized by the presence of a neoplasm or tumor resulting from abnormal uncontrolled growth of cells (such cells being "cancer cells"). As used herein, the term cancer explicitly includes, but is not limited to, hepatocellular carcinoma, colon carcinogenesis and ovarian cancer.

The term "mammal" as used herein includes both humans and non-humans and includes but is not limited to humans, non-human primates, canines, felines, murines, bovines, equines, and porcines. In a particularly preferred embodiment, the term "mammal" refers to humans.

### Compounds of the Invention

The present invention provides a compound of formula (Ic) 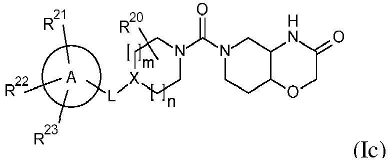 or a pharmaceutically acceptable salt thereof, wherein:
- L: is selected from -CR¹R²-(CH₂)ₚ-, -(CH₂)ₚ-CR¹R²-, -OCR³R⁴-, -CR³R⁴O- and a covalent bond;
- m: is 0, n is 0 or 1 and X is CR²⁴; or
- m: is 1, n is 1 or 2 and X is CR²⁴ or N;
- p: is 0, 1 or 2;
- R¹: is selected from halogen, C₁₋₆-alkoxy, C₁₋₆-alkyl, halo-C₁₋₆-alkyl, hydroxy-C₁₋₆-alkyl, C₁₋₆-alkoxy-C₁₋₆-alkyl-, halo-C₁₋₆-alkoxy, halo-C₁₋₆-alkoxy-C₁₋₆-alkyl-, cyano, and a group 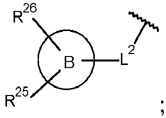 and R² is selected from hydrogen, halogen, and hydroxy; or
- R¹ and R²,: taken together with the carbon atom to which they are attached, form a C₃₋₁₂-cycloalkyl or a C₂₋₉-heterocyclyl;
- R³: is selected from C₁₋₆-alkyl, halo-C₁₋₆-alkyl, C₃₋₁₂-cycloalkyl, and C₆₋₁₄-aryl; and R⁴ is hydrogen; or
- R³ and R⁴,: taken together with the carbon atom to which they are attached, form a C₃₋₁₂-cycloalkyl or a C₂₋₉-heterocyclyl;
- R²⁰: is hydrogen or C₁₋₆-alkyl;
- R²¹, R²², and R²³: are independently selected from hydrogen, halogen, cyano, hydroxy, C₁₋₆-alkoxy, halo-C₁₋₆-alkoxy, C₁₋₆-alkyl, halo-C₁₋₆-alkyl, hydroxy-C₁₋₆-alkyl, (halo-C₁₋₆-alkyl)-hydroxy-C₁₋₆-alkyl, C₁₋₆-alkoxycarbonyl-C₁₋₆-alkyl-, C₁₋₆-alkoxycarbonyl-NH-C₁₋₆-alkoxy-, C₁₋₆-alkoxycarbonyl-NH-(C₁₋₆-alkoxy)₂-C₁₋₆-alkyl-C(O)-NH-C₁₋₆-alkoxy-, C₁₋₆-alkoxycarbonyl-NH-C₁₋₆-alkoxy-C₁₋₆-alkyl-C(O)-NH-C₁₋₆-alkoxy-, SF₅, (C₁₋₆-alkyl)₃Si-O-C₁₋₆-alkyl-, a group 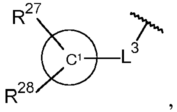 and a group 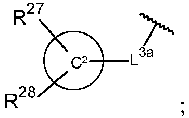
- R²⁴: is selected from hydrogen, halogen, hydroxy, halo-C₁₋₆-alkyl and C₁₋₆-alkyl;
- R²⁵ and R²⁶: are independently selected from hydrogen, halogen, C₁₋₆-alkyl, C₁₋₆-alkoxy, halo-C₁₋₆-alkyl, halo-C₁₋₆-alkoxy, C₁₋₆-alkyl-SO₂-, and C₃₋₁₂-cycloalkyl;
- R²⁷ and R²⁸: are independently selected from hydrogen, halogen, C₁₋₆-alkoxy, halo-C₁₋₆-alkoxy, C₁₋₆-alkyl, halo-C₁₋₆-alkyl, hydroxy-C₁₋₆-alkyl, halogen, hydroxy, C₁₋₆-alkylsulfonyl, carbamoyl, cyano, cycloalkyl-C₁₋₆-alkoxy-, C₁₋₆-alkyl-NH-C(O)-, and cycloalkyl;
- A, and B: are independently selected from C₆₋₁₄-aryl, C₁₋₁₃-heteroaryl, C₃₋₁₂-cycloalkyl, and C₂₋₉-heterocyclyl;
- C¹: is C₃₋₁₂-cycloalkyl or C₂₋₉-heterocyclyl;
- C²: is C₆₋₁₄-aryl;
- L²: is selected from a covalent bond, -O-, -CH₂O-, -CH₂OCH₂-, and -CH₂-;
- L³: is selected from a covalent bond, -O-, -CH₂O-, -OCH₂-, -CH₂OCH₂-, and-CH₂-; and
- L^{3a}: is selected from a covalent bond, -CH₂O-, -OCH₂-, -CH₂OCH₂-, and -CH₂-.

In one embodiment, the present invention provides a compound of formula (Ic) as described herein, or a pharmaceutically acceptable salt thereof, wherein the compound of formula (Ic) is a compound of formula (Id): 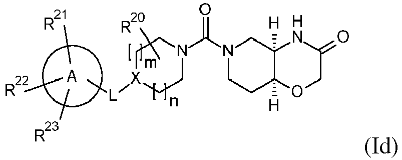 wherein A, L, X, m, n, and R²⁰- R²³ are as defined in claim 1.

In one embodiment, the present invention provides a compound of formula (Ic) as described herein, or a pharmaceutically acceptable salt thereof, wherein the compound of formula (Ic) is a compound of formula (Ie): 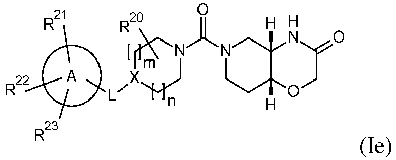 wherein A, L, X, m, n, and R²⁰ - R²³ are as defined in claim 1.

In one embodiment, the present invention provides a compound of formula (Ic) as described herein, or a pharmaceutically acceptable salt thereof, wherein the compound of formula (Ic) is a compound of formula (If): 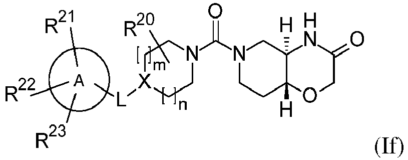 wherein A, L, X, m, n, and R²⁰- R²³ are as defined in claim 1.

In one embodiment, the present invention provides a compound of formula (Ic) as described herein, or a pharmaceutically acceptable salt thereof, wherein the compound of formula (Ic) is a compound of formula (Ig): 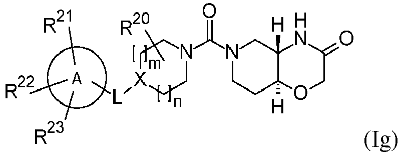 wherein A, L, X, m, n, and R²⁰- R²³ are as defined in claim 1.

In one embodiment, the present invention provides a compound of formula (Ic) as described herein, or a pharmaceutically acceptable salt thereof, wherein:
m is 0, n is 0 or 1 and X is CR²⁴; or
m is 1, n is 1 and X is CR²⁴ or N.

In one embodiment, the present invention provides a compound of formula (Ic) as described herein, or a pharmaceutically acceptable salt thereof, wherein p is 0 or 1.

In a preferred embodiment, the present invention provides a compound of formula (Ic) as described herein, or a pharmaceutically acceptable salt thereof, wherein p is 0.

In one embodiment, the present invention provides a compound of formula (Ic) as described herein, or a pharmaceutically acceptable salt thereof, wherein:
R¹ is selected from halogen, C₁₋₆-alkoxy, C₁₋₆-alkyl, halo-C₁₋₆-alkyl, hydroxy-C₁₋₆-alkyl, C₁₋₆-alkoxy-C₁₋₆-alkyl-, halo-C₁₋₆-alkoxy, halo-C₁₋₆-alkoxy-C₁₋₆-alkyl-, cyano, and a group 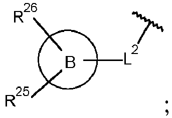 and R² is selected from hydrogen, halogen, and hydroxy; or
R¹ and R², taken together with the carbon atom to which they are attached, form a C₃₋₁₂-cycloalkyl.

In a preferred embodiment, the present invention provides a compound of formula (Ic) as described herein, or a pharmaceutically acceptable salt thereof, wherein R¹ is selected from halogen, C₁₋₆-alkyl, hydroxy-C₁₋₆-alkyl, C₁₋₆-alkoxy-C₁₋₆-alkyl-, halo-C₁₋₆-alkoxy, and a group 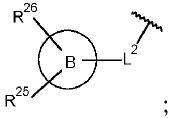 and R² is hydrogen or halogen.

In a particularly preferred embodiment, the present invention provides a compound of formula (Ic) as described herein, or a pharmaceutically acceptable salt thereof, wherein R¹ is selected from 2-methoxyethyl, methyl, 2,2,2-trifluoroethoxy, fluoro, 2-hydroxyethyl, and a group 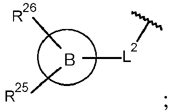 and R² is hydrogen or fluoro.

In one embodiment, the present invention provides a compound of formula (Ic) as described herein, or a pharmaceutically acceptable salt thereof, wherein R³ is C₁₋₆-alkyl or halo-C₁₋₆-alkyl; and R⁴ is hydrogen.

In a preferred embodiment, the present invention provides a compound of formula (Ic) as described herein, or a pharmaceutically acceptable salt thereof, wherein R³ is C₁₋₆-alkyl; and R⁴ is hydrogen.

In a particularly preferred embodiment, the present invention provides a compound of formula (Ic) as described herein, or a pharmaceutically acceptable salt thereof, wherein R³ is methyl; and R⁴ is hydrogen.

In a preferred embodiment, the present invention provides a compound of formula (Ic) as described herein, or a pharmaceutically acceptable salt thereof, wherein R²⁰ is hydrogen.

In one embodiment, the present invention provides a compound of formula (Ic) as described herein, or a pharmaceutically acceptable salt thereof, wherein R²¹ is selected from hydrogen, halogen, hydroxy, C₁₋₆-alkoxy, halo-C₁₋₆-alkoxy, C₁₋₆-alkyl, halo-C₁₋₆-alkyl, hydroxy-C₁₋₆-alkyl, (halo-C₁₋₆-alkyl)-hydroxy-C₁₋₆-alkyl, C₁₋₆-alkoxycarbonyl-C₁₋₆-alkyl-, C₁₋₆-alkoxycarbonyl-NH-C₁₋₆-alkoxy-, C₁₋₆-alkoxycarbonyl-NH-(C₁₋₆-alkoxy)₂-C₁₋₆-alkyl-C(O)-NH-C₁₋₆-alkoxy-, C₁₋₆-alkoxycarbonyl-NH-C₁₋₆-alkoxy-C₁₋₆-alkyl-C(O)-NH-C₁₋₆-alkoxy-, SF₅, (C₁₋₆-alkyl)₃Si-O-C₁₋₆-alkyl-, a group 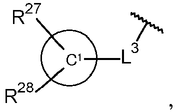 and a group 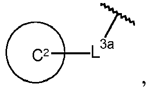 wherein R²⁷, R²⁸, C¹, C², L³ and L^{3a} are as defined herein.

In a preferred embodiment, the present invention provides a compound of formula (Ic) as described herein, or a pharmaceutically acceptable salt thereof, wherein R²¹ is selected from halogen, C₁₋₆-alkoxy, halo-C₁₋₆-alkoxy, C₁₋₆-alkyl, halo-C₁₋₆-alkyl, SF₅, C₆₋₁₄-aryl, and a group 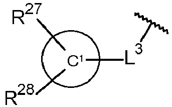 , wherein R²⁷, R²⁸, C¹ and L³ are as defined herein.

In a particularly preferred embodiment, the present invention provides a compound of formula (Ic) as described herein, or a pharmaceutically acceptable salt thereof, wherein R²¹ is selected from fluoro, chloro, bromo, methyl, methoxy, tert-butyl, propyl, trifluoromethoxy, 2-fluoroethoxy, 2,2,2-trifluoroethoxy, trifluoromethyl, 2,2,2-trifluoroethyl, 1,1-difluoroethyl, SF₅, phenyl, and a group 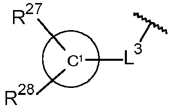 , wherein R²⁷, R²⁸, C¹ and L³ are as defined herein.

In one embodiment, the present invention provides a compound of formula (Ic) as described herein, or a pharmaceutically acceptable salt thereof, wherein R²² is selected from hydrogen, halogen, C₁₋₆-alkoxy, halo-C₁₋₆-alkoxy, C₁₋₆-alkyl, and cyano.

In a preferred embodiment, the present invention provides a compound of formula (Ic) as described herein, or a pharmaceutically acceptable salt thereof, wherein R²² is selected from hydrogen, halogen, C₁₋₆-alkoxy and halo-C₁₋₆-alkoxy.

In a particularly preferred embodiment, the present invention provides a compound of formula (Ic) as described herein, or a pharmaceutically acceptable salt thereof, wherein R²² is selected from hydrogen, fluoro, chloro, methoxy, methyl, and trifluoromethyl.

In a preferred embodiment, the present invention provides a compound of formula (Ic) as described herein, or a pharmaceutically acceptable salt thereof, wherein R²³ is hydrogen or halogen.

In a particularly preferred embodiment, the present invention provides a compound of formula (Ic) as described herein, or a pharmaceutically acceptable salt thereof, wherein R²³ is hydrogen or fluoro.

In one embodiment, the present invention provides a compound of formula (Ic) as described herein, or a pharmaceutically acceptable salt thereof, wherein R²⁴ is hydrogen.

In one embodiment, the present invention provides a compound of formula (Ic) as described herein, or a pharmaceutically acceptable salt thereof, wherein R²⁵ is selected from hydrogen, halogen, C₁₋₆-alkyl, C₁₋₆-alkoxy, halo-C₁₋₆-alkyl, halo-C₁₋₆-alkoxy, C₁₋₆-alkyl-SO₂-, and C₃₋₁₂-cycloalkyl.

In a preferred embodiment, the present invention provides a compound of formula (Ic) as described herein, or a pharmaceutically acceptable salt thereof, wherein R²⁵ is selected from hydrogen, halogen, C₁₋₆-alkoxy, and C₃₋₁₂-cycloalkyl.

In a particularly preferred embodiment, the present invention provides a compound of formula (Ic) as described herein, or a pharmaceutically acceptable salt thereof, wherein R²⁵ is selected from hydrogen, methoxy, fluoro, and cyclopropyl.

In one embodiment, the present invention provides a compound of formula (Ic) as described herein, or a pharmaceutically acceptable salt thereof, wherein R²⁶ is selected from hydrogen, C₁₋₆-alkyl, and C₁₋₆-alkoxy.

In a preferred embodiment, the present invention provides a compound of formula (Ic) as described herein, or a pharmaceutically acceptable salt thereof, wherein R²⁶ is hydrogen or C₁₋₆-alkoxy.

In a particularly preferred embodiment, the present invention provides a compound of formula (Ic) as described herein, or a pharmaceutically acceptable salt thereof, wherein R²⁶ is hydrogen or methoxy.

In a preferred embodiment, the present invention provides a compound of formula (Ic) as described herein, or a pharmaceutically acceptable salt thereof, wherein R²⁷ is selected from hydrogen, halo-C₁₋₆-alkoxy, C₁₋₆-alkyl, halo-C₁₋₆-alkyl, and halogen.

In a particularly preferred embodiment, the present invention provides a compound of formula (Ic) as described herein, or a pharmaceutically acceptable salt thereof, wherein R²⁷ is selected from methyl, trifluoromethoxy, trifluoromethyl, 2,2,2-trifluoro-1-methyl-ethoxy, 2,2,2-trifluoro-1,1-dimethyl-ethoxy, 2,2,2-trifluoroethoxy, fluoro, and chloro.

In a preferred embodiment, the present invention provides a compound of formula (Ic) as described herein, or a pharmaceutically acceptable salt thereof, wherein R²⁸ is selected from hydrogen, C₁₋₆-alkyl, and halogen.

In a particularly preferred embodiment, the present invention provides a compound of formula (Ic) as described herein, or a pharmaceutically acceptable salt thereof, wherein R²⁸ is selected from hydrogen, methyl, fluoro, and chloro.

In a preferred embodiment, the present invention provides a compound of formula (Ic) as described herein, or a pharmaceutically acceptable salt thereof, wherein A is C₆₋₁₄-aryl or C₁₋₁₃-heteroaryl.

In a particularly preferred embodiment, the present invention provides a compound of formula (Ic) as described herein, or a pharmaceutically acceptable salt thereof, wherein A is selected from phenyl, indol-3-yl, 2-pyridyl, and 3-pyridyl.

In a preferred embodiment, the present invention provides a compound of formula (Ic) as described herein, or a pharmaceutically acceptable salt thereof, wherein B is C₆₋₁₄-aryl or C₁₋₁₃-heteroaryl.

In a particularly preferred embodiment, the present invention provides a compound of formula (Ic) as described herein, or a pharmaceutically acceptable salt thereof, wherein B is phenyl or 1,2,4-oxadiazol-5-yl.

In a preferred embodiment, the present invention provides a compound of formula (Ic) as described herein, or a pharmaceutically acceptable salt thereof, wherein L is selected from -CR¹R²-(CH₂)ₚ-, -OCR³R⁴-, -CR³R⁴O- and a covalent bond; wherein R¹ to R⁴ and p are as defined herein.

In one embodiment, the present invention provides a compound of formula (Ic) as described herein, or a pharmaceutically acceptable salt thereof, wherein L² is selected from a covalent bond, -O-, and -CH₂-.

In a preferred embodiment, the present invention provides a compound of formula (Ic) as described herein, or a pharmaceutically acceptable salt thereof, wherein L² is a covalent bond.

In one embodiment, the present invention provides a compound of formula (Ic) as described herein, or a pharmaceutically acceptable salt thereof, wherein:
- m: is 0, n is 0 or 1 and X is CR²⁴; or
- m: is 1, n is 1 and X is CR²⁴ or N;
- L: is selected from -CR¹R²-(CH₂)ₚ-, -OCHR³-, -CHR³O- and a covalent bond;
- p: is 0 or 1;
- R¹: is selected from halogen, C₁₋₆-alkoxy, C₁₋₆-alkyl, halo-C₁₋₆-alkyl, hydroxy-C₁₋₆-alkyl, C₁₋₆-alkoxy-C₁₋₆-alkyl-, halo-C₁₋₆-alkoxy, halo-C₁₋₆-alkoxy-C₁₋₆-alkyl-, cyano, and a group 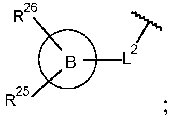 and R² is selected from hydrogen, halogen, and hydroxy; or
- R¹ and R²,: taken together with the carbon atom to which they are attached, form a C₃₋₁₂-cycloalkyl;
- R³: is C₁₋₆-alkyl or halo-C₁₋₆-alkyl;
- R²⁰: is hydrogen or C₁₋₆-alkyl;
- R²¹: is selected from hydrogen, halogen, hydroxy, C₁₋₆-alkoxy, halo-C₁₋₆-alkoxy, C₁₋₆-alkyl, halo-C₁₋₆-alkyl, hydroxy-C₁₋₆-alkyl, (halo-C₁₋₆-alkyl)-hydroxy-C₁₋₆-alkyl, C₁₋₆-alkoxycarbonyl-C₁₋₆-alkyl-, C₁₋₆-alkoxycarbonyl-NH-C₁₋₆-alkoxy-, SF₅, (C₁₋₆-alkyl)₃Si-O-C₁₋₆-alkyl-, a group 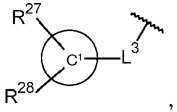 and a group 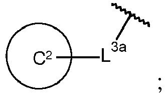
- R²²: is selected from hydrogen, halogen, C₁₋₆-alkoxy, halo-C₁₋₆-alkoxy, C₁₋₆-alkyl, and cyano;
- R²³: is hydrogen or halogen;
- R²⁴: is selected from hydrogen, halogen, hydroxy, and C₁₋₆-alkyl;
- R²⁵: is selected from hydrogen, halogen, C₁₋₆-alkyl, C₁₋₆-alkoxy, halo-C₁₋₆-alkyl, halo-C₁₋₆-alkoxy, C₁₋₆-alkyl-SO₂-, and C₃₋₁₂-cycloalkyl;
- R²⁶: is selected from hydrogen, C₁₋₆-alkyl, and C₁₋₆-alkoxy;
- R²⁷: is selected from hydrogen, halogen, C₁₋₆-alkoxy, halo-C₁₋₆-alkoxy, C₁₋₆-alkyl, halo-C₁₋₆-alkyl, hydroxy-C₁₋₆-alkyl, halogen, hydroxy, C₁₋₆-alkylsulfonyl, carbamoyl, cyano, cycloalkyl-C₁₋₆-alkoxy-, and cycloalkyl;
- R²⁸: is selected from hydrogen, C₁₋₆-alkyl, and halogen;
- A: is C₆₋₁₄-aryl or C₁₋₁₃-heteroaryl;
- B: is C₆₋₁₄-aryl or C₁₋₁₃-heteroaryl;
- C¹: is C₃₋₁₂-cycloalkyl or C₂₋₉-heterocyclyl;
- C²: is C₆₋₁₄-aryl;
- L²: is selected from a covalent bond, -O-, and -CH₂-;
- L³: is selected from a covalent bond, -CH₂O-, and -CH₂-; and
- L^{3a}: is a covalent bond or -CH₂-.

In a preferred embodiment, the present invention provides a compound of formula (Ic) as described herein, or a pharmaceutically acceptable salt thereof, wherein:
- m: is 0, n is 0 or 1 and X is CH; or
- m: is 1, n is 1 and X is CH or N;
- L: is selected from -CR¹R²-, -OCHR³-, -CHR³O- and a covalent bond;
- R¹: is selected from halogen, C₁₋₆-alkyl, hydroxy-C₁₋₆-alkyl, C₁₋₆-alkoxy-C₁₋₆-alkyl-, halo-C₁₋₆-alkoxy, and a group 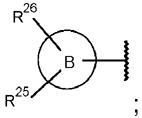
- R²: is hydrogen or halogen;
- R³: is C₁₋₆-alkyl;
- R²⁰: is hydrogen;
- R²¹: is selected from halogen, C₁₋₆-alkoxy, halo-C₁₋₆-alkoxy, C₁₋₆-alkyl, halo-C₁₋₆-alkyl, SF₅, C₆₋₁₄-aryl, and a group 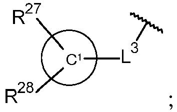
R²² is selected from hydrogen, halogen, C₁₋₆-alkoxy and halo-C₁₋₆-alkoxy;
R²³ is hydrogen or halogen;
R²⁵ is selected from hydrogen, halogen, C₁₋₆-alkoxy, and C₃₋₁₂-cycloalkyl;
R²⁶ is hydrogen or C₁₋₆-alkoxy;
R²⁷ is selected from hydrogen, halo-C₁₋₆-alkoxy, C₁₋₆-alkyl, halo-C₁₋₆-alkyl, and halogen;
R²⁸ is selected from hydrogen, C₁₋₆-alkyl, and halogen;
A is C₆₋₁₄-aryl or C₁₋₁₃-heteroaryl;
B is C₆₋₁₄-aryl or C₁₋₁₃-heteroaryl;
C¹ is C₃₋₁₂-cycloalkyl or C₂₋₉-heterocyclyl;
L³ is a covalent bond or -CH₂-.

In a particularly preferred embodiment, the present invention provides a compound of formula (Ic) as described herein, or a pharmaceutically acceptable salt thereof, wherein:
- m: is 0, n is 0 or 1 and X is CH; or
- m: is 1, n is 1 and X is CH or N;
- L: is selected from -CR¹R²-, -OCHR³-, -CHR³O- and a covalent bond;
- R¹: is selected from 2-methoxyethyl, methyl, 2,2,2-trifluoroethoxy, fluoro, 2-hydroxyethyl, and a group 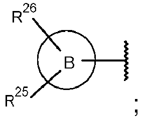
R² is hydrogen or fluoro;
R³ is methyl;
R²⁰ is hydrogen;
R²¹ is selected from fluoro, chloro, bromo, methyl, methoxy, tert-butyl, propyl, trifluoromethoxy, 2-fluoroethoxy, 2,2,2-trifluoroethoxy, trifluoromethyl, 2,2,2-trifluoroethyl, 1,1-difluoroethyl, SF₅, phenyl, and a group 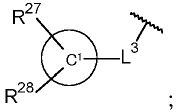
R²² is selected from hydrogen, fluoro, chloro, methoxy, methyl, and trifluoromethyl;
R²³ is hydrogen or fluoro;
R²⁵ is selected from hydrogen, methoxy, fluoro, and cyclopropyl;
R²⁶ is hydrogen or methoxy;
R²⁷ is selected from methyl, trifluoromethoxy, trifluoromethyl, 2,2,2-trifluoro-1-methyl-ethoxy, 2,2,2-trifluoro-1,1-dimethyl-ethoxy, 2,2,2-trifluoroethoxy, fluoro, and chloro;
R²⁸ is selected from hydrogen, methyl, fluoro, and chloro;
A is selected from phenyl, indol-3-yl, 2-pyridyl, and 3-pyridyl;
B is phenyl or 1,2,4-oxadiazol-5-yl;
C¹ is selected from azetidin-1-yl, pyrrolidin-1-yl, cyclopropyl, and oxetan-3-yl; and
L³ is a covalent bond or -CH₂-.

In one embodiment, the present invention provides a compound of formula (Ic) as described herein, or a pharmaceutically acceptable salt thereof, wherein:
- m: is 0, n is 0 or 1 and X is CR²⁴; or
- m: is 1, n is 1 and X is CR²⁴ or N; and
- R²⁴: is selected from hydrogen, halogen, hydroxy, and C₁₋₆-alkyl.

In a preferred embodiment, the present invention provides a compound of formula (Ic) as described herein, or a pharmaceutically acceptable salt thereof, wherein:
- m: is 0, n is 0 or 1 and X is CH; or
- m: is 1, n is 1 and X is CH or N.

In one embodiment, the present invention provides a compound of formula (Ic) as described herein, or a pharmaceutically acceptable salt thereof, wherein:
- L: is selected from -CR¹R²-(CH₂)ₚ-, -OCHR³-, -CHR³O- and a covalent bond;
- p: is 0 or 1;
- R¹: is selected from halogen, C₁₋₆-alkoxy, C₁₋₆-alkyl, halo-C₁₋₆-alkyl, hydroxy-C₁₋₆-alkyl, C₁₋₆-alkoxy-C₁₋₆-alkyl-, halo-C₁₋₆-alkoxy, halo-C₁₋₆-alkoxy-C₁₋₆-alkyl-, cyano, and a group 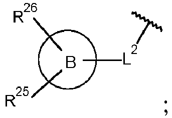 and R² is selected from hydrogen, halogen, and hydroxy; or
- R¹ and R²,: taken together with the carbon atom to which they are attached, form a C₃₋₁₂-cycloalkyl;
- R³: is C₁₋₆-alkyl or halo-C₁₋₆-alkyl;
- R²⁵: is selected from hydrogen, halogen, C₁₋₆-alkyl, C₁₋₆-alkoxy, halo-C₁₋₆-alkyl, halo-C₁₋₆-alkoxy, C₁₋₆-alkyl-SO₂-, and C₃₋₁₂-cycloalkyl;
- R²⁶: is selected from hydrogen, C₁₋₆-alkyl, and C₁₋₆-alkoxy;
- B: is C₆₋₁₄-aryl or C₁₋₁₃-heteroaryl; and
- L²: is selected from a covalent bond, -O-, and -CH₂-.

In a preferred embodiment, the present invention provides a compound of formula (Ic) as described herein, or a pharmaceutically acceptable salt thereof, wherein:
- L: is selected from -CR¹R²-, -OCHR³-, -CHR³O- and a covalent bond;
- R¹: is selected from halogen, C₁₋₆-alkyl, hydroxy-C₁₋₆-alkyl, C₁₋₆-alkoxy-C₁₋₆-alkyl-, halo-C₁₋₆-alkoxy, and a group

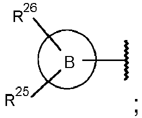

- R²: is hydrogen or halogen;
- R³: is C₁₋₆-alkyl;
- R²⁵: is selected from hydrogen, halogen, C₁₋₆-alkoxy, and C₃₋₁₂-cycloalkyl;
- R²⁶: is hydrogen or C₁₋₆-alkoxy; and
- B: is C₆₋₁₄-aryl or C₁₋₁₃-heteroaryl.

In a particularly preferred embodiment, the present invention provides a compound of formula (Ic) as described herein, or a pharmaceutically acceptable salt thereof, wherein:
- L: is selected from -CR¹R²-, -OCHR³-, -CHR³O- and a covalent bond;
- R¹: is selected from 2-methoxyethyl, methyl, 2,2,2-trifluoroethoxy, fluoro, 2-hydroxyethyl, and a group 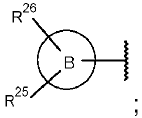
- R²: is hydrogen or fluoro;
- R³: is methyl;
- R²⁵: is selected from hydrogen, methoxy, fluoro, and cyclopropyl;
- R²⁶: is hydrogen or methoxy; and
- B: is phenyl or 1,2,4-oxadiazol-5-yl.

In one embodiment, the present invention provides a compound of formula (Ic) as described herein, or a pharmaceutically acceptable salt thereof, wherein:
- R²¹: is selected from hydrogen, halogen, hydroxy, C₁₋₆-alkoxy, halo-C₁₋₆-alkoxy, C₁₋₆-alkyl, halo-C₁₋₆-alkyl, hydroxy-C₁₋₆-alkyl, (halo-C₁₋₆-alkyl)-hydroxy-C₁₋₆-alkyl, C₁₋₆-alkoxycarbonyl-C₁₋₆-alkyl-, C₁₋₆-alkoxycarbonyl-NH-C₁₋₆-alkoxy-, SF₅, (C₁₋₆-alkyl)₃Si-O-C₁₋₆-alkyl-, a group 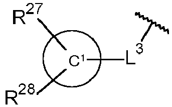 , and a group 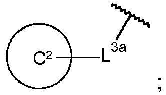
- R²²: is selected from hydrogen, halogen, C₁₋₆-alkoxy, halo-C₁₋₆-alkoxy, C₁₋₆-alkyl, and cyano;
- R²³: is hydrogen or halogen;
- R²⁷: is selected from hydrogen, halogen, C₁₋₆-alkoxy, halo-C₁₋₆-alkoxy, C₁₋₆-alkyl, halo-C₁₋₆-alkyl, hydroxy-C₁₋₆-alkyl, halogen, hydroxy, C₁₋₆-alkylsulfonyl, carbamoyl, cyano, cycloalkyl-C₁₋₆-alkoxy-, and cycloalkyl;
- R²⁸: is selected from hydrogen, C₁₋₆-alkyl, and halogen;
- A: is C₆₋₁₄-aryl or C₁₋₁₃-heteroaryl;
- C¹: is C₃₋₁₂-cycloalkyl or C₂₋₉-heterocyclyl;
- C²: is C₆₋₁₄-aryl;
- L³: is selected from a covalent bond, -CH₂O-, and -CH₂-; and
- L^{3a}: is a covalent bond or -CH₂-.

In a preferred embodiment, the present invention provides a compound of formula (Ic) as described herein, or a pharmaceutically acceptable salt thereof, wherein:
- R²¹: is selected from halogen, C₁₋₆-alkoxy, halo-C₁₋₆-alkoxy, C₁₋₆-alkyl, halo-C₁₋₆-alkyl, SF₅, C₆₋₁₄-aryl, and a group 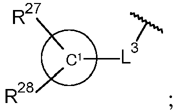
- R²²: is selected from hydrogen, halogen, C₁₋₆-alkoxy and halo-C₁₋₆-alkoxy;
- R²³: is hydrogen or halogen;
- R²⁷: is selected from hydrogen, halo-C₁₋₆-alkoxy, C₁₋₆-alkyl, halo-C₁₋₆-alkyl, and halogen;
- R²⁸: is selected from hydrogen, C₁₋₆-alkyl, and halogen;
- A: is C₆₋₁₄-aryl or C₁₋₁₃-heteroaryl;
- C¹: is C₃₋₁₂-cycloalkyl or C₂₋₉-heterocyclyl; and
- L³: is a covalent bond or -CH₂-.

In a particularly preferred embodiment, the present invention provides a compound of formula (Ic) as described herein, or a pharmaceutically acceptable salt thereof, wherein:
- R²¹: is selected from fluoro, chloro, bromo, methyl, methoxy, tert-butyl, propyl, trifluoromethoxy, 2-fluoroethoxy, 2,2,2-trifluoroethoxy, trifluoromethyl, 2,2,2-trifluoroethyl, 1,1-difluoroethyl, SF₅, phenyl, and a group 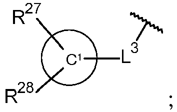
- R²²: is selected from hydrogen, fluoro, chloro, methoxy, methyl, and trifluoromethyl;
- R²³: is hydrogen or fluoro;
- R²⁷: is selected from methyl, trifluoromethoxy, trifluoromethyl, 2,2,2-trifluoro-1-methyl-ethoxy, 2,2,2-trifluoro-1,1-dimethyl-ethoxy, 2,2,2-trifluoroethoxy, fluoro, and chloro;
- R²⁸: is selected from hydrogen, methyl, fluoro, and chloro;
- A: is selected from phenyl, indol-3-yl, 2-pyridyl, and 3-pyridyl;
- C¹: is selected from azetidin-1-yl, pyrrolidin-1-yl, cyclopropyl, and oxetan-3-yl; and
- L³: is a covalent bond or -CH₂-.

In one embodiment, the present invention provides a compound of formula (Ic) as described herein, or a pharmaceutically acceptable salt thereof, selected from the compounds presented in Table 1 and Table 3.

In a preferred embodiment, the present invention provides a compound of formula (Ic) as described herein, or a pharmaceutically acceptable salt thereof, selected from the compounds presented in Table 1.

In a particularly preferred embodiment, the present invention provides a compound of formula (Ic) as described herein, or a pharmaceutically acceptable salt thereof, wherein the compound of formula (Ic) is selected from:
rac-cis-6-(4-(5-Chloro-1-(cyclopropylmethyl)-1H-indol-3-yl)piperidine-1-carbonyl)hexahydro-2H-pyrido[4,3-b][1,4]oxazin-3(4H)-one;
rac-cis-6-(4-(5-Chloro-1-methyl-1H-indol-3-yl)piperidine-1-carbonyl)hexahydro-2H-pyrido[4,3-b] [1,4]oxazin-3(4H)-one;
rac-cis-6-(4-(1-(4-Fluorophenyl)-3-methoxypropyl)piperidine-1-carbonyl)hexahydro-2H-pyrido[4,3-b] [1,4]oxazin-3(4H)-one;
rac-cis-6-(4-((4-Chlorophenyl)(phenyl)methyl)piperazine-1-carbonyl)hexahydro-2H-pyrido[4,3-b] [1,4]oxazin-3(4H)-one;
(+)-cis-6-(4-(bis(4-Fluorophenyl)methyl)piperazine-1-carbonyl)hexahydro-2H-pyrido[4,3-b] [1,4]oxazin-3(4H)-one;
(+)- or (-)-cis-6-(4-(5-Chloro-1-cyclopropyl-1H-indol-3-yl)piperidine-1-carbonyl)hexahydro-2H-pyrido[4,3-b][1,4]oxazin-3(4H)-one;
(-i-)-or (-)-cis-6-(4-(5-Chloro-1-(oxetan-3-yl)-1H-indol-3-yl)piperidine-1-carbonyl)hexahydro-2H-pyrido[4,3-b][1,4]oxazin-3(4H)-one;
rac-cis-6-(4-(1-(4-Fluorophenyl)-3-hydroxypropyl)piperidine-1-carbonyl)hexahydro-2H-pyrido[4,3-b] [1,4]oxazin-3(4H)-one;
(4aR,8aS)-6-(4-(Difluoro(4-(trifluoromethyl)phenyl)methyl)piperidine-1-carbonyl)hexahydro-2H-pyrido[4,3-b][1,4]oxazin-3(4H)-one;
(+) or (-)-cis-6-(4-((R or S)-1-(2-chloro-4-fluorophenoxy)ethyl)piperidine-1-carbonyl)hexahydro-2H-pyrido[4,3-b][1,4]oxazin-3(4H)-one;
(+) or (-)-cis-6-(4-((4-Chlorophenyl)difluoromethyl)piperidine-1-carbonyl)hexahydro-2H-pyrido[4,3-b] [1,4]oxazin-3(4H)-one;
(+) or (-)-cis-6-(4-((2-Chloro-4-fluorophenyl)difluoromethyl)piperidine-1-carbonyl)hexahydro-2H-pyrido[4,3-b][1,4]oxazin-3(4H)-one;
(4aR,8aS)-6-(4-((R or S)-1-(4-(Trifluoromethyl)phenyl)ethyl)piperidine-1-carbonyl)hexahydro-2H-pyrido[4,3-b][1,4]oxazin-3(4H)-one;
(4aR,8aS)-6-(4-((S or R)-(4-Fluorophenyl)(2,2,2-trifluoroethoxy)methyl)piperidine-1-carbonyl)hexahydro-2H-pyrido[4,3-b][1,4]oxazin-3(4H)-one;
(4aR,8aS)-6-[4-[(S or R)-(4-Fluorophenyl)-(3-methoxyphenyl)methyl]piperidine-1-carbonyl]-4,4a,5,7,8,8a-hexahydropyrido[4,3-b][1,4]oxazin-3-one;
(4aR,8aS)-6-(4-((R or S)-(4-Fluorophenyl)(3-methoxyphenyl)methyl)piperidine-1-carbonyl)hexahydro-2H-pyrido[4,3-b][1,4]oxazin-3(4H)-one;
(4aR,8aS)-6-(4-((S or R)-(3-(2-Fluoroethoxy)phenyl)(phenyl)methyl)piperidine-1-carbonyl)hexahydro-2H-pyrido[4,3-b][1,4]oxazin-3(4H)-one;
(4aR,8aS)-6-(4-((3-Cyclopropyl-1,2,4-oxadiazol-5-yl)(4-fluorophenyl)methyl)piperidine-1-carbonyl)hexahydro-2H-pyrido[4,3-b][1,4]oxazin-3(4H)-one;
(4aR,8aS)-6-(4-((S or R)-(4-Fluorophenyl)(4-methoxyphenyl)methyl)piperidine-1-carbonyl)hexahydro-2H-pyrido[4,3-b][1,4]oxazin-3(4H)-one;
(4aR,8aS)-6-(4-((S or R)-(3,4-Dimethoxyphenyl)(4-fluorophenyl)methyl)piperidine-1-carbonyl)hexahydro-2H-pyrido[4,3-b][1,4]oxazin-3(4H)-one;
(4aR,8aS)-6-(4-((R or S)-(4-Fluorophenyl)(4-methoxyphenyl)methyl)piperidine-1-carbonyl)hexahydro-2H-pyrido[4,3-b][1,4]oxazin-3(4H)-one;
(4aR,8aS)-6-(4-((S or R)-(4-(2-Fluoroethoxy)phenyl)(4-fluorophenyl)methyl)piperidine-1-carbonyl)hexahydro-2H-pyrido[4,3-b][1,4]oxazin-3(4H)-one;
(4aR,8aS)-6-[3-[4-(Trifluoromethoxy)phenyl]azetidine-1-carbonyl]-4,4a,5,7,8,8a-hexahydropyrido[4,3-b][1,4]oxazin-3-one;
(4aR,8aS)-6-(3-(2'-Trifluoromethoxy)-[1,1'-biphenyl]-4-yl)azetidine-1-carbonyl)hexahydro-2H-pyrido[4,3-b][1,4]oxazin-3(4H)-one;
(4aR,8aS)-6-(3-(2'-(Trifluoromethyl)-[1,1'-biphenyl]-4-yl)azetidine-1-carbonyl)hexahydro-2H-pyrido[4,3-b][1,4]oxazin-3(4H)-one;
(4aR,8aS)-6-(3-(2',4'-Difluoro-[1,1'-biphenyl]-4-yl)azetidine-1-carbonyl)hexahydro-2H-pyrido[4,3-b][1,4]oxazin-3(4H)-one;
(4aR, 8 aS)-6-(3-(4-(3 -(Trifluoromethyl) azetidin-1-yl)phenyl) azetidine-1-carbonyl)hexahydro-2H-pyrido[4,3-b][1,4]oxazin-3(4H)-one;
(4aR,8aS)-6-(3-(4-Bromo-3-chlorophenyl)azetidine-1-carbonyl)hexahydro-2H-pyrido[4,3-b][1,4]oxazin-3(4H)-one;
(4aR,8aS)-6-[3-[4-(4-Chloro-2-fluoro-phenyl)phenyl]azetidine-1-carbonyl]-4,4a,5,7,8,8a-hexahydropyrido[4,3-b][1,4]oxazin-3-one;
(4aR,8aS)-6-[3-[4-(2-Chloro-4-fluoro-phenyl)phenyl]azetidine-1-carbonyl]-4,4a,5,7,8,8a-hexahydropyrido[4,3-b][1,4]oxazin-3-one;
(4aR,8aS)-6-(3-(4-(3-((1,1,1-Trifluoropropan-2-yl)oxy)azetidin-1-yl)phenyl)azetidine-1-carbonyl)hexahydro-2H-pyrido[4,3-b][1,4]oxazin-3(4H)-one;
(4aR,8aS)-6-(3-(4-(tert-Butyl)phenyl)azetidine-1-carbonyl)hexahydro-2H-pyrido[4,3-b][1,4]oxazin-3(4H)-one;
(4aR,8aS)-6-(3-(4-(Trifluoromethyl)phenyl)azetidine-1-carbonyl)hexahydro-2H-pyrido[4,3-b][1,4]oxazin-3(4H)-one;
(4aR,8aS)-6-(3-(4-(3-((1,1,1-Trifluoro-2-methylpropan-2-yl)oxy)azetidin-1-yl)phenyl)azetidine-1-carbonyl)hexahydro-2H-pyrido[4,3-b][1,4]oxazin-3(4H)-one;
(4aR,8aS)-6-(3-(4-(1,1-Difluoroethyl)phenyl)azetidine-1-carbonyl)hexahydro-2H-pyrido[4,3-b][1,4]oxazin-3(4H)-one;
(4aR,8aS)-6-(3-(4-(3,3-Dimethylpyrrolidin-1-yl)phenyl)azetidine-1-carbonyl)hexahydro-2H-pyrido[4,3-b] [1,4]oxazin-3(4H)-one;
(4aR,8aS)-6-(3-(4-(3-(Trifluoromethoxy)azetidin-1-yl)phenyl)azetidine-1-carbonyl)hexahydro-2H-pyrido[4,3-b][1,4]oxazin-3(4H)-one;
(4aR,8aS)-6-(3-(6-(2,4-Dichlorophenyl)pyridin-3-yl)azetidine-1-carbonyl)hexahydro-2H-pyrido[4,3-b][1,4]oxazin-3(4H)-one;
(4aR,8aS)-6-[3-[4-(2,2,2-Trifluoroethoxy)phenyl]azetidine-1-carbonyl]-4,4a,5,7,8,8a-hexahydropyrido[4,3-b][1,4]oxazin-3-one;
(4aR,8aS)-6-[3-[4-[3-[(1S or 1R)-2,2,2-Trifluoro-1-methyl-ethoxy]azetidin-1-yl]phenyl]azetidine-1-carbonyl]-4,4a,5,7,8,8a-hexahydropyrido[4,3-b] [1,4]oxazin-3-one;
(4aR,8aS)-6-[3-[4-[3-[(1R or 1S)-2,2,2-Trifluoro-1-methyl-ethoxy]azetidin-1-yl]phenyl]azetidine-1-carbonyl]-4,4a,5,7,8,8a-hexahydropyrido[4,3-b] [1,4]oxazin-3-one;
(4aR,8aS)-6-(3-(4-(2-(Trifluoromethyl)pyrrolidin-1-yl)phenyl)azetidine-1-carbonyl)hexahydro-2H-pyrido[4,3-b][1,4]oxazin-3(4H)-one;
(4aR, 8 aS)-6-(3-(4-(3 -(Trifluoromethyl)pyrrolidin-1-yl)phenyl) azetidine-1-carbonyl)hexahydro-2H-pyrido[4,3-b][1,4]oxazin-3(4H)-one;
(4aR, 8 aS)-6-(3-(4-(3 -(2, 2, 2-trifluoroethoxy) azetidin-1-yl)phenyl) azetidine-1-carbonyl)hexahydro-2H-pyrido[4,3-b][1,4]oxazin-3(4H)-one;
(4aR, 8 aS)-6-(3-(6-(2-(trifluoromethyl)pyrrolidin-1-yl)pyridin-3-yl)azetidine-1-carbonyl)hexahydro-2H-pyrido[4,3-b][1,4]oxazin-3(4H)-one;
(4aR,8aS)-6-(3-(4-(Pentafluoro-16-sulfaneyl)phenyl)azetidine-1-carbonyl)hexahydro-2H-pyrido[4,3-b][1,4]oxazin-3(4H)-one;
(4aR,8aS)-6-(3-(2-Fluoro-4-(trifluoromethoxy)phenyl)azetidine-1-carbonyl)hexahydro-2H-pyrido[4,3-b] [1,4]oxazin-3(4H)-one;
(4aR,8aS)-6-[3-[4-(2,2,2-Trifluoroethyl)phenyl]azetidine-1-carbonyl]-4,4a,5,7,8,8a-hexahydropyrido[4,3-b][1,4]oxazin-3-one;
(4aR,8aS)-6-[3-[4-[1-(Trifluoromethyl)cyclopropyl]phenyl]azetidine-1-carbonyl] - 4,4a,5,7,8,8a-hexahydropyrido[4,3-b][1,4]oxazin-3-one;
(4aR,8aS)-6-[3-[4-(6,6-Difluoro-2-azaspiro[3.3]heptan-2-yl)phenyl]azetidine-1-carbonyl]-4,4a,5,7,8,8a-hexahydropyrido[4,3-b][1,4]oxazin-3-one;
(4aR,8aS)-6-(3-(5-(2,4-Dichlorophenyl)pyridin-2-yl)azetidine-1-carbonyl)hexahydro-2H-pyrido[4,3-b][1,4]oxazin-3(4H)-one;
(4aR,8aS)-6-(3-(4-((R or S)-2-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-((S or R)-2-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-((R or S)-3-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-((S or R)-3-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(3-Fluoro-4-(trifluoromethoxy)phenyl)azetidine-1-carbonyl)hexahydro-2H-pyrido[4,3-b] [1,4]oxazin-3(4H)-one;
(4aR,8aS)-6-(3-(3-Methyl-4-(trifluoromethoxy)phenyl)azetidine-1-carbonyl)hexahydro-2H-pyrido[4,3-b] [1,4]oxazin-3(4H)-one;
(4aR,8aS)-6-(3-(3,5-Difluoro-4-(trifluoromethoxy)phenyl)azetidine-1-carbonyl)hexahydro-2H-pyrido[4,3-b][1,4]oxazin-3(4H)-one;
(4aR,8aS)-6-(3-(3-Chloro-4-(trifluoromethoxy)phenyl)azetidine-1-carbonyl)hexahydro-2H-pyrido[4,3-b] [1,4]oxazin-3(4H)-one;
(4aR,8aS)-6-((R or S)-3-(3-Chloro-5-(2,2,2-trifluoroethoxy)phenyl)pyrrolidine-1-carbonyl)hexahydro-2H-pyrido[4,3-b][1,4]oxazin-3(4H)-one;
(4aR,8aS)-6-((S or R)-3-(3-Chloro-5-(2,2,2-trifluoroethoxy)phenyl)pyrrolidine-1-carbonyl)hexahydro-2H-pyrido[4,3-b][1,4]oxazin-3(4H)-one;
(4aR,8aS)-6-(3-(4-(tert-Butyl)-3-methoxyphenyl)azetidine-1-carbonyl)hexahydro-2H-pyrido[4,3-b][1,4]oxazin-3(4H)-one;
(4aR,8aS)-6-(3-(4-Propylphenyl)azetidine-1-carbonyl)hexahydro-2H-pyrido[4,3-b][1,4]oxazin-3(4H)-one;
(4aR,8aS)-6-(3-(4-(Trifluoromethoxy)-3-(trifluoromethyl)phenyl)azetidine-1-carbonyl)hexahydro-2H-pyrido[4,3-b][1,4]oxazin-3(4H)-one;
(4aR,8aS)-6-(3-(5-((R or S)-3-(Trifluoromethyl)pyrrolidin-1-yl)pyridin-2-yl)azetidine-1-carbonyl)hexahydro-2H-pyrido[4,3-b][1,4]oxazin-3(4H)-one;
(4aR,8aS)-6-(3-(5-((S or R)-3-(Trifluoromethyl)pyrrolidin-1-yl)pyridin-2-yl)azetidine-1-carbonyl)hexahydro-2H-pyrido[4,3-b][1,4]oxazin-3(4H)-one;
(4aR,8aS)-6-[3-[6-[3-(R or S)-(trifluoromethyl)pyrrolidin-1-yl]-3-pyridyl]azetidine-1-carbonyl]-4,4a,5,7,8,8a-hexahydropyrido[4,3-b][1,4]oxazin-3-one;
(4aR,8aS)-6-[3-[6-[3-(S or R)-(trifluoromethyl)pyrrolidin-1-yl]-3-pyridyl]azetidine-1-carbonyl]-4,4a,5,7,8,8a-hexahydropyrido[4,3-b][1,4]oxazin-3-one;
(4aR,8aS)-6-[3-(4-Tetrahydropyran-3-ylphenyl)azetidine-1-carbonyl]-4,4a,5,7,8,8a-hexahydropyrido[4,3-b][1,4]oxazin-3-one;
(4aR,8aS)-6-[3-[2-Methoxy-4-(2,2,2-trifluoroethyl)phenyl]azetidine-1-carbonyl]-4,4a,5,7,8,8a-hexahydropyrido[4,3-b][1,4]oxazin-3-one; and
(4aR,8aS)-6-(3-(4-(Neopentyloxy)phenyl)azetidine-1-carbonyl)hexahydro-2H-pyrido[4,3-b] [1,4]oxazin-3(4H)-one.

In one embodiment, the present invention provides pharmaceutically acceptable salts or esters of the compounds of formula (Ic) as described herein. In a particular embodiment, the present invention provides pharmaceutically acceptable salts of the compounds according to formula (Ic) as described herein, especially hydrochloride salts. In a further particular embodiment, the present invention provides pharmaceutically acceptable esters of the compounds according to formula (Ic) as described herein. In yet a further particular embodiment, the present invention provides compounds according to formula (I) as described herein.

### Processes of Manufacturing

The preparation of compounds of formula (Ic) of the present invention may be carried out in sequential or convergent synthetic routes. Syntheses of the invention are shown in the following general schemes. The skills required for carrying out the reaction and purification of the resulting products are known to those persons skilled in the art. The substituents and indices used in the following description of the processes have the significance given herein, unless indicated to the contrary.

If one of the starting materials, intermediates or compounds of formula (Ic) contain one or more functional groups which are not stable or are reactive under the reaction conditions of one or more reaction steps, appropriate protective groups (as described e.g., in "Protective Groups in Organic Chemistry" by T. W. Greene and P. G. M. Wutts, 5th Ed., 2014, John Wiley & Sons, N.Y.) can be introduced before the critical step applying methods well known in the art. Such protective groups can be removed at a later stage of the synthesis using standard methods described in the literature.

If starting materials or intermediates contain stereogenic centers, compounds of formula (Ic) can be obtained as mixtures of diastereomers or enantiomers, which can be separated by methods well known in the art e.g., chiral HPLC, chiral SFC or chiral crystallization. Racemic compounds can e.g., be separated into their antipodes via diastereomeric salts by crystallization with optically pure acids or by separation of the antipodes by specific chromatographic methods using either a chiral adsorbent or a chiral eluent. It is equally possible to separate starting materials and intermediates containing stereogenic centers to afford diastereomerically/enantiomerically enriched starting materials and intermediates. Using such diastereomerically/enantiomerically enriched starting materials and intermediates in the synthesis of compounds of formula (Ic) will typically lead to the respective diastereomerically/enantiomerically enriched compounds of formula (Ic)

A person skilled in the art will acknowledge that in the synthesis of compounds of formula (I) - insofar not desired otherwise - an "orthogonal protection group strategy" will be applied, allowing the cleavage of several protective groups one at a time each without affecting other protective groups in the molecule. The principle of orthogonal protection is well known in the art and has also been described in literature (e.g. Barany and R. B. Merrifield, J. Am. Chem. Soc. 1977, 99, 7363; H. Waldmann et al., Angew. Chem. Int. Ed. Engl. 1996, 35, 2056).

A person skilled in the art will acknowledge that the sequence of reactions may be varied depending on reactivity and nature of the intermediates.

In more detail, the compounds of formula (Ic) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 2nd Edition, Richard C. Larock. John Wiley & Sons, New York, NY. 1999*).* It was found convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux. The time required for the reaction may also vary widely, depending on many factors, notably the reaction temperature and the nature of the reagents. However, a period of from 0.5 hours to several days will usually suffice to yield the described intermediates and compounds. The reaction sequence is not limited to the one displayed in the schemes, however, depending on the starting materials and their respective reactivity, the sequence of reaction steps can be freely altered.

If starting materials or intermediates are not commercially available or their synthesis not described in literature, they can be prepared in analogy to existing procedures for close analogues or as outlined in the experimental section.

The following abbreviations are used in the present text:
AcOH = acetic acid, ACN = acetonitrile , Boc = tert-butyloxycarbonyl, CAS RN = chemical abstracts registration number, Cbz = benzyloxycarbonyl, Cs₂CO₃ = cesium carbonate, CO = carbon monoxide, CuCl = copper(I) chloride, CuCN = copper(I) cyanide, CuI = copper(I) iodide, DMAP = 4-dimethylaminopyridine, DME = dimethoxyethane , DMEDA = N,N'-dimethylethylenediamine, DMF = N,N-dimethylformamide, DIPEA = N,N-diisopropylethylamine, dppf = 1,1 bis(diphenyl phosphino)ferrocene, EDC.HCl = N-(3-dimethylaminopropyl)-N'-ethylcarbodiimide hydrochloride, EI = electron impact, ESI = electrospray ionization, EtOAc = ethyl acetate, EtOH = ethanol, h = hour(s), FA = formic acid, H₂O = water, H₂SO₄ = sulfuric acid, Hal = halogen, HATU = 1-[bis(dimethylamino)methylene]-1H-1,2,3-triazolo[4,5-b]pyridinium-3-oxide hexafluorophosphate, HBTU = O-benzotriazole-N,N,N',N'-tetramethyl-uronium-hexafluoro-phosphate, HCl = hydrogen chloride, HOBt = 1-hydroxy-1H-benzotriazole;
HPLC = high performance liquid chromatography, iPrMgCl = isopropylmagnesium chloride, I₂ = iodine, IPA = 2-propanol, (Ir[dF(CF₃)ppy]₂(dtbpy))PF₆ = [4,4'-bis(1,1-dimethylethyl)-2,2'-bipyridine-N1,N1']bis[3,5-difluoro-2-[5-(trifluoromethyl)-2-pyridinyl-N]phenyl-C]Iridium(III) hexafluorophosphate, ISP = ion spray positive (mode), ISN = ion spray negative (mode), K₂CO₃ = potassium carbonate, KHCO₃ = potassium bicarbonate, KI = potassium iodide, KOH = potassium hydroxide, K₃PO₄ = potassium phosphate tribasic, LiAlH₄ or LAH = lithium aluminium hydride, LiHMDS = lithium bis(trimethylsilyl)amide, LiOH = lithium hydroxide, MgSO₄ = magnesium sulfate, min = minute(s), mL = milliliter, MPLC = medium pressure liquid chromatography, MS = mass spectrum, NaH = sodium hydride, NaHCO₃ = sodium hydrogen carbonate, NaNO₂ = sodium nitrite, NaOH = sodium hydroxide, Na₂CO₃ = sodium carbonate, Na₂SO₄ = sodium sulfate, Na₂S₂O₃ = sodium thiosulfate, NBS = N-bromosuccinimide, nBuLi = n-butyllithium, NEt₃ = triethylamine (TEA), NH₄Cl = ammonium chloride, NiCl₂ glyme = Nickel(II) chloride ethylene glycol dimethyl ether complex, NMP = N-methyl-2-pyrrolidone, OAc = Acetoxy, T₃P = propylphosphonic anhydride, P2O5 = phosphorus pentoxide, PE = petroleum ether, PG = protective group, Pd-C = palladium on activated carbon, PdCl₂(dppf)-CH₂Cl₂ = 1,1'-bis(diphenylphosphino)ferrocene-palladium(II)dichloride dichloromethane complex, Pd₂(dba)₃ = tris(dibenzylideneacetone)dipalladium(0), Pd(OAc)₂ = palladium(II) acetate, Pd(OH)₂ = palladium hydroxide, Pd(PPh₃)₄ = tetrakis(triphenylphosphine)palladium(0), PTSA = p-toluenesulfonic acid, R = any group, RT = room temperature, SFC = Supercritical Fluid Chromatography, S-PHOS = 2-dicyclohexylphosphino-2',6'-dimethoxybiphenyl, T3P = propylphosphonic anhydride, TBAI = tetra butyl ammonium iodine, TEA = triethylamine, TFA = trifluroacetic acid, THF = tetrahydrofuran, TMEDA = N,N,N',N'-tetramethylethylenediamine, ZnCl₂ = zinc chloride, Xantphos = 4,5-Bis(diphenylphosphino)-9,9-dimethylxanthene.

Compounds of formula **I** wherein A, L, X, n and m are as described herein can be synthesized in analogy to literature procedures and/or as depicted for example in Scheme 1.

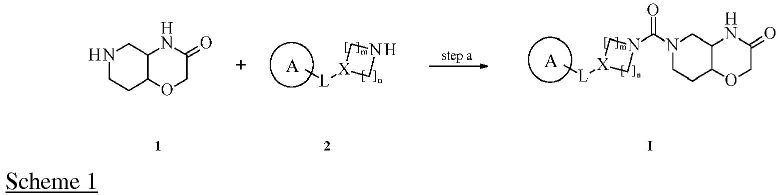

Accordingly, 4a,5,6,7,8,8a-Hexahydro-4H-pyrido[4,3-b][1,4]oxazin-3-ones 1 are reacted with intermediates **2** in the presence of a urea forming reagent such as bis(trichloromethyl) carbonate using a suitable base and solvent such as, e.g. sodium bicarbonate in DCM, to give compounds of formula I (step a). Further urea forming reagents include but are not limited to phosgene, trichloromethyl chloroformate, (4-nitrophenyl)carbonate or 1,1'-carbonyldiimidazole. Reactions of this type and the use of these reagents are widely described in literature (e.g. G. Sartori et al., Green Chemistry 2000, 2, 140). A person skilled in the art will acknowledge that the order of the addition of the reagents can be important in this type of reactions due to the reactivity and stability of the intermediary formed carbamoyl chlorides, as well as for avoiding formation of undesired symmetrical urea by-products.

The compounds of formula (Ic) described herein may be prepared in analogy to the compounds of formula (I), as described herein in Schemes 1 to 18. For example, in analogy to the procedure described in Scheme 1 above, 4a,5,6,7,8,8a-Hexahydro-4H-pyrido[4,3-b][1,4]oxazin-3-ones **1** are reacted with intermediates **2a** to yield compounds of formula (Ic) (Scheme 1A, step a).

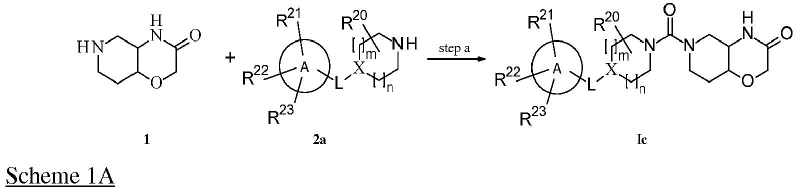

Intermediates 1 may be synthesized as depicted for example in Scheme 2 and/or in analogy to methods described in literature.

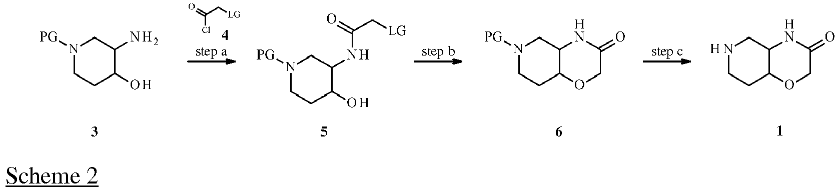

Thus, 3-aminopiperidin-4-ol derivatives **3** in which "PG" signifies a suitable protective group such as a Cbz or Boc protective group can be acylated for example with chloro- or bromoacetyl chloride **4,** in which "LG" signifies a suitable leaving group (e.g., Cl or Br), using a suitable base such as sodium or potassium carbonate, sodium hydroxide or sodium acetate in an appropriate solvent such as THF, water, acetone or mixtures thereof, to provide intermediates **5** (step a).

Intermediates **5** can be cyclized to intermediates **6** using methods well known in the art, for example by treatment of **5** with sodium hydride in THF or potassium tert-butoxide in IPA and water (step b). Reactions of that type are described in literature (e.g. Z. Rafinski et al., J. Org. Chem. 2015, 80, 7468; S. Dugar et al., Synthesis 2015, 47(5), 712; WO2005/066187).

Removal of the protective group in intermediates **6,** applying methods known in the art (e.g., a Boc group using TFA in DCM at temperatures between 0°C and room temperature, a Cbz group using hydrogen in the presence of a suitable catalyst such as Pd or Pd(OH)₂ on charcoal in a suitable solvent such as MeOH, EtOH, EtOAc or mixtures therefore and as described for example in "Protective Groups in Organic Chemistry" by T.W. Greene and P.G.M. Wuts, 4th Ed., 2006, Wiley N.Y.), furnishes intermediates **1** (step c).

Intermediates **1** can be obtained as mixtures of diastereomers and enantiomers, respectively, or as single stereoisomers depending on whether racemic mixtures or enantiomerically pure forms of cis- or trans-3-aminopiperidin-4-ol derivatives **3** are employed in their syntheses. Intermediates **3** are commercially available and their synthesis has also been described in literature (e.g. WO2005/066187; WO2011/0059118; WO2016/185279).

Optically pure cis-configured intermediates **1B** and **1C** can be obtained for example according to Scheme 3 by chiral separation of commercially available (cis-rac)-4a,5,6,7,8,8a-hexahydro-4H-pyrido[4,3-b][1,4]oxazin-3-one (**1A**) (optionally in form of a salt such as, e.g. a hydrochloride salt) using methods known in the art, e.g. by diastereomeric salt crystallization or by chiral chromatography (step a).

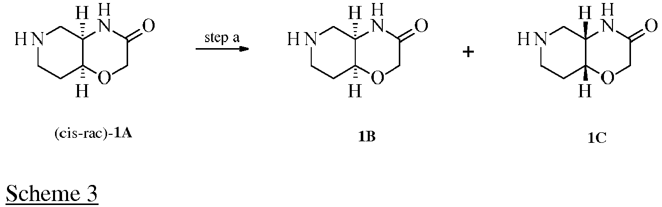

In some embodiment, intermediates **2** are intermediates of type **B**, **C**, **D** or **E**. Intermediates of type **B**, **C**, **D** or **E** can be prepared for example by the synthetic procedures outlined in Schemes 4, 5, 6 and 7.

Intermediates of type **B** in which R¹ is as defined herein, can be prepared by a variety of conditions, which may be exemplified by the general synthetic procedure outlined in Scheme 4.

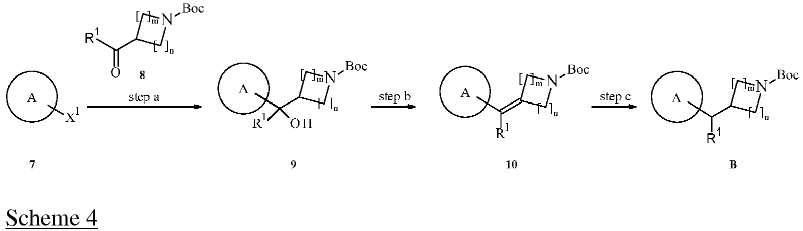

Starting from aryl or heteroaryl halides 7, wherein X¹ is selected from Cl, Br or I and A is as defined herein, a lithium halogen exchange reaction can be performed using a solution of LiHMDS or *n*-BuLi, preferably n-BuLi in a solvent like THF, diethyl ether, n-pentane, n-hexane or mixtures thereof, preferably THF and in a temperature range between -20°C and -78°C, preferably at -78°C, to generate the corresponding lithiated aryl or heteroaryl intermediate. Nucleophilic addition of the in situ prepared lithiated aryl or heteroaryl intermediate to ketones of type **8,** wherein R¹, m and n are as defined herein, in a solvent such as THF and preferably at a temperature of -78°C gives the corresponding tertiary alcohols **9** (step a).

Subsequent elimination of the tertiary hydroxy group with concomitant removal of the Boc protective group using acidic conditions such as 4M HCl in dioxane in a solvent like MeOH, or, preferably, TFA in DCM yields the corresponding olefins **10** (step b).

Heterogeneous catalytic hydrogenation of olefins **10** using a catalyst such as Pd(OH)₂ or Pd/C in a solvent like THF, MeOH, EtOH, EtOAc or a mixture thereof, preferably Pd/C in THF under e.g., atmospheric pressure of hydrogen, affords intermediates of type **B** (step c).

Intermediates **8** are commercially available and/or can be prepared in analogy to methods described in literature, e.g. Bioorg. Med. Chem. Lett. 2011, 27(18), 5191, WO2012/155199, WO2016/180536, Bioorg. Med. Chem. Lett. 2008, 18(18), 5087, WO2007/117557, J. Am. Chem. Soc. 2017, 139(33), 11353, J. Med. Chem. 2017, 60(13), 5507.

Intermediates of type **C**, wherein A is as defined herein and n = 1, 2 or 3 can be prepared by a variety of conditions, which may be exemplified by the general synthetic procedures outlined in Scheme 5.

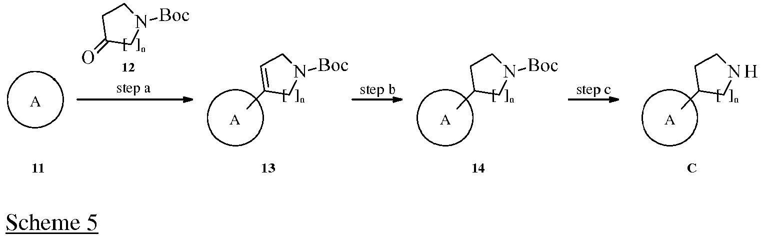

Treatment of a mixture of aryl or heteroaryl compounds **11**, wherein A is as defined herein, preferably wherein A is substituted heteroaryl as defined herein, most preferably substituted indolyl as defined herein, and ketones **12,** wherein n is as defined herein, with a base such as NaOH or KOH in a solvent like EtOH or MeOH and in a temperature range between room temperature and 80°C, preferably around the reflux temperature of the mixture, gives olefins **13** (step a).

Subsequent heterogeneous catalytic hydrogenation using a transition metal catalyst, such as PtO₂ in a polar solvent like MeOH, EtOH, AcOEt, AcOH or a mixture thereof, preferably a mixture of EtOH/AcOH at around room temperature from low to high pressure, preferably around 5 bar pressure of hydrogen gas, yields intermediates **14** (step b).

Removal of the Boc protective group using acidic conditions such as treatment with TFA in DCM or preferably with 4M HCl in dioxane in a solvent like MeOH gives the corresponding intermediates of type **C** (step c).

In some embodiments, intermediate **14** is an intermediate of formula **14a,** wherein ring A is heteroaryl comprising a secondary amino group (i.e., "-NH-", such as in indolyl) and m and n are as defined herein. Intermediates **14a** may be transformed to intermediates of type **D,** wherein A is heteroaryl comprising at least one nitrogen atom, m and n are as defined herein and R¹⁴ is selected from alkyl, cycloalkyl, hydroxyalkyl, cycloalkylalkyl, heterocyclyl and heterocyclylalkyl, prefereably from methyl, cyclopropyl, cyclopropylmethyl, hydroxyethyl, oxetan-3-yl and oxetan-3-ylmethyl, for example as outlined in Scheme 6.

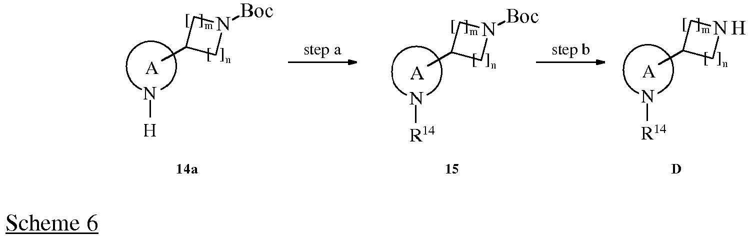

Thus, intermediates **14a** can be *N*-functionalized by treatment with an appropriate base, such as NaH, KH, NaHMDS, LiHMDS, LDA, preferably with NaH in a suitable solvent like DMF, THF, dioxane, or a mixture thereof, preferably DMF and in a temperature range between -78°C and room temperature, preferably at 0°C, followed by addition of compounds R¹⁴-LG in which LG signifies an appropriate leaving group such as chlorine, bromine, iodine, OSO₂alkyl (*e.g.* mesylate (methanesulfonate), OSO₂fluoroalkyl (*e.g.* triflate (trifluoromethanesulfonate) or OSO₂aryl (*e*.*g*. tosylate (p-toluenesulfonate)) to give the corresponding *N*-functionalized compounds **15,** wherein R¹⁴ is as defined above for intermediate **D.** A person skilled in the art will acknowledge that depending on the nature of R¹⁴ additional or different reagents may be applied such as using cyclopropylboronic acid in the presence of copper(II)acetate and a base such as DMAP with NaHMDS in a solvent such as toluene at temperatures up to the boiling point of the solvent in case R¹⁴ signifies a cyclopropyl group (step a).

Deprotection of compounds **15** using the same conditions as described above for compounds **14** (see Scheme 5, step c) affords intermediates of type **D** (step b).

In one embodiment, intermediate **D** is an indazole derivative of type **E,** wherein m, n, and R¹⁴ are as defined herein. In addition to the procedure outlined in Scheme 6, intermediates of type **E** can be prepared by a variety of conditions, in particular by the general synthetic procedure outlined in Scheme 7.

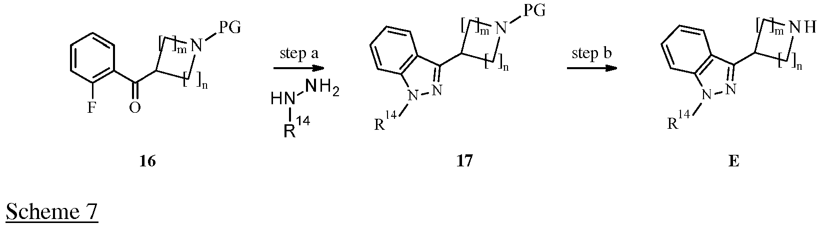

Accordingly, condensation of intermediates **16,** wherein PG is a protective group such as Boc, Cbz or Bn and m and n are as described herein, with a hydrazine derivative of type R¹⁴NHNH₂ in which R¹⁴ is as described as herein in a solvent like n-BuOH, DMA, DMF, DMSO, or a mixture thereof, preferably in n-BuOH, in a sealed reaction vessel at elevated temperature, e.g. 120°C, yields indazole compounds **17** (step a).

Subsequent, removal of the protective group, in case of a Boc group using for example acidic conditions, such as treatment with HCl in dioxane or TFA in DCM, preferably with 4M HCl in dioxane in a solvent like MeOH, preferably at around room temperature affords intermediates of type **E** (step b).

In some embodiments, intermediates **2** are intermediates of type **F, G, H, J, K, L and M.** Intermediates of type **F, G, H, J, K, L and M** in which A, m and n are as described herein, R¹⁵ and R¹⁶ are each independently selected from alkyl, cycloalkyl, hydroxyalkyl, cycloalkylalkyl, heterocyclyl and heterocyclylalkyl, prefereably from methyl, cyclopropyl, cyclopropylmethyl, hydroxyethyl, oxetan-3-yl and oxetan-3-ylmethyl; and R¹⁷ is selected from substituted or unsubstituted aryl, substituted or unsubstituted heteroaryl and substituted or unsubstituted alkyl, can be prepared by methods well known by a person skilled in the art and as exemplified by the general synthetic procedures outlined in Scheme 8.

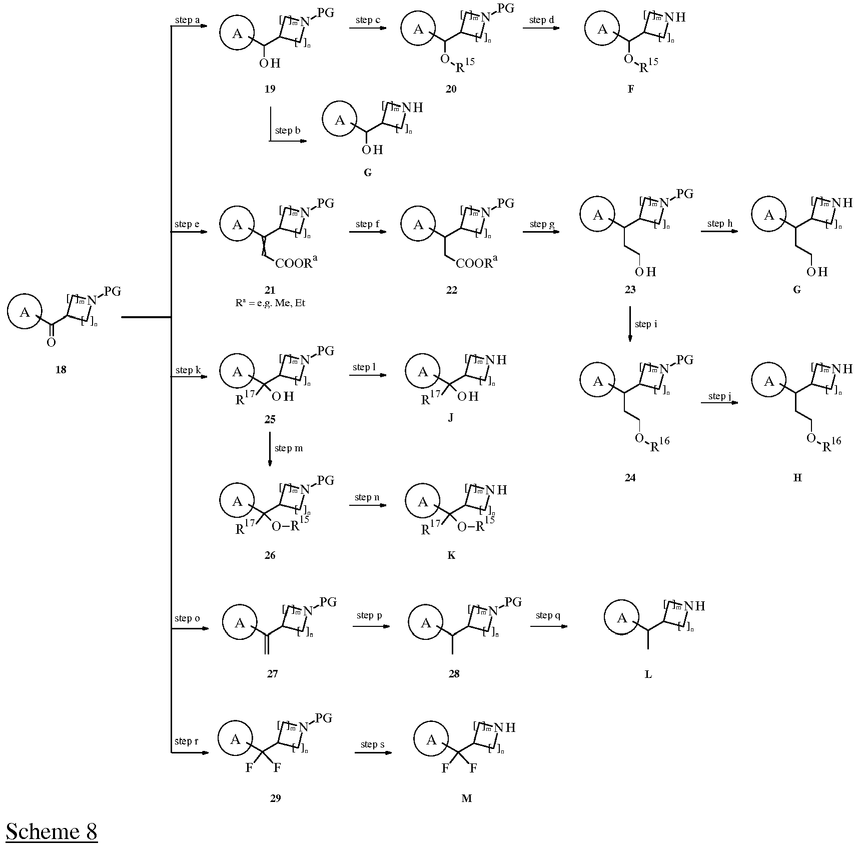

The carbonyl group in intermediates **18,** wherein A, m and n are as described herein and PG is a suitable protective group such as a Boc, Cbz or Bn group, either commercially available or prepared according to methods described in literature (e.g. J. Am. Chem. Soc. 2017, 139(33), 11353, J. Med. Chem. 2017, 60(13), 5507, RSC Advances 2015, 5(61), 40964), can be reduced by methods well known in the art, e.g. using NaBH₄ in MeOH, to give intermediates **19** (step a).

Removal of the protective group from intermediates **19** applying methods known in the art (e.g., a Boc group using TFA in DCM or 4M HCl in dioxane at temperatures between 0°C and room temperature, a Cbz group using hydrogen in the presence of a suitable catalyst such as Pd or Pd(OH)₂ on charcoal in a suitable solvent such as MeOH, EtOH, EtOAc or mixtures therefore and as described for example in "Protective Groups in Organic Chemistry" by T.W. Greene and P.G.M. Wuts, 4th Ed., 2006, Wiley N.Y.), furnishes intermediates **G** (step b).

Alkylation of the hydroxy group of intermediates **19** with an alkylating agent of type R¹⁵LG in which LG is a suitable leaving group such as chlorine, bromine, iodine, OSO₂alkyl (e.g. mesylate (methanesulfonate), OSO₂fluoroalkyl (e.g. triflate (trifluoromethanesulfonate) or OSO₂aryl (e.g. tosylate (p-toluenesulfonate)) and R¹⁵ is as described herein, using a suitable base in an appropriate solvent (e.g. sodium hydride in DMF) at temperatures between 0 °C and the boiling temperature of the solvent gives intermediates **20** (step c).

Removal of the protective group from intermediates **20** applying methods well known in the art and as described above (step b) yields intermediates **F** (step d).

Intermediates **18** can be converted into intermediates **21** in which R^{a} is alkyl, preferably methyl or ethyl by using an olefination reaction such as the widely described Wittig or Horner Emmons reaction, e.g. using ethyl 2-(diethoxyphosphoryl)acetate and LiHMDS in dioxane at temperatures ranging from 0°C to the boiling point of the solvent (step e).

The double bond in intermediates **21** can be reduced for example by hydrogenation in the presence of a suitable catalyst such as Pd-C in a suitable solvent such as MeOH, EtOH or EtOAc or mixtures thereof to yield intermediates **22** (step f).

Reduction of the ester functionality in intermediates **22** using for example LiBH₄ in THF at temperatures ranging from 0°C to the boiling point of the solvent provides intermediates **23** (step g).

Removal of the protective group from intermediates **23** applying methods well known in the art and as described above (step b) yields intermediates **G** (step h).

Alkylation of the hydroxy group of intermediates **23** with an alkylating agent of type R¹⁶LG in which LG is a suitable leaving group and R¹⁶ as described herein, using conditions as described above (step c), gives intermediates **24** (step i).

Removal of the protective group from intermediates **24** applying methods well known in the art and as described above (step b) yields intermediates **H** (step j).

Addition of an organometallic compound of type R¹⁷M in which M is for example MgCl, MgCl or Li and R¹⁷ as described herein to intermediates **18** provides intermediates **25** (step k). Reactions of this type are well known in the art and described in literature *(*J. Med. Chem. 1989, 32(1), 105, J. Med. Chem. 2014, 57(4), 1543, Bioorg. Med. Chem Lett. 2015, 25(13), 2720).

Removal of the protective group from intermediates **25** applying methods well known in the art and as described above (step b) furnishes intermediates **J** (step 1).

Alkylation of the hydroxy group of intermediates **25** with an alkylating agent of type R¹⁵LG in which LG is a suitable leaving group and R¹⁵ as described herein, using conditions as described above (step c), gives intermediates **26** (step m).

Removal of the protective group from intermediates **26** applying methods well known in the art and as described above (step b) furnishes intermediates **K** (step n).

Intermediates **18** can be converted into intermediates **27** applying methods known in the art and described in literature (e.g. J. Med. Chem. 2003, 46(25), 5445; WO2016/205590), for example by Wittig olefination using, e.g. methyl triphenylphosphonium bromide and potassium tert-butoxide in toluene or LiHMDS in THF (step o).

Reduction of the double bond of intermediates **27** applying for example hydrogenation conditions (e.g. hydrogen in the presence of a suitable catalyst such as Pd-C or PtO₂) or using 9-borabicyclo(3.3.1)nonan in THF (in analogy to literature methods, e.g. WO2007/002057) gives intermediates **28** (step p).

Removal of the protective group from intermediates **28** applying methods well known in the art and as described above (step b) furnishes intermediates **L** (step q).

Conversion of the ketone of intermediates **18** applying for example fluorination conditions using aminosulfurans such as DAST, gives intermediates **29** (step r).

Removal of the protective group from intermediates **29** applying methods well known in the art and as described above (step b) furnishes intermediates **M** (step s).

In some embodiment, intermediates **2** are intermediates of type **N** and **O** in which A, m, n and R¹⁵ are as described herein can be prepared by methods well known by a person skilled in the art and as exemplified by the general synthetic procedures outlined in Scheme 9.

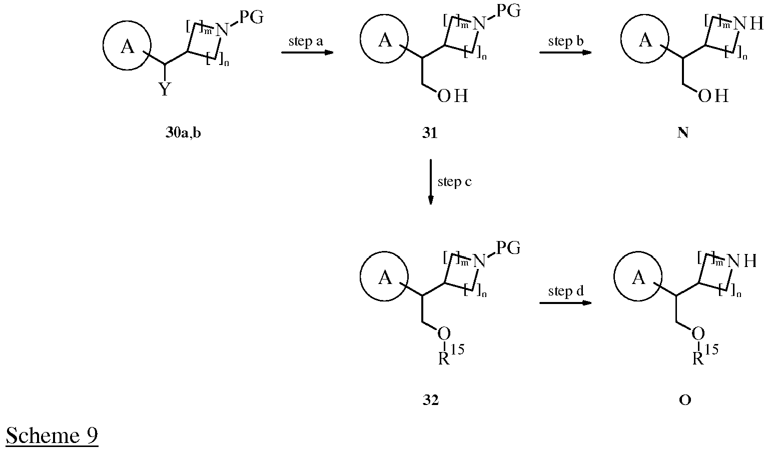

Accordingly, intermediates **30a** in which A, m and are as defined herein, PG signifies a suitable protective group such as a Boc, Cbz or Bn and Y is a formyl group (commercially available or prepared by literature methods, e.g. Bioorg. Med. Chem. Lett. 2006, 16(14), 3668; WO2013/179024) can be treated with a reducing agent such as NaBH₄ in MeOH to yield intermediates **31.** Reactions of this type are also described in literature (e.g. WO2013/179024) (step a).

Alternatively, intermediates **31** can be prepared from intermediates **30b** in which Y is a carboxyl group (commercially available or prepared in analogy to methods described in literature, e.g. Bioorg. Med. Chem. 2013, 21(15), 4600; WO2016/109501) by using an appropriate reducing agent such as borane tetrahydrofuran complex in a solvent such as THF. Reactions of this type are also widely described in literature, e.g. Bioorg. Med. Chem. 2013, 27(15), 4600) (step a).

Removal of the protective group from intermediates **31** applying methods well known in the art and as described above furnishes intermediates **N** (step b).

Alkylation of the hydroxy group of intermediates **31** with an alkylating agent of type R¹⁵LG in which LG is a suitable leaving group and R¹⁵ as described herein, using conditions as described above, gives intermediates **32** (step c).

Removal of the protective group from intermediates **32** applying methods well known in the art and as described under Scheme 8, step b, furnishes intermediates **O** (step d).

In another embodiment, intermediates **2** are intermediates of type **P,** in which A is as defined herein, m is 1 or 2, Ar signifies an aryl group and HET signifies an heterocyclyl or heteroaryl group. Intermediates of that type can be prepared in analogy to literature methods (e.g. Bioorg. Med. Chem. 2013, 21(7), 1756) or as exemplified by the synthetic procedure outlined in Scheme 10.

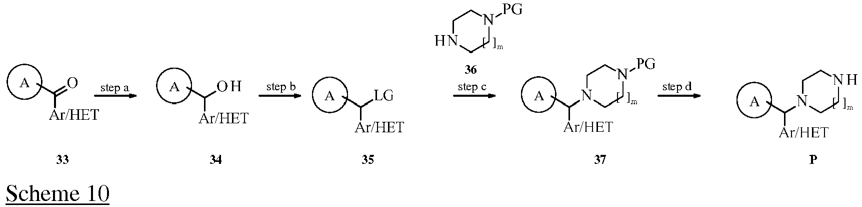

The carbonyl group in intermediates **33**, wherein Ar is aryl and HET is heteroaryl or heterocyclyl can be reduced by methods well known in the art, e.g. using NaBH₄ in MeOH, to give intermediates **34** (step a).

The hydroxyl group of intermediates **34** can be converted into a suitable leaving group such as chlorine, bromine, OSO₂alkyl (e.g. mesylate (methanesulfonate), OSO₂fluoroalkyl (e.g. triflate (trifluoromethanesulfonate) or OSO₂aryl (e.g. tosylate (p-toluenesulfonate)), e.g. a chlorine group by reaction with thionyl chloride in toluene, to give intermediates **34** (step b).

Intermediates **35** can be reacted with commercially available monoprotected piperazines (m = 1) or homopiperazines (m = 2) **36,** wherein PG is a protective group, such as Boc, Cbz or Bn, optionally in the presence of KI, using a suitable base and solvent such as TEA or Huenig's base in AcCN to yield intermediates **37** (step c).

Removal of the protective group from intermediates **37** applying methods well known in the art and as described above furnishes intermediates **P** (step d).

In another embodiment, intermediates **2** are intermediates of type **Q** in which A is as defined herein, m is 1 or 2, Ar signifies an aryl group and HET signifies a heteroaryl group. Intermediates of that type can be prepared in analogy to literature methods (e.g. Bioorg. Med. Chem. Lett. 2006, 16(16), 4349; Bioorg. Med. Chem. Lett. 2010, 20(12), 3788) and as exemplified by the synthetic procedure outlined in Scheme 11.

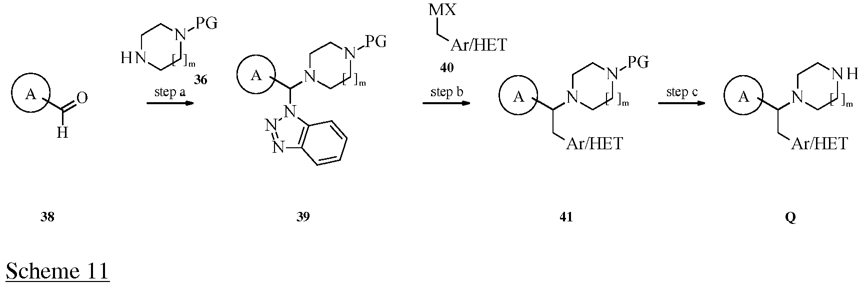

Condensation of aryl or heteroaryl aldehydes **38** with monoprotected piperazines (m = 1) or homopiperazines (m = 2) **36** in which PG is a suitable protective group such as a Boc group and benzotriazole for example in refluxing toluene under azeotropic removal of H₂O gives intermediates **39** (step a).

Intermediates **39** are reacted in situ with benzylic Grignard or zinc reagents **40** of the type Ar/HETCH₂MX with MX being a group such as MgCl, MgBr, ZnCl or ZnBr to afford intermediates **41** (step b).

Removal of the protective group from intermediates **41** applying methods well known in the art and as described above furnishes intermediates **Q** (step c).

In another embodiment, intermediates **2** in which X = N are intermediates of type **R** and **S** wherein m and R¹⁴ are as defined herein and R¹⁸ and R¹⁹ are each independently selected from hydrogen, substituted or unsubstituted alkyl, cyano, alkoxy and halogen, wherein substituted alkyl is as defined herein. Intermediates of that type can be prepared in analogy to literature methods (e.g. WO2007/098418) and as exemplified by the synthetic procedure outlined in Scheme 12.

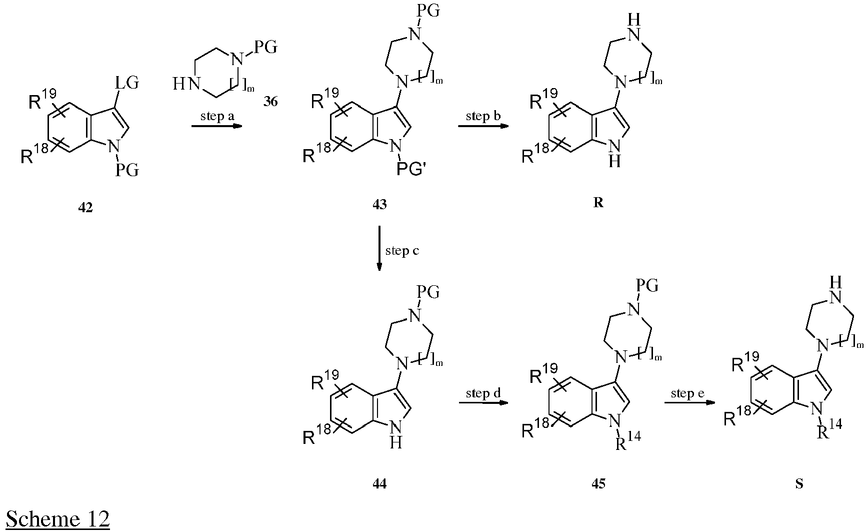

Indole intermediates **42** can be reacted with monoprotected piperazines (m = 1) or homopiperazines (m = 2) **36** in which PG is a suitable protective group such as a Boc, Cbz or Bn group and LG is an appropriate leaving group such as -OAc or iodine, to yield intermediates **43** (step a). Reactions of this type are described in literature (e.g. WO2007/098418).

Concomitant or sequential removal of the protective groups from intermediates **43** applying methods well known in the art and as described above furnishes intermediates **R** (step b).

Selective removal of the indole protective group from intermediates **43** by methods well-known in the art gives intermediates **44** (step c). Intermediates **44** can be converted into intermediates **45** using compounds of the type R¹⁴LG by methods know in the art and as described under Scheme 6, step a (step d). Removal of the protective group from intermediates **45** applying methods well known in the art and as described above furnishes intermediates **S** (step e).

In another embodiment, intermediates **2** are intermediates of type **T,** in which A is as defined herein, m is 1 or 2 and R¹ is selected from substituted or unsubstituted alkyl, substituted or unsubstituted cycloalkyl, substituted or unsubstituted aryl and substituted or unsubstituted heteroaryl. Intermediates of that type can be prepared in analogy to literature methods (e.g. Tetrahedron Letters 1990, 31(39), 5547) or as exemplified by the synthetic procedure outlined in Scheme 13.

The carbonyl group in intermediates **46** be reacted by reductive amination with an amine **36** by methods well known in the art, for example catalyzed by an acid, such as TiCl₄, leading to the imine, which is then directly reduced *in situ* to the corresponding amine intermediate **47** with a reducing agent such as sodium cyanoborohydride (step a).

Removal of the protective group from intermediates **47** applying methods well known in the art and as described before furnishes intermediates **T** (step b).

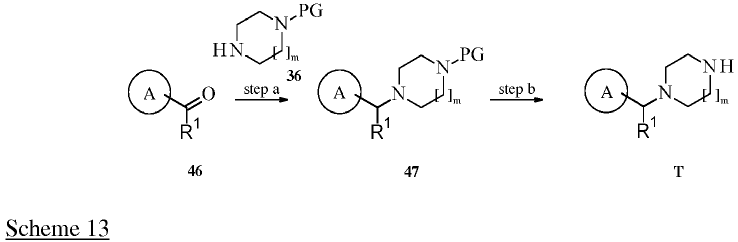

In another embodiment, intermediates **2** are intermediates of type **U,** in which A is an aryl-substituted pyrazole and m is as described herein. Intermediates of that type can be prepared in analogy to literature methods (e.g. J. Med. Chem. 2018, 67(7), 3008) or as exemplified by the synthetic procedure outlined in Scheme 14.

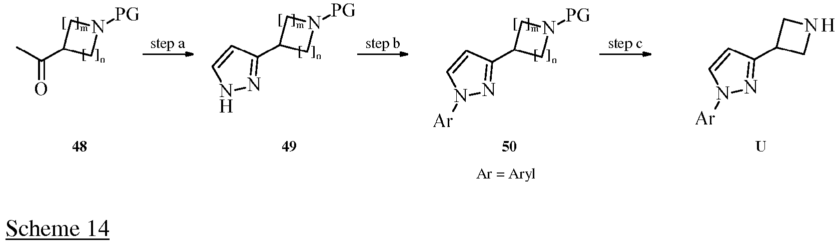

The ketone in intermediates **48** is reacted with N,N-dimethylformamide dimethyl acetal and hydrazine dihydrochloride to yield the pyrazole intermediate **49** (step a).

An aryl-boronic acid is then reacted, using the reagents copper(II)acetate and pyridine to facilitate the reaction, to form intermediate **50** (step b). Removal of the protective group from intermediates **50** applying methods well known in the art and as described above furnishes intermediates **U** (step c).

In some embodiments, intermediates **2** are intermediates of type **V** in which m, n are as described herein, A is an optionally further substituted aryl or heteroaryl ring and R²¹ to R²³ are each independently selected from hydrogen, substituted or unsubstituted (cyclo)alkyl, (cyclo)alkoxy, substituted or unsubstituted aryl, R^{b}R^{c}N, cyano, heterocycle, methylsulfonyl and halogen, wherein substituted alkyl, aryl and heteroaryl is as defined herein. Intermediates of that type can be prepared by methods well known in the art and as exemplified by the general synthetic procedures outlined in Scheme 15.

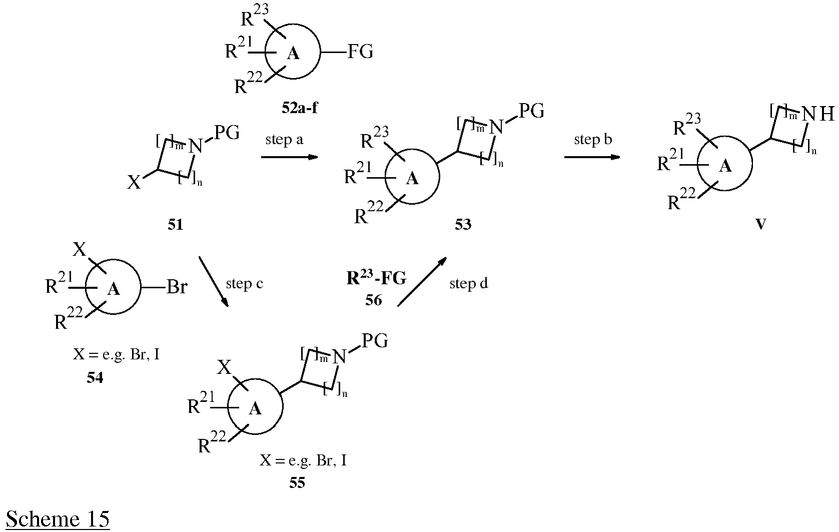

Commercially available intermediates **51** in which PG signifies a suitable protecting group and X is bromide or iodide can be subjected to cross-coupling reactions such as Negishi, Heck, Stille, Suzuki, Sonogashira or Buchwald-Hartwig coupling reactions with compounds **52,** either commercially available or prepared by methods known in the art, in which FG signifies a suitable functional group such as, e.g. chloro, bromo, iodo, - OSO₂alkyl (e.g. mesylate (methanesulfonate), -OSO₂fluoroalkyl (e.g. triflate (trifluoromethanesulfonate) or -OSO₂aryl (e.g. tosylate (p-toluenesulfonate) (step a). Reactions of this type are broadly described in literature and well known to persons skilled in the art.

For example, intermediates **51** can be reacted with aryl or heteroaryl boronic acids **52a** (FG = B(OH)₂) or boronic esters **52b** (FG = e.g. 4,4,5,5-tetramethyl-2-phenyl-1,3,2-dioxaborolane (pinacol) ester) either commercially available or prepared using literature procedures as described for example in "Boronic Acids - Preparation and Applications in Organic Synthesis and Medicine" by Dennis G. Hall (ed.) 1st Ed., 2005, John Wiley & Sons, New York) using a suitable catalyst (e.g. dichloro[1,1'-bis(diphenylphosphino)-ferrocene]palladium(II) dichloromethane adduct, tetrakis(triphenylphosphine)palladium(0) or palladium(II)acetate with triphenylphosphine) in an appropriate solvent (e.g. dioxane, dimethoxyethane, water, toluene, DMF or mixtures thereof) and a suitable base (e.g. Na₂CO₃, NaHCO₃, KF, K₂CO₃ or TEA) at temperatures between room temperature and the boiling point of the solvent or solvent mixture, to yield intermediates **53** (step a). Suzuki reactions of this type are broadly described in literature (e.g. A. Suzuki, Pure Appl. Chem. 1991, 63, 419-422; A. Suzuki, N. Miyaura, Chem. Rev. 1995, 95, 2457-2483; A. Suzuki, J. Organomet. Chem. 1999, 576, 147-168; V. Polshettiwar et al., Chem. Sus. Chem. 2010, 3, 502-522) and are well known to those skilled in the art. Alternatively, aryl- or heteroaryl-trifluoroborates **52c** (FG = BF₃) can be used in the cross-coupling reaction applying a palladium catalyst such as, e.g. tetrakis(triphenylphosphine)-palladium(0), palladium(II) acetate or dichloro[1,1'-bis(diphenylphosphino)ferrocene]-palladium(II) dichloromethane adduct in the presence of a suitable base such as cesium carbonate or potassium phosphate in solvents such as toluene, THF, dioxane, water or mixtures thereof, at temperatures between room temperature and the boiling point of the solvent or solvent mixture.

Alternatively, intermediates **51** can be reacted with aryl or heteroaryl stannanes **52d** in which FG is Sn(alkyl)₃ and alkyl is perferable n-butyl or methyl, using a suitable catalyst and solvent such as, e.g. tetrakis(triphenylphosphine)-palladium(0) in DMF at temperatures between room temperature and the boiling point of the solvent or solvent mixture to provide intermediates **53** (step a). Stille reactions of that type are well known in the art and described in literature, e.g. Org. React. 1997, 50, 1-652, ACS Catal. 2015, 5, 3040-3053.

Furthermore, intermediates **51** can be reacted with aryl or heteroarylzinc halides **52e** in which FG is ZnHal and Hal preferably bromide or iodide, either commercially available or prepared by literature methods, using an appropriate catalyst and solvent system such as, e.g. [1,1'-bis(diphenylphosphino)ferrocene]dichloropalladium(II) and copper(I)iodide in DMA, or tetrakis(triphenylphosphine)palladium(0) in THF or DMF at temperatures between room temperature and the boiling point of the solvent to provide intermediates **53.** (step a). Negishi reactions of that type are well known in the art and also described in literature, e.g. Org. Lett., 2005, 7, 4871, ACS Catal. 2016, 6 (3), 1540-1552. Acc. Chem. Res. 1982, 15 (11), pp 340-348. Alternatively, intermediates **53** may be prepared by converting intermediates **51** in which X is for example iodide into the corresponding zinc species by applying literature methods (e.g. reaction of **51** with Zn powder in the presence of chlorotrimethylsilane and 1,2-dibromoethane in a suitable solvent such as DMA) and coupling of the zinc species with aryl- or heteroarylbromides- or iodides under the conditions mentioned before.

Alternatively, intermediates **51** in which X is preferably bromide can be subjected to a cross-electrophile coupling with aryl- or heteroarylbromides **52f** in which FG signifies bromide under irradiation with a 420 nm blue light lamp using an appropriate photo catalyst such as [Ir{dF(CF₃)ppy}2(dtbpy)]PF₆ ([4,4'-bis(1,1-dimethylethyl)-2,2'-bipyridine-N1,N1']bis[3,5-difluoro-2-[5-(trifluoromethyl)-2-pyridinyl-N]phenyl-C]Iridium(III) hexafluorophosphate), a Nickel catalyst like NiCl₂ glyme (dichloro(dimethoxyethane)nickel), 4,4'-di-tert-butyl-2,2'-dipyridyl and tris(trimethylsilyl)silane, in the presence of a suitable base such as anhydrous sodium carbonate in a solvent like DME. Reactions of this type are described in literature, e.g. J. Am. Chem. Soc. 2016, 138, 8084. (step a).

Removal of the protective group from intermediates **53** applying methods well known in the art and as described for example under Scheme 2, step c, furnishes intermediates **V** (step b).

Intermediates **53** may alternatively be prepared from intermediates **51** and aryl or heteroaryl bromides **54,** either commercially available or prepared by methods known in the art, applying the transformations described before under step a to furnish intermediates **55** (step c).

Intermediates **55** can be further reacted with compounds **56** applying the same synthetic strategies as described before under step a to provide intermediates **53** (step d).

Intermediates **53** in which R²³ signifies an amine group of type R^{b}R^{c}N in which R^{b} is hydrogen, alkyl or aryl and R^{c} is alkyl or aryl or in which R^{b} and R^{c}, taken together with the nitogen atom to which they are attached, form an optionally further substituted 4-11-membered, mono- or bicyclic heterocyclic ring, can be synthesized for example from reaction of **55** with primary or secondary amines R^{b}R^{c}NH and using for example a suitable catalyst (e.g. Pd(OAc)₂, Pd₂(dba)₃), ligand (e.g. BINAP, Xphos, BrettPhos, RuPhos), base (e.g. Cs₂CO₃, K₂CO₃, KOt-Bu, LiHMDS, K₃PO₄) and solvent (e.g. toluene, THF, dioxane). Buchwald-Hartwig reactions of that type are known in the art and described in literature (e.g. Angew. Chem. Int. Ed. 2008, 47, 6338; Chem. Rev. 2008, 108, 3054, J. Organomet. Chem. 2018, 861, 17) (step d).

In some embodiment, compounds for Formula (I) are compounds **Id** and **Ie** in which m and n are as described herein, R²¹ and R²² are each independently selected from hydrogen, substituted or unsubstituted (cyclo)alkyl, haloalkyl, (cyclo)alkoxy, substituted or unsubstituted aryl, R^{b}R^{c}N, cyano, heterocycle, methylsulfonyl and halogen, X is Hal, A is optionally substituted aryl or optionally substituted heteroaryl, C is optionally substituted aryl or a 4-7-membered heterocyle containing at least one nitrogen atom to which it is linked to ring A. Intermediates of that type can be prepared by methods well known in the art and as exemplified by the general synthetic procedures outlined in Scheme 16.

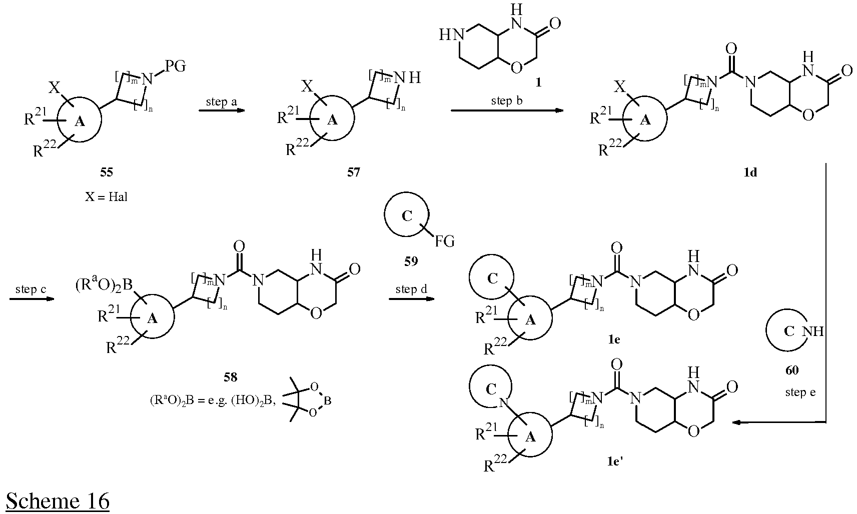

The protective group of intermediates **55** can be removed applying methods well known in the art and as described for example under Scheme 2, step c, to give intermediates **57** (step a).

### Intermediates 50 can be coupled with intermediates 1 under the conditions described under Scheme 1, step a, to provide compounds 1d (step b).

The bromo or iodo substituent in compounds **1d** can be converted into a boronic acid or boronic ester (e.g. pinacol ester) according to methods described in literature or as outlined under Scheme 15, step a, to yield intermediates **58** (step c).

Intermediates **58** can be reacted with compounds **59,** either commercially available or prepared by literature methods and in which FG is an appropriate functional group such as chloride, bromide, OSO₂alkyl (e.g. mesylate (methanesulfonate), -OSO₂fluoroalkyl (e.g. triflate (trifluoromethanesulfonate) or -OSO₂aryl (e.g. tosylate (p-toluenesulfonate) in a Suzuki coupling using for example the reaction conditions described under Scheme 15, step a, to provide compounds **1e** (step d).

Compounds **1e** in which ring C is a 4-7-membered heterocyle linked via its nitrogen to ring A can be prepared for example by Buchwald-Hartwig coupling reaction of compounds **1d** with compounds **60** applying for example the conditions described under Scheme 15, step d (step e).

In some embodiment, intermediates **2** are intermediates of type **W, X** and **Y** in which m, n are as described herein, L is a covalent bond and A is a 5-membered heteroaryl ring further substituted by an optionally further substituted aryl ring. Compounds of that type can be prepared by methods well known in the art or by the methods exemplified in Schemes 17, 18 and 19 below.

Intermediates of type **W,** wherein each Y is independently CH or a heteroatom, e.g. a heteroatom selected from N, O and S, can be prepared by a variety of conditions, which may be exemplified by the general synthetic procedure outlined in Scheme 17. For example, if the 5-membered heteroaryl ring in compounds **62** is a bromo-substituted 1H-pyrrole, 1H-imidazole, 1H-pyrazole or 1H-triazole, it can be subjected to a Suzuki coupling reaction with optionally substituted phenyl-boronic acids **61a** (B(OR^{a})₂ = B(OH)₂) or boronic esters **61b** (e.g. B(OR^{a})₂ = 4,4,5,5-tetramethyl-2-phenyl-1,3,2-dioxaborolane (pinacol) ester)) in which R²⁷ and R²⁸ are each independently selected from hydrogen, substituted or unsubstituted (cyclo)alkyl, haloalkyl, (cyclo)alkoxy, substituted or unsubstituted aryl, R^{b}R^{c}N, cyano, substituted or unsubstituted heteroaryl, heterocycle, methylsulfonyl and halogen applying methods known in the art and as described under Scheme 15, step a, to provide intermediates **63** (step a).

Intermediates **63** can be reacted with compounds **64,** either commercially available or prepared in analogy to literature methods, for example applying photoredox conditions as described under Scheme 15, step a, to give intermediates **65** (step b).

Removal of the protective group from intermediates **65** applying methods well known in the art and as described for example under Scheme 2, step c, furnishes intermediates **W** (step c).

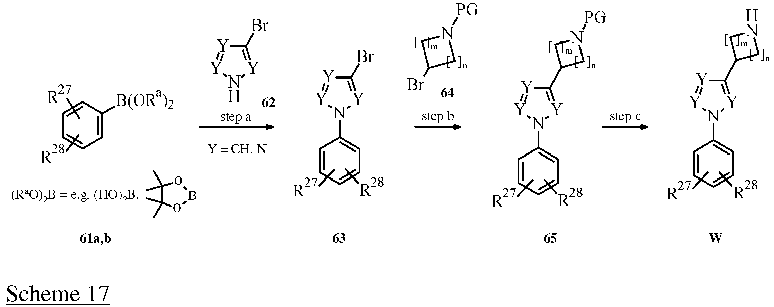

Intermediates of type **X** in which the 5-membered heteroaryl ring is an 1,2,4-oxadiazole further substituted at position-3 with an optionally further substituted phenyl ring can be prepared for example by the general synthetic procedure outlined in Scheme 18.

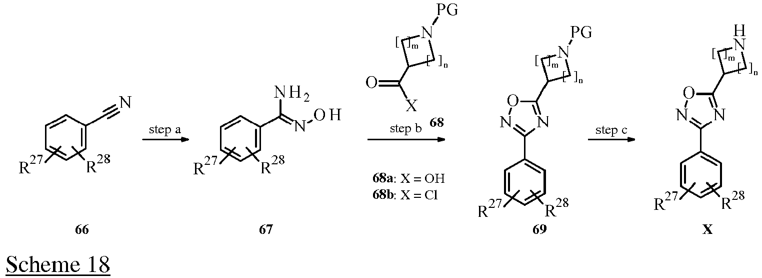

Benzonitriles **66,** commercially available or synthesized by methods known in the art, can be reacted with hydroxylamine, for example using hydroxylamine hydrochloride, and K₂CO₃ in EtOH at temperatures ranging from RT to the boiling point of the solvent to provide the amidoximes **67** (step a).

Coupling of intermediates **67** with activated carboxylic acids **68a** (X = H) or carboxylic acid chlorides **68b** (X = Cl), in which PG signifies a suitable protecting group, with subsequent cyclodehydration provides intermediates **69** (step b). Amide couplings of this type are widely described in the literature and can be accomplished by the usage of coupling reagents such as CDI, DCC, HATU, HBTU, HOBT, TBTU, T3P or Mukaiyama reagent (Mukaiyama T. Angew. Chem., Int. Ed. Engl. 1979, 18, 707) in a suitable solvent e.g., DMF, DMA, DCM or dioxane, optionally in the presence of a base (e.g., NEt₃, DIPEA (Huenig's base) or DMAP). Alternatively, the carboxylic acids **68a** can be converted into their acid chlorides **68b** by treatment with, e.g. thionyl chloride or oxalyl chloride, neat or optionally in a solvent such as DCM. Reaction of the acid chloride with intermediates **68** in an appropriate solvent such as DCM or DMF and a base, e.g. NEt₃, Huenig's base, pyridine, DMAP or lithium bis(trimethylsilyl)amide at temperatures ranging from 0°C to the reflux temperature of the solvent or solvent mixture yields the acylated form of intermediates **67** that can be dehydrated for example under elevated temperatures to provide intermediates **69** (step b).

Removal of the protective group from intermediates **69** applying methods well known in the art and as described for example under Scheme 2, step c, furnishes intermediates **X** (step c).

Intermediates of type **Y** in which the 5-membered heteroaryl ring is an 1,3,4-oxadiazole further substituted at position-5 with an optionally further substituted phenyl ring can be prepared by methods described in literature or for example by the general synthetic procedure outlined in Scheme 19.

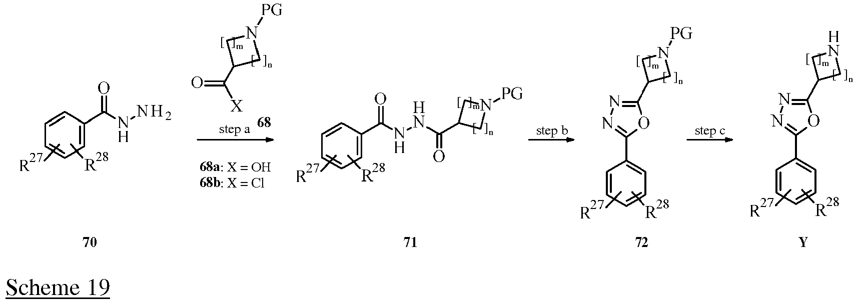

Acylation of acylhydrazides **70,** either commercially available or prepared my methods well known in the art, with activated carboxylic acids **68a** (X = H) or carboxylic acid chlorides **68b** (X = Cl) applying the conditions described under Scheme 18, step b, provides intermediates **71** (step a).

Subsequent cyclodehydration of intermediates **71,** for example by heating, yields intermediates **72** (step b).

Removal of the protective group from intermediates **72** applying methods well known in the art and as described for example under Scheme 2, step c, furnishes intermediates **Y** (step c).

In one aspect, the present invention provides a process of manufacturing the compounds of formula (I) as described herein, comprising:
reacting 4a,5,6,7,8,8a-hexahydro-4H-pyrido[4,3-b][1,4]oxazin-3-one (**1**), 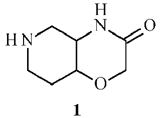
with a heterocyclic amine **2,** wherein A, L, X, m and n are as defined herein 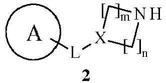
in the presence of a base and a urea forming reagent,
   to form said compounds of formula (**I**).

In a further aspect, the present invention provides a process of manufacturing the compounds of formula (Ic) as described herein, comprising:
reacting 4a,5,6,7,8,8a-hexahydro-4H-pyrido[4,3-b][1,4]oxazin-3-one (**1**), 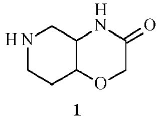
with a heterocyclic amine **2a,** wherein A, L, X, m, n, and R²⁰ to R²³ are as defined herein 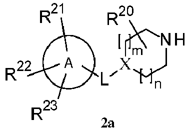
in the presence of a base and a urea forming reagent, to form said compounds of formula (Ic).

In one embodiment, there is provided a process according to the invention, wherein said base is sodium bicarbonate.

In one embodiment, there is provided a process according to the invention, wherein said urea forming reagent is selected from bis(trichloromethyl) carbonate, phosgene, trichloromethyl chloroformate, (4-nitrophenyl)carbonate and 1,1'-carbonyldiimidazole, preferably wherein said urea forming reagent is bis(trichloromethyl) carbonate.

### MAGL Inhibitory Activity

Compounds of the present invention are MAGL inhibitors. Thus, in one aspect, the present invention provides the use of compounds of formula (Ic) as described herein for inhibiting MAGL in a mammal.

In a further aspect, the present invention provides compounds of formula (Ic) as described herein for use in a method of inhibiting MAGL in a mammal.

In a further aspect, the present invention provides the use of compounds of formula (Ic) as described herein for the preparation of a medicament for inhibiting MAGL in a mammal.

In a further aspect, the present invention provides a method for inhibiting MAGL in a mammal, which method comprises administering an effective amount of a compound of formula (Ic) as described herein to the mammal.

Compounds were profiled for MAGL inhibitory activity by measuring the enzymatic activity of MAGL by following the hydrolysis of 4-nitrophenylacetate resulting in 4-nitrophenol, which absorbs at 405-412 nm (G.G. Muccioli, G. Labar, D.M. Lambert, Chem. Bio. Chem. 2008, 9, 2704-2710). This assay is hereinafter abbreviated "4-NPA assay".

The assay was carried out in 384 well assay plates (black with clear bottom, non-binding surface treated, Corning Ref. 3655) in a total volume of 40 µL. Compound dilutions were made in 100% DMSO (VWR Chemicals 23500.297) in a polypropylene plate in 3-fold dilution steps to give a final concentration range in the assay from 25 µM to 1.7 nM. 1 µL compound dilutions (100% DMSO) were added to 19 µL MAGL (recombinant wild-type) in assay buffer (50 mM TRIS (GIBCO, 15567-027), 1 mM EDTA (Fluka, 03690-100mL)). The plate was shaked for 1 min at 2000 rpm (Variomag Teleshake) and then incubated for 15 min at RT. To start the reaction, 20 µL 4-Nitrophenlyacetate (Sigma N-8130) in assay buffer with 6% EtOH was added. The final concentrations in the assay were 1 nM MAGL and 300 µM 4-Nitrophenylacetate. After shaking (1 min, 2000 rpm) and 5 min incubation at RT, the absorbance at 405 nm was measured for a fist time (Molecular Devices, SpectraMax Paradigm). A second measurement was then done after incubation for 80 min at RT. From the two measurements, the slope was calculated by substracting the first from the second measurement.

Alternatively, compounds were profiled for MAGL inhibitory activity by determining the enzymatic activity by following the hydrolysis of the natural substrate 2-arachidonoylglycerol (2-AG) resulting in arachidonic acid, which can be followed by mass spectrometry. This assay is hereinafter abbreviated "2-AG assay".

The 2-AG assay was carried out in 384 well assay plates (PP, Greiner Cat# 784201) in a total volume of 20 µL. Compound dilutions were made in 100% DMSO (VWR Chemicals 23500.297) in a polypropylene plate in 3-fold dilution steps to give a final concentration range in the assay from 12.5 µM to 0.8 pM. 0.25µL compound dilutions (100% DMSO) were added to 9 µL MAGL in assay buffer (50 mM TRIS (GIBCO, 15567-027), 1 mM EDTA (Fluka, 03690-100 mL), 0.01% (v/v) Tween. After shaking, the plate was incubated for 15 min at RT. To start the reaction, 10 µL 2-arachidonoylglycerol in assay buffer was added. The final concentrations in the assay was 50 pM MAGL and 8 µM 2-arachidonoylglyerol. After shaking and 30 min incubation at RT, the reaction was quenched by the addition of 40µL of ACN containing 4µM of d8-arachidonic acid. The amount of arachidonic acid was traced by an online SPE system (Agilent Rapidfire) coupled to a triple quadrupole mass spectrometer (Agilent 6460). A C18 SPE cartridge (G9205A) was used in an ACN/water liquid setup. The mass spectrometer was operated in negative electrospray mode following the mass transitions 303.1 → 259.1 for arachidonic acid and 311.1 → 267.0 for d8-arachidonic acid. The activity of the compounds was calculated based on the ratio of intensities [arachidonic acid / d8-arachidonic acid].

**Table 1**

| **Example** | **Name** | **Structure** | **IC₅₀ MAGL [µM]^{[a]}** |
|---|---|---|---|
| **1** | rac-(4aR,8aS)-6-(4-Benzhydrylpiperidine-1-carbonyl)-4,4a,5,7,8,8a-hexahydropyrido [4,3-b][1,4]oxazin-3-one | 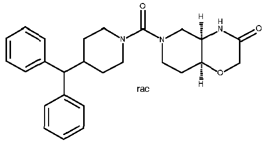 | 0.003 |
| **2** | rac-(4aR,8aS)-6-(4-(5-Chloro-1-(cyclopropylmethyl)-1H-indol-3-yl)piperidine-1-carbonyl)hexahydro-2H-pyrido[4,3-b][1,4]oxazin-3(4H)-one | 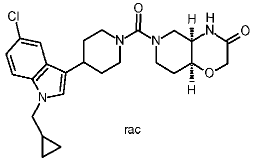 | 0.002 |
| **3** | rac-(4aR,8aS)-6-(4-(5-Chloro-1-methyl-1H-indol-3-yl)piperidine-1-carbonyl)hexahydro-2H-pyrido[4,3-b][1,4]oxazin-3(4H)-one | 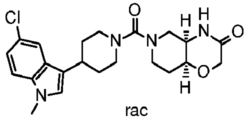 | 0.003 |
| **4** | rac-(4aR,8aS)-6-(4-(9H-Fluoren-9-yl)piperidine-1-carbonyl)hexahydro-2H-pyrido[4,3-b][1,4]oxazin-3(4H)-one | 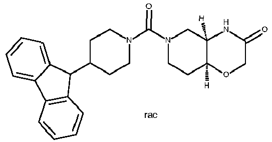 | 0.005 |
| **5** | (+)-cis-6-[4-(6-Fluoro-1H-indol-3-yl)piperidine-1-carbonyl]-4,4a,5,7,8,8a-hexahydropyrido[4,3-b][1,4]oxazin-3-one | 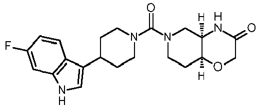 | 0.014 |
| **6** | (4aR,8aS)-6-(4-(1-(4-Fluorophenyl)-3-methoxypropyl)piperidine-1-carbonyl)hexahydro-2H-pyrido[4,3-b][1,4]oxazin-3(4H)-one | 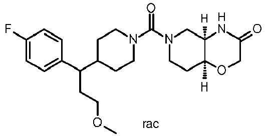 | 0.015 |
| **7** | rac-(4aR,8aS)-6-[4-(6-Fluoro-1H-indol-3-yl)piperidine-1-carbonyl]-4,4a,5,7,8,8a-hexahydropyrido [4,3-b][1,4]oxazin-3-one | 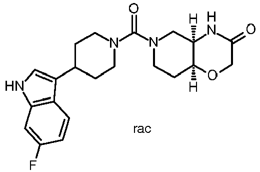 | 0.043 |
| **8** | (4aR,8aS)-6-(4-((4-Fluorophenyl)(methoxy)meth yl)piperidine-1-carbonyl)hexahydro-2H-pyrido[4,3-b][1,4]oxazin-3(4H)-one | 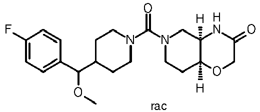 | 0.091 |
| **9** | rac-(4aR,8aS)-6-(4-(5-Fluorobenzo[d]isoxazol-3-yl)piperidine-1-carbonyl)hexahydro-2H-pyrido[4,3-b][1,4]oxazin-3(4H)-one | 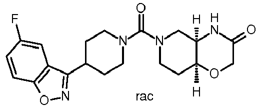 | 0.644 |
| **10** | cis-6-(4-((4-Chlorophenyl)(phenyl)methy l)piperazine-1-carbonyl)hexahydro-2H-pyrido[4,3-b][1,4]oxazin-3(4H)-one | 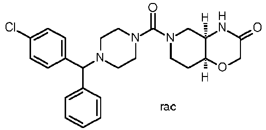 | 0.005 |
| **11** | (+)-cis-6-(4-(bis(4-Fluorophenyl)methyl)piperaz ine-1-carbonyl)hexahydro-2H-pyrido[4,3-b][1,4]oxazin-3(4H)-one | 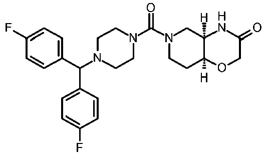 | 0.008 |
| **12** | rac-(4aR,8aS)-6-[4-[bis(4-Fluorophenyl)methyl]piperaz ine-1-carbonyl]-4,4a,5,7,8,8a-hexahydropyrido[4,3-b][1,4]oxazin-3-one | 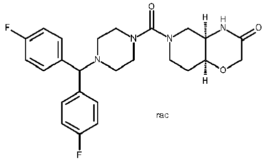 | 0.012 |
| **13** | (-)-cis-6-(4-(bis(4-Fluorophenyl)methyl)piperaz ine-1-carbonyl)hexahydro-2H-pyrido[4,3-b][1,4]oxazin-3(4H)-one | 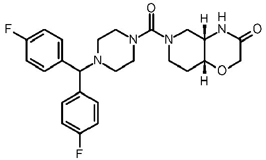 | 0.088 |
| **14** | rac-(4aR,8aS)-6-(4-(5-Chloro-1H-indol-3-yl)piperazine-1-carbonyl)hexahydro-2H-pyrido[4,3-b][1,4]oxazin-3(4H)-one | 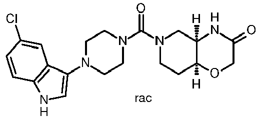 | 0.133 |
| **15** | rac-(4aR,8aS)-6-(4-(1-Phenylethyl)piperazine-1-carbonyl)hexahydro-2H-pyrido[4,3-b][1,4]oxazin-3(4H)-one | 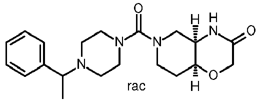 | 3.0 |
| **16** | rac-(4aR,8aS)-6-(4-(1-Methyl-1H-indazol-5-yl)piperidine-1-carbonyl)hexahydro-2H-pyrido[4,3-b][1,4]oxazin-3(4H)-one | 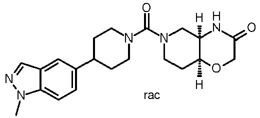 | 1.5 |
| **17** | rac-(4aR,8aS)-6-[4-[(4-Fluorophenyl)-phenylmethyl]piperazine-1-carbonyl]-4,4a,5,7,8,8a-hexahydropyrido [4,3-b][1,4]oxazin-3-one | 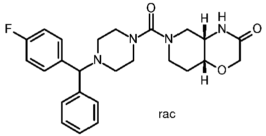 | 0.006 |
| **19** | (+)- or (-)-cis-6-(4-(5-Chloro-1-cyclopropyl-1H-indol-3-yl)piperidine-1-carbonyl)hexahydro-2H-pyrido[4,3-b][1,4]oxazin-3(4H)-one | 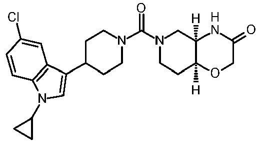 | 0.001 |
| **20** | rac-(4aR,8aS)-6-(4-(5-Chloro-1-(1-chloro-3-hydroxypropan-2-yl)-1H-indol-3-yl)piperidine-1-carbonyl)hexahydro-2H-pyrido[4,3-b][1,4]oxazin-3(4H)-one | 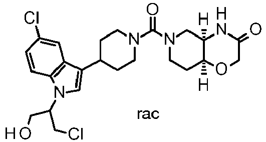 | 0.002 |
| **21** | (+)- or (-)-cis-6-(4-(bis(4-Fluorophenyl)methyl)piperid ine-1-carbonyl)hexahydro-2H-pyrido[4,3-b][1,4]oxazin-3(4H)-one | 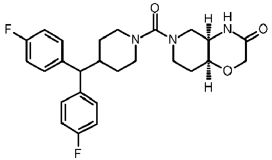 | 0.001 |
| **22** | (+)-or (-)-cis-6-(4-(5-Chloro-1-(oxetan-3-yl)-1H-indol-3-yl)piperidine-1-carbonyl)hexahydro-2H-pyrido[4,3-b][1,4]oxazin-3(4H)-one | 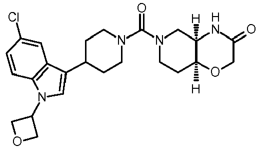 | 0.002 |
| **23** | (+)- or (-)-cis-6-(4-(5-Chloro-1-(oxetan-3-ylmethyl)-1H-indol-3-yl)piperidine-1-carbonyl)hexahydro-2H-pyrido[4,3-b][1,4]oxazin-3(4H)-one | 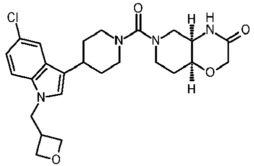 | 0.003 |
| **24** | (+)- or (-)-cis-6-(4-(1-(2-((tertButyldimethylsilyl)oxy)ethyl )-5-chloro-1H-indol-3-yl)piperidine-1-carbonyl)hexahydro-2H-pyrido[4,3-b][1,4]oxazin-3(4H)-one | 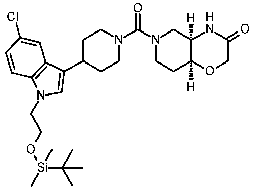 | 0.0007 |
| **25** | (+)- or (-)-cis-6-(4-(5-Chloro-1-(2-hydroxyethyl)-1H-indol-3-yl)piperidine-1-carbonyl)hexahydro-2H-pyrido[4,3-b][1,4]oxazin-3(4H)-one | 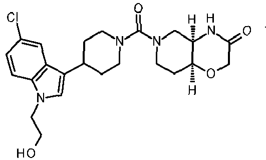 | 0.002 |
| **26** | rac-cis-6-(4-(1-(4-Fluorophenyl)-3-hydroxypropyl)piperidine-1-carbonyl)hexahydro-2H-pyrido[4,3-b][1,4]oxazin-3(4H)-one | 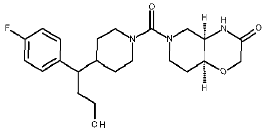 | 0.033 |
| **27** | rac-(4aR,8aS)-6-(4-(1-(4-(Trifluoromethyl)phenyl)ethy l)piperidine-1-carbonyl)hexahydro-2H-pyrido[4,3-b][1,4]oxazin-3(4H)-one | 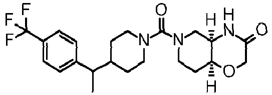 | 0.005 |
| **28** | rac-(4aR,8aS)-6-(4-(1-(2-Chloro-4-fluorophenoxy)ethyl)piperidi ne-1-carbonyl)hexahydro-2H-pyrido[4,3-b][1,4]oxazin-3(4H)-one | 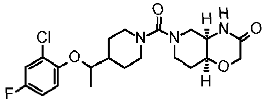 | 0.002 |
| **29** | (4aR,8aS)-6-(4-(5-(Trifluoromethyl)pyridin-3-yl)piperidine-1-carbonyl)hexahydro-2H-pyrido[4,3-b][1,4]oxazin-3(4H)-one | 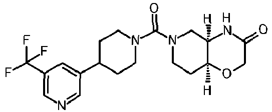 | 0.703 |
| **30** | (4aR,8aS)-6-(4-(Difluoro(4-(trifluoromethyl)phenyl)meth yl)piperidine-1-carbonyl)hexahydro-2H-pyrido[4,3-b][1,4]oxazin-3(4H)-one | 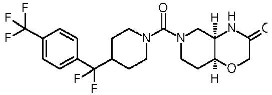 | 0.025 |
| **31** | (+)-(4aR,8aS)-6-(4-((R or S)-1-(4-Fluorophenyl)-3-hydroxypropyl)piperidine-1-carbonyl)hexahydro-2H-pyrido[4,3-b][1,4]oxazin-3(4H)-one | 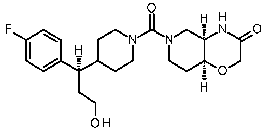 | 0.017 |
| **32** | (-)-(4aR,8aS)--6-(4-((S or R)-1-(4-Fluorophenyl)-3-hydroxypropyl)piperidine-1-carbonyl)hexahydro-2H-pyrido[4,3-b][1,4]oxazin-3(4H)-one | 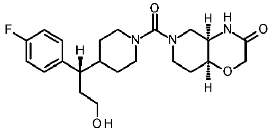 | 0.203 |
| **33** | (+) or (-)-cis-6-(4-((S or R)-1-(2-Chloro-4-fluorophenoxy)ethyl)piperidi ne-1-carbonyl)hexahydro-2H-pyrido[4,3-b][1,4]oxazin-3(4H)-one | 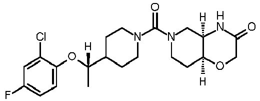 | 0.051 |
| **34** | (+) or (-)-cis-6-(4-((R or S)-1-(2-Chloro-4-fluorophenoxy)ethyl)piperidine-1-carbonyl)hexahydro-2H-pyrido[4,3-b][1,4]oxazin-3(4H)-one | 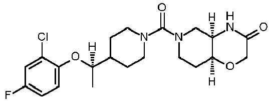 | 0.001 |
| **35** | (+) or (-)-cis-6-(4-(5-Methoxypyridin-3-yl)piperidine-1-carbonyl)hexahydro-2H-pyrido [4,3-b][1,4]oxazin-3(4H)-one | 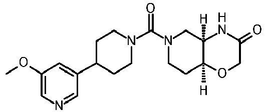 | 2.4 |
| **36** | (+) or (-)-cis-6-(4-(Bis(4-fluorophenyl)methyl)piperidi ne-1-carbonyl)hexahydro-2H-pyrido[4,3-b][1,4]oxazin-3(4H)-one | 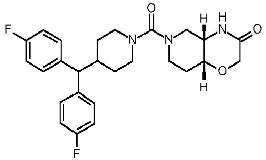 | 0.085 |
| **37** | (+) or (-)-cis-6-(4-((4-Chlorophenyl)difluoromethyl )piperidine-1-carbonyl)hexahydro-2H-pyrido[4,3-b][1,4]oxazin-3(4H)-one | 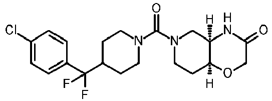 | 0.034 |
| **38** | (+) or (-)-cis-6-(4-(5-(Trifluoromethoxy)pyridin-2-yl)piperidine-1-carbonyl)hexahydro-2H-pyrido [4,3-b][1,4]oxazin-3(4H)-one | 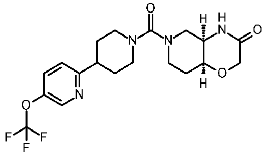 | 0.644 |
| **39** | (+) or (-)-cis-6-(4-((2-Chloro-4-fluorophenyl)difluoromethyl) piperidine-1-carbonyl)hexahydro-2H-pyrido[4,3-b][1,4]oxazin-3(4H)-one | 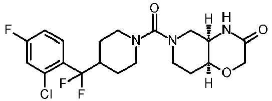 | 0.021 |
| **40** | (+) or (-)-cis-6-(4-(5-Ethylpyridin-3-yl)piperidine-1-carbonyl)hexahydro-2H-pyrido[4,3-b][1,4]oxazin-3(4H)-one | 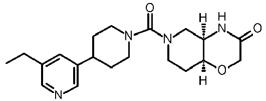 | 0.683 |
| **41** | (+) or (-)-cis-6-(4-(5-(1,1-Difluoroethyl)pyridin-2-yl)piperidine-1-carbonyl)hexahydro-2H-pyrido[4,3-b][1,4]oxazin-3(4H)-one | 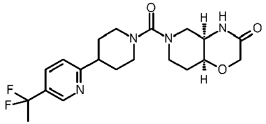 | 0.485 |
| **42** | (+) or (-)-cis-6-(4-(6-Chloro-1-methyl-1H-indazol-3-yl)piperidine-1-carbonyl)hexahydro-2H-pyrido [4,3-b][1,4]oxazin-3(4H)-one | 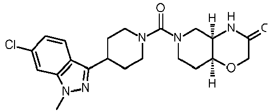 | 0.103 |
| **43** | (4aR,8aS)-6-((3R or 3S)-4-(5-(1,1-Difluoroethyl)pyridin-2-yl)-3-methylpiperidine-1-carbonyl)hexahydro-2H-pyrido[4,3-b][1,4]oxazin-3(4H)-one | 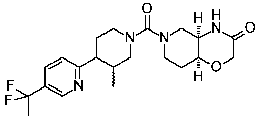 | 0.156^{[b]} |
| **44** | (4aR,8aS)-6-((3S or 3R)-4-(5-(1,1-Difluoroethyl)pyridin-2-yl)-3-methylpiperidine-1-carbonyl)hexahydro-2H-pyrido[4,3-b][1,4]oxazin-3(4H)-one | 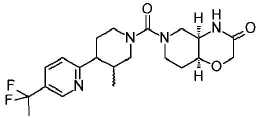 | 0.381^{[b]} |
| **45** | (4aR,8aS)-6-((4R or 4S)-(5-(1,1-Difluoroethyl)pyridin-2-yl)-3-methylpiperidine-1-carbonyl)hexahydro-2H-pyrido[4,3-b][1,4]oxazin-3(4H)-one | 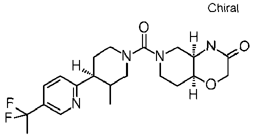 | 3.3^{[b]} |
| **46** | (4aR,8aS)-6-(4-((R or S)-1-(4-(Trifluoromethyl)phenyl)ethy l)piperidine-1-carbonyl)hexahydro-2H-pyrido[4,3-b][1,4]oxazin-3(4H)-one | 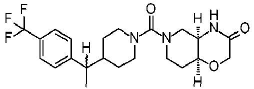 | 0.003^{[b]} |
| **47** | (4aR,8aS)-6-(4-((S or R)-1-(4-(Trifluoromethyl)phenyl)ethy l)piperidine-1-carbonyl)hexahydro-2H-pyrido[4,3-b][1,4]oxazin-3(4H)-one | 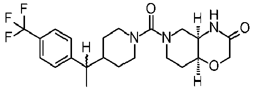 | 0.514^{[b]} |
| **48** | 2-(4-Fluorophenyl)-2-(1-((4aR,8aS)-3-oxooctahydro-2H-pyrido[4,3-b][1,4]oxazine-6-carbonyl)piperidin-4-yl)acetonitrile | 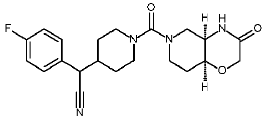 | 0.361^{[b]} |
| **49** | (4aR,8aS)-6-(4-(2,2,2-Trifluoro-1-phenylethyl)piperazine-1-carbonyl)hexahydro-2H-pyrido[4,3-b][1,4]oxazin-3(4H)-one | 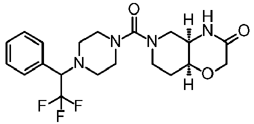 | 1.1^{[b]} |
| **50** | (4aR,8aS)-6-(4-((4-Fluorophenyl)(2,2,2-trifluoroethoxy)methyl)piperi dine-1-carbonyl)hexahydro-2H-pyrido[4,3-b][1,4]oxazin-3(4H)-one | 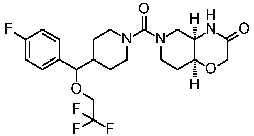 | 0.009^{[b]} |
| **51** | (4aR,8aS)-6-(4-(1-(4-Fluorophenyl)-2-(3-methyl-1,2,4-oxadiazol-5-yl)ethyl)piperidine-1-carbonyl)hexahydro-2H-pyrido[4,3-b][1,4]oxazin-3(4H)-one | 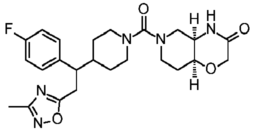 | 0.063^{[b]} |
| **52** | (4aR,8aS)-6-(4-(1-(4-(Trifluoromethyl)phenyl)cycl opropyl)piperidine-1-carbonyl)hexahydro-2H-pyrido[4,3-b][1,4]oxazin-3(4H)-one | 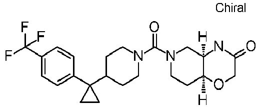 | 0.021^{[b]} |
| **53** | (4aR,8aS)-6-(4-(1-(2,4-Difluorophenyl)-2,2,2-trifluoroethyl)piperazine-1-carbonyl)hexahydro-2H-pyrido[4,3-b][1,4]oxazin-3(4H)-one | 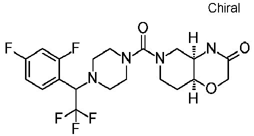 | 0.416^{[b]} |
| **54** | (4aR,8aS)-6-(3-(Bis(4-fluorophenyl)(hydroxy)meth yl)azetidine-1-carbonyl)hexahydro-2H-pyrido[4,3-b][1,4]oxazin-3(4H)-one | 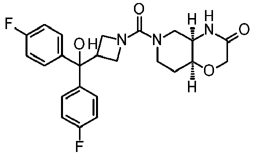 | 0.804^{[b]} |
| **55** | (4aR,8aS)-6-(3-(Fluorobis(4-fluorophenyl)methyl)azetidin e-1-carbonyl)hexahydro-2H-pyrido[4,3-b][1,4]oxazin-3(4H)-one | 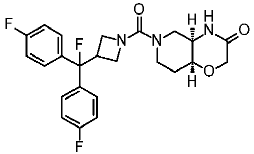 | 0.419^{[b]} |
| **56** | (4aR,8aS)-6-(4-(1-(4-Fluorophenyl)-2-(2,2,2-trifluoroethoxy)ethyl)piperidi ne-1-carbonyl)hexahydro-2H-pyrido[4,3-b][1,4]oxazin-3(4H)-one | 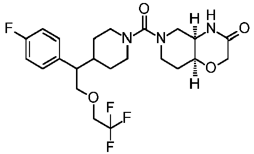 | 0.021^{[b]} |
| **57** | (4aR,8aS)-6-(4-((R or S)-(4-Fluorophenyl)(2,2,2-trifluoroethoxy)methyl)piperidine-1-carbonyl)hexahydro-2H-pyrido[4,3-b][1,4]oxazin-3(4H)-one | 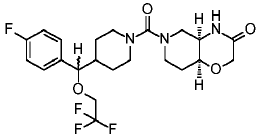 | 0.029^{[b]} |
| **58** | (4aR,8aS)-6-(4-((S or R)-(4-Fluorophenyl)(2,2,2-trifluoroethoxy)methyl)piperi dine-1-carbonyl)hexahydro-2H-pyrido[4,3-b][1,4]oxazin-3(4H)-one | 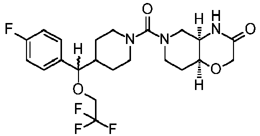 | 0.007^{[b]} |
| **59** | (4aR,8aS)-6-(4-((R or S)-1-(4-Fluorophenyl)-2-(3-methyl-1,2,4-oxadiazol-5-yl)ethyl)piperidine-1-carbonyl)hexahydro-2H-pyrido[4,3-b][1,4]oxazin-3(4H)-one | 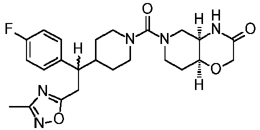 | 0.060^{[b]} |
| **60** | (4aR,8aS)-6-(4-((S or R)-1-(4-Fluorophenyl)-2-(3-methyl-1,2,4-oxadiazol-5-yl)ethyl)piperidine-1-carbonyl)hexahydro-2H-pyrido[4,3-b][1,4]oxazin-3(4H)-one | 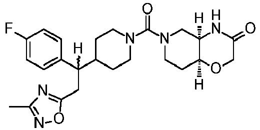 | 0.067^{[b]} |
| **61** | (4aR,8aS)-6-(4-(2-Cyclopropylpyridin-4-yl)piperidine-1-carbonyl)hexahydro-2H-pyrido[4,3-b][1,4]oxazin-3 (4H)-one | 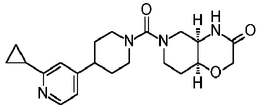 | 0.569^{[b]} |
| **62** | (4aR,8aS)-6-[4-[(S or R)-(4-Fluorophenyl)-(3-methoxyphenyl)methyl]piper idine-1-carbonyl]-4,4a,5,7,8,8a-hexahydropyrido [4,3-b] [1,4]oxazin-3-one | 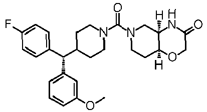 | 0.0004^{[b]} |
| **63** | (4aR,8aS)-6-(4-((R or S)-(4-Fluorophenyl) (3-methoxyphenyl)methyl)piper idine-1-carbonyl)hexahydro-2H-pyrido[4,3-b][1,4]oxazin-3(4H)-one | 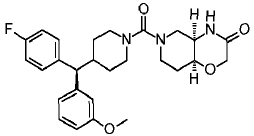 | 0.0003^{[b]} |
| **64** | (4aR,8aS)-6-(4-((S or R)-(3-Methoxyphenyl)(phenyl)met hyl)piperidine-1-carbonyl)hexahydro-2H-pyrido[4,3-b][1,4]oxazin-3(4H)-one | 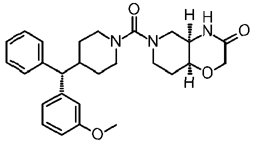 | 0.0002^{[b]} |
| **65** | (4aR,8aS)-6-(4-((R or S)-(3-Methoxyphenyl)(phenyl)met hyl)piperidine-1-carbonyl)hexahydro-2H-pyrido[4,3-b][1,4]oxazin-3(4H)-one | 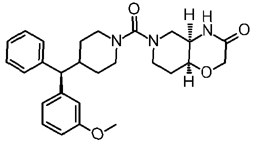 | 0.0002^{[b]} |
| **66** | (4aR,8aS)-6-(4-((S or R)-(3-(2-Fluoroethoxy)phenyl)(phenyl )methyl)piperidine-1-carbonyl)hexahydro-2H-pyrido[4,3-b][1,4]oxazin-3(4H)-one | 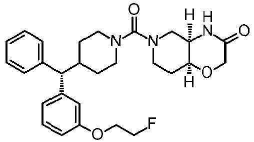 | 0.0004^{[b]} |
| **67** | (4aR,8aS)-6-(4-((S)-(4-Fluorophenyl)(phenyl)methyl )piperidine-1-carbonyl)hexahydro-2H-pyrido[4,3-b][1,4]oxazin-3(4H)-one | 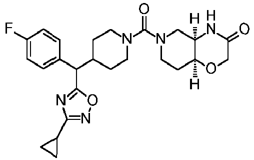 | 0.0039^{[b]} |
| **68** | (4aR,8aS)-6-(4-((S or R)-(4-Fluorophenyl)(phenyl)methyl )piperidine-1-carbonyl)hexahydro-2H-pyrido[4,3-b][1,4]oxazin-3(4H)-one | 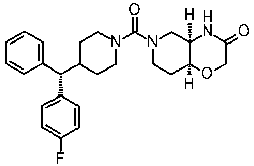 | 0.0002^{[b]} |
| **69** | (4aR,8aS)-6-[4-[(S or R)-(4-Fluorophenyl)-(4-methylphenyl)methyl]piperid ine-1-carbonyl]-4,4a,5,7,8,8a-hexahydropyrido [4,3-b][1,4]oxazin-3-one | 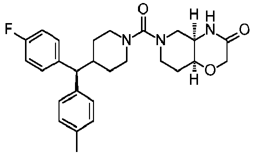 | 0.0001^{[b]} |
| **70** | (4aR,8aS)-6-[4-[(R or S)-[4-(2-Fluoroethoxy)phenyl] - phenylmethyl]piperidine-1-carbonyl]-4,4a,5,7,8,8a-hexahydropyrido [4,3-b][1,4]oxazin-3-one | 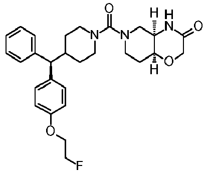 | 0.004^{[b]} |
| **71** | (4aR,8aS)-6-(4-((S or R)-(4-Fluorophenyl) (4-methoxyphenyl)methyl)piper idine-1 -carbonyl)hexahydro-2H-pyrido[4,3-b][1,4]oxazin-3(4H)-one | 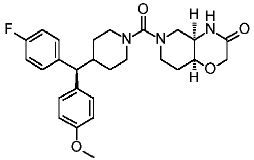 | 0.0002^{[b]} |
| **72** | (4aR,8aS)-6-(4-((R or S)-(4-Fluorophenyl) (p-tolyl)methyl)piperidine-1-carbonyl)hexahydro-2H-pyrido[4,3-b][1,4]oxazin-3(4H)-one | 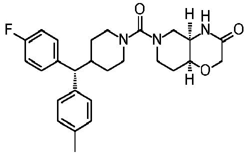 | 0.0001^{[b]} |
| **73** | (4aR,8aS)-6-(4-((S or R)-(3,4-Dimethoxyphenyl)(4-fluorophenyl)methyl)piperidi ne-1-carbonyl)hexahydro-2H-pyrido[4,3-b][1,4]oxazin-3(4H)-one | 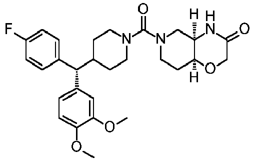 | 0.0004^{[b]} |
| **74** | (4aR,8aS)-6-(4-((R or S)-(3,4-Dimethoxyphenyl)(phenyl)m ethyl)piperidine-1-carbonyl)hexahydro-2H-pyrido[4,3-b][1,4]oxazin-3(4H)-one | 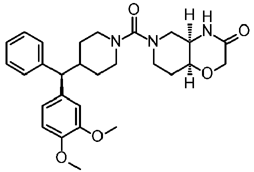 | 0.002^{[b]} |
| **75** | (4aR,8aS)-6-(3-(1-(2-Chloro-4-(trifluoromethyl)phenoxy)eth yl)azetidine-1-carbonyl)hexahydro-2H-pyrido[4,3-b][1,4]oxazin-3(4H)-one | 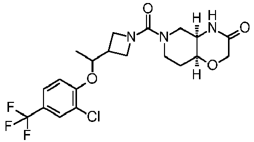 | 0.028^{[b]} |
| **76** | (4aR,8aS)-6-(3-(1-(2-Chloro-4-(trifluoromethyl)phenoxy)eth yl)azetidine-1-carbonyl)hexahydro-2H-pyrido[4,3-b][1,4]oxazin-3(4H)-one | 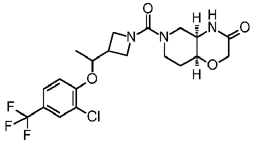 | 0.001^{[b]} |
| **77** | (4aR,8aS)-6-(4-((R or S)-(4-Fluorophenyl)(phenyl)methyl )piperidine-1-carbonyl)hexahydro-2H-pyrido[4,3-b][1,4]oxazin-3(4H)-one | 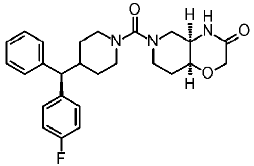 | 0.5^{[a]} |
| **78** | (4aR,8aS)-6-(4-((S or R)-Phenyl(m-tolyl)methyl)piperidine-1-carbonyl)hexahydro-2H-pyrido[4,3-b][1,4]oxazin-3(4H)-one | 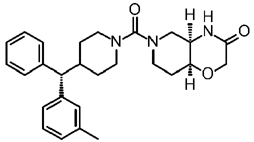 | 0.3^{[a]} |
| **79** | (4aR,8aS)-6-[4-[(S or R)-[4-(2-Fluoroethoxy)phenyl] - phenylmethyl]piperidine-1-carbonyl]-4,4a,5,7,8,8a-hexahydropyrido [4,3-b][1,4]oxazin-3-one | 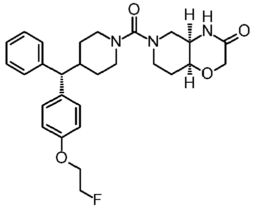 | 0.0002^{[b]} |
| **80** | (4aR,8aS)-6-(4-((S or R)-((S)-3-Methylcyclohexa-2,4-dien-1-yl) (phenyl)methyl)piperidine -1-carbonyl)hexahydro-2H-pyrido[4,3-b][1,4]oxazin-3(4H)-one | 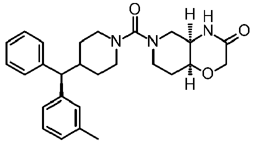 | 0.0004^{[a]} |
| **81** | (4aR,8aS)-6-(4-(1-(2-Chloro-4-fluorophenoxy)-2,2,2-trifluoroethyl)piperidine-1-carbonyl)hexahydro-2H-pyrido[4,3-b][1,4]oxazin-3(4H)-one | 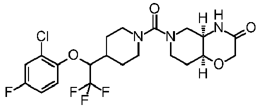 | 0.003^{[b]} |
| **82** | (4aR,8aS)-6-(3-(bis(4-Fluorophenyl)methyl) azetidi ne-1-carbonyl)hexahydro-2H-pyrido[4,3-b][1,4]oxazin-3(4H)-one | 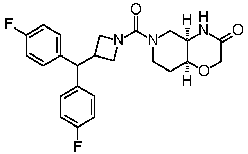 | 1.2^{[a]} |
| **83** | (4aR,8aS)-6-[3-[1-[4-(Trifluoromethyl)phenyl]ethy 1]azetidine-1-carbonyl]-4,4a,5,7,8,8a-hexahydropyrido[4,3-b][1,4]oxazin-3-one | 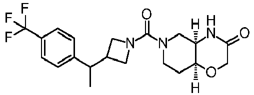 | 0.114^{[b]} |
| **84** | (4aR,8aS)-6-[4-[(S or R)-Phenyl(p-tolyl)methyl]piperidine-1-carbonyl]-4,4a,5,7,8,8a-hexahydropyrido[4,3-b][1,4]oxazin-3-one | 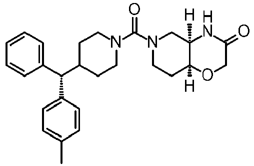 | 0.0001^{[b]} |
| **85** | (4aR,8aS)-6-(4-((R or S)-Phenyl(p-tolyl)methyl)piperidine-1-carbonyl)hexahydro-2H-pyrido[4,3-b][1,4]oxazin-3(4H)-one | 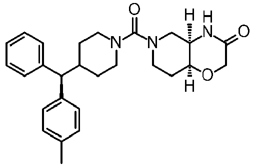 | 0.0001^{[b]} |
| **86** | (4aR,8aS)-6-(3-Benzhydrylazetidine-1-carbonyl)hexahydro-2H-pyrido[4,3-b][1,4]oxazin-3(4H)-one | 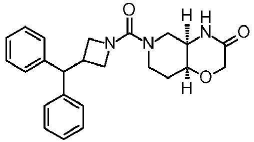 | 0.155^{[b]} |
| **87** | (4aR,8aS)-6-(4-((S or R)-(3,4-Dimethoxyphenyl)(phenyl)m ethyl)piperidine-1-carbonyl)hexahydro-2H-pyrido[4,3-b][1,4]oxazin-3(4H)-one | 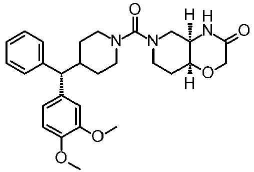 | 0.0004^{[b]} |
| **88** | (4aR,8aS)-6-(4-((R or S)-(3,4-Dimethoxyphenyl)(4-fluorophenyl)methyl)piperidi ne-1-carbonyl)hexahydro-2H-pyrido[4,3-b][1,4]oxazin-3(4H)-one | 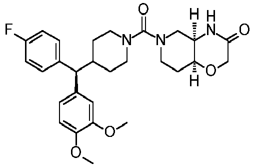 | 0.0022^{[b]} |
| **89** | (4aR,8aS)-6-(4-((R or S)-(4-Fluorophenyl)(4-methoxyphenyl)methyl)piper idine-1-carbonyl)hexahydro-2H-pyrido[4,3-b][1,4]oxazin-3(4H)-one | 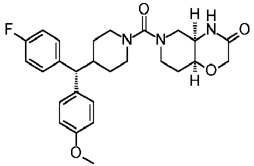 | 0.0006^{[b]} |
| **90** | (4aR,8aS)-6-[3-[1-[4-(Trifluoromethyl)phenyl]etho xy]azetidine-1-carbonyl]-4,4a,5,7,8,8a-hexahydropyrido[4,3-b][1,4]oxazin-3-one | 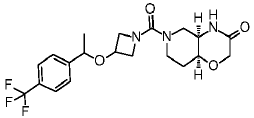 | 0.049^{[b]} |
| **91** | (4aR,8aS)-6-(3-(1-(2-Fluoro-4-(trifluoromethyl)phenoxy)eth yl)azetidine-1-carbonyl)hexahydro-2H-pyrido[4,3-b][1,4]oxazin-3(4H)-one | 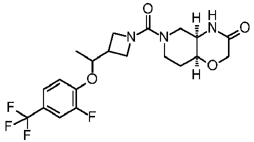 | 0.015^{[b]} |
| **92** | (4aR,8aS)-6-(3-(1-(4-(Trifluoromethyl)phenoxy)et hyl)azetidine-1-carbonyl)hexahydro-2H-pyrido[4,3-b][1,4]oxazin-3(4H)-one | 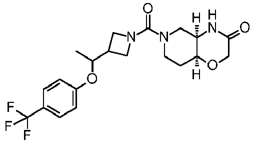 | 0.020^{[b]} |
| **93** | (4aR,8aS)-6-[4-[1-[4-(Trifluoromethyl)phenyl]etho xy]piperidine-1-carbonyl]-4,4a,5,7,8,8a-hexahydropyrido[4,3-b][1,4]oxazin-3-one | 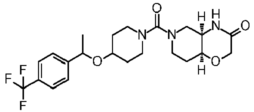 | 0.073^{[b]} |
| **94** | (4aR,8aS)-6-(4-((R or S)-(3-Cyclopropyl-1,2,4-oxadiazol-5-yl)(4-fluorophenyl)methyl)piperidi ne-1-carbonyl)hexahydro-2H-pyrido[4,3-b][1,4]oxazin-3(4H)-one | 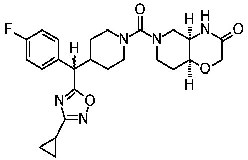 | 0.002^{[b]} |
| **95** | (4aR,8aS)-6-(4-((S or R)-(3-Cyclopropyl-1,2,4-oxadiazol-5-yl)(4-fluorophenyl)methyl)piperidi ne-1-carbonyl)hexahydro-2H-pyrido[4,3-b][1,4]oxazin-3(4H)-one | 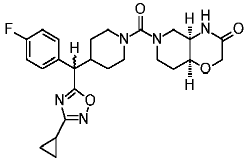 | 0.016^{[b]} |
| **96** | (4aR,8aS)-6-(3-((R or S)-1-(2-Fluoro-4-(trifluoromethyl)phenoxy)eth yl)azetidine-1-carbonyl)hexahydro-2H-pyrido[4,3-b][1,4]oxazin-3(4H)-one | 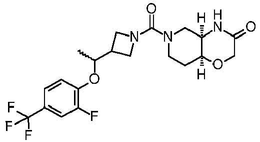 | 0.044^{[b]} |
| **97** | (4aR,8aS)-6-(3-((S or R)-1-(2-Fluoro-4-(trifluoromethyl)phenoxy)eth yl)azetidine-1-carbonyl)hexahydro-2H-pyrido[4,3-b][1,4]oxazin-3(4H)-one | 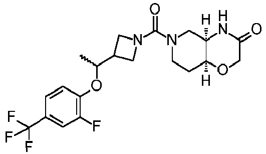 | 0.016^{[b]} |
| **98** | (4aR,8aS)-6-(3-((R or S)-1-(4-(Trifluoromethyl)phenoxy)et hyl)azetidine-1-carbonyl)hexahydro-2H-pyrido[4,3-b][1,4]oxazin-3(4H)-one | 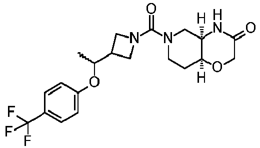 | 0.062^{[b]} |
| **99** | (4aR,8aS)-6-(3-((S or R)-1-(4-(Trifluoromethyl)phenoxy)et hyl)azetidine-1-carbonyl)hexahydro-2H-pyrido [4,3-b][1,4]oxazin-3(4H)-one | 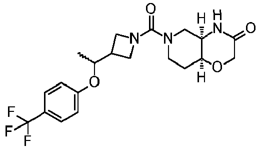 | 0.027^{[b]} |
| **100** | (4aR,8aS)-6-(4-((R or S)-(4-(2-Fluoroethoxy)phenyl)(4-fluorophenyl)methyl)piperidi ne-1-carbonyl)hexahydro-2H-pyrido[4,3-b][1,4]oxazin-3(4H)-one | 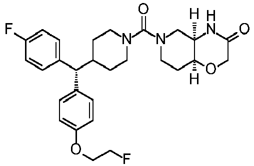 | 0.004^{[b]} |
| **101** | (4aR,8aS)-6-(4-(5-Methyl-6-(trifluoromethyl)pyridin-3-yl)piperidine-1-carbonyl)hexahydro-2H-pyrido[4,3-b][1,4]oxazin-3(4H)-one | 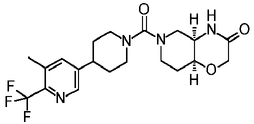 | 0.219^{[b]} |
| **102** | (4aR,8aS)-6-(4-((4-Fluorophenyl)(3-(trifluoromethyl)- 1,2,4-oxadiazol-5-yl)methyl)piperidine-1-carbonyl)hexahydro-2H-pyrido[4,3-b][1,4]oxazin-3(4H)-one | 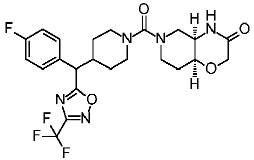 | 0.005^{[b]} |
| **103** | (4aR,8aS)-6-(3-((S or R)-1-(4-(Trifluoromethyl)phenyl)ethy l)azetidine-1-carbonyl)hexahydro-2H-pyrido[4,3-b][1,4]oxazin-3(4H)-one | 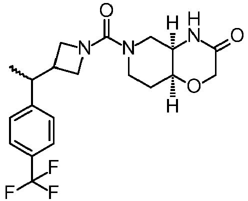 | 0.099^{[b]} |
| **104** | (4aR,8aS)-6-(3-((R or S)-1-(4-(Trifluoromethyl)phenyl)ethy l)azetidine-1-carbonyl)hexahydro-2H-pyrido[4,3-b][1,4]oxazin-3(4H)-one | 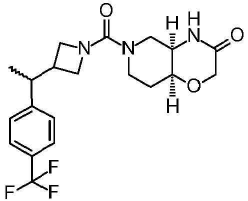 | 0.111^{[b]} |
| **105** | (4aR,8aS)-6-[3-(S or R)-[(4-Fluorophenyl)-(2,2,2-trifluoroethoxy)methyl]azetid ine-1-carbonyl]-4,4a,5,7,8,8a-hexahydropyrido[4,3-b][1,4]oxazin-3-one | 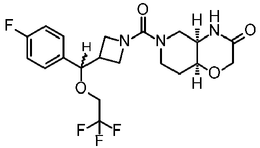 | 0.434^{[b]} |
| **106** | (4aR,8aS)-6-[3-(R or S)-[(4-Fluorophenyl)-(2,2,2-trifluoroethoxy)methyl]azetid ine-1-carbonyl]-4,4a,5,7,8,8a-hexahydropyrido[4,3-b][1,4]oxazin-3-one | 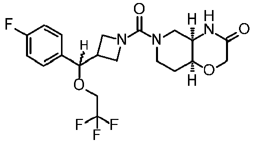 | 0.318^{[b]} |
| **107** | (4aR,8aS)-6-(4-(5,6,7,8-Tetrahydroquinolin-4-yl)piperidine-1-carbonyl)hexahydro-2H-pyrido[4,3-b][1,4]oxazin-3(4H)-one | 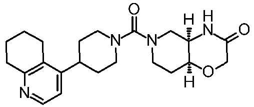 | 0.576^{[b]} |
| **108** | (4aR,8aS)-6-(4-((R or S)-(3-(2-Fluoroethoxy)phenyl)(phenyl )methyl)piperidine-1-carbonyl)hexahydro-2H-pyrido[4,3-b][1,4]oxazin-3(4H)-one | 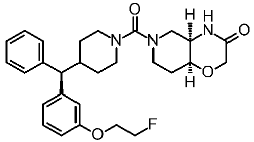 | 0.0004^{[b]} |
| **109** | (4aR,8aS)-6-(4-((S or R)-(4-(2-Fluoroethoxy)phenyl)(4-fluorophenyl)methyl)piperidi ne-1-carbonyl)hexahydro-2H-pyrido[4,3-b][1,4]oxazin-3(4H)-one | 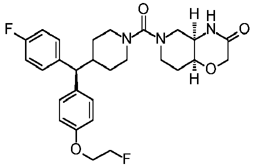 | 0.0004^{[b]} |
| **110** | (4aR,8aS)-6-(3-(4-Bromophenyl)azetidine-1-carbonyl)hexahydro-2H-pyrido[4,3-b][1,4]oxazin-3(4H)-one | 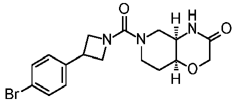 | 0.029^{[b]} |
| **111** | (4aR,8aS)-6-(3-(4'-Chloro-[1,1'-biphenyl]-4-yl)azetidine-1-carbonyl)hexahydro-2H-pyrido[4,3-b][1,4]oxazin-3(4H)-one | 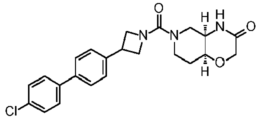 | 0.0004^{[b]} |
| **112** | (4aR,8aS)-6-(3-(2'-Chloro-[1,1'-biphenyl]-4-yl)azetidine-1-carbonyl)hexahydro-2H-pyrido[4,3-b][1,4]oxazin-3(4H)-one | 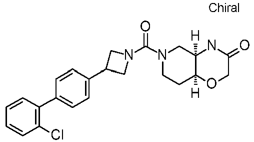 | 0.0001^{[b]} |
| **113** | (4aR,8aS)-6-(3-(2',4'-Dichloro-[1,1'-biphenyl]-4-yl)azetidine-1-carbonyl)hexahydro-2H-pyrido[4,3-b][1,4]oxazin-3(4H)-one | 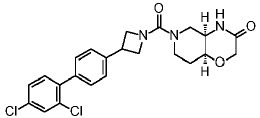 | 0.00004^{[b]} |
| **114** | (4aR,8aS)-6-(3-(1-(R or S)-(4-(T rifluoromethyl)phenyl)ethox y)azetidine-1-carbonyl)hexahydro-2H-pyrido[4,3-b][1,4]oxazin-3(4H)-one | 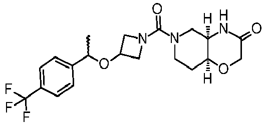 | 0.049^{[b]} |
| **115** | (4aR,8aS)-6-(3-(1-(S or R)-(4-(Trifluoromethyl)phenyl)etho xy)azetidine-1-carbonyl)hexahydro-2H-pyrido[4,3-b][1,4]oxazin-3(4H)-one | 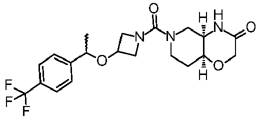 | 0.079^{[b]} |
| **116** | (4aR,8aS)-6-[3-(S or R)-[1-(2-Chloro-4-fluorophenyl)ethoxy]azetidine-1-carbonyl]-4,4a,5,7,8,8a-hexahydropyrido [4,3-b][1,4]oxazin-3-one | 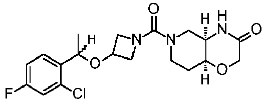 | 0.092^{[b]} |
| **117** | (4aR,8aS)-6-[3-(R or S)-[1-(2-Chloro-4-fluorophenyl)ethoxy]azetidine-1-carbonyl]-4,4a,5,7,8,8a-hexahydropyrido [4,3-b][1,4]oxazin-3-one | 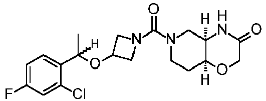 | 0.030^{[b]} |
| **118** | (4aR,8aS)-6-[4-[(R or S)-(3,4-Dimethoxyphenyl)-(2-pyridyl)methyl]piperidine-1-carbonyl]-4,4a,5,7,8,8a-hexahydropyrido [4,3-b][1,4]oxazin-3-one | 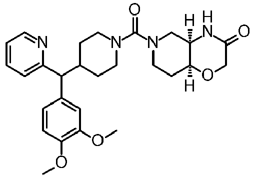 | 0.025^{[b]} |
| **119** | (4aR,8aS)-6-[4-[(S or R)-(3,4-Dimethoxyphenyl)-(2-pyridyl)methyl]piperidine-1-carbonyl]-4,4a,5,7,8,8a-hexahydropyrido [4,3-b][1,4]oxazin-3-one | 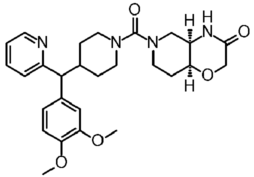 | 0.017^{[b]} |
| **120** | (4aR,8aS)-6-[3-[4-(Trifluoromethoxy)phenyl]az etidine-1-carbonyl]-4,4a,5,7,8,8a-hexahydropyrido [4,3-b][1,4]oxazin-3-one | 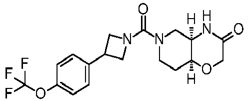 | 0.007^{[b]} |
| **121** | (4aR,8aS)-6-(3-(2'-(Trifluoromethoxy)-[1,1'-biphenyl]-4-yl)azetidine-1-carbonyl)hexahydro-2H-pyrido[4,3-b][1,4]oxazin-3(4H)-one | 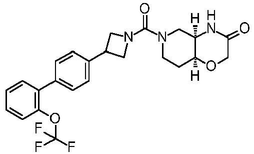 | 0.00003^{[b]} |
| **122** | (4aR,8aS)-6-(3-(4-Bromophenyl)-3-fluoroazetidine-1-carbonyl)hexahydro-2H-pyrido[4,3-b][1,4]oxazin-3(4H)-one | 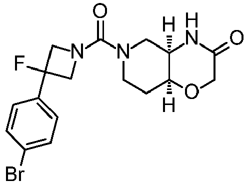 | 0.090^{[b]} |
| **123** | (4aR,8aS)-6-(3-(4-Bromophenyl)-3-hydroxyazetidine-1-carbonyl)hexahydro-2H-pyrido[4,3-b][1,4]oxazin-3(4H)-one | 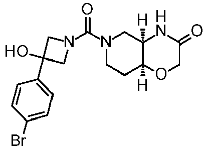 | 0.226^{[b]} |
| **124** | (4aR,8aS)-6-(3-(4-Bromophenyl)-3-methylazetidine-1-carbonyl)hexahydro-2H-pyrido[4,3-b][1,4]oxazin-3(4H)-one | 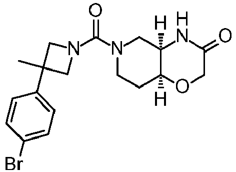 | 0.113^{[b]} |
| **125** | (4aR,8aS)-6-(3-(2'-(Trifluoromethyl)-[1,1'-biphenyl]-4-yl)azetidine-1-carbonyl)hexahydro-2H-pyrido[4,3-b][1,4]oxazin-3(4H)-one | 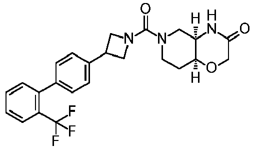 | 0.00004^{[b]} |
| **126** | (4aR,8aS)-6-(3-(2',4'-Difluoro-[1,1'-biphenyl]-4-yl)azetidine-1-carbonyl)hexahydro-2H-pyrido[4,3-b][1,4]oxazin-3(4H)-one | 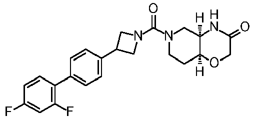 | 0.0003^{[b]} |
| **127** | 4'-(1-((4aR,8aS)-3-Oxooctahydro-2H-pyrido[4,3-b][1,4]oxazine-6-carbonyl)azetidin-3-yl)-[1,1'-biphenyl]-2-carbonitrile | 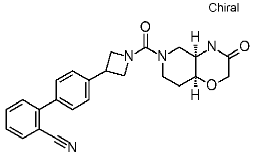 | 0.0005^{[b]} |
| **128** | (4aR,8aS)-6-(3-(4-(3-Methoxyazetidin-1-yl)phenyl)azetidine-1-carbonyl)hexahydro-2H-pyrido[4,3-b][1,4]oxazin-3(4H)-one | 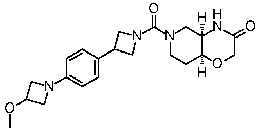 | 0.045^{[b]} |
| **129** | (4aR,8aS)-6-(3-(4-(3-(Trifluoromethyl)azetidin-1 - yl)phenyl)azetidine-1-carbonyl)hexahydro-2H-pyrido[4,3-b][1,4]oxazin-3(4H)-one | 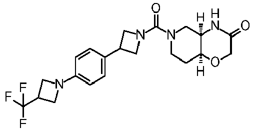 | 0.002^{[b]} |
| **130** | 2-[4-[1-[(4aR,8aS)-3-Oxo-4,4a,5,7,8,8a-hexahydropyrido [4,3-b][1,4]oxazine-6-carbonyl]azetidin-3-yl]phenyl]benzamide | 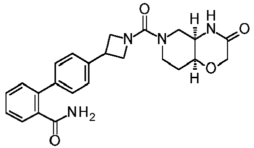 | 0.004^{[b]} |
| **131** | (4aR,8aS)-6-(3-(2-Chloro-[1,1'-biphenyl]-4-yl)azetidine-1-carbonyl)hexahydro-2H-pyrido[4,3-b][1,4]oxazin-3(4H)-one | 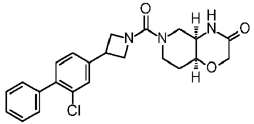 | 0.0004^{[b]} |
| **132** | (4aR,8aS)-6-(3-(4-Bromo-3-chlorophenyl)azetidine-1-carbonyl)hexahydro-2H-pyrido[4,3-b][1,4]oxazin-3(4H)-one | 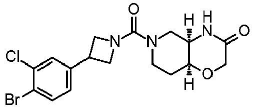 | 0.010^{[b]} |
| **133** | (4aR,8aS)-6-[3-[4-(4-Chloro-2-fluorophenyl)phenyl]azetidine-1-carbonyl]-4,4a,5,7,8,8a-hexahydropyrido [4,3-b][1,4]oxazin-3-one | 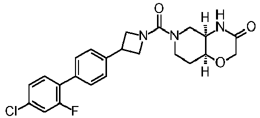 | 0.0002^{[b]} |
| **134** | (4aR,8aS)-6-[3-[4-(2-Chloro-4-fluorophenyl)phenyl]azetidine-1-carbonyl]-4,4a,5,7,8,8a-hexahydropyrido [4,3-b][1,4]oxazin-3-one | 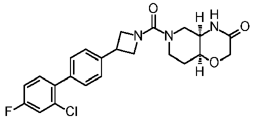 | 0.0001^{[b]} |
| **135** | (4aR,8aS)-6-[4-[(R or S)-(3,4-Dimethoxyphenyl)-(3-pyridyl)methyl]piperidine-1 - carbonyl]-4,4a,5,7,8,8a-hexahydropyrido [4,3-b][1,4]oxazin-3-one | 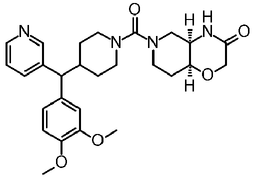 | 0.007^{[b]} |
| **136** | (4aR,8aS)-6-[4-[(S or R)-(3,4-dimethoxyphenyl)-(3-pyridyl)methyl]piperidine-1 - carbonyl]-4,4a,5,7,8,8a-hexahydropyrido [4,3-b][1,4]oxazin-3-one | 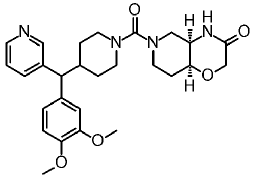 | 0.014^{[b]} |
| **137** | (4aR,8aS)-6-(3-(2'-Methyl-[1,1'-biphenyl]-4-yl)azetidine-1-carbonyl)hexahydro-2H-pyrido[4,3-b][1,4]oxazin-3(4H)-one | 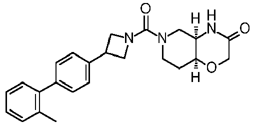 | 0.0001^{[b]} |
| **138** | (4aR,8aS)-6-(3-(4-(3-((1,1,1-Trifluoropropan-2-yl)oxy)azetidin-1-yl)phenyl)azetidine-1-carbonyl)hexahydro-2H-pyrido[4,3-b][1,4]oxazin-3(4H)-one | 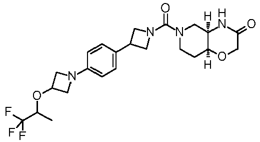 | 0.001^{[b]} |
| **139** | (4aR,8aS)-6-(3-(3-Bromophenyl)azetidine-1-carbonyl)hexahydro-2H-pyrido[4,3-b][1,4]oxazin-3(4H)-one | 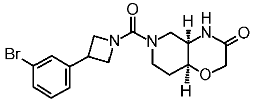 | 0.027^{[b]} |
| **140** | (4aR,8aS)-6-(3-(4-(tert-Butyl)phenyl)azetidine-1-carbonyl)hexahydro-2H-pyrido[4,3-b][1,4]oxazin-3(4H)-one | 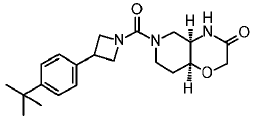 | 0.0006^{[b]} |
| **141** | (4aR,8aS)-6-[3-(4-Phenylphenyl)azetidine-1-carbonyl]-4,4a,5,7,8,8a-hexahydropyrido[4,3-b][1,4]oxazin-3-one | 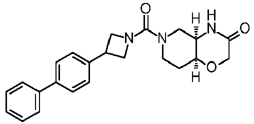 | 0.002^{[b]} |
| **142** | (4aR,8aS)-6-[3-[4-[2-(Difluoromethyl)phenyl]phen yl]azetidine-1-carbonyl]-4,4a,5,7,8,8a-hexahydropyrido [4,3-b][1,4]oxazin-3-one | 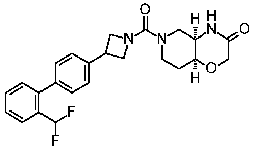 | 0.0001^{[b]} |
| **143** | (4aR,8aS)-6-(3-(3'-Methyl-[1,1'-biphenyl]-4-yl)azetidine-1-carbonyl)hexahydro-2H-pyrido[4,3-b][1,4]oxazin-3(4H)-one | 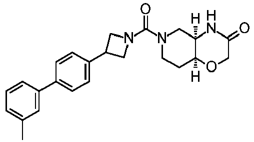 | 0.001^{[b]} |
| **144** | (4aR,8aS)-6-(3-(4'-Methyl-[1,1'-biphenyl]-4-yl)azetidine-1-carbonyl)hexahydro-2H-pyrido[4,3-b][1,4]oxazin-3(4H)-one | 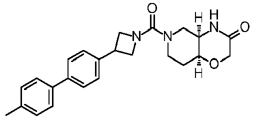 | 0.001^{[b]} |
| **145** | (4aR,8aS)-6-(3-(6-Chloropyridin-3-yl)azetidine-1-carbonyl)hexahydro-2H-pyrido[4,3-b][1,4]oxazin-3(4H)-one | 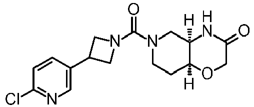 | 4.3^{[b]} |
| **146** | (4aR,8aS)-6-(3-(4-(Trifluoromethyl)phenyl)azet idine-1-carbonyl)hexahydro-2H-pyrido[4,3-b][1,4]oxazin-3(4H)-one | 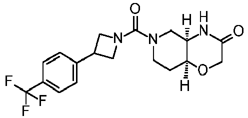 | 0.034^{[b]} |
| **147** | (4aR,8 aS)-6-(3-(4-(3-((1,1,1-Trifluoro-2-methylpropan-2-yl)oxy)azetidin-1-yl)phenyl)azetidine-1-carbonyl)hexahydro-2H-pyrido[4,3-b][1,4]oxazin-3(4H)-one | 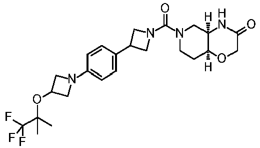 | 0.001^{[b]} |
| **148** | (4aR,8aS)-6-(3-(4-(1,1-Difluoroethyl)phenyl)azetidi ne-1-carbonyl)hexahydro-2H-pyrido[4,3-b][1,4]oxazin-3(4H)-one | 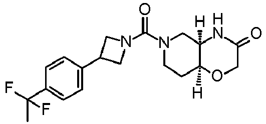 | 0.014^{[b]} |
| **149** | (4aR,8aS)-6-[4-[(R or S)-(3,4-Dimethoxyphenyl)-(4-pyridyl)methyl]piperidine-1-carbonyl]-4,4a,5,7,8,8a-hexahydropyrido[4,3-b][1,4]oxazin-3-one | 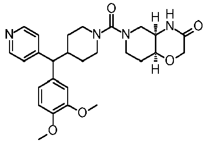 | 0.051^{[b]} |
| **150** | (4aR,8aS)-6-[4-[(S or R)-(3,4-Dimethoxyphenyl)-(4-pyridyl)methyl]piperidine-1-carbonyl]-4,4a,5,7,8,8a-hexahydropyrido[4,3-b][1,4]oxazin-3-one | 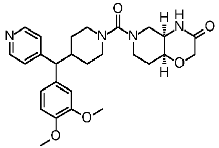 | 0.008^{[b]} |
| **151** | (4aR,8aS)-6-(3-(4-(3-Methylazetidin-1-yl)phenyl)azetidine-1-carbonyl)hexahydro-2H-pyrido[4,3-b][1,4]oxazin-3(4H)-one | 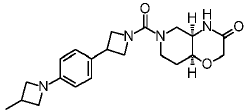 | 0.005^{[b]} |
| **152** | (4aR,8aS)-6-(3-(4-(3,3-Dimethylpyrrolidin-1-yl)phenyl)azetidine-1-carbonyl)hexahydro-2H-pyrido[4,3-b][1,4]oxazin-3(4H)-one | 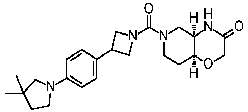 | 0.0004^{[b]} |
| **153** | (4aR,8aS)-6-(3-(4-(3-(Trifluoromethoxy)azetidin-1-yl)phenyl)azetidine-1-carbonyl)hexahydro-2H-pyrido[4,3-b][1,4]oxazin-3(4H)-one | 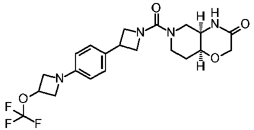 | 0.002^{[b]} |
| **154** | (4aR,8aS)-6-(3-(6-(2,4-Dichlorophenyl)pyridin-3-yl)azetidine-1-carbonyl)hexahydro-2H-pyrido[4,3-b][1,4]oxazin-3(4H)-one | 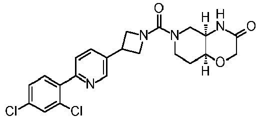 | 0.001^{[b]} |
| **155** | (4aR,8aS)-6-(3-(2'-Chloro-4'-(methylsulfonyl)-[1,1'-biphenyl]-4-yl)azetidine-1-carbonyl)hexahydro-2H-pyrido[4,3-b][1,4]oxazin-3(4H)-one | 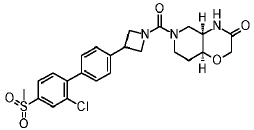 | 0.0008^{[b]} |
| **156** | (4aR,8aS)-6-(3-(4-(3,3-Difluoroazetidin-1-yl)phenyl)azetidine-1-carbonyl)hexahydro-2H-pyrido[4,3-b][1,4]oxazin-3(4H)-one | 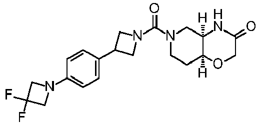 | 0.024^{[b]} |
| **157** | (4aR,8aS)-6-(4-(4-Bromophenyl)piperidine-1-carbonyl)hexahydro-2H-pyrido[4,3-b][1,4]oxazin-3(4H)-one | 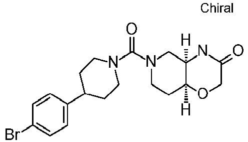 | 0.289^{[b]} |
| **158** | (4aR,8aS)-6-[3-[4-(2,2,2-Trifluoroethoxy)phenyl] azeti dine-1-carbonyl]-4,4a,5,7,8,8a-hexahydropyrido [4,3-b][1,4]oxazin-3-one | 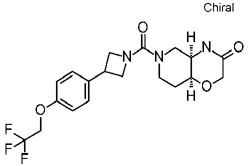 | 0.007^{[b]} |
| **159** | 2-[4-[1-[(4aR,8aS)-3-Oxo-4,4a,5,7,8,8a-hexahydropyrido [4,3-b][1,4]oxazine-6-carbonyl]azetidin-3-yl]phenyl]-5-chloro-benzonitrile | 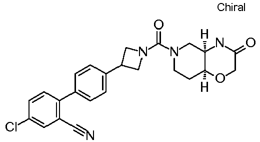 | 0.0001^{[b]} |
| **160** | (4aR,8aS)-6-[3-[4-[3-[(1S or 1R)-2,2,2-Trifluoro-1-methyl-ethoxy]azetidin-1-yl]phenyl]azetidine-1-carbonyl]-4,4a,5,7,8,8a-hexahydropyrido [4,3-b][1,4]oxazin-3-one | 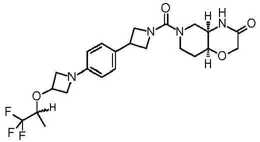 | 0.001^{[b]} |
| **161** | (4aR,8aS)-6-[3-[4-[3-[(1R or 1S)-2,2,2-Trifluoro-1-methyl-ethoxy]azetidin-1-yl]phenyl]azetidine-1-carbonyl]-4,4a,5,7,8,8a-hexahydropyrido [4,3-b][1,4]oxazin-3-one | 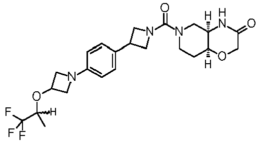 | 0.004^{[b]} |
| **162** | (4aR,8aS)-6-(3-(4-(2-(Trifluoromethyl)pyrrolidin-1-yl)phenyl)azetidine-1-carbonyl)hexahydro-2H-pyrido[4,3-b][1,4]oxazin-3(4H)-one | 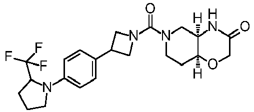 | 0.0003^{[b]} |
| **163** | (4aR,8aS)-6-(3-(4-(3-(Trifluoromethyl)pyrrolidin-1-yl)phenyl)azetidine-1-carbonyl)hexahydro-2H-pyrido[4,3-b][1,4]oxazin-3(4H)-one | 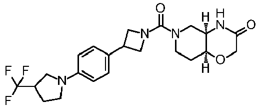 | 0.0007^{[b]} |
| **164** | (4aR,8aS)-6-(3-(3-Bromophenyl)pyrrolidine-1-carbonyl)hexahydro-2H-pyrido[4,3-b][1,4]oxazin-3(4H)-one | 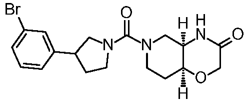 | 0.082^{[b]} |
| **165** | (4aR,8aS)-6-[3-[4-(2-Ethylpyrrolidin-1-yl)phenyl]azetidine-1-carbonyl]-4,4a,5,7,8,8a-hexahydropyrido[4,3-b][1,4]oxazin-3-one | 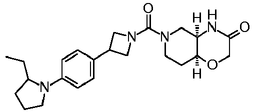 | 0.0004^{[b]} |
| **166** | 2-[4-[1-[(4aR,8aS)-3-Oxo-4,4a,5,7,8,8a-hexahydropyrido[4,3-b][1,4]oxazine-6-carbonyl]azetidin-3-yl]phenyl]-3-chloro-benzonitrile | 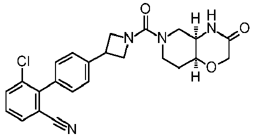 | 0.0001^{[b]} |
| **167** | (4aR,8aS)-6-(3-(4-(2-Cyclopropylpyrrolidin-1-yl)phenyl)azetidine-1-carbonyl)hexahydro-2H-pyrido[4,3-b][1,4]oxazin-3(4H)-one | 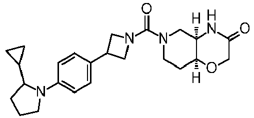 | 0.0002^{[b]} |
| **168** | (4aR,8aS)-6-(4-(4-Bromophenyl)piperazine-1-carbonyl)hexahydro-2H-pyrido[4,3-b][1,4]oxazin-3(4H)-one | 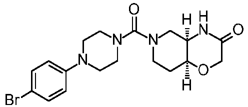 | 3.7^{[b]} |
| **169** | (4aR,8aS)-6-(4-(2',4'-Dichloro-[1,1'-biphenyl]-4-yl)piperidine-1-carbonyl)hexahydro-2H-pyrido[4,3-b][1,4]oxazin-3(4H)-one | 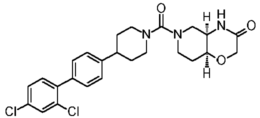 | 0.004^{[b]} |
| **170** | (4aR,8aS)-6-(3-(4-(3-Azabicyclo[3.1.0]hexan-3-yl)phenyl)azetidine-1-carbonyl)hexahydro-2H-pyrido[4,3-b][1,4]oxazin-3(4H)-one | 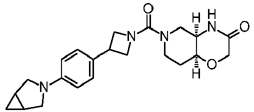 | 0.002^{[b]} |
| **171** | (4aR,8aS)-6-(3-(3-(Trifluoromethoxy)phenyl)az etidine-1-carbonyl)hexahydro-2H-pyrido[4,3-b][1,4]oxazin-3(4H)-one | 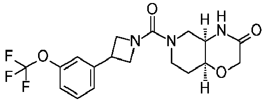 | 0.044^{[b]} |
| **172** | (4aR,8aS)-6-(3-(4-(3-(2,2,2-Trifluoroethoxy)azetidin-1 - yl)phenyl)azetidine-1-carbonyl)hexahydro-2H-pyrido[4,3-b][1,4]oxazin-3(4H)-one | 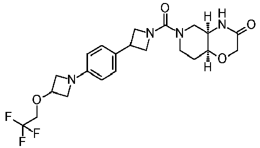 | 0.004^{[b]} |
| **173** | (4aR,8aS)-6-(3-(4'-chloro-2'-(methylsulfonyl)-[1,1'-biphenyl]-4-yl)azetidine-1-carbonyl)hexahydro-2H-pyrido[4,3-b][1,4]oxazin-3(4H)-one | 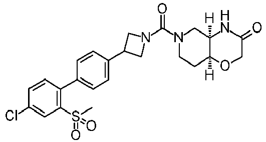 | 0.0004^{[b]} |
| **174** | (4aR,8aS)-6-(3-(6-(2-(Trifluoromethyl)pyrrolidin-1-yl)pyridin-3-yl)azetidine-1-carbonyl)hexahydro-2H-pyrido[4,3-b][1,4]oxazin-3(4H)-one | 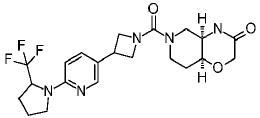 | 0.007^{[b]} |
| **175** | (4aR,8aS)-6-[4-[(R or S)-(3-methylsulfonylphenyl)-(3-pyridyl)methyl]piperidine-1 - carbonyl]-4,4a,5,7,8,8a-hexahydropyrido [4,3-b][1,4]oxazin-3-one | 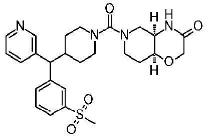 | 0.115^{[b]} |
| **176** | (4aR,8aS)-6-[4-[(S or R)-(3-methylsulfonylphenyl)-(3-pyridyl)methyl]piperidine-1 - carbonyl]-4,4a,5,7,8,8a-hexahydropyrido [4,3-b][1,4]oxazin-3-one | 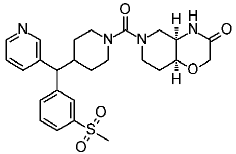 | 0.170^{[b]} |
| **177** | (4aR,8aS)-6-[4-[(R or S)-(3-methylsulfonylphenyl)-(4-pyridyl)methyl]piperidine-1- carbonyl]-4,4a,5,7,8,8a-hexahydropyrido[4,3-b][1,4]oxazin-3-one | 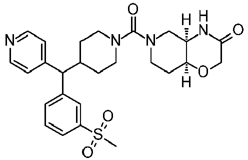 | 0.428^{[b]} |
| **178** | (4aR,8aS)-6-[4-[(S or R)-(3-methylsulfonylphenyl)-(4-pyridyl)methyl]piperidine-1 - carbonyl]-4,4a,5,7,8,8a-hexahydropyrido[4,3-b][1,4]oxazin-3-one | 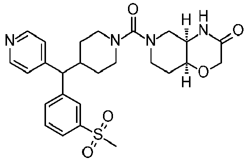 | 0.131^{[b]} |
| **179** | (4aR,8aS)-6-(3-(4-(5-Azaspiro[2.4]heptan-5-yl)phenyl)azetidine-1-carbonyl)hexahydro-2H-pyrido[4,3-b][1,4]oxazin-3(4H)-one | 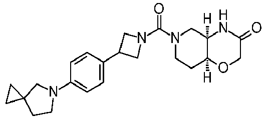 | 0.001^{[b]} |
| **180** | (4aR,8aS)-6-(3-(4-(Pentafluoro-16-sulfaneyl)phenyl)azetidine-1-carbonyl)hexahydro-2H-pyrido[4,3-b][1,4]oxazin-3(4H)-one | 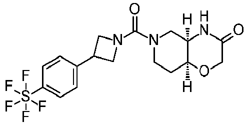 | 0.009^{[b]} |
| **181** | (4aR,8aS)-6-(3-(5-Chloropyridin-2-yl)azetidine-1-carbonyl)hexahydro-2H-pyrido[4,3-b][1,4]oxazin-3(4H)-one | 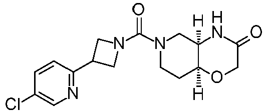 | 0.593^{[b]} |
| **182** | (4aR,8aS)-6-(3-(2-Fluoro-4-(trifluoromethoxy)phenyl)aze tidine-1-carbonyl)hexahydro-2H-pyrido[4,3-b][1,4]oxazin-3(4H)-one | 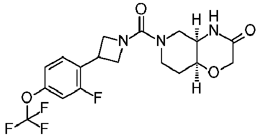 | 0.008^{[b]} |
| **183** | (4aR,8aS)-6-[3-(6-methoxypyridin-3 - yl)azetidine-1-carbonyl]-4,4a,5,7,8,8a-hexahydropyrido [4,3-b][1,4]oxazin-3-one | 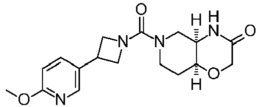 | 1.6^{[b]} |
| **184** | (4aR,8aS)-6-(3-(4-Bromophenyl)pyrrolidine-1-carbonyl)hexahydro-2H-pyrido[4,3-b][1,4]oxazin-3(4H)-one | 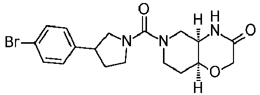 | 0.146^{[b]} |
| **185** | (4aR,8aS)-6-(3-Phenylazetidine-1-carbonyl)hexahydro-2H-pyrido[4,3-b][1,4]oxazin-3(4H)-one | 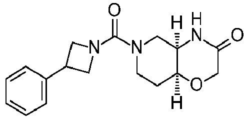 | 0.139^{[b]} |
| **186** | (4aR,8aS)-6-(4-Phenylpiperidine-1-carbonyl)hexahydro-2H-pyrido[4,3-b][1,4]oxazin-3(4H)-one | 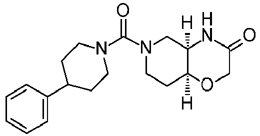 | 1.0^{[b]} |
| **187** | (4aR,8aS)-6-[3-[4-(2,2,2-Trifluoroethyl)phenyl]azetidi ne-1-carbonyl]-4,4a,5,7,8,8a-hexahydropyrido[4,3-b][1,4]oxazin-3-one | 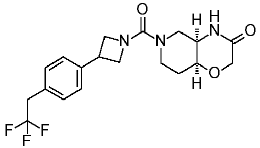 | 0.001^{[b]} |
| **188** | (4aR,8aS)-6-[3-[4-[1-(Trifluoromethyl)cyclopropyl ]phenyl]azetidine-1-carbonyl]-4,4a,5,7,8,8a-hexahydropyrido[4,3-b][1,4]oxazin-3-one | 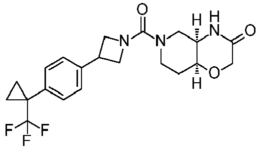 | 0.004^{[b]} |
| **189** | (4aR,8aS)-6-[4-[(R or S)-(3-Methylsulfonylphenyl)-(2-pyridyl)methyl]piperidine-1- carbonyl]-4,4a,5,7,8,8a-hexahydropyrido[4,3-b][1,4]oxazin-3-one | 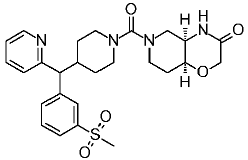 | 0.068^{[b]} |
| **190** | (4aR,8aS)-6-[4-[(S or R)-(3-Methylsulfonylphenyl)-(2-pyridyl)methyl]piperidine-1 - carbonyl]-4,4a,5,7,8,8a-hexahydropyrido[4,3-b][1,4]oxazin-3-one | 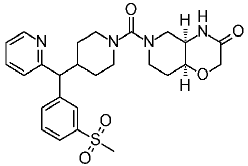 | 0.261^{[b]} |
| **191** | (4aR,8aS)-6-[3-[4-(6,6-Difluoro-2-azaspiro[3.3]heptan-2-yl)phenyl]azetidine-1-carbonyl]-4,4a,5,7,8,8a-hexahydropyrido[4,3-b] [1,4]oxazin-3-one | 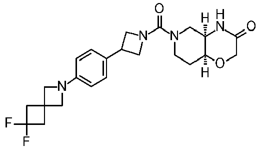 | 0.0005^{[b]} |
| **192** | (4aR,8aS)-6-(3-(4-(8-Oxa-3-azabicyclo[3.2.1]octan-3-yl)phenyl)azetidine-1-carbonyl)hexahydro-2H-pyrido[4,3-b][1,4]oxazin-3(4H)-one | 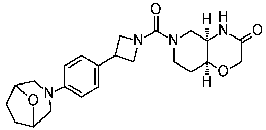 | 0.008^{[b]} |
| **193** | (4aR,8aS)-6-(3-(5-(2,4-Dichlorophenyl)pyridin-2-yl)azetidine-1-carbonyl)hexahydro-2H-pyrido[4,3-b][1,4]oxazin-3(4H)-one | 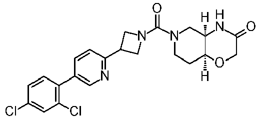 | 0.001^{[b]} |
| **194** | (4aR,8aS)-6-(3-(4-((R or S)-2-(Trifluoromethyl)pyrrolidin-1-yl)phenyl)azetidine-1-carbonyl)hexahydro-2H-pyrido[4,3-b][1,4]oxazin-3(4H)-one | 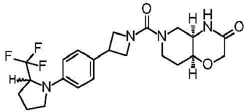 | 0.0001^{[b]} |
| **195** | (4aR,8aS)-6-(3-(4-((S or R)-2-(Trifluoromethyl)pyrrolidin-1-yl)phenyl)azetidine-1-carbonyl)hexahydro-2H-pyrido[4,3-b][1,4]oxazin-3(4H)-one | 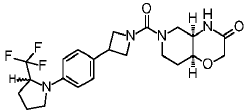 | 0.0004^{[b]} |
| **196** | (4aR,8aS)-6-[3-[4-(1-Piperidyl)phenyl]azetidine-1-carbonyl]-4,4a,5,7,8,8a-hexahydropyrido[4,3-b][1,4]oxazin-3-one | 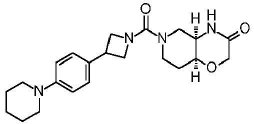 | 0.002^{[b]} |
| **197** | (4aR,8aS)-6-(3-(4-((R or S)-3-(Trifluoromethyl)pyrrolidin-1-yl)phenyl)azetidine-1-carbonyl)hexahydro-2H-pyrido[4,3-b][1,4]oxazin-3(4H)-one | 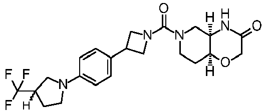 | 0.0005^{[b]} |
| **198** | (4aR,8aS)-6-(3-(4-((S or R)-3-(Trifluoromethyl)pyrrolidin-1-yl)phenyl)azetidine-1-carbonyl)hexahydro-2H-pyrido[4,3-b][1,4]oxazin-3(4H)-one | 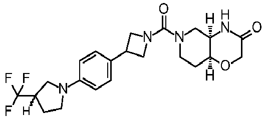 | 0.001^{[b]} |
| **199** | (4aR,8aS)-6-[3-[4-(3-hydroxy-3-methylazetidin-1-yl)phenyl]azetidine-1-carbonyl]-4,4a,5,7,8,8a-hexahydropyrido[4,3-b][1,4]oxazin-3-one | 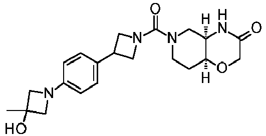 | 0.278^{[b]} |
| **200** | (4aR,8aS)-6-[3-[4-(3-Fluoro-3-methyl-azetidin-1-yl)phenyl]azetidine-1-carbonyl]-4,4a,5,7,8,8a-hexahydropyrido[4,3-b][1,4]oxazin-3-one | 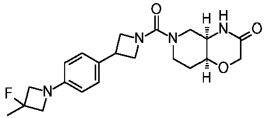 | 0.014^{[b]} |
| **201** | (4aR,8aS)-6-(3-(4-(3-Fluoroazetidin-1-yl)phenyl)azetidine-1-carbonyl)hexahydro-2H-pyrido[4,3-b][1,4]oxazin-3(4H)-one | 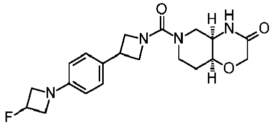 | 0.101^{[b]} |
| **202** | (4aR,8aS)-6-(3-(3-Fluoro-4-(trifluoromethoxy)phenyl)aze tidine-1-carbonyl)hexahydro-2H-pyrido[4,3-b][1,4]oxazin-3(4H)-one | 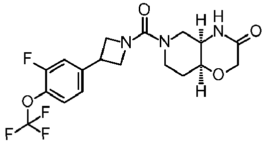 | 0.012^{[b]} |
| **203** | (4aR,8aS)-6-(3-(3-Methyl-4-(trifluoromethoxy)phenyl)aze tidine-1-carbonyl)hexahydro-2H-pyrido[4,3-b][1,4]oxazin-3(4H)-one | 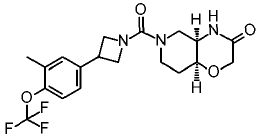 | 0.0008^{[b]} |
| **204** | (4aR,8aS)-6-(3-(3,5-Difluoro-4-(trifluoromethoxy)phenyl)aze tidine-1-carbonyl)hexahydro-2H-pyrido[4,3-b][1,4]oxazin-3(4H)-one | 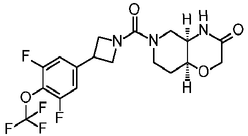 | 0.01^{[b]} |
| **205** | (4aR,8aS)-6-(3-(2-Chloro-4-(trifluoromethoxy)phenyl)aze tidine-1-carbonyl)hexahydro-2H-pyrido[4,3-b][1,4]oxazin-3(4H)-one | 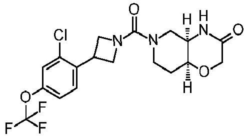 | 0.018^{[b]} |
| **206** | (4aR,8aS)-6-(3-(4-(Bicyclo[1.1.1]pentan-1-yl)phenyl)azetidine-1-carbonyl)hexahydro-2H-pyrido[4,3-b][1,4]oxazin-3(4H)-one | 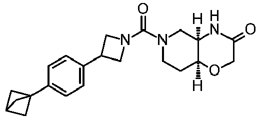 | 0.005^{[b]} |
| **207** | (4aR,8aS)-6-(3-(5-(2-(Trifluoromethyl)pyrrolidin-1-yl)pyridin-2-yl)azetidine-1-carbonyl)hexahydro-2H-pyrido[4,3-b][1,4]oxazin-3(4H)-one | 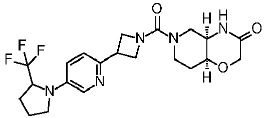 | 0.006^{[b]} |
| **208** | (4aR,8aS)-6-(3-(5-Fluoro-1H-indol-3-yl)pyrrolidine-1-carbonyl)hexahydro-2H-pyrido[4,3-b][1,4]oxazin-3(4H)-one | 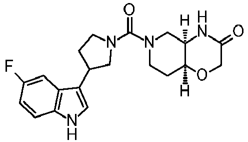 | 0.048^{[b]} |
| **209** | (4aR,8aS)-6-(3-(1-Benzyl-5-chloro-1H-indol-3-yl)pyrrolidine-1-carbonyl)hexahydro-2H-pyrido[4,3-b][1,4]oxazin-3(4H)-one | 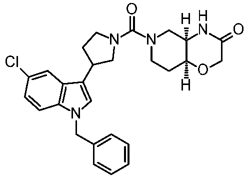 | 0.0008^{[b]} |
| **210** | (4aR,8aS)-6-(3-(2-Fluoro-4-(trifluoromethyl)phenyl)azeti dine-1-carbonyl)hexahydro-2H-pyrido[4,3-b][1,4]oxazin-3(4H)-one | 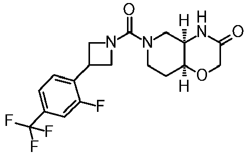 | 0.112^{[b]} |
| **211** | (4aR,8aS)-6-(3-(3-Chloro-4-(trifluoromethoxy)phenyl)aze tidine-1-carbonyl)hexahydro-2H-pyrido[4,3-b][1,4]oxazin-3(4H)-one | 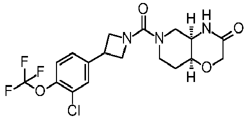 | 0.011^{[b]} |
| **212** | (4aR,8aS)-6-[3-[4-[3-(Cyclopropylmethoxy) azetidi n-1-yl]phenyl]azetidine-1-carbonyl]-4,4a,5,7,8,8a-hexahydropyrido[4,3-b][1,4]oxazin-3-one | 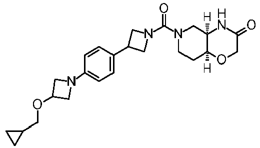 | 0.003^{[b]} |
| **213** | 5-(1-((4aR,8aS)-3-Oxooctahydro-2H-pyrido[4,3-b][1,4]oxazine-6-carbonyl)azetidin-3-yl)-2-(trifluoromethoxy)benzonitril e | 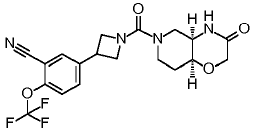 | 0.114^{[b]} |
| **214** | (4aR,8aS)-6-(3-(4-(1-(Hydroxymethyl)cyclopropyl )phenyl)azetidine-1-carbonyl)hexahydro-2H-pyrido[4,3-b][1,4]oxazin-3(4H)-one | 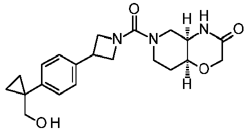 | 0.062^{[b]} |
| **215** | (4aR,8aS)-6-(3-(1-Methyl-1H-indazol-4-yl)azetidine-1-carbonyl)hexahydro-2H-pyrido[4,3-b][1,4]oxazin-3(4H)-one | 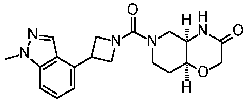 | 0.550^{[b]} |
| **216** | (4aR,8aS)-6-(3-(4-(3-Fluoropyrrolidin-1-yl)phenyl)azetidine-1-carbonyl)hexahydro-2H-pyrido[4,3-b][1,4]oxazin-3(4H)-one | 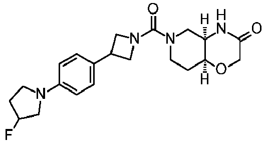 | 0.023^{[b]} |
| **217** | (4aR,8aS)-6-(3-(4-(2-Methylazetidin-1-yl)phenyl)azetidine-1-carbonyl)hexahydro-2H-pyrido[4,3-b][1,4]oxazin-3(4H)-one | 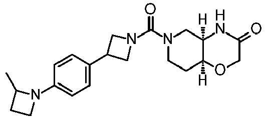 | 0.006^{[b]} |
| **218** | (4aR,8aS)-6-[4-[phenyl(pyridazin-3-yl)methyl]piperidine-1-carbonyl]-4,4a,5,7,8,8a-hexahydropyrido[4,3-b][1,4]oxazin-3-one | 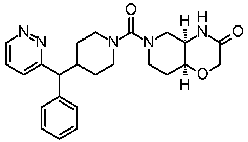 | 0.054^{[b]} |
| **219** | (4aR,8aS)-6-[3-[4-(Trifluoromethoxy)phenyl]py rrolidine-1-carbonyl]-4,4a,5,7,8,8a-hexahydropyrido[4,3-b] [1,4]oxazin-3-one | 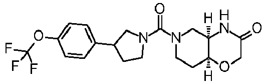 | 0.276^{[b]} |
| **220** | (4aR,8aS)-6-(3-(1-Methyl-1H-indazol-6-yl)azetidine-1-carbonyl)hexahydro-2H-pyrido[4,3-b][1,4]oxazin-3(4H)-one | 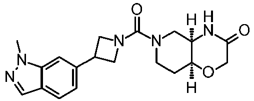 | 0.413^{[b]} |
| **221** | (4aR,8aS)-6-[3-(S or R)-[3-(Trifluoromethoxy)phenyl]py rrolidine-1-carbonyl]-4,4a,5,7,8,8a-hexahydropyrido[4,3-b][1,4]oxazin-3-one | 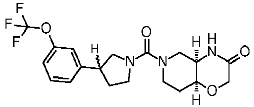 | 0.178^{[b]} |
| **222** | (4aR,8aS)-6-[3-(R or S)-[3-(Trifluoromethoxy)phenyl]py rrolidine-1-carbonyl]-4,4a,5,7,8,8a-hexahydropyrido[4,3-b][1,4]oxazin-3-one | 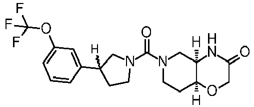 | 0.114^{[b]} |
| **223** | (4aR,8aS)-6-(3-(3-Chloro-4-hydroxyphenyl)azetidine-1-carbonyl)hexahydro-2H-pyrido[4,3-b][1,4]oxazin-3(4H)-one | 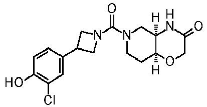 | 0.188^{[b]} |
| **224** | (4aR,8aS)-6-(3-(3-Chloro-4-(3-methylazetidin-1-yl)phenyl)azetidine-1-carbonyl)hexahydro-2H-pyrido[4,3-b][1,4]oxazin-3(4H)-one | 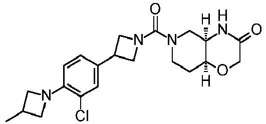 | 0.006^{[b]} |
| **225** | (4aR,8aS)-6-[3-[4-(Oxetan-3-yl)phenyl]azetidine-1-carbonyl]-4,4a,5,7,8,8a-hexahydropyrido[4,3-b][1,4]oxazin-3-one | 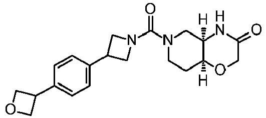 | 0.100^{[b]} |
| **226** | (4aR,8aS)-6-(3-(3-Chloro-4-(3,3-difluoroazetidin-1-yl)phenyl)azetidine-1-carbonyl)hexahydro-2H-pyrido[4,3-b][1,4]oxazin-3(4H)-one | 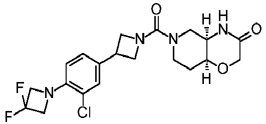 | 0.019^{[b]} |
| **227** | (4aR,8aS)-6-(3-(3-(3-Methylazetidin-1-yl)-4-(trifluoromethoxy)phenyl)aze tidine-1-carbonyl)hexahydro-2H-pyrido[4,3-b][1,4]oxazin-3(4H)-one | 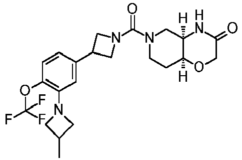 | 0.014^{[b]} |
| **228** | (4aR,8aS)-6-[(3S or R)-3-(3-Bromophenyl)pyrrolidine-1-carbonyl]-4,4a,5,7,8,8a-hexahydropyrido[4,3-b][1,4]oxazin-3-one | 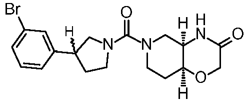 | 0.162^{[b]} |
| **229** | (4aR,8aS)-6-[(3R or S)-3-(3-Bromophenyl)pyrrolidine-1-carbonyl]-4,4a,5,7,8,8a-hexahydropyrido[4,3-b][1,4]oxazin-3-one | 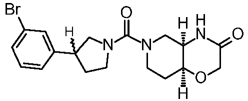 | 0.229^{[b]} |
| **230** | (4aR,8aS)-6-[3-[4-(3-Azabicyclo[3.1.1]heptan-3-yl)phenyl]azetidine-1-carbonyl]-4,4a,5,7,8,8a-hexahydropyrido[4,3-b][1,4]oxazin-3-one | 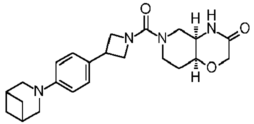 | 0.0002^{[b]} |
| **231** | (4aR,8aS)-6-(2-Methyl-3-(4-(trifluoromethoxy)phenyl)aze tidine-1-carbonyl)hexahydro-2H-pyrido[4,3-b][1,4]oxazin-3(4H)-one | 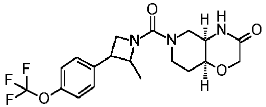 | 0.114^{[b]} |
| **232** | (4aR,8aS)-6-(3-(3,3-Dimethyl-2,3-dihydrobenzofuran-6-yl)azetidine-1-carbonyl)hexahydro-2H-pyrido[4,3-b][1,4]oxazin-3(4H)-one | 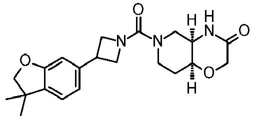 | 0.007^{[b]} |
| **233** | (4aR,8aS)-6-(3-(3-Chloro-5-(2,2,2-trifluoroethoxy)phenyl)pyrrol idine-1-carbonyl)hexahydro-2H-pyrido[4,3-b][1,4]oxazin-3(4H)-one | 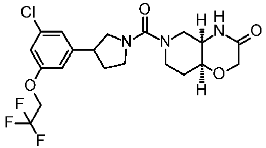 | 0.061^{[b]} |
| **234** | Methyl 2-methyl-2-(4-(1-((4aR,8aS)-3-Oxooctahydro-2H-pyrido[4,3-b][1,4]oxazine-6-carbonyl)azetidin-3-yl)phenyl)propanoate | 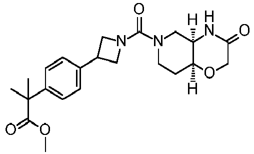 | 0.002^{[b]} |
| **235** | (4aR,8aS)-6-(3-(3,5-Dichlorophenyl)pyrrolidine-1-carbonyl)hexahydro-2H-pyrido[4,3-b][1,4]oxazin-3(4H)-one | 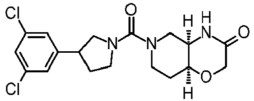 | 0.243^{[b]} |
| **236** | (4aR,8aS)-6-(3-(4-(3-Methoxy-3-methylazetidin-1-yl)phenyl)azetidine-1-carbonyl)hexahydro-2H-pyrido[4,3-b][1,4]oxazin-3(4H)-one | 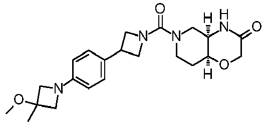 | 0.015^{[b]} |
| **237** | tert-Butyl (2-(3-((1-((4aR,8aS)-3-oxooctahydro-2H-pyrido[4,3-b][1,4]oxazine-6-carbonyl)piperidin-4-yl)(phenyl)methyl)phenoxy)e thyl)carbamate | 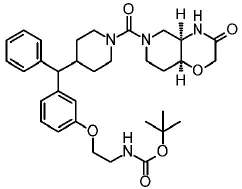 | 0.001^{[b]} |
| **238** | (4aR,8aS)-6-((R or S)-3-(3-Chloro-5-(2,2,2-trifluoroethoxy)phenyl)pyrrol idine-1-carbonyl)hexahydro-2H-pyrido[4,3-b][1,4]oxazin-3(4H)-one | 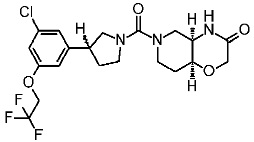 | 0.156^{[b]} |
| **239** | (4aR,8aS)-6-((S or R)-3-(3-Chloro-5-(2,2,2-trifluoroethoxy)phenyl)pyrrolidine-1-carbonyl)hexahydro-2H-pyrido[4,3-b][1,4]oxazin-3(4H)-one | 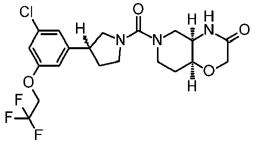 | 0.039^{[b]} |
| **240** | (4aR,8aS)-6-(3-(4-(tert-Butyl)-3-methoxyphenyl)azetidine-1-carbonyl)hexahydro-2H-pyrido[4,3-b][1,4]oxazin-3(4H)-one | 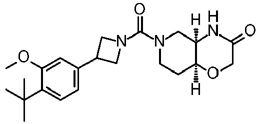 | 0.002^{[b]} |
| **241** | (4aR,8aS)-6-(3-(1-Methyl-1H-indazol-5-yl)azetidine-1-carbonyl)hexahydro-2H-pyrido[4,3-b][1,4]oxazin-3(4H)-one | 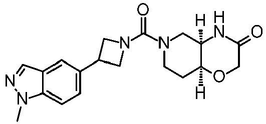 | 0.245^{[b]} |
| **242** | (4aR,8aS)-6-(3-(4-Propylphenyl)azetidine-1-carbonyl)hexahydro-2H-pyrido[4,3-b][1,4]oxazin-3(4H)-one | 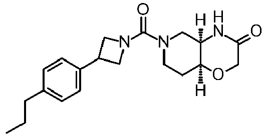 | 0.002^{[b]} |
| **243** | (4aR,8aS)-6-(3-(4-(Trifluoromethoxy)-3-(trifluoromethyl)phenyl)azeti dine-1-carbonyl)hexahydro-2H-pyrido[4,3-b][1,4]oxazin-3(4H)-one | 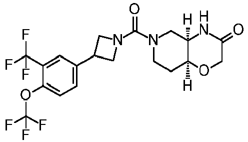 | 0.010^{[b]} |
| **244** | (4aR,8aS)-6-[3-[4-[[1-(Trifluoromethyl)cyclopropyl ]methoxy]phenyl]azetidine-1-carbonyl]-4,4a,5,7,8,8a-hexahydropyrido[4,3-b] [1,4]oxazin-3-one | 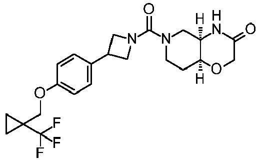 | 0.0003^{[b]} |
| **245** | (4aR,8aS)-6-[3-[4-(2,2,2-Trifluoro-1,1-dimethylethyl)phenyl]azetidine-1-carbonyl]-4,4a,5,7,8,8a-hexahydropyrido[4,3-b][1,4]oxazin-3-one | 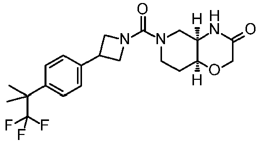 | 0.0004^{[b]} |
| **246** | (4aR,8aS)-6-[3-(R or S)-[4-(2,2,2-Trifluoro-1-methyl-ethoxy)phenyl]azetidine-1-carbonyl]-4,4a,5,7,8,8a-hexahydropyrido[4,3-b][1,4]oxazin-3-one | 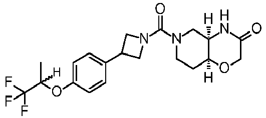 | 0.001^{[b]} |
| **247** | (4aR,8aS)-6-[3-[4-(3-Fluoropropyl)phenyl]azetidin e-1-carbonyl]-4,4a,5,7,8,8a-hexahydropyrido[4,3-b][1,4]oxazin-3-one | 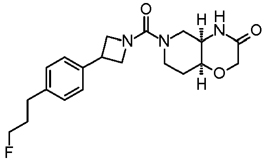 | 0.006^{[b]} |
| **248** | (4aR,8aS)-6-[3-(S or R)-[4-(2,2,2-Trifluoro-1-methyl-ethoxy)phenyl]azetidine-1-carbonyl]-4,4a,5,7,8,8a-hexahydropyrido[4,3-b][1,4]oxazin-3-one | 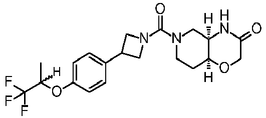 | 0.001^{[b]} |
| **249** | (4aR,8aS)-6-[3-(4-Cyclobutylphenyl)azetidine-1-carbonyl]-4,4a,5,7,8,8a-hexahydropyrido[4,3-b][1,4]oxazin-3-one | 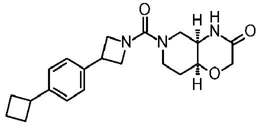 | 0.001^{[b]} |
| **250** | (4aR,8aS)-6-[3-(3-Methoxy-4-methyl-phenyl)azetidine-1-carbonyl]-4,4a,5,7,8,8a-hexahydropyrido[4,3-b][1,4]oxazin-3-one | 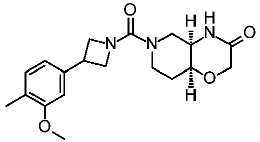 | 0.106^{[b]} |
| **251** | (4aR,8aS)-6-(4-((4-Fluorophenyl)((1-methyl-5-(trifluoromethyl)-1H-pyrazol-3-yl)oxy)methyl)piperidine-1-carbonyl)hexahydro-2H-pyrido[4,3-b][1,4]oxazin-3(4H)-one | 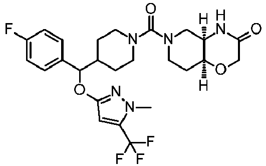 | 0.001^{[b]} |
| **252** | (4aR,8aS)-6-[4-[[3-(2-Aminoethoxy)phenyl]-phenyl-methyl]piperidine-1-carbonyl]-4,4a,5,7,8,8a-hexahydropyrido[4,3-b][1,4]oxazin-3-one | 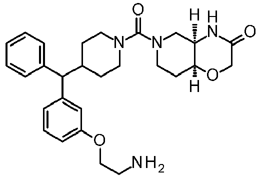 | 0.002^{[b]} |
| **253** | (4aR,8aS)-6-[3-[5-(2,4-Dichlorophenyl)-1,3,4-oxadiazol-2-yl]azetidine-1-carbonyl]-4,4a,5,7,8,8a-hexahydropyrido [4,3-b][1,4]oxazin-3-one | 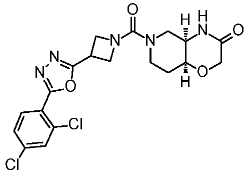 | 0.004^{[b]} |
| **254** | (4aR,8aS)-6-[3-[3-Fluoro-4-(trifluoromethyl)phenyl]azeti dine-1-carbonyl]-4,4a,5,7,8,8a-hexahydropyrido[4,3-b][1,4]oxazin-3-one | 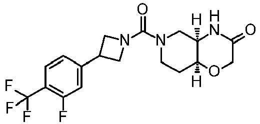 | 0.082^{[b]} |
| **255** | tert-Butyl N-[2-[3-[2-[3-[[1-[(4aR,8aS)-3-oxo-4,4a,5,7,8,8a-hexahydropyrido[4,3-b][1,4]oxazine-6-carbonyl]-4-piperidyl]-phenylmethyl]phenoxy]ethylamino] -3-oxo-propoxy]ethyl]carbamate | 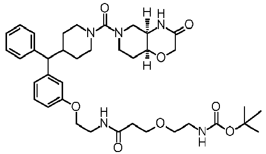 | 0.002^{[b]} |
| **256** | tert-Butyl N-[2-[2-[3-[2-[3-[[1-[(4aR,8aS)-3-oxo-4,4a,5,7,8,8a-hexahydropyrido[4,3-b][1,4]oxazine-6-carbonyl]-4-piperidyl]-phenylmethyl]phenoxy]ethylamino] -3-oxo-propoxy]ethoxy]ethyl]carba mate | 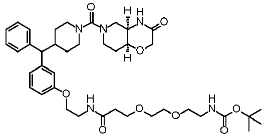 | 0.002^{[b]} |
| **257** | (4aR,8aS)-6-[3-[1-(2,4-Dichlorophenyl)pyrazol-3-yl]azetidine-1-carbonyl]-4,4a,5,7,8,8a-hexahydropyrido[4,3-b][1,4]oxazin-3-one | 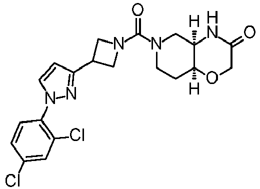 | 0.233^{[b]} |
| **258** | (4aR,8aS)-6-[3-(4-Propoxyphenyl)azetidine-1-carbonyl]-4,4a,5,7,8,8a-hexahydropyrido[4,3-b][1,4]oxazin-3-one | 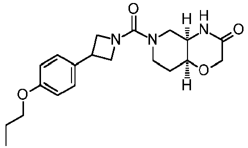 | 0.005^{[b]} |
| **259** | (4aR,8aS)-6-[3-(3,4-Pimethylphenyl)azetidine-1-carbonyl]-4,4a,5,7,8,8a-hexahydropyrido[4,3-b][1,4]oxazin-3-one | 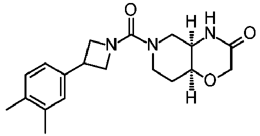 | 0.030^{[b]} |
| **260** | 2-[4-[1-[(4aR,8aS)-3-Oxo-4,4a,5,7,8,8a-hexahydropyrido[4,3-b][1,4]oxazine-6-carbonyl]azetidin-3-yl]phenyl]-N-ethyl-5-methyl-benzamide | 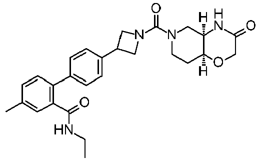 | 0.003^{[b]} |
| **261** | (4aR,8aS)-6-[3-[4-(2,2-Dimethylpropyl)phenyl]azeti dine-1-carbonyl]-4,4a,5,7,8,8a-hexahydropyrido[4,3-b][1,4]oxazin-3-one | 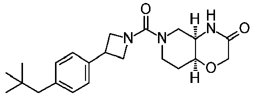 | 0.00007^{[b]} |
| **262** | (4aR,8aS)-6-[3-(4-tert-Butoxyphenyl)azetidine-1-carbonyl]-4,4a,5,7,8,8a-hexahydropyrido[4,3-b][1,4]oxazin-3-one | 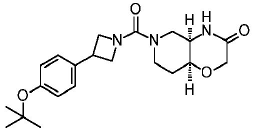 | 0.001^{[b]} |
| **263** | (4aR,8aS)-6-[4-(5-Chloroindolin-1-yl)piperidine-1-carbonyl]-4,4a,5,7,8,8a-hexahydropyrido[4,3-b][1,4]oxazin-3-one | 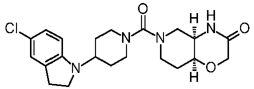 | 0.016^{[b]} |
| **264** | (4aR,8aS)-6-[4-(4-Chloroisoindolin-2-yl)piperidine-1-carbonyl]-4,4a,5,7,8,8a-hexahydropyrido[4,3-b][1,4]oxazin-3-one | 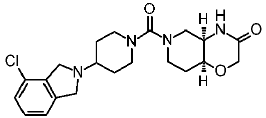 | 0.146^{[b]} |
| **265** | (4aR,8aS)-6-[4-(5'-Chlorospiro[cyclopropane-1,3'-indoline]-1'-yl)piperidine-1-carbonyl]-4,4a,5,7,8,8a-hexahydropyrido[4,3-b][1,4]oxazin-3-one | 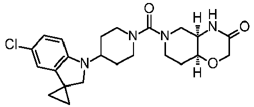 | 0.058^{[b]} |
| **266** | (4aR,8aS)-6-[3-(4-Chloroisoindolin-2-yl)azetidine-1-carbonyl]-4,4a,5,7,8,8a-hexahydropyrido[4,3-b][1,4]oxazin-3-one | 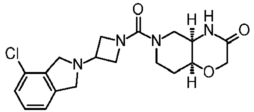 | 0.457^{[b]} |
| **267** | (4aR,8aS)-6-[4-(5-Chloroisoindolin-2-yl)piperidine-1-carbonyl]-4,4a,5,7,8,8a-hexahydropyrido[4,3-b][1,4]oxazin-3-one | 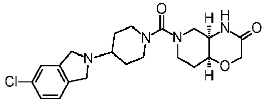 | ND |
| **268** | (4aS,8aS)-6-[4-[1-[2-[tert-Butyl(dimethyl)silyl]oxyethy 1]-5-chloro-indol-3-yl]piperidine-1-carbonyl]-4,4a,5,7,8,8a-hexahydropyrido[4,3-b][1,4]oxazin-3-one | 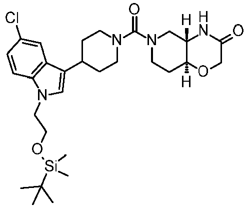 | 0.670^{[b]} |
| **269** | (4aS,8aS)-6-[4-[5-Chloro-1-(2-hydroxyethyl)indol-3-yl]piperidine-1-carbonyl]-4,4a,5,7,8,8a-hexahydropyrido[4,3-b][1,4]oxazin-3-one | 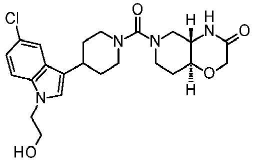 | 0.530^{[b]} |
| **270** | (4aR,8aS)-6-(3-(5-(3-(Trifluoromethyl)pyrrolidin-1-yl)pyridin-2-yl)azetidine-1-carbonyl)hexahydro-2H-pyrido[4,3-b][1,4]oxazin-3(4H)-one | 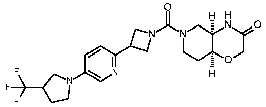 | 0.026^{[b]} |
| **271** | (4aR,8aS)-6-(3-(5-((R or S)-3-(Trifluoromethyl)pyrrolidin-1-yl)pyridin-2-yl)azetidine-1-carbonyl)hexahydro-2H-pyrido[4,3-b][1,4]oxazin-3(4H)-one | 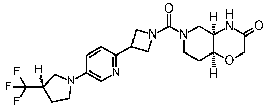 | 0.022^{[b]} |
| **272** | (4aR,8aS)-6-(3-(5-((S or R)-3-(Trifluoromethyl)pyrrolidin-1-yl)pyridin-2-yl)azetidine-1-carbonyl)hexahydro-2H-pyrido[4,3-b][1,4]oxazin-3(4H)-one | 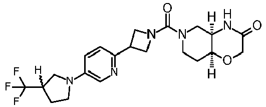 | 0.017^{[b]} |
| **273** | (4aR,8aS)-6-[3-[6-[3-(Trifluoromethyl)pyrrolidin-1-yl]-3-pyridyl]azetidine-1-carbonyl]-4,4a,5,7,8,8a-hexahydropyrido[4,3-b][1,4]oxazin-3-one | 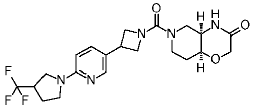 | 0.002^{[b]} |
| **274** | (4aR,8aS)-6-[3-[6-[3-(R or S)-(Trifluoromethyl)pyrrolidin-1-yl]-3-pyridyl]azetidine-1-carbonyl]-4,4a,5,7,8,8a-hexahydropyrido[4,3-b] [1,4]oxazin-3-one | 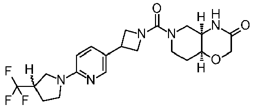 | 0.002^{[b]} |
| **275** | (4aR,8aS)-6-[3-[6-[3S- or 3R-(trifluoromethyl)pyrrolidin-1-yl]-3-pyridyl]azetidine-1-carbonyl]-4,4a,5,7,8,8a-hexahydropyrido[4,3-b][1,4]oxazin-3-one | 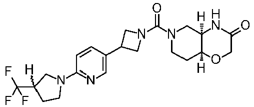 | 0.004^{[b]} |
| **276** | (4aR,8aS)-6-[3-(4-Tetrahydropyran-3-ylphenyl)azetidine-1-carbonyl]-4,4a,5,7,8,8a-hexahydropyrido[4,3-b][1,4]oxazin-3-one | 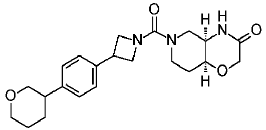 | 0.004^{[b]} |
| **277** | (4aR,8aS)-6-[3-[2-Methoxy-4-(2,2,2-trifluoroethyl)phenyl]azetidin e-1-carbonyl]-4,4a,5,7,8,8a-hexahydropyrido[4,3-b][1,4]oxazin-3-one | 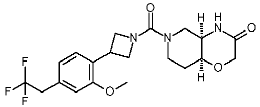 | 0.002^{[b]} |
| **278** | (4aR,8aS)-6-(3-(4-(Neopentyloxy)phenyl)azetid ine-1-carbonyl)hexahydro-2H-pyrido[4,3-b][1,4]oxazin-3(4H)-one | 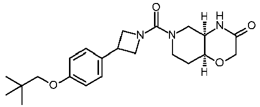 | 0.0001^{[b]} |

| | | | |
|---|---|---|---|
| *[a]: if not indicated otherwise (see [b]), the activity was measured in 4-NPA assay; [b]: measured in 2-AG assay.* | | | |

In one aspect, the present invention provides compounds of formula (Ic) and their pharmaceutically acceptable salts or esters as described herein, wherein said compounds of formula (Ic) and their pharmaceutically acceptable salts or esters have IC_{50'}s for MAGL inhibition below 25 µM, preferably below 10 µM, more preferably below 5 µM as measured in the MAGL assay described herein.

In one embodiment, compounds of formula (Ic) and their pharmaceutically acceptable salts or esters as described herein have IC₅₀ (MAGL inhibition) values between 0.000001 µM and 25 µM, particular compounds have IC₅₀ values between 0.000005 µM and 10 µM, further particular compounds have IC₅₀ values between 0.00005 µM and 5 µM, as measured in the MAGL assay described herein.

In one embodiment, the present invention provides compounds of formula (Ic) and their pharmaceutically acceptable salts or esters as described herein, wherein said compounds of formula (Ic) and their pharmaceutically acceptable salts or esters have an IC₅₀ for MAGL below 25 µM, preferably below 10 µM, more preferably below 5 µM as measured in an assay comprising the steps of:
a) providing a solution of a compound formula (Ic), or a pharmaceutically acceptable salt or ester thereof, in DMSO;
b) providing a solution of MAGL (recombinant wild-type) in assay buffer (50 mM tris(hydroxymethyl)aminomethane; 1 mM ethylenediaminetetraacetic acid);
c) adding 1 µL of compound solution from step a) to 19 µL of MAGL solution from step b);
d) shaking the mixture for 1 min at 2000 rpm;
e) incubating for 15 min at RT;
f) adding 20 µL of a solution of 4-nitrophenlyacetate in assay buffer (50 mM tris(hydroxymethyl)aminomethane; 1 mM ethylenediaminetetraacetic acid, 6% EtOH);
g) shaking the mixture for 1 min at 2000 rpm;
h) incubating for 5 min at RT;
i) measuring the absorbance of the mixture at 405 nm a fist time;
j) incubating a further 80 min at RT;
k) measuring the absorbance of the mixture at 405 nm a second time;
l) substracting the absorbance measured under i) from the absorbance measured under k) and calculating the slope of absorbance;
   wherein:
   i) the concentration of the compound of formula (Ic), or the pharmaceutically acceptable salt or ester thereof in the assay after step f) is in the range of 25 µM to 1.7 nM;
   ii) the concentration of MAGL in the assay after step f) is 1 nM;
   iii) the concentration of 4-nitrophenylacetate in the assay after step f) is 300 µM; and
   iv) steps a) to 1) are repeated for at least 3 times, each time with a different concentration of the compound of formula (Ic), or the pharmaceutically acceptable salt or ester thereof.

### Using the Compounds of the Invention

In one aspect, the present invention provides compounds of formula (Ic) as described herein for use as therapeutically active substance.

In a further aspect, the present invention provides the use of compounds of formula (Ic) as described herein for the treatment or prophylaxis of neuroinflammation, neurodegenerative diseases, pain, cancer and/or mental disorders in a mammal.

In one embodiment, the present invention provides the use of compounds of formula (Ic) as described herein for the treatment or prophylaxis of neuroinflammation and/or neurodegenerative diseases in a mammal.

In one embodiment, the present invention provides the use of compounds of formula (Ic) as described herein for the treatment or prophylaxis of neurodegenerative diseases in a mammal.

In one embodiment, the present invention provides the use of compounds of formula (Ic) as described herein for the treatment or prophylaxis of cancer in a mammal.

In one aspect, the present invention provides the use of compounds of formula (Ic) as described herein for the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

In a preferred embodiment, the present invention provides the use of compounds of formula (Ic) as described herein for the treatment or prophylaxis of multiple sclerosis, Alzheimer's disease and/or Parkinson's disease in a mammal.

In a particularly preferred embodiment, the present invention provides the use of compounds of formula (Ic) as described herein for the treatment or prophylaxis of multiple sclerosis in a mammal.

In one aspect, the present invention provides compounds of formula (Ic) as described herein for use in the treatment or prophylaxis of neuroinflammation, neurodegenerative diseases, pain, cancer and/or mental disorders in a mammal.

In one embodiment, the present invention provides compounds of formula (Ic) as described herein for use in the treatment or prophylaxis of neuroinflammation and/or neurodegenerative diseases in a mammal.

In one embodiment, the present invention provides compounds of formula (Ic) as described herein for use in the treatment or prophylaxis of cancer in a mammal.

In one embodiment, the present invention provides compounds of formula (Ic) as described herein for use in the treatment or prophylaxis of neurodegenerative diseases in a mammal.

In one aspect, the present invention provides compounds of formula (Ic) as described herein for use in the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

In a preferred embodiment, the present invention provides compounds of formula (Ic) as described herein for use in the treatment or prophylaxis of multiple sclerosis, Alzheimer's disease and/or Parkinson's disease in a mammal.

In a particularly preferred embodiment, the present invention provides compounds of formula (Ic) as described herein for use in the treatment or prophylaxis of multiple sclerosis in a mammal.

In one aspect, the present invention provides the use of compounds of formula (Ic) as described herein for the preparation of a medicament for the treatment or prophylaxis of neuroinflammation, neurodegenerative diseases, pain, cancer and/or mental disorders in a mammal.

In one embodiment, the present invention provides the use of compounds of formula (Ic) as described herein for the preparation of a medicament for the treatment or prophylaxis of neuroinflammation and/or neurodegenerative diseases in a mammal.

In one embodiment, the present invention provides the use of compounds of formula (Ic) as described herein for the preparation of a medicament for the treatment or prophylaxis of neurodegenerative diseases in a mammal.

In one embodiment, the present invention provides the use of compounds of formula (Ic) as described herein for the preparation of a medicament for the treatment or prophylaxis of cancer in a mammal.

In a further aspect, the present invention provides the use of compounds of formula (Ic) as described herein for the preparation of a medicament for the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

In a preferred embodiment, the present invention provides the use of compounds of formula (Ic) as described herein for the preparation of a medicament for the treatment or prophylaxis of multiple sclerosis, Alzheimer's disease and/or Parkinson's disease in a mammal.

In a particularly preferred embodiment, the present invention provides the use of compounds of formula (Ic) as described herein for the preparation of a medicament for the treatment or prophylaxis of multiple sclerosis in a mammal.

### Pharmaceutical Compositions and Administration

In one aspect, the present invention provides a pharmaceutical composition comprising a compound of formula (Ic) as described herein and a therapeutically inert carrier.

The compounds of formula (Ic) and their pharmaceutically acceptable salts and esters can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions).

The compounds of formula (Ic) and their pharmaceutically acceptable salts and esters can be processed with pharmaceutically inert, inorganic or organic adjuvants for the production of tablets, coated tablets, dragées and hard gelatin capsules. Lactose, corn starch or derivatives thereof, talc, stearic acid or its salts etc. can be used, for example, as such adjuvants for tablets, dragées and hard gelatin capsules.

Suitable adjuvants for soft gelatin capsules are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

In accordance with the invention, the compounds of formula (Ic) or their pharmaceutically acceptable salts and esters can be used for the treatment or prophylaxis of type 2 diabetes related microvascular complications (such as, but not limited to diabetic retinopathy, diabetic neuropathy and diabetic nephropathy), coronary artery disease, obesity and underlying inflammatory diseases, chronic inflammatory and autoimmune/inflammatory diseases.

### Examples

The invention will be more fully understood by reference to the following examples. The claims should not, however, be construed as limited to the scope of the examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

### Method A1

### Example 1

### rac-(4aR,8aS)-6-(4-Benzhydrylpiperidine-1-carbonyl)-4,4a,5,7,8,8a-hexahydropyrido[4,3-b][1,4]oxazin-3-one

To a solution of 4-nitrophenyl 4-benzhydrylpiperidine-1-carboxylate (40 mg, 96 µmol, BB1) in DMF (1 mL), TEA (19.4 mg, 26.8 µL, 192 µmol), rac-cis-hexahydro-2H-pyrido[4,3-b][1,4]oxazin-3(4H)-one (15 mg, 96 µmol, ChemBridge Corporation) was added and the resultant reaction mixture was heated at 80 °C for 18 hours resulting in reaction completion. The reaction mixture was diluted with H₂O and NaHCO₃ and extracted three times with EtOAc. The combined organic layers were washed with brine, dried over Na₂SO₄, filtered and evaporated. The product was purified by preparative HPLC (Gemini NX column) using a gradient of ACN : H₂O (containing 0.1% FA) (20 : 80 to 98 : 2) to yield the desired compound as a colorless solid (0.015 g; 36.5%). MS (ESI): m/z = 434.2 [M+H]⁺.

### Method A2

### Example 2

### rac-(4aR,8aS)-6-(4-(5-Chloro-1-(cyclopropylmethyl)-1H-indol-3-yl)piperidine-1-carbonyl)hexahydro-2H-pyrido[4,3-b][1,4]oxazin-3(4H)-one

To an ice-cold suspension of bis(trichloromethyl) carbonate (45.3 mg, 153 µmol, CAS RN 32315-10-9) and NaHCO₃ (73.3 mg, 873 µmol) in DCM (1 mL) was added 5-chloro-1-(cyclopropylmethyl)-3-(piperidin-4-yl)-1H-indole acetate formate (76.1 mg, 218 µmol, BB2) in one portion and the mixture was stirred at RT overnight. After cooling down in an-ice bath rac-cis-hexahydro-2H-pyrido[4,3-b][1,4]oxazin-3(4H)-one dihydrochloride (50 mg, 218 µmol, ChemBridge Corporation) and DIPEA (112 mg, 152 µL, 870 µmol) were added. The suspension was stirred at RT for 6 hours. The reaction mixture was poured on H₂O and DCM and the layers were separated. The aqueous layer was extracted twice with DCM. The organic layers were dried over MgSO₄, filtered, treacted with silica gel and evaporated. The compound was purified by silica gel chromatography on a 4 g column using an MPLC system eluting with a gradient of DCM : MeOH (100 : 0 to 90 : 10) to get the desired compound as a colorless gum (0.070 g; 68.1%). MS (ESI): m/z = 471.2 [M+H]⁺.

### Method A3

### Example 22

### (+)- or (-)-cis-6-(4-(5-Chloro-1-(oxetan-3-yl)-1H-indol-3-yl)piperidine-1-carbonyl)hexahydro-2H-pyrido[4,3-bj[1,4]oxazin-3(4H)-one

To an ice-cold solution of bis(trichloromethyl) carbonate (26.6 mg, 89.6 µmol) in DCM (1 mL) were added NaHCO₃ (32.3 mg, 384 µmol) and rac-cis-hexahydro-2H-pyrido[4,3-b][1,4]oxazin-3(4H)-one (20 mg, 128 µmol, ChemBridge Corporation) and the mixture was stirred at RT overnight. The suspension was cooled down to 0°C before 5-chloro-1-(oxetan-3-yl)-3-(piperidin-4-yl)-1H-indole (37.2 mg, 128 µmol, BB10) and DIPEA (49.7 mg, 67.1 µL, 384 µmol) were added. The mixture was stirred at RT for 1 hour. The reaction mixture was poured on water and DCM and the layers were separated. The aqueous layer was extracted twice with DCM. The organic layers were dried over MgSO₄, filtered, treated with silica gel and evaporated. The compound was purified by silica gel chromatography on a 4 g column using an MPLC system eluting with a gradient of DCM : MeOH (100 : 0 to 90 : 10) to get the desired compound as a colorless solid (0.025 g; 41.3%). MS (ESI): m/z = 473.2 [M+H]⁺.

### Method A4

### Example 67

### (4aR,8aS)-6-(4-((3-Cyclopropyl-1,2,4-oxadiazol-5-yl)(4-fluorophenyl)methyl)piperidine-1-carbonyl)hexahydro-2H-pyrido[4,3-b][1,4]oxazin-3(4H)-one

To a suspension of 4-nitrophenyl (4aR,8aS)-3-oxohexahydro-2H-pyrido[4,3-b][1,4]oxazine-6(5H)-carboxylate (50 mg, 156 µmol, BB15a) and DIPEA (50.3 mg, 68 µL, 389 µmol) in ACN (0.7 mL) was added a solution of 3-cyclopropyl-5-((4-fluorophenyl)(piperidin-4-yl)methyl)-1,2,4-oxadiazole hydrochloride (55.2 mg, 163 µmol, BB39) in ACN (0.7 mL) and the solution was stirred at RT overnight. The light yellow solution was stirred at reflux for 23 h and then evaporated. The residue was dissolved in 2M aqueous Na₂CO₃ solution and EtOAc and the layers were separated. The aqueous layer was extracted twice with EtOAc. The organic layers were washed once with water, dried over MgSO₄, filtered, treated with silica gel and evaporated. The compound was purified by silica gel chromatography on a 4 g column using an MPLC system eluting with a gradient of n-heptane : EtOAc/EtOH 3/1 (70 : 30 to 90 : 10) to get the desired compound as a light brown solid (0.022 g; 29.2%). MS (ESI): m/z = 484.2 [M+H]⁺.

### Method A5

### Example 111

### (4aR,8aS)-6-(3-(4'-Chloro-[1,1'-biphenyl]-4-yl)azetidine-1-carbonyl)hexahydro-2H-pyrido[4,3-b][1,4]oxazin-3(4H)-one

A suspension of (4-chlorophenyl)boronic acid (15.9 mg, 101 µmol, CAS RN 1679-18-1), (4aR,8aS)-6-(3-(4-bromophenyl)azetidine-1-carbonyl)hexahydro-2H-pyrido[4,3-b][1,4]oxazin-3(4H)-one (40 mg, 101 µmol, example 110), potassium carbonate (70.1 mg, 507 µmol), water (100 µL) and tetrakis(triphenylphosphine)palladium (0) (5.86 mg, 5.07 µmol, CAS RN 14221-01-3) in THF (1 mL) was stirred at 80°C for 3 h. The reaction mixture was poured on water and EtOAc and the layers were separated. The aqueous layer was extracted twice with EtOAc. The organic layers were dried over MgSO₄, filtered, treated with silica gel and evaporated. The compound was purified by silica gel chromatography on a 4 g column using an MPLC system eluting with a gradient of n-heptane : EtOAc/EtOH (3/1 v/v) (70 : 30 to 10 : 90) to yield the desired compound as a colorless solid (0.020 g; 46.3%). MS (ESI): m/z = 426.16 [M+H]⁺.

### Method A6

### Example 128

### (4aR,8aS)-6-(3-(4-(3-Methoxyazetidin-1-yl)phenyl)azetidine-1-carbonyl)hexahydro-2H-pyrido[4,3-b][1,4]oxazin-3(4H)-one

To a solution of (4aR,8aS)-6-(3-(4-bromophenyl)azetidine-1-carbonyl)hexahydro-2H-pyrido[4,3-b][1,4]oxazin-3(4H)-one (50 mg, 127 µmol, example 110) in tert-butanol (1 mL) under argon were added XPhos (5.44 mg, 11.4 µmol, CAS RN 564483-18-7), tris(dibenzylideneacetone)dipalladium (0) chloroform adduct (3.94 mg, 3.8 µmol, CAS RN 52522-40-4), 3-methoxyazetidine hydrochloride (23.5 mg, 190 µmol, CAS RN 148644-09-1) and cesium carbonate (165 mg, 507 µmol) and the mixture was heated to 85°C for 21 h. The mixture was filtered and the filtrate was evaporated. The product was purified on a preparative HPLC (Gemini NX column) using a gradient of ACN : water (containing 0.1% formic acid) (20: 80 to 98 : 2) to get the desired compound as a colorless solid (0.006 g; 11.8%). MS (ESI): m/z = 401.2 [M+H]⁺. Alternatively, the reaction can also be carried out in a microwave oven at 100°C.

### Method A7

### Example 225

### (4aR,8aS)-6-[3-[4-(Oxetan-3-yl)phenyl]azetidine-1-carbonyl]-4,4a,5,7,8,8a-hexahydropyrido[4,3-b][1,4]oxazin-3-one

To a 5 mL vial equipped with a stirring bar was added (Ir[dF(CF₃)ppy]₂(dtbpy))PF₆ (1.42 mg, 1.27 µmol, CAS RN 870987-63-6 ), 3-bromooxetane (17.4 mg, 127 µmol, CAS RN 39267-79-3), (4aR,8aS)-6-(3-(4-bromophenyl)azetidine-1-carbonyl)hexahydro-2H-pyrido[4,3-b][1,4]oxazin-3(4H)-one (50 mg, 127 µmol, example 110), tris(trimethylsilyl)silane (31.5 mg, 39.1 µL, 127 µmol, CAS RN 1873-77-4) and anhydrous sodium carbonate (26.9 mg, 254 µmol). The vial was sealed and placed under argon before DME (1 mL) was added. To a separate vial was added nickel(II) chloride ethylene glycol dimethyl ether complex (2.79 mg, 12.7 µmol, CAS RN 29046-78-4) and 4,4'-di-tert-butyl-2,2'-bipyridine (3.4 mg, 12.7 µmol, CAS RN 72914-19-3 ). The precatalyst vial was sealed, purged with argon and then DME (0.4 mL) was added. The precatalyst vial was sonicated for 5 min, after which, 0.4 mL (0.5 mol% catalyst, 0.01eq) of it was syringed into the reaction vessel. The reaction mixture was degassed with argon. The reaction was stirred and irradiated with a 420 nm lamp for 4 h. The reaction was quenched by exposure to air, filtered and washed with a small volume of EtOAc. The filtrate was treated with silica gel and evaporated. The compound was purified by silica gel chromatography on a 4 g column using an MPLC (ISCO) system eluting with a gradient of n-heptane : EtOAc/EtOH 3/1 (v/v) (70 : 30 to 10 : 90) to furnish the desired compound as a light yellow solid (0.010 g; 21.2%). MS (ESI): m/z = 372.3 [M+H]⁺.

### Method A8

### Example 159

### 2-[4-[1-[(4aR,8aS)-3-Oxo-4,4a,5,7,8,8a-hexahydropyrido[4,3-b][1,4]oxazine-6-carbonyl]azetidin-3-yl]phenyl]-5-chloro-benzonitrile

To a solution of [4-[1-[(4aR,8aS)-3-oxo-4,4a,5,7,8,8a-hexahydropyrido[4,3-b][1,4]oxazine-6-carbonyl]azetidin-3-yl]phenyl]boronic acid (100.0 mg, 0.280 mmol, BB38) and 2-bromo-5-chlorobenzonitrile (120.5 mg, 0.560 mmol, CAS RN 57381-37-0) and CsF (127 mg, 0.840 mmol) in DMF (5 mL) was added Pd(dppf)Cl₂ (40.8 mg, 0.060 mmol), the mixture was stirred at 90 °C under N₂ atmosphere for 12 h.The mixture was filtered and the filtrate was diluted with water (20 mL) and extracted with EtOAc (10 mL three times), the combined organic phase was washed with brine, dried over Na₂SO₄ and concentrated, the residue was purified by prep-HPLC (FA) and lyophilized to get the desired compound as white solid (38.8 mg, 30.7%). MS (ESI): m/z = 451.1 [M+H]⁺.

### Method A9

### Examples 75 and 76

### (4aR,8aS)-6-(3-(R or S)-(1-(2-chloro-4-(trifluoromethyl)phenoxy)ethyl)azetidine-1-carbonyl)hexahydro-2H-pyrido[4,3-b][1,4]oxazin-3(4H)-one and

### (4aR,8aS)-6-(3-(S or R)-(1-(2-chloro-4-(trifluoromethyl)phenoxy)ethyl)azetidine-1-carbonyl)hexahydro-2H-pyrido[4,3-b][1,4]oxazin-3(4H)-one

To a solution of 3-(1-(2-chloro-4-(trifluoromethyl)phenoxy)ethyl)azetidine 2,2,2-trifluoroacetate (BB94) (133.5 mg, 207 µmol) in a mixture of CH₃CN (517 µL) and 2-propanol (517 µL) was added DIPEA (66.8 mg, 90.3 µL, 517 µmol) and 4-nitrophenyl (4aR,8aS)-3-oxohexahydro-2H-pyrido[4,3-b][1,4]oxazine-6(5H)-carboxylate (66.5 mg, 207 µmol, BB15a). The reaction vial was stirred at 90 °C for 21 h. A further portion of 4-nitrophenyl (4aR,8aS)-3-oxohexahydro-2H-pyrido[4,3-b][1,4]oxazine-6(5H)-carboxylate (19.9 mg, 62.1 µmol, BB15a) and DIPEA (8.02 mg, 10.8 µL, 62.1 µmol) were added and the mixture was heated to 95 °C for 23 h.The crude material was submitted for reversed-phase achiral HPLC purification to yield (4aR,8aS)-6-(3-(1-(2-chloro-4-(trifluoromethyl)phenoxy)ethyl)azetidine-1-carbonyl)hexahydro-2H-pyrido[4,3-b][1,4]oxazin-3(4H)-one (88 mg, 92.1%) as a white solid. The epimers were separated by chiral HPLC to yield examples 75 (33.6 mg) and example 76 (43.2 mg) as yellow solids. MS (ESI): m/z = 462.2 [M+H]⁺ for both compounds.

### Method A10

### Examples 91, 96 and 97

### (4aR,8aS)-6-(3-(1-(2-Fluoro-4-(trifluoromethyl)phenoxy)ethyl)azetidine-1-carbonyl)hexahydro-2H-pyrido[4,3-b][1,4]oxazin-3(4H)-one, and

### (4aR,8aS)-6-(3-((R or S)-1-(2-Fluoro-4-(trifluoromethyl)phenoxy)ethyl)azetidine-1-carbonyl)hexahydro-2H-pyrido[4,3-b][1,4]oxazin-3(4H)-one, and

### (4aR,8aS)-6-(3-((S or R)-1-(2-Fluoro-4-(trifluoromethyl)phenoxy)ethyl)azetidine-1-carbonyl)hexahydro-2H-pyrido[4,3-b][1,4]oxazin-3(4H)-one

To a solution of 3-(1-(2-fluoro-4-(trifluoromethyl)phenoxy)ethyl)azetidine 2,2,2-trifluoroacetate, BB97 (51.4 mg, 136 µmol) in CH₃CN (681 µL) was added DIPEA (52.8 mg, 71.4 µL, 409 µmol) and 4-nitrophenyl (4aR,8aS)-3-oxohexahydro-2H-pyrido[4,3-b][1,4]oxazine-6(5H)-carboxylate (43.8 mg, 136 µmol, BB15a). The reaction vial was stirred at 95 °C for 3 h. The crude material was submitted for reversed-phase HPLC purification to yield (4aR,8aS)-6-(3-(1-(2-fluoro-4-(trifluoromethyl)phenoxy)ethyl)azetidine-1-carbonyl)hexahydro-2H-pyrido[4,3-b][1,4]oxazin-3(4H)-one (example 91; 31 mg, 51%) as an off-white solid. MS (ESI): m/z = 446.3 [M+H]⁺. This racemic product was submitted to chiral SFC separation to yield (4aR,8aS)-6-(3-((R or S)-1-(2-fluoro-4-(trifluoromethyl)phenoxy)ethyl)azetidine-1-carbonyl)hexahydro-2H-pyrido[4,3-b][1,4]oxazin-3(4H)-one (15.9 mg, example 96) and (4aR,8aS)-6-(3-((S or R)-1-(2-fluoro-4-(trifluoromethyl)phenoxy)ethyl)azetidine-1-carbonyl)hexahydro-2H-pyrido[4,3-b][1,4]oxazin-3(4H)-one (15.2 mg, example 97) as white solids. MS (ESI): m/z = 446.2 [M+H]⁺ for both compounds.

### Method A11

### Example 55

### (4aR,8aS)-6-(3-(Fluorobis(4-fluorophenyl)methyl)azetidine-1-carbonyl)hexahydro-2H-pyrido[4,3-b][1,4]oxazin-3(4H)-one

To a ice-cold solution of triethylamine trihydrofluoride (31.8 mg, 32.1 µL, 197 µmol) in DCM (493 µL) under argon was added (diethylamino)difluorosulfonium tetrafluoroborate (33.9 mg, 148 µmol, CAS RN 63517-29-3) followed by (4aR,8aS)-6-(3-(bis(4-fluorophenyl)(hydroxy)methyl)azetidine-1-carbonyl)hexahydro-2H-pyrido[4,3-b][1,4]oxazin-3(4H)-one (example 54) (45.1 mg, 98.6 µmol) in DCM (493 µL), and the mixture was stirred for 70 min at 0 °C and at then at RT for 25 min. The reaction was quenched at 0 °C with saturated aqueous NaHCO₃ solution and stored in the fridge overnight. The phases were separated and the aqueous layer was extracted twice with DCM. The combined organic layers were washed with brine, dried over Na₂SO₄ and concentrated to give a pale oil. The crude material was submitted for reversed-phase HPLC purification to yield the desired compound as a white solid (20 mg, 41.5%). MS (ESI): m/z = 504.18 [M+HCOO]⁻.

### Method A12

### Example 224

### (4aR,8aS)-6-(3-(3-Chloro-4-(3-methylazetidin-1-yl)phenyl)azetidine-1-carbonyl)hexahydro-2H-pyrido[4,3-b][1,4]oxazin-3(4H)-one

To a solution of (4aR,8aS)-6-(3-(4-bromo-3-chlorophenyl)azetidine-1-carbonyl)hexahydro-2H-pyrido[4,3-b][1,4]oxazin-3(4H)-one (example 132, 0.020 g, 0.047 mmol) in dioxane (0.5 mL) was added Pd₂(dba)₃ (0.004 g, 0.0047 mmol), Xantphos (0.003 g, 0.0047 mmol) and cesium carbonate (0.030 g, 0.093 mmol). The mixture was degassed with argon, then 3-methylazetidine hydrochloride (CAS RN 1375472-05-1, 0.010 g, 0.093 mmol) was added and the reaction mixture was heated to 100°C for 16 h. After this time, 3-methylazetidine hydrochloride (0.005 g, 0.047 mmol), Pd₂(dba)₃ (0.004 g, 0.0047 mmol), Xantphos (0.003 g, 0.0047 mmol) and cesium carbonate (0.015 g, 0.047 mmol) were added and the reaction mixture was heated to 100°C for another 2 h. The mixture was diluted with EtOAc and washed with H₂O and brine. The organic phase was dried over Na₂SO₄, filtered and concentrated in vacuo. The residue was purified by prep-HPLC to give the title compound (0.007 g, 36%) as a yellow solid. MS (ESI): m/z = 419.3 [M+H]⁺.

### Method B1

### Example 5

### (+)-cis-6-[4-(6-Fluoro-1H-indol-3-yl)piperidine-1-carbonyl]-4,4a,5,7,8,8a-hexahydropyrido[4,3-b][1,4]oxazin-3-one

The racemate (product of example 7) was separated on a preparative chiral HPLC (Reprosil Chiral NR column) using an isocratic mixture of EtOH (containing 0.05% of NH₄OAc): n-heptane (60: 40). The fractions were evaporated to get the desired compound as a light yellow solid (0.009 mg). MS (ESI): m/z = 401.2 [M+H]⁺.

### Method B2

### Example 11

### (+)-cis-6-(4-(bis(4-Fluorophenyl)methyl)piperazine-1-carbonyl)hexahydro-2H-pyrido[4,3-b][1,4]oxazin-3(4H)-one

The enantiomers of example 12 were separated on a preparative chiral HPLC (Chiralcel OD column) using an isocratic mixture of EtOH (containing 0.05% of NH₄OA_{C}) : n-heptane (80 : 20). The fractions were evaporated to get the desired compound as a colorless solid (0.014 g). MS (ESI): m/z = 471.3 [M+H]⁺.

### Method B3

### Examples 31 and 32

### (+)-cis-6-(4-((R or S)-]-(4-Fluorophenyl)-3-hydroxypropyl)piperidine-1-carbonyl)hexahydro-2H-pyrido[4,3-b][1,4]oxazin-3(4H)-one and

### (-)-cis-6-(4-((S or R)-1-(4-Fluorophenyl)-3-hydroxypropyl)piperidine-1-carbonyl)hexahydro-2H-pyrido[4,3-b][1,4]oxazin-3(4H)-one

The enantiomers of racemic example 26 were separated by preparative chiral HPLC (Chiralpak AD column) using an isocratic mixture of EtOH (containing 0.05% of NH₄OA_{C}): n-heptane (40 : 60). The fractions were evaporated. The residue was taken up in EtOH, DCM and n-heptane and again evaporated to remove excess of AcOH. The compounds were purified by silica gel chromatography on a 4 g column using an MPLC (ISCO) system eluting with a gradient of DCM : MeOH (100 : 0 to 80 : 20) to provide the desired products as colorless gums.(0.004 g; 7.7% and 0.010 g, 19%). MS (ESI): m/z = 420.2 [M+H]⁺ for each stereoisomer.

### Method B4

### Example 94

### (4aR,8aS)-6-(4-((R or S)-(3-Cyclopropyl-1,2,4-oxadiazol-5-yl)(4-fluorophenyl)methyl)piperidine-1-carbonyl)hexahydro-2H-pyrido[4,3-b][1,4]oxazin-3(4H)-one

The racemate of example 68 was separated by preparative chiral SFC (IA, 12 nm, 5 µm, 250 x 4.6 mm column) using an isocratic mixture of MeOH : carbon dioxide (25 : 75). The fractions containing the were evaporated to get the desired product as a light brown solid (0.016 g, 40.0%). MS (ESI): m/z = 484.2 [M+H]⁺.

### Method B5

### Examples 114 and 115

### (4aR,8aS)-6-[3-[(1R)-1-[4-(Trifluoromethyl)phenyl]ethoxy]azetidine-1-carbonyl]-4,4a,5,7,8,8a-hexahydropyrido[4,3-b][1,4)oxazin-3-one and

### (4aR,8aS)-6-[3-[(1S)-[4-(Trifluoromethyl)phenyl]ethoxy]azetidine-1-carbonyl]-4,4a,5,7,8,8a-hexahydropyrido[4,3-b][1,4]oxazin-3-one

The enantiomers of example 90 were separated by preparative chiral SFC (OZ-H, 220 nm, 250 x 10 mm column) using an isocratic mixture of MeOH : carbon dioxide (20 : 80). The fractions were evaporated to give the title products as colorless amorphous solids (0.005 g). MS (ESI): m/z = 428.4 [M+H]⁺ for both enantiomers.

### Example 25

### (+)- or (-)-cis-6-(4-(5-Chloro-1-(2-hydroxyethyl)-1H-indol-3-yl)piperidine-1-carbonyl)hexahydro-2H-pyrido[4,3-b][1,4]oxazin-3(4H)-one

To a solution of (+)- or (-)-cis-6-(4-(1-(2-((tert-butyldimethylsilyl)oxy)ethyl)-5-chloro-1H-indol-3-yl)piperidine-1-carbonyl)hexahydro-2H-pyrido[4,3-b][1,4]oxazin-3(4H)-one (39 mg, 67.8 µmol, example 24) in DCM (0.5 mL) was added TFA (77.3 mg, 52.2 µL, 678 µmol) and the solution was stirred at RT for 1.5 hours. To the light yellow solution were added THF (0.1 mL) and H₂O (0.1 mL) and stirring was continued at RT for 96 hours. The reaction mixture was evaporated. The residue was poured on saturated aqueous NaHCO₃ solution and DCM and the layers were separated. The aqueous layer was extracted twice with DCM. The organic layers were dried over MgSO₄, filtered, treated with silica gel and evaporated. The compound was purified by silica gel chromatography on a 4 g column using an MPLC system eluting with a gradient of DCM : MeOH (100 : 0 to 90 : 10) to provide the desired compound as a colorless solid (0.014 g; 44.8%). MS (ESI): m/z = 461.2 [M+H]⁺.

### Example 173

### (4aR,8aS)-6-(3-(4'-Chloro-2'-(methylsulfonyl)-[1,1-biphenyl]-4-yl)azetidine-1-carbonyl)hexahydro-2H-pyrido[4,3-b][1,4]oxazin-3(4H)-one

A solution of (4aR,8aS)-6-(3-(4'-chloro-2'-fluoro-[1,1'-biphenyl]-4-yl)azetidine-1-carbonyl)hexahydro-2H-pyrido[4,3-b][1,4]oxazin-3(4H)-one (27 mg, 60.8 µmol, example 133) in DMSO (0.5 mL) was treated with sodium methanethiolate (4.26 mg, 60.8 µmol) and stirred at 100°C overnight. Another batch of sodium methanethiolate (4.26 mg, 60.8 µmol) were added and stirring was continued at 100°C for 2 h. After cooling down, the mixture was poured on water and the layers were separated. The organic layers were extracted twice with EtOAc. The organic layers were dried over MgSO₄, filtered and evaporated. The obtained crude intermediate (112 mg, light yellow oil) was dissolved in DCM (0.7 mL) and m-chloroperbenzoic acid (98.2 mg, 195 µmol) was added at RT. The mixture was stirred at RT and stirring was continued at RT for 1 h. The reaction mixture was poured on saturated aqueous NaHCO₃ solution and DCM and the layers were separated. The aqueous layer was extracted twice with DCM. The organic layers were dried over MgSO₄, filtered, treated with silica gel and evaporated. The compound was purified by silica gel chromatography on a 4 g column using an MPLC system eluting with a gradient of DCM : MeOH (100 : 0 to 90 : 10) to get the desired intermediate sulfoxide as a colorless gum (0.014 g). It was dissolved in DCM (0.7 mL) and m-chlorperbenzoic acid (43.6 mg, 195 µmol) was added. Stirring was continued at RT for 3 h. The reaction mixture was poured on saturated aqueous NaHCO₃ solution and DCM and the layers were separated. The aqueous layer was extracted twice with DCM. The organic layers were dried over MgSO₄, filtered, treated with silica gel and evaporated. The compound was purified by silica gel chromatography on a 4 g column using an MPLC system eluting with a gradient of DCM : MeOH (100 : 0 to 90 : 10) to get the desired compound as a colorless solid (0.004 g; 13.0%). MS (ESI): m/z = 504.1 [M+H]⁺.

### Example 193

### (4aR,8aS)-6-(3-(5-(2,4-Dichlorophenyl)pyridin-2-yl)azetidine-1-carbonyl)hexahydro-2H-pyrido[4,3-b][1,4]oxazin-3(4H)-one

To a solution of (4aR,8aS)-6-(3-(5-chloropyridin-2-yl)azetidine-1-carbonyl)hexahydro-2H-pyrido[4,3-b][1,4]oxazin-3(4H)-one (63 mg, 180 µmol, example 181) in dioxane (0.5 mL) under argon were added tricyclohexylphosphine (4.03 mg, 14.4 µmol, CAS RN 2622-14-2), tris(dibenzylideneacetone)dipalladium (0) chloroform adduct (7.44 mg, 7.18 µmol), (2,4-dichlorophenyl)boronic acid (36 mg, 189 µmol, CAS RN 68716-47-2), and Cs₂CO₃ (70.2 mg, 216 µmol) and the mixture was stirred at 100°C overnight. The reaction mixture was poured on water and EtOAc and the layers were separated. The aqueous layer was extracted twice with EtOAc. The combined organic layers were dried over MgSO₄, filtered and evaporated. The product was purified by preparative HPLC (Gemini NX column) using a gradient of ACN : water (containing 0.1% TEA) (20: 80 to 98 : 2) to furnish the desired compound as a light brown solid (0.0038 g; 4.6%). MS (ESI): m/z = 461.1 [M+H]⁺.

### Example 218

### (4aR,8aS)-6-[4-[Phenyl(pyridazin-3-yl)methyl)piperidine-1-carbonyl)-4,4a,5,7,8,8a-hexahydropyrido[4,3-b][1,4]oxazin-3-one

A solution of 3-[phenyl(4-piperidyl)methyl]pyridazine (70 mg, 0.28 mmol, BB86) and (4-nitrophenyl) (4aR,8aS)-3-oxo-4,4a,5,7,8,8a-hexahydropyrido[4,3-b][1,4]oxazine-6-carboxylate (intermediate BB15a) (106 mg, 0.330 mmol) in pyridine (3 mL) was stirred at 80 °C for 16 h. The solution was filtered and concentrated under vacuum to give a residue, which was purified by prep-HPLC (basic conditions) to yield (4aR,8a5)-6-[4-[phenyl(pyridazin-3-yl)methyl]piperidine-1-carbonyl]-4,4a,5,7,8,8a-hexahydropyrido[4,3-b][1,4]oxazin-3-one (5.9 mg, 5%) as a grey solid. MS (ESI): m/z = 436.2 [M+H]⁺.

### Example 247

### (4aR,8aS)-6-[3-[4-(3-Fluoropropyl)phenyl]azetidine-1-carbonyl]-4,4a,5,7,8,8a-hexahydropyrido[4,3-b][1,4]oxazin-3-one

To a stirred solution of 3-[4-(3-fluoropropyl)phenyl]azetidine (40 mg, 0.21 mmol, BB87) and DIPEA (0.07 mL, 0.41 mmol) in MeCN (0.9 mL) at 20 °C was added (4-nitrophenyl) (4aR,8aS)-3-oxo-4,4a,5,7,8,8a-hexahydropyrido[4,3-b][1,4]oxazine-6-carboxylate (66 mg, 0.210 mmol; BB15a). The resulting mixture was stirred at 80 °C for 16 h. The solution was concentrated under vacuum to give a residue, which was purified by prep-HPLC (TFA condition) to give (4aR,8aS)-6-[3-[4-(3-fluoropropyl)phenyl]azetidine-1-carbonyl]-4,4a,5,7,8,8a-hexahydropyrido[4,3-b][1,4]oxazin-3-one (16 mg, 17%, 84% purity) as white solid. This material was combined with the product obtained from a second reaction on similar scale (0.23 mmol 3-[4-(3-fluoropropyl)phenyl]azetidine), and purified a second time by prep-HPLC (TFA conditions) to give the title compound as a white solid. MS (ESI): m/z = 376.3 [M+H]⁺.

### Example 252

### (4aR,8aS)-6-[4-[[3-(2-Aminoethoxy)phenyl]-phenyl-methyl]piperidine-1-carbonyl]-4,4a,5,7,8,8a-hexahydropyrido[4,3-b][1,4]oxazin-3-one

To a solution of tert-butyl (2-(3-((1-((4aR,8aS)-3-oxooctahydro-2H-pyrido[4,3-b] [1,4]oxazine-6-carbonyl)piperidin-4-yl)(phenyl)methyl)phenoxy)ethyl)carbamate (Example 237; 178 mg, 300 µmol) in DCM (2.5 mL) was added TFA (274 mg, 185 µL, 2.4 mmol) and the reaction mixture was stirred at ambient temperature for 3 hours. EtOAc and aqueous KHCO₃ solution were added. The layers were separated and the aqueous phase was extracted with EtOAc. The combined organic layers were dried over Na₂SO₄. The solvent was removed under reduced pressure to give the title compound (148 mg, quant.) as a light yellow foam. MS (ESI): m/z = 493.3 [M+H]⁺.

### Example 255

### tert-Butyl N-[2-[3-[2-[3-[[1-[(4aR,8aS)-3-oxo-4,4a,5,7,8,8a-hexahydropyrido[4,3-b][1,4]oxazine-6-carbonyl]-4-piperidyl]-phenyl-methyl]phenoxy]ethylamino]-3-oxo-propoxy]ethyl]carbamate

DIPEA (52.5 mg, 70.9 µL, 406 µmol) was added to a mixture of (4aR,8aS)-6-(4-((3-(2-aminoethoxy)phenyl)(phenyl)methyl)piperidine-1-carbonyl)hexahydro-2H-pyrido[4,3-b][1,4]oxazin-3(4H)-one (Example 252; 50 mg, 101 µmol), 3-(2-t-boc-aminoethoxy)propanoic acid (CAS RN 1260092-44-1; 23.7 mg, 101 µmol) and HATU (42.5 mg, 112 µmol) in DMF (203 µL). The mixture was stirred at ambient temperature for 18 h. Ethyl acetate and aqueous KHCO₃ solution were added. The layers were separated and the organic phase was washed two times with water. The organic layer was dried over Na₂SO₄. The solvent was removed under reduced pressure to give the title compound (71 mg, quant.) as coloreless oil, MS (ESI): m/z = 708.4 [M+H]⁺.

### Example 256

### tert-Butyl N-[2-[2-[3-[2-[3-[[1-[(4aR,8aS)-3-oxo-4,4a,5,7,8,8a-hexahydropyrido[4,3-b][1,4]oxazine-6-carbonyl]-4-piperidyl]-phenyl-methyl]phenoxy]ethylamino]-3-oxo-propoxy]ethoxy]ethyl]carbamate

DIPEA (52.5 mg, 70.9 µL, 406 µmol) was added to a mixture of (4aR,8aS)-6-(4-((3-(2-aminoethoxy)phenyl)(phenyl)methyl)piperidine-1-carbonyl)hexahydro-2H-pyrido[4,3-b][1,4]oxazin-3(4H)-one (Example 252; 50 mg, 101 µmol), 13,13-dimethyl-11-oxo- 4-7,12-tioxa-10-azatetradecanoic acid (CAS RN 1365655-91-9; 28.1 mg, 101 µmol) and HATU (42.5 mg, 112 µmol) in DMF (135 µL). The mixture was stirred at ambient temperature for 18 h. Ethyl acetate and aqueous KHCO₃ solution were added. The layers were separated and the organic phase was washed two times with water. The organic layer was dried over Na₂SO₄. The solvent was removed under reduced pressure to give the title compound (76 mg, quant.) as coloreless oil, MS (ESI): m/z = 774.4 [M+Na]⁺.

### Example 258

### (4aR,8aS)-6-[3-(4-Propoxyphenyl)azetidine-1-carbonyl]-4,4a,5,7,8,8a-hexahydropyrido[4,3-b][1,4]oxazin-3-one

To a suspension of (4aR,8aS)-6-(3-(4-hydroxyphenyl)azetidine-1-carbonyl)hexahydro-2H-pyrido[4,3-b][1,4]oxazin-3(4H)-one (0.032 g, 96.6 µmol, BB145) and potassium carbonate (16 mg, 116 µmol) in DMF (0.4 mL) was added 1-iodopropane (19.7 mg, 11.3 µL, 116 µmol) and the mixture was stirred at 50°C in a sealed tube overnight. The mixture was filtered and the filter cake was washed with a few drops of DMF. The product was purified on a preparative HPLC (YMC Triart C18 column) using a gradient of ACN : water (containing 0.1% TEA) (20: 80 to 100 : 0) to provide the desired compound as a colorless solid (0.013 g; 36.0%). MS (ESI): m/z = 374.3 [M+H]⁺.

### Example 260

### 2-[4-[1-[(4aR,8aS)-3-Oxo-4,4a,5,7,8,8a-hexahydropyrido[4,3-b][1,4]oxazine-6-carbonyl]azetidin-3-yl]phenyl]-N-ethyl-5-methyl-benzamide

To a solution of (4aR,8aS)-6-[3-[4-(4,4,5,5-tetramethyl-1,3,2-dioxaborolan-2-yl)phenyl]azetidine-1-carbonyl]-4,4a,5,7,8,8a-hexahydropyrido[4,3-b] [1,4]oxazin-3-one (100.0 mg, 0.230 mmol, BB91), Na₂CO₃ (48.03 mg, 0.450 mmol) and 2-bromo-N-ethyl-5-methyl-benzamide (54.86 mg, 0.230 mmol, BB92) in 1,4-dioxane (4 mL) and water (1 mL) was added Pd(dppf)Cl₂ (16.58 mg, 0.020 mmol) and the mixture was stirred at 100 °C under N₂ atmosphere. After stirring at this temperature for 12 h the mixture was poured into water (20 mL) and extracted three times with EtOAc (10 mL). The combined organic layers were washed with brine and dried over Na₂SO₄, concentrated, the residue was purified by reverse flash chromatography (0.1% v/v FA) followed by prep-HPLC (0.225% v/v FA) to give the desired product (13.8 mg, 12.5%) as a white solid. MS (ESI): m/z = 477.3 [M+H]⁺.

If not indicated otherwise the following examples of *Table 2* were synthesized from the suitable building block(s) in analogy to the reaction methods described herein.

**Table 2**

| **Ex.** | **Systematic Name / Structure** | **Building block(s)** | **MS, m/z** | **Method** |
|---|---|---|---|---|
| **3** | rac-(4aR,8aS)-6-(4-(5-Chloro-1-methyl-1H-indol-3-yl)piperidine-1-carbonyl)hexahydro-2H-pyrido[4,3-b] [1,4]oxazin-3(4H)-one | BB3 | 431.2 [M+H]⁺ | A2 |
| **4** | rac-(4aR,8aS)-6-(4-(9H-Fluoren-9-yl)piperidine-1-carbonyl)hexahydro-2H-pyrido[4,3-b] [1,4]oxazin-3(4H)-one | BB4 | 432.4 [M+H]⁺ | A2 |
| **6** | rac-(4aR,8aS)-6-(4-(1-(4-Fluorophenyl)-3-methoxypropyl)piperidine-1-carbonyl)hexahydro-2H-pyrido[4,3-b] [1,4]oxazin-3(4H)-one | BBS | 434.2 [M+H]⁺ | A2 |
| **7** | rac-(4aR,8aS)-6-[4-(6-Fluoro-1H-indol-3-yl)piperidine-1-carbonyl]-4,4a,5,7,8,8a-hexahydropyrido[4,3-b][1,4]oxazin-3-one | 6-Fluoro-3-(4-piperidyl)-1H-indole | 399.4 [M-H]⁻ | A1 |
| | | (CAS RN 76315-55-4) | | |
| **8** | rac-(4aR,8aS)-6-(4-((4-Fluorophenyl)(methoxy)methyl)p iperidine- 1-carbonyl)hexahydro-2H-pyrido[4,3-b][1,4]oxazin-3 (4H)-one | BB6 | 406.2 [M+H]⁺ | A2 |
| **9** | rac-(4aR,8aS)-6-(4-(5-Fluorobenzo[d]isoxazol-3-yl)piperidine-1-carbonyl)hexahydro-2H-pyrido[4,3-b] [1,4]oxazin-3(4H)-one | 5-Fluoro-3-(4-piperidyl)-1,2-benzoxazole | 447.2 [M+HCO O]⁻ | A2 |
| | | (CAS RN 84163-16-6) | | |
| **10** | rac-(4aR,8aS)-6-(4-(rac-(4-Chlorophenyl)(phenyl)methyl)pi perazine-1-carbonyl)hexahydro-2H-pyrido[4,3-b][1,4]oxazin-3(4H)-one | 1-[(4-Chlorophenyl)-phenylmethyl]piperazine | 469.2 [M+H]⁺ | A2 |
| | | (CAS RN 303-26-4) | | |
| **12** | rac-(4aR,8aS)-6-[4-[bis(4-Fluorophenyl)methyl]piperazine-1-carbonyl]-4,4a,5,7,8,8a-hexahydropyrido [4,3-b][1,4]oxazin-3-one | 1-[Bis(4-fluorophenyl)methyl] piperazine | 471.4 [M+H]⁺ | A2 |
| | | (CAS RN 27469-60-9) | | |
| **13** | (-)-(cis)-6-(4-(bis(4-Fluorophenyl)methyl)piperazine-1-carbonyl)hexahydro-2H-pyrido[4,3-b] [1,4]oxazin-3(4H)-one | Example 12 | 471.2 [M+H]⁺ | B2 |
| **14** | rac-(4aR,8aS)-6-(4-(5-Chloro-1H-indol-3-yl)piperazine-1-carbonyl)hexahydro-2H-pyrido[4,3-b] [1,4]oxazin-3(4H)-one | 5-Chloro-3-piperazin-1-yl-1H-indole | 418.3 [M+H]⁺ | A2 |
| | | (Aurora Fine Chemicals, catalog number: A36.683.325) | | |
| **15** | rac-(4aR,8aS)-6-(4-(1-Phenylethyl)piperazine-1-carbonyl)hexahydro-2H-pyrido[4,3-b] [1,4]oxazin-3(4H)-one | 1-(1-Phenylethyl)piperazine | 373.2 [M+H]⁺ | A2 |
| | | (CAS RN 69628-75-7) | | |
| **16** | rac-(4aR,8aS)-6-(4-(1-Methyl-1H-indazol-5 -yl)piperidine-1-carbonyl)hexahydro-2H-pyrido[4,3-b] [1,4]oxazin-3(4H)-one | BB7 | 398.2 [M+H]⁺ | A2 |
| **17** | rac-(4aR,8aS)-6-[4-[rac-(4-Fluorophenyl)-phenylmethyl]piperazine-1-carbonyl]-4,4a,5,7,8,8a-hexahydropyrido [4,3-b][1,4]oxazin-3-one | 1-[(4-Fluorophenyl)-phenylmethyl]piperazine | 453.3 [M+H]⁺ | A2 |
| | | (CAS RN 27064-89-7) | | |
| **19** | (+)- or (-)-cis-6-(4-(5-Chloro-1-cyclopropyl-1H-indol-3-yl)piperidine-1-carbonyl)hexahydro-2H-pyrido[4,3-b][1,4]oxazin-3(4H)-one | BB9 | 457.2 [M+H]⁺ | A2 |
| | | BB14a | | |
| **20** | rac-(4aR,8aS)-6-(4-(5-Chloro-1-(1-chloro-3-hydroxypropan-2-yl)-1H-indol-3-yl)piperidine-1-carbonyl)hexahydro-2H-pyrido[4,3-b][1,4]oxazin-3(4H)-one | BB10 | 509.2 [M+H]⁺ | A2 |
| | | BB14a | | |
| **21** | (+)- or (-)-cis-6-(4-(bis(4-Fluorophenyl)methyl)piperidine-1-carbonyl)hexahydro-2H-pyrido[4,3-b][1,4]oxazin-3(4H)-one | BB11 | 470.2 [M+H]⁺ | A2 |
| | | BB14a | | |
| **23** | (+)- or (-)-cis-6-(4-(5-Chloro-1-(oxetan-3-ylmethyl)-1H-indol-3-yl)piperidine-1-carbonyl)hexahydro-2H-pyrido[4,3-b][1,4]oxazin-3(4H)-one | BB12 | 487.2 [M+H]⁺ | A3 |
| | | BB14a | | |
| **24** | (+)- or (-)-cis-6-(4-(1-(2-((tertButyldimethylsilyl)oxy)ethyl)-5-chloro-1H-indol-3-yl)piperidine-1-carbonyl)hexahydro-2H-pyrido[4,3-b] [1,4]oxazin-3(4H)-one | BB13 | 575.3 [M+H]⁺ | A3 |
| | | BB14a | | |
| **26** | rac-cis-6-(4-(1-(4-Fluorophenyl)-3-hydroxypropyl)piperidine-1-carbonyl)hexahydro-2H-pyrido[4,3-b] [1,4]oxazin-3(4H)-one | BB16 | 420.2 [M+H]⁺ | A2 |
| | | BB14a | | |
| **27** | (4aR,8aS)-6-(4-(1-(4-(Trifluoromethyl)phenyl)ethyl)pi peridine-1-carbonyl)hexahydro-2H-pyrido[4,3-b][1,4]oxazin-3 (4H)-one | BB14a | 440.2 [M+H]⁺ | A2 |
| | | BB127 | | |
| **28** | rac-(4aR,8aS)-6-(4-(1-(2-Chloro-4-fluorophenoxy)ethyl)piperidine-1-carbonyl)hexahydro-2H-pyrido[4,3-b] [1,4]oxazin-3(4H)-one | BB17 | 440.2 [M+H]⁺ | A2 |
| | | BB14a | | |
| **29** | (4aR,8aS)-6-(4-(5-(Trifluoromethyl)pyridin-3-yl)piperidine-1-carbonyl)hexahydro-2H-pyrido[4,3-b] [1,4]oxazin-3(4H)-one | BB15a | 413.18 [M+H]⁺ | A2 |
| | | BB128 | | |
| **30** | (4aR,8aS)-6-(4-(Difluoro(4-(trifluoromethyl)phenyl)methyl)p iperidine- 1-carbonyl)hexahydro-2H-pyrido[4,3-b][1,4]oxazin-3(4H)-one | BB15a | 462.2 [M+H]⁺ | A2 |
| | | BB129 | | |
| **33** | (+) or (-)-cis-6-(4-((S or R)-1-(2-Chloro-4-fluorophenoxy)ethyl)piperidine-1-carbonyl)hexahydro-2H-pyrido[4,3-b][1,4]oxazin-3(4H)-one | Example 28 | 440.2 [M+H]⁺ | B3 |
| **34** | (+) or (-)-cis-6-(4-((R or S)-1-(2-Chloro-4-fluorophenoxy)ethyl)piperidine-1-carbonyl)hexahydro-2H-pyrido[4,3-b][1,4]oxazin-3(4H)-one | Example 28 | 440.2 [M+H]⁺ | B3 |
| **35** | (+) or (-)-cis-6-(4-(5-Methoxypyridin-3-yl)piperidine-1-carbonyl)hexahydro-2H-pyrido[4,3-b][1,4]oxazin-3(4H)-one | BB14a | 375.2 [M+H]⁺ | A2 |
| | | BB130 | | |
| **36** | (+) or (-)-cis-6-(4-(Bis(4-fluorophenyl)methyl)piperidine-1-carbonyl)hexahydro-2H-pyrido[4,3-b] [1,4]oxazin-3(4H)-one | BB14b | 470.2 [M+H]⁺ | A2 |
| | | BB11 | | |
| **37** | (+) or (-)-cis-6-(4-((4-Chlorophenyl)difluoromethyl)pip eridine-1-carbonyl)hexahydro-2H-pyrido[4,3-b][1,4]oxazin-3(4H)-one | BB14a | 428.2 [M+H]⁺ | A2 |
| | | BB131 | | |
| **38** | (+) or (-)-cis-6-(4-(5-(Trifluoromethoxy)pyridin-2-yl)piperidine-1-carbonyl)hexahydro-2H-pyrido[4,3-b] [1,4]oxazin-3(4H)-one | BB 14a | 429.2 [M+H]⁺ | A2 |
| | | BB 132 | | |
| **39** | (+) or (-)-cis-6-(4-((2-Chloro-4-fluorophenyl)difluoromethyl)pip eridine-1-carbonyl)hexahydro-2H-pyrido[4,3-b][1,4]oxazin-3(4H)-one | BB 14a | 446.2 [M+H]⁺ | A2 |
| | | BB 133 | | |
| **40** | (+) or (-)-cis-6-(4-(5-Ethylpyridin-3-yl)piperidine-1-carbonyl)hexahydro-2H-pyrido[4,3-b] [1,4]oxazin-3(4H)-one | BB14a | 373.2 [M+H]⁺ | A2 |
| | | BB134 | | |
| **41** | (+) or (-)-cis-6-(4-(5-(1,1-Difluoroethyl)pyridin-2-yl)piperidine-1-carbonyl)hexahydro-2H-pyrido[4,3-b][1,4]oxazin-3(4H)-one | BB 14a | 409.2 [M+H]⁺ | A2 |
| | | BB 135 | | |
| **42** | (+) or (-)-cis-6-(4-(6-Chloro-1-methyl-1H-indazol-3-yl)piperidine-1-carbonyl)hexahydro-2H-pyrido[4,3-b] [1,4]oxazin-3(4H)-one | BB 14a | 432.2 [M+H]⁺ | A2 |
| | | BB18 | | |
| **43** | (4aR,8aS)-6-((3R or 3S)-4-(5-(1,1-Difluoroethyl)pyridin-2-yl)-3-methylpiperidine-1-carbonyl)hexahydro-2H-pyrido[4,3-b] [1,4]oxazin-3(4H)-one | BB136 | 423.2 [M+H]⁺ | B4 OD-H column, 15% MeOH |
| **44** | (4aR,8aS)-6-((3S or 3R)-4-(5-(1,1-Difluoroethyl)pyridin-2-yl)-3-methylpiperidine-1-carbonyl)hexahydro-2H-pyrido[4,3-b] [1,4]oxazin-3(4H)-one | BB136 | 423.2 [M+H]⁺ | B4 OD-H column, 15% MeOH |
| **45** | (4aR,8aS)-6-((4R or 4S)-(5-(1,1-Difluoroethyl)pyridin-2-yl)-3-methylpiperidine-1-carbonyl)hexahydro-2H-pyrido[4,3-b] [1,4]oxazin-3(4H)-one | BB136 | 423.4 [M+H]⁺ | B4 OD-H column, 15% MeOH |
| **46** | (4aR,8aS)-6-(4-((R or S)-1-(4-(Trifluoromethyl)phenyl)ethyl)pi peridine-1-carbonyl)hexahydro-2H-pyrido[4,3-b] [1,4]oxazin-3(4H)-one | Example 27 | 440.2 [M+H]⁺ | B3 |
| **47** | (4aR,8aS)-6-(4-((S or R)-1-(4-(Trifluoromethyl)phenyl)ethyl)pi peridine-1-carbonyl)hexahydro-2H-pyrido[4,3-b] [1,4]oxazin-3(4H)-one | Example 27 | 440.2 [M+H]⁺ | B3 |
| **48** | 2-(4-Fluorophenyl)-2-(1-((4aR,8aS)-3-oxooctahydro-2H-pyrido[4,3-b][1,4]oxazine-6-carbonyl)piperidin-4-yl)acetonitrile | BB 14a | 401.2 [M+H]⁺ | A3 |
| | | BB19 | | |
| **49** | (4aR,8aS)-6-(4-(2,2,2-Trifluoro-1-phenylethyl)piperazine-1-carbonyl)hexahydro-2H-pyrido[4,3-b] [1,4]oxazin-3(4H)-one | BB 14a | 427.2 [M+H]⁺ | A2 |
| | | BB 137 | | |
| **50** | (4aR,8aS)-6-(4-((4-Fluorophenyl)(2,2,2-trifluoroethoxy)methyl)piperidin e-1-carbonyl)hexahydro-2H-pyrido[4,3-b] [1,4]oxazin-3(4H)-one | BB14a | 474.2 [M+H]⁺ | A2 |
| | | BB20 | | |
| **51** | (4aR,8aS)-6-(4-(1-(4-Fluorophenyl)-2-(3-methyl-1,2,4-oxadiazol-5-yl)ethyl)piperidine-1-carbonyl)hexahydro-2H-pyrido[4,3-b] [1,4]oxazin-3(4H)-one | BB14a | 472.2 [M+H]⁺ | A2 |
| | | BB21 | | |
| **52** | (4aR,8aS)-6-(4-(1-(4-(Trifluoromethyl)phenyl)cyclopr opyl)piperidine-1-carbonyl)hexahydro-2H-pyrido[4,3-b] [1,4]oxazin-3(4H)-one | BB 14a | 452.2 [M+H]⁺ | A2 |
| | | BB22 | | |
| **53** | (4aR,8aS)-6-(4-(1-(2,4-Difluorophenyl)-2,2,2-trifluoroethyl)piperazine-1-carbonyl)hexahydro-2H-pyrido[4,3-b] [1,4]oxazin-3(4H)-one | BB 14a | 463.2 [M+H]⁺ | A2 |
| | | BB 138 | | |
| **54** | (4aR,8aS)-6-(3-(Bis(4-fluorophenyl)(hydroxy)methyl)az etidine-1-carbonyl)hexahydro-2H-pyrido[4,3-b][1,4]oxazin-3(4H)-one | BB 15a | 458.3 [M+H]⁺ | A9 |
| | | BB93 | | |
| **56** | (4aR,8aS)-6-(4-(1-(4-Fluorophenyl)-2-(2,2,2-trifluoroethoxy)ethyl)piperidine-1-carbonyl)hexahydro-2H-pyrido[4,3-b] [1,4]oxazin-3(4H)-one | BB 14a | 488.2 [M+H]⁺ | A2 |
| | | BB23 | | |
| **57** | (4aR,8aS)-6-(4-((R or S)-(4-Fluorophenyl)(2,2,2-trifluoroethoxy)methyl)piperidin e-1-carbonyl)hexahydro-2H-pyrido[4,3-b] [1,4]oxazin-3(4H)-one | Example 50 | 474.2 [M+H]⁺ | B3 |
| **58** | (4aR,8aS)-6-(4-((S or R)-(4-Fluorophenyl)(2,2,2-trifluoroethoxy)methyl)piperidin e-1-carbonyl)hexahydro-2H-pyrido[4,3-b] [1,4]oxazin-3(4H)-one | Example 50 | 474.2 [M+H]⁺ | B3 |
| **59** | (4aR,8aS)-6-(4-((R or S)-1-(4-Fluorophenyl)-2-(3-methyl-1,2,4-oxadiazol-5-yl)ethyl)piperidine-1-carbonyl)hexahydro-2H-pyrido[4,3-b][1,4]oxazin-3(4H)-one | Example 51 | 472.2 [M+H]⁺ | B2 |
| **60** | (4aR,8aS)-6-(4-((S or R)-1-(4-Fluorophenyl)-2-(3-methyl-1,2,4-oxadiazol-5-yl)ethyl)piperidine-1-carbonyl)hexahydro-2H-pyrido[4,3-b][1,4]oxazin-3(4H)-one | Example 51 | 472.2 [M+H]⁺ | B2 |
| **61** | (4aR,8aS)-6-(4-(2-Cyclopropylpyridin-4-yl)piperidine-1-carbonyl)hexahydro-2H-pyrido[4,3-b][1,4]oxazin-3(4H)-one | BB14a | 385.2 [M+H]⁺ | A2 |
| | | BB139 | | |
| **62** | (4aR,8aS)-6-[4-[(S or R)-(4-Fluorophenyl)-(3-methoxyphenyl)methyl]piperidin e-1-carbonyl]-4,4a,5,7,8,8a-hexahydropyrido [4,3-b][1,4]oxazin-3-one | BB14a | 482.2 [M+H]⁺ | A2 |
| | | BB99 | | |
| **63** | (4aR,8aS)-6-(4-((R or S)-(4-Fluorophenyl)(3-methoxyphenyl)methyl)piperidin e-1-carbonyl)hexahydro-2H-pyrido[4,3-b][1,4]oxazin-3(4H)-one | BB14a | 482.2 [M+H]⁺ | A2 |
| | | BB100 | | |
| **64** | (4aR,8aS)-6-(4-((S or R)-(3-Methoxyphenyl)(phenyl)methyl) piperidine-1-carbonyl)hexahydro-2H-pyrido[4,3-b][1,4]oxazin-3(4H)-one | BB14a | 464.3 [M+H]⁺ | A2 |
| | | BB101 | | |
| **65** | (4aR,8aS)-6-(4-((R or S)-(3-Methoxyphenyl)(phenyl)methyl) piperidine-1-carbonyl)hexahydro-2H-pyrido[4,3-b][1,4]oxazin-3(4H)-one | BB14a | 464.3 [M+H]⁺ | A2 |
| | | BB102 | | |
| **66** | (4aR,8aS)-6-(4-((S or R)-(3-(2-Fluoroethoxy)phenyl)(phenyl)me thyl)piperidine-1-carbonyl)hexahydro-2H-pyrido[4,3-b][1,4]oxazin-3(4H)-one | BB14a | 496.3 [M+H]⁺ | A2 |
| | | BB103 | | |
| **68** | (4aR,8aS)-6-(4-((S or R)-(4-Fluorophenyl)(phenyl)methyl)pip eridine-1-carbonyl)hexahydro-2H-pyrido[4,3-b][1,4]oxazin-3(4H)-one | BB14a | 425.2 [M+H]⁺ | A2 |
| | | BB104 | | |
| **69** | (4aR,8aS)-6-[4-[(S or R)-(4-Fluorophenyl)-(4-methylphenyl)methyl]piperidine-1-carbonyl]-4,4a,5,7,8,8a-hexahydropyrido[4,3-b][1,4]oxazin-3-one | BB14a | 464.3 [M+H]⁺ | A2 |
| | | BB105 | | |
| **70** | (4aR,8aS)-6-[4-[(R or S)-[4-(2-Fluoroethoxy)phenyl] - phenylmethyl]piperidine-1-carbonyl]-4,4a,5,7,8,8a-hexahydropyrido [4,3-b][1,4]oxazin-3-one | BB14a | 496.3 [M+H]⁺ | A2 |
| | | BB106 | | |
| **71** | (4aR,8aS)-6-(4-((S or R)-(4-Fluorophenyl)(4-methoxyphenyl)methyl)piperidin e-1-carbonyl)hexahydro-2H-pyrido[4,3-b][1,4]oxazin-3(4H)-one | BB14a | 482.2 [M+H]⁺ | A2 |
| | | BB107 | | |
| **72** | (4aR,8aS)-6-(4-((R or S)-(4-Fluorophenyl)(p-tolyl)methyl)piperidine-1-carbonyl)hexahydro-2H-pyrido[4,3-b][1,4]oxazin-3(4H)-one | BB14a | 466.3 [M+H]⁺ | A2 |
| | | BB108 | | |
| **73** | (4aR,8aS)-6-(4-((S or R)-(3,4-Dimethoxyphenyl)(4-fluorophenyl)methyl)piperidine-1-carbonyl)hexahydro-2H-pyrido[4,3-b][1,4]oxazin-3(4H)-one | BB14a | 512.3 [M+H]⁺ | A2 |
| | | BB109 | | |
| **74** | (4aR,8aS)-6-(4-((R or S)-(3,4-Dimethoxyphenyl)(phenyl)methy 1)piperidine-1-carbonyl)hexahydro-2H-pyrido[4,3-b][1,4]oxazin-3(4H)-one | BB14a | 494.2 [M+H]⁺ | A2 |
| | | BB110 | | |
| **75** | (4aR,8aS)-6-(3-(R or S)-(1-(2-chloro-4-(trifluoromethyl)phenoxy)ethyl)a zetidine-1-carbonyl)hexahydro-2H-pyrido[4,3-b][1,4]oxazin-3(4H)-one | BB15a | 462.1 [M+H]⁺ | A9 |
| | | BB94 | | |
| **76** | (4aR,8aS)-6-(3-(S or R)-(1-(2-Chloro-4-(trifluoromethyl)phenoxy)ethyl)a zetidine-1-carbonyl)hexahydro-2H-pyrido[4,3-b][1,4]oxazin-3(4H)-one | BB15a | 462.1 [M+H]⁺ | A9 |
| | | BB94 | | |
| **77** | (4aR,8aS)-6-(4-((R or S)-(4-Fluorophenyl)(phenyl)methyl)pip eridine-1-carbonyl)hexahydro-2H-pyrido[4,3-b][1,4]oxazin-3(4H)-one | BB14a | 452.2 [M+H]⁺ | A2 |
| | | BB111 | | |
| **78** | (4aR,8aS)-6-[4-[(R or S)-m-Tolyl(phenyl)methyl]piperidine-1-carbonyl]-4,4a,5,7,8,8a-hexahydropyrido [4,3-b][1,4]oxazin-3-one | BB14a | 448.3 [M+H]⁺ | A2 |
| | | BB112 | | |
| **79** | (4aR,8aS)-6-[4-[(S or R)-[4-(2-Fluoroethoxy)phenyl] - phenylmethyl]piperidine-1-carbonyl]-4,4a,5,7,8,8a-hexahydropyrido [4,3-b][1,4]oxazin-3-one | BB14a | 496.3 [M+H]⁺ | A2 |
| | | BB113 | | |
| **80** | (4aR,8aS)-6-[4-[(S or R)-m-Tolyl(phenyl)methyl]piperidine-1-carbonyl]-4,4a,5,7,8,8a-hexahydropyrido [4,3-b][1,4]oxazin-3-one | BB14a | 448.3 [M+H]⁺ | A2 |
| | | BB114 | | |
| **81** | (4aR,8aS)-6-(4-(1-(2-Chloro-4-fluorophenoxy)-2,2,2-trifluoroethyl)piperidine-1-carbonyl)hexahydro-2H-pyrido[4,3-b][1,4]oxazin-3(4H)-one | BB15a | 494.2 [M+H]⁺ | A9 |
| | | BB95 | | |
| **82** | (4aR,8aS)-6-(3-(bis(4-Fluorophenyl)methyl)azetidine-1-carbonyl)hexahydro-2H-pyrido[4,3-b][1,4]oxazin-3(4H)-one | BB15a | 442.3 [M+H]⁺ | A9 |
| | | BB96 | | |
| **83** | (4aR,8aS)-6-[3-[1-[4-(Trifluoromethyl)phenyl]ethyl]az etidine-1-carbonyl]-4,4a,5,7,8,8a-hexahydropyrido[4,3-b][1,4]oxazin-3-one | BB15a | 412.3 [M+H]⁺ | A4 |
| | | BB24 | | |
| **84** | (4aR,8aS)-6-[4-[(S or R)-Phenyl(p-tolyl)methyl]piperidine-1-carbonyl]-4,4a,5,7,8,8a-hexahydropyrido[4,3-b][1,4]oxazin-3-one | BB14a | 448.3 [M+H]⁺ | A2 |
| | | BB115 | | |
| **85** | (4aR,8aS)-6-(4-((R or S)-Phenyl(p-tolyl)methyl)piperidine-1-carbonyl)hexahydro-2H-pyrido[4,3-b][1,4]oxazin-3(4H)-one | BB14a | 448.3 [M+H]⁺ | A2 |
| | | BB116 | | |
| **86** | (4aR,8aS)-6-(3-Benzhydrylazetidine-1-carbonyl)hexahydro-2H-pyrido[4,3-b][1,4]oxazin-3(4H)-one | BB14a | 405.2 [M+H]⁺ | A2 |
| | | BB117 | | |
| **87** | (4aR,8aS)-6-(4-((S or R)-(3,4-Dimethoxyphenyl)(phenyl)methy 1)piperidine-1-carbonyl)hexahydro-2H-pyrido[4,3-b][1,4]oxazin-3(4H)-one | BB14a | 494.3 [M+H]⁺ | A2 |
| | | BB118 | | |
| **88** | (4aR,8aS)-6-(4-((R or S)-(3,4-Dimethoxyphenyl)(4-fluorophenyl)methyl)piperidine-1-carbonyl)hexahydro-2H-pyrido[4,3-b][1,4]oxazin-3(4H)-one | BB14a | 512.3 [M+H]⁺ | A2 |
| | | BB119 | | |
| **89** | (4aR,8aS)-6-(4-((R or S)-(4-Fluorophenyl)(4-methoxyphenyl)methyl)piperidin e-1-carbonyl)hexahydro-2H-pyrido[4,3-b][1,4]oxazin-3(4H)-one | BB14a | 482.2 [M+H]⁺ | A2 |
| | | BB120 | | |
| **90** | (4aR,8aS)-6-[3-[1-[4-(Trifluoromethyl)phenyl]ethoxy] azetidine-1-carbonyl]-4,4a,5,7,8,8a-hexahydropyrido [4,3-b][1,4]oxazin-3-one | BB15a | 428.3 [M+H]⁺ | A4 |
| | | BB74 | | |
| **91** | (4aR,8aS)-6-(3-(1-(2- Fluoro-4-(trifluoromethyl)phenoxy)ethyl)a zetidine-1-carbonyl)hexahydro-2H-pyrido[4,3-b] [1,4]oxazin-3(4H)-one | BB15a | 446.3 [M+H]⁺ | A10 |
| | | BB97 | | |
| **92** | (4aR,8aS)-6-(3-(1-(4-(Trifluoromethyl)phenoxy)ethyl) azetidine-1-carbonyl)hexahydro-2H-pyrido[4,3-b][1,4]oxazin-3(4H)-one | BB15a | 428.3 [M+H]⁺ | A10 |
| | | BB98 | | |
| **93** | (4aR,8aS)-6-[4-[1-[4-(Trifluoromethyl)phenyl]ethoxy] piperidine-1-carbonyl]-4,4a,5,7,8,8a-hexahydropyrido[4,3-b][1,4]oxazin-3-one | BB15a | 456.3 [M+H]⁺ | A4 |
| | | BH5 | | |
| **95** | (4aR,8aS)-6-(4-((S or R)-(3-Cyclopropyl-1,2,4-oxadiazol-5-yl)(4-fluorophenyl)methyl)piperidine-1-carbonyl)hexahydro-2H-pyrido[4,3-b][1,4]oxazin-3(4H)-one | Example 67 | 484.2 [M+H]⁺ | B4 |
| **96** | (4aR,8aS)-6-(3-((R or S)-1-(2-Fluoro-4-(trifluoromethyl)phenoxy)ethyl)a zetidine-1-carbonyl)hexahydro-2H-pyrido[4,3-b][1,4]oxazin-3(4H)-one | BB15a | 446.2 [M+H]⁺ | A10 |
| | | BB97 | | |
| **97** | (4aR,8aS)-6-(3-((S or R)-1-(2-Fluoro-4-(trifluoromethyl)phenoxy)ethyl)a zetidine-1-carbonyl)hexahydro-2H-pyrido[4,3-b][1,4]oxazin-3(4H)-one | BB15a | 446.2 [M+H]⁺ | A10 |
| | | BB97 | | |
| **98** | (4aR,8aS)-6-(3-((R or S)-1-(4-(Trifluoromethyl)phenoxy)ethyl) azetidine-1-carbonyl)hexahydro-2H-pyrido[4,3-b][1,4]oxazin-3(4H)-one | BB15a | 428.3 [M+H]⁺ | A10 |
| | | BB98 | | |
| **99** | (4aR,8aS)-6-(3-((S or R)-1-(4-(Trifluoromethyl)phenoxy)ethyl) azetidine-1-carbonyl)hexahydro-2H-pyrido[4,3-b][1,4]oxazin-3(4H)-one | BB15a | 428.2 [M+H]⁺ | A10 |
| | | BB98 | | |
| **100** | (4aR,8aS)-6-(4-((R or S)-(4-(2-Fluoroethoxy)phenyl)(4-fluorophenyl)methyl)piperidine-1-carbonyl)hexahydro-2H-pyrido[4,3-b][1,4]oxazin-3(4H)-one | BB14a | 514.3 [M+H]⁺ | A2 |
| | | BBN121 | | |
| **101** | (4aR,8aS)-6-(4-(5-Methyl-6-(trifluoromethyl)pyridin-3-yl)piperidine-1-carbonyl)hexahydro-2H-pyrido[4,3-b][1,4]oxazin-3(4H)-one | BB15a | 427.2 [M+H]⁺ | A2 |
| | | BB140 | | |
| **102** | (4aR,8aS)-6-(4-((4-Fluorophenyl)(3-(trifluoromethyl)- 1,2,4-oxadiazol-5-yl)methyl)piperidine-1-carbonyl)hexahydro-2H-pyrido[4,3-b][1,4]oxazin-3(4H)-one | BB15a | 512.2 [M+H]⁺ | A4 |
| | | BB25 | | |
| **103** | (4aR,8aS)-6-(3-((S or R)-1-(4-(Trifluoromethyl)phenyl)ethyl) az etidine-1-carbonyl)hexahydro-2H-pyrido[4,3-b][1,4]oxazin-3(4H)-one | Example 83 | 412.3 [M+H]⁺ | B4 |
| **104** | (4aR,8aS)-6-(3-((R or S)-1-(4-(Trifluoromethyl)phenyl)ethyl)az etidine-1-carbonyl)hexahydro-2H-pyrido[4,3-b][1,4]oxazin-3 (4H)-one | Example 83 | 412.3 [M+H]⁺ | B4 |
| **105** | (4aR,8aS)-6-[3-(S or R)-[(4-Fluorophenyl)-(2,2,2-trifluoroethoxy)methyl]azetidine-1-carbonyl]-4,4a,5,7,8,8a-hexahydropyrido[4,3-b][1,4]oxazin-3-one | BB26 | 446.2 [M+H]⁺ | B3 |
| **106** | (4aR,8aS)-6-[3-(R or S)-[(4-Fluorophenyl)-(2,2,2-trifluoroethoxy)methyl]azetidine-1-carbonyl]-4,4a,5,7,8,8a-hexahydropyrido[4,3-b][1,4]oxazin-3-one | BB26 | 446.2 [M+H]⁺ | B3 |
| **107** | (4aR,8aS)-6-(4-(5,6,7,8-Tetrahydroquinolin-4-yl)piperidine-1-carbonyl)hexahydro-2H-pyrido[4,3-b][1,4]oxazin-3(4H)-one | BB15a | 399.2 [M+H]⁺ | A2 |
| | | BB140 | | |
| **108** | (4aR,8aS)-6-(4-((R or S)-(3-(2-Fluoroethoxy)phenyl)(phenyl)me thyl)piperidine-1-carbonyl)hexahydro-2H-pyrido[4,3-b][1,4]oxazin-3(4H)-one | BB14a | 496.3 [M+H]⁺ | A2 |
| | | BB122 | | |
| **109** | (4aR,8aS)-6-(4-((S or R)-(4-(2-Fluoroethoxy)phenyl)(4-fluorophenyl)methyl)piperidine-1-carbonyl)hexahydro-2H-pyrido[4,3-b][1,4]oxazin-3(4H)-one | BB14a | 514.3 [M+H]⁺ | A2 |
| | | BB123 | | |
| **110** | (4aR,8aS)-6-(3-(4-Bromophenyl)azetidine-1-carbonyl)hexahydro-2H-pyrido[4,3-b][1,4]oxazin-3(4H)-one | BB15a | 396.1 [M+H]⁺ | A4 |
| | | 3-(4-Bromophenyl)azetidine hydrochloride | | |
| | | (CAS RN 90561-74-3) | | |
| **112** | (4aR,8aS)-6-(3-(2'-Chloro-[1,1'-biphenyl]-4-yl)azetidine-1-carbonyl)hexahydro-2H-pyrido[4,3-b][1,4]oxazin-3(4H)-one | Example 110 | 426.2 [M+H]⁺ | A5 |
| | | 2-Chlorophenylboronic acid (CAS RN 3900-89-8) | | |
| **113** | (4aR,8aS)-6-(3-(2',4'-Dichloro-[1,1'-biphenyl]-4-yl)azetidine- 1-carbonyl)hexahydro-2H-pyrido[4,3-b][1,4]oxazin-3(4H)-one | Example 110 | 460.3 [M+H]⁺ | A5 |
| | | 2,4-Dichlorophenylboronic acid (CAS RN 68716-47-2) | | |
| **114** | (4aR,8aS)-6-(3-(1-(R or S)-(4-(Trifluoromethyl)phenyl)ethoxy) azetidine-1-carbonyl)hexahydro-2H-pyrido[4,3-b][1,4]oxazin-3(4H)-one | Example 90 | 428.3 [M+H]⁺ | B5 |
| **115** | (4aR,8aS)-6-(3-(1-(S or R)-(4-(Trifluoromethyl)phenyl)ethoxy) azetidine-1-carbonyl)hexahydro-2H-pyrido[4,3-b][1,4]oxazin-3(4H)-one | Example 90 | 428.3 [M+H]⁺ | B5 |
| **116** | (4aR,8aS)-6-[3-(S or R)-[1-(2-Chloro-4-fluorophenyl)ethoxy]azetidine-1-carbonyl]-4,4a,5,7,8,8a-hexahydropyrido[4,3-b][1,4]oxazin-3-one | BB27 | 412.1 [M+H]⁺ | B4 |
| **117** | (4aR,8aS)-6-[3-(R or S)-[1-(2-Chloro-4-fluorophenyl)ethoxy]azetidine-1-carbonyl]-4,4a,5,7,8,8a-hexahydropyrido[4,3-b][1,4]oxazin-3-one | BB27 | 412.1 [M+H]⁺ | B4 |
| **118** | (4aR,8aS)-6-[4-[(R or S)-(3,4-dimethoxyphenyl)-(2-pyridyl)methyl]piperidine-1 - carbonyl]-4,4a,5,7,8,8a-hexahydropyrido[4,3-b][1,4]oxazin-3-one | BB80 | 495.3 [M+H]⁺ | B4 |
| **119** | (4aR,8aS)-6-[4-[(S or R)-(3,4-dimethoxyphenyl)-(2-pyridyl)methyl]piperidine-1 - carbonyl]-4,4a,5,7,8,8a-hexahydropyrido[4,3-b][1,4]oxazin-3-one | BB80 | 495.3 [M+H]⁺ | B4 |
| **120** | (4aR,8aS)-6-[3-[4-(Trifluoromethoxy)phenyl]azetid ine-1-carbonyl]-4,4a,5,7,8,8a-hexahydropyrido[4,3-b][1,4]oxazin-3-one | BB 15a | 400.1 [M+H]⁺ | A4 |
| | | 3-[4-(Trifluoromethoxy)phen yl]azetidine trifluoroacetate (CAS RN 1443220-35-6) | | |
| **121** | (4aR,8aS)-6-(3-(2'-(Trifluoromethoxy)-[1,1'-biphenyl]-4-yl)azetidine-1-carbonyl)hexahydro-2H-pyrido[4,3-b][1,4]oxazin-3(4H)-one | Example 110 | 476.2 [M+H]⁺ | A5 |
| | | (2-(Trifluoromethoxy)phen yl)boronic acid | | |
| | | (CAS RN 175676-65-0) | | |
| **122** | (4aR,8aS)-6-(3-(4-Bromophenyl)-3-fluoroazetidine-1-carbonyl)hexahydro-2H-pyrido[4,3-b][1,4]oxazin-3(4H)-one | BB15a | 412.1 [M+H]⁺ | A4 |
| | | 3-(4-Bromophenyl)-3-fluoroazetidine hydrochloride (CAS RN 1780825-67-3) | | |
| **123** | (4aR,8aS)-6-(3-(4-Bromophenyl)-3-hydroxyazetidine-1-carbonyl)hexahydro-2H-pyrido[4,3-b][1,4]oxazin-3(4H)-one | BB15a | 412.2 [M+H]⁺ | A4 |
| | | 3-(4-Bromophenyl)azetidin-3-ol hydrochloride (CAS RN 1989671-90-0) | | |
| **124** | (4aR,8aS)-6-(3-(4-Bromophenyl)-3-methylazetidine-1-carbonyl)hexahydro-2H-pyrido[4,3-b][1,4]oxazin-3(4H)-one | BB15a | 408.1 [M+H]⁺ | A4 |
| | | 3-(4-Bromophenyl)-3-methylazetidine (CAS RN 1368527-28-9) | | |
| **125** | (4aR,8aS)-6-(3-(2'-(Trifluoromethyl)-[1,1'-biphenyl]-4-yl)azetidine-1-carbonyl)hexahydro-2H-pyrido[4,3-b][1,4]oxazin-3(4H)-one | Example 110 | 460.2 [M+H]⁺ | A5 |
| | | (2-(Trifluoromethyl)pheny l)boronic acid (CAS RN 1423-27-4) | | |
| **126** | (4aR,8aS)-6-(3-(2',4'-Difluoro-[1,1'-biphenyl]-4-yl)azetidine- 1-carbonyl)hexahydro-2H-pyrido[4,3-b][1,4]oxazin-3(4H)-one | Example 110 | 428.2 [M+H]⁺ | A5 |
| | | (2,4-Difluorophenyl)boronic acid (CAS RN 144025-03-6) | | |
| **127** | 4'-(1-((4aR,8aS)-3-Cxooctahydro-2H-pyrido[4,3-b][1,4]oxazine-6-carbonyl)azetidin-3-yl)-[1,1'-biphenyl]-2-carbonitrile | Example 110 | 417.2 [M+H]⁺ | A5 |
| | | (2-Cyanophenyl)boronic acid (CAS RN 138642-62-3) | | |
| **129** | (4aR,8aS)-6-(3-(4-(3-(Trifluoromethyl)azetidin-1-yl)phenyl)azetidine-1-carbonyl)hexahydro-2H-pyrido[4,3-b][1,4]oxazin-3(4H)-one | Example 110 | 439.2 [M+H]⁺ | A6 |
| | | 3-(Trifluoromethyl)azetidi ne (CAS RN 1221349-18-3) | | |
| **130** | 2- [4- [1- [(4aR,8aS)-3-Oxo-4,4a,5,7,8,8a-hexahydropyrido [4,3-b][1,4]oxazine-6-carbonyl]azetidin-3-yl]phenyl]benzamide | Example 110 | 435.2 [M+H]⁺ | A5 |
| | | (2-Cyanophenyl)boronic acid (CAS RN 138642-62-3) | | |
| **131** | (4aR,8aS)-6-(3-(2-Chloro-[1, 1'-biphenyl]-4-yl)azetidine-1-carbonyl)hexahydro-2H-pyrido[4,3-b][1,4]oxazin-3(4H)-one | BB15a | 426.2 [M+H]⁺ | A4 |
| | | BB28 | | |
| **132** | (4aR,8aS)-6-(3-(4-Bromo-3-chlorophenyl)azetidine-1-carbonyl)hexahydro-2H-pyrido[4,3-b][1,4]oxazin-3(4H)-one | BB15a | 430.0 [M+H]⁺ | A4 |
| | | BB29 | | |
| **133** | (4aR,8aS)-6-[3-[4-(4-Chloro-2-fluoro-phenyl)phenyl]azetidine-1-carbonyl]-4,4a,5,7,8,8a-hexahydropyrido[4,3-b][1,4]oxazin-3-one | Example 110 | 444.1 [M+H]⁺ | A5 |
| | | 4-Chloro-2-fluorophenylboronic acid (CAS RN 160591-91-3) | | |
| **134** | (4aR,8aS)-6-[3-[4-(2-Chloro-4-fluoro-phenyl)phenyl]azetidine-1-carbonyl]-4,4a,5,7,8,8a-hexahydropyrido[4,3-b][1,4]oxazin-3-one | Example 110 | 444.2 [M+H]⁺ | A5 |
| | | 2-Chloro-4-fluorophenylboronic acid (CAS RN 313545-72-1) | | |
| **135** | (4aR,8aS)-6-[4-[(R or S)-(3,4-Dimethoxyphenyl)-(3-pyridyl)methyl]piperidine-1-carbonyl]-4,4a,5,7,8,8a-hexahydropyrido[4,3-b][1,4]oxazin-3-one | BB81 | 495.2 [M+H]⁺ | B4 |
| **136** | (4aR,8aS)-6-[4-[(S or R)-(3,4-dimethoxyphenyl)-(3-pyridyl)methyl]piperidine-1 - carbonyl]-4,4a,5,7,8,8a-hexahydropyrido[4,3-b][1,4]oxazin-3-one | BB81 | 495.2 [M+H]⁺ | B4 |
| **137** | (4aR,8aS)-6-(3-(2'-Methyl-[1,1'-biphenyl]-4-yl)azetidine-1-carbonyl)hexahydro-2H-pyrido[4,3-b][1,4]oxazin-3(4H)-one | Example 110 | 406.2 [M+H]⁺ | A5 |
| | | o-Tolylboronic acid (CAS RN 16419-60-6) | | |
| **138** | (4aR,8aS)-6-(3-(4-(3-((1,1,1-Trifluoropropan-2-yl)oxy)azetidin-1-yl)phenyl)azetidine-1-carbonyl)hexahydro-2H-pyrido[4,3-b][1,4]oxazin-3(4H)-one | Example 110 | 483.2 [M+H]⁺ | A6 |
| | | 3-((1,1,1-Trifluoropropan-2-yl)oxy)azetidine hydrochloride (CAS RN 1803585-68-3) | | |
| **139** | (4aR,8aS)-6-(3-(3-Bromophenyl)azetidine-1-carbonyl)hexahydro-2H-pyrido[4,3-b][1,4]oxazin-3(4H)-one | BB15a | 394.1 [M+H]⁺ | A4 |
| | | BB30 | | |
| **140** | (4aR,8aS)-6-(3-(4-(tert-Butyl)phenyl)azetidine-1-carbonyl)hexahydro-2H-pyrido[4,3-b][1,4]oxazin-3(4H)-one | BB15a | 372.2 [M+H]⁺ | A4 |
| | | BB31 | | |
| **141** | (4aR,8aS)-6-[3-(4-Phenylphenyl)azetidine-1-carbonyl]-4,4a,5,7,8,8a-hexahydropyrido[4,3-b][1,4]oxazin-3-one | Example 110 | 392.2 [M+H]⁺ | A5 |
| | | Phenylboronic acid (CAS RN 98-80-6) | | |
| **142** | (4aR,8aS)-6-[3-[4-[2-(Difluoromethyl)phenyl]phenyl] a zetidine-1-carbonyl]-4,4a,5,7,8,8a-hexahydropyrido[4,3-b][1,4]oxazin-3-one | Example 110 | 442.1 [M+H]⁺ | A5 |
| | | 2-[2-(Difluoromethyl)phenyl ]-4,4,5,5-tetramethyl-1,3,2-dioxaborolane (CAS RN 879275-72-6) | | |
| **143** | (4aR,8aS)-6-(3-(3'-Methyl-[1,1'-biphenyl]-4-yl)azetidine-1-carbonyl)hexahydro-2H-pyrido[4,3-b][1,4]oxazin-3(4H)-one | Example 110 | 450.2 [M+HCO O]⁻ | A5 |
| | | m-Tolylboronic acid | | |
| | | (CAS RN 17933-03-8) | | |
| **144** | (4aR,8aS)-6-(3-(4'-Methyl-[1,1'-biphenyl]-4-yl)azetidine-1-carbonyl)hexahydro-2H-pyrido[4,3-b][1,4]oxazin-3(4H)-one | Example 110 | 450.2 [M+HCO O]⁻ | A5 |
| | | p-Tolylboronic acid | | |
| | | (CAS RN 5720-05-8) | | |
| **145** | (4aR,8aS)-6-(3-(6-Chloropyridin-3-yl)azetidine-1-carbonyl)hexahydro-2H-pyrido[4,3-b][1,4]oxazin-3(4H)-one | BB15a | 395.1 [H+HCO O]⁻ | A4 |
| | | BB32 | | |
| **146** | (4aR,8aS)-6-(3-(4-(Trifluoromethyl)phenyl)azetidin e-1-carbonyl)hexahydro-2H-pyrido[4,3-b][1,4]oxazin-3(4H)-one | BB15a | 428.1 [M+HCO O]⁻ | A4 |
| | | BB33 | | |
| **147** | (4aR,8 aS)-6-(3-(4-(3-((1,1,1-Trifluoro-2-methylpropan-2-yl)oxy)azetidin-1-yl)phenyl)azetidine-1-carbonyl)hexahydro-2H-pyrido[4,3-b][1,4]oxazin-3(4H)-one | Example 110 | 497.2 [M+H]⁺ | A6 |
| | | BB34 | | |
| **148** | (4aR,8aS)-6-(3-(4-(1,1-Difluoroethyl)phenyl)azetidine-1-carbonyl)hexahydro-2H-pyrido[4,3-b][1,4]oxazin-3(4H)-one | BB15a | 380.2 [M+H]⁺ | A4 |
| | | BB35 | | |
| **149** | (4aR,8aS)-6-[4-[(R or S)-(3,4-Dimethoxyphenyl)-(4-pyridyl)methyl]piperidine-1-carbonyl]-4,4a,5,7,8,8a-hexahydropyrido[4,3-b][1,4]oxazin-3-one | BB82 | 495.2 [M+H]⁺ | B4 |
| **150** | (4aR,8aS)-6-[4-[(S or R)-(3,4-Dimethoxyphenyl)-(4-pyridyl)methyl]piperidine-1-carbonyl]-4,4a,5,7,8,8a-hexahydropyrido [4,3-b][1,4]oxazin-3-one | BB82 | 495.2 [M+H]⁺ | B4 |
| **151** | (4aR,8aS)-6-(3-(4-(3-Methylazetidin-1-yl)phenyl)azetidine-1-carbonyl)hexahydro-2H-pyrido[4,3-b][1,4]oxazin-3(4H)-one | Example 110 | 385.4 [M+H]⁺ | A5 |
| | | 3-Methylazetidine hydrochloride (CAS RN 1375472-05-1) | | |
| **152** | (4aR,8aS)-6-(3-(4-(3,3-Dimethylpyrrolidin-1-yl)phenyl)azetidine-1-carbonyl)hexahydro-2H-pyrido[4,3-b][1,4]oxazin-3(4H)-one | Example 110 | 413.5 [M+H]⁺ | A5 |
| | | 3,3-Dimethylpyrrolidine hydrochloride (CAS RN 792915-20-9) | | |
| **153** | (4aR,8aS)-6-(3-(4-(3-(Trifluoromethoxy)azetidin-1-yl)phenyl)azetidine-1-carbonyl)hexahydro-2H-pyrido[4,3-b][1,4]oxazin-3(4H)-one | Example 110 | 455.2 [M+H]⁺ | A6 |
| | | 3-(Trifluoromethoxy)azeti dine hydrochloride (CAS RN 1422766-39-9) | | |
| **154** | (4aR,8aS)-6-(3-(6-(2,4-Dichlorophenyl)pyridin-3-yl)azetidine-1-carbonyl)hexahydro-2H-pyrido[4,3-b][1,4]oxazin-3(4H)-one | Example 145 | 461.1 [M+H]⁺ | A5 |
| | | (2,4-Dichlorophenyl)boronic acid (CAS RN 68716-47-2) | | |
| **155** | (4aR,8aS)-6-(3-(2'-Chloro-4'-(methylsulfonyl)-[1,1'-biphenyl] - 4-yl)azetidine-1-carbonyl)hexahydro-2H-pyrido[4,3-b][1,4]oxazin-3(4H)-one | Example 110 | 504.1 [M+H]⁺ | A5 |
| | | 2-(2-Chloro-4-(methylsulfonyl)phenyl) -4,4,5,5-tetramethyl-1,3,2-dioxaborolane (MFCD18760183; Enamine) | | |
| **156** | (4aR,8aS)-6-(3-(4-(3,3-Difluoroazetidin-1-yl)phenyl)azetidine-1-carbonyl)hexahydro-2H-pyrido[4,3-b][1,4]oxazin-3(4H)-one | Example 110 | 407.2 [M+H]⁺ | A6 |
| | | 3,3 -Difluoroazetidine hydrochloride (CAS RN 288315-03-7) | | |
| **157** | (4aR,8aS)-6-(4-(4-Bromophenyl)piperidine-1-carbonyl)hexahydro-2H-pyrido[4,3-b][1,4]oxazin-3(4H)-one | BB15a | 424.2 [M+H]⁺ | A4 |
| | | 4-(4-Bromophenyl)piperidin e (CAS RN 769944-79-8) | | |
| **158** | (4aR,8aS)-6-[3-[4-(2,2,2-Trifluoroethoxy)phenyl]azetidine -1-carbonyl]-4,4a,5,7,8,8a-hexahydropyrido[4,3-b][1,4]oxazin-3-one | BB15a | 414.1 [M+H]⁺ | A4 |
| | | BB37 | | |
| **160** | (4aR,8aS)-6-[3-[4-[3-[(1S or 1R)-2,2,2-Trifluoro-1-methyl-ethoxy]azetidin-1-yl]phenyl]azetidine-1-carbonyl]-4,4a,5,7,8,8a-hexahydropyrido [4,3-b][1,4]oxazin-3-one | Example 138 | 483.5 [M+H]⁺ | B4 |
| **161** | (4aR,8aS)-6-[3-[4-[3-[(1R or 1S)-2,2,2-Trifluoro-1-methyl-ethoxy]azetidin-1-yl]phenyl]azetidine-1-carbonyl]-4,4a,5,7,8,8a-hexahydropyrido [4,3-b][1,4]oxazin-3-one | Example 138 | 483.5 [M+H]⁺ | B4 |
| **162** | (4aR,8aS)-6-(3-(4-(2-(Trifluoromethyl)pyrrolidin-1-yl)phenyl)azetidine-1-carbonyl)hexahydro-2H-pyrido[4,3-b] [1,4]oxazin-3(4H)-one | Example 110 | 453.2 [M+H]⁺ | A6 |
| | | 2-(Trifluoromethyl)pyrrol idine (CAS RN 119618-29-0) | | |
| **163** | (4aR,8aS)-6-(3-(4-(3-(Trifluoromethyl)pyrrolidin-1-yl)phenyl)azetidine-1-carbonyl)hexahydro-2H-pyrido[4,3-b][1,4]oxazin-3(4H)-one | Example 110 | 453.2 [M+H]⁺ | A6 |
| | | 3-(Trifluoromethyl)pyrrol idine (CAS RN 644970-41-2) | | |
| **164** | (4aR,8aS)-6-(3-(3-Bromophenyl)pyrrolidine-1-carbonyl)hexahydro-2H-pyrido[4,3-b][1,4]oxazin-3(4H)-one | BB 15a | 410.2 [M+H]⁺ | A4 |
| | | 3-(3-Bromophenyl)pyrrolidi ne hydrochloride (CAS RN 1203681-69-9) | | |
| **165** | (4aR,8aS)-6-[3-[4-(2-Ethylpyrrolidin-1-yl)phenyl]azetidine-1-carbonyl]-4,4a,5,7,8,8a-hexahydropyrido[4,3-b][1,4]oxazin-3-one | Example 110 | 413.3 [M+H]⁺ | A6 |
| | | 2-Ethylpyrrolidine (CAS RN 1003-28-7) | | |
| **166** | 2-[4-[1-[(4aR,8aS)-3-Oxo-4,4a,5,7,8,8a-hexahydropyrido [4,3-b][1,4]oxazine-6-carbonyl]azetidin-3-yl]phenyl]-3-chloro-benzonitrile | BB38 | 451.2 [M+H]⁺ | A8 |
| | | 2-Bromo-3-chloro-benzonitrile (CAS RN 1031929-33-5) | | |
| **167** | (4aR,8aS)-6-(3-(4-(2-Cyclopropylpyrrolidin-1 - yl)phenyl)azetidine-1-carbonyl)hexahydro-2H-pyrido[4,3-b][1,4]oxazin-3(4H)-one | Example 110 | 425.3 [M+H]⁺ | A6 |
| | | 2-Cyclopropylpyrrolidine hydrochloride (CAS RN 558478-81-2) | | |
| **168** | (4aR,8aS)-6-(4-(4-Bromophenyl)piperazine-1-carbonyl)hexahydro-2H-pyrido[4,3-b][1,4]oxazin-3(4H)-one | 1-(4-Bromophenyl)piperazin e (CAS RN 66698-28-0) | 425.1 [M+H]⁺ | A4 |
| | | BB 15a | | |
| **169** | (4aR,8aS)-6-(4-(2',4'-Dichloro-[1,1'-biphenyl]-4-yl)piperidine-1-carbonyl)hexahydro-2H-pyrido[4,3-b][1,4]oxazin-3(4H)-one | Example 110 | 532.1 [M+HCO O]⁻ | A5 |
| | | (2,4-Dichlorophenyl)boronic acid (CAS RN 68716-47-2) | | |
| **170** | (4aR,8aS)-6-(3-(4-(3-Azabicyclo[3.1.0]hexan-3-yl)phenyl)azetidine-1-carbonyl)hexahydro-2H-pyrido[4,3-b][1,4]oxazin-3(4H)-one | Example 110 | 397.2 [M+H]⁺ | A6 |
| | | 3-Azabicyclo[3.1.0]hexan e hydrochloride (CAS RN 73799-64-1) | | |
| **171** | (4aR,8aS)-6-(3-(3-(Trifluoromethoxy)phenyl)azetid ine-1-carbonyl)hexahydro-2H-pyrido[4,3-b][1,4]oxazin-3(4H)-one | BB15a | 400.3 [M+H]⁺ | A4 |
| | | BB40 | | |
| **172** | (4aR,8aS)-6-(3-(4-(3-(2,2,2-Trifluoroethoxy)azetidin-1-yl)phenyl)azetidine-1-carbonyl)hexahydro-2H-pyrido[4,3-b][1,4]oxazin-3(4H)-one | Example 110 | 397.4 [M+H]⁺ | A6 |
| | | 3-(2,2,2-Trifluoroethoxy) azetidi ne (CAS RN 1333106-09-4) | | |
| **174** | (4aR,8aS)-6-(3-(6-(2-(Trifluoromethyl)pyrrolidin-1-yl)pyridin-3-yl)azetidine-1-carbonyl)hexahydro-2H-pyrido[4,3-b][1,4]oxazin-3(4H)-one | Example 145 | 454.3 [M+H]⁺ | A6 |
| | | 2-(Trifluoromethyl)pyrrol idine (CAS RN 119618-29-0) | | |
| **175** | (4aR,8aS)-6-[4-[(R or S)-(3-methylsulfonylphenyl)-(3-pyridyl)methyl]piperidine-1 - carbonyl]-4,4a,5,7,8,8a-hexahydropyrido[4,3-b][1,4]oxazin-3-one | BB83 | 513.3 [M+H]⁺ | B4 |
| **176** | (4aR,8aS)-6-[4-[(R or S)-(3-methylsulfonylphenyl)-(3-pyridyl)methyl]piperidine-1 - carbonyl]-4,4a,5,7,8,8a-hexahydropyrido [4,3-b][1,4]oxazin-3-one | BB83 | 513.3 [M+H]⁺ | B4 |
| **177** | (4aR,8aS)-6-[4-[(R or S)-(3-methylsulfonylphenyl)-(4-pyridyl)methyl]piperidine-1 - carbonyl]-4,4a,5,7,8,8a-hexahydropyrido [4,3-b][1,4]oxazin-3-one | BB84 | 513.3 [M+H]⁺ | B4 |
| **178** | (4aR,8aS)-6-[4-[(S or R)-(3-methylsulfonylphenyl)-(4-pyridyl)methyl]piperidine-1 - carbonyl]-4,4a,5,7,8,8a-hexahydropyrido[4,3-b][1,4]oxazin-3-one | BB84 | 513.3 [M+H]⁺ | B4 |
| **179** | (4aR,8aS)-6-(3-(4-(5-Azaspiro[2.4]heptan-5-yl)phenyl)azetidine-1-carbonyl)hexahydro-2H-pyrido[4,3-b][1,4]oxazin-3(4H)-one | Example 110 | 411.4 [M+H]⁺ | A6 |
| | | 5-Azaspiro[2.4]heptane hydrochloride (CAS RN 3659-21-0) | | |
| **180** | (4aR,8aS)-6-(3-(4-(Pentafluoro-16-sulfaneyl)phenyl)azetidine-1-carbonyl)hexahydro-2H-pyrido[4,3-b][1,4]oxazin-3(4H)-one | BB15a | 442.2 [M+H]⁺ | A4 |
| | | BB41 | | |
| 1**8**1 | (4aR,8aS)-6-(3-(5-Chloropyridin-2-yl)azetidine-1-carbonyl)hexahydro-2H-pyrido[4,3-b] [1,4]oxazin-3(4H)-one | BB15a | 351.2 [M+H]⁺ | A4 |
| | | BB42 | | |
| **182** | (4aR,8aS)-6-(3-(2-Fluoro-4-(trifluoromethoxy)phenyl)azetidi ne-1-carbonyl)hexahydro-2H-pyrido[4,3-b][1,4]oxazin-3(4H)-one | BB15a | 418.2 [M+H]⁺ | A4 |
| | | BB43 | | |
| **183** | (4aR,8aS)-6-[3-(6-Methoxypyridin-3-yl)azetidine-1-carbonyl]-4,4a,5,7,8,8a-hexahydropyrido[4,3-b][1,4]oxazin-3-one | BB15a | 347.3 [M+H]+ | A4 |
| | | BB76 | | |
| **184** | (4aR,8aS)-6-(3-(4-Bromophenyl)pyrrolidine-1-carbonyl)hexahydro-2H-pyrido[4,3-b][1,4]oxazin-3(4H)-one | BB15a | 410.2 [M+H]⁺ | A4 |
| | | 3-(4-Bromophenyl)pyrrolidi ne hydrochloride (CAS RN 1187931-39-0) | | |
| **185** | (4aR,8aS)-6-(3-Phenylazetidine-1-carbonyl)hexahydro-2H-pyrido[4,3-b][1,4]oxazin-3(4H)-one | BB15a | 316.3 [M+H]⁺ | A4 |
| | | 3-Phenylazetidine hydrochloride (CAS RN 7606-30-6) | | |
| **186** | (4aR,8aS)-6-(4-Phenylpiperidine-1-carbonyl)hexahydro-2H-pyrido[4,3-b][1,4]oxazin-3(4H)-one | BB15a | 344.3 [M+H]⁺ | A4 |
| | | 4-Phenylpiperidine (CAS RN 771-99-3) | | |
| **187** | (4aR,8aS)-6-[3-[4-(2,2,2-Trifluoroethyl)phenyl]azetidine-1-carbonyl]-4,4a,5,7,8,8a-hexahydropyrido [4,3-b][1,4]oxazin-3-one | BB15a | 398.1 [M+H]⁺ | A4 |
| | | BB44 | | |
| **188** | (4aR,8aS)-6-[3-[4-[1-(Trifluoromethyl)cyclopropyl]ph enyl]azetidine-1-carbonyl]-4,4a,5,7,8,8a-hexahydropyrido [4,3-b][1,4]oxazin-3-one | BB15a | 424.1 [M+H]⁺ | A4 |
| | | B45 | | |
| **189** | (4aR,8aS)-6-[4-[(R or S)-(3-methylsulfonylphenyl)-(2-pyridyl)methyl]piperidine-1 - carbonyl]-4,4a,5,7,8,8a-hexahydropyrido[4,3-b][1,4]oxazin-3-one | BB85 | 513.3 [M+H]⁺ | B4 |
| **190** | (4aR,8aS)-6-[4-[(S or R)-(3-methylsulfonylphenyl)-(2-pyridyl)methyl]piperidine-1 - carbonyl]-4,4a,5,7,8,8a-hexahydropyrido[4,3-b][1,4]oxazin-3-one | BB85 | 513.3 [M+H]⁺ | B4 |
| **191** | (4aR,8aS)-6-[3-[4-(6,6-Difluoro-2-azaspiro[3.3]heptan-2-yl)phenyl]azetidine-1-carbonyl]-4,4a,5,7,8,8a-hexahydropyrido[4,3-b][1,4]oxazin-3-one | Example 110 | 447.2 [M+H]⁺ | A6 |
| | | 6,6-Difluoro-2-aza-spiro[3.3]heptane trifluoroacetate (CAS RN 1427367-47-2) | | |
| **192** | (4aR,8aS)-6-(3-(4-(8-Oxa-3-azabicyclo[3.2.1]octan-3-yl)phenyl)azetidine-1-carbonyl)hexahydro-2H-pyrido[4,3-b][1,4]oxazin-3(4H)-one | Example 110 | 427.2 [M+H]⁺ | A6 |
| | | 8-Oxa-3-azabicyclo[3.2.1]octane hydrochloride (CAS RN 54745-74-3) | | |
| **194** | (4aR,8aS)-6-(3-(4-((S)-2-(Trifluoromethyl)pyrrolidin-1-yl)phenyl)azetidine-1-carbonyl)hexahydro-2H-pyrido[4,3-b] [1,4]oxazin-3(4H)-one | Example 162 | 453.2 [M+H]⁺ | B4 |
| **195** | (4aR,8aS)-6-(3-(4-((R)-2-(Trifluoromethyl)pyrrolidin-1-yl)phenyl)azetidine-1-carbonyl)hexahydro-2H-pyrido[4,3-b][1,4]oxazin-3(4H)-one | Example 162 | 453.2 [M+H]⁺ | B4 |
| **196** | (4aR,8aS)-6-[3-[4-(1-Piperidyl)phenyl] azetidine-1 - carbonyl]-4,4a,5,7,8,8a-hexahydropyrido[4,3-b][1,4]oxazin-3-one | Example 110 | 399.1 [M+H]⁺ | A6 |
| | | Piperidine (CAS RN 110-89-4) | | |
| **197** | (4aR,8aS)-6-(3-(4-((R or S)-3-(Trifluoromethyl)pyrrolidin-1-yl)phenyl)azetidine-1-carbonyl)hexahydro-2H-pyrido[4,3-b][1,4]oxazin-3(4H)-one | Example 163 | 453.2 [M+H]⁺ | B6 |
| **198** | (4aR,8aS)-6-(3-(4-((S or R)-3-(Trifluoromethyl)pyrrolidin-1-yl)phenyl)azetidine-1-carbonyl)hexahydro-2H-pyrido[4,3-b][1,4]oxazin-3(4H)-one | Example 163 | 453.2 [M+H]⁺ | B6 |
| **199** | (4aR,8aS)-6-[3-[4-(3-Hydroxy-3-methylazetidin-1-yl)phenyl]azetidine-1-carbonyl]-4,4a,5,7,8,8a-hexahydropyrido[4,3-b][1,4]oxazin-3-one | Example 110 | 401.2 [M+H]⁺ | A6 |
| | | 3-Methylazetidin-3-ol (CAS RN 256931-54-1) | | |
| **200** | (4aR,8aS)-6-[3-[4-(3-Fluoro-3-methyl-azetidin-1-yl)phenyl]azetidine-1-carbonyl]-4,4a,5,7,8,8a-hexahydropyrido[4,3-b][1,4]oxazin-3-one | Example 110 | 403.2 [M+H]⁺ | A6 |
| | | 3-Fluoro-3-methylazetidine hydrochloride (CAS RN 1427379-42-7) | | |
| **201** | (4aR,8aS)-6-(3-(4-(3-Fluoroazetidin-1-yl)phenyl)azetidine-1-carbonyl)hexahydro-2H-pyrido[4,3-b][1,4]oxazin-3(4H)-one | Example 110 | 389.2 [M+H]⁺ | A6 |
| | | 3-Fluoro-azetidine (CAS RN 690257-76-2) | | |
| **202** | (4aR,8aS)-6-(3-(3-Fluoro-4-(trifluoromethoxy)phenyl)azetidi ne-1-carbonyl)hexahydro-2H-pyrido[4,3-b][1,4]oxazin-3(4H)-one | BB15a | 418.3 [M+H]⁺ | A4 |
| | | BB46 | | |
| **203** | (4aR,8aS)-6-(3-(3-Methyl-4-(trifluoromethoxy)phenyl)azetidi ne-1-carbonyl)hexahydro-2H-pyrido[4,3-b][1,4]oxazin-3(4H)-one | BB15a | 414.2 [M+H]⁺ | A4 |
| | | BB47 | | |
| **204** | (4aR,8aS)-6-(3-(3,5-Difluoro-4-(trifluoromethoxy)phenyl)azetidi ne-1-carbonyl)hexahydro-2H-pyrido[4,3-b][1,4]oxazin-3(4H)-one | BB15a | 436.1 [M+H]⁺ | A4 |
| | | BB48 | | |
| **205** | (4aR,8aS)-6-(3-(2-Chloro-4-(trifluoromethoxy)phenyl)azetidi ne-1-carbonyl)hexahydro-2H-pyrido[4,3-b][1,4]oxazin-3(4H)-one | BB15a | 434.1 [M+H]⁺ | A4 |
| | | BB49 | | |
| **206** | (4aR,8aS)-6-(3-(4-(Bicyclo[1.1.1]pentan-1-yl)phenyl)azetidine-1-carbonyl)hexahydro-2H-pyrido[4,3-b][1,4]oxazin-3(4H)-one | BB15a | 382.3 [M+H]⁺ | A4 |
| | | BB50 | | |
| **207** | (4aR,8aS)-6-(3-(5-(2-(Trifluoromethyl)pyrrolidin-1-yl)pyridin-2-yl)azetidine-1-carbonyl)hexahydro-2H-pyrido[4,3-b][1,4]oxazin-3(4H)-one | Example 181 | 454.3 [M+H]⁺ | A6 |
| | | 2-(Trifluoromethyl)pyrrol idine (CAS RN 119618-29-0) | | |
| **208** | (4aR,8aS)-6-(3-(5-Fluoro-1H-indol-3-yl)pyrrolidine-1-carbonyl)hexahydro-2H-pyrido[4,3-b][1,4]oxazin-3(4H)-one | BB15a | 387.3 [M+H]⁺ | A4 |
| | | 5-Fluoro-3-(pyrrolidin-3-yl)-1H-indole (CAS RN 198474-06-5) | | |
| **209** | (4aR,8aS)-6-(3-(1-Benzyl-5-chloro-1H-indol-3-yl)pyrrolidine-1-carbonyl)hexahydro-2H-pyrido[4,3-b][1,4]oxazin-3(4H)-one | BB15a | 493.3 [M+H]⁺ | A4 |
| | | 1-Benzyl-5-chloro-3- (pyrrolidin-3-yl)-1H-indole | | |
| **210** | (4aR,8aS)-6-(3-(2-Fluoro-4-(trifluoromethyl)phenyl)azetidine -1-carbonyl)hexahydro-2H-pyrido[4,3-b][1,4]oxazin-3(4H)-one | BB14a | 400.1 [M-H]⁻ | A2 |
| | | BB142 | | |
| **211** | (4aR,8aS)-6-(3-(3-Chloro-4-(trifluoromethoxy)phenyl)azetidi ne-1-carbonyl)hexahydro-2H-pyrido[4,3-b][1,4]oxazin-3(4H)-one | BB15a | 434.2 [M+H]⁺ | A4 |
| | | BB77 | | |
| **212** | (4aR,8aS)-6-[3-[4-[3-(Cyclopropylmethoxy)azetidin-1 - yl]phenyl]azetidine-1-carbonyl]-4,4a,5,7,8,8a-hexahydropyrido[4,3-b][1,4]oxazin-3-one | Example 110 | 441.3 [M+H]⁺ | A6 |
| | | 3-(Cyclopropylmethoxy)a zetidine (CAS RN 1219976-56-3) | | |
| **213** | 5-(1-((4aR,8aS)-3-Oxooctahydro-2H-pyrido[4,3-b][1,4]oxazine-6-carbonyl)azetidin-3-yl)-2-(trifluoromethoxy)benzonitrile | BB15a | 425.1 [M+H]⁺ | A4 |
| | | BB51 | | |
| **214** | (4aR,8aS)-6-(3-(4-(1-(Hydroxymethyl)cyclopropyl)ph enyl)azetidine-1-carbonyl)hexahydro-2H-pyrido[4,3-b][1,4]oxazin-3(4H)-one | BB15a | 386.3 [M+H]⁺ | A4 |
| | | BB52 | | |
| **215** | (4aR,8aS)-6-(3-(1-Methyl-1H-indazol-4-yl)azetidine-1-carbonyl)hexahydro-2H-pyrido[4,3-b][1,4]oxazin-3(4H)-one | BB15a | 370.2 [M+H]⁺ | A4 |
| | | BB53 | | |
| **216** | (4aR,8aS)-6-(3-(4-(3-Fluoropyrrolidin-1-yl)phenyl)azetidine-1-carbonyl)hexahydro-2H-pyrido[4,3-b][1,4]oxazin-3(4H)-one | Example 110 | 403.2 [M+H]⁺ | A6 |
| | | 3-Fluoropyrrolidine hydrochloride (CAS RN 169750-17-8) | | |
| **217** | (4aR,8aS)-6-(3-(4-(2-Methylazetidin-1-yl)phenyl)azetidine-1-carbonyl)hexahydro-2H-pyrido[4,3-b][1,4]oxazin-3(4H)-one | Example 110 | 385.2 [M+H]⁺ | A6 |
| | | 2-Methylazetidine hydrochloride (CAS RN 1152113-37-5) | | |
| **219** | (4aR,8aS)-6-[3-[4-(Trifluoromethoxy)phenyl]pyrrol idine-1-carbonyl]-4,4a,5,7,8,8a-hexahydropyrido[4,3-b][1,4]oxazin-3-one | BB15a | 414.3 [M+H]⁺ | A4 |
| | | BB54 | | |
| **220** | (4aR,8aS)-6-(3-(1-Methyl-1H-indazol-6-yl)azetidine-1-carbonyl)hexahydro-2H-pyrido[4,3-b][1,4]oxazin-3(4H)-one | BB15a | 370.3 [M+H]⁺ | A4 |
| | | BB56 | | |
| **221** | (4aR,8aS)-6-[3-(S or R)-[3-(Trifluoromethoxy)phenyl]pyrrol idine-1-carbonyl]-4,4a,5,7,8,8a-hexahydropyrido [4,3-b][1,4]oxazin-3-one | BB57 | 414.3 [M+H]⁺ | prep-HPLC H₂O(0.225 %FA)/ACN (gradient 35%-62% ACN) |
| **222** | (4aR,8aS)-6-[3-(R or S)-[3-(Trifluoromethoxy)phenyl]pyrrol idine-1-carbonyl]-4,4a,5,7,8,8a-hexahydropyrido[4,3-b][1,4]oxazin-3-one | BB57 | 414.3 [M+H] ⁺ | prep-HPLC H₂O(0.225 %FA)/ACN (gradient 35%-62% ACN) |
| **223** | (4aR,8aS)-6-(3-(3-Chloro-4-hydroxyphenyl)azetidine-1-carbonyl)hexahydro-2H-pyrido[4,3-b][1,4]oxazin-3(4H)-one | 3-Methylazetidine hydrochloride (CAS RN 1375472-05-1) | 366.2 [M+H]⁺ | A6 |
| | | | | Heating 30 min µ-wave (120°), then at 110°C |
| **224** | (4aR,8aS)-6-(3-(3-Chloro-4-(3-methyl azetidin-1-yl)phenyl)azetidine-1-carbonyl)hexahydro-2H-pyrido[4,3-b][1,4]oxazin-3(4H)-one | Example 132 | 419.3 [M+H]⁺ | A6 |
| | | 3-Methylazetidine hydrochloride (CAS RN 1375472-05-1) | | (Pd₂(dba)₃, Xantphos, dioxane) |
| **226** | (4aR,8aS)-6-(3-(3-Chloro-4-(3,3-difluoroazetidin-1-yl)phenyl)azetidine-1-carbonyl)hexahydro-2H-pyrido[4,3-b][1,4]oxazin-3(4H)-one | Example 132 | 441.2 [M+H]⁺ | A9 |
| | | 3,3-Difluoroazetidine hydrochloride (CAS RN 288315-03-7) | | |
| **227** | (4aR,8aS)-6-(3-(3-(3-Methylazetidin-1-yl)-4-(trifluoromethoxy)phenyl)azetidi ne-1-carbonyl)hexahydro-2H-pyrido[4,3-b] [1,4]oxazin-3(4H)-one | BB78 | 469.3 [M+H]⁺ | A9 |
| | | 3-Methylazetidine hydrochloride (CAS RN 1375472-05-1) | | |
| **228** | (4aR,8aS)-6-[(3S or R)-3-(3-Bromophenyl)pyrrolidine-1-carbonyl]-4,4a,5,7,8,8a-hexahydropyrido[4,3-b][1,4]oxazin-3-one | Example 164 | 408.1 [M+H]⁺ | B3 |
| **229** | (4aR,8aS)-6-[(3R or S)-3-(3-Bromophenyl)pyrrolidine-1-carbonyl]-4,4a,5,7,8,8a-hexahydropyrido [4,3-b][1,4]oxazin-3-one | Example 164 | 408.1 [M+H]⁺ | B3 |
| **230** | (4aR,8aS)-6-[3-[4-(3-Azabicyclo[3.1.1]heptan-3-yl)phenyl]azetidine-1-carbonyl]-4,4a,5,7,8,8a-hexahydropyrido[4,3-b][1,4]oxazin-3-one | Example 110 | 411.2 [M+H]⁺ | A6 |
| | | 3-Azabicyclo[3.1.1]hepta ne hydrochloride (CAS RN 286-35-1) | | |
| **231** | (4aR,8aS)-6-(2-Methyl-3-(4-(Trifluoromethoxy)phenyl)azetid ine-1-carbonyl)hexahydro-2H-pyrido[4,3-b][1,4]oxazin-3(4H)-one | BB15a | 414.2 [M+H]⁺ | A4 |
| | | BB58 | | |
| **232** | (4aR,8aS)-6-(3-(3,3-Dimethyl-2,3-dihydrobenzofuran-6-yl)azetidine-1-carbonyl)hexahydro-2H-pyrido[4,3-b][1,4]oxazin-3(4H)-one | BB15a | 386.3 [M+H]⁺ | A4 |
| | | BB59 | | |
| **233** | (4aR,8aS)-6-(3-(3-Chloro-5-(2,2,2-trifluoroethoxy)phenyl)pyrrolidin e-1-carbonyl)hexahydro-2H-pyrido[4,3-b][1,4]oxazin-3(4H)-one | BB15a | 462.2 [M+H]⁺ | A4 |
| | | BB60 | | |
| **234** | Methyl 2-methyl-2-(4-(1-((4aR,8aS)-3-Oxooctahydro-2H-pyrido[4,3-b][1,4]oxazine-6-carbonyl)azetidin-3-yl)phenyl)propanoate | BB15a | 416.3 [M+H]⁺ | A4 |
| | | BB61 | | |
| **235** | (4aR,8aS)-6-(3-(3,5-Dichlorophenyl)pyrrolidine-1-carbonyl)hexahydro-2H-pyrido[4,3-b][1,4]oxazin-3(4H)-one | BB15a | 398.2 [M+H]⁺ | A4 |
| | | BB62 | | |
| **236** | (4aR,8aS)-6-(3-(4-(3-Methoxy-3-methyl azetidin-1-yl)phenyl)azetidine-1-carbonyl)hexahydro-2H-pyrido[4,3-b][1,4]oxazin-3(4H)-one | Example 110 | 415.2 [M+H]⁺ | A6 |
| | | 3-Methoxy-3-methylazetidine hydrochloride (CAS RN 905843-93-8) | | |
| **237** | tert-Butyl (2-(3-((1-((4aR,8aS)-3-oxooctahydro-2H-pyrido[4,3-b][1,4]oxazine-6-carbonyl)piperidin-4-yl)(phenyl)methyl)phenoxy)ethyl )carbamate | BB14a | 415.2 [M+H]⁺ | A2 |
| | | BB124 | | |
| **238** | (4aR,8aS)-6-((R or S)-3-(3-Chloro-5-(2,2,2-trifluoroethoxy)phenyl)pyrrolidin e-1-carbonyl)hexahydro-2H-pyrido[4,3-b][1,4]oxazin-3(4H)-one | Example 233 | 462.2 [M+H]⁺ | B4 |
| **239** | (4aR,8aS)-6-((S or R)-3-(3-Chloro-5-(2,2,2-trifluoroethoxy)phenyl)pyrrolidin e-1-carbonyl)hexahydro-2H-pyrido[4,3-b][1,4]oxazin-3(4H)-one | Example 233 | 462.2 [M+H]⁺ | B4 |
| **240** | (4aR,8aS)-6-(3-(4-(tert-Butyl)-3-methoxyphenyl)azetidine-1-carbonyl)hexahydro-2H-pyrido[4,3-b][1,4]oxazin-3(4H)-one | BB15a | 402.3 [M+H]⁺ | A4 |
| | | BB64 | | |
| **241** | (4aR,8aS)-6-(3-(1-Methyl-1H-indazol-5-yl)azetidine-1-carbonyl)hexahydro-2H-pyrido[4,3-b][1,4]oxazin-3(4H)-one | BB15a | 370.2 [M+H]⁺ | A4 |
| | | BB65 | | |
| **242** | (4aR,8aS)-6-(3-(4-Propylphenyl)azetidine-1-carbonyl)hexahydro-2H-pyrido[4,3-b][1,4]oxazin-3(4H)-one | BB15a | 358.2 [M+H]⁺ | A4 |
| | | BB66 | | |
| **243** | (4aR,8aS)-6-(3-(4-(Trifluoromethoxy)-3-(trifluoromethyl)phenyl)azetidine -1-carbonyl)hexahydro-2H-pyrido[4,3-b][1,4]oxazin-3(4H)-one | BB15a | 468.1 [M+H]⁺ | A4 |
| | | BB67 | | |
| **244** | (4aR,8aS)-6-[3-[4-[[1-(Trifluoromethyl)cyclopropyl]me thoxy]phenyl]azetidine-1-carbonyl]-4,4a,5,7,8,8a-hexahydropyrido[4,3-b][1,4]oxazin-3-one | BB15a | 454.1 [M+H]⁺ | A4 |
| | | BB68 | | |
| **245** | (4aR,8aS)-6-[3-[4-(2,2,2-Trifluoro-1,1-dimethylethyl)phenyl]azetidine-1-carbonyl]-4,4a,5,7,8,8a-hexahydropyrido [4,3-b][1,4]oxazin-3-one | BB15a | 426.1 [M+H]⁺ | A4 |
| | | BB69 | | |
| **246** | (4aR,8aS)-6-[3-(R or S)-[4-(2,2,2-Trifluoro-1-methyl-ethoxy)phenyl]azetidine-1-carbonyl]-4,4a,5,7,8,8a-hexahydropyrido [4,3-b][1,4]oxazin-3-one | BB71 | 428.2 [M+H]⁺ | B2 |
| **248** | (4aR,8aS)-6-[3-(S or R)-[4-(2,2,2-Trifluoro-1-methyl-ethoxy)phenyl]azetidine-1-carbonyl]-4,4a,5,7,8,8a-hexahydropyrido [4,3-b][1,4]oxazin-3-one | BB71 | 428.2 [M+H]⁺ | B2 |
| **249** | (4aR,8aS)-6-[3-(4-Cyclobutylphenyl)azetidine-1-carbonyl]-4,4a,5,7,8,8a-hexahydropyrido[4,3-b][1,4]oxazin-3-one | Example 110 | 370.3 [M+H]⁺ | A7 |
| | | Bromocyclobutane (CAS RN 4399-47-7) | | |
| **250** | (4aR,8aS)-6-[3-(3-Methoxy-4-methyl-phenyl)azetidine-1-carbonyl]-4,4a,5,7,8,8a-hexahydropyrido[4,3-b][1,4]oxazin-3-one | BB15a | 396.2 [M+H]⁺ | A4 |
| | | BB72 | | |
| **251** | (4aR,8aS)-6-(4-((4-Fluorophenyl)((1-methyl-5-(trifluoromethyl)-1H-pyrazol-3-yl)oxy)methyl)piperidine-1-carbonyl)hexahydro-2H-pyrido[4,3-b][1,4]oxazin-3(4H)-one | BB14a | 540.2 [M+H]⁺ | A2 |
| | | BB73 | | |
| **253** | (4aR,8aS)-6-[3-[5-(2,4-Dichlorophenyl)-1,3,4-oxadiazol-2-yl]azetidine-1-carbonyl]-4,4a,5,7,8,8a-hexahydropyrido [4,3-b][1,4]oxazin-3-one | BB15a | 452.2 [M+H]⁺ | A4 |
| | | BB88 | | |
| **254** | (4aR,8aS)-6-[3-[3-Fluoro-4-(trifluoromethyl)phenyl]azetidine -1-carbonyl]-4,4a,5,7,8,8a-hexahydropyrido[4,3-b][1,4]oxazin-3-one | BB15a | 402.2 [M+H]⁺ | A4 |
| | | BB79 | | |
| **257** | (4aR,8aS)-6-[3-[1-(2,4-Dichlorophenyl)pyrazol- 3 - yl]azetidine-1-carbonyl]-4,4a,5,7,8,8a-hexahydropyrido[4,3-b][1,4]oxazin-3-one | BB15a | 450.1 [M+H]⁺ | A4 |
| | | BB89 | | |
| **259** | (4aR,8aS)-6-[3-(3,4-Dimethylphenyl)azetidine-1-carbonyl]-4,4a,5,7,8,8a-hexahydropyrido[4,3-b][1,4]oxazin-3-one | BB15a | 344.2 [M+H]⁺ | A4 |
| | | BB146 | | |
| **261** | (4aR,8aS)-6-[3-[4-(2,2-Dimethylpropyl)phenyl]azetidine -1-carbonyl]-4,4a,5,7,8,8a-hexahydropyrido[4,3-b][1,4]oxazin-3-one | BB15a | 386.3 [M+H]⁺ | A4 |
| | | BB90 | | |
| **262** | (4aR,8aS)-6-[3-(4-tert-Butoxyphenyl)azetidine-1-carbonyl]-4,4a,5,7,8,8a-hexahydropyrido[4,3-b][1,4]oxazin-3-one | BB15a | 388.3 [M+H]⁺ | A4 |
| | | BB147 | | |
| **263** | (4aR,8aS)-6-[4-(5-Chloroindolin-1-yl)piperidine-1-carbonyl]-4,4a,5,7,8,8a-hexahydropyrido[4,3-b][1,4]oxazin-3-one | BB15a | 419.2 [M+H]⁺ | A4 |
| | | BB148 | | |
| **264** | (4aR,8aS)-6-[4-(4-Chloroisoindolin-2-yl)piperidine-1-carbonyl]-4,4a,5,7,8,8a-hexahydropyrido[4,3-b][1,4]oxazin-3-one | BB15a | 419.4 [M+H]⁺ | A4 |
| | | BB149 | | |
| **265** | (4aR,8aS)-6-[4-(5'-Chlorospiro[cyclopropane-1,3'-indoline]-1'-yl)piperidine-1-carbonyl]-4,4a,5,7,8,8a-hexahydropyrido[4,3-b][1,4]oxazin-3-one | BB15a | 445.1 [M+H]⁺ | A4 |
| | | BB150 | | |
| **266** | (4aR,8aS)-6-[3-(4-Chloroisoindolin-2-yl)azetidine-1-carbonyl]-4,4a,5,7,8,8a-hexahydropyrido[4,3-b][1,4]oxazin-3-one | BB15a | 391.3 [M+H]⁺ | A4 |
| | | BB151 | | |
| **267** | (4aR,8aS)-6-[4-(5-Chloroisoindolin-2-yl)piperidine-1-carbonyl]-4,4a,5,7,8,8a-hexahydropyrido[4,3-b][1,4]oxazin-3-one | BB15a | 419.3 [M+H]⁺ | A4 |
| | | BB125 | | |
| **268** | (4aS,8aS)-6-[4-[1-[2-[tert-Butyl(dimethyl)silyl]oxyethyl]-5-chloro-indol-3-yl]piperidine-1-carbonyl]-4,4a,5,7,8,8a-hexahydropyrido[4,3-b][1,4]oxazin-3-one | BB13 | 575.3 [M+H]⁺ | A1 |
| | | BB126 | | |
| **269** | (4aS,8aS)-6-[4-[5-Chloro-1-(2-hydroxyethyl)indol-3-yl]piperidine-1-carbonyl]-4,4a,5,7,8,8a-hexahydropyrido[4,3-b][1,4]oxazin-3-one | Example 268 | 461.2 [M+H]⁺ | In analogy to example 25 |
| **270** | (4aR,8aS)-6-(3-(5-(3-(Trifluoromethyl)pyrrolidin-1-yl)pyridin-2-yl)azetidine-1-carbonyl)hexahydro-2H-pyrido[4,3-b][1,4]oxazin-3(4H)-one | Example 181 | 454.3 [M+H]⁺ | A6 |
| | | 3-(Trifluoromethyl)pyrrol idine hydrochloride (CAS RN 1189485-03-7) | | |
| **271** | (4aR,8aS)-6-(3-(5-((R or S)-3-(Trifluoromethyl)pyrrolidin-1-yl)pyridin-2-yl)azetidine-1-carbonyl)hexahydro-2H-pyrido[4,3-b][1,4]oxazin-3(4H)-one | Example 270 | 454.3 [M+H]⁺ | B4 |
| 272 | (4aR,8aS)-6-(3-(5-((S or R)-3-(Trifluoromethyl)pyrrolidin-1-yl)pyridin-2-yl)azetidine-1-carbonyl)hexahydro-2H-pyrido[4,3-b][1,4]oxazin-3(4H)-one | Example 270 | 454.3 [M+H]⁺ | B4 |
| **273** | (4aR,8aS)-6-[3-[6-[3-(Trifluoromethyl)pyrrolidin-1-yl]-3-pyridyl]azetidine-1-carbonyl]-4,4a,5,7,8,8a-hexahydropyrido[4,3-b][1,4]oxazin-3-one | Example 145 | 454.2 [M+H]⁺ | A6 |
| | | 3-(Trifluoromethyl)pyrrol idine hydrochloride (CAS RN 1189485-03-7) | | |
| **274** | (4aR,8aS)-6-[3-[6-[3-(R or S)-(Trifluoromethyl)pyrrolidin-1-yl]-3-pyridyl]azetidine-1-carbonyl]-4,4a,5,7,8,8a-hexahydropyrido[4,3-b][1,4]oxazin-3-one | Example 273 | 454.4 [M+H]⁺ | B4 |
| **275** | (4aR,8aS)-6-[3-[6-[3S- or 3R-(trifluoromethyl)pyrrolidin-1-yl]-3-pyridyl]azetidine-1-carbonyl]-4,4a,5,7,8,8a-hexahydropyrido[4,3-b][1,4]oxazin-3-one | Example 273 | 454.4 [M+H]⁺ | B4 |
| **276** | (4aR,8aS)-6-[3-(4-Tetrahydropyran-3-ylphenyl)azetidine-1-carbonyl]-4,4a,5,7,8,8a-hexahydropyrido[4,3-b][1,4]oxazin-3-one | Example 110 | 400.2 [M+H]⁺ | A7 |
| | | 3-Bromotetrahydro-2H-pyran (CAS RN 13047-01-3) | | |
| **277** | (4aR,8aS)-6-[3-[2-Methoxy-4-(2,2,2-trifluoroethyl)phenyl] azetidine-1 - carbonyl]-4,4a,5,7,8,8a-hexahydropyrido [4,3-b][1,4]oxazin-3-one | BB15a | 428.2 [M+H]⁺ | A4 |
| | | BB152 | | |
| **278** | (4aR,8aS)-6-(3-(4-(Neopentyloxy)phenyl)azetidine-1-carbonyl)hexahydro-2H-pyrido[4,3-b][1,4]oxazin-3(4H)-one | BB15a | 402.2 [M+H]⁺ | A4 |
| | | BB153 | | |

### Syntheses of Building Blocks

### BB1

### 4-Nitrophenyl 4-benzhydrylpiperidine-1-carboxylate

To a solution of 4-benzhydrylpiperidine (50 mg, 199 µmol, CAS RN 19841-73-7) in DCM (1.2 mL), TEA (40.3 mg, 55.4 µL, 398 µmol) was added. On cooling to 0 °C, 4-nitrophenyl carbonochloridate (44.1 mg, 219 pmol, CAS RN 7693-46-1) was added, the reaction mixture was allowed to warm to RT and stirred for 18 hours. The reaction mixture was diluted with DCM and subsequently washed with H₂O and sat. aqueous NaHCO₃ solution, the combined organic layers were washed with brine, dried over Na₂SO₄, filtered and concentrated in vacuo. The crude material was purified by flash chromatography (silica gel 10 g, eluting with EtOAc/n-heptane 0-50 %), to afford the title compound as a light yellow solid (75 mg, 90.5%). MS (ESI): m/z = 417.4 [M+H]⁺.

### BB2

### 5-Chloro-1-(cyclopropylmethyl)-3-(piperidin-4-yl)-1H-indole (FA salt)

A mixture of tert-butyl 4-[5-chloro-1-(cyclopropylmethyl)indol-3-yl]piperidine-1-carboxylate (400.0 mg, 1.03 mmol) in HCl/EtOAc (4M, 15.0 mL) was stirred at 20°C for 15 hours. The mixture was concentrated in vacuum and the residue was suspended in H2O (5 mL). The pH was adjusted to pH=7 with ammonia, then purified by prep-HPLC (0.1% FA in H₂O and ACN) to afford the title compound as a white solid (215.9 mg, 62.6%). MS (ESI): m/z = 289.1 [M+H]⁺.

### Intermediate: tert-Butyl4-(5-chloro-1-(cyclopropylmethyl)-1H-indol-3-yl)piperidine-1-carboxylate

A mixture of (bromomethyl)cyclopropane (0.17 mL, 1.79 mmol; CAS RN 7051-34-5), tert-butyl 4-(5-chloro-1H-indol-3-yl)piperidine-1-carboxylate (500.0 mg, 1.49 mmol, BB9, intermediate step b), 15-crown-5 (0.06 mL, 0.300 mmol; CAS RN 33100-27-5) and sodium tert-butoxide (287.01 mg, 2.99 mmol; CAS RN 865-48-5) in anhydrous EtOH (5 mL) was stirred at 25°C for 12 hours. The mixture was diluted with H₂O (20 mL), extracted three times with EtOAc (40 mL each) and concentrated under vacuum. The residue was purified by flash silica gel column chromatography (100-200 mesh) using a gradient of PE : EtOAc (50 : 1 to 20 : 1) to afford the title compound as a light yellow solid (400 mg, 68.8%). MS (ESI): m/z = 411.1 [M+Na]⁺.

### BB3

### 5-Chloro-1-methyl-3-(piperidin-4-yl)-1H-indole

A mixture of tert-butyl 4-(5-chloro-1-methyl-indol-3-yl)piperidine-1-carboxylate (480.0 mg, 1.38 mmol) in HCl/EtOAc (20.0 mL, 6M) was stirred at 20°C for 15 hours. The mixture was concentrated under vacuum, adjusted to pH~7 using aqueous ammonia, and then concentrated to give a residue which was purified by prep-HPLC to afford the title compound as a colorless powder (172.3 mg, 49.3%). MS (ESI): m/z = 249.2 [M+H]⁺.

### Intermediate: tert-Butyl 4-(5-chloro-1-methyl-indol-3-yl)piperidine-1-carboxylate

To a mixture of NaH 60% in mineral oil (119.46 mg, 2.99 mmol) in THF (20 mL) was added a solution of tert-butyl 4-(5-chloro-1H-indol-3-yl)piperidine-1-carboxylate (500.0 mg, 1.49 mmol, BB9, intermediate step b) in THF (5 mL) at 0°C. The mixture was stirred at 0°C for 0.5 hours, then MeI (211.95 mg, 1.49 mmol) were added. The mixture was stirred at 0°C for 1 hour, poured into H₂O (5 mL) and extracted with EtOAc (20 mL × 3). The combined organic layers were washed with brine (10 mL), dried over Na₂SO₄, filtered and concentrated to afford the title compound as light yellow solid (480 mg, 92.1%). MS (ESI): m/z = 349.1 [M-Boc+H]⁺.

### BB4

### 4-(9H-Fluoren-9-yl)piperidine

To a solution of 4-(9H-fluoren-9-yl)pyridine (70 mg, 288 µmol) in AcOH (1 mL) was added platinum(IV) oxide (10 mg, 44 µmol). The reaction mixture was stirred overnight under a hydrogen atmosphere of 20 bar at 40°C. The reaction mixture was filtered, and the filtrate was washed with AcOH. The thus obtained solution was concentrated in vacuo. The residue was dissolved in DCM, and the DCM was washed three times with aqueous saturated NaHCO₃ solution. The organic layer was dried over MgSO₄, filtered, and concentrated to give the desired product. (0.060 g; 83%). MS (ESI): m/z = 250.2 [M+H]⁺.

### Intermediates:

### a) 4-(9H-Fluoren-9-yl)pyridine

To a solution of diphenyl(4-pyridyl)methanol (135 mg, 517 µmol) in FA (2 mL) was added sulfuric acid (0,8 mL) dropwise. The mixture was heated to 100 °C for 15 minutes, cooled to RT and poured into 20 mL aqueous 5N NaOH solution, which caused the product to precipitate. The product was collected by filtration, to give the product as a light yellow solid (75 mg, 60%). MS (ESI): m/z = 244.2 [M+H]⁺.

### b) Diphenyl(4-pyridyl)methanol

To a solution of phenyl(pyridin-4-yl)methanone (0.52 g, 2.84 mmol, CAS RN 14548-46-0) in THF (10 mL) was added dropwise phenylmagnesium bromide 1M solution in THF (9 mL, 9 mmol, CAS RN 100-58-3) at RT for 2 hours. The reaction mixture was poured on saturated aqueous NH₄Cl solution and EtOAc and the layers were separated. The aqueous layer was extracted twice with EtOAc. The organic layers were washed with brine, dried over MgSO₄, filtered, treated with silica gel and evaporated. The compound was purified by silica gel chromatography on a 4 g column using an MPLC system eluting with a gradient of DCM : MeOH (100 : 0 to 90 : 10) to yield the desired compound as a colorless solid (0.290 g; 39.0%). MS (ESI): m/z = 262.2 [M+H]⁺.

### BBS

### 4-[1-(4-Fluorophenyl)-3-methoxy-propyl]piperidine

To a solution of tert-butyl 4-(1-(4-fluorophenyl)-3-methoxy-propyl)piperidine-1-carboxylate (95 mg, 270 µmol) in dioxane (0.3 mL) was added HCl 4M in dioxane (338 µL, 1.35 mmol) and the mixture was stirred at RT for 2 h. The light yellow solution was completely evaporated. The residue was taken up in saturated aqueous NaHCO₃ solution and DCM and the layers were separated. The aqueous layer was extracted twice with DCM. The organic layers were dried over MgSO₄, filtered and evaporated to yield the desired compound as a light yellow oil (0.057 g; 83.9%). MS (ESI): m/z = 252.3 [M+H]⁺.

### Intermediates:

### a) tert-Butyl 4-[1-(4fluorophenyl)-3-methoxy-propyl]piperidine-1-carboxylate

To an ice-cold solution of tert-butyl 4-(1-(4-fluorophenyl)-3-hydroxy-propyl)piperidine-1-carboxylate (95 mg, 282 µmol) in THF (1 mL) was added NaH 55% in mineral oil (13.5 mg, 310 µmol) and the mixture was stirred at this temperature for 30 minutes before iodomethane (47.9 mg, 21.1 µL, 337 µmol) was added. After stirring at this temperature for 30 minutes, the turbid solution was allowed to warm up to RT. After stirring at RT for 4 hours, the reaction mixture was poured on H₂O and EtOAc and the layers were separated. The aqueous layer was extracted twice with EtOAc. The organic layers were dried over MgSO₄, filtered, and evaporated to get the desired compound as a light yellow oil (0.097 g; 98.0%). MS (ESI): m/z = 252 [M-Boc+H]⁺.

### b) tert-Butyl 4-[1-(4-fluorophenyl)-3-hydroxy-propyl]piperidine-1-carboxylate

To a solution of tert-butyl 4-[3-ethoxy-1-(4-fluorophenyl)-3-oxo-propyl]piperidine-1-carboxylate (148 mg, 390 µmol) in THF (1.5 mL) at 0°C was added in one portion lithium borohydride (21.2 mg, 975 µmol) and the mixture was stirred in an ice-bath for 1.25 hours. The ice-bath was removed and stirring continued at RT for 20 hours. The reaction mixture was poured on saturated aqueous NH₄Cl solution and EtOAc and the layers were separated. The aqueous layer was extracted twice with EtOAc. The organic layers were dried over MgSO₄, filtered, treated with silica gel and evaporated. The compound was purified by silica gel chromatography on a 4 g column using an MPLC system eluting with a gradient of n-heptane : EtOAc (100 : 0 to 0 : 100) to yield the desired compound as a colorless oil (0.097 g; 73.7%). MS (ESI): m/z = 282.3 [M-C₄H₈+H]⁺.

### c) tert-Butyl 4-[3-ethoxy-1-(4fluorophenyl)-3-oxo-propyl]piperidine-1-carboxylate

To a solution of tert-butyl (E)and (Z)-4-(3-ethoxy-1-(4-fluorophenyl)-3-oxoprop-1-en-1-yl)piperidine-1-carboxylate (1.74 g, 4.63 mmol) in EtOAc (17.1 mL) was added. Pd/C 10% (175 mg) and the suspension was stirred at RT under an hydrogen atmosphere at 1.5 bar for 4 hours. The reaction mixture was filtered over a microfilter. The compound was purified by silica gel chromatography on a 24 g column using an MPLC system eluting with a gradient of n-heptane : EtOAc (100 : 0 to 50 : 50) to get the desired compound as a colorless liquid (1.23 g; 69.8%). MS (ESI): m/z = 280 [M-Boc+H]⁺.

### d) tert-Butyl 4-[(E) and (Z)-3-ethoxy-1-(4fluorophenyl)-3-oxo-prop-1-enyl)piperidine-1-carboxylate

To a solution of ethyl 2-(diethoxyphosphoryl)acetate (2.1 g, 1.86 mL, 9.36 mmol, cas RN 867-13-0) in 1,4-dioxane (9.73 mL) was added dropwise LiHMDS 1.0 M in hexanes (15 mL, 15 mmol) and the solution was stirred for 15 minutes. A solution of tert-butyl 4-(4-fluorobenzoyl)piperidine-1-carboxylate (2.878 g, 9.36 mmol, CAS RN 160296-40-2) in 1,4-dioxane (9.73 mL) was added dropwise to the mixture and stirring was continued at reflux over two days. The reaction mixture was poured on saturated aqueous NH₄Cl solution and EtOAc and the layers were separated. The aqueous layer was extracted twice with EtOAc. The combined organic layers were washed once with H2O, dried over MgSO₄, filtered, treated with silica gel and evaporated. The compound was purified by silica gel chromatography on a 40 g column using an MPLC system eluting with a gradient of n-heptane : EtOAc (100 : 0 to 50 : 50) to yield the desired compound as a colorless liquid (1.74 g; 49.4%). MS (ESI): m/z = 278.2 [M-Boc+H]⁺.

### BB6

### 4-[(4-Fluorophenyl)-methoxy-methyl]piperidine

To a solution of tert-butyl 4-[(4-fluorophenyl)-methoxy-methyl]piperidine-1-carboxylate (57 mg, 176 µmol) in dioxane (200 µL) was added HCl 4M in dioxane (220 µL, 880 µmol) and the mixture was stirred at RT for 2 hours. The light yellow solution was completly evaporated. The residue was taken up in saturated aqueous NaHCO₃ solution and DCM and the layers were separated. The aqueous layer was extracted twice with DCM. The combined organic layers were dried over MgSO₄, filtered and evaporated to yield the desired compound as a light yellow oil (0.036 g; 91.5%). MS (ESI): m/z = 224.2 [M+H]⁺.

### Intermediates:

### a) tert-Butyl 4-[(4-fluorophenyl)-methoxy-methyl]piperidine-1-carboxylate

To an ice-cold solution of tert-butyl 4-((4-fluorophenyl)(hydroxy)methyl)piperidine-1-carboxylate (60 mg, 194 µmol, CAS RN160296-41-3 ) in THF (1 mL) was added NaH 55% in mineral oil (9.31 mg, 213 µmol) and the mixture was stirred at this temperature for 30 minutes before iodomethane (33.1 mg, 14.6 µL, 233 µmol) was added. After stirring at this temperature for 30 minutes, the turbid solution was allowed to warm up to RT. After stirring for 2 hours at RT another batch of iodomethane (33 mg, 14.6 µL, 233 µmol) was added. After 4.5 hours, the reaction mixture was poured on H2O and EtOAc and the layers were separated. The aqueous layer was extracted twice with EtOAc. The combined organic layers were dried over MgSO₄, filtered and evaporated to get the desired compound as a light yellow oil (0.057 g; 90.9%). MS (ESI): m/z = 268.2 [M-C₄H₈+H]⁺.

### BB7

### 1-Methyl-5-(4-piperidyl)indazole hydrochloride

To a solution of tert-butyl 4-(1-methyl-1H-indazol-5-yl)piperidine-1-carboxylate (155 mg, 491 µmol) in dioxane (0.5 mL) was added HCl in 4M in dioxan (614 µL, 2.46 mmol). The colorless suspension was stirred at RT for 3.5 hours. Ether (4 mL) was added and the suspension was filtered. The filter cake was washed with ether to get the desired product as a light yellow solid (0.107 g; 86.5%). MS (ESI): m/z = 216.2 [M+-HCl+H]⁺.

### Intermediates:

### a) tert-Butyl 4-(1-methylindazol-5-yl)piperidine-1-carboxylate

To a solution of tert-butyl 4-(1-methyl-1H-indazol-5-yl)-3,6-dihydropyridine-1(2H)-carboxylate (160 mg, 511 µmol) in MeOH (3 mL) and EtOAc (1 mL) was added under argon Pd-C 10% (16.3 mg, 15.3 µmol) and the suspension was stirred under a hydrogen atmosphere at 1.7 bar for 2.5 hours. The solids were filtered off and the filtrate was evaporated to give the desired compound as a colorles oil (0.156 g; 96.9%). MS (ESI): m/z = 316.3 [M+H]⁺.

### b) tert-Butyl 4-(1-methyl-1H-indazol-5-yl)-3, 6-dihydropyridine-1(2H)-carboxylate

To a mixture of tert-butyl 4-(((trifluoromethyl)sulfonyl)oxy)-3,6-dihydropyridine-1(2H)-carboxylate (200 mg, 604 pmol, CAS RN 138647-49-1) in DME (3 mL) and 2M aqueous Na₂CO₃ (770 µL, 1.54 mmol) were added palladium (II) acetate (2.71 mg, 12.1 µmol), triphenylphosphine (7.92 mg, 30.2 µmol) and 1-methyl-5-(4,4,5,5-tetramethyl-1,3,2-dioxaborolan-2-yl)-1H-indazole (171 mg, 664 pmol, CAS RN 1235469-00-7) and the clear two-layer mixture was heated to 90°C for 2 hours. The reaction mixture was poured on H2O and EtOAc and the layers were separated. The aqueous layer was extracted twice with EtOAc. The combined organic layers were dried over MgSO₄, filtered, treated with silica gel and evaporated. The compound was purified by silica gel chromatography on a 12 g column using an MPLC system eluting with a gradient of n-heptane : EtOAc (100 : 0 to 00 : 100) to yield the desired compound as a colorless solid (0.163 g; 86.2%). MS (ESI): m/z = 314.3 [M+H]⁺.

### BB8

### (E or Z)-2-Phenyl-3-piperazin-1-yl-prop-2-enenitrile hydrochloride

(E or Z)-*tert*-Butyl 4-[2-cyano-2-phenyl-vinyl]piperazine-1-carboxylate (300 mg, 951 µmol) was treated with a 4 M HCl solution in dioxane (3.6 g, 3 mL, 98.7 mmol). The reaction mixture was stirred at RT for 15h, then concentrated in vacuo to give a light yellow solid (240 mg, 100%); MS (ESI): m/z = 214.2 [M+H]⁺.

### Intermediate: (E or Z)-tert-Butyl 4-[2-cyano-2-phenyl-vinyl]piperazine-1-carboxylate

tert-Butyl piperazine-1-carboxylate (200 mg, 1.07 mmol, CAS RN 57260-71-6), 3-oxo-2-phenylpropanenitrile (156 mg, 1.07 mmol, CAS RN 5841-70-3) and sodium triacetoxyborohydride (228 mg, 1.07 mmol, CAS RN 56553-60-7) were disolved in DCM (5 mL). The reaction mixture was stirred at RT for 48 hours. The reaction mixture was washed with H₂O, NaHCO₃ and brine. The organic phase was dried over Na₂SO₄, filtered and the filtrate concentrated in vacuo to give a brown solid (300 mg). MS (ESI); m/z = 314.2 [M+H]⁺.

### BB9

### 5-Chloro-1-cyclopropyl-3-(4-piperidyl)indole hydrochloride

To a mixture of tert-butyl 4-(5-chloro-1-cyclopropyl-1H-indol-3-yl)piperidine-1-carboxylate (275.0 mg, 0.730 mmol) in EtOAc (5 mL) was added HCl/EtOAc (4M, 4 mL), and the mixture was stirred at 20°C for 3 hours. The mixture was concentrated and the residue was diluted with H₂O (30 mL), washed with EtOAc (10 mL × 2). The aqueous layer was lyophilized to afford the title compound as light yellow solid (189.4 mg, 81.3%). ¹H NMR (400 MHz, DMSO-d6) δ 9.29 - 8.87 (m, 2H), 7.76 (d, J = 1.9 Hz, 1H), 7.53 (d, J = 8.7 Hz, 1H), 7.23 - 7.10 (m, 2H), 3.38 - 3.27 (m, 3H), 3.08 - 2.90 (m, 3H), 2.07 - 1.98 (m, 2H), 1.98 - 1.83 (m, 2H), 1.09 - 1.00 (m, 2H), 0.96 - 0.88 (m, 2H). MS (ESI): m/z = 275.1 [M+H]⁺.

### Intermediates:

### a) tert-Butyl 4-(5-chloro-1-cyclopropyl-1H-indol-3-yl)piperidine-1-carboxylate

A mixture of tert-butyl 4-(5-chloro-1H-indol-3-yl)piperidine-1-carboxylate (500 mg, 1.49 mmol), cyclopropylboronic acid (153.92 mg, 1.79 mmol; CAS RN 411235-57-9), 1M NaHMDS solution in THF (3 mL, 2.99 mmol; CAS RN 1070-89-9), DMAP (0.22 mL, 1.79 mmol) and copper(II) acetate (270.22 mg, 1.49 mmol; CAS RN 142-71-2) in toluene (25 mL) were stirred at 95°C for 12 h (bearing an oxygen balloon). The mixture was poured into H₂O (20 mL) and extracted with EtOAc (50 mL × 3). The combined organic layers were washed with brine (50 mL), dried over Na₂SO₄, filtered and concentrated in vacuum. The residue was purified by flash silica gel column chromatography (100-200 mesh) using a gradient of PE : EtOAc (50 : 1 to 20 : 1) to afford the title compound as light yellow oil (275 mg, 0.730 mmol, 49.1%). MS (ESI): m/z = 275.1 [M-Boc+H]⁺.

### b) tert-Butyl 4-(5-chloro-1H-indol-3-yl)piperidine-1-carboxylate

A mixture of tert-butyl 4-(5-chloro-1H-indol-3-yl)-3,6-dihydro-2H-pyridine-1-carboxylate (1.003 g, 3.01 mmol) and platinum(IV) oxide (100.0 mg, 0.350 mmol; CAS RN 1314-15-4 ) in AcOH (10.0 mL) and EtOH (20 mL) was stirred at 40°C for 15 h under H₂ (1520 mm Hg). The mixture was filtered and the filtrated was concentrated under vacuum. The residue was triturated with PE (50 mL) and filtered. The filter cake was collected and dried under vacuum to afford the title compound as light grey solid (780 mg, 77.3%). MS (ESI): m/z = 235.1 [M-Boc+H]⁺.

### c) tert-Butyl 4-(5-chloro-1H-indol-3-yl)-3,6-dihydro-2H-pyridine-1-carboxylate

To a mixture of 5-chloroindole (1 g, 6.6 mmol; CAS RN 17422-32-1) in MeOH (20 mL) was added tert-butyl 4-oxopiperidine-1-carboxylate (1.97 g, 9.9 mmol; CAS RN 79099-07-3). The mixture was stirred at 20°C for 1 hours, and then potassium hydroxide (1.48 g, 26.39 mmol) was added. The mixture was stirred at 70°C for 15 h. The mixture was concentrated under vacuum. The residue was triturated with a mixture of PE and EtOAc (5:1, 50 mL) and filtered. The filter cake was collected and dried under vacuum to afford the title compound as light yellow solid (2 g, 91.3%). MS (ESI): m/z = 227.1 [M-C₄H₈+H]⁺.

### BB10

### 5-Chloro-1-(oxetan-3-yl)-3-(4-piperidyl)indole

To a mixture of tert-butyl 4-[5-chloro-1-(oxetan-3-yl)-1H-indol-3-yl]piperidine-1-carboxylate (1 g, 2.56 mmol) in DCM (20 mL) was added TFA (5.0 mL). The mixture was stirred at 20°C for 12 hours. The mixture was concentrated in vacuum, purified by prep-HPLC (0.1% FA in H₂O and ACN) to afford the title compound as light yellow solid (311.3 mg, 37.3%). ¹H NMR (400 MHz, DMSO-d6) δ 7.76 (d, J = 1.8 Hz, 1H), 7.65 (s, 1H), 7.57 (d, J = 8.8 Hz, 1H), 7.17 (dd, J = 1.9, 8.7 Hz, 1H), 5.73 (quin, J = 7.0 Hz, 1H), 5.01 (t, J = 7.2 Hz, 2H), 4.90 (t, J = 6.5 Hz, 2H), 3.38 (br d, J = 12.2 Hz, 2H), 3.14 - 3.00 (m, 3H), 2.07 (br d, J = 13.2 Hz, 2H), 1.96 - 1.78 (m, 2H). MS (ESI): m/z = 291.1 [M+H]⁺.

### Intermediate: tert-Butyl 4-(5-chloro-1-(oxetan-3-yl)-1H-indol-3-yl)piperidine-1-carboxylate

To a mixture of tert-butyl 4-(5-chloro-1H-indol-3-yl)piperidine-1-carboxylate (1 g, 2.99 mmol, BB9, intermediate step b) in DMF (20 mL) was added NaH 60% in mineral oil (143.35 mg, 3.58 mmol) at 0°C. The mixture was stirred at 20°C for 1 hour, and then oxetan-3-yl 4-methylbenzenesulfonate (1022.55 mg, 4.48 mmol; CAS RN 26156-48-9) was added and the mixture was stirred at 85°C for 12 hours. The mixture was poured into H₂O (60 mL) and extracted with EtOAc (50 mL x 3). The combined organic layers were washed with brine (50 mL), dried over Na₂SO₄ and concentrated over vacuum to afford the title compound as light yellow solid (1.1 g, 2.81 mmol, 94.2%). MS (ESI): m/z = 291.1 [M-Boc+H]⁺.

### BB11

### 4-(bis(4-Fluorophenyl)methyl)piperidine formic acid salt

To a solution of 4-[bis(4-Fluorophenyl)methylene]piperidine (700.0 mg, 1.75 mmol) in AcOH (50.0 mL) was added Pd/C (1.0 g), the reaction was purged with hydrogen for three times and stirred at 90°C (under hydrogen balloon) for 48 hours. The reaction mixture was filtered and concentrated in vacuum. The crude product was purified by prep-HPLC (0.1% FA in water and MeCN) to afford the title compound as a light grey solid (263.5 mg, 32.1%). ¹H NMR (DMSO-d6, 400MHz,) δ 8.39 (s, 1H), 7.45 - 7.37 (m, 4H), 7.15 - 7.07 (m, 4H), 3.68 (d, J = 11.2 Hz, 1H), 3.17 (br d, J = 12.3 Hz, 2H), 2.80 - 2.68 (m, 1H), 2.46 (br d, J = 11.2 Hz, 1H), 1.54 - 1.32 (m, 2H), 1.27 - 1.09 (m, 2H). MS (ESI): m/z = 288.1 [M+H]⁺.

### Intermediates:

### a) 4-[bis(4-Fluorophenyl)methylene]piperidine

To a solution of bis(4-fluorophenyl)(piperidin-4-yl)methanol (900.0 mg, 2.59 mmol) in DCM (30 mL) was added TFA (10.0 mL) and the reaction mixture was stirred at 20°C for 12 hours. The reaction mixture was concentrated in vacuum to afford the crude title compound which was used in the next step without further purification. MS (ESI): m/z = 286.1 [M+H]⁺.

### b) bis(4-Fluorophenyl)(piperidin-4-yl)methanol

A mixture of Mg (1.4 g, 58.29 mmol) and I₂ (20.0 mg, 0.080 mmol) were suspended in THF (40 mL) at 10 °C. To the above yellow solution was added 1-bromo-4-fluoro benzene (1.0 g, 5.75 mmol, CAS RN 460-00-4), and the reaction mixture was heated to 45 °C until the solution became clear. Another batch of 1-bromo-4-fluoro benzene (9.14 g, 52.54 mmol, CAS RN 460-00-4) (dissolved in 10 mL of THF) was added in. The reaction mixture was stirred at 45 °C for 30 mins. Ethyl N-BOC-piperidine-4-carboxylate (1.5 g, 5.83 mmol, CAS RN 142851-03-4) was added and the reaction mixture was stirred at 80 °C for 12 hours. The mixture was poured into sat. NH₄Cl aq. solution (50 mL), the mixture was extracted twice with EtOAc (50 mLeach), the combined organic layer was washed with water (30 mL x 2) and brine (30 mL), dried over Na₂SO₄ and concentrated in vacuum to afford the title compound (900 mg, 38.2%). 1H NMR (400 MHz, DMSO-d6) δ 8.70 (br s, 1H), 7.57 - 7.47 (m, 4H), 7.12 (t, J = 8.8 Hz, 4H), 5.73 (s, 1H), 3.23 (br d, J = 12.3 Hz, 2H), 2.92 - 2.79 (m, 3H), 1.68 - 1.54 (m, 2H), 1.39 (br d, J = 13.6 Hz, 2H). MS (ESI): m/z = 304.1 [M+H]⁺.

### BB12

### 5-Chloro-1-(oxetan-3-ylmethyl)-3-(4-piperidyl)indole

To a solution of tert-butyl 4-[5-chloro-1-(oxetan-3-ylmethyl)indol-3-yl]piperidine-1-carboxylate (700.0 mg, 1.73 mmol) in DCM (20 mL) was added TFA (3.38 mL). The mixture was stirred at 20°C for 12 h. The mixture was concentrated under vacuum, purified by prep-HPLC (0.1% FA in H2O and ACN) to afford the title compound as light yellow solid (154.57 mg, 29.0%). ¹H NMR (400 MHz, DMSO-d6) δ 8.60 (br s, 1H), 7.71 (d, J = 1.7 Hz, 1H), 7.54 (d, J = 8.8 Hz, 1H), 7.33 (s, 1H), 7.13 (dd, J = 1.8, 8.7 Hz, 1H), 4.59 (dd, J = 6.2, 7.6 Hz, 2H), 4.47 - 4.33 (m, 4H), 3.43 - 3.37 (m, 3H), 3.08 - 2.99 (m, 3H), 2.04 (d, J = 13.0 Hz, 2H), 1.89 - 1.76 (m, 2H). MS (ESI): m/z = 305.2 [M+H]⁺.

### Intermediates:

### a) tert-Butyl 4-[5-chloro-1-(oxetan-3-ylmethyl)indol-3-yl]piperidine-1-carboxylate

To a solution of tert-butyl 4-(5-chloro-1H-indol-3-yl)piperidine-1-carboxylate (400.0 mg, 1.19 mmol, BB9, intermediate step b) in DMF (8 mL) was added NaH 60% in mineral oil (57.34 mg, 2.39 mmol) at 0°C. The mixture was stirred at 20°C for 1 hour. Then oxetan-3-ylmethyl 4-methylbenzenesulfonate (434.16 mg, 1.79 mmol) was added and the mixture was stirred at 85°C for 12 hours. The mixture was poured into H₂O (60 mL) and extracted with EtOAc (50 mL x 3). The combined organic layers were washed with brine (50 mL), dried over Na₂SO₄ and concentrated in vacuum to afford the title compound as yellow oil (629 mg, crude). MS (ESI): m/z = 305.0 [M-Boc+H]⁺.

### b) Oxetan-3-ylmethyl 4-methylbenzenesulfonate

To a solution of 3-oxetanemethanol (500.0 mg, 5.67 mmol; CAS RN 6246-06-6) in DCM (10 mL) was added TEA (1.19 mL, 8.51 mmol), DMAP (69.33 mg, 0.570 mmol) and 4-methylbenzene-1-sulfonyl chloride (1293.77 mg, 6.81 mmol; CAS RN 98-59-9). The mixture was stirred at 20°C for 6 hours. The mixture was diluted with a mixture of EtOAc and saturated aqueous NH₄Cl solution (1:1, 20 mL). The mixture was extracted with EtOAc (40 mL × 3). The combined organic layers were washed with brine, dried over Na₂SO₄, filtered and concentrated to afford the title compound as brown solid (1.25 g, 5.16 mmol, 78.7%). MS (ESI): m/z = 243.1 [M+H]⁺.

### BB13

### 1-(2-((tert-Butyldimethylsilyl)oxy)ethyl)-5-chloro-3-(piperidin-4-yl)-1H-indole

To a solution of 2-[5-chloro-3-(4-piperidyl)indol-1-yl]ethanol (600.0 mg, 1.39 mmol) in pyridine (5 mL) was added t-butyldimethylchlorosilane (251.35 mg, 1.67 mmol; CAS RN 18162-48-6) and the mixture was stirred at 20°C for 12 hours. The mixture was poured into H₂O (10 mL) and extracted with EtOAc (20 mL × 3). The combined organic layers were washed with brine (10 mL), dried over Na₂SO₄, filtered and concentrated. The residue was triturated with PE (10 mL) and filtered. The filter cake was washed with PE (5 mL × 2 the solid collected and dried in vacuum to afford the title compound as light yellow solid (164.7 mg, 29.8%). ¹H NMR (400 MHz, DMSO-d6) δ 8.97 (br s, 1H), 7.72 (d, J = 2.0 Hz, 1H), 7.47 (d, J = 8.7 Hz, 1H), 7.20 (s, 1H), 7.10 (dd, J = 1.8, 8.7 Hz, 1H), 4.22 (br t, J = 4.8 Hz, 2H), 3.84 (t, J = 4.9 Hz, 2H), 3.44 - 3.32 (m, 2H), 3.12 - 2.94 (m, 3H), 2.08 - 1.96 (m, 2H), 1.95 - 1.81 (m, 2H), 0.73 (s, 9H), -0.24 (s, 6H). MS (ESI): m/z = 393.1 [M+H]⁺.

### Intermediates:

### a) 2-[5-Chloro-3-(4-piperidyl)indol-1-yl)ethanol

To a mixture of tert-butyl 4-[5-chloro-1-(2-hydroxyethyl)indol-3-yl]piperidine-1-carboxylate (1.1 g, 2.9 mmol) in EtOAc (10 mL) was added HCl/ETOAC (4M, 0.73 mL). The mixture was stirred at 20°C for 3 hours and then concentrated in vacuum. The residue was diluted with DCM (20 mL) and washed with saturated aqueous NaHCO₃ solution (10 mL). The aqueous layer was extracted with DCM : MeOH (5 : 1 v/v; 20 mL × 3). The combined organic layers were dried over Na₂SO₄ and filtered. The filtrate was concentrated in vacuum to afford the title compound as light yellow oil (750 mg, 59.8%). MS (ESI): m/z = 279.1 [M+H]⁺.

### b) tert-Butyl 4-[5-chloro-1-(2-hydroxyethyl)indol-3-yl]piperidine-1-carboxylate

A mixture of tert-butyl 4-(5-chloro-1H-indol-3-yl)piperidine-1-carboxylate (1 g, 2.99 mmol, intermediate BB9, step b), 2-bromoethanol (0.32 mL, 4.48 mmol; CAS RN 540-51-2) and potassium hydroxide (335.12 mg, 5.97 mmol) in DMF (10 mL) was stirred at 100°C for 12 hours. The mixture was purified by prep-HPLC (0.1% ammonia in H2O and ACN) to afford the title compound as light yellow oil (1.1 g, 97.2%). MS (ESI): m/z = 323.1 [M-C₄H₈+H]⁺.

### BB14a & BB14b

### (+)-4a,5,6,7,8,8a-Hexahydro-4H-pyrido[4,3-b][1,4]oxazin-3-one and

### (-)-cis-4a,5,6,7,8,8a-Hexahydro-4H-pyrido[4,3-b][1,4]oxazin-3-one

The enantiomers of rac-(4aR,8aS)-hexahydro-2H-pyrido[4,3-b][1,4]oxazin-3(4H)-one dihydrochloride (500 mg, 2.18 mmol, ChemBridge Corporation) were separated by preparative chiral HPLC (ReprosilChiral NR column) using an isocratic mixture of EtOH (containing 0.05% of NH₄OAc) : n-heptane (30 : 70).

First eluting enantiomer: (+)-cis-4a,5,6,7,8,8a-Hexahydro-4H-pyrido[4,3-b][1,4]oxazin-3-one. Yellow solid (0.150 g; 44.0%). MS (ESI): m/z = 157.1 [M+H]⁺.

Second eluting enantiomer: (-)-cis-4a,5,6,7,8,8a-Hexahydro-4H-pyrido[4,3-b][1,4]oxazin-3-one. Yellow solid (0.152 g; 44.6%). MS (ESI): m/z = 157.1 [M+H]⁺.

### BB15a and BB15b

### 4-Nitrophenyl (4aR,8aS)-3-oxohexahydro-2H-pyrido[4,3-b][1,4]oxazine-6(5H)-carboxylate (BB15a) and

### 4-Nitrophenyl (4aS,8aR)-3-oxohexahydro-2H-pyrido[4,3-b][1,4]oxazine-6(5H)-carboxylate (BB15b)

To a suspension of rac-(4aR,8aS)-hexahydro-2H-pyrido[4,3-b][1,4]oxazin-3(4H)-one; dihydrochloride salt (4.5 g, 19.6 mmol, BB1) in dry DCM (125 mL) at 0 °C was added DIPEA (6.35 g, 8.58 mL, 49.1 mmol) followed by 4-nitrophenyl carbonochloridate (4.35 g, 21.6 mmol). The reaction mixture was stirred at 0 °C for 10 min and at RT for 2 h. The crude reaction was diluted with DCM and transferred into a separating funnel for extraction with sat. aq. Na₂CO₃ solution. The organic phase was collected and the aqueous phase was back-extracted with DCM. The combined organic phases were dried over Na₂SO₄ and evaporated down to dryness to yield 6.62 g of a crude racemic product (BB7) as a yellow solid. The crude material was directly submitted for a chiral SFC separation to yield enantiomer BB15b (2.72 g, second eluting enantiomer) as a yellow solid and enantiomer BB15a (3.25 g, first eluting enantiomer) as a light beige solid but contaminated with BB15b. A further SFC chiral separation was carried out to yield 2.71 g of BB15a. MS (ESI): m/z = 322.2 [M+H]⁺ for both enantiomers.

### BB16

### 3-(4-Fluorophenyl)-3-(piperidin-4-yl)propan-1-ol hydrochloride

A solution of tert-butyl 4-(1-(4-fluorophenyl)-3-hydroxypropyl)piperidine-1-carboxylate (119 mg, 353 µmol, BB5, intermediate b) in HCl 2M in diethyl ether (1.76 mL, 3.52 mmol) was stirred at RT for 4.5 h before another batch of HCl 2M in diethyl ether (1.76 mL, 3.53 mmol) was added. Stirring was continued at RT overnight. The suspension was homogenized in an ice-cold ultrasonic and filtered. The filter cake was washed with a small volume of diethyl ether to get the desired compound as a colorless solid (0.067 g; 69.4%). MS (ESI): m/z = 238.2 [M+H]⁺.

### BB17

### 4-[1-[4-(Trifluoromethyl)phenyl]ethyl]piperidine formate

A solution of tert-butyl 4-[1-[4-(trifluoromethyl)phenyl]ethyl]piperidine-1-carboxylate (1.5 g, 4.2 mmol) in a mixture of HCl/dioxane (50.0 mL, 1/1 v/v) was stirred at 20 °C for 2 h. The mixture was concentrated and the residue was purified by prep-HPLC (FA) and lyophilized to get the desired compound as light yellow oil (838.4 mg, 77.1%). MS (ESI): m/z = 258.1 [M+H]⁺.

### Intermediates:

### a) tert-Butyl 4-[1-[4-(trifluoromethyl)phenyl]ethyl]piperidine-1-carboxylate

To a solution of tert-butyl 4-[1-[4-(trifluoromethyl)phenyl]vinyl]piperidine-1-carboxylate (2.0 g, 5.63 mmol) in EtOAc (100 mL) was added Pd/C (1.0 g, 5.63 mmol), the mixture was stirred at 20 °C under H₂ atmosphere (balloon) for 12 h. The mixture was filtered and the filtrate was concentrated to get the desired compound as a colorless oil (1.8 g, 89.5%). MS (ESI): m/z = 302.2 [M-C₄H₈+H]⁺.

### b) tert-Butyl4-[1-[4-(trifluoromethyl)phenyl]vinyl]piperidine-1-carboxylate

To a solution of methyltriphenylphosphonium bromide (10.0 g, 27.98 mmol, CAS RN 1779-49-3) in THF (250 mL) was added lithium bis(trimethylsilyl)amide in THF (41.97 mL, 41.97 mmol) dropwise at 0 °C and he mixture was stirred at 0 °C for 1h. Then tert-butyl 4-[4-(trifluoromethyl)benzoyl]piperidine-1-carboxylate (10.0 g, 27.98 mmol, CAS RN 725229-27-6) in THF (50 mL) was added dropwise and the mixture was allowed to warm to 20 °C over 2h.The mixture was poured into aq. NH₄Cl solution (500 mL) and extracted three times with EtOAc (200 mL each). The organic phase was washed with brine and dried over Na₂SO₄, filtered and the filtrate was purified by silica gel column (PE : EtOAc = 20 :1) to provide the desired compound as light yellow oil. (5.1 g, 51.3%). 1H NMR (400 MHz, CHLOROFORM-d) δ = 7.61 (d, J=8.2 Hz, 2H), 7.44 (d, J=8.1 Hz, 2H), 5.25 (s, 1H), 5.13 (s, 1H), 4.27 - 4.14 (m, 2H), 2.77 (br t, J=12.3 Hz, 2H), 2.58 (br t, J=11.6 Hz, 1H), 1.78 (br d, J=13.0 Hz, 2H), 1.48 (s, 9H), 1.43 - 1.32 (m, 2H)..

### BB18

### 6-Chloro-1-methyl-3-(4-piperidyl)indazole

A mixture of 1-[4-(6-chloro-1-methyl-indazol-3-yl)-1-piperidyl]ethanone (3.95 g, 14 mmol) was refluxed in aqueous 25% HCl solution (25.75 mL) for 4 h. After cooling down to RT the mixture was basified using concentrated aqueous NaOH solution. The aqueous layer was extracted twice with DCM. The organic layers were dried over Na₂SO₄, filtered and evaporated to get the desired compound as a light red oil (3.6 g, 98.2%). MS (ESI): m/z = 250.2 [M+H]⁺. The compound was used in the next step without further purification.

### Intermediate:

### 1-[4-(6-Chloro-1-methyl-indazol-3-yl)-1-piperidyl]ethanone

In a sealed tube were mixed 1-[4-(4-chloro-2-fluoro-benzoyl)-1-piperidyl]ethanone (3.9 g, 14 mmol; Novel Chemical Solutions) and methylhydrazine (0.94 mL, 18 mmol) and the mixture was heated at 120°C for 16 h. After cooling down the mixture was basified using concentrated aqueous NaHCO₃ solution. The aqueous layer was extracted twice with DCM. The organic layers were dried over Na₂SO₄, filtered and evaporated to provide the desired compound as a yellow oil (3.97 g, 100%). MS (ESI): m/z = 292.3 [M+H]⁺. The compound was used in the next step without further purification.

### BB19

### 2-(4-Fluorophenyl)-2-(piperidin-4-yl)acetonitrile hydrochloride

The product was obtained in analogy to BB16 from tert-butyl 4-(cyano(4-fluorophenyl)methyl)piperidine-1-carboxylate (MFCD28112711, A Chemtek) as a light yellow solid. (0.101 g; 87.1%). MS (ESI): m/z = 219.1 [M+H]⁺.

### BB20

### 4-((4-Fluorophenyl)(2,2,2-trifluoroethoxy)methyl)piperidine hydrochloride

The product was obtained in analogy to BB16 from tert-butyl 4-((4-fluorophenyl)(2,2,2-trifluoroethoxy)methyl)piperidine-1-carboxylate as a colorless foam (0.151 g; 100.0%). MS (ESI): m/z = 292.2 [M+H]⁺.

### Intermediate:

### tert-Butyl 4-((4-fluorophenyl)(2,2,2-trifluoroethoxy)methyl)piperidine-1-carboxylate

To a yellow solution of tri-n-butylphosphine (262 mg, 319 µL, 1.29 mmol) and azodicarboxylic dipiperidide (326 mg, 1.29 mmol) in toluene (10 mL) was added tert-butyl 4-((4-fluorophenyl)(hydroxy)methyl)piperidine-1-carboxylate (200 mg, 646 µmol, CAS RN 160296-41-3) and the mixture was stirred at RT for 10 min. Addition of 2,2,2-trifluoroethanol (711 mg, 514 µL, 7.11 mmol) gave a suspension. After heating at reflux for 20 h, the reaction mixture was cooled down to RT. Silica gel was added and the mixture was evaporated. The compound was purified by silica gel chromatography on a 12 g column using an MPLC (ISCO) system eluting with a gradient of n-heptane : EtOAc (100 : 0 to 50 : 50) to get the desired compound as a colorless oil (0.180 g; 71.1%). MS (ESI): m/z = 391.1 [M+H]⁺.

### BB21

### 5-(2-(4-Fluorophenyl)-2-(piperidin-4-yl)ethyl)-3-methyl-1,2,4-oxadiazole hydrochloride

The product was obtained in analogy to BB16 from tert-butyl 4-(1-(4-fluorophenyl)-2-(3-methyl-1,2,4-oxadiazol-5-yl)ethyl)piperidine-1-carboxylate as a colorless solid (0.051 g; 98.3%). MS (ESI): m/z = 290.2 [M+H]⁺.

### Intermediates:

### a) tert-Butyl 4-(1-(4-fluorophenyl)-2-(3-methyl-1,2,4-oxadiazol-5-yl)ethyl)piperidine-1-carboxylate

To a mixture of tert-butyl 4-(3-ethoxy-1-(4-fluorophenyl)-3-oxopropyl)piperidine-1-carboxylate (100 mg, 264 µmol) and (Z)-N'-hydroxyacetimidamide (39 mg, 527 µmol) in toluene (2.5 mL) was added K₂CO₃ (72.8 mg, 527 µmol) and the reaction mixture stirred at 130°C for 14 days. Reaction was not finished so (Z)-N'-hydroxyacetimidamide (39 mg, 527 µmol) and K₂CO₃ (72.8 mg, 527 µmol) were added and the RM stirred over the weekend at 130°C. The reaction mixture was poured on saturated aqueous NH₄Cl solution and EtOAc and the layers were separated. The aqueous layer was extracted twice with EtOAc. The organic layers were dried over MgSO₄, filtered, treated with silica gel and evaporated. The compound was purified by silica gel chromatography on a 4 g column using an MPLC system eluting with a gradient of n-heptane : EtOAc (100 : 0 to 0 : 100) to get the desired compound as a colorless solid. MS (ESI): m/z = 290.3 [M-Boc+H]⁺.

### b) tert-Butyl 4-(3-ethoxy-1-(4-fluorophenyl)-3-oxopropyl)piperidine-l-carboxylate

In a 25 mL round-bottomed flask, tert-butyl (E)-4-(3-ethoxy-1-(4-fluorophenyl)-3-oxoprop-1-en-1-yl)piperidine-1-carboxylate (1.747 g, 4.63 mmol) was combined with EtOAc (17.1 mL) to give a colorless solution. Pd/C 10% (175 mg, 4.63 mmol) was added added under argon. The suspension was stirred under an hydrogen atmosphere at 1.5 bar for 4 h. The reaction mixture was filtered and the filtrate was concentrated in vacuo. The compound was purified by silica gel chromatography on a 24 g column using an MPLC (ISCO) system eluting with a gradient of n-heptane : EtOAc (100 : 0 to 50 : 50) to get the desired compound as a colorless oil (1.22 g; 69.8%). MS (ESI): m/z = 280 [M-Boc+H]⁺.

### c) tert-Butyl (E)-4-(3-ethoxy-1-(4-fluorophenyl)-3-oxoprop-1-en-1-yl)piperidine-1-carboxylate

To a solution of ethyl 2-(diethoxyphosphoryl)acetate (2.1 g, 1.86 mL, 9.36 mmol) in 1,4-dioxane (9.73 mL) was added dropwise LiHMDS 1.0 M in hexanes (15 mL, 15 mmol) and the solution was stirred for 15 min. tert-Butyl 4-(4-fluorobenzoyl)piperidine-1-carboxylate (2.878 g, 9.36 mmol, CAS RN 160296-40-2) dissolved in 1,4-dioxane (9.73 mL) was added dropwise to the mixture and stirring was continued at reflux over two days. The reaction mixture was poured on saturated aqueous NH₄Cl solution and EtOAc and the layers were separated. The aqueous layer was extracted twice with EtOAc. The organic layers were washed once with water, dried over MgSO₄, filtered, treated with silica gel and evaporated. The compound was purified by silica gel chromatography on a 40 g column using an MPLC system eluting with a gradient of n-heptane : EtOAc (100 : 0 to 50 : 50) to get the desired compound as a colorless liquid (1.74 g, 49.4%). MS (ESI): m/z = 278.2 [M-Boc+H]⁺.

### BB22

### 4-(1-(4-(Trifluoromethyl)phenyl)cyclopropyl)piperidine formate

To a solution of tert-butyl 4-[1-[4-(trifluoromethyl)phenyl]cyclopropyl]piperidine-1-carboxylate (250.0 mg, 0.680 mmol) in DCM (5 mL) was added TFA (1.0 mL, 0.680 mmol) and the mixture was stirred at 20 °C for 16 h. The mixture was concentrated and the residue was purified by prep-HPLC (FA) to yield the desired compound as a yellow oil (181 mg, 81.5%). MS (ESI): m/z = 270.1 [M+H]⁺.

### Intermediates:

### a) tert-Butyl 4-[1-[4-(trifluoromethyl)phenyl)cyclopropyl)piperidine-1-carboxylate

To a solution of tert-butyl 4-[2,2-dibromo-1-[4-(trifluoromethyl)phenyl]cyclopropyl]piperidine-1-carboxylate (800.0 mg, 1.52 mmol) and titanium(IV) isopropoxide (862.56 mg, 3.03 mmol) in THF (30 mL) was added a 3M solution of EtMgBr inTHF (5.06 mL, 15.17 mmol) dropwise at 0 °C. The mixture was warmed to 20 °C over 16 h. The mixture was poured into NH₄Cl (aq. 100 mL) and extracted three times with EtOAc (30 mL each). The organic phase was washed with brine and dried over Na₂SO₄, filtered and the filtrate was concentrated. The residue was purified by reversed flash chromatography (FA) to provide the desired compound as a light yellow oil (250 mg, 44.6%). MS (ESI): m/z = 314.1 [M-C₄H₈+H]⁺.

### b) tert-Butyl 4-[2,2-dibromo-1-[4-(trifluoromethyl)phenyl]cyclopropyl]piperidine-1-carboxylate

To a vigorously stirred mixture of tert-butyl 4-[1-[4-(trifluoromethyl)phenyl]vinyl]piperidine-1-carboxylate (1.2 g, 3.38 mmol, BB17, intermediate b), bromoform (2.56 g, 10.13 mmol, CAS RN 75-25-2) and cetrimide (0.37 g, 1.01 mmol, CAS RN 57-09-0 ) in DCM (10 mL) was added 50% of aq. NaOH (1.35 mL, 16.88 mmol) dropwise. The reaction mixture was vigorously stirred at 50 °C for 16 h, and then H2O (20 mL) was added. The organic phase was separated the aqueous phase was extracted three times with with DCM (10 mL each). The combined organic phases were washed with H2O, 2% HCl and brine, and dried (Na₂SO₄), filtered, and concentrated. The residue was purified by prep-TLC (PE : EtOAc = 20 : 1) to give the desired compound as colorless oil (900 mg, 50.5%). MS (ESI): m/z = 471.9 [M-C₄H₈+H]⁺.

### BB23

### 4-(1-(4-Fluorophenyl)-2-(2,2,2-trifluoroethoxy)ethyl)piperidine hydrochloride

The product was obtained in analogy to BB16 from tert-butyl 4-(1-(4-fluorophenyl)-2-(2,2,2-trifluoroethoxy)ethyl)piperidine-1-carboxylate as a colorless foam (0.173 g; 97.7%). MS (ESI): m/z = 306.2 [M+H]⁺.

### Intermediate:

### tert-Butyl 4-(1-(4-fluorophenyl)-2-(2,2,2-trifluoroethoxy)ethyl)piperidine-1-carboxylate

To a yellow solution of tri-n-butylphosphine (273 mg, 333 µL, 1.35 mmol) and azodicarboxylic dipiperidide (340 mg, 1.35 mmol) in Toluene (11.1 mL) was added tert-butyl 4-(1-(4-fluorophenyl)-2-hydroxyethyl)piperidine-1-carboxylate (218 mg, 674 µmol, BB5, intermediate b) and the mixture was stirred at RT for 10 minutes. Addition of 2,2,2-trifluoroethanol (742 mg, 536 µL, 7.42 mmol) gave a suspension. Upon heating to 65°C again a yellow solution was formed. After heating at reflux for 17 hours, the reaction mixture was cooled down to RT. Silica gel was added and the mixture was evaporated. The compound was purified by silica gel chromatography on a 12 g column using an MPLC (ISCO) system eluting with a gradient of n-heptane : EtOAc (100 : 0 to 50 : 50) to get the desired compound as a colorless oil (0.211 g; 77.2%). MS (ESI): m/z = 350.1 [M-C₄H₈+H]⁺.

### BB24

### 3-(1-(4-(Trifluoromethyl)phenyl)ethyl)azetidine formate

The product was obtained in analogy to BB22 from tert-butyl 3-[1-[4-(trifluoromethyl)phenyl]ethyl]azetidine-1-carboxylate as light yellow oil (151.5 mg, 27.4%). MS (ESI): m/z = 230.1 [M+H]⁺.

### Intermediates:

### a) tert-Butyl 3-[1-[4-(trifluoromethyl)phenyl]ethyl]azetidine-1-carboxylate

The product was obtained in analogy to BB17, intermediate a from tert-butyl 3-[1-[4-(trifluoromethyl)phenyl]vinyl]azetidine-1-carboxylate as colorless oil (1 g, 99.4%). MS (ESI): m/z = 274.0 [M-C₄H₈+H]⁺.

### b) tert-Butyl 3-[1-[4-(trifluoromethyl)phenyl]vinyl]azetidine-1-carboxylate

To a solution of methyltriphenylphosphonium bromide (1952.56 mg, 5.47 mmol, CAS RN 1779-49-3) in THF (25 mL) was added lithium bis(trimethylsilyl)amide/THF (9.11 mL, 9.11 mmol) dropwise at 0 °C, the mixture was stirred at 0 °C for 1h, then tert-butyl 3-[4-(trifluoromethyl)benzoyl]azetidine-1-carboxylate (1500.0 mg, 4.55 mmol, MFCD24368873, FCH group) in THF (5 mL) was added dropwise, the mixture was warmed to 20 °C for 2h. The mixture was poured into aq. NH₄Cl solution (100 mL) and extracted three times with EtOAc (50 mL each), the organic phase was washed with brine and dried over Na₂SO₄, filtered and the filtrate was purified by silica gel column (PE : EtOAc = 20 :1) to give the desired compound as light yellow oil (1000 mg, 67.0%). 1H NMR (400 MHz, CHLOROFORM-d) δ = 7.61 (d, J=8.1 Hz, 2H), 7.42 (d, J=8.1 Hz, 2H), 5.61 (s, 1H), 5.32 (s, 1H), 4.20 (t, J=8.4 Hz, 2H), 3.89 (br s, 2H), 3.83 - 3.73 (m, 1H), 1.46 (s, 9H).

### BB25

### 5-((4-Fluorophenyl)(piperidin-4-yl)methyl)-3-(trifluoromethyl)-1,2,4-oxadiazole hydrochloride

The product was obtained in analogy to BB16 from tert-butyl 4-((4-fluorophenyl)(3-(trifluoromethyl)-1,2,4-oxadiazol-5-yl)methyl)piperidine-1-carboxylate as a colorless solid (0.119 g; 90.1%). MS (ESI): m/z = 330.2 [M+H]⁺.

### Intermediate:

### tert-Butyl 4-((4-fluorophenyl)(3-(trifluoromethyl)-1,2,4-oxadiazol-5-yl)methyl)piperidine-1-carboxylate

To a solution of 2-(1-(tert-butoxycarbonyl)piperidin-4-yl)-2-(4-fluorophenyl)acetic acid (250 mg, 741 µmol, MFCD17214722, A Chemtek) in DMF (2 mL) was added CDI (120 mg, 741 µmol) and the mixture was stirred at RT for 30 minutes. To the clear solution was added a solution of (Z)-2,2,2-trifluoro-N'-hydroxyacetimidamide (94.9 mg, 741 µmol) in DMF (0.5 mL) and stirring was continued at R for 1 hour. The reaction mixture was heated in a microwave at 120°C for 30 minutes followed by heating at 150°C for 30 minutes. The reaction mixture was poured on water and EtOAc and the layers were separated. The aqueous layer was extracted twice with EtOAc. The organic layers were washed twice with water, dried over MgSO₄, filtered, treated with silica gel and evaporated. The compound was purified by silica gel chromatography on a 12 g column using an MPLC system eluting with a gradient of n-heptane : EtOAc (100 : 0 to 0 : 100) to provide the desired compound as a colorless foam (0.155 g; 48.7%). MS (ESI): m/z = 428.3 [M-H]⁻.

### BB26

### (4aR,8aS)-6-[3-[(4-Fluorophenyl)-(2,2,2-trifluoroethoxy)methyl]azetidine-1-carbonyl]-4,4a,5,7,8,8a-hexahydropyrido[4,3-b][1,4]oxazin-3-one

The product was obtained in analogy to method A4 using 3-[(4-fluorophenyl)-(2,2,2-trifluoroethoxy)methyl]azetidine 2,2,2-trifluoroacetate to get the desired product as white solid. MS (ESI): m/z = 446.2 [M-C₄H₈+H]⁺. The product was used in the next step without further purification.

### Intermediates:

### a) 3-[(4-Fluorophenyl)-(2,2,2-trifluoroethoxy)methyl]azetidine 2,2,2-trifluoroacetate

To a solution of tert-butyl 3-[(4-fluorophenyl)-(2,2,2-trifluoroethoxy)methyl]azetidine-1-carboxylate (1000.0 mg, 2.75 mmol) in DCM (20 mL) was added TFA (2.0 mL, 2.75 mmol), the mixture was stirred at 20 °C for 12 h. The mixture was concentrated, the residue was purified by prep-HPLC (TFA) to get the desired product as off-white solid (608.8 mg, 57.6%). MS (ESI): m/z = 264.1 [M+H]⁺.

### b) tert-Butyl 3-[(4-fluorophenyl)-(2,2,2-trifluoroethoxy)methyl]azetidine-1-carboxylate

To a solution of tert-butyl 3-[(4-fluorophenyl)-hydroxy-methyl]azetidine-1-carboxylate (3000.0 mg, 10.66 mmol), 2,2,2-trifluoroethanol (1.55 mL, 21.33 mmol) and triphenylphosphine (4195.52 mg, 16 mmol) in THF (50 mL) was added diisopropyl azodicarboxylate (3.15 mL, 16 mmol) and the mixture was stirred at 80 °C for 12 h. The mixture was concentrated and the residue was purified by reversed flash (FA) to give the desired product as yellow oil (1 g, 25.8%). MS (ESI): m/z = 308.1 [M-C₄H₈+H]⁺.

### c) tert-Butyl 3-[(4-fluorophenyl)-hydroxy-methyl]azetidine-1-carboxylate

To a solution of tert-butyl 3-(4-fluorobenzoyl)azetidine-1-carboxylate (2 g, 7.16 mmol, MFCD24368873, FCH group ) in MeOH (30 mL) was added NaBH4 (541.33 mg, 14.32 mmol) and the mixture was stirred at 20 °C for 1 h. The mixture was concentrated and dissolved in EtOAc (100 mL), washed with a.q NH₄Cl and brine, dried over Na₂SO₄ and concentrated to give the desired product as light yellow oil (2 mg, 99.3%). The compound was used in the next step without any further purification.

### BB27

### (4aR,8aS)-6-[3-[1-(2-Chloro-4-fluoro-phenyl)ethoxy]azetidine-1-carbonyl]-4,4a,5,7,8,8a-hexahydropyrido[4,3-b][1,4]oxazin-3-one

The product was obtained in analogy to method A4 using 3-[1-(2-chloro-4-fluorophenyl)ethoxy]azetidine 2,2,2-trifluoroacetate to give the desired product as off-white solid (230 mg, 59.8%). MS (ESI): m/z = 412.2 [M+H]⁺.

### Intermediates:

### a) 3-[1-(2-Chloro-4-fluoro-phenyl)ethoxy]azetidine2,2,2-trifluoroacetate

The product was obtained in analogy to BB26, intermediate a using tert-butyl 3-[1-(2-chloro-4-fluoro-phenyl)ethoxy]azetidine-1-carboxylate to give the desired product as light yellow gum (1.4 g, 67.2%). MS (ESI): m/z = 230.1 [M+H]⁺.

### b) tert-Butyl 3-[1-(2-chloro-4-fluoro-phenyl)ethoxy]azetidine-1-carboxylate

A mixture of tert-butyl 3-hydroxyazetidine-1-carboxylate (5.34 g, 30.82 mmol, CAS RN 141699-55-0) in DMF (60 mL) was added sodium hydride (1.54 g, 38.53 mmol) at 0 °C. The mixture was stirred at 20 °C for 1 h. Then 1-(1-bromoethyl)-2-chloro-4-fluorobenzene (6.1 g, 25.68 mmol, CAS RN 1341821-29-1) was adeed and the mixtrue was stirred at 20 °C for 12 h. The mixture was poured into ice water (200 mL) and extracted three times with EtOAc (100 mL each). The combined organic layer was washed with brine (100 mL), dried over Na₂SO₄ and filtered. The filtrate was concentrated. The residue was purified by prep-HPLC (FA) and concentrated under vacuum to give the desired product as light yellow oil (2 g, 23.6%). MS (ESI): m/z = 230 [M+H-Boc]⁺.

### BB28

### 3-(2-Chloro-[1,1'-biphenyl]-4-yl)azetidine 4-methylbenzenesulfonate

To an solution of tert-butyl 3-(2-chloro-[1,1'-biphenyl]-4-yl)azetidine-1-carboxylate (60 mg, 174 µmol) in EtOAc (2 mL) was added 4-methylbenzenesulfonic acid hydrate (39.8 mg, 209 µmol) and the mixture was heated at reflux for 1 hour. The clear, colorless solution was evaporated to get the desired product as a colorless solid (0.10 g; 100%). MS (ESI): m/z = 244.2 [M+H]⁺.

### Intermediate:

### tert-Butyl 3-(2-chloro-[1,1'-biphenyl]-4-yl)azetidine-1-carboxylate

The product was obtained in analogy to Method A5 from phenylboronic acid (CAS RN 98-80-6) and tert-butyl 3-(4-bromo-3-chlorophenyl)azetidine-1-carboxylate (CAS RN 2222937-56-4 as a colorless solid. MS (ESI): m/z = 288.2 [M-C₄H₈+H]⁺.

### BB29

### 3-(4-Bromo-3-chlorophenyl)azetidine 4-methylbenzenesulfonate

The product was obtained in analogy to BB28 from tert-butyl 3-(4-bromo-3-chlorophenyl)azetidine-1-carboxylate (CAS RN 2222937-56-4) as a colorless solid. MS (ESI): 248.1 [M+H]⁺.

### BB30

### 3-(3-Bromophenyl)azetidine 4-methylbenzenesulfonate

The product was obtained in analogy to BB28 from tert-butyl 3-(3-bromophenyl)azetidine-1-carboxylate (CAS RN 1203681-54-2) as a colorless solid. MS (ESI): 212.1 [M+H]⁺.

### BB31

### 3-(4-tert-Butylphenyl)azetidine;4-methylbenzenesulfonic acid

The product was obtained in analogy to BB28 from tert-butyl 3-(4-(tert-butyl)phenyl)azetidine-1-carboxylate (CAS RN 1629889-13-9) as a colorless solid. MS (ESI): 190.2 [M+H]⁺.

### BB32

### 5-(Azetidin-3-yl)-2-chloropyridine 4-methylbenzenesulfonate

The product was obtained in analogy to BB28 from tert-butyl 3-(6-chloropyridin-3-yl)azetidine-1-carboxylate (CAS RN 870689-19-3) as a colorless solid. MS (ESI): 169.1 [M+H]⁺.

### BB33

### 3-(4-(Trifluoromethyl)phenyl)azetidine 4-methylbenzenesulfonate

The product was obtained in analogy to BB28 from tert-butyl 3-(4-(trifluoromethyl)phenyl)azetidine-1-carboxylate (CAS RN 1638255-66-9 ) as a colorless solid. MS (ESI): 202.2 [M+H]⁺.

### BB34

### 3-((1,1,1-Trifluoro-2-methylpropan-2-yl)oxy)azetidine hydrochloride

To a solution of 1-benzhydryl-3-((1,1,1-trifluoro-2-methylpropan-2-yl)oxy)azetidine (114 mg, 326 µmol) in Ethanol (0.5 mL) and EtOAc (0.5 mL) were added HCl 1M in H₂0 (326 µL, 326 µmol) and Pd/C 10% (20 mg, 326 µmol) and the mixture was stirred under a hydrogen atmosphere at 1.7 bar for 5 h. The suspension was filtered over a microfilter and the filtrate was evaporated to get the desired compound as a colorless amorphous (0.105 g). MS (ESI): m/z = 184.2 [M+H]⁺. Used without further purification in the next step.

### Intermediate:

### Benzhydryl-3-((1,1,1-trifluoro-2-methylpropan-2-yl)oxy)azetidine

To a solution of 1, 1, 1-trifluoro-2-methyl-propan-2-ol (807 mg, 690 µL, 6.3 mmol, CAS RN 507-52-8) in DMF (5 mL) under argon was added sodium hydride 60% in mineral oil (252 mg, 6.3 mmol) and the mixture was stirred at RT for 30 minutes. After addition of (1-benzhydrylazetidin-3-yl) methanesulfonate (1 g, 3.15 mmol, CAS RN 33301-41-6) the reaction mixture was heated in a microwave oven at 130°C for 60 minutes. The reaction mixture was poured on water and EtOAc and the layers were separated. The aqueous layer was extracted twice with EtOAc. The organic layers were washed once with water, dried over MgSO₄, filtered, treated with silica gel and evaporated. The compound was purified by silica gel chromatography on a 12 g column using an MPLC system eluting with a gradient of n-heptane : EtOAc (100 : 0 to 50 : 50) to yield the desired compound as a yellow solid (0.324 g; 29.4%). MS (ESI): m/z = 350.3 [M+H]⁺.

### BB35

### 3-(4-(1,1-Difluoroethyl)phenyl)azetidine 4-methylbenzenesulfonate

The product was obtained in analogy to BB28 from tert-butyl 3-(4-(1,1-difluoroethyl)phenyl)azetidine-1-carboxylate as a colorless solid (0.050 g; 35.9%). MS (ESI): m/z = 198.2 [M+H]⁺.

### Intermediate:

### tert-Butyl 3-(4-(1,1-difluoroethyl)phenyl)azetidine-l-carboxylate

To a stirred suspension of 2-(4-(1,1-difluoroethyl)phenyl)-4,4,5,5-tetramethyl-1,3,2-dioxaborolane (568 mg, 2.12 mmol, CAS RN 1000994-94-4) in 2-rpopanol (1.5 mL) was added a solution of tert-butyl 3-iodoazetidine-1-carboxylate (300 mg, 1.06 mmol, CAS RN 254454-54-1) in 2-propanol (1.5 mL) at RT, to give a solution. To the mixture was added rac-(1R,2R)-2-aminocyclohexan-1-ol (7.32 mg, 63.6 µmol, CAS RN 13374-31-7), nickel(II) iodide (19.9 mg, 63.6 µmol) and sodium bis(trimethylsilyl)amide (1.06 mL, 2.12 mmol) under argon.The mixture was heated in a microwave oven for 30 minutes at 100°C.

The reaction mixture was poured on water and EtOAc and the layers were separated. The aqueous layer was extracted twice with EtOAc. The organic layers were dried over MgSO₄, filtered, treated with silica gel and evaporated. The compound was purified by silica gel chromatography on a 4 g column using an MPLC system eluting with a gradient of n-heptane : EtOAc (100 : 0 to 70 : 30) to furnish the desired compound as a colorless oil (0.0112 g; 35.5%). MS (ESI): m/z = 242.2 [M-C₄H₈+H]⁺.

### BB36

### 5-(4-(Azetidin-3-yl)phenyl)-1-methyl-1H-pyrazole 4-methylbenzenesulfonate

The product was obtained in analogy to BB28 from tert-butyl 3-(4-(1-methyl-1H-pyrazol-5-yl)phenyl)azetidine-1-carboxylate as a light brown gum which was used in the . MS (ESI): m/z = 214.1 [M+H]⁺. The product was used in the next step without further purification.

### Intermediate:

### tert-Butyl 3-(4-(1-methyl-1H-pyrazol-5-yl)phenyl)azetidine-1-carboxylate

The product was obtained in analogy to BB35, intermediate from (4-(1-methyl-1H-pyrazol-5-yl)phenyl)boronic acid (CAS RN 1487353-57-0) as a light brown gum. MS (ESI): m/z = 314.3 [M+H]⁺. The product was used in the next step without further purification.

### BB37

### 3-[4-(2,2,2-Trifluoroethoxy)phenyl]azetidine trifluoroacetate

The product was obtained in analogy to BB26, intermediate a, from tert-butyl 3-[4-(2,2,2-trifluoroethoxy)phenyl]azetidine-1-carboxylate as a light yellow oil. MS (ESI): m/z = 232.1 [M+H]⁺. The product was used in the next step without further purification.

### Intermediate:

### tert-Butyl 3-[4-(2,2,2-trifluoroethoxy)phenyl]azetidine-1-carboxylate

To a solution of 1-bromo-4-(2,2,2-trifluoroethoxy)benzene (0.45 g, 1.77 mmol, CAS RN 106854-77-7), 1-BOC-3-iodoazetidine (0.5 g, 1.77 mmol, CAS RN 254454-54-1), 1,10-phenanthroline (63.65 mg, 0.350 mmol, CAS RN 5144-89-8), NaBF4 (96.95 mg, 0.880 mmol, CAS RN 13755-29-8), NiCl₂ glyme (38.8 mg, 0.180 mmol, CAS RN 29046-78-4) and Mn powder (194.06 mg, 3.53 mmol) in MeOH (10 mL) was added 4-ethylpyridine (94.62 mg, 0.880 mmol, CAS RN 536-75-4). The mixture was stirred at 60 °C under N2 atmosphere for 16 h. The mixture was filtered and the filtrate was concentrated, the residue was purified by reverse flash chromatography (FA) to get the desired product as light yellow foam (60 mg, 10.2%). MS (ESI): m/z = 276.0 [M-C₄H₈+H]⁺.

### BB38

### [4-[1-[(4aR,8aS)-3-oxo-4,4a,5,7,8,8a-hexahydropyrido[4,3-b][1,4]oxazine-6-carbonyl]azetidin-3-yl]phenyl]boronic acid

To a solution of (4aR,8aS)-6-[3-(4-bromophenyl)azetidine-1-carbonyl]-4,4a,5,7,8,8a-hexahydropyrido[4,3-b][1,4]oxazin-3-one (300.0 mg, 0.760 mmol, example 110) and PdCl₂(dppf).CH₂Cl₂ (53.34 mg, 0.080 mmol) was added potassium acetate (224.04 mg, 2.28 mmol) and bis(pinacolato)diboron (289.84 mg, 1.14 mmol, CAS RN 73183-34-3), the reaction was purged with nitrogen and stirred at 90 °C for 12 h. The reaction was diluted with water and extracted with EtOAc for three times, the combined organic layer was washed with water and brine, dried over sodium sulfate and concentrated in vacuum to get yellow residue, which was purified with reverse phase chromatograph(FA) to get the desired product as light yellow solid (230 mg, 84.1%). MS (ESI): m/z = 360.5 [M+H]⁺.

### BB39

### 3-Cyclopropyl-5-((4-fluorophenyl)(piperidin-4-yl)methyl)-1,2,4-oxadiazole hydrochloride

The product was obtained in analogy to BB16 from tert-butyl 4-((3-cyclopropyl-1,2,4-oxadiazol-5-yl)(4-fluorophenyl)methyl)piperidine-1-carboxylate as a colorless foam (0.170 g; 84.7%). MS (ESI): m/z = 302.3 [M+H]⁺.

### Intermediates:

### a) tert-Butyl 4-((3-cyclopropyl-1,2,4-oxadiazol-5-yl)(4-fluorophenyl)methyl)piperidine-1-carboxylate

The product was obtained in analogy to BB25, intermediate from 2-(1-(tert-butoxycarbonyl)piperidin-4-yl)-2-(4-fluorophenyl)acetic acid and N'-hydroxycyclopropanecarboxamidine as a colorless foam (0.240 g; 80.7%). MS (ESI): m/z = 346.2 [M-C₄H₈+H]⁺.

### b) 2-(1-(tert-Butoxycarbonyl)piperidin-4-yl)-2-(4-fluorophenyl)acetic acid

To a turbid solution of 2-(4-fluorophenyl)-2-(piperidin-4-yl)acetic acid hydrobromide (535 mg, 1.55 mmol) in NaOH 1M in H₂O (3.09 mL, 3.09 mmol) was added dropwise a solution of di-tert-butyl dicarbonate (368 mg, 391 µL, 1.68 mmol) in DME (5 mL) and the mixture was stirred at RT for 3 h. DME was evaporated. The residue was taken up in approx. 1.2 mL citric acid 10% in water (pH approx. 4) and EtOAc and the layers were separated. The aqueous layer was extracted once with EtOAc. The organic layers dried over MgSO₄, filtered and evaporated to get the desired compound as a light brown solid (0.520 g; 99.6%). MS (ESI): m/z = 336.3 [M-H]⁻.

### c) 2-(4-Fluorophenyl)-2-(piperidin-4-yl)acetic acid hydrobromide

A solution of tert-butyl 4-(cyano(4-fluorophenyl)methyl)piperidine-1-carboxylate (555 mg, 1.74 mmol, CAS RN 1824014-64-3) in HBr 48% in water (8.27 g, 5.55 mL, 49.1 mmol) was stirred at reflux for 4.5 hours. The mixture was evaporated. The light brown solid was supended in 2-propanol (2 mL), homogenized and filtered. The filter cake was washed three times with 2-propanol (1 mL each). The mother liquor was completly evaporated and dried for 2 hours at high vacuum in the presence of P₂O₅ to yield the desired product as a light brown solid (0.535 g; 96.5%). MS (ESI): m/z = 238.2 [M-HBr+H]⁺.

### BB40

### 3-(3-(Trifluoromethoxy)phenyl)azetidine 4-methylbenzenesulfonate

The product was obtained in analogy to BB28 from tert-butyl 3-(3-(trifluoromethoxy)phenyl)azetidine-1-carboxylate (CAS RN 2222044-21-3) as a colorless solid. MS (ESI): 218.1 [M+H]⁺.

### BB41

### [4-(Azetidin-3-yl)phenyl]-pentafluoro-lambda6-sulfane 4-methylbenzenesulfonate

The product was obtained in analogy to BB28 from tert-butyl 3-(4-(pentafluoro-16-sulfaneyl)phenyl)azetidine-1-carboxylate as a colorless solid. MS (ESI): 260.1 [M+H]⁺.

### Intermediate:

### tert-Butyl 3-(4-(pentafluoro-l6-sulfaneyl)phenyl)azetidine-1-carboxylate

The product was obtained in analogy to BB35, intermediate from (4-(pentafluoro-l6-sulfaneyl)phenyl)boronic acid (CAS RN 871507-70-9) as a colorless oil. MS (ESI): m/z = 304.1 [M-C₄H₈+H]⁺.

### BB42

### 2-(Azetidin-3-yl)-5-chloropyridine bis(4-methylbenzenesulfonate)

The product was obtained in analogy to BB28 from tert-butyl 3-(5-chloropyridin-2-yl)azetidine-1-carboxylate and twice the amount of p-TsOH as a colorless solid. MS (ESI): m/z = 169.1 [M+H]⁺.

### Intermediate:

### tert-Butyl 3-(5-chloropyridin-2-yl)azetidine-1-carboxylate

The product was obtained in analogy to BB37, intermediate from (5-chloropyridin-2-yl)boronic acid and tert-butyl 3-iodoazetidine-1-carboxylate as a colorless oil (0.045 g; 11.2%). MS (ESI): m/z = 213.1 [M-C₄H₈+H]⁺.

### BB43

### 3-(2-Fluoro-4-(trifluoromethoxy)phenyl)azetidine 4-methylbenzenesulfonate

The product was obtained in analogy to BB28 from tert-butyl 3-(2-fluoro-4-(trifluoromethoxy)phenyl)azetidine-1-carboxylate as a colorless solid. MS (ESI): m/z = 236.1 [M+H]⁺.

### Intermediate:

### tert-Butyl 3-(2-fluoro-4-(trifluoromethoxy)phenyl)azetidine-1-carboxylate

The product was obtained in analogy to BB35, intermediate from (2-fluoro-4-(trifluoromethoxy)phenyl)boronic acid (CAS RN 503309-10-2) as a colorless oil. MS (ESI): m/z = 280.1 [M-C₄H₈+H]⁺.

### BB44

### 3-[4-(2,2,2-Ttrifluoroethyl)phenyl]azetidine trifluoroacetate

The product was obtained in analogy to BB26, intermediate a from tert-butyl 3-[4-(2,2,2-trifluoroethyl)phenyl]azetidine-1-carboxylate as a yellow oil. MS (ESI): m/z = 216.2 [M+H]⁺.

### Intermediate:

### tert-Butyl 3-[4-(2,2,2-trifluoroethyl)phenyl]azetidine-1-carboxylate

The product was obtained in analogy to BB37, intermediate a from 1-bromo-4-(2,2,2-trifluoroethyl)benzene (CAS RN 155820-88-5) as a yellow oil. MS (ESI): m/z = 260.0 [M-C₄H₈+H]⁺.

### BB45

### 3-[4-[1-(Trifluoromethyl)cyclopropyl]phenyl]azetidine ttrifluoroacetate

The product was obtained in analogy to BB26, intermediate a from tert-butyl 3-[4-[1-(trifluoromethyl)cyclopropyl]phenyl]azetidine-1-carboxylate as a yellow oil. MS (ESI): m/z = 242.1 [M+H]⁺.

### Intermediate:

### tert-Butyl 3-[4-[1-(trifluoromethyl)cyclopropyl]phenyl]azetidine-1-carboxylate

The product was obtained in analogy to BB37, intermediate from 1-bromo-4-[1-(trifluoromethyl)cyclopropyl]benzene (CAS RN 1227160-18-0) as a yellow oil. MS (ESI): m/z = 286.0 [M-C₄H₈+H]⁺.

### BB46

### 3-(3-Fluoro-4-(trifluoromethoxy)phenyl)azetidine 4-methylbenzenesulfonate

The product was obtained in analogy to BB28 a from tert-butyl 3-(3-fluoro-4-(trifluoromethoxy)phenyl)azetidine-1-carboxylate as a colorless solid. MS (ESI): m/z = 236.1 [M+H]⁺.

### Intermediate:

### tert-Butyl 3-(3-fluoro-4-(trifluoromethoxy)phenyl)azetidine-1-carboxylate

The product was obtained in analogy to BB37, intermediate from 4-bromo-2-fluoro-1-(trifluoromethoxy)benzene (CAS RN 105529-58-6) as a colorless oil. MS (ESI): m/z = 280.1 [M-C₄H₈+H]⁺.

### BB47

### 3-(3-Methyl-4-(trifluoromethoxy)phenyl)azetidine 4-methylbenzenesulfonate

The product was obtained in analogy to BB28 a from tert-butyl 3-(3-methyl-4-(trifluoromethoxy)phenyl)azetidine-1-carboxylate as a colorless solid. MS (ESI): m/z = 232.1 [M+H]⁺.

### Intermediate:

### tert-Butyl 3-(3-methyl-4-(trifluoromethoxy)phenyl)azetidine-1-carboxylate

The product was obtained in analogy to BB37, intermediate from 4-bromo-2-methyl-1-(trifluoromethoxy)benzene (CAS RN 887268-26-0) as a colorless oil. MS (ESI): m/z = 276.2 [M-C₄H₈+H]⁺.

### BB48

### 3-(3,5-Difluoro-4-(trifluoromethoxy)phenyl)azetidine 4-methylbenzenesulfonate

The product was obtained in analogy to BB28 a from tert-butyl 3-(3,5-difluoro-4-(trifluoromethoxy)phenyl)azetidine-1-carboxylate as a colorless solid. MS (ESI): m/z = 254.1 [M+H]⁺.

### Intermediate:

### tert-butyl 3-(3,5-Difluoro-4-(trifluoromethoxy)phenyl)azetidine-1-carboxylate

The product was obtained in analogy to BB37, intermediate a from 5-bromo-1,3-difluoro-2-(trifluoromethoxy)benzene (CAS RN 115467-07-7) as a colorless solid. MS (ESI): m/z = 298.1 [M-C₄H₈+H]⁺.

### BB49

### 3-(2-Chloro-4-(trifluoromethoxy)phenyl)azetidine 4-methylbenzenesulfonate

The product was obtained in analogy to BB28 a from tert-butyl 3-(2-chloro-4-(trifluoromethoxy)phenyl)azetidine-1-carboxylate as a colorless solid. MS (ESI): m/z = 252.1 [M+H]⁺.

### Intermediate:

### tert-Butyl 3-(2-chloro-4-(trifluoromethoxy)phenyl)azetidine-1-carboxylate

The product was obtained in analogy to BB37, intermediate a from 1-bromo-2-chloro-4-(trifluoromethoxy)benzene (CAS RN 892845-59-9) as a colorless oil. MS (ESI): m/z = 296.1 [M-C₄H₈+H]⁺.

### BB50

### 3-(4-(Bicyclo[1.1.1]pentan-1-yl)phenyl)azetidine 4-methylbenzenesulfonate

The product was obtained in analogy to BB28 a from tert-butyl 3-(4-(bicyclo[1.1.1]pentan-1-yl)phenyl)azetidine-1-carboxylate as a colorless solid. MS (ESI): m/z = 200.2 [M+H]⁺.

### Intermediate:

### tert-Butyl 3-(4-(bicyclo[1.1.1]pentan-1-yl)phenyl)azetidine-1-carboxylate

The product was obtained in analogy to BB37, intermediate a from 1-(4-bromophenyl)bicyclo[1.1.1]pentane (CAS RN 1823935-76-7) as a colorless oil. MS (ESI): m/z = 244.2 [M-C₄H₈+H]⁺.

### BB51

### 5-(Azetidin-3-yl)-2-(trifluoromethoxy)benzonitrile 4-methylbenzenesulfonate

The product was obtained in analogy to BB28 from tert-butyl 3-(3-cyano-4-(trifluoromethoxy)phenyl)azetidine-1-carboxylate as a light brown gum. MS (ESI): m/z = 243.1 [M+H]⁺. The compound was used in the next step without further purification.

### Intermediate:

### tert-Butyl 3-(3-cyano-4-(trifluoromethoxy)phenyl)azetidine-1-carboxylate

The product was obtained in analogy to BB37, intermediate a from 5-bromo-2-(trifluoromethoxy)benzonitrile (CAS RN 1210906-15-2) as a light yellow oil. MS (ESI): m/z = 287.1 [M-C₄H₈+H]⁺.

### BB52

### (1 -(4-(Azetidin-3-yl)phenyl)cyclopropyl)methanol hydrochloride

To a solution of tert-butyl 3-(4-(1-(hydroxymethyl)cyclopropyl)phenyl)azetidine-1-carboxylate (40 mg, 132 µmol) in DCM (0.7 mL) was added HCl in dioxane 4M (330 µL, 1.32 mmol) and the mixture was stirred at RT for 3 hours. To the light yellow suspension was added diethyl ether (2 mL) and the oily mixture was evaporated to get the desired compound as a light yellow oil. MS (ESI): m/z = 204.2 [M+H]⁺. The compound was used in the next step without further purification.

### Intermediates:

### a) tert-Butyl 3-(4-(1-(hydroxymethyl)cyclopropyl)phenyl)azetidine-1-carboxylate

To an ice-cold solution of tert-butyl 3-(4-(1-(methoxycarbonyl)cyclopropyl)phenyl)azetidine-1-carboxylate (238 mg, 718 µmol) in THF (2 mL) was added dropwise a solution of LAH 1M in THF (718 µL, 718 µmol). The solution was stirred at 0°C for 1.25 hours and then poured on half-saturated aqueous NH₄Cl solution and EtOAc and the layers were separated. The aqueous layer was extracted twice with EtOAc. The organic layers were dried over MgSO₄, filtered, treated with silica gel and evaporated. The compound was purified by silica gel chromatography on a 4 g column using an MPLC system eluting with a gradient of n-heptane : EtOAc (100 : 0 to 50 : 50) to provide the desired compound as a colorless oil (0.151 g; 69.3%). MS (ESI): m/z = 248.2 [M-C₄H₈+H]⁺.

### b) tert-Butyl 3-(4-(1-(methoxycarbonyl)cyclopropyl)phenyl)azetidine-1-carboxylate

The product was obtained in analogy to Method A7 from methyl 1-(4-bromophenyl)cyclopropane-1-carboxylate (CAS RN 638220-35-6) and tert-butyl 3-bromoazetidine-1-carboxylate (CAS RN 1064194-10-0) as a colorless solid. MS (ESI): m/z = 276.2 [M-C₄H₈+H]⁺.

### BB53

### 4-(Azetidin-3-yl)-1-methyl-1H-indazole hydrochloride

The product was obtained in analogy to BB52 a from tert-butyl 3-(1-methyl-1H-indazol-4-yl)azetidine-1-carboxylate as a light yellow solid. MS (ESI): m/z = 188.2 [M+H]⁺.

### Intermediates:

### a) tert-Butyl 3-(1-methyl-1H-indazol-4-yl)azetidine-1-carboxylate

The product was obtained in analogy to Method A7 from 4-bromo-1-methyl-1H-indazole (CAS RN 365427-30-1) and tert-butyl 3-bromoazetidine-1-carboxylate (CAS RN 1064194-10-0) as a light brown oil. MS (ESI): m/z = 232.1 [M-C₄H₈+H]⁺.

### BB54

### 3-[4-(Trifluoromethoxy)phenyl]pyrrolidine trifluoroacetate

The product was obtained in analogy to BB26, intermediate a from tert-butyl 3-[4-(trifluoromethoxy)phenyl]pyrrolidine-1-carboxylate as light brown solid. MS (ESI): m/z = 232.6 [M+H]⁺.

### Intermediates:

### a) tert-Butyl 3-[4-(trifluoromethoxy)phenyl]pyrrolidine-1-carboxylate

The product was obtained in analogy to BB17, intermediate a from tert-butyl 3-[4-(trifluoromethoxy)phenyl]-2,5-dihydropyrrole-1-carboxylate as light yellow oil (200 mg, 66.3%). MS (ESI): m/z = 276.5 [M- C₄H₈+H]⁺.

### b) tert-Butyl 3-[4-(trifluoromethoxy)phenyl]-2,5-dihydropyrrole-1-carboxylate

To a solution of tert-butyl 3-(((trifluoromethyl)sulfonyl)oxy)-2,5-dihydro-1H-pyrrole-1-carboxylate (1 g, 3.15 mmol, CAS RN 630121-86-7) and 4-(trifluoromethoxy)phenylboronic acid (973.57 mg, 4.73 mmol, CAS RN 139301-27-2), Na2CO3 (668.1 mg, 6.3 mmol) in 1,4-Dioxane (20 mL) and water (5 mL) was added [1,1'-bis(diphenylphosphino)ferrocene]dichloropalladium(II) (461.24 mg, 0.630 mmol) and the mixture was stirred at 100 °C under N2 atmosphere for 12 h. The reaction was concentrated and purified by silica gel chromatography (PE:EtOAc=20:1) to get the desired product as light yellow oil (320 mg, 30.8%). MS (ESI): m/z = 274.5 [M-C₄H₈+H]⁺.

### BB55

### Tributyl-(3-chloro-2-pyridyl)stannane

To a solution of 2-bromo-3-chloropyridine (576.0 mg, 2.99 mmol, CAS RN 96424-68-9) in toluene (20 mL) under N2 was added dropwise n-BuLi 2.5 M in hexane (1.32 mL, 3.29 mmol) at -78 °C. The reaction mixture was stirred for 2 h, followed by the addition of tributyltin chloride (1071.73 mg, 3.29 mmol, CAS RN 1461-22-9). The reaction mixture was stirred 2 h at -78 °C, warmed to RT and stirred another 12 h, and then quenched with saturated NH4C1 solution (50 mL). The mixture was extracted with EtOAc (30 mL three times), and the combined organic layers were washed with brine (15 mL), dried (Na₂SO₄), and filtered. The filtrate was concentrated in vacuo to the desired product as a light yellow oil (1.1 g, 91.3%). MS (ESI): m/z = 404.1 [M+H]⁺.

### BB56

### 6-(Azetidin-3-yl)-1-methyl-1H-indazole hydrochloride

The product was obtained in analogy to BB52 a from tert-butyl 3-(1-methyl-1H-indazol-6-yl)azetidine-1-carboxylate as a colorless solid. MS (ESI): m/z = 188.1 [M+H]⁺.

### Intermediates:

### a) tert-Butyl 3-(1-methyl-1H-indazol-6-yl)azetidine-1-carboxylate

The product was obtained in analogy to Method A7 from 6-bromo-1-methyl-1H-indazole (CAS RN 365427-30-1) and tert-butyl 3-bromoazetidine-1-carboxylate (CAS RN 1064194-10-0) as a light yellow oil. MS (ESI): m/z = 288.2 [M+H]⁺.

### BB57

### (4aR,8aS)-6-[3-[3-(Trifluoromethoxy)phenyl]pyrrolidine-1-carbonyl]-4,4a,5,7,8,8a-hexahydropyrido[4,3-b][1,4]oxazin-3-one

The product was obtained in analogy to Method A4 using 3-[3-(trifluoromethoxy)phenyl]pyrrolidine 2,2,2-trifluoroacetate to get the desired product as light yellow solid. MS (ESI): m/z = 414.3 [M+H]⁺.

### Intermediates:

### a) 3-[3-(Trifluoromethoxy)phenyl]pyrrolidine trifluoroacetate

The product was obtained in analogy to BB26, intermediate a from tert-butyl 3-[3-(trifluoromethoxy)phenyl]pyrrolidine-1-carboxylate as light brown oil. MS (ESI): m/z = 232.6 [M+H]⁺.

### b) tert-Butyl 3-[3-(trifluoromethoxy)phenyl]pyrrolidine-1-carboxylate

The product was obtained in analogy to BB54, intermediate a from tert-butyl 3-[3-(trifluoromethoxy)phenyl]-2,5-dihydropyrrole-1-carboxylate to get the desired product as light yellow oil. MS (ESI): m/z = 276.5 [M-C₄H₈+H]⁺.

### c) tert-Butyl 3-[3-(trifluoromethoxy)phenyl)-2,5-dihydropyrrole-1-carboxylate

The product was obtained in analogy to BB54, intermediate b from 3-(trifluoromethoxy)phenylboronic acid (CAS RN 179113-90-7) to get the desired product as a light brown oil. MS (ESI): m/z = 274.5 [M-C₄H₈+H]⁺.

### BB58

### 2-Methyl-3-(4-(trifluoromethoxy)phenyl)azetidine 2,2,2-trifluoroacetate

The product was obtained in analogy to BB26, intermediate a from tert-butyl 2-methyl-3-(4-(trifluoromethoxy)phenyl)azetidine-1-carboxylate as a crude. MS (ESI): m/z = 232.2 [M+H]⁺.

### Intermediates:

### a) tert-Butyl 2-methyl-3-(4-(trifluorometlioxy)phenyl)azetidine-1-carboxylate

A microwave tube was charged with a solution of tert-butyl (Z)-3-(2-((4-methoxyphenyl)sulfonyl)hydrazineylidene)-2-methylazetidine-1-carboxylate (265.5 mg, 719 µmol) and (4-(trifluoromethoxy)phenyl)boronic acid (222 mg, 1.08 mmol, CAS RN 139301-27-2) in Dioxane (2.87 mL). To the solution cesium carbonate (351 mg, 1.08 mmol) was added. The RM was degassed with argon, the vial sealed and heated whislt stirring to 110C for 18 h. The RM was cooled to RT before being quenched with 2 mL saturated aqueous NaHCO₃ solution and extracted three times with DCM. The combined organic layers were dried over MgSO₄ and concentrated in vacuo yielding desired product as a yellow oil (71.1 mg; 29.9%). MS (ESI): m/z = 276.2 [M-C₄H₈+H]⁺.

### b) tert-Butyl (Z)-3-(2-((4-methoxyphenyl)sulfonyl)hydrazineylidene)-2-methylazetidine-1-carboxylate

A solution of 4-methoxybenzenesulfonohydrazide (180 mg, 890 µmol, CAS RN1950-68-1 ) and tert-butyl 2-methyl-3-oxoazetidine-1-carboxylate (165 mg, 890 µmol, CAS RN 1408076-36-7 ) in DMSO d6 (593 µL) was heated to 60C whilst stirring for 1h. The reaction mixture was cooled to RTand poured on stirring H₂O, liberating a white precipitate. The precipitate was filtered and redissolved in MeOH. Solvent was removed in vacuo yielding desired product as a yellow oil (265.5 mg; 80.7%). MS (ESI): m/z = 368.3 [M-H]⁻.

### BB59

### 3-(3,3-Dimethyl-2,3-dihydrobenzofuran-6-yl)azetidine 4-methylbenzenesulfonate

The product was obtained in analogy to BB28 from tert-butyl 3-(3,3-dimethyl-2,3-dihydrobenzofuran-6-yl)azetidine-1-carboxylate as a colorless solid. MS (ESI): m/z = 204.2 [M+H]⁺.

### Intermediate:

### tert-Butyl 3-(3,3-dimethyl-2,3-dihydrobenzofuran-6-yl)azetidine-1-carboxylate

The product was obtained in analogy to method A7 from 6-bromo-3,3-dimethyl-2,3-dihydrobenzofuran (CAS RN 140896-85-1) as a colorless solid. MS (ESI): m/z = 248.2 [M-C₄H₈+H]⁺.

### BB60

### 3-(3-Chloro-5-(2,2,2-trifluoroethoxy)phenyl)pyrrolidine 4-methylbenzenesulfonate

The product was obtained in analogy to BB28 from tert-butyl 3-(3-chloro-5-(2,2,2-trifluoroethoxy)phenyl)pyrrolidine-1-carboxylate as a colorless oil. MS (ESI): m/z = 280.1 [M+H]⁺.

### Intermediates:

### a) tert-Butyl 3-(3-chloro-5-(2,2,2-trifluoroethoxy)phenyl)pyrrolidine-1-carboxylate

The product was obtained in analogy to method A7 from 1-bromo-3-chloro-5-(2,2,2-trifluoroethoxy)benzene as a colorless oil. MS (ESI): m/z = 324.1 [M-C₄H₈+H]⁺.

### b) 1-Bromo-3-chloro-5-(2,2,2-trifluoroethoxy)benzene

To a solution of 3-bromo-5-chlorophenol (330 mg, 1.59 mmol, CAS RN 56962-04-0) and 2,2,2-trifluoroethyl trifluoromethanesulfonate (554 mg, 2.39 mmol) in DMF (3 mL) was added potassium carbonate (440 mg, 3.18 mmol) and the mixture was stirred at 50°C over weekend. After cooling down, the reaction mixture was poured on water and EtOAc and the layers were separated. The aqueous layer was extracted twice with EtOAc. The organic layers were washed twice with water, dried over MgSO₄, filtered and evaporated to furnish the desired compound as a colorless oil (0.484 g; 100%). Compound was used in the next step without further purification.

### BB61

### Methyl 2-(4-(azetidin-3-yl)phenyl)-2-methylpropanoate 4-methylbenzenesulfonate

The product was obtained in analogy to BB28 from tert-butyl 3-(4-(1-methoxy-2-methyl-1-oxopropan-2-yl)phenyl)azetidine-1-carboxylate as a colorless gum. MS (ESI): m/z = 234.2 [M+H]⁺.

### Intermediate:

### tert-Butyl 3-(3,3-dimethyl-2,3-dihydrobenzofuran-6-yl)azetidine-1-carboxylate

The product was obtained in analogy to method A7 from methyl 2-(4-bromophenyl)-2-methylpropanoate (CAS RN 154825-97-5) as a light yellow oil. MS (ESI): m/z = 278.2 [M-C₄H₈+H]⁺.

### BB62

### 3-(3,5-Dichlorophenyl)pyrrolidine 4-methylbenzenesulfonate

The product was obtained in analogy to BB28 from tert-butyl 3-(3,5-dichlorophenyl)pyrrolidine-1-carboxylate as a brown oil. MS (ESI): m/z = 216.0 [M+H]⁺.

### Intermediate:

### tert-Butyl 3-(3,5-dichlorophenyl)pyrrolidine-l-carboxylate

The product was obtained in analogy to method A7 from 1-bromo-3,5-dichlorobenzene (CAS RN 19752-55-7) as a colorless oil. MS (ESI): m/z = 260.1 [M-C₄H₈+H]⁺.

### BB63

### 2-[4-(Azetidin-3-yl)phenyl)-5-(2,2-dimethylpropyl)-1,3,4-oxadiazole 2,2,2-trifluoroacetate

The product was obtained in analogy to BB26, intermediate a from tert-butyl 3-[4-[5-(2,2-dimethylpropyl)-1,3,4-oxadiazol-2-yl]phenyl]azetidine-1-carboxylate as a light brown oil. MS (ESI): m/z = 272.6 [M+H]⁺.

### Intermediates:

### a) tert-Butyl 3-[4-[5-(2,2-dimethylpropyl)-1,3,4-oxadiazol-2-yl]phenyl]azetidine-1-carboxylate

The product was obtained in analogy to method A7 from 2-(4-bromophenyl)-5-(2,2-dimethylpropyl)-1,3,4-oxadiazole as a light brown oil. MS (ESI): m/z = 372.5 [M+H]⁺.

### b) 2-(4-Bromophenyl)-5-(2,2-dimethylpropyl)-1,3,4-oxadiazole

To a solution of 4-bromo-N'-(3,3-dimethylbutanoyl)benzohydrazide (5 g, 15.96 mmol) in toluene (102 mL) was added p-toluenesulfonic acid (5.5 g, 31.93 mmol) then stirred at 110 °C for 12 h. LCMS showed the reaction was complete, the mixture was concentrated and the residue was purified by silica gel chromatography eluted with PE : EtOAc = 10 : 1 to give the desired product as a light yellow solid (1 g, 21.2%). MS (ESI): m/z = 295.4 [M+H]⁺.

### c) 4-Bromo-N'-(3,3-dimethylbutanoyl)benzohydrazide

A solution of 4-bromobenzohydrazide (5.0 g, 23.25 mmol, CAS RN 5933-32-4) and DIPEA (12.39 mL, 69.75 mmol) in DCM (50 mL) was added 3,3-dimethylbutyryl chloride (3.76 g, 27.9 mmol, CAS RN 7065-46-5) under 0°C, the mixture was stirred at 20 °C for 12 h. The aqueous layer was extracted by EtOAc (200 mL, 3 times) and water (100 mL,3 times). The separated organic layer was washed with water, dried over Na₂SO₄ and evaporated to give the desired product as light yellow solid (7 g, 96.1%). MS (ESI): m/z = 315.4 [M+H]⁺.

### BB64

### 3-(4-(tert-Butyl)-3-methoxyphenyl)azetidine 4-methylbenzenesulfonate

The product was obtained in analogy to BB28 from tert-butyl 3-(4-(tert-butyl)-3-methoxyphenyl)azetidine-1-carboxylate as crude. MS (ESI): m/z = 220.2 [M+H]⁺.

### Intermediate:

### tert-Butyl 3-(4-(tert-butyl)-3-methoxyphenyl)azetidine-1-carboxylate

The product was obtained in analogy to method A7 from 4-bromo-1-(tert-butyl)-2-methoxybenzene (CAS RN 30788-02-4) as a light yellow oil. MS (ESI): m/z = 264.2 [M-C₄H₈+H]⁺.

### BB65

### 5-(Azetidin-3-yl)-1-methyl-IH-indazole 4-methylbenzenesulfonate

The product was obtained in analogy to BB28 from tert-butyl 3-(1-methyl-1H-indazol-5-yl)azetidine-1-carboxylate as crude. MS (ESI): m/z = 188.1 [M+H]⁺.

### Intermediate:

### tert-Butyl 3-(1-methyl-1H-indazol-5-yl)azetidine-1-carboxylate

The product was obtained in analogy to method A7 from 5-bromo-1-methyl-1H-indazole (CAS RN 465529-57-1) as a yellow oil. MS (ESI): m/z = 288.2 [M+H]⁺.

### BB66

### 3-(4-Propylphenyl)azetidine 4-methylbenzenesulfonate

The product was obtained in analogy to BB28 from tert-butyl 3-(4-propylphenyl)azetidine-1-carboxylate as crude. MS (ESI): m/z = 176.1 [M+H]⁺.

### Intermediate:

### tert-Butyl 3-(4-propylphenyl)azetidine-1-carboxylate

The product was obtained in analogy to method A7 from 1-bromo-4-propylbenzene (CAS RN 588-93-2) as a light yellow oil. MS (ESI): m/z = 220.2 [M-C₄H₈+H]⁺.

### BB67

### 3-(4-(Trifluoromethoxy)-3-(trifluoromethyl)phenyl)azetidine 4-methylbenzenesulfonate

The product was obtained in analogy to BB28 from tert-butyl 3-(4-(trifluoromethoxy)-3-(trifluoromethyl)phenyl)azetidine-1-carboxylate as crude. MS (ESI): m/z = 286.1 [M+H]⁺.

### Intermediate:

### tert-Butyl 3-(4-(trifluoromethoxy)-3-(trifluoromethyl)phenyl)azetidine-1-carboxylate

The product was obtained in analogy to method A7 from 4-bromo-1-(trifluoromethoxy)-2-(trifluoromethyl)benzene (CAS RN 933674-89-6) as a light yellow oil. MS (ESI): m/z = 330.1 [M-C₄H₈+H]⁺.

### BB68

### 3-[4-[[1-(Trifluoromethyl)cyclopropyl]methoxy]phenyl]azetidine 4-2,2,2-trifluoroacetate

The product was obtained in analogy to BB26, intermediate a from tert-butyl 3-[4-[[1-(trifluoromethyl)cyclopropyl]methoxy]phenyl]azetidine-1-carboxylate as a yellow oil. MS (ESI): m/z = 272.1 [M+H]⁺.

### Intermediate:

### tert-Butyl 3-[4-[[1-(trifluoromethyl)cyclopropyl]methoxy]phenyl]azetidine-1-carboxylate

The product was obtained in analogy to method A7 from 1-bromo-4-[[1-(trifluoromethyl)cyclopropyl]methoxy]benzene (CAS RN 1594130-28-5) as a light yellow oil. MS (ESI): m/z = 316.1 [M-C₄H₈+H]⁺.

### BB69

### 3-[4-(2,2,2-Trifluoro-I,I-dimethyl-ethyl)phenyl]azetidine 2,2,2-trifluoroacetate

The product was obtained in analogy to BB26, intermediate a from tert-butyl 3-[4-(2,2,2-trifluoro-1,1-dimethyl-ethyl)phenyl]azetidine-1-carboxylate as a yellow oil. MS (ESI): m/z = 244.1[M+H]⁺.

### Intermediate:

### tert-Butyl 3-[4-(2,2,2-trifluoro-1,1-dimethyl-ethyl)phenyl]azetidine-1-carboxylate

The product was obtained in analogy to method A7 from 1-bromo-4-(2,2,2-trifluoro-1,1-dimethyl-ethyl)benzene (CAS RN 1225380-05-1) as a light yellow oil. MS (ESI): m/z = 288.1 [M-C₄H₈+H]⁺.

### BB70

### 3-[4-(Azetidin-3-yl)phenyl]-5-(2,2-dimethylpropyl)-1,2,4-oxadiazole 2,2,2-trifluoroacetate

The product was obtained in analogy to BB26, intermediate a from tert-butyl 3-[4-[5-(2,2-dimethylpropyl)-1,2,4-oxadiazol-3-yl]phenyl]azetidine-1-carboxylate as a light yellow oil. MS (ESI): m/z = 272.6 [M+H]⁺.

### Intermediates:

### a) tert-Butyl 3-[4-[5-(2,2-dimethylpropyl)-1,2,4-oxadiazol-3-yl]phenyl]azetidine-1-carboxylate

A solution of tert-butyl 3-[4-(N-hydroxycarbamimidoyl)phenyl]azetidine-1-carboxylate (770.0 mg, 2.64 mmol) and DIPEA (1.41 mL, 7.93 mmol) in toluene (6 mL) was added 3,3-dimethylbutyryl chloride (426.88 mg, 3.17 mmol, CAS RN 7065-46-5) under 0□, the mixture was stirred at 25°C for 10 min, then the temperature was turn up to 80 °C and stirred for 12 h. The mixture was evaporated and the residue was purified by silica gel chromatography (PE : EtOAc = 10 : 1) to give the desired product as light brown oil (620 mg, 63.2%). MS (ESI): m/z = 316.5 [M-C₄H₈+H]⁺.

### b) tert-Butyl 3-[4-(N-hydroxycarbamimidoyl)phenyl]azetidine-1-carboxylate

A solution of hydroxylamine hydrochloride (349.71 mg, 5.03 mmol) in Ethanol (8 mL) was added sodium carbonate (266.69 mg, 2.52 mmol) in water (2 mL),and stirred at 20°C for 25 min. Then tert-butyl 3-(4-cyanophenyl)azetidine-1-carboxylate (1000.0 mg, 3.87 mmol, CAS RN 206446-41-5) was added, the mixture was stirred at 95 °C for 12 h.

The mixture was diluted with water, concentrated under vacuum to remove the EtOH, the residue was partitioned between EtOAc (100 mL) with water (100 mL ×3), then saturated sodium chloride (50 mL), dried over sodium sulfate, and evaporated to give the desired product as light yellow oil (773 mg, 68.5%). MS (ESI): m/z = 292.5 [M+H]⁺.

### BB71

### (4aR,8aS)-6-[3-[4-(2,2,2-Trifluoro-1-methyl-ethoxy)phenyl]azetidine-1-carbonyl]-4,4a,5,7,8,8a-hexahydropyrido[4,3-b][1,4]oxazin-3-one

The product was obtained in analogy to method A4 from 3-[4-(2,2,2-trifluoro-1-methyl-ethoxy)phenyl]azetidine 2,2,2-trifluoroacetate as a light yellow solid. MS (ESI): m/z = 428.3 [M+H]⁺.

### Intermediates:

### a) 3-[4-(2,2,2-Trifluoro-1-methyl-ethoxy)phenyl]azetidine 2,2,2-trifluoroacetate

The product was obtained in analogy to BB26, intermediate a from tert-butyl 3-[4-(2,2,2-trifluoro-1-methyl-ethoxy)phenyl]azetidine-1-carboxylate as a light yellow oil. MS (ESI): m/z = 246.1 [M+H]⁺.

### b) tert-butyl 3-[4-(2,2,2-trifluoro-1-methyl-ethoxy)phenyl]azetidine-1-carboxylate

The product was obtained in analogy to method A7 from 1-bromo-4-(2,2,2-trifluoro-1-methyl-ethoxy)benzene (CAS RN 1239611-43-8 ) as a colorless solid. MS (ESI): m/z = 290.1 [M-C₄H₈+H]⁺.

### BB72

### 3-(3-Methoxy-4-methylphenyl)azetidine 4-methylbenzenesulfonate

The product was obtained in analogy to BB28 from tert-butyl 3-(3-methoxy-4-methylphenyl)azetidine-1-carboxylate as colorless solid. MS (ESI): m/z = 178.1[M+H]⁺.

### Intermediate:

### tert-Butyl 3-(3-methoxy-4-methylphenyl)azetidine-1-carboxylate

The product was obtained in analogy to method A7 from 4-bromo-2-methoxy-1-methylbenzene (CAS RN 67868-73-9) as a light brown oil. MS (ESI): m/z = 222.1 [M-C₄H₈+H]⁺.

### BB73

### 4-((4-Fluorophenyl)((1-methyl-5-(trifluoromethyl)-1H-pyrazol-3-yl)oxy)methyl)piperidine hydrochloride

The product was obtained in analogy to BB16 from tert-butyl 4-((4-fluorophenyl)((1-methyl-5-(trifluoromethyl)-1H-pyrazol-3-yl)oxy)methyl)piperidine-1-carboxylate as colorless foam (0.194 g; 98.0%). MS (ESI): m/z = 358.2 [M+H]⁺.

### Intermediate:

### tert-Butyl 4-((4fluorophenyl)((1-methyl-5-(trifluoromethyl)-1H-pyrazol-3-yl)oxy)methyl)piperidine-1-carboxylate

To a yellow solution of tri-n-butylphosphine (327 mg, 399 µL, 1.62 mmol) and azodicarboxylic dipiperidide (408 mg, 1.62 mmol) in toluene (12.5 mL) was added tert-butyl 4-((4-fluorophenyl)(hydroxy)methyl)piperidine-1-carboxylate (250 mg, 808 µmol, CAS RN 160296-41-3) and the mixture was stirred at RT for 20 min. This led to a pale yellow solution. Addition of 1-methyl-5-(trifluoromethyl)-1H-pyrazol-3-ol (268 mg, 1.62 mmol, CAS RN 119022-51-4 ) gave a suspension. After stirring at RT for 75 min. heating was installed. After stirring at 65°C for 19 h, the reaction mixture was cooled down to RT. Silica gel was added and the mixture was evaporated. The compound was purified by silica gel chromatography on a 12 g column using an MPLC (ISCO) system eluting with a gradient of n-heptane : EtOAc (100 : 0 to 50 : 50) to get the desired compound as a colorless gum (0.230 g; 62.2%). MS (ESI): m/z = 358.2 [M-Boc+H]⁺.

### BB74

### 3-[1-[4-Trifluoromethyl)phenyl]ethoxy]azetidine

To a solution of tert-butyl 3-(1-(4-(trifluoromethyl)phenyl)ethoxy)azetidine-1-carboxylate (0.04 g, 0.116 mmol) in DCM (0.6 mL) was added TFA (0.178 mL, 2.32 mmol) and the reaction mixture was stirred at RT for 30 min. The solvent was removed under reduced pressure and the residue was taken up in EtOAc, poured into a sat. aq. Na₂CO₃ solution (5 mL) and the aqueous layer was extracted twice with EtOAc (10 mL each). The combined organic layers were washed with brine, dried over Na₂SO₄, filtered and concentrated *in vacuo* to give the crude title compound (0.025 g, 88%) as a colorless gum; MS (ESI): m/z = 246.2 [M+H]⁺.

### Intermediate:

### tert-Butyl 3-[1-[4-(trifluoromethyl)phenyl]ethoxy]azetidine-1-carboxylate

To a solution of tert-butyl 3-hydroxyazetidine-1-carboxylate (0.1 g, 0.577 mmol) in DMF (5 mL) cooled to 0°C with an ice bath, was added NaH (60% in mineral oil; 0.023 g, 0.577 mmol) and the reaction mixture was stirred at this temperature for 15 min. Then, 1-(1-bromoethyl)-4-(trifluoromethyl)benzene (0.161 g, 0.635 mmol, CAS RN 68120-42-3) was added and the mixture was allowed to warm up to RT and stirring was continued overnight. The mixture was poured into a sat. aq. solution of ammonium chloride (15 mL) and extracted twice with EtOAc (20 mL each). The organic layers were dried over Na₂SO₄ and concentrated *in vacuo.* The residue was purified by silica gel flash chromatography eluting with a gradient of EtOAc in n-heptane (0% to 70%) to give the title compound (0.045 g, 22.6%) as a colorless amorphous solid. MS (ESI): m/z = 290.2 [M-C₄H₈+H]⁺.

### BB75

### 4-[1-[4-(Trifluoromethyl)phenyl]ethoxy]piperidine hydrochloride

The product was obtained in analogy to BH1, using tert-butyl 4-hydroxypiperidine-1-carboxylate (CAS RN 109384-19-2) in the intermediate step, and then a solution of 4M HCl/dioxane in MeOH instead of TFA/DCM for the deprotection step. Colorless solid; MS (ESI): 274.1 [M+H]⁺.

### BB76

### 5-(Azetidin-3-yl)-2-methoxy-pyridine 4-methylbenzenesulfonate

The product was obtained in analogy to BB28, using tert-butyl 3-(6-methoxypyridin-3-yl)azetidine-1-carboxylate. Colorless solid. MS (ESI) = 165.1 [M+H]⁺.

### Intermediate:

### tert-Butyl 3-(6-methoxypyridin-3-yl)azetidine-1-carboxylate

The product was obtained in analogy to BB35, intermediate, using (6-methoxypyridin-3-yl)boronic acid (CAS RN 163105-89-3), as a colorless oil. MS (ESI) = 265.2 [M+H]⁺.

### BB77

### 3-[3-Chloro-4-(trifluoromethoxy)phenyl)azetidine 4-methylbenzenesulfonate

The product was obtained in analogy to BB28, using tert-butyl 3-[3-chloro-4-(trifluoromethoxy)phenyl]azetidine-1-carboxylate, as a colorless solid; MS (ESI) = 252.1 [M+H]⁺.

### Intermediate:

### tert-Butyl 3-[3-chloro-4-(trifluoromethoxy)phenyl]azetidine-1-carboxylate

The product was obtained in analogy to BB35, intermediate, using (3-chloro-4-(trifluoromethoxy)phenyl)boronic acid (CAS RN 870822-79-0), as a colorless liquid. MS (ESI) = 296.1 [M-C₄H₈+H]⁺.

### BB78

### (4aR,8aS)-6-[3-[3-Bromo-4-(trifluoromethoxy)phenyl]azetidine-1-carbonyl]-4,4a,5,7,8,8a-hexahydropyrido[4,3-b][1,4]oxazin-3-one

The product was obtained in analogy to example 132, using 3-[3-bromo-4-(trifluoromethoxy)phenyl]azetidine 4-methylbenzenesulfonate, as a light yellow solid. MS (ESI) = 480.1 [M+H]⁺.

### Intermediates:

### a) 3-[3-Bromo-4-(trifluoromethoxy)phenyl]azetidine 4-methylbenzenesulfonate

The product was obtained in analogy to BB28, using tert-butyl 3-[3-bromo-4-(trifluoromethoxy)phenyl]azetidine-1-carboxylate, as a colorless solid. MS (ESI) = 296.0 [M+H]⁺.

### b) tert-Butyl 3-[3-bromo-4-(trifluoromethoxy)phenyl]azetidine-1-carboxylate

The product was obtained in analogy to BB35, intermediate, using (3-bromo-4-(trifluoromethoxy)phenyl)boronic acid (MFCD22580724; Apollo Scientific), as a colorless viscous oil. MS (ESI) = 342.0 [M-C₄H₈+H]⁺.

### BB79

### 3-[3-Fluoro-4-(trifluoromethyl)phenyl]azetidine 4-methylbenzenesulfonate

The product was obtained in analogy to BB28, using tert-butyl 3-[3-fluoro-4-(trifluoromethyl)phenyl]azetidine-1-carboxylate. Colorless solid. MS (ESI): 220.1 [M+H]⁺.

### Intermediate:

### tert-Butyl 3-[3fluoro-4-(trifluoromethyl)phenyl)azetidine-1-carboxylate

The product was obtained in analogy to BB37, intermediate, using 4-bromo-2-fluoro-1-(trifluoromethyl)benzene, as a colorless viscous oil. MS (ESI): 264.1 [M-C₄H₈+H]⁺.

### BB80

### (4aR,8aS)-6-[4-[(3,4-Dimethoxyphenyl)-(2-pyridyl)methyl]piperidine-1-carbonyl]-4,4a,5,7,8,8a-hexahydropyrido[4,3-b][1,4]oxazin-3-one

A solution of 2-[(3,4-dimethoxyphenyl)-(4-piperidyl)methyl]pyridine (101 mg, 0.320 mmol), cesium carbonate (97 mg, 0.30 mmol) and (4-nitrophenyl) (4aR,8aS)-3-oxo-4,4a,5,7,8,8a-hexahydropyrido[4,3-b][1,4]oxazine-6-carboxylate (intermediate BB15a) (80 mg, 0.25 mmol) in DMF (5 mL) was stirred at 25 °C for 16 h. The solution was poured into brine (10 mL) and extracted twice with EtOAc (10 mL each). The combined organic layers were concentrated under vacuum to give a residue, that was purified by Prep-HPLC (TFA condition) to give (4aR,8aS)-6-[4-[(3,4-dimethoxyphenyl)-(2-pyridyl)methyl]piperidine-1-carbonyl]-4,4a,5,7,8,8a-hexahydropyrido[4,3-b][1,4]oxazin-3-one (60 mg, 49%) as white solid. MS (ESI): m/z = 495.3 [M+H]⁺.

### Intermediates:

### a) tert-Butyl 4-(3,4-dimethoxybenzoyl)piperidine-1-carboxylate

To a solution at -78 °C of 4-bromoveratrole (12.4 g, 57.3 mmol) in THF (200 mL) was added a butyllithium solution (28.6 mL, 71.6 mmol) and the solution was stirred at -78 °C for 1 h. Then tert-butyl 4-[methoxy(methyl)carbamoyl]piperidine-1-carboxylate (CAS Nr. 139290-70-3) (13.0 g, 47.7 mmol) was added at -78 °C and stirring was continued at -78 °C for 5 h. The solution was poured into brine (20 mL) and extracted with EtOAc (2 × 10 mL). The combined organic layers were concentrated under vacuum to give a residue which was purified by flash column chromatography (PE : EtOAc = 2 : 1) to give the title compound as white solid. MS (ESI): m/z = 372.1 [M+Na]⁺.

### b) tert-Butyl 4-[C-(3,4-dimethoxyphenyl)-N-(p-tolylsulfonylamino)carbonimidoyl]piperidine-1-carboxylate

A solution of 4-methylbenzenesulfonhydrazide (6.40 g, 34.3 mmol) and tert-butyl 4-(3,4-dimethoxybenzoyl)piperidine-1-carboxylate (10.0 g, 28.6 mmol) in 1,4-dioxane (200 mL) was stirred at 80 °C for 24 h. The reaction solution was concentrated under vacuum to give a residue that was purified by flash column chromatography (petroleum ether : EtOAc = 2 : 1) to give the title compound (8.0 g, 54%) as light yellow solid. MS (ESI): m/z = 540.2 [M+Na]⁺.

### c) tert-Butyl 4-[(3,4-dimethoxyphenyl)-(2-pyridyl)methylene]piperidine-1-carboxylate

A mixture of 2-bromopyridine (1.11 mL, 11.6 mmol), tert-butyl 4-[*C*-(3,4-dimethoxyphenyl)-*N*-(*p*-tolylsulfonylamino)carbonimidoyl]piperidine-1-carboxylate (3.00 g, 5.8 mmol), lithium tert-butoxide (557 mg, 6.95 mmol) and bis(triphenylphosphine)palladium(II) chloride (407 mg, 0.580 mmol) in 1,4-dioxane (37 mL) was stirred at 90 °C for 16 h under N₂. Solids were filtered off and the solution was poured into brine (30 mL) and then extracted with EtOAc (2 × 20 mL). The combined organic layers were concentrated under vacuum. The residue was purified by flash column chromatography (petroleum ether : EtOAc = 1 : 1) to give the desired compound as a colorless oil (280 mg, 12%). MS (ESI): m/z = 411.3 [M+H]⁺.

### d) 2-[(3,4-Dimethoxyphenyl)-(4-piperidylidene)methyl]pyridine

A solution of tert-butyl 4-[(3,4-dimethoxyphenyl)-(2-pyridyl)methylene]piperidine-1-carboxylate (280 mg, 0.680 mmol) and trifluoroacetic acid (0.53 mL, 6.82 mmol) in DCM (10 mL) was stirred at 25 °C for 4 h. The solution was concentrated under vacuum to give the title compound as colorless oil (200 mg, 94%). MS (ESI): m/z = 311.1 [M+H]⁺.

### e) 2-[(3,4-Dimethoxyphenyl)-(4-piperidyl)methyl]pyridine

A solution of 2-[(3,4-dimethoxyphenyl)-(4-piperidylidene)methyl]pyridine (200 mg, 0.640

mmol) and Pd/C (69 mg, 0.060 mmol) in DMF (5 mL) was stirred at 25 °C for 6 h under H₂ (760 mmHg). The solution was concentrated under vacuum to give a residue, that was purified by prep-HPLC (TFA condition) to give the compound as colorless oil (150 mg, 71%). MS (ESI): m/z = 313.3 [M+H]⁺.

### BB81

### d) (4aR, 8aS)-6-[4-[(3,4-Dimethoxyphenyl)-(3pyridyl)methyl)piperidine-1-carbonyl)-4,4a,5,7,8,8a-hexahydropyrido[4,3-b][1,4]oxazin-3-one

A solution of 3-[(3,4-dimethoxyphenyl)-(4-piperidyl)methyl]pyridine (126 mg, 0.400 mmol), cesium carbonate (122 mg, 0.370 mmol) and (4-nitrophenyl) (4aR,8aS)-3-oxo-4,4a,5,7,8,8a-hcxahydropyrido[4,3-b][1,4]oxazine-6-carboxylate (intermediate BB15a) (100 mg, 0.310 mmol) in DMF (5 mL) was stirred at 25 °C for 16 h. The solution was poured into brine (10 mL) and extracted with EtOAc (2 × 10 mL). The combined organic layers were concentrated under vacuum to give a residue, that was purified by prep-HPLC (TFA condition) to give the dsesired compound as white solid (80 mg, 52%). MS (ESI): m/z = 495.3 [M+H]⁺.

### Intermediates:

### a) tert-Butyl 4-[(3,4-dimethoxyphenyl)-(3pyridyl)methylene)piperidine-1-carboxylate

A mixture of tert-butyl 4-[*C*-(3,4-dimethoxyphenyl)-*N*-(*p-*tolylsulfonylamino)carbonimidoyl]piperidine-1-carboxylate (intermediate b, examples 118/119) (2.00 g, 3.86 mmol), 3-bromopyridine (0.56 mL, 5.8 mmol), lithium *tert-*butoxide (464 mg, 5.8 mmol) and bis(triphenylphosphine)palladium(II) chloride (271 mg, 0.390 mmol) in DMF (30 mL) was stirred at 90 °C for 16 h under N₂. The mixture was filtered and the filtrate was poured into brine (50 mL) and then extracted with EtOAc (2 × 40 mL). The combined organic layers were concentrated under vacuum to give a residue which was purified by flash column chromatography (petroleum ether : EtOAc = 2 : 1). *tert*-Butyl 4-[(3,4-dimethoxyphenyl)-(3-pyridyl)methylene]piperidine-1-carboxylate was obtained as colorless oil (600 mg, 38%). MS (ESI): m/z = 411.2 [M+H]⁺.

### b) 3-[(3,4-Dimethoxyphenyl)-(4-piperidylidene)methyl]pyridine trifluoroacetate

A solution of tert-butyl 4-[(3,4-dimethoxyphenyl)-(3-pyridyl)methylene]piperidine-1-carboxylate (600 mg, 1.46 mmol) and trifluoroacetic acid (1.13 mL, 15 mmol) in DCM (10 mL) was stirred at 25 °C for 4 h. The solution was concentrated under vacuum to give crude 3-[(3,4-dimethoxyphenyl)-(4-piperidylidene)methyl]pyridine (450 mg, 49%, as TFA salt) as a brown oil, which was used directly in the next step. MS (ESI): m/z = 311.1 [M+H]⁺.

### c) 3-[(3,4-Dimethoxyphenyl)-(4-piperidyl)methyl]pyridine

A mixture of 3-[(3,4-dimethoxyphenyl)-(4-piperidylidene)methyl]pyridine (450 mg, 0.710 mmol) and Pd/C (38 mg, 0.040 mmol) in DMF (10 mL) was stirred at 25 °C for 16 h under H₂ (760 mmHg). The mixture was filtered and concentrated under vacuum to give a residue, which was purified by prep-HPLC (TFA condition) to give the desired compound as colorless oil (220 mg, 99%). MS (ESI): m/z = 313.2 [M+H]⁺.

### BB82

### (4aR,8aS)-6-[4-[(3,4-Dimethoxyphenyl)-(4-pyridyl)methyl]piperidine-1-carbonyl]-4,4a,5,7,8,8a-hexahydropyrido[4,3-b][1,4]oxazin-3-one

A solution of 4-[(3,4-dimethoxyphenyl)-(4-piperidyl)methyl]pyridine (100 mg, 0.320 mmol), cesium carbonate (97 mg, 0.30 mmol) and (4-nitrophenyl) (4aR,8aS)-3-oxo-4,4a,5,7,8,8a-hexahydropyrido[4,3-b][1,4]oxazine-6-carboxylate (intermediate BB15a) (80 mg, 0.25 mmol) in DMF (4 mL) was stirred at 25 °C for 16 h. The solution was poured into brine (10 mL) and extracted twice with EtOAc (10 mL each). The combined organic layers were concentrated under vacuum to give a residue, which was purified by prep-HPLC (TFA condition) to give the title compound as white solid (60 mg, 49%). MS (ESI): m/z = 495.3 [M+H]⁺.

### Intermediates:

### a) tert-Butyl 4-[(3,4-dimethoxyphenyl)-(4pyridyl)methylene)piperidine-1-carboxylate

A mixture of 4-bromopyridine (0.56 mL, 5.8 mmol), tert-butyl 4-[*C*-(3,4-dimethoxyphenyl)-N-(p-tolylsulfonylamino)carbonimidoyl]piperidine-1-carboxylate (intermediate b, examples 118/119) (2.00 g, 3.86 mmol), lithium *tert*-butoxide (464 mg, 5.8 mmol) and bis(triphenylphosphine)palladium(II) chloride (271 mg, 0.390 mmol) in DMF (30 mL) was stirred under N₂ at 90 °C for 16 h. The mixture was filtered and the filtrate was poured into brine (30 mL) and then extracted with EtOAc (2 × 20 mL). The combined organic layers were concentrated under vacuum to give a residue which was purified by flash column chromatography (petroleum ether : EtOAc = 2:1). *tert*-Butyl 4-[(3,4-dimethoxyphenyl)-(4-pyridyl)methylene]piperidine-1-carboxylate (400 mg, 25°%) was obtained as colorless oil. MS (ESI): m/z = 411.3[M+H]⁺.

### b) 4-[(3,4-Dimethoxyphenyl)-(4-piperidylidene)methyl]pyridine

A solution of *tert*-butyl 4-[(3,4-dimethoxyphenyl)-(4-pyridyl)methylene]piperidine-1-carboxylate (400 mg, 0.970 mmol) and trifluoroacetic acid (0.75 mL, 9.7 mmol) in DCM (15 mL) was stirred at 25 °C for 4 h. The solution was concentrated under vacuum to give crude 4-[(3,4-dimethoxyphenyl)-(4-piperidylidene)methyl]pyridine (as TFA salt; 250 mg, 83%) as a brown oil, which was used directly in the next step. MS (ESI): m/z = 311.2 [M+H]⁺.

### c) 4-[(3,4-Dimethoxyphenyl)-(4-piperidyl)methyl]pyridine

A mixture of 4-[(3,4-dimethoxyphenyl)-(4-piperidylidene)methyl]pyridine (250 mg, 0.810 mmol) and Pd/C (43 mg, 0.040 mmol) in DMF (6 mL) was stirred at 25 °C for 16 h under H₂ (760 mmHg). The mixture was filtered and concentrated under vacuum to give a residue, that was purified by prep-HPLC (TFA condition) to give 4-[(3,4-dimethoxyphenyl)-(4-piperidyl)methyl]pyridine (200 mg, 79%) as colorless oil. MS (ESI): m/z = 313.2 [M+H]⁺.

### BB83

### (4aR,8aS)-6-[4-[(3-Methylsulfonylphenyl)-(3-pyridyl)methyl)piperidine-1-carbonyl)-4,4a,5,7,8,8a-hexahydropyrido[4,3-b][1,4]oxazin-3-one

A solution of 3-[(3-methylsulfonylphenyl)-(4-piperidyl)methyl]pyridine (110 mg, 0.330 mmol), and (4-nitrophenyl) (4aR,8aS)-3-oxo-4,4a,5,7,8,8a-hexahydropyrido[4,3-b][1,4]oxazine-6-carboxylate (intermediate BB15a) (160 mg, 0.500 mmol) in pyridine (5.5 mL) was stirred at 90 °C for 16 h. The solution was concentrated under vacuum to give a residue, that was purified by prep-HPLC (TFA condition) to give (4aR,8aS)-6-[4-[(3-methylsulfonylphenyl)-(3-pyridyl)methyl]piperidine-1-carbonyl]-4,4a,5,7,8,8a-hexahydropyrido[4,3-b][1,4]oxazin-3-one (95 mg, 61%) as white solid. MS (ESI): m/z = 513.3 [M+H]⁺.

### Intermediates:

### a) tert-Butyl 4-[N-(p-tolylsulfonylamino)-C-(3-pyridyl)carbonimidoyl]piperidine-1-carboxylate

A solution of 4-methylbenzenesulfonhydrazide (1.54 g, 8.27 mmol) and tert-butyl 4-(pyridine-3-carbonyl)piperidine-1-carboxylate (CAS Nr. 148148-35-0) (2.00 g, 6.89 mmol) in 1,4-dioxane (200 mL) was stirred at 80 °C for 16 h. The reaction mixture was concentrated under vacuum to give a residue that was purified by flash column chromatography (petroleum ether : EtOAc = 2:1) to give *tert*-butyl 4-[*N-*(*p-*tolylsulfonylamino)-C-(3-pyridyl)carbonimidoyl]piperidine-1-carboxylate (2.0 g, 63%) as light yellow solid MS (ESI): m/z = 403.1 [M-C₄H₈+H]⁺.

### b) tert-Butyl 4-[(3-methylsulfonylphenyl)-(3-pyridyl)methylene]piperidine-1-carboxylate

A solution of 3-bromophenylmethylsulfone (1.54 g, 6.54 mmol), *tert*-butyl *4-[N-(p-*tolylsulfonylamino)-C-(3-pyridyl)carbonimidoyl]piperidine-1-carboxylate (2.00 g, 4.36 mmol), lithium tert-butoxide (524 mg, 6.54 mmol)
and bis(triphenylphosphine)palladium(II) chloride (918 mg, 1.31 mmol) in 1,4-dioxane (30 mL) was stirred at 90 °C for 16 h. The reaction was diluted with EtOAc (20 mL) and then filtered through Celite. The filtrate was dried over Na₂SO₄, filtered and concentrated under vacuum. The residue was purified by silica gel chromatography (petroleum ether : EtOAc = 20:1 to 1:1) to afford tert-butyl 4-[(3-methylsulfonylphenyl)-(3-pyridyl)methylene]piperidine-1-carboxylate (400 mg, 21%) as light yellow oil. MS (ESI): m/z = 429.2 [M+H]⁺.

### c) tert-Butyl 4-[(3-methylsulfonylphenyl)-(3pyridyl)methyl)piperidine-1-carboxylate

A mixture of *tert*-butyl 4-[(3-methylsulfonylphenyl)-(3-pyridyl)methylene]piperidine-1-carboxylate (350 mg, 0.820 mmol) and Pd/C (43 mg, 0.410 mmol) in DMF (10 mL) was stirred at 25 °C for 16 h under H₂ (760 mmHg). The mixture was filtered and concentrated under vacuum to give a residue, that was purified by prep-HPLC (basic condition) to give tert-butyl 4-[(3-methylsulfonylphenyl)-(3-pyridyl)methyl]piperidine-1-carboxylate (80 mg, 23%) as colorless oil and recovery of starting material (300 mg), The recovered starting material was subjected again to hydrogenation using the same conditions as above, yielding a second batch of product (110 mg, 31%) as colorless oil. Total yield 190 mg (54%). MS (ESI): m/z = 375.0 [M-C₄H₈+H]⁺.

### d) 3-[(3-Methylsulfonylphenyl)-(4-piperidyl)methyl]pyridine

A solution of tert-butyl 4-[(3-methylsulfonylphenyl)-(3-pyridyl)methyl]piperidine-1-carboxylate (220.0 mg, 0.510 mmol) and trifluoroacetic acid (0.2 mL, 2.55 mmol) in DCM (4.4 mL) was stirred at 25 °C for 4 h. The solution was concentrated under vacuum to give 3-[(3-methylsulfonylphenyl)-(4-piperidyl)methyl]pyridine (as TFA salt; 160 mg, quant.) as colorless oil which was used in the next step without further purification.

### BB84

### (4aR,8aS)-6-[4-[(3-Methylsulfonylphenyt)-(4-pyridyl)methyllpiperidine-1-carbonyl]-4,4a,5,7,8,8a-hexahydropyrido[4,3-bj[1,4]oxazin-3-one

A solution of 4-[(3-methylsulfonylphenyl)-(4-piperidyl)methyl]pyridine (150 mg, 0.450 mmol), and (4-nitrophenyl) (4a*R*,8a*S*)-3-oxo-4,4a,5,7,8,8a-hexahydropyrido[4,3-*b*][1,4]oxazine-6-carboxylate (intermediate BB15a) (219 mg, 0.680 mmol) in pyridine (6 mL) was stirred at 90 °C for 16 h. The solution was concentrated under vacuum to give a reside, which was purified by prep-HPLC (TFA condition) to give (4a*R*,8a*S*)-6-[4-[(3-methylsulfonylphenyl)-(4-pyridyl)methyl]piperidine-1-carbonyl]-4,4a,5,7,8,8a-hexahydropyrido[4,3-*b*][1,4]oxazin-3-one (60 mg, 26%) as colorless oil. MS (ESI): m/z = 513.3 [M+H]⁺.

### Intermediates:

### a) tert-Butyl 4-[N-(p-tolylsulfonylamino)-C-(4-pyridyl)carbonimidoyl]piperidine-1-carboxylate

A solution of 4-methylbenzenesulfonhydrazide (1.31 g, 7.03 mmol) and *tert*-butyl 4-(pyridine-4-carbonyl)piperidine-1-carboxylate (CAS Nr. 1334415-27-8) (1.70 g, 5.85 mmol) in 1,4-dioxane (170 mL) was stirred at 80 °C for 16 h. The reaction mixture was concentrated under vacuum to give a residue which was purified by flash column chromatography (petroleum ether : EtOAc = 2:1) to give *tert*-butyl 4-[*N*-(*p-*tolylsulfonylamino)-C-(4-pyridyl)carbonimidoyl]piperidine-1-carboxylate (1.60 g, 60%) as light yellow solid. MS (ESI): m/z = 459.2 [M+H]⁺.

### b) tert-Butyl 4-[(3-methylsulfonylphenyl)-(4-pyridyl)methylene]piperidine-1-carboxylate

A solution of 3-bromophenylmethylsulfone (1.23 g, 5.23 mmol), *tert*-butyl 4-[*N-*(*p-*tolylsulfonylamino)-C-(4-pyridyl)carbonimidoyl]piperidine-1-carboxylate (1.60 g, 3.49 mmol), lithium *tert*-butoxide (419 mg, 5.23 mmol)
and bis(triphenylphosphine)palladium(II) chloride (735 mg, 1.05 mmol) in 1,4-dioxane (32 mL) was stirred at 90 °C for 16 h. The reaction was diluted with EtOAc (5 mL), then filtered through Celite. The filtrate was separated, dried over Na₂SO₄, filtered and concentrated under vacuum. The residue was purified by silica gel chromatography (petroleum ether : EtOAc = 20:1 to 1:1) to afford *tert*-butyl 4-[(3-methylsulfonylphenyl)-(4-pyridyl)methylene]piperidine-1-carboxylate (420 mg, 28%) as colorless oil. MS (ESI): m/z = 429.2 [M+H]⁺.

### c) tert-Butyl 4-[(3-methylsulfonylphenyl)-(4pyridyl)methyl)piperidine-1-carboxylate

A mixture of tert-butyl 4-[(3-methylsulfonylphenyl)-(4-pyridyl)methylene]piperidine-1-carboxylate (320 mg, 0.750 mmol) and Pd/C (795 mg, 0.750 mmol) in DMF (6.4 mL) was stirred at 25 °C for 16 h under H₂ (760 mmHg). The mixture was filtered and concentrated under vacuum to give *tert*-butyl 4-[(3-methylsulfonylphenyl)-(4-pyridyl)methyl]piperidine-1-carboxylate (280 mg, 87%) as colorless oil. MS (ESI): m/z = 431.2 [M+H]⁺.

### d) 4-[(3-Methylsulfonylphenyl)-(4-piperidyl)methyl]pyridine

A solution of *tert*-butyl 4-[(3-methylsulfonylphenyl)-(4-pyridyl)methyl]piperidine-1-carboxylate (280 mg, 0.650 mmol) and trifluoroacetic acid (0.25 mL, 3.2 mmol) in DCM (6 mL) was stirred at 25 °C for 4 h. The solution was concentrated under vacuum to give a residue, which was purified by prep-HPLC (basic condition) to afford 4-[(3-methylsulfonylphenyl)-(4-piperidyl)methyl]pyridine (150 mg, 61%) as colorless oil. MS (ESI): m/z = 331.2 [M+H]⁺.

### BB85

### (4aR,8aS)-6-[4-[(3-Methylsulfonylphenyl)-(2pyridyl)methyl)piperidine-1-carbonyl)-4,4a,5,7,8,8a-hexahydropyrido[4,3-b][1,4]oxazin-3-one

A solution of oxone (256 mg, 0.420 mmol) and (4aR,8aS)-6-[4-[(3-methylsulfanylphenyl)-(2-pyridyl)methyl]piperidine-1-carbonyl]-4,4a,5,7,8,8a-hexahydropyrido[4,3-b][1,4]oxazin-3-one (100 mg, 0.210 mmol) in water (5 mL) and MeCN (5 mL) was stirred at 25 °C for 16 h. The solution was poured into sat. aq. Na₂CO₃ (10 mL) and extracted with EtOAc (2 × 20 mL). The combined organic layers were concentrated under vacuum to give a residue, that was purified by prep-HPLC (TFA condition) to yield (4a*R*,8a*S*)-6-[4-[(3-methylsulfonylphenyl)-(2-pyridyl)methyl]piperidine-1-carbonyl]-4,4a,5,7,8,8a-hexahydropyrido[4,3-b][1,4]oxazin-3-one (60 mg, 56%) as colorless oil. MS (ESI): m/z = 513.3 [M+H]⁺.

### Intermediates:

### a) tert-Butyl 4-[hydroxy-(3-methylsulfanylphenyl)-(2-pyridyl)methyl]piperidine-1-carboxylate

To a solution at -78 °C of 3-bromothioanisole (1.00 mg, 4.92 mmol) in THF (30 mL) was added over a time of 0.5 h a solution of butyllithium (2.36 mL, 5.91 mmol). Then *tert-*butyl 4-(pyridine-2-carbonyl)piperidine-1-carboxylate (CAS Nr. 416852-19-2) (1.43 g, 4.92 mmol) was added and stirring was continued at -78 °C for 4.5 h. The solution was quenched by sat. aq. NH₄Cl (50 mL) and extracted with EtOAc (2 × 30 mL). The combined organic layers were concentrated under vacuum to give a residue, which was purified by prep-HPLC (TFA condition) to yield tert-butyl 4-[hydroxy-(3-methylsulfanylphenyl)-(2-pyridyl)methyl]piperidine-1-carboxylate (1.40 g, 69%) as colorless oil. MS (ESI): m/z = 415.2 [M+H]⁺.

### b) 2-[Chloro-(3-methylsulfanylphenyl)-(4-piperidyl)methyl]pyridine

A mixture of *tert*-butyl 4-[hydroxy-(3-methylsulfanylphenyl)-(2-pyridyl)methyl]piperidine-1-carboxylate (300 mg, 0.720 mmol), sulfurous dichloride (86 mg, 0.72 mmol) and in Tol (5 mL) was stirred at 25 °C for 6 h under N₂. The mixture was concentrated under vacuum to give 2-[chloro-(3-methylsulfanylphenyl)-(4-piperidyl)methyl]pyridine (220 mg, 91%) as yellow solid, which was used as crude material in the next step. MS (ESI): m/z = 333.1 [M+H]⁺.

### c) 2-[(3-Methylsulfanylphenyl)-(4-piperidyl)methyl]pyridine

A mixture of 2-[chloro-(3-methylsulfanylphenyl)-(4-piperidyl)methyl]pyridine (220 mg, 0.660 mmol), ammonium chloride (35 mg, 0.66 mmol) and zinc (238 mg, 3.64 mmol) in MeOH (6 mL) was stirred at 25 °C for 0.5 h. The mixture was filtered and concentrated under vacuum to give a residue, that was purified by prep-HPLC (basic condition) to yield 2-[(3-methylsulfanylphenyl)-(4-piperidyl)methyl]pyridine (160 mg, 81%) as colorless oil. MS (ESI): m/z = 299.1 [M+H]⁺.

### d) (4aR,8aS)-6-[4-[(3-Methylsulfanylphenyl)-(2pyridyl)methyl)piperidine-1-carbonyl)-4,4a,5,7,8,8a-hexahydropyrido[4,3-b][1,4]oxazin-3-one

A solution of 2-[(3-methylsulfanylphenyl)-(4-piperidyl)methyl]pyridine (160 mg, 0.540 mmol) and (4-nitrophenyl) (4a*R*,8a*S*)-3-oxo-4,4a,5,7,8,8a-hexahydropyrido[4,3-*b*][1,4]oxazine-6-carboxylate (intermediate BB15a) (207 mg, 0.640 mmol) in pyridine (10 mL) was stirred at 90 °C for 16 h. The solution was filtered and concentrated under vacuum to give a residue, which was purified by prep-HPLC (TFA condition) to give (4a*R*,8a*S*)-6-[4-[(3-methylsulfanylphenyl)-(2-pyridyl)methyl]piperidine-1-carbonyl]-4,4a,5,7,8,8a-hexahydropyrido[4,3-*b*][1,4]oxazin-3-one (200 mg, 78%) as white solid. MS (ESI): m/z = 481.2 [M+H]⁺.

### BB86

### 3-[Phenyl(4-piperidyl)methyl)pyridazine hydrochloride

A solution of *tert*-butyl 4-[phenyl(pyridazin-3-yl)methyl]piperidine-1-carboxylate (150 mg, 0.420 mmol) and trifluoroacetic acid (0.1 mL, 1.3 mmol) in DCM (3 mL) was stirred at 25 °C for 4 h. The solution was concentrated under vacuum to give a residue, which was purified by prep-HPLC (HCl condition) to yield 3-[phenyl(4-piperidyl)methyl]pyridazine (as HCl salt, 70 mg, quant.) as grey solid. MS (ESI): m/z = 254.2 [M+H]⁺.

### Intermediates:

### tert-Butyl 4-(chloro-phenyl-pyridazin-3-yl-methyl)piperidine-1-carboxylate

A solution of *tert*-butyl 4-(pyridazine-3-carbonyl)piperidine-1-carboxylate (CAS Nr. 2044281-15-2) (250 mg, 0.860 mmol) and phenylmagnesium bromide (3 M, 0.34 mL, 1.03 mmol) in THF (6 mL) was stirred at 0 °C for 3 h under N₂. The solution was poured into brine (20 mL) and extracted with EtOAc (20 mL). The organic layer was concentrated under vacuum to give a residue, that was purified by prep-HPLC (TFA condition) to afford tert-butyl 4-(hydroxy-phenyl-pyridazin-3-yl-methyl)piperidine-1-carboxylate (200 mg, 63%) as grey solid. MS (ESI): m/z = 314.1 [M-C₄H₈+H]⁺.

### tert-Butyl 4-(chloro-phenyl-pyridazin-3-yl-methyl)piperidine-1-carboxylate

A mixture of *tert*-butyl 4-(hydroxy-phenyl-pyridazin-3-yl-methyl)piperidine-1-carboxylate (200 mg, 0.520 mmol) and sulfurous dichloride (306 mg, 2.59 mmol) in Tol (10 mL) was stirred at 25 °C for 6 h . The mixture was filtered and concentrated under vacuum to give *tert*-butyl 4-(chloro-phenyl-pyridazin-3-yl-methyl)piperidine-1-carboxylate (200 mg, 99%) as yellow solid, that was used as crude material in the next step. MS (ESI): m/z = 410.2 [M+Na]⁺.

### tert-Butyl 4-[phenyl(pyridazin-3-yl)methyl]piperidine-1-carboxylate

A solution of *tert*-butyl 4-(chloro-phenyl-pyridazin-3-yl-methyl)piperidine-1-carboxylate (200 mg, 0.52 mmol), ammonium chloride (28 mg, 0.52 mmol,) and zinc (185 mg, 2.84 mmol) in MeOH (4.7 mL) was stirred at 25 °C for 0.5 h. The mixture was concentrated under vacuum to give a residue, which was purified by prep-HPLC (TFA condition) to yield tert-butyl 4-[phenyl(pyridazin-3-yl)methyl]piperidine-1-carboxylate (150 mg, 82%) as yellow solid. MS (ESI): m/z = 298.1 [M-C₄H₈+H]⁺.

### BB87

### 3-[4-(3-Fluoropropyl)phenyl]azetidine

A solution of *tert*-butyl 3-[4-(3-fluoropropyl)phenyl]azetidine-1-carboxylate (80 mg, 0.27 mmol) and trifluoroacetic acid (0.07 mL, 0.85 mmol) in DCM (2 mL) was stirred at 25 °C for 4 h. The mixture was concentrated under vacuum to give 3-[4-(3-fluoropropyl)phenyl]azetidine (40 mg, 76%) as colorless oil, which was used as such in the next step. MS (ESI): m/z = 194.1 [M+H]⁺.

### Intermediates:

### a) 2-[4-(3-Fluoropropyl)phenyl)-4,4,5,5-tetramethyl-1,3,2-dioxaborolane

A solution of 1-bromo-4-(3-fluoropropyl)benzene (CAS Nr. 168104-62-9) (1.00 g, 4.61 mmol), bis(pinacolato)diboron (2.34 g, 9.21 mmol), [Pd(dppf)Cl₂]·CH₂Cl₂ (376 mg, 0.460 mmol) and KOAc (1.35 g, 13.8 mmol) in 1,4-dioxane (40 mL) was stirred at 100 °C for 16 h under N₂. The mixture was filtered and concentrated to give a residue, that was purified by flash column chromatography (petroleum ether : EtOAc = 10:1) to give 2-[4-(3-fluoropropyl)phenyl]-4,4,5,5-tetramethyl-1,3,2-dioxaborolane (1.2 g, 99%) as colorless oil. MS (ESI): m/z = 265.1 [M+H]⁺.

### b) [4-(3-Fluoropropyl)phenyl]boronic acid

To a solution of 2-[4-(3-fluoropropyl)phenyl]-4,4,5,5-tetramethyl-1,3,2-dioxaborolane (600 mg, 2.27 mmol) in H₂O (12 mL) and acetone (60 mL) was added HCl (1M, 1 mL). The solution was stirred at 25 °C for 16 h. The solution was poured into brine (40 mL) and extracted with EtOAc (2 × 20 mL). The combined organic layers were concentrated under vacuum to give [4-(3-fluoropropyl)phenyl]boronic acid (300 mg, 73%) as light yellow solid. ¹H NMR (400 MHz, CHLOROFORM-d) *δ* = 8.19 (d, *J*=7.9 Hz, 2H), 7.37 (d, *J*=7.8 Hz, 2H), 4.52 (dt, *J*=47.2, 5.9 Hz, 2H), 2.87 (t, *J*=7.1 Hz, 2H), 2.16 - 2.02 (m, 2H).

### c) tert-butyl 3-[4-(3-fluoropropyl)phenyl]azetidine-1-carboxylate

A solution of [4-(3-fluoropropyl)phenyl]boronic acid (145 mg, 0.790 mmol), *tert*-butyl 3-iodoazetidine-1-carboxylate (CAS Nr. 254454-54-1) (150 mg, 0.530 mmol), nickel(II) iodide (99 mg, 0.32 mmol) (1S,2S)-2-aminocyclohexanol (37 mg, 0.32 mmol) and sodium bis(trimethylsilyl)amide (1.06 mL, 1.06 mmol) in 2-propanol (4 mL) was stirred at 80 °C for 1 h in a sealed tube. The mixture was filtered and concentrated under vacuum to give a residue, which was purified by prep-HPLC (TFA condition) to yield *tert*-butyl 3-[4-(3-fluoropropyl)phenyl]azetidine-1-carboxylate (82 mg, 46%) as colorless oil. MS (ESI): m/z = 238.1 [M-C₄H₈+H]⁺.

### BB88

### 2-(Azetidin-3-yl)-5-(2,4-dichlorophenyl)-1,3,4-oxadiazole

Compound tert-butyl 3-[[(2,4-dichlorobenzoyl)amino]carbamoyl]azetidine-1-carboxylate (800.0 mg, 2.06 mmol) was suspended in polyphosphoric acid (5 mL), the mixture was stirred at 180 °C for 2 h. The mixture was poured into ice ammonia (100 mL), stirred for 10 min, then extracted three times with EtOAc (100 mL each), the combined organic phase was washed with brine, dried over Na₂SO₄ and concentrated. The residue was purified by reversed flash column chromatography (0.1% v/v FA) to give the desired product (130 mg, 17%) as a light brown oil. MS (ESI): m/z = 270.4 [M+H]⁺.

### Intermediate:

### tert-Butyl 3-[[(2,4-dichlorobenzoyl)amino)carbamoyl)azetidine-1-carboxylate

To a solution of 1-Boc-azetidine-3-carboxylic acid (490.68 mg, 2.44 mmol, CAS RN 142253-55-2) and 2,4-dichlorobenzhydrazide (500.0 mg, 2.44 mmol, CAS RN 5814-06-2), DIPEA (1.27 mL, 7.32 mmol) in DMF (20 mL) was added T₃P (1122.42 mg, 4.88 mmol, 50% in EtOAc), the mixture was stirred at 80 °C for 12h. The mixture was diluted with EtOAc (50 mL), washed with NaHCO₃ (100 mL) and brine, then dried over Na₂SO₄ and evaporated to give the crude product (800 mg, 84.5%) as a light brown oil. MS (ESI): m/z = 332.4 [M-C₄H₈+H]⁺.

### BB89

### 3-(Azetidin-3-yl)-1-(2,4-dichlorophenyl)pyrazole, trifluoroacetic acid salt

To a solution of tert-butyl 3-[1-(2,4-dichlorophenyl)pyrazol-3-yl]azetidine-1-carboxylate (350.0 mg, 0.950 mmol) in DCM (5 mL) was added TFA (1.0 mL, 0.950 mmol) and the mixture was stirred at 20 °C for 12 h. The mixture was concentrated to give the desired product as a yellow oil (350 mg, 96.4%). MS (ESI): m/z = 268.1 [M+H]⁺.

### Intermediates:

### a) tert-Butyl 3-[methoxy(methyl)carbamoyl]azetidine-1-carboxylate

To a solution of 1-BOC-azetidine-3-carboxylic acid (10.0 g, 49.7 mmol, CAS RN 142253-55-2) in DCM (200 mL) was added CDI (8.06 g, 49.7 mmol), the mixture was stirred at 20 °C for 1 h, then TEA (13.85 mL, 99.39 mmol) and O,N-dimethylhydroxylamine HCl salt (5.82 g, 59.64 mmol, CAS RN 6638-79-5) was added, the mixture was stirred at 20 °C for 15h, then the mixture was washed with aqueous Na₂CO₃ and brine, dried over Na₂SO₄, filtered and the filtrate was concentrated to give crude product as light yellow oil (12 g) which was used in the next step without further purification.

### b) tert-Butyl 3-acetylazetidine-1-carboxylate

To a solution of tert-butyl 3-[methoxy(methyl)carbamoyl]azetidine-1-carboxylate (1000.0 mg, 4.09 mmol) in THF (30 mL) was added MeMgBr/THF (1.77 mL, 3M) at 0 °C, the mixture was stirred at 20 °C for 2 h, then the mixture was poured into sat. NH₄Cl solution (100 mL) and extracted with EtOAc (30 mL three times), the combined organic phase was washed with brine, dried over Na₂SO₄, and concentrated to give the desired product as light yellow oil (810 mg, 99.3%). ¹H NMR (400 MHz, CHLOROFORM-d) δ = 4.05 (br d, J = 7.4 Hz, 4H), 3.48 - 3.34 (m, 1H), 2.18 (d, J = 2.0 Hz, 2H), 1.44 (d, J = 2.0 Hz, 9H).

### c) tert-Butyl 3-[(E)-3-(dimethylamino)prop-2-enoyl]azetidine-1-carboxylate

A solution of tert-butyl 3-acetylazetidine-1-carboxylate (500.0 mg, 2.51 mmol) in N,N-dimethylformamide dimethyl acetal (10.0 mL) was stirred at 110 °C for 12 h, the mixture was concentrated to give the title compound as yellow oil (640 mg, 2.52 mmol). MS (ESI): m/z = 199.2 [M-C₄H₈+H]⁺.

### d) tert-Butyl 3-[1-(2,4-dichlorophenyl)pyrazol-3-yl]azetidine-1-carboxylate

A solution of tert-butyl 3-[(E)-3-(dimethylamino)prop-2-enoyl]azetidine-1-carboxylate (500.0 mg, 1.97 mmol) and 2,4-dichlorobenzohydrazide hydrochloride (474.78 mg, 1.97 mmol) in EtOH (30 mL) was stirred at 80 °C for 12 h, the mixture was concentrated, the residue was purified by silica gel column (PE : EtOAc = 20 : 1 to 3 : 1) to give the desired product (450 mg, 62.2%) as yellow oil. MS (ESI): m/z = 368.1 [M+H]⁺.

### BB90

### 3-[4-(2,2-Dimethylpropyl)phenyl]azetidine, trifluoroacetic acid salt

To a solution of tert-butyl 3-[4-(2,2-dimethylpropyl)phenyl]azetidine-1-carboxylate (500.0 mg, 1.65 mmol) in DCM (10 mL) was added TFA (2.0 mL) and the mixture was stirred at 20 °C for 12 h. The mixture was concentrated to give crude product as a light yellow oil (520 mg). MS (ESI): m/z = 204.2 [M+H]⁺.

### Intermediates:

### a) 1-Bromo-4-(2,2-dimethylpropyl)benzene

To a solution of 2,2-dimethylpropylbenzene (500.0 mg, 3.37 mmol, CAS RN 1007-26-7) in acetic acid (10 mL) was added bromine (0.17 mL, 3.37 mmol). The mixture was stirred at 20 °C in the dark for 12 h, and then the mixture was poured into sat. aq. Na₂SO₃ (30 mL) and extracted three times with EtOAc (10 mL each). The combined organic phases were washed with brine, dried over Na₂SO₄ and concentrated to give the crude product as a light yellow oil (600 mg, 78.3%) that was used in the next step without further ourification.

### b) tert-Butyl 3-[4-(2,2-dimethylpropyl)phenyl]azetidine-1-carboxylate

To an 40 mL vial equipped with a stirring bar was added tert-butyl 3-bromoazetidine-1-carboxylate (519.75 mg, 2.2 mmol, CAS RN 1064194-10-0), 1-bromo-4-(2,2-dimethylpropyl)benzene (500.0 mg, 2.2 mmol), Ir[dF(CF₃)ppy]₂(dtbbpy)PF₆ (24.68 mg, 0.020 mmol, CAS RN 870987-63-6), NiCl₂glyme (2.42 mg, 0.010 mmol, CAS RN 29046-78-4), 4-tert-butyl-2-(4-tert-butyl-2-pyridyl)pyridine (3.54 mg, 0.010 mmol, CAS RN 69641-93-6), bis(trimethylsilyl)silyl-trimethyl-silane (547.37 mg, 2.2 mmol, CAS RN 1873-77-4) and Na₂CO₃ (466.63 mg, 4.4 mmol) then DME (20 mL). The vial was sealed and placed under nitrogen. The reaction mixture was stirred and irradiated with a 34W blue LED lamp (7 cm distance), with cooling fan to keep the reaction temperature at 25 °C for 14 h. The mixture was filtered and the filtrate was concentrated. The residue was purified by reversed flash (0.1% v/v FA) to give desired product (500 mg, 74.8%) as a light yellow oil. MS (ESI): m/z = 248.2 [M-C₄H₈+H]⁺.

### BB91

### (4aR,8aS)-6-[3-[4-(4,4,5,5-Tetramethyl-1,3,2-dioxaborolan-2-yl)phenyl]azetidine-1-carbonyl)-4,4a,5,7,8,8a-hexahydropyrido[4,3-b][1,4]oxazin-3-one

To a solution of (4aR,8aS)-6-[3-(4-bromophenyl)azetidine-1-carbonyl]-4,4a,5,7,8,8a-hexahydropyrido[4,3-b][1,4]oxazin-3-one (200.0 mg, 0.510 mmol, Example 110) and PdCl₂(dppf)·DCM (35.56 mg, 0.050 mmol) was added potassium acetate (149.36 mg, 1.52 mmol) and bis(pinacolato)diboron (193.23 mg, 0.760 mmol), the reaction mixture was purged with nitrogen and stirred at 90 °C for 12 h. The reaction mixture was diluted with water and extracted three times with EtOAc. The combined organic layers were washed with water and brine, dried over sodium sulfate and concentrated in vacuum to get yellow residue, which was purified by reverse flash chromatography (0.1% v/v FA) to procide the desired product (140 mg, 0.320 mmol, 62.5%) as light yellow solid. MS (ESI): m/z = 442.3 [M+H]⁺.

### BB92

### 2-Bromo-N-ethyl-5-methyl-benzamide

To a solution of 2-bromo-5-methylbenzoic acid (500.0 mg, 2.33 mmol, CAS RN 6967-82-4) and HOBT (534.11 mg, 3.49 mmol), EDCI (541.43 mg, 3.49 mmol) and DIPEA (1.21 mL, 6.98 mmol) in DMF (10 mL) was added ethylamine hydrochloride (227.51 mg, 2.79 mmol) and the mixture was stirred at 20 °C for 12 h. Then the mixture was poured into sat. aq. Na₂CO₃ solution (50 mL) and extracted three times with EtOAc (20 mL each). The combined organic layers were washed with brine and dried over Na₂SO₄ and concentrated to give the desired product as light yellow solid (450 mg, 79.9%). MS (ESI): m/z = 242.0 [M+H]⁺.

### BB93

### Azetidin-3-ylbis(4-fluorophenyl)methanol

A dried 100 mL two-neck round-bottom flask with equipped reflux condenser under argon was charged with THF (18.6 mL) and 1-(tert-butyl) 3-methyl azetidine-1,3-dicarboxylate (800 mg, 746 µL, 3.72 mmol, CAS RN 610791-05-4). The mixture was purged with argon for 5 min and cooled to 0 °C. Then, (4-fluorophenyl)magnesium bromide, 0.8 M solution in THF (18.6 mL, 14.9 mmol) was added over 10 min. After complete addition the reaction mixture was heated to 85 °C (oil bath) for 17 h. The reaction was quenched with water (5 mL), diluted with EtOAc (10 mL) and the resulting slurry was stirred for 15 min. Water was added and the mixture was acidified with 2 M HCl (20 mL) until the aq. phase became a colorless solution. The phases were separated and the aqueous phase was extracted with EtOAc (100 mL). The pH was adjusted to 7 and the aqueous phase was extracted four times with EtOAc. The combined organic layers were concentrated to about 50 mL. A precipitate formed and the flask was cooled to 4 °C. The precipitate was filtered and the white solid was washed with small portions of EtOAc to yield the title compound (317 mg, 31%). MS (ESI): m/z = 276.2 [M+H]⁺.

### BB94

### 3-(1-(2-Chloro-4-(trifluoromethyl)phenoxy)ethyl)azetidine 2,2,2-trifluoroacetate

Trifluoroacetic acid (400 mg, 270 µL, 3.51 mmol) was added to a solution of tert-butyl 3-(1-(2-chloro-4-(trifluoromethyl)phenoxy)ethyl)azetidine-1-carboxylate (78.4 mg, 206 µmol) in DCM (1.03 mL) and the solution was stirred at RT for 2 h. The solvent was removed under reduced pressure to afford the compound as a light-yellow oil (134 mg, 100%.The crude product was used in the next step without further purification. MS (ESI): m/z = 280.1 [M+H]⁺.

### Intermediate

### tert-Butyl 3-(1-(2-chloro-4-(trifluoromethyl)phenoxy)ethyl)azetidine-1-carboxylate

To a solution of 2-chloro-4-(trifluoromethyl)phenol (500 mg, 340 µL, 2.54 mmol), tert-butyl 3-(1-hydroxyethyl)azetidine-1-carboxylate (512 mg, 2.54 mmol, CAS RN 1138331-90-4) and triphenylphosphine (734 mg, 2.8 mmol) in DCM (12.7 mL) was added DIAD (566 mg, 544 µL, 2.8 mmol) dropwise at 0 °C and the reaction was stirred at 0 °C for 10 min and at RT for 6 h. The reaction mixture was quenched by addition of sat. aq. NaHCO₃ solution (20 mL). The phases were separated and the aq. phase was extracted with DCM twice. The combined organic layers were dried over Na₂SO₄, filtered and stored at 4 °C for four days. The crude product was concentrated to dryness, immobilized on Isolute^{®} and purified by flash column chromatography (eluting with a gradient of 0 to 30% EtOAc in n-heptane) to afford the title compound as a pale oil (497 mg, 48.9%). MS (ESI): m/z = 324.1 [M-C₄H₈+H]⁺.

### BB95

### 4-(1-(2-Chloro-4-fluorophenoxy)-2,2,2-trifluoroethyl)piperidine hydrochloride

Hydrochloric acid (4M in dioxane) (174 µL, 695 µmol) was added to a solution of tert-butyl 4-(1-(2-chloro-4-fluorophenoxy)-2,2,2-trifluoroethyl)piperidine-1-carboxylate (17.9 mg, 43.5 µmol) in dioxane (435 µL) and the solution was stirred for 2 h at RT. The solvent was removed under reduced pressure to afford the desired compound as a light-yellow oil (15 mg, 95%). The crude product was used in the next step without further purification. MS (ESI): m/z = 312.1 [M+H]⁺.

### Intermediates

### a) tert-Butyl 4-(1-(2-chloro-4-fluorophenoxy)-2,2,2-trifluoroethyl)piperidine-1-carboxylate

Tert-butyl 4-(2,2,2-trifluoro-1-(((trifluoromethyl)sulfonyl)oxy)ethyl)piperidine-1-carboxylate (126 mg, 303 µmol), 2-chloro-4-fluorophenol (48.9 mg, 36.4 µL, 334 µmol) and cesium carbonate (109 mg, 334 µmol) were suspended in DMF (1.52 mL) and stirred at RT for 20 h. The reaction mixture was then quenched with water and extracted two times with EtOAc. The combined organic phases were washed with water and brine, dried over MgSO₄ and concentrated in vacuo. The residue was immobilized on Isolute^{®} and purified by flash column chromatography (0 to 30% gradient EtOAc in n-heptane) to afford the title compound as a colourless oil (20 mg, 15%). MS (ESI): m/z = 356.1 [M-C₄H₈+H]⁺.

### b) Tert-Butyl 4-(2,2,2-trifluoro-1-(((trifluoromethyl)sulfonyl)oxy)ethyl)piperidine-1-carboxylate

A microwave vial was charged with tert-butyl 4-(2,2,2-trifluoro-1-hydroxyethyl)piperidine-1-carboxylate (100 mg, 353 µmol, CAS RN 184042-83-9). The vial was placed under argon, DCM (1.76 mL) was added and cooled to 0°C. Pyridine (33.5 mg, 34.3 µL, 424 µmol) was added, followed by triflic anhydride (110 mg, 65.6 µL, 388 µmol). The reaction mixture was stirred at 0° C for 1 h and then quenched with water. The mixture was separated and the organic phase was washed with water and extracted three times with DCM. The combined organic layers were washed with brine, dried over MgSO₄ and concentrated in vacuo affording the desired compound as a yellow oil (126 mg, 85.9%). The compound was used in the next step without further purification.

### BB96

### 3-(bis(4-Fluorophenyl)methyl)azetidine

A solution of 3-(bis(4-fluorophenyl)methylene)azetidine (20 mg, 77.7 µmol) in MeOH (777 µL) was evacuated and back-filled with argon five times. Under an argon atmosphere, Pd-C (4.14 mg, 3.89 µmol) was added, and the atmosphere was replaced with hydrogen three times. The reaction was stirred under a hydrogen atmosphere (balloon) at 1 bar for 19 h. The atmosphere was replaced with argon and the reaction mixture was filtered over a pad of Dicalite. The filter cake was washed with MeOH. The filtrate was concentrated to give the desired compound as a yellow solid (20.1 mg, 94.7%) which was used in the next step without further purification. MS (ESI): m/z = 260.2 [M+H]⁺.

### Intermediate

### 3-(bis(4-Fluorophenyl)methylene)azetidine

To a suspension of azetidin-3-ylbis(4-fluorophenyl)methanol (244 mg, 886 µmol, BB93) in DCM (2.22 mL) was added TFA (2.22 mL) and the mixture was stirred for 3.5 h. This afforded a homogeneous solution. The reaction was evaporated to dryness, the resulting residue diluted with EtOAc, washed twice with saturated aqueous sodium bicarbonate solution, then with brine, dried over Na₂SO₄ and filtered. The filtrate was concentrated in vacuo to dryness and the residue was trituated with EtOAc/n-heptane and filtered. This afforded the title compound as a white solid (20 mg, 8.3%). MS (ESI): m/z = 258.2 [M+H]⁺.

### BB97

### 3-(1-(2-Fluoro-4-(trifluoromethyl)phenoxy)ethyl)azetidine 2,2,2-trifluoroacetate

To a solution of tert-butyl 3-(1-(2-fluoro-4-(trifluoromethyl)phenoxy)ethyl)azetidine-1-carboxylate (93 mg, 256 µmol) in DCM (1 mL) was added 2,2,2-trifluoroacetic acid (467 mg, 316 µL, 4.1 mmol) and the reaction was stirred at RT for 20 h. The reaction mixture was concentrated to afford the desired compound as a colorless oil (112.8 mg, 99%) which was used in the next step without further purification. MS (ESI): m/z = 264.1 [M+H]⁺.

### Intermediate

### a) tert-Butyl 3-(1-(2-fluoro-4-(trifluoromethyl)phenoxy)ethyl)azetidine-1-carboxylate

To a solution of 2-fluoro-4-(trifluoromethyl)phenol (100 mg, 69.9 µL, CAS RN 77227-78-2), tert-butyl 3-(1-hydroxyethyl)azetidine-1-carboxylate (112 mg, 555 µmol, CAS RN 1138331-90-4) and triphenylphosphine (160 mg, 611 µmol) in DCM (2.8 mL) was added DIAD (124 mg, 119 µL, 611 µmol) dropwise and the reaction was stirred at RT for 1.5 h.

The reaction mixture was quenched by addition of sat. aq. NaHCO₃ solution. The phases were separated and the aqueous phase was extracted with DCM. The combined organic layers were dried over Na₂SO₄, filtered and concentrated in vacuo. The residue was immobilized on Isolute and purified by flash column chromatography (0 to 20% gradient of EtOAc in n-heptane) to afford the desired compound as a colorless oil (93 mg, 43.8%). MS (ESI): m/z = 308.1 [M-C₄H₈+H]⁺.

### BB98

### 3-(1-(4-Trifluoromethyl)phenoxy)ethyl)azetidine 2,2,2-trifluoroacetate

In analogy to BB97, BB98 was generated from 4-(trifluoromethyl)phenol and tert-butyl 3-(1-hydroxyethyl)azetidine-1-carboxylate. The compound was used in the next step without further purification.

### BB99

### (S or R)-4-((4-Fluorophenyl)(3-methoxyphenyl)methyl)piperidine

A solution of 4-[(4-fluorophenyl)-(3-methoxyphenyl)methyl]piperidine (760 mg, 2.54 mmol) in MeOH (5 mL) was purified by SFC separation to afford 4-[(S or R)-(4-fluorophenyl)-(3-methoxyphenyl)methyl]piperidine (128 mg, 0.43 mmol, 17%) as light yellow semisolid; MS (ESI): m/z = 300.1 [M+H]⁺; and 4-[(R or S)-(4-fluorophenyl)-(3-methoxyphenyl)methyl]piperidine (170 mg, 0.57 mmol, 22%) as light yellow semisolid; MS (ESI): m/z = 300.1 [M+H]⁺.

### Intermediates

### a) tert-Butyl 4-[(4fluorophenyl)(hydroxy)(3-methoxyphenyl)methyl]piperidine-1-carboxylate

To a solution of 3-bromoanisole (487 mg, 2.6 mmol) in THF (40 mL) was added butyllithium (1.5 mL, 3.75 mmol, 2.5 M) at -78 °C. After stirring for 1 h, tert-butyl 4-(4-fluorobenzoyl)piperidine-1-carboxylate (CAS RN 160296-40-2; 800 mg, 2.6 mmol) was added to the mixture and stirring was continued at -78 °C for 1 h. Then the reaction was warmed to 25 °C and stirred for another 13 h. The reaction was quenched with NH₄Cl (sat. aq., 50 mL) and extracted with EtOAc (100 mL). The organic layer was separated, dried over Na₂SO₄, filtered and concentrated under *vacuum* to afford the desired compound (1100 mg, 63%) as yellow oil. MS (ESI): m/z = 438.1 [M+Na]⁺.

### b) 4-[(4-Fluorophenyl)(3-methoxyphenyl)methylene]piperidine trifluoroacetate

A solution of tert-butyl 4-[(4-fluorophenyl)(hydroxy)(3-methoxyphenyl)methyl]piperidine-1-carboxylate (1.1 g, 1.64 mmol) and trifluoroacetic acid (5.0 mL, 65 mmol) in DCM (10 mL) was stirred at 25 °C for 5 h. The reaction was concentrated under *vacuum.* The residue was dissolved in EtOAc (50 mL) and washed with Na₂CO₃ (aq., 10%, 50 mL). The organic layer was separated, dried with over Na₂SO₄, filtered and concentrated under *vacuum.* The resulting oil was purified by preparative HPLC (TFA as additive) to afford 4-[(4-fluorophenyl)(3-methoxyphenyl)methylene]piperidine (TFA salt, 430 mg, 39%) as a white solid. MS (ESI): m/z = 298.1 [M+H]⁺.

### c) 4-[(4-Fluorophenyl)(3-methoxyphenyl)methyl]piperidine trifluoroacetate

A solution of 4-[(4-fluorophenyl)(3-methoxyphenyl)methylene]piperidine (410 mg, 1.38 mmol) and Pd/C (100 mg, 1.38 mmol) in THF (10 mL) was stirred at 25 °C for 16 h under H₂ (760 mm Hg). The reaction mixture was filtered through the Celite, then concentrated under *vacuum* to afford 4-[(4-fluorophenyl)(3-methoxyphenyl)methyl]piperidine (TFA salt, 260 mg, 59%) as yellow oil. MS (ESI): m/z = 300.1 [M+H]⁺.

### BB100

### (R or S)-4-((4-Fluorophenyl)(3-methoxyphenyl)methyl)piperidine

A solution of 4-[(4-fluorophenyl)-(3-methoxyphenyl)methyl]piperidine (BB99, intermediate c, 760 mg, 2.54 mmol) in MeOH (5 mL) was purified by SFC separation to afford 4-[(S or R)-(4-fluorophenyl)-(3-methoxyphenyl)methyl]piperidine (128 mg, 17%) as light yellow semisolid; LCMS: 300.1 [M+H]⁺; and 4-[(R or S)-(4-fluorophenyl)-(3-methoxyphenyl)methyl]piperidine or 4-[(S)-(4-fluorophenyl)-(3-methoxyphenyl)methyl]piperidine (170 mg, 22%) as light yellow semisolid; MS (ESI): m/z = 300.1 [M+H]⁺.

### BB101

### (S or R)-4-((3-Methoxyphenyl)(phenyl)methyl)piperidine

A solution of 4-[(3-methoxyphenyl)-phenyl-methyl]piperidine (960 mg, 3.41 mmol) in MeOH (5 mL) was purified by preparative HPLC using TFA as additive to afford 4-[-(3-methoxyphenyl)-phenyl-methyl]piperidine as its TFA salt (1260 mg). 4-[-(3-methoxyphenyl)-phenyl-methyl]piperidine TFA salt (1260 mg) was purified by SFC separation to afford (S or R)-4-((3-methoxyphenyl)(phenyl)methyl)piperidine (443 mg, 45%) as light yellow solid; MS (ESI): m/z = 282.2 [M+H]⁺; and (R or S)-4-((3-methoxyphenyl)(phenyl)methyl)piperidine (383 mg, 39%) as yellow solid; MS (ESI): m/z = 282.2 [M+H]⁺.

### Intermediate

### 4-((3-Methoxyphenyl)(phenyl)methyl)piperidine

The title compound was prepared in analogy to intermediate 1-[(4-fluorophenyl)-phenylmethyl]piperazine (CAS RN 27064-89-7), starting from 3-bromoanisole and tert-butyl 4-benzoylpiperidine-1-carboxylate (CAS RN 922504-27-6) to give the title compound as light yellow semisolid. MS (ESI): m/z = 282.1 [M+H]⁺.

### BB102

### (R or S)-4-((3-Methoxyphenyl)(phenyl)methyl)piperidine

A solution of 4-[(3-methoxyphenyl)-phenyl-methyl]piperidine (BB101, intermediate, 960 mg, 3.41 mmol) in MeOH (5 mL) was purified by preparative HPLC using TFA as additive to afford 4-[-(3-methoxyphenyl)-phenyl-methyl]piperidine as its TFA salt (1260 mg). 4-[-(3-Methoxyphenyl)-phenyl-methyl]piperidine TFA salt (1260 mg) was purified by SFC separation to afford (S or R)-4-((3-methoxyphenyl)(phenyl)methyl)piperidine (443 mg, 45%) as light yellow solid; MS (ESI): m/z = 282.2 [M+H]⁺; and (R or S)-4-((3-methoxyphenyl)(phenyl)methyl)piperidine (383 mg, 39%) as yellow solid; MS (ESI): m/z = 282.2 [M+H]⁺.

### BB103

### (S or R)-4-((3-(2-Fluoroethoxy)phenyl)(phenyl)methyl)piperidine

A solution of 4-[[3-(2-fluoroethoxy)phenyl]-phenyl-methyl]piperidine (800 mg, 2.55 mmol) in MeOH (5 mL) was purified by SFC separation to afford (S or R)-4-((3-(2-fluoroethoxy)phenyl)(phenyl)methyl)piperidine (214 mg, 27%) as white solid; MS (ESI): m/z = 314.1 [M+H]⁺; and (R or S)-4-((3-(2-fluoroethoxy)phenyl)(phenyl)methyl)piperidine (305 mg, 38%) as white solid; MS (ESI): m/z = 314.1 [M+H]⁺.

### Intermediate

### 4-((3-(2-Fluoroethoxy)phenyl)(phenyl)methyl)piperidine

The title compound was prepared in analogy to intermediate 1-[(4-fluorophenyl)-phenylmethyl]piperazine (CAS RN 27064-89-7), starting from 1-bromo-3-(2-fluoroethoxy)benzene (CAS RN 132837-02-6) and tert-butyl 4-benzoylpiperidine-1-carboxylate (CAS RN 922504-27-6) to give the title compound as colorless oil. MS (ESI): m/z = 314.1 [M+H]⁺.

### BB104

### (S or R)-4-((4-Fluorophenyl)(phenyl)methyl)piperidine

A solution of 4-[(4-fluorophenyl)-phenyl-methyl]piperidine TFA salt (690 mg, 1.8 mmol) in MeOH (10 mL) was purified by SFC separation to afford (S or R)-4-((4-fluorophenyl)(phenyl)methyl)piperidine (172 mg, 35%) as light yellow solid; MS (ESI): m/z = 270.1 [M+H]⁺; and (R or S)-4-((4-fluorophenyl)(phenyl)methyl)piperidine. (R or S)-4-((4-Fluorophenyl)(phenyl)methyl)piperidine was dissolved in EtOAc (15 mL) and washed with Na₂CO₃ (5 mL, aq., 30%) and water (5 mL). The organic layer was separated, dried over Na₂SO₄, filtered and concentrated under vacuum. The residue was lyophilized to give (R or S)-4-((4-fluorophenyl)(phenyl)methyl)piperidine (227 mg, 46%) as light yellow solid. MS (ESI): m/z = 270.1 [M+H]⁺.

### Intermediates

### a) tert-Butyl 4-[(4-fluorophenyl)-hydroxy-phenyl-methyl]piperidine-1-carboxylate

To a solution of 4-bromofluorobenzene (2 g, 11.4 mmol) in THF (20 mL) cooled to -78 °C, was added n-butyllithium dropwise (5.81 mL, 14.51 mmol) and the reaction mixture was stirred for 30 min. Then, a solution of tert-butyl 4-benzoylpiperidine-1-carboxylate (CAS RN 922504-27-6; 3 g, 10.37 mmol) in THF (15 mL) was added to the mixture which was stirred at -78 °C for 2 hours. The mixture was allowed to warm up to RT, poured into a solution of sat. aqueous NH₄Cl solution (50 mL) and extracted with EtOAc. The combined organic layers were dried over Na₂SO₄, filtered and concentrated in vacuo. The residue was purified peparative HPLC (Gemini NX column) to give the title compound (1.18 g, 29%) as colorless solid. MS: 312.1 [M-C₄H₈-H₂O+H]⁺.

### b) 4-[(4-Fluorophenyl)-phenyl-methylene]piperidine

To a solution of tert-butyl 4-[(4-fluorophenyl)-hydroxy-phenyl-methyl]piperidine-1-carboxylate (0.7 g, 1.82 mmol) in DCM (10 mL) was added trifluoroacetic acid (1.4 mL, 18.16 mmol) and the reaction was stirred at RT for 8 h. The mixture was concentrated in vacuo to give the crude title compound (0.3 g, 62%) as a light yellow solid. MS: 268.0 [M+H]⁺.

### c) 4-[(4-Fluorophenyl)-phenyl-methyl]piperidine trifluoroacetate

A solution of 4-[(4-fluorophenyl)-phenyl-methylene]piperidine (1600 mg, 5.98 mmol) and Pd/C (300 mg, 5.98 mmol) in THF (100 mL) was stirred at RT for 16 h under H₂ atmosphere (760 mm Hg). The suspension was filtered and the filtrate concentrated in vacuo. The residue was purified by preparative HPLC using TFA as additive to obtain the title compound as 2,2,2-trifluoroacetic acid salt (700 mg, 29%) as white solid. MS (ESI): m/z = 270.1 [M+H]⁺.

### BB 105

### (S or R)-4-((4-Fluorophenyl)(p-tolyl)methyl)piperidine

A solution of 4-[(4-fluorophenyl)-(p-tolyl)methyl]piperidine (870 mg, 3.07 mmol) was separated by SFC (Method: Column DAICEL CHIRALPAK AD 250 mm* 30mm, 5*µ*m, Condition 0.1 % NH₃•H₂O IPA, Begin B 35, End B 35, Gradient Time (min) 4.9 min; 110 min, 100 % B Hold Time (min) 0; FlowRate (mL/min) 60) to afford (R or S)-4-[(4-fluorophenyl)(p-tolyl)methyl]piperidine (253 mg, 28%; MS (ESI): m/z = 284.1 [M+H]⁺) and (S or R)-4-[(4-fluorophenyl)(p-tolyl)methyl]piperidine (356 mg, 40% yield; MS (ESI): m/z = 284.1 [M+H]⁺).

### Intermediates

### a) tert-Butyl 4-((4fluorophenyl)(hydroxy)(p-tolyl)methyl)piperidine-1-carboxylate

To a stirred solution at -78 °C of 4-bromotoluene (1.70 g, 9.94 mmol) in THF (40 mL) was added a butyllithium solution (5.57 mL, 13.9 mmol). After stirring for 1 h, tert-butyl 4-(4-fluorobenzoyl)piperidine-1-carboxylate (CAS RN 160296-40-2; 3.05 g, 9.94 mmol) was added to the mixture and stirring was continued at -78 °C for 1 h. Then the reaction was warmed to 25 °C and stirred for another 13 h. The reaction was quenched with NH₄Cl (sat. aq., 50 mL) and extracted with EtOAc (100 mL). The organic layer was separated, dried with over Na₂SO₄, filtered and concentrated under *vacuum* to afford the desired compound as a light yellow oil (2.85 g, 71%). MS (ESI): m/z = 422.1 [M+Na]⁺.

### b) 4-[(4-Fluorophenyl)-(p-tolyl)methylene]piperidine

A solution of tert-butyl 4-[(4-fluorophenyl)-hydroxy-(p-tolyl)methyl]piperidine-1-carboxylate (2.83 g, 7.08 mmol) and trifluoroacetic acid (11 mL, 142 mmol) in DCM (20 mL) was stirred at 25 °C for 3 h. The reaction was concentrated under *vacuum.* The residue was purification by prep-HPLC (TFA as additive) to afford the title compound as light-yellow semisolid (1.36 mg, 67%). MS (ESI): m/z = 282.1 [M+H]⁺.

### c) 4-[(4-Fluorophenyl)-(p-tolyl)methyl]piperidine trifluoroacetate

A solution of 4-[(4-fluorophenyl)-(p-tolyl)methylene]piperidine (1.36 g, 4.83 mmol) and Pd/C (300 mg) in DMF (50 mL) was stirred at 25 °C for 16 h under H₂ (2280 mm Hg). The reaction solution was filtered through Celite and concentrated under *vacuum.* The residue was purified by prep-HPLC (TFA as additive) to afford the desired compound as white semisolid (TFA salt, 870 mg, 45%). MS (ESI): m/z = 284.1 [M+H]⁺.

### BB106

### (R or S)-4-((4-(2-Fluoroethoxy)phenyl)(phenyl)methyl)piperidine

A solution of 4-[[4-(2-fluoroethoxy)phenyl]-phenyl-methyl]piperidine (800 mg, 2.55 mmol) in MeOH (5 mL) was purified and the enantiomers separated by SFC to afford (S or R)-4-[[4-(2-fluoroethoxy)phenyl]-phenyl-methyl]piperidine (279 mg, 0.89 mmol, 35%) as white solid; MS (ESI): m/z = 314.2 [M+H]⁺; and (R or S)-4-[[4-(2-fluoroethoxy)phenyl]-phenyl-methyl]piperidine (373 mg, 47%) as white solid. MS (ESI): m/z = 314.1 [M+H]⁺.

### Intermediate

### 4-((4-(2-Fluoroethoxy)phenyl)(phenyl)methyl)piperidine

The title compound was prepared in analogy to intermediate 1-[(4-fluorophenyl)-phenylmethyl]piperazine (CAS RN 27064-89-7), starting from 1-bromo-4-(2-fluoroethoxy)benzene (CAS RN 332-47-8) and tert-butyl 4-benzoylpiperidine-1-carboxylate (CAS RN 922504-27-6) to give the title compound as colorless oil. MS (ESI): m/z = 314.1 [M+H]⁺.

### BB107

### (S or R)-4-[(4-Fluorophenyl)-(4-methoxyphenyl)methyl]piperidine

A solution of 4-[(4-fluorophenyl)-(4-methoxyphenyl)methyl]piperidine (930 mg, 3.11 mmol) in MeOH (5 mL) was purified by SFC separation to afford (S or R)-4-[(4-fluorophenyl)-(4-methoxyphenyl)methyl]piperidine (277 mg, 0.93 mmol, 27%) as light yellow semisolid; MS (ESI): m/z = 300.1 [M+H]⁺; and (R or S)-4-[(4-fluorophenyl)-(4-methoxyphenyl)methyl]piperidine (291 mg, 30%) as light yellow semisolid; MS (ESI): m/z = 300.1 [M+H]⁺.

### Intermediate

### 4-[(4-Fluorophenyl)-(4-methoxyphenyl)methyl]piperidine

The title compound was prepared in analogy to intermediate 1-[(4-fluorophenyl)-phenylmethyl]piperazine (CAS RN 27064-89-7), starting from 1-bromo-4-methoxy-benzene (CAS RN 104-92-7) and tert-butyl 4-benzoylpiperidine-1-carboxylate (CAS RN 922504-27-6) to give the title compound as colorless semisolid. MS (ESI): m/z = 300.1 [M+H]⁺.

### BB108

### (R or S)-4-((4-Fluorophenyl)(p-tolyl)methyl)piperidine

A solution of 4-[(4-fluorophenyl)-(p-tolyl)methyl]piperidine (870 mg, 3.07 mmol, BB105, intermediate c) was separated by SFC (Method: Column DAICEL CHIRALPAK AD 250 mm* 30mm, 5µm, Condition 0.1 % NH₃•H₂O IPA, Begin B 35, End B 35, Gradient Time (min) 4.9 min; 110 min, 100 % B Hold Time (min) 0; FlowRate (mL/min) 60) to afford (R or S)-4-[(4-fluorophenyl)(p-tolyl)methyl]piperidine (253 mg, 28%).; MS (ESI): m/z = 284.1 [M+H]⁺; and (S or R)-4-[(4-fluorophenyl)(p-tolyl)methyl]piperidine (356 mg, 40%). MS (ESI): m/z = 284.1 [M+H]⁺.

### BB109

### (S or R)-4-((3,4-Dimethoxyphenyl)(4fluorophenyl)methyl)piperidine

4-((3,4-Dimethoxyphenyl)(4-fluorophenyl)methyl)piperidine (900 mg) was separated by SFC (Method: Column DAICEL CHIRALPAK AD (250 mm *50 mm,10 µm), Condition 0.1% NH₃•H₂O MeOH, Begin B 25, End B 25 Gradient Time (min) 5.5 min:900 min, 100% B Hold Time (min) 0, FlowRate (mL/min) 50 g/min) to give (S or R)-4-((3,4-dimethoxyphenyl)(4-fluorophenyl)methyl)piperidine (278 mg, 29%d). MS (ESI): m/z = 330.3 [M+H]⁺ and (R or S)-4-((3,4-dimethoxyphenyl)(4-fluorophenyl)methyl)piperidine (426 mg, 44%; MS (ESI): m/z = 330.1 [M+H]⁺) as a off-white solids.

### Intermediates

### a) tert-Butyl 4-((3,4-dimethoxyphenyl)(4-fluorophenyl)(hydroxy)methyl)piperidine-1-carboxylate

To a solution of 4-bromoveratrole (CAS RN 2859-78-1; 1.27 g, 5.86 mmol) in THF (30 mL) was added dropwise butyllithium (3.28 mL, 8.2 mmol) with stirring at -78 °C. Stirring was continued at -78 °C for 1 h. Then tert-butyl 4-(4-fluorobenzoyl)piperidine-1-carboxylate (CAS RN 160296-40-2; 1.80 g, 5.86 mmol) was added and the mixture was stirred at -78 °C for 5 h. The mixture was poured into brine (50 mL) and extracted twice with EtOAc (30 mL each). The combined organic layers were concentrated *in vacuo* to give the desired compound as a colorless oil (2.4 g, 91%). MS (ESI): m/z = 468.4 [M+Na]⁺.

### b) 4-((3,4-Dimethoxyphenyl)(4-fluorophenyl)methylene)piperidine trifluoroacetate

A mixture of *tert*-butyl 4-((3,4-dimethoxyphenyl)(4-fluorophenyl)(hydroxy)methyl)piperidine-1-carboxylate (2.4 g, 5.4 mmol) and trifluoroacetic acid (4.15 mL, 53.87 mmol) in DCM (20 mL)was stirred at 25 °C for 4 h. The reaction mixture was concentrated *in vacuo* to give 4-((3,4-dimethoxyphenyl)(4-fluorophenyl)methylene)piperidine (TFA salt, 1.7 g, 96%) as brown oil. MS (ESI): m/z = 328.3 [M+H]⁺.

### c) 4-((3,4-Dimethoxyphenyl)(4-fluorophenyl)methyl)piperidine

A mixture of 4-((3,4-dimethoxyphenyl)(4-fluorophenyl)methylene)piperidine (1.7 g, 5.2 mmol, BB109, intermediate b) and Pd/C (276 mg, 0.260 mmol) in THF (10 mL) was stirred at 25 °C for 16 h under H₂ (760 mm Hg). The mixture was filtered and concentrated *in vacuo* to give a residue, which was purified by preparative HPLC (TFA conditions) to give 4-((3,4-dimethoxyphenyl)(4-fluorophenyl)methyl)piperidine (900 mg, 52%) as white solid. MS (ESI): m/z = 330.3 [M+H]⁺.

### BB110

### (R or S)-4-((3,4-Dimethoxyphenyl)(phenyl)methyl)piperidine

A solution of 4-[(3,4-dimethoxyphenyl)-phenyl-methyl]piperidine (900 mg, 2.89 mmol) in MeOH (5 mL) was purified by SFC separation to afford (S or R)-(3,4-dimethoxyphenyl)-phenyl-methyl]piperidine (326 mg, 36%) as white solid; MS (ESI): m/z = 312.3 [M+H]⁺; and (R or S)-(3,4-dimethoxyphenyl)-phenyl-methyl]piperidine (222 mg, 24%) as white solid; MS (ESI): m/z = 312.3 [M+H]⁺.

### Intermediate

### 4-[(3,4-Dimethoxyphenyl)-phenyl-methyl]piperidine

The title compound was prepared in analogy to intermediate 1-[(4-fluorophenyl)-phenylmethyl]piperazine (CAS RN 27064-89-7), starting from 4-bromo-1,2-dimethoxy-benzene (CAS RN 2859-78-1) and tert-butyl 4-benzoylpiperidine-1-carboxylate (CAS RN 922504-27-6) to give the title compound as a white solid. MS (ESI): m/z = 312.3 [M+H]⁺.

### BB111

### (R or S)-4-((4-Fluorophenyl)(phenyl)methyl)piperidine

A solution of 4-[(4-fluorophenyl)-phenyl-methyl]piperidine TFA salt (690 mg, 1.8 mmol) in MeOH (10 mL) was purified by SFC separation to afford (S or R)-4-((4-fluorophenyl)(phenyl)methyl)piperidine (172 mg, 35%) as a light yellow solid. MS (ESI): m/z = 270.1 [M+H]⁺; and (R or S)-4-((4-fluorophenyl)(phenyl)methyl)piperidine. (R or S)-4-((4-Fluorophenyl)(phenyl)methyl)piperidine was dissolved in EtOAc (15 mL) and washed with aqueous Na₂CO₃ solution (5 mL, 30%) and water (5 mL). The organic layer was separated, dried over Na₂SO₄, filtered and concentrated under vacuum. The residue was lyophilized to give (R or S)-4-((4-fluorophenyl)(phenyl)methyl)piperidine (227 mg, 0.84 mmol, 46%) as light yellow solid; MS (ESI): m/z = 270.1 [M+H]⁺.

### BB112

### (S or R)-4-(Phenyl(m-tolyl)methyl)piperidine

A solution of 4-(phenyl(m-tolyl)methyl)piperidine (730 mg, 2.75 mmol) in MeOH (5 mL) was purified by SFC separation to afford (S or R)-4-(phenyl(m-tolyl)methyl)piperidine (221 mg, 0.83 mmol, 30%) as yellow semisolid. MS (ESI): m/z = 266.2 [M+H]⁺; and (R or S)-4-(phenyl(m-tolyl)methyl)piperidine (228 mg, 31%) as yellow semisolid. MS (ESI): m/z = 266.2 [M+H]⁺.

### Intermediate

### 4-(Phenyl(m-tolyl)methyl)piperidine

The title compound was prepared in analogy to intermediate 1-[(4-fluorophenyl)-phenylmethyl]piperazine (CAS RN 27064-89-7), starting from 1-bromo-3-methyl-benzene (CAS RN 591-17-3) and tert-butyl 4-benzoylpiperidine-1-carboxylate (CAS RN 922504-27-6) to give the title compound as a light yellow solid. MS (ESI): m/z = 266.1 [M+H]⁺.

### BB113

### (S or R)-4-((4-(2-Fluoroethoxy)phenyl)(phenyl)methyl)piperidine

A solution of 4-[[4-(2-fluoroethoxy)phenyl]-phenyl-methyl]piperidine (BB106, intermediate; 800 mg, 2.55 mmol) in MeOH (5 mL) was purified and the enantiomers separated by SFC separation to afford (S or R)-4-[[4-(2-fluoroethoxy)phenyl]-phenylmethyl]piperidine (279 mg, 35%) as white solid, MS (ESI): m/z = 314.2 [M+H]⁺; and (R or S)-4-[(S)-[4-(2-fluoroethoxy)phenyl]-phenyl-methyl]piperidine (373 mg, 47%) as white solid; MS (ESI): m/z = 314.1 [M+H]⁺.

### BB114

### (R or S)-4-(Phenyl(m-tolyl)methyl)piperidine

A solution of 4-(phenyl(m-tolyl)methyl)piperidine (BB112, intermediate, 730 mg, 2.75 mmol) in MeOH (5 mL) was purified and the enantiomers separated using SFC to afford (S or R)-4-(phenyl(m-tolyl)methyl)piperidine (221 mg, 30%) as yellow semisolid; MS (ESI): m/z = 266.2 [M+H]⁺; and (R or S)-4-(phenyl(m-tolyl)methyl)piperidine (228 mg, 31%) as yellow semisolid. MS (ESI): m/z = 266.2 [M+H]⁺.

### BB115

### (S or R)-4-(Phenyl(p-tolyl)methyl)piperidine

A solution of 4-(phenyl(p-tolyl)methyl)piperidine (720 mg, 2.71 mmol) in MeOH (5 mL) was purified and the enatiomers separated using SFC to afford ((S or R)-4-(phenyl(p-tolyl)methyl)piperidine (277 mg, 35%) as off-white solid; MS (ESI): m/z = 266.2 [M+H]⁺; and (R or S)-4-(phenyl(p-tolyl)methyl)piperidine (313 mg, 43%) as off-white solid; MS (ESI): m/z = 266.2 [M+H]⁺.

### Intermediate

### 4-(Phenyl(p-tolyl)methyl)piperidine

The title compound was prepared in analogy to intermediate 1-[(4-fluorophenyl)-phenylmethyl]piperazine (CAS RN 27064-89-7), starting from 1-bromo-4-methyl-benzene (CAS RN 106-38-7) and tert-butyl 4-benzoylpiperidine-1-carboxylate (CAS RN 922504-27-6) to give the title compound as white solid, MS (ESI): m/z = 266.2 [M+H]⁺.

### BB116

### (R or S)-4-(Phenyl(p-tolyl)methyl)piperidine

A solution of 4-(phenyl(p-tolyl)methyl)piperidine (BB117, intermediate, 720 mg, 2.71 mmol) in MeOH (5 mL) was purified and the enatiomers separated using SFC to afford ((S or R)-4-(phenyl(p-tolyl)methyl)piperidine (277 mg, 1.04 mmol, 35%) as off-white solid; MS (ESI): m/z = 266.2 [M+H]⁺; and (R or S)-4-(phenyl(p-tolyl)methyl)piperidine (313 mg, 1.18 mmol, 43%) as off-white solid; MS (ESI): m/z = 266.2 [M+H]⁺.

### BB117

### 3-Benzhydrylazetidine

A solution of 3-(diphenylmethylene)azetidine (800 mg, 3.62 mmol) and Pd/C (1923 mg) in THF (20 mL) was stirred at 25 °C under H₂ atmosphere (760 mm Hg) for 5 h. The solution was filtered and concentrated *in vacuo* to give a residue, which was purified by preparative HPLC (TFA condition) to give 3-benzhydrylazetidine (315 mg, 37%) as white solid. MS (ESI): m/z = 224.1 [M+H]⁺.

### Intermediates

### a) tert-Butyl 3-[methoxy(methyl)carbamoyl]azetidine-1-carboxylate

A mixture of 1-boc-azetidine-3-carboxylic acid (CAS RN 142253-55-2; 50 g, 248 mmol), triethylamine (69.3 mL, 497 mmol), 1-hydroxybenzotriazole (33.5 g, 248 mmol) and EDCl (47.6 g, 248 mmol) and O,N-dimethylhydroxylamine hydrochloride (24.24 g, 248.5 mmol) in DMF (1000 mL) was stirred at 25 °C for 16 h. The mixture was concentrated *in vacuo* to give a residue, which was neutralized by HCl (1M) to pH = 7 and extracted three times with EtOAc (200 mL each). The combined organic layers were washed twice with 200 mL aqueous NaHCO₃ solution, dried over Na₂SO₄ and concentrated *in vacuo* to give tert-butyl 3-[methoxy(methyl)carbamoyl]azetidine-1-carboxylate (55 g, 72%) as colorless oil. MS (ESI): m/z = 189.1 [M-C₄H₈+H]⁺.

### b) tert-Butyl 3-benzoylazetidine-1-carboxylate

To a solution of tert-butyl 3-[methoxy(methyl)carbamoyl]azetidine-1-carboxylate (55 g, 225 mmol) in THF (600 mL) was added phenylmagnesium bromide (82 mL, 248 mmol) with stirring at 0 °C and then the solution was stirred at 0 °C for 3 h. The solution was poured into sat.aq. NH₄Cl solution (300 mL) and extracted twice with EtOAc (150 mL each). The combined organic layers were dried over Na₂SO₄, filtered and concentrated *in vacuo* to give a residue, which was purified by column chromatography (petroleum ether : EtOAc 10 : 1) to give the desired compound as light yellow solid (28 g, 46%). MS (ESI): m/z = 206.1 [M- C₄H₈+H]⁺.

### c) tert-Butyl 3-(hydroxydiphenylmethyl)azetidine-1-carboxylate

A solution of phenylmagnesium bromide (3.06 mL, 9.18 mmol) and *tert-butyl 3-*benzoylazetidine-1-carboxylate (2.0 g, 7.65 mmol) in THF (20 mL) was stirred at 0 °C for 2 h. The solution was quenched by addition of sat. aq. NH₄Cl solution (50 mL) and extracted twice with EtOAc (20 mL each). The combined organic layers were concentrated *in vacuo* to give a residue, which was purified by flash column chromatography (PE : EtOAc 5 : 1) to afford the desired compound as white solid (1.03 g, 39%). MS (ESI): m/z = 362.2 [M+Na]⁺.

### d) 3-(Diphenylmethylene)azetidine trifluoroacetate

A solution of tert-butyl 3-(hydroxydiphenylmethyl)azetidine-1-carboxylate (1.00 g, 2.95 mmol) and TFA (2.27 mL, 29.5 mmol) in DCM (15 mL) was stirred at 25 °C for 4 h. The solution was concentrated *in vacuo* to provide the title compound as brown oil (TFA salt, 650 mg, 99%). MS (ESI): m/z = 222.1 [M+H]⁺.

### BB118

### (S or R)-4-((3,4-Dimethoxyphenyl)(phenyl)methyl)piperidine

A solution of 4-[(3,4-dimethoxyphenyl)-phenyl-methyl]piperidine (BB110, intermediate; 900 mg, 2.89 mmol) in MeOH (5 mL) was purified and the enatiomers separated using SFC to afford (S or R)-(3,4-dimethoxyphenyl)-phenyl-methyl]piperidine (326 mg, 1.05 mmol, 36%) as white solid; MS (ESI): m/z = 312.3 [M+H]⁺; and (R or S)-(3,4-dimethoxyphenyl)-phenyl-methyl]piperidine (222 mg, 0.71 mmol, 24%) as white solid. MS (ESI): m/z = 312.3 [M+H]⁺.

### BB119

### (R or S)-4-((3,4-Dimethoxyphenyl)(4fluorophenyl)methyl)piperidine

4-((3,4-Dimethoxyphenyl)(4-fluorophenyl)methyl)piperidine (BB109, intermediate c; 900 mg) was separated by SFC (Method: Column DAICEL CHIRALPAK AD (250 mm *50 mm,10 µm), Condition 0.1% NH₃•H₂O MeOH, Begin B 25, End B 25 Gradient Time (min) 5.5 min:900 min, 100% B Hold Time (min) 0, FlowRate (mL/min) 50 g/min) to give (S or R)-4-((3,4-dimethoxyphenyl)(4-fluorophenyl)methyl)piperidine (278 mg, 29%; MS (ESI): m/z = 330.3 [M+H]⁺) and (R or S)-4-((3,4-dimethoxyphenyl)(4-fluorophenyl)methyl)piperidine (426 mg, 44%; MS (ESI): m/z = 330.1 [M+H]⁺) as off-white solids.

### BB120

### (R or S)-4-[(4-Fluorophenyl)-(4-methoxyphenyl)methyl]piperidine

A solution of 4-[(4-fluorophenyl)-(4-methoxyphenyl)methyl]piperidine (BB107, intermediate; 930 mg, 3.11 mmol) in MeOH (5 mL) was purified and the enantiomers separated using SFC to afford (S or R)-4-[(4-fluorophenyl)-(4-methoxyphenyl)methyl]piperidine (277 mg, 27%) as light yellow semisolid; MS (ESI): m/z = 300.1 [M+H]⁺; and (R or S)-4-[(4-fluorophenyl)-(4-methoxyphenyl)methyl]piperidine (291 mg, 30%) as light yellow semisolid; MS (ESI): m/z = 300.1 [M+H]⁺.

### BB121

### (R or S)-4-((4-(2-Fluoroethoxy)phenyl)(4fluorophenyl)methyl)piperidine

A solution of 4-[[4-(2-fluoroethoxy)phenyl]-(4-fluorophenyl)methyl]piperidine (700 mg, 2.11 mmol) in MeOH (5 mL) was purified and the enantiomers separated using SFC to afford (R or S)-4-[[4-(2-fluoroethoxy)phenyl]-(4-fluorophenyl)methyl]piperidine (203 mg, 29%) as colorless oil; MS (ESI): m/z = 332.2 [M+H]⁺; and (S or R)-4-[[4-(2-fluoroethoxy)phenyl]-(4-fluorophenyl)methyl]piperidine (160 mg, 23%) as colorless oil; MS (ESI): m/z = 332.2 [M+H]⁺.

### Intermediate

### 4-[[4-(2-Fluoroethoxy)phenyl]-(4-fluorophenyl)methyl]piperidine

The title compound was prepared in analogy to intermediate 1-[(4-fluorophenyl)-phenylmethyl]piperazine (CAS RN 27064-89-7), starting from 1-bromo-4-(2-fluoroethoxy)benzene (CAS RN 332-47-8) and tert-butyl 4-(4-fluorobenzoyl)piperidine-1-carboxylate (CAS RN 160296-40-2) to give the title compound as colorless oil. MS (ESI): m/z = 332.1 [M+H]⁺.

### BB122

### (R or S)-4-((3-(2-Fluoroethoxy)phenyl)(phenyl)methyl)piperidine

A solution of 4-[[3-(2-fluoroethoxy)phenyl]-phenyl-methyl]piperidine (BB103, intermediate; 800 mg, 2.55 mmol) in MeOH (5 mL) was purified and the enatiomers separated using SFC to afford (S or R)-4-((3-(2-fluoroethoxy)phenyl)(phenyl)methyl)piperidine (214 mg, 27%) as white solid; MS (ESI): m/z = 314.1 [M+H]⁺; and (R or S)-4-((3-(2-fluoroethoxy)phenyl)(phenyl)methyl)piperidine (305 mg, 38%) as white solid. MS (ESI): m/z = 314.1 [M+H]⁺.

### BB123

### (S or R)-4-((4-(2-Fluoroethoxy)phenyl)(4fluorophenyl)methyl)piperidine

A solution of 4-[[4-(2-fluoroethoxy)phenyl]-(4-fluorophenyl)methyl]piperidine (BB121, intermediate; 700 mg, 2.11 mmol) in MeOH (5 mL) was purified and the enatiomers separated by SFC to afford (R or S)-4-[[4-(2-fluoroethoxy)phenyl]-(4-fluorophenyl)methyl]piperidine (203 mg, 29%) as colorless oil; MS (ESI): m/z = 332.2 [M+H]⁺; and (S or R)-4-[[4-(2-fluoroethoxy)phenyl]-(4-fluorophenyl)methyl]piperidine (160 mg, 23%) as colorless oil. MS (ESI): m/z = 332.2 [M+H]⁺.

### BB124

### tert-Butyl (2-(3-(phenyl(piperidin-4-yl)methyl)phenoxy)ethyl)carbamate

A mixture of benzyl 4-((3-(2-((tert-butoxycarbonyl)amino)ethoxy)phenyl)(phenyl)methylene)piperidine-1-carboxylate (60 mg, 111 µmol), methanol (3 mL) and AcOH (60 µL) was charged with Pt-Pd/C (60 mg, 2.5%+2.5%, 63% H₂O) and put under a hydrogen gas atmosphere (50 bar). The mixture was shaken at 50 °C for 2 h, diluted with EtOAc and filtered. The filtrate was washed with KHCO₃ and the organic layer was dried over Na₂SO₄. The solvent was removed under reduced pressure to give crude title compound (33 mg, 68%) as white foam which was used in the next reaction step without further purification. MS (ESI): m/z = 411.3 [M+H]⁺.

### Intermediates

### a) Benzyl (Z)-4-(phenyl(2-tosylhydrazineylidene)methyl)piperidine-1-carboxylate

A solution of 4-methylbenzenesulfonhydrazide (1.54 g, 8.29 mmol, CAS RN 1576-35-8;) and tert-butyl 4-benzoylpiperidine-1-carboxylate (2 g, 6.91 mmol, CAS RN 922504-27-6) in 1,4-dioxane (150 mL) was stirred at 100 °C for 16 h. EtOAc and water were added. The layers were separated and the organic layer was dried over Na₂SO₄. The solvent was removed under reduced pressure and the residue purified by flash chromatography (25 g silica gel; gradient of EtOAc (0-30%) in n-heptane) to give the title compound (3.55 g, 82%) as light brown oil. MS (ESI): m/z = 492.2 [M+H]⁺.

### b) 4-((3-(2-((tert-Butoxycarbonyl)amino)ethoxy)phenyl)(phenyl)methylene)piperidine-1-carboxylate

A mixture of benzyl (Z)-4-(phenyl(2-tosylhydrazineylidene)methyl)piperidine-1-carboxylate (1 g mg, 2.03 mmol), bis(triphenylphosphine)palladium(II) chloride (143 mg, 203 µmol), lithium tert-butoxide (246 mg, 3.05 mmol) and N-boc-2-(3-bromophenoxy)ethylamine (CAS RN 1098107-26-6; 772 mg, 2.44 µmol) in 1,4-dioxane (65 mL) was stirred at 80 °C for 12 h. EtOAc and water were added. The layers were separated and the organic layer was dried over Na₂SO₄. The solvent was removed under reduced pressure and the residue purified by flash chromatography (80 g silica gel; gradient of EtOAc in n-heptane 0-30%) to give the title compound (280 mg, 22%) as light yellow foam. MS (ESI): m/z = 443.2 [M-Boc+H]⁺.

### BB125

### 5-Chloro-2-(piperidin-4-yl)isoindoline hydrochloride

The compound was obtained in analogy to BB52 from tert-butyl 4-(5-chloroisoindolin-2-yl)piperidine-1-carboxylate to get the desired compound as a light green solid. MS (ESI): m/z = 237.2 [M+H]⁺.

### Intermediate

### tert-Butyl 4-(5-chloroisoindolin-2-yl)piperidine-1-carboxylate

The compound was obtained in analogy to BB148, intermediate, from 5-chloroisoindoline hydrochloride (CAS RN 912999-79-2) to get the desired compound as a light brown solid. MS (ESI): m/z = 337.3 [M+H]⁺.

### BB126

### (4aS,8aS)-Hexahydro-2H-pyrido[4,3-b][1,4]oxazin-3(4H)-one hydrochloride

A white suspension of tert-butyl (4aS,8aS)-3-oxohexahydro-2H-pyrido[4,3-b][1,4]oxazine-6(5H)-carboxylate (330 mg, 1.29 mmol) in 2M hydrogen chloride in ether (6.44 ml, 12.9 mmol) was stirred at RT for 22 hours. The colorless suspension was filtered and washed with diethylether to get the desired product as a colorless solid (0.172 g; 69.3%). MS (ESI): m/z = 157.0990 [M+H]⁺.

### Intermediates

### a) tert-Butyl (4aS,8aS)-3-oxohexahydro-2H-pyrido[4,3-b][1,4]oxazine-6(5H)-carboxylate

To an ice-cold solution of tert-butyl (3S,4S)-3-(2-chloroacetamido)-4-hydroxypiperidine-1-carboxylate (436 mg, 1.49 mmol) in DCM (7 ml) was added dropwise a solution of potassium tert-butoxide (668 mg, 5.96 mmol) in 2-Propanol (18 ml). The ice-bath was removed and the mixture was stirred at RT for 24 hours while getting a wite suspension. The mixture was evaporated. The residue was taken up in ethyl acetate and water and the layers were separated. The aqueous layer was extracted twice with ethyl acetate. The organic layers were dried over MgSO4, filtered, treated with silica gel and evaporated. The compound was purified by silica gel chromatography on a 40 g column using an MPLC system eluting with a gradient of DCM : MeOH (100 : 0 to 90 : 10) to get the desired compound as a colorless foam (330 mg; 86.5 % yield). MS (ESI): m/z = 201.1 [M-C4H8+H]⁺.

### b) tert-Butyl (3S,4S)-3-(2-chloroacetamido)-4-hydroxypiperidine-1-carboxylate

To an ice-cold suspension of tert-butyl (3S,4S)-3-amino-4-hydroxypiperidine-1-carboxylate (500 mg, 2.31 mmol, CAS RN 1312812-78-4) and sodium acetate trihydrate (629 mg, 4.62 mmol) in acetone (4 ml) and water (300 µl) was added dropwise a solution of 2-chloroacetyl chloride (261 mg, 184 µl, 2.31 mmol) in acetone (500 µl). The mixture was stirred at RT for 3 hours before silica gel was added. The suspension was evaporated. The compound was purified by silica gel chromatography on a 12 g column using an MPLC (ISCO) system eluting with a gradient of n-heptane : ethyl acetate (100 : 0 to 0 : 100) to get the desired compound as a colorless foam (436 mg; 64.4%). MS (ESI): m/z = 291.3 [M-H]⁻.

### BB127

### 4-[1-[4-(Trifluoromethyl)phenyl]ethyl]piperidine formate

A solution of tert-butyl 4-[1-[4-(trifluoromethyl)phenyl]ethyl]piperidine-1-carboxylate (1500.0 mg, 4.2 mmol) in HCl in dioxane (50.0 mL, 200 mmol) was stirred at 20 °C for 2 h. The mixture was concentrated and the residue was purified by prep-HPLC (FA) and lyophilized to give the desired compound as a light yellow oil (838.4 mg, 77.1%). MS (ESI): m/z = 258.1 [M+H]⁺.

### Intermediates

### a) tert-Butyl 4-[1-[4-(trifluoromethyl)phenyl]vinyl]piperidine-1-carboxylate

To a solution of methyltriphenylphosphonium bromide (499.8 mg, 1.4 mmol, CAS RN 1779-49-3) in THF (10 mL) was added potassium tert-butoxide (235.5 mg, 2.1 mmol) portionwise at 0 °C and the mixture was stirred at 0°C for 30 min. tert-Butyl 4-[4-(trifluoromethyl)benzoyl]piperidine-1-carboxylate (500.0 mg, 1.4 mmol, CAS RN 725229-27-6) in THF (5 mL) was added dropwise and the mixture was warmed to 20 °C and stirred at RT for 12 h. The mixture was poured into saturated aq. NH₄Cl solution (50 mL) and extracted twice with EtOAc (20 mL each). The layers were separated, the organic phase washed with brine, dried over Na₂SO₄ and filtered. The filtrate was purified by silica gel column (PE : EtOAc = 20 :1) to give the desired compound as light yellow oil (200 mg, 40.2%). MS: MS (ESI): m/z = 300.0 [M-C₄H₈+H]⁺.

### b) tert-Butyl 4-[1-[4-(trifluoromethyl)phenyl]ethyl]piperidine-1-carboxylate

To a solution of tert-butyl 4-[1-[4-(trifluoromethyl)phenyl]vinyl]piperidine-1-carboxylate (2.0 g, 5.63 mmol) in EtOAc (100 mL) was added Pd/C (1.0 g, 5.63 mmol) and the mixture was stirred at 20 °C under an hydrogen atmosphere for 12 h. The mixture was filtered and the filtrate was concentrated to give the crude product (1.8 g, 89.5%) as a colorless oil which was used in the next step without further purification. MS (ESI): m/z = 302.2 [M-C₄H₈+H]⁺.

### BB128

### 3-(Piperidin-4-yl)-5-(trifluoromethyl)pyridine dihydrochloride

In a 25 mL tube tert-butyl 4-(5-(trifluoromethyl)pyridin-3-yl)piperidine-1-carboxylate (110 mg, 333 µmol) was dissolved in DCM (1 mL) and HCl in ether 2M (2 mL, 4 mmol) was added. The reaction was stirred 6 h at RT. The solvent was removed *in vacuo* to obtain the crude product as a white foam, 100 mg (99%). Used without further purification. MS (ESI): m/z = 231.1 [M+H]⁺.

### Intermediates:

### a) tert-Butyl 4-(5-(trifluoromethyl)pyridin-3-yl)piperidine-1-carboxylate

In a dried 25 mL three-necked flask, zinc powder (172 mg, 2.63 mmol) was combined with 1 mL DMA (over molecular sieve) to give a grey suspension. The mixture was stirred at RT while a 7:5 v/v mixture of chlorotrimethylsilane (32 ul) and 1,2-dibromoethane (22 ul) as solution in DMA (1 mL) was added at a rate to maintain the temperature below 65°C (slightly exothermic). The resulting slurry was stirred for 20 minutes. A solution of tert-butyl 4-iodopiperidine-1-carboxylate (688 mg, 2.21 mmol) in 2 mL DMA was slowly added to the mixture. The resulting reaction mixture was then stirred for 30 minutes at RT. The reaction was allowed to stand for 15 min without stirring for decantation.

In a 25 mL three-necked flask, 3-bromo-5-(trifluoromethyl)pyridine (250mg, 1.11 mmol) was combined with 1.5 mL DMA to give a colorless solution, copper (I) iodide (21.1 mg, 111 µmol) and [1,1'-Bis(diphenylphosphino)ferrocene]dichloropalladium(II), complex with dichloromethane (45.2 mg, 55.3 µmol) were added. The reaction mixture was degassed with argon, the freshly prepared Zinc-reagent solution was added, again degassed with argon, and the reaction mixture was stirred over night at 80°C. The reaction mixture was quenched with 10 mL sat. aqueous NH₄Cl solution and extracted twice withEtOAc (40 mL each). The organic layers were washed with brine. The organic layers were combined, dried over MgSO₄ and concentrated *in vacuo.* Purification by SiO₂ flash chromatography using n-heptane /EtOAc 0 to 80% in 35 min, afforded the desired product as light yellow oil (112 mg, 31%). MS (ESI): m/z = 331.2 [M+H]⁺.

### BB129

### 4-(Difluoro(4-(trifluoromethyl)phenyl)methyl)piperidine hydrochloride

tert-Butyl 4-(difluoro(4-(trifluoromethyl)phenyl)methyl)piperidine-1-carboxylate (0.338 g, 891 µmol) was dissolved in DCM (1 mL) and HCl in ether 2M (4.45 mL, 8.91 mmol) was added. The reaction was stirred 16 h at RT. The solvent was removed *in vacuo* to obtain the crude product as a off-white solid, 246 mg (82%). Used without further purification. MS (ESI): m/z = 280.11 [M+H]⁺.

### Intermediates:

### a) tert-Butyl 4-(4-(trifluoromethyl)benzoyl)piperidine-1-carboxylate

To a solution of piperidin-4-yl(4-(trifluoromethyl)phenyl)methanone hydrochloride (0.500 g, 1.7 mmol), triethylamine (345 mg, 475 µl, 3.4 mmol) and DMAP (62.4 mg, 511 µmol) in Acetonitrile (10 mL), was added at 0°C under argon Boc₂O (446 mg, 2.04 mmol). The reaction mixture was stirred at RT for 4 hours. The reaction mixture was poured into 15 mL sat NaHCO₃ and extracted twice with EtOAc (25 mL each). The organic layers were combined, washed with brine, dried over Na₂SO₄ and concentrated *in vacuo.* The crude material was triturated with n-heptane to yield the desired product as an off white solid (354mg, 58%), used directly for next step. MS (ESI): m/z = 302.1 [M-C₄H₈+H]⁺.

### b) tert-Butyl 4-(difluoro(4-(trifluoromethyl)phenyl)methyl)piperidine-1-carboxylate

To a solution of tert-butyl 4-(4-(trifluoromethyl)benzoyl)piperidine-1-carboxylate (0.350 g, 979 µmol) in DCM (3 mL), DAST (5.53 g, 34.3 mmol) was added. The reaction mixture was stirred at 45°C for three days. The reaction mixture was poured into 15 mL H₂O and ice and extracted twice with DCM (20 mL each). The organic layers were combined, washed with brine, dried over Na₂SO₄ and concentrated *in vacuo.* The crude material was purified by flash chromatography (silica gel, 20 g, 0% to 20% EtOAc in n-heptane) to provide the desired compound as a yellow oil (341 mg,92%). MS (ESI): m/z = 379.1 [M+H]⁺.

### BB130

### 3-Methoxy-5-(piperidin-4-yl)pyridine dihydrochloride

In a 25 mL tube tert-butyl 4-(5-methoxypyridin-3-yl)piperidine-1-carboxylate (150 mg, 513 µmol) was dissolved in DCM (1 mL) and HCl in ether 2M (2.57 mL, 5.13 mmol) was added. The reaction was stirred 3 h at RT. The solvent was removed *in vacuo* to obtain the crude product as a light yellow foam, 136 mg (100%). Used without further purification. MS (ESI): m/z = 193.1 [M+H]⁺.

### Intermediate:

### tert-Butyl 4-(5-methoxypyridin-3-yl)piperidine-1-carboxylate

In a dried 25 mL three-necked flask, zinc powder (232 mg, 3.54 mmol) was combined with 1 mL DMA (over molecular sieve) to give a grey suspension. The mixture was stirred at RT while a 7:5 v/v mixture of chlorotrimethylsilane (32 uL) and 1,2-dibromoethane (22 ul) as solution in DMA (1 mL) was added at a rate to maintain the temperature below 65°C (slightly exothermic). The resulting slurry was stirred for 20 minutes. A solution of tert-butyl 4-iodopiperidine-1-carboxylate (927 mg, 2.98 mmol) in 2 mL DMA was slowly added to the mixture. The resulting reaction mixture was then stirred for 30 minutes at RT. The reaction was allowed to stand for 15 min without stirring for decantation.

In a 25 mL three-necked flask, 3-bromo-5-methoxypyridine (280 mg, 1.49 mmol) was combined with 1.5 mL DMA to give a colorless solution, copper (I) iodide (28.4 mg, 149 µmol) and [1,1'-Bis(diphenylphosphino)ferrocene]dichloropalladium(II), complex with dichloromethane (60.8 mg, 74.5 µmol) were added. The reaction mixture was degassed with argon, the freshly prepared Zinc-reagent solution was added, again degassed with argon, and the reaction mixture was stirred for 6 hr at 80°C. The reaction mixture was quenched with 10 mL sat. NH₄Cl and extracted two times with EtOAc (40 mL each). The organic layers were washed with brine. The organic layers were combined, dried over MgSO₄ and concentrated *in vacuo.* Purification by SiO₂ flash chromatography using n-heptane /EtOAc 0 to 100% in 35 min, afforded the desired product as light yellow oil (150 mg, 34.5%). MS (ESI): m/z = 293.2 [M+H]⁺.

### BB131

### 4-((4-Chlorophenyl)difluoromethyl)piperidine hydrochloride

tert-butyl 4-(difluoro(4-(trifluoromethyl)phenyl)methyl)piperidine-1-carboxylate (440 mg, 1.27 mmol) was dissolved in DCM (2 mL) and HCl in ether 2M (6.36 mL, 12.7 mmol) was added. The reaction was stirred for 64 hr at RT. The solvent was removed *in vacuo* to obtain the crude product as a off-white solid, 323 mg (90%). Used without further purification. MS (ESI): m/z = 246.1 [M+H]⁺.

### Intermediate

### tert-Butyl 4-(difluoro(4-(trifluoromethyl)phenyl)methyl)piperidine-1-carboxylate

To a solution of tert-butyl 4-(4-chlorobenzoyl)piperidine-1-carboxylate (0.500 g, 1.54 mmol) in DCM (3 mL), DAST (8.71 g, 54 mmol) was added. The reaction mixture was stirred at 45°C for eight days. The reaction mixture was poured into 15 mL H₂O and ice and extracted twice with DCM (20 mL each). The organic layers were combined, washed with brine, dried over Na₂SO₄ and concentrated *in vacuo.* The crude material was purified by flash chromatography (silica gel, 20 g, 0% to 20% EtOAc in n-heptane) to provide the desired product as yellow oil (440 mg, 82%). MS (ESI): m/z = 345.1 [M+H]⁺.

### BB132

### 2-(Piperidin-4-yl)-5-(trifluoromethoxy)pyridine dihydrochloride

In a 25 mL tube tert-butyl 4-(5-(trifluoromethoxy)pyridin-2-yl)piperidine-1-carboxylate (380mg, 1.1 mmol) was dissolved in DCM (2 mL) and HCl in ether 2M (3.29 mL, 6.58 mmol) was added. The reaction was stirred at RT for 4 h. The solvent was removed *in vacuo* to obtain the crude product as a light yellow foam (349 mg, 99%) which was used in the next step without further purification. MS (ESI): m/z = 246.2 [M+H]⁺.

### Intermediate:

### tert-Butyl 4-(5-(trifluoromethoxy)pyridin-2-yl)piperidine-1-carboxylate

In a dried 25 mL three-necked flask, zinc powder (193 mg, 2.38 mmol) was combined with 1.5 mL DMA (over molecular sieve) to give a grey suspension. The mixture was stirred at RT while a 7:5 v/v mixture of chlorotrimethylsilane (32 ul) and 1,2-dibromoethane (22 ul) as solution in DMA (1 mL) was added at a rate to maintain the temperature below 65°C (slightly exothermic). The resulting slurry was stirred for 20 minutes. A solution of tert-butyl 4-iodopiperidine-1-carboxylate (771 mg, 2.48 mmol) in 2 mL DMA was slowly added to the mixture. The resulting reaction mixture was then stirred for 30 minutes at RT. The reaction was allowed to stand for 15 min without stirring for decantation.

In a 25 mL three-necked flask, 2-bromo-5-(trifluoromethoxy)pyridine (300 mg, 172 µl, 1.24 mmol) was combined with 1.5 mL DMA to give a colorless solution, copper (I) iodide (23.6 mg, 124 µmol) and [1,1'-Bis(diphenylphosphino)ferrocene]dichloropalladium(II), complex with dichloromethane (50.6 mg, 62 µmol) were added. The reaction mixture was degassed with argon, the freshly prepared Zinc-reagent solution was added, again degassed with argon, and the reaction mixture was stirred for 6 hr at 80°C. The reaction mixture was quenched with 10 mL sat. NH₄Cl and extracted twice withEtOAc (40 mL each). The organic layers were washed with brine. The organic layers were combined, dried over MgSO₄ and concentrated *in vacuo.* Purification by SiO₂ flash chromatography using n-heptane /EtOAc 0 to 50% in 35 min, afforded the desired product as light yellow oil (387 mg, 90.1%). MS (ESI): m/z = 347.1 [M+H]⁺.

### BB133

### 4-((2-Chloro-4fluorophenyl)difluoromethyl)piperidine hydrochloride

In a 25 mL tube tert-butyl 4-((2-chloro-4-fluorophenyl)difluoromethyl)piperidine-1-carboxylate (0.363g, 998 µmol) was dissolved in DCM (2 mL) and HCl in ether 2M (4.99 mL, 9.98 mmol) was added. The reaction was stirred at RT over night. The solvent was removed *in vacuo* to obtain the crude product as a light brown solid, 296 mg (99%). Used without further purification. MS (ESI): m/z = 264.08 [M+H]⁺.

### Intermediate

### tert-Butyl 4-((2-chloro-4-fluorophenyl)difluoromethyl)piperidine-1-carboxylate

To a solution of tert-butyl 4-(2-chloro-4-fluorobenzoyl)piperidine-1-carboxylate (0.48 g, 1.4 mmol) in DCM (4 mL), DAST (7.92 g, 49.2 mmol) was added. The reaction mixture was stirred at 45°C for ten days. The reaction mixture was poured into 15 mL H₂O + ice and extracted with DCM (2 x 20 mL). The organic layers were combined, washed with brine, dried over Na₂SO₄ and concentrated *in vacuo.* The crude material was purified by flash chromatography (silica gel, 20 g, 0% to 20% EtOAc in n-heptane): 363 mg (71%), light brown oil, desired product. MS (ESI): m/z = 363.1 [M+H]⁺.

### BB134

### 3-Ethyl-5-(piperidin-4-yl)pyridine dihydrochloride

In a 25 mL tube tert-butyl 4-(5-ethylpyridin-3-yl)piperidine-1-carboxylate (295mg, 1.02 mmol) was dissolved in DCM (3 mL) and HCl in ether 2M (3.05 mL, 6.09 mmol) was added. The reaction was stirred 4 h at RT. The solvent was removed *in vacuo* to obtain the crude product as a yellow solid, 266 mg (99%). Used without further purification. MS (ESI): m/z = 191.1 [M+H]⁺.

### Intermediate

### tert-Butyl 4-(5-ethylpyridin-3-yl)piperidine-1-carboxylate

In a dried 25 mL three-necked flask, zinc powder (234 mg, 3.58 mmol) was combined with 1.5 mL DMA (over molecular sieve) to give a grey suspension. The mixture was stirred at RT while a 7:5 v/v mixture of chlorotrimethylsilane (32 ul) and 1,2-dibromoethane (22 ul) as solution in DMA (1 mL) was added at a rate to maintain the temperature below 65°C (slightly exothermic). The resulting slurry was stirred for 20 minutes. A solution of tert-butyl 4-iodopiperidine-1-carboxylate (937 mg, 3.01 mmol) in 2 mL DMA was slowly added to the mixture. The resulting reaction mixture was then stirred for 30 minutes at RT. The reaction was allowed to stand for 15 min without stirring for decantation.

In a 25 mL three-necked flask, 3-bromo-5-ethylpyridine (280 mg, 1.5 mmol) was combined with 1.5 mL DMA to give a colorless solution, copper (I) iodide (28.7 mg, 150 µmol) and [1,1'-Bis(diphenylphosphino)ferrocene]dichloropalladium(II), complex with dichloromethane (61.5 mg, 75.2 µmol) were added. The reaction mixture was degassed with argon, the freshly prepared Zinc-reagent solution was added, again degassed with argon, and the reaction mixture was stirred for 2.5 hr at 80°C. The reaction mixture was quenched with 10 mL sat. aqueous NH₄Cl solution and extracted withEtOAc (40 mL each). The organic layers were washed with brine. The organic layers were combined, dried over MgSO₄ and concentrated *in vacuo.* Purification by SiO₂ flash chromatography using n-heptane / EtOAc 0 to 60% in 40 min, afforded the desired product as light yellow oil (299 mg, 68.4%). MS (ESI): m/z = 291.2 [M+H]⁺.

### BB135

### 5-(1,1 -Difluoroethyl)-2-(piperidin-4-yl)pyridine dihydrochloride

In a 25 mL tube tert-butyl 4-(5-(1,1-difluoroethyl)pyridin-2-yl)piperidine-1-carboxylate (416mg, 1.27 mmol) was dissolved in DCM (3 mL) and HCl in ether 2M (3.82 mL, 7.65 mmol) was added. The reaction was stirred 4 h at RT. The solvent was removed *in vacuo* to obtain the crude product as a yellow solid, 381 mg (99%). Used without further purification. MS (ESI): m/z = 227.2 [M+H]⁺.

### Intermediate:

### tert-Butyl 4-(5-(1,1-difluoroethyl)pyridin-2-yl)piperidine-1-carboxylate

In a dried 25 mL three-necked flask, zinc powder (210 mg, 3.22 mmol) was combined with 1.5 mL DMA (over molecular sieve) to give a grey suspension. The mixture was stirred at RT while a 7:5 v/v mixture of chlorotrimethylsilane (32 ul) and 1,2-dibromoethane (22 ul) as solution in DMA (1 mL) was added at a rate to maintain the temperature below 65°C (slightly exothermic). The resulting slurry was stirred for 20 minutes. A solution of tert-butyl 4-iodopiperidine-1-carboxylate (841 mg, 2.7 mmol) in 2 mL DMA was slowly added to the mixture. The resulting reaction mixture was then stirred for 30 minutes at RT. The reaction was allowed to stand for 15 min without stirring for decantation.

In a 25 mL three-necked flask, 2-bromo-5-(1,1-difluoroethyl)pyridine (300 mg, 1.35 mmol) was combined with 1.5 mL DMA to give a colorless solution, copper (I) iodide (25.7 mg, 135 µmol) and [1,1'-Bis(diphenylphosphino)ferrocene]dichloropalladium(II), complex with dichloromethane (55.2 mg, 67.6 µmol) were added. The reaction mixture was degassed with argon, the freshly prepared Zinc-reagent solution was added, again degassed with argon, and the reaction mixture was stirred for 2.5 hr at 80°C. The reaction mixture was quenched with 10 mL sat. NH₄Cl and extracted twice with EtOAc (40 mL each), The organic layers were washed with brine. The organic layers were combined, dried over MgSO₄ and concentrated *in vacuo.* Purification by SiO₂ flash chromatography using n-heptane / EtOAc 0 to 50% in 40 min, afforded the desired product as yellow oil (419 mg, 85%). MS (ESI): m/z = 327.2 [M+H]⁺.

### BB136

### (4aR,8aS)-6-(4-(5-(1,1-Difluoroethyl)pyridin-2-yl)-3-methylpiperidine-1-carbonyl)hexahydro-2H-pyrido[4,3-b][1,4]oxazin-3(4H)-one

The title compound was prepared in analogy to Method A2 from BB14a and 5-(1,1-difluoroethyl)-2-(3-methyl-4-piperidyl)pyridine dihydrochloride. MS (ESI): m/z = 423.4 [M+H]⁺.

### Intermediates:

### a) 5-(1,1-Difluoroethyl)-2-(3-methyl-4-piperidyl)pyridine dihydrochloride

In a 25 mL tube tert-butyl 4-(5-(1,1-difluoroethyl)pyridin-2-yl)-3-methylpiperidine-1-carboxylate (265 mg, 778 µmol) was dissolved in DCM (3 mL) and HCl in ether 2M (3.11 mL, 6.23 mmol) was added. The reaction was stirred 4 h at RT. The solvent was removed in vacuo to obtain the crude product as a light yellow solid (242 mg, 99%). Used without further purification. MS (ESI): m/z = 241.2 [M+H]⁺.

### b) tert-Butyl 4-[5-(1,1-difluoroethyl)-2-pyridyl]-3-methyl-piperidine-1-carboxylate

In a dried 25 mL three-necked flask, zinc powder (140 mg, 2.14 mmol) was combined with 1.5 mL DMA (over molecular sieve) to give a grey suspension. The mixture was stirred at RT while a 7:5 v/v mixture of chlorotrimethylsilane (32 ul) and 1,2-dibromoethane (22 ul) as solution in DMA (1 mL) was added at a rate to maintain the temperature below 65°C (slightly exothermic). The resulting slurry was stirred for 20 minutes. A solution of tert-butyl 4-iodo-3-methylpiperidine-1-carboxylate (586 mg, 1.8 mmol) in 2mL DMA was slowly added to the mixture. The resulting reaction mixture was then stirred for 30 minutes at RT.The reaction was allowed to stand for 15 min without stirring for decantation.

In a 25 mL three-necked flask, 2-bromo-5-(1,1-difluoroethyl)pyridine (200 mg, 901 µmol, CAS RN 1211521-60-6) was combined with 1.5 mL DMA to give a colorless solution, copper (I) iodide (17.2 mg, 90.1 µmol) and [1,1'-bis(diphenylphosphino)ferrocene]dichloropalladium(II), complex with dichloromethane (36.8 mg, 45 µmol) were added. The reaction mixture was degassed with argon, the freshly prepared Zinc-reagent solution was added, again degassed with argon, and the reaction mixture was stirred for 3hr at 80°C. The reaction mixture was quenched with 10 mL sat. NH₄Cl and extracted twice with EtOAc (40 mL each). The organic layers were washed with brine. The organic layers were combined, dried over MgSO₄ and concentrated *in vacuo.* Purification by SiO₂ flash chromatograpy using n-heptane / EtOAc (0 to 50% over 35 min), afforded the desired product as yellow oil (266 mg, 87%). MS (ESI): m/z = 341.2 [M+H]⁺.

### BB137

### 1-(2,2,2-Trifluoro-1-phenyl-ethyl)piperazine hydrochloride

In a 25 mL tube tert-butyl 4-(2,2,2-trifluoro-1-phenylethyl)piperazine-1-carboxylate (155 mg, 450 µmol) was dissolved in DCM (2 mL) and HCl in ether 2M (1.8 mL, 3.6 mmol) was added. The reaction was stirred 4 h at RT. The solvent was removed *in vacuo* to obtain the crude product as a light brown solid, 127 mg (100%). Used without further purification. MS (ESI): m/z = 245.3 [M+H]⁺.

### Intermediate

### tert-Butyl 4-(2,2,2-trifluoro-1-phenyl-ethyl)piperazine-1-carboxylate

To a dry 100 mL flask with septum and N₂ bubbler was added tert-butyl piperazine-1-carboxylate (1000 mg, 5.37 mmol), triethylamine (1.63 g, 2.25 mL, 16.1 mmol), 2,2,2-trifluoro-1-phenylethan-1-one (935 mg, 754 µl, 5.37 mmol) and DCM (33 mL). Titanium tetrachloride 1M in DCM (2.68 mL, 2.68 mmol) was added via syringe. The reaction was stirred for 18h, carefully quenched with a methanolic solution of NaCNBH₃ (sodium cyanoborohydride (1.01 g, 16.1 mmol) in methanol (12.8 mL, 316 mmol) and stirred for 15 min. The reaction was basified to pH 13 with 5 M NaOH (0.5 mL), extracted with DCM (2x 60 mL), dried (Na₂SO₄) and evaporated to yield a yellow oil (2 g). Purification by SiO₂ flash chromatography using a gradient of EtOAc in n-heptane(0 to 30% over 40 min), afforded the desired product as a light yellow oil (156 mg, 8.4%). MS (ESI): m/z = 345.2 [M+H]⁺.

### BB138

### 1-[1-(2,4-Difluorophenyl)-2,2,2-trifluoro-ethyl)piperazine;hydrochloride

In a 25 mL tube tert-butyl 4-(2,2,2-trifluoro-1-phenylethyl)piperazine-1-carboxylate (330 mg, 868 µmol) was dissolved in DCM (2 mL) and HCl in ether 2M (2.6 mL, 5.1 mmol) was added. The reaction was stirred 4 h at RT. The solvent was removed *in vacuo* to obtain the crude product as a brown viscous oil, 266 mg (97%). Used without further purification. MS (ESI): m/z = 282.3 [M+H]⁺.

### Intermediate

### tert-Butyl 4-[1-(2,4-difluorophenyl)-2,2,2-trifluoro-ethyl]piperazine-1-carboxylate

To a dry 100 mL flask with septum and N₂ bubbler was added tert-butyl piperazine-1-carboxylate (1000 mg, 5.37 mmol), triethylamine (1.63 g, 2.25 mL, 16.1 mmol), 1-(2,4-difluorophenyl)-2,2,2-trifluoroethan-1-one (1.13 g, 5.37 mmol) and DCM (33 mL). Titanium tetrachloride 1M in DCM (2.68 mL, 2.68 mmol) was added via syringe. The reaction was stirred for 18h, carefully quenched with a methanolic solution of NaCNBH₃ (sodium cyanoborohydride (1.01 g, 16.1 mmol) in methanol (4.3 mL, 107 mmol) and stirred for 6 hr. The reaction was basified with NaHCO₃ sat. (10 mL). The insoluble material obtained was filtered off using celite, the filtrate was extracted with DCM (2x 60 mL), dried (Na₂SO₄) and evaporated to yield a yellow oil (2.1 g).

Purification by SiO₂ flash chromatography n-heptane / EtOAc 0 to 20% in 40 min, afforded the desired product as a yellow oil (340 mg, 13.3%). MS (ESI): m/z = 381.1 [M+H]⁺.

### BB139

### 2-Cyclopropyl-4-(4-piperidyl)pyridine dihydrochloride

In a 25 mL tube tert-butyl 4-(2-cyclopropylpyridin-4-yl)piperidine-1-carboxylate (278mg, 919 µmol) was dissolved in DCM (2 mL) and HCl in ether 2M (2.76 mL, 5.52 mmol) was added. The reaction was stirred 4 h at RT. The solvent was removed *in vacuo* to obtain the crude product as a light yellow solid, 250 mg (99%). Used without further purification. MS (ESI): m/z = 202.4 [M+H]⁺.

### Intermediate

### tert-Butyl 4-(2-cyclopropyl-4-pyridyl)piperidine-1-carboxylate

In a dried 25 mL three-necked flask, zinc powder (196 mg, 3 mmol) was combined with 1.5 mL DMA (over molecular sieve) to give a grey suspension. The mixture was stirred at RT while a 7:5 v/v mixture of chlorotrimethylsilane (32 ul) and 1,2-dibromoethane (22 ul) as solution in DMA (1 mL) was added at a rate to maintain the temperature below 65°C (slightly exothermic). The resulting slurry was stirred for 20 minutes. A solution tert-butyl 4-iodopiperidine-1-carboxylate (786 mg, 2.52 mmol) in 2mL DMA was slowly added to the mixture. The resulting reaction mixture was then stirred for 30 minutes at RT.The reaction was allowed to stand for 15 min without stirring for decantation.

In a 25 mL three-necked flask, 4-bromo-2-cyclopropylpyridine (250 mg, 1.26 mmol) was combined with 1.5 mL DMA to give a colorless solution, copper (I) iodide (24 mg, 126 µmol) and [1,1'-Bis(diphenylphosphino)ferrocene]dichloropalladium(II), complex with dichloromethane (51.1 mg, 63.1 µmol) were added. The reaction mixture was degassed with argon, the freshly prepared Zinc-reagent solution was added, again degassed with argon, and the reaction mixture was stirred for 3hr at 80°C. The reaction mixture was quenched with 10 mL sat. aqueous NH₄Cl solution and extracted with EtOAc (40 mL each). The organic layers were washed with brine. The organic layers were combined, dried over MgSO₄ and concentrated *in vacuo.* Purification by SiO₂ flash chromatography using n-heptane / 0 to 60% in 45min, afforded the desired product as light yellow oil (278 mg, 73%). MS (ESI): m/z = 303.2 [M+H]⁺.

### BB140

### 3-Methyl-5-(4-piperidyl)-2-(trifluoromethyl)pyridine dihydrochloride

In a 25 mL tube tert-butyl 4-(5-methyl-6-(trifluoromethyl)pyridin-3-yl)piperidine-1-carboxylate (360 mg, 1.05 mmol) was dissolved in DCM (3 mL) and HCl in ether 2M (2.61 mL, 5.23 mmol) was added. The reaction was stirred 4 h at RT. The solvent was removed *in vacuo* to obtain the crude product as a white solid, 314 mg (95%). Used without further purification. MS (ESI): m/z = 245.2 [M+H]⁺.

### Intermediate

### tert-Butyl 4-[5-methyl-6-(trifluoromethyl)-3pyridyl]piperidine-1-carboxylate

In a dried 25 mL three-necked flask, zinc powder (182 mg, 2.78 mmol) was combined with 1.5 mL DMA (over molecular sieve) to give a grey suspension. The mixture was stirred at RT while a 7:5 v/v mixture of chlorotrimethylsilane (32 ul) and 1,2-dibromoethane (22 ul) as solution in DMA (1 mL) was added at a rate to maintain the temperature below 65°C (slightly exothermic). The resulting slurry was stirred for 20 minutes. A solution tert-butyl 4-iodopiperidine-1-carboxylate (726 mg, 2.33 mmol) in 2mL DMA was slowly added to the mixture. The resulting reaction mixture was then stirred for 30 minutes at RT.The reaction was allowed to stand for 15 min without stirring for decantation.

In a 25 mL three-necked flask, 5-bromo-3-methyl-2-(trifluoromethyl)pyridine (280 mg, 1.17 mmol) was combined with 1.5 mL DMA to give a colorless solution, copper (I) iodide (22.2 mg, 117 µmol) and [1,1'-Bis(diphenylphosphino)ferrocene]dichloropalladium(II), complex with dichloromethane (48 mg, 58 µmol) were added. The reaction mixture was degassed with argon, the freshly prepared Zinc-reagent solution was added, again degassed with argon, and the reaction mixture was stirred for 2.5 hr at 70°C. The reaction mixture was quenched with 10 mL sat. NH₄Cl and extracted twice with EtOAc (40 mL each). The organic layers were washed with brine. The organic layers were combined, dried over MgSO₄ and concentrated *in vacuo.* Purification by SiO₂ flash chromatography using n-heptane / EtOAc 0 to 50% in 40 min, afforded the desired product as a white, crystalline solid (360 mg, 90%). MS (ESI): m/z = 345.2 [M+H]⁺.

### BB141

### 4-(4-Piperidyl)-5,6, 7, 8-tetrahydroquinoline dihydrochloride

In a 25 mL tube tert-butyl 4-(5,6,7,8-tetrahydroquinolin-4-yl)piperidine-1-carboxylate (255mg, 806 µmol) was dissolved in DCM (3 mL) and HCl in ether 2M (2.01 mL, 4.03 mmol) was added. The reaction was stirred 8 h at RT. The solvent was removed *in vacuo* to obtain the crude product as a light yellow solid, 230 mg (99%). Used without further purification. MS (ESI): m/z = 217.4 [M+H]⁺.

### Intermediate

### tert-Butyl 4-(5,6,7,8-tetrahydroquinolin-4-yl)piperidine-1-carboxylate

In a dried 25 mL three-necked flask, zinc powder (177 mg, 2.71 mmol) was combined with 1.5 mL DMA (over molecular sieve) to give a grey suspension. The mixture was stirred at RT while a 7:5 v/v mixture of chlorotrimethylsilane (32 ul) and 1,2-dibromoethane (22 ul) as solution in DMA (1 mL) was added at a rate to maintain the temperature below 65°C (slightly exothermic). The resulting slurry was stirred for 20 minutes. A solution tert-butyl 4-iodopiperidine-1-carboxylate (734 mg, 2.36 mmol) in 2mL DMA was slowly added to the mixture. The resulting reaction mixture was then stirred for 30 minutes at RT.The reaction was allowed to stand for 15 min without stirring for decantation.

In a 25 mL three-necked flask, 4-bromo-5,6,7,8-tetrahydroquinoline (250 mg, 1.18 mmol) was combined with 1.5 mL DMA to give a colorless solution, copper (I) iodide (22.4 mg, 118 µmol) and [1,1'-Bis(diphenylphosphino)ferrocene]dichloropalladium(II), complex with dichloromethane (48 mg, 58 µmol) were added. The reaction mixture was degassed with argon, the freshly prepared Zinc-reagent solution was added, again degassed with argon, and the reaction mixture was stirred for 2.5 hr at 75°C. The reaction mixture was quenched with 10 mL sat. NH₄Cl and extracted twice with EtOAc (40 mL each). The organic layers were washed with brine. The organic layers were combined, dried over MgSO₄ and concentrated *in vacuo.* Purification by SiO₂ flash chromatography using a gradient of EtOAc in n-heptane / (0 to 70% over 40 min), afforded the desired product as a colorless, viscous oil (255 mg, 68.4%). MS (ESI): m/z = 317.3 [M+H]⁺.

### BB142

### 3-[2-Fluoro-4-(trifluoromethyl)phenyl]azetidine trifluoroacetate

To a solution of tert-butyl 3-(2-fluoro-4-(trifluoromethyl)phenyl)azetidine-1-carboxylate (0.078 g, 244 µmol) in DCM (2 mL) was added TFA (94.1 µl, 1.22 mmol). The resulting reaction mixture was stirred at RT for 1 hour. The reaction mixture was concentrated *in vacuo* (co-evaporated with toluol) to yield the desired product as as colorless oil (85 mg, 100%). MS (ESI): m/z = 220.1 [M+H]⁺.

### Intermediate

### tert-Butyl 3-[2-fluoro-4-(trifluoromethyl)phenyl]azetidine-1-carboxylate

To an 20 mL vial equipped with a stir bar was added photocatalyst (Ir[dF(CF3)ppy]2(dtbpy))PF6 (6.93 mg, 6.17 µmol), 1-bromo-2-fluoro-4-(trifluoromethyl)benzene (150 mg, 88.5 µl, 617 µmol), tert-butyl 3-bromoazetidine-1-carboxylate (219 mg, 152 µl, 926 µmol), Tris(trimethylsilyl)silane (153 mg, 617 µmol) and anhydrous sodium carbonate (131 mg, 1.23 mmol). The vial was sealed and placed under argon before DME (5 mL) was added. To a separate vial was added NiCl₂ glyme (1.36 mg, 6.17 µmol) and 4,4'-di-tert-butyl-2,2'-dipyridyl (1.66 mg, 6.17 µmol). The precatalyst vial was sealed, purged with argon then DME (2 mL) was added to it. The precatalyst vial was sonicated for 5 min, after which, 1 mL (0.5 mol% catalayst, 0.005eq) was syringed into the reaction vessel.The solution was degassed by sparging with argon. The reaction was stirred and irradiated with a 420 nm lamp for 5 hours. The reaction was quenched by exposure to air and concentrated *in vacuo.* The crude material was purified by flash chromatography (silica gel, 50 g, gradient of 0% to 50% EtOAc in n-heptane) and a second flash chromatography (silica gel, 50 g, 0 to 20% EtOAc in n-heptane). The product was obtained as a colorless liquid (197 mg, 44%). MS (ESI): m/z = 264.2 [M-C₄H₈+H]⁺.

### BB143

### 4-(1-(4-Fluorophenyl)-1H-pyrazol-3-yl)piperidine hydrochloride

In a 25 mL tube tert-butyl 4-(1-(4-fluorophenyl)-1H-pyrazol-3-yl)piperidine-1-carboxylate (100mg, 290 µmol) was dissolved in DCM (3 mL) and HCl in ether 2M (1.16 mL, 2.23 mmol) was added. The reaction was stirred at RT for 6 h. The solvent was removed *in vacuo* to obtain the crude product as a light yellow solid (81 mg, 99%). The compound was used without further purification. MS (ESI): m/z = 246.2 [M+H]⁺.

### Intermediates:

### a) tert-Butyl 4-(1H-pyrazol-3-yl)piperidine-1-carboxylate

tert-butyl 4-acetylpiperidine-1-carboxylate (620 mg, 2.73 mmol) was combined with N,N-dimethylformamide dimethyl acetal (6.7 g, 54.6 mmol) and heated at 100°C for 15 hr. LCMS showed complete reaction. The reaction mixture was directly concentrated *in vacuo* and the residue was combined with EtOH (8 mL) and hydrazine dihydrochloride (344 mg, 3.27 mmol). The mixture was stirred at reflux for 1.5 hr. LCMS showed again complete reaction. The reaction mixture was concetrated to yield the crude product as a light yellow solid (650 mg, 95%). Used for next step without further purification.

### b) tert-Butyl 4-[1-(4-fluorophenyl)pyrazol-3-yl]piperidine-1-carboxylate

In a 100 mL flask purged with argon, tert-butyl 4-(1H-pyrazol-3-yl)piperidine-1-carboxylate (300 mg, 1.19 mmol) was suspended in DMF (8 mL), pyridine (386 µl, 4.77 mmol) , (4-fluorophenyl)boronic acid (217 mg, 1.55 mmol) and copper (II) acetate (325 mg, 1.79 mmol) were added. The resulting green solution was stirred 60 hr at RT. The solvent was removed *in vacuo,* the residue was extracted with ethyl acetat / water / sat. NaCl, dried over MgSO4, filtered and the solvent was *in vacuo.* The crude material was purified by flash chromatography (silica gel, gradient of 0% to 40% EtOAc in n-heptane over 40 minutes). The product was obtained as a colorless, viscous oil (290 mg, 70%). MS (ESI): m/z = 290.2 [M-C₄H₈+H]⁺.

### BB144

### 4-(4-Piperidyl)-3-(trifluoromethyl)pyridazine hydrochloride

In a 25 mL tert-butyl 4-(3-(trifluoromethyl)pyridazin-4-yl)piperidine-1-carboxylate (180mg, 543 µmol) was dissolved in DCM (1 mL) and HCl in ether 2M (2.72 mL, 5.43 mmol) was added. The reaction was stirred 6 h at RT. The solvent was removed *in vacuo* to obtain the crude product as a yellow solid, 145 mg (100%). Used without further purification. MS (ESI): m/z = 232.2 [M+H]⁺.

### Intermediate

### tert-Butyl 4-(3-(trifluoromethyl)pyridazin-4-yl)piperidine-1-carboxylate

Potassium (1-(tert-butoxycarbonyl)piperidin-4-yl)trifluoroborate (649 mg, 2.23 mmol), silver nitrate (68.8 mg, 405 µmol) and potassium persulfate (2.74 g, 10.1 mmol) were added to a reaction tube equipped with a stir bar. 1,2-Dichloroethane (2 mL), water (2 mL), 3-(trifluoromethyl)pyridazine (300mg, 2.03 mmol) and TFA (462 mg, 312 µl, 4.05 mmol) were successively added, and the tube was sealed. The reaction was vigorously stirred at RT for 24 h.

Then the reaction mixture was poured into 20 mL of a 1/1 v/v mixture of sat. aq. NaHCO₃ and 5% aq. NaS₂O₃ and the resulting solution was extracted three times with DCM. The combined organic layers were dried (MgSO4) and evaporated to afford the crude product. Purification by SiO₂ flash chromatography n-heptane / EtOAc 0 to 80% in 35min, afforded the desired product as a yellow, viscous oil (180 mg, 80% pure, 21.5%). Regioisomer confirmed by NMR. MS (ESI): m/z = 332.2 [M+H]⁺.

### BB145

### (4aR,8aS)-6-(3-(4-Hydroxyphenyl)azetidine-1-carbonyl)hexahydro-2H-pyrido[4,3-b][1,4]oxazin-3(4H)-one

The compound was obtained in analogy to method A4 from 3-(4-hydroxyphenyl)azetidine 4-methylbenzenesulfonate. Light brown solid. MS (ESI): m/z = 332.2 [M+H]⁺.

### Intermediates

### a) 3-(4-Hydroxyphenyl)azetidine 4-methylbenzenesulfonate

A mixture of tert-butyl 3-(4-(tert-butoxy)phenyl)azetidine-1-carboxylate (70 mg, 229 µmol) and 4-methylbenzenesulfonic acid hydrate (52.3 mg, 275 µmol) in EtOAc (1 mL) was stirred at reflux for 30 min. The mixture was evaporated to provide the desired product which was used in the next step without further purification.

### b) tert-Butyl 3-(4-(tert-butoxy)phenyl)azetidine-1-carboxylate

The compound was obtained in analogy to method A7 from 1-bromo-4-(tert-butoxy)benzene (CAS RN 60876-70-2) to furnish the desired compound as a colorless solid. MS (ESI): m/z = 250.2 [M-C₄H₈+H]⁺.

### BB146

### 3-(3,4-Dimethylphenyl)azetidine 4-methylbenzenesulfonate

The compound was obtained in analogy to BB28 from tert-butyl 3-(3,4-dimethylphenyl)azetidine-1-carboxylate to provide the desired compound as a colorless solid. MS (ESI): m/z = 162.1 [M+H]⁺.

### Intermediate

### tert-Butyl 3-(3,4-dimethylphenyl)azetidine-1-carboxylate

The compound was obtained in analogy to BB35, intermediate, from (3,4-dimethylphenyl)boronic acid to yield the desired compound as a colorless oil. MS (ESI): m/z = 206.1 [M-C₄H₈+H]⁺.

### BB147

### 3-(4-tert-Butoxyphenyl)azetidine 4-methylbenzenesulfonate

The compound was obtained in analogy to BB28 from tert-butyl 3-(4-tert-butoxyphenyl)azetidine-1-carboxylate (BB145, intermediate b) at RT as a colorless solid. MS (ESI): m/z = 206.2 [M+H]⁺.

### BB148

### 5-Chloro-1-(4-piperidyl)indoline

The compound was obtained in analogy to BB26, intermediate a, from tert-butyl 4-(5-chloroindolin-1-yl)piperidine-1-carboxylate to provide the desired compound as a grey solid. MS (ESI): m/z = 237.1 [M+H]⁺.

### Intermediate

### tert-Butyl 4-(5-chloroindolin-1-yl)piperidine-1-carboxylate

To a solution of 1-Boc-4-piperidone (972.84 mg, 4.88 mmol, CAS RN 79099-07-3) in MeOH (13 mL) was added AcOH (605.43 mg, 9.76 mmol), 5-chloroindoline (500.0 mg, 3.25 mmol, CAS RN 25658-80-4) and NaBH₃(CN) (613.63 mg, 9.76 mmol) at 25 °C and the reaction mixture was stirred for 2 h. The reaction was poured into sat. aqueous NH₄Cl solution (20 mL) and extracted three times with EtOAc (20 mL each). The combined organic layers were washed three times with water (20 mL each) and brine (20 mL), dried over Na₂SO₄ and concentrated in vacuum to provide the product as a white solid (1 g, 118.3%). MS (ESI): m/z = 337.1 [M+H]⁺. The crude product was directly used in next step without further purification.

### BB149

### 4-Chloro-2-(piperidin-4-yl)isoindoline hydrochloride

The compound was obtained in analogy to BB52 from tert-butyl 4-(4-chloroisoindolin-2-yl)piperidine-1-carboxylate to yield the desired compound. MS (ESI): m/z = 237.2 [M+H]⁺. Used in the next step without further purification.

### Intermediate

### tert-Butyl 4-(4-chloroisoindolin-2-yl)piperidine-1-carboxylate

The compound was obtained in analogy to BB148, intermediate, from 4-chloroisoindoline (CAS RN 123594-04-7 ) to provide the desired compound as a grey oil. MS (ESI): m/z = 337.3 [M+H]⁺.

### BB150

### 5'-Chloro-1'-(4-piperidyl)spiro[cyclopropane-1,3'-indoline]

The compound was obtained in analogy to BB26, intermediate, a from tert-butyl 4-(5'-chlorospiro[cyclopropane-1,3'-indoline]-1'-yl)piperidine-1-carboxylate to furnish the desired compound as a yellow solid. MS (ESI): m/z = 263.1 [M+H]⁺.

### Intermediate

### tert-Butyl 4-(5'-chlorospiro[cyclopropane-1,3'-indoline]-1'-yl)piperidine-1-carboxylate

The compound was obtained in analogy to BB148, intermediate, from 5'-chlorospiro[cyclopropane-1,3'-indoline] (CAS RN 1538359-43-1) to yield the desired compound as a pink solid. MS (ESI): m/z = 307.1 [M-C₄H₈+H]⁺.

### BB151

### 2-(Azetidin-3-yl)-4-chloroisoindoline hydrochloride

The compound was obtained in analogy to BB52 from tert-butyl 3-(4-chloroisoindolin-2-yl)azetidine-1-carboxylate to provide the desired compound as a light green solid. MS (ESI): m/z = 209.2 [M+H]⁺.

### Intermediate

### tert-Butyl 3-(4-chloroisoindolin-2-yl)azetidine-1-carboxylate

The compound was obtained in analogy to BB148, intermediate from 4-chloroisoindoline hydrochloride (CAS RN 924304-73-4) to yield the desired compound as a colorless amorphous solid. MS (ESI): m/z = 309.2 [M+H]⁺.

### BB152

### 3-[2-Methoxy-4-(2,2,2-trifluoroethyl)phenyl]azetidine 2,2,2-trifluoroacetate

The compound was obtained in analogy to BB26, intermediate a from tert-butyl 3-[2-methoxy-4-(2,2,2-trifluoroethyl)phenyl] azetidine-1-carboxylate to furnish the desired compound as a yellow oil. MS (ESI): m/z = 246.1 [M+H]⁺.

### Intermediates

### a) tert-Butyl 3-[2-methoxy-4-(2,2,2-trifluoroethyl)phenyl]azetidine-1-carboxylate

To a solution of tert-butyl 3-[2-methoxy-4-[2,2,2-trifluoro-1-(p-tolylsulfonyloxy)ethyl]phenyl]azetidine-1-carboxylate (480.0 mg, 0.930 mmol) in EtOH (24 mL) was added Pd/C (120.0 mg, 0.930 mmol) and the mixture was stirred at 20 °C under H₂ atmosphere (balloon) for 24 h. The mixture was filtered and the filtrate was concentrated. The residue was dissolved in EtOAc (30 mL) and washed with aqueous Na₂CO₃ solution (20 mL) folowed by brine, dried over Na₂SO₄ and concentrated to give the desired product as light yellow foam. (300 mg, 93.3%). MS (ESI): m/z = 290.1 [M-C₄H₈+H]⁺.

### b) tert-Butyl 3-[2-methoxy-4-[2,2,2-trifluoro-1-(p-tolylsulfonyloxy)ethyl]phenyl]azetidine-1-carboxylate

The compound was obtained in analogy to method A7 from [1-(4-bromo-3-methoxyphenyl)-2,2,2-trifluoro-ethyl] 4-methylbenzenesulfonate to get the desired compound as a light yellow oil. MS (ESI): m/z = 460.1 [M-C₄H₈+H]⁺.

### c) [1-(4-Bromo-3-methoxy-phenyl)-2,2,2-trifluoro-ethyl] 4-methylbenzenesulfonate

To a solution of 1-(4-bromo-3-methoxy-phenyl)-2,2,2-trifluoro-ethanol (1000.0 mg, 3.51 mmol), p-toluenesulfonyl chloride (668.81 mg, 3.51 mmol) and DMAP (20.0 mg, 0.180 mmol) in DCM (20 mL) was added TEA (708.62 mg, 7.02 mmol) at 0°C. The mixture was stirred at 20 °C for 12 h. The mixture was washed with water and brine, dried over Na₂SO₄, and concentrated to give the desired product as light yellow oil (1.5 g, 97.4%). The compound was used in the next step without further purification.

### d) 1-(4-Bromo-3-methoxy-phenyl)-2,2,2-trifluoro-ethanol

To a solution of 4-bromo-3-methoxy-benzaldehyde (2.0 g, 9.3 mmol, CAS RN 43192-34-3) and trifluoromethyltrimethylsilane (1586.94 mg, 11.16 mmol) in THF (30 mL) was added TBAF/THF (0.09 mL, 0.090 mmol) at 0°C, the mixture was stirred at 20 °C for 12h, then 1M aq. HCl (18.6 mL, 18.6 mmol) was added slowly. The mixture was stirred at 20°C for another 2h. The mixture was poured into water (50 mL) and extracted three times with EtOAc (30 mL each). The combined organic phase was washed with brine, dried over Na₂SO₄ and concentrated to give the desired product as light yellow oil (2.5 g, 94.3%). 1H NMR (400 MHz, CHLOROFORM-d) δ = 7.57 (d, J = 8.1 Hz, 1H), 7.05 (s, 1H), 6.94 (d, J = 8.1 Hz, 1H), 5.01 (q, J = 6.5 Hz, 1H), 3.93 (s, 3H).

### BB153

### 3-[4-(2,2-Dimethylpropoxy)phenyl]azetidine 4-methylbenzenesulfonate

The compound was obtained in analogy to BB28 at RT for 5 hours from tert-butyl 3-[4-(2,2-dimethylpropoxy)phenyl]azetidine-1-carboxylate to get the desired compound as white solid. Used as is for next step.

### Intermediate

### tert-Butyl 3-[4-(2,2-dimethylpropoxy)phenyl]azetidine-1-carboxylate

The compound was obtained in analogy to method A7 a from 1-bromo-4-(neopentyloxy)benzene (CAS RN 528528-58-7) to yield the desired compound as a colorless solid. MS (ESI): m/z = 264.2 [M-C₄H₈+H]⁺.

### BB154

### rac-(4aS,8aS)-Hexahydro-2H-pyrido[4,3-b][1,4]oxazin-3(4H)-one

rac-Benzyl (4aS,8aS)-3-oxohexahydro-2H-pyrido[4,3-b][1,4]oxazine-6(SH)-carboxylate (125 mg, 431 µmol) was dissolved in MeOH (5 mL). The reaction solution was degassed in vacuo and backfilled with argon. Pd-C (20 mg, 188 µmol) was added under an argon atmosphere. Argon was evacuated from the reaction mixture and the reaction flask backfilled with hydrogen. The reaction mixture was stirred at RT for 15 h under a hydrogen atmosphere. The reaction mixture was filtered through a syringe filter and concentrated in vacuo to afford the desired product as a white solid (62 mg, 92.2%). MS (ESI): m /z = 157.098 [M+H]⁺.

### Intermediates

### a) rac-Benzyl (4aS,8aS)-3-oxohexahydro-2H-pyrido[4,3-b][1,4]oxazine-6(5H)-carboxylate

To a stirred solution of rac-Benzyl (3S,4S)-3-(2-chloroacetamido)-4-hydroxypiperidine-1-carboxylate (385 mg, 1.18 mmol) in dry THF (4 mL) was added NaH (67.9 mg, 1.7 mmol) at 0°C. The mixture was allowed to reach RT and then stirred for 90 min under an argon atmosphere. H₂O (5 mL) was added and stirring was continued at RT for 10 min. THF was removed in vacuo from the reaction mixture. The residue was treated with DCM and the organic phase was washed with H₂O and brine, dried over Na₂SO₄, filtered and then concentrated in vacuo. The residue was purified by flash chromatography (12 g reversed phase column, gradient 0-100% ACN (0.1% FA) in water (0.1% FA) to afford the desired product as a white solid (133 mg, 38.9%). MS (ESI): m/z = 291.3 [M+H]⁺.

### b) rac-Benzyl (3S,4S)-3-(2-chloroacetamido)-4-hydroxypiperidine-1-carboxylate

To a stirred suspension of rac-benzyl (3S,4S)-3-amino-4-hydroxypiperidine-1-carboxylate (317 mg, 1.27 mmol, synthesized according to patent US 2011/59118 A1) and sodium acetate (208 mg, 2.53 mmol, CAS RN 127-09-3) in a mixture of acetone (4 mL)/H₂O (0.5 mL) was added dropwise a solution of chloroacetyl chloride (150 mg, 107 µL, 1.33 mmol, CAS RN 79-04-9) in acetone (3 mL) between 0-5°C. After the addition the reaction mixture was stirred at RT for 1 h and subsequently evaporated to dryness giving a yellow gum. The crude product was purified by silica gel chromatography to afford the desired product as a yellow solid (385 mg, 93%). MS (ESI): m /z = 325.2 [M-H]⁻.

**Table 3**

| **Ex.** | **Name** |
|---|---|
| **A1** | rac-(4aR,8aS)-6-[4-(3-Methoxy-1-phenyl-propyl)piperidine-1-carbonyl]-4,4a,5,7,8,8a-hexahydropyrido[4,3-b][1,4]oxazin-3-one |
| **A2** | rac-(4aR,8aS)-6-[4-[Phenyl(3-pyridyl)methyl]piperidine-1-carbonyl]-4,4a,5,7,8,8a-hexahydropyrido[4,3-b][1,4]oxazin-3-one |
| **A3** | rac-(4aR,8aS)-6-[4-[(5-Methyl-1,2,4-oxadiazol-3-yl)-phenylmethyl]piperidine-1-carbonyl]-4,4a,5,7,8,8a-hexahydropyrido[4,3-b][1,4]oxazin-3-one |
| **A4** | rac-(4aR,8aS)-6-[4-(5-Fluoro-1-methyl-indol-3-yl)piperidine-1-carbonyl]-4,4a,5,7,8,8a-hexahydropyrido[4,3-b][1,4]oxazin-3-one |
| **AS** | tert-Butyl 3-[5-chloro-3-[1-[rac-(4aR,8aS)-3-oxo-4,4a,5,7,8,8a-hexahydropyrido[4,3-b] [1,4]oxazine-6-carbonyl]-4-piperidyl]indol-1-yl]azetidine-1-carboxylate |
| **A6** | tert-Butyl 3-[5-chloro-3-[1-[rac-(4aR,8aS)-3-oxo-4,4a,5,7,8,8a-hexahydropyrido[4,3-b] [1,4]oxazine-6-carbonyl]-4-piperidyl]indol-1-yl]azetidine-1-carboxylate |
| **A7** | rac-(4aR,8aS)-6-[4-[1-(2-Chloro-4-fluoro-phenoxy)propyl]piperidine-1-carbonyl]-4,4a,5,7,8,8a-hexahydropyrido[4,3-b][1,4]oxazin-3-one |
| **A8** | rac-(4aR,8aS)-6-[4-[(2-Chloro-4-fluoro-phenoxy)-cyclopropylmethyl]piperidine-1-carbonyl]-4,4a,5,7,8,8a-hexahydropyrido[4,3-b][1,4]oxazin-3-one |
| **A9** | rac-(4aR,8aS)-6-[4-[(4-Fluorophenoxy)-phenyl-methyl]piperidine-1-carbonyl]-4,4a,5,7,8,8a-hexahydropyrido[4,3-b][1,4]oxazin-3-one |
| **A10** | rac-(4aR,8aS)-6-[4-[5-(Trifluoromethyl)-2-pyridyl]piperidine-1-carbonyl]-4,4a,5,7,8,8a-hexahydropyrido[4,3-b][1,4]oxazin-3-one |
| **All** | rac-(4aR,8aS)-6-[4-[Cyclopropylmethoxy-[4-(trifluoromethyl)phenyl]methyl]piperidine-1-carbonyl]-4,4a,5,7,8,8a-hexahydropyrido [4,3 -b] [1,4]oxazin-3-one |
| **A12** | rac-(4aR,8aS)-6-[4-[1-(4-Fluorophenyl)-2-hydroxy-ethyl]piperazine-1-carbonyl]-4,4a,5,7,8,8a-hexahydropyrido[4,3-b][1,4]oxazin-3-one |
| **A13** | rac-(4aR,8aS)-6-[4-[1-(4-Fluorophenyl)-2-methoxy-ethyl]piperazine-1-carbonyl]-4,4a,5,7,8,8a-hexahydropyrido[4,3-b][1,4]oxazin-3-one |
| **A14** | rac-(4aR,8aS)-6-[4-[1-(3,4-Difluorophenyl)-2-hydroxy-ethyl]piperazine-1-carbonyl]-4,4a,5,7,8,8a-hexahydropyrido[4,3-b][1,4]oxazin-3-one |
| **A15** | rac-(4aR,8aS)-6-[4-[1-(3,4-Difluorophenyl)-2-methoxy-ethyl]piperazine-1-carbonyl]-4,4a,5,7,8,8a-hexahydropyrido[4,3-b][1,4]oxazin-3-one |
| **A16** | rac-(4aR,8aS)-6-[4-[1-(4,4-Difluoro-1-piperidyl)cyclopropyl]piperidine-1-carbonyl]-4,4a,5,7,8,8a-hexahydropyrido[4,3-b][1,4]oxazin-3-one |
| **A17** | rac-(4aR,8aS)-6-[4-[1-[4-(Trifluoromethyl)imidazol-1-yl]ethyl]piperidine- 1-carbonyl]-4,4a,5,7,8,8a-hexahydropyrido[4,3-b][1,4]oxazin-3-one |
| **A18** | rac-(4aR,8aS)-6-[4-[1-[4-(Trifluoromethyl)pyrazol-1-yl]cyclopropyl]piperidine-1-carbonyl]-4,4a,5,7,8,8a-hexahydropyrido [4,3 -b] [1,4]oxazin-3-one |
| **A19** | rac-(4aR,8aS)-6-[4-[(4-tert-Butylpyrazol-1-yl)-(4-fluorophenyl)methyl]piperidine-1-carbonyl]-4,4a,5,7,8,8a-hexahydropyrido [4,3 -b] [1,4]oxazin-3-one |
| **A20** | rac-(4aR,8aS)-6-[4-[3-[4-(Trifluoromethyl)phenyl]oxetan-3-yl]piperidine-1-carbonyl]-4,4a,5,7,8,8a-hexahydropyrido[4,3-b][1,4]oxazin-3-one |
| **A21** | rac-(4aR,8aS)-6-[4-[4-(Trifluoromethyl)benzoyl]piperidine-1-carbonyl]-4,4a,5,7,8,8a-hexahydropyrido[4,3-b][1,4]oxazin-3-one |
| **A22** | rac-(4aR,8aS)-6-[4-[1-[4-(Trifluoromethyl)pyrazol-1-yl]ethyl]piperidine-1-carbonyl]-4,4a,5,7,8,8a-hexahydropyrido[4,3-b][1,4]oxazin-3-one |
| **A23** | rac-(4aR,8aS)-6-[4-[(4-Fluorophenyl)-morpholino-methyl]piperidine-1-carbonyl]-4,4a,5,7,8,8a-hexahydropyrido[4,3-b][1,4]oxazin-3-one |
| **A24** | rac-(4aR,8aS)-6-[4-[(4-tert-Butyloxazol-2-yl)-difluoro-methyl]piperidine-1-carbonyl]-4,4a,5,7,8,8a-hexahydropyrido[4,3-b][1,4]oxazin-3-one |
| **A25** | rac-(4aR,8aS)-6-[4-[1-(4-Chlorophenyl)-2-hydroxy-ethyl]piperazine-1-carbonyl]-4,4a,5,7,8,8a-hexahydropyrido[4,3-b][1,4]oxazin-3-one |
| **A26** | rac-(4aR,8aS)-6-[4-[1-(4-Chlorophenyl)-2-methoxy-ethyl]piperazine-1-carbonyl]-4,4a,5,7,8,8a-hexahydropyrido[4,3-b][1,4]oxazin-3-one |
| **A27** | rac-(4aR,8aS)-6- [4- [2-Hydroxy-1- [4-(trifluoromethyl)phenyl]ethyl]piperazine-1-carbonyl]-4,4a,5,7,8,8a-hexahydropyrido [4,3 -b] [1,4]oxazin-3-one |
| **A28** | rac-(4aR,8aS)-6- [4- [2-Methoxy-1- [4-(trifluoromethyl)phenyl]ethyl]piperazine-1-carbonyl]-4,4a,5,7,8,8a-hexahydropyrido [4,3 -b] [1,4]oxazin-3-one |
| **A29** | rac-(4aR,8aS)-6-[4-(1-Cyclohexyl-2-methoxy-ethyl)piperazine-1-carbonyl]-4,4a,5,7,8,8a-hexahydropyrido[4,3-b][1,4]oxazin-3-one |
| **A30** | rac-(4aR,8aS)-6-[4-(1-Cyclohexyl-2-hydroxy-ethyl)piperazine-1-carbonyl]-4,4a,5,7,8,8a-hexahydropyrido[4,3-b][1,4]oxazin-3-one |
| **A31** | rac-(4aR,8aS)-6-[4-[1-(4-Fluorophenyl)cyclopropyl]piperazine-1-carbonyl]-4,4a,5,7,8,8a-hexahydropyrido[4,3-b][1,4]oxazin-3-one |
| **A32** | rac-(4aR,8aS)-6-[4-[2-(4-Fluorophenyl)cyclopropyl]piperazine-1-carbonyl]-4,4a,5,7,8,8a-hexahydropyrido[4,3-b][1,4]oxazin-3-one |
| **A33** | rac-(4aR,8aS)-6-[4-[1-(4-Fluorophenyl)cyclobutyl]piperazine-1-carbonyl]-4,4a,5,7,8,8a-hexahydropyrido[4,3-b][1,4]oxazin-3-one |
| **A34** | rac-(4aR,8aS)-6-[4-[Cyclopropyl-(4-fluorophenyl)methyl]piperazine-1-carbonyl]-4,4a,5,7,8,8a-hexahydropyrido[4,3-b][1,4]oxazin-3-one |
| **A35** | rac-(4aR,8aS)-6-[4-[Cyclohexyl-(4-fluorophenyl)methyl]piperazine-1-carbonyl]-4,4a,5,7,8,8a-hexahydropyrido[4,3-b][1,4]oxazin-3-one |
| **A36** | rac-(4aR, 8 aS)-6- [4- [(4-Fluorophenyl)-(4-pyridyl) methyl]piperazine-1-carbonyl]-4,4a,5,7,8,8a-hexahydropyrido[4,3-b][1,4]oxazin-3-one |
| **A37** | rac-(4aR,8aS)-6-[4-[2,2,2-Trifluoro-1-(4-fluorophenyl)ethyl]piperazine-1-carbonyl]-4,4a,5,7,8,8a-hexahydropyrido[4,3-b][1,4]oxazin-3-one |
| **A38** | rac-(4aR, 8 aS)-6- [4- [(4-Fluorophenyl)-oxazol-5-yl-methyl]piperazine-1-carbonyl]-4,4a,5,7,8,8a-hexahydropyrido[4,3-b][1,4]oxazin-3-one |
| **A39** | rac-(4aR,8aS)-6-[4-[bis(4-Chlorophenyl)methyl]piperazine-1-carbonyl]-4,4a,5,7,8,8a-hexahydropyrido[4,3-b][1,4]oxazin-3-one |
| **A40** | rac-(4aS,8aS)-6-[4-[5-Chloro-1-(cyclopropylmethyl)indol-3-yl]piperidine-1-carbonyl]-4,4a,5,7,8,8a-hexahydropyrido[4,3-b][1,4]oxazin-3-one |
| **A41** | rac-(4aS,8aS)-6-[4-[5-Chloro-1-(cyclopropylmethyl)indol-3-yl]piperidine-1-carbonyl]-4,4a,5,7,8,8a-hexahydropyrido[4,3-b][1,4]oxazin-3-one |
| **A42** | rac-(4aR,8aR)-6-[4-[5-Chloro-1-(cyclopropylmethyl)indol-3-yl]piperidine-1-carbonyl]-4,4a,5,7,8,8a-hexahydropyrido[4,3-b][1,4]oxazin-3-one |
| **A43** | rac-(4aR,8aS)-6-[4-[(4-Chlorophenyl)-(4-fluorophenyl)methyl]piperazine-1-carbonyl]-4,4a,5,7,8,8a-hexahydropyrido [4,3 -b] [1,4]oxazin-3-one |
| **A44** | rac-(4aR,8aS)-6-(4-Benzhydrylpiperazine-1-carbonyl)-4,4a,5,7,8,8a-hexahydropyrido[4,3-b][1,4]oxazin-3-one |
| **A45** | rac-(4aR,8aS)-6-[4-[4-(2,2,2-Trifluoroethyl)phenyl]piperazine-1-carbonyl]-4,4a,5,7,8,8a-hexahydropyrido[4,3-b][1,4]oxazin-3-one |
| **A46** | rac-(4aR,8aS)-6-[4-[3-(2,2,2-Trifluoroethyl)phenyl]piperazine-1-carbonyl]-4,4a,5,7,8,8a-hexahydropyrido[4,3-b][1,4]oxazin-3-one |
| **A47** | rac-(4aR,8aS)-6-[4-[4-Chloro-3-(trifluoromethyl)phenyl]piperazine-1-carbonyl]-4,4a,5,7,8,8a-hexahydropyrido[4,3-b][1,4]oxazin-3-one |
| **A48** | rac-(4aR,8aS)-6-[4-[1-[4-(Trifluoromethyl)phenyl]ethyl]piperazine-1-carbonyl]-4,4a,5,7,8,8a-hexahydropyrido[4,3-b][1,4]oxazin-3-one |
| **A49** | rac-(4aR,8aS)-6-[3-[1-(2-Chloro-4-fluoro-phenoxy)ethyl]azetidine-1-carbonyl]-4,4a,5,7,8,8a-hexahydropyrido[4,3-b][1,4]oxazin-3-one |
| **A50** | rac-(4aR,8aS)-6-[3-[5-Chloro-1-(cyclopropylmethyl)indol-3-yl]azetidine-1-carbonyl]-4,4a,5,7,8,8a-hexahydropyrido[4,3-b][1,4]oxazin-3-one |

### Example A

A compound of formula (Ic) can be used in a manner known per se as the active ingredient for the production of tablets of the following composition:

| | Per tablet |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example B

A compound of formula (Ic) can be used in a manner known per se as the active ingredient for the production of capsules of the following composition:

| | Per capsule |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

## Claims

1. A compound of formula (Ic) 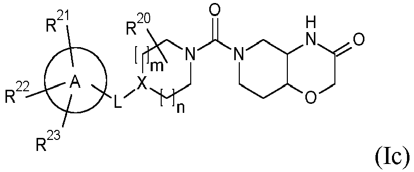 or a pharmaceutically acceptable salt thereof, wherein:
L is selected from -CR¹R²-(CH₂)ₚ-, -(CH₂)ₚ-CR¹R²-, -OCR³R⁴-, -CR³R⁴O- and a covalent bond;
m is 0, n is 0 or 1 and X is CR²⁴; or
m is 1, n is 1 or 2 and X is CR²⁴ or N;
p is 0, 1 or 2;
R¹ is selected from halogen, C₁₋₆-alkoxy, C₁₋₆-alkyl, halo-C₁₋₆-alkyl, hydroxy-C₁₋₆-alkyl, C₁₋₆-alkoxy-C₁₋₆-alkyl-, halo-C₁₋₆-alkoxy, halo-C₁₋₆-alkoxy-C₁₋₆-alkyl-, cyano, and a group 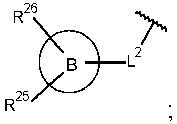 and R² is selected from hydrogen, halogen, and hydroxy; or
R¹ and R², taken together with the carbon atom to which they are attached, form a C₃₋₁₂-cycloalkyl or a C₂₋₉-heterocyclyl;
R³ is selected from C₁₋₆-alkyl, halo-C₁₋₆-alkyl, C₃₋₁₂-cycloalkyl, and C₆₋₁₄-aryl; and R⁴ is hydrogen; or
R³ and R⁴, taken together with the carbon atom to which they are attached, form a C₃₋₁₂-cycloalkyl or a C₂₋₉-heterocyclyl;
R²⁰ is hydrogen or C₁₋₆-alkyl;
R²¹, R²², and R²³ are independently selected from hydrogen, halogen, cyano, hydroxy, C₁₋₆-alkoxy, halo-C₁₋₆-alkoxy, amino-C₁₋₆-alkoxy, C₁₋₆-alkyl, halo-C₁₋₆-alkyl, hydroxy-C₁₋₆-alkyl, (halo-C₁₋₆-alkyl)-hydroxy-C₁₋₆-alkyl, C₁₋₆-alkoxycarbonyl-C₁₋₆-alkyl-, C₁₋₆-alkoxycarbonyl-NH-C₁₋₆-alkoxy-, C₁₋₆-alkoxycarbonyl-NH-(C₁₋₆-alkoxy)₂-C₁₋₆-alkyl-C(O)-NH-C₁₋₆-alkoxy-, C₁₋₆-alkoxycarbonyl-NH-C₁₋₆-alkoxy-C₁₋₆-alkyl-C(O)-NH-C₁₋₆-alkoxy-, SF₅, (C₁₋₆-alkyl)₃Si-O-C₁₋₆-alkyl-, a group 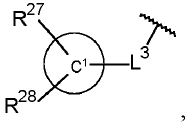 and a group 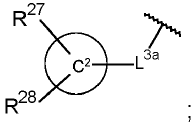
R²⁴ is selected from hydrogen, halogen, hydroxy, halo-C₁₋₆-alkyl, and C₁₋₆-alkyl;
R²⁵ and R²⁶ are independently selected from hydrogen, halogen, C₁₋₆-alkyl, C₁₋₆-alkoxy, halo-C₁₋₆-alkyl, halo-C₁₋₆-alkoxy, C₁₋₆-alkyl-SO₂-, and C₃₋₁₂-cycloalkyl;
R²⁷ and R²⁸ are independently selected from hydrogen, halogen, C₁₋₆-alkoxy, halo-C₁₋₆-alkoxy, C₁₋₆-alkyl, halo-C₁₋₆-alkyl, hydroxy-C₁₋₆-alkyl, halogen, hydroxy, C₁₋₆-alkylsulfonyl, carbamoyl, cyano, cycloalkyl-C₁₋₆-alkoxy-, C₁₋₆-alkyl-NH-C(O)-, and cycloalkyl;
A, and B are independently selected from C₆₋₁₄-aryl, C₁₋₁₃-heteroaryl, C₃₋₁₂-cycloalkyl, and C₂₋₉-heterocyclyl;
C¹ is C₃₋₁₂-cycloalkyl or C₂₋₉-heterocyclyl;
C² is C₆₋₁₄-aryl;
L² is selected from a covalent bond, -O-, -CH₂O-, -CH₂OCH₂-, and -CH₂-;
L³ is selected from a covalent bond, -O-, -CH₂O-, -OCH₂-, -CH₂OCH₂-, and -CH₂-; and
L^{3a} is selected from a covalent bond, -CH₂O-, -OCH₂-, -CH₂OCH₂-, and -CH₂-.

2. The compound of formula (Ic) according to claims 1, or a pharmaceutically acceptable salt thereof, wherein:
m is 0, n is 0 or 1 and X is CR²⁴; or
m is 1, n is 1 and X is CR²⁴ or N; and
R²⁰ and R²⁴ are both hydrogen.

3. The compound of formula (Ic) according to any one of claims 1 and 2, or a pharmaceutically acceptable salt thereof, wherein:
L is selected from -CR¹R²-(CH₂)ₚ-, -OCR³R⁴-, -CR³R⁴O- and a covalent bond;
p is 0 or 1;
R¹ is selected from halogen, C₁₋₆-alkoxy, C₁₋₆-alkyl, halo-C₁₋₆-alkyl, hydroxy-C₁₋₆-alkyl, C₁₋₆-alkoxy-C₁₋₆-alkyl-, halo-C₁₋₆-alkoxy, halo-C₁₋₆-alkoxy-C₁₋₆-alkyl-, cyano, and a group 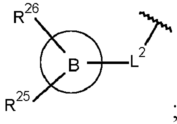 and R² is selected from hydrogen, halogen, and hydroxy; or
R¹ and R², taken together with the carbon atom to which they are attached, form a C₃₋₁₂-cycloalkyl;
R³ is C₁₋₆-alkyl or halo-C₁₋₆-alkyl;
R⁴ is hydrogen;
R²¹ is selected from hydrogen, halogen, hydroxy, C₁₋₆-alkoxy, halo-C₁₋₆-alkoxy, C₁₋₆-alkyl, halo-C₁₋₆-alkyl, hydroxy-C₁₋₆-alkyl, (halo-C₁₋₆-alkyl)-hydroxy-C₁₋₆-alkyl, C₁₋₆-alkoxycarbonyl-C₁₋₆-alkyl-, C₁₋₆-alkoxycarbonyl-NH-C₁₋₆-alkoxy-, SF₅, (C₁₋₆-alkyl)₃Si-O-C₁₋₆-alkyl-, a group 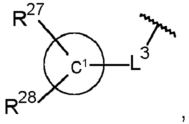 and a group 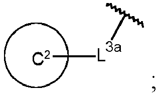
R²² is selected from hydrogen, halogen, C₁₋₆-alkoxy, halo-C₁₋₆-alkoxy, C₁₋₆-alkyl, and cyano;
R²³ is hydrogen or halogen;
R²⁵ is selected from hydrogen, halogen, C₁₋₆-alkyl, C₁₋₆-alkoxy, halo-C₁₋₆-alkyl, halo-C₁₋₆-alkoxy, C₁₋₆-alkyl-SO₂-, and C₃₋₁₂-cycloalkyl;
R²⁶ is selected from hydrogen, C₁₋₆-alkyl, and C₁₋₆-alkoxy;
R²⁷ is selected from hydrogen, halogen, C₁₋₆-alkoxy, halo-C₁₋₆-alkoxy, C₁₋₆-alkyl, halo-C₁₋₆-alkyl, hydroxy-C₁₋₆-alkyl, halogen, hydroxy, C₁₋₆-alkylsulfonyl, carbamoyl, cyano, cycloalkyl-C₁₋₆-alkoxy-, and cycloalkyl;
R²⁸ is selected from hydrogen, C₁₋₆-alkyl, and halogen;
A is C₆₋₁₄-aryl or C₁₋₁₃-heteroaryl;
B is C₆₋₁₄-aryl or C₁₋₁₃-heteroaryl;
L² is selected from a covalent bond, -O-, and -CH₂-;
L³ is selected from a covalent bond, -CH₂O-, and -CH₂-; and
L^{3a} is a covalent bond or -CH₂-.

4. The compound of formula (Ic) according to any one of claims 1 to 4, or a pharmaceutically acceptable salt thereof, wherein:
L is selected from -CR¹R²-(CH₂)ₚ-, -OCR³R⁴-, -CR³R⁴O- and a covalent bond;
p is 0;
R¹ is selected from halogen, C₁₋₆-alkyl, hydroxy-C₁₋₆-alkyl, C₁₋₆-alkoxy-C₁₋₆-alkyl-, halo-C₁₋₆-alkoxy, and a group 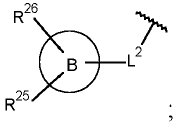
R² is hydrogen or halogen;
R³ is C₁₋₆-alkyl;
R⁴ is hydrogen;
R²¹ is selected from halogen, C₁₋₆-alkoxy, halo-C₁₋₆-alkoxy, C₁₋₆-alkyl, halo-C₁₋₆-alkyl, SF₅, C₆₋₁₄-aryl, and a group 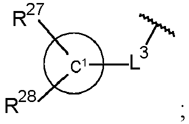
R²² is selected from hydrogen, halogen, C₁₋₆-alkoxy and halo-C₁₋₆-alkoxy;
R²³ is hydrogen or halogen;
R²⁵ is selected from hydrogen, halogen, C₁₋₆-alkoxy, and C₃₋₁₂-cycloalkyl;
R²⁶ is hydrogen or C₁₋₆-alkoxy;
R²⁷ is selected from hydrogen, halo-C₁₋₆-alkoxy, C₁₋₆-alkyl, halo-C₁₋₆-alkyl, C₁₋₆-alkyl-NH-C(O)-, and halogen;
R²⁸ is selected from hydrogen, C₁₋₆-alkyl, and halogen;
A is C₆₋₁₄-aryl or C₁₋₁₃-heteroaryl;
B is C₆₋₁₄-aryl or C₁₋₁₃-heteroaryl;
C¹ is C₃₋₁₂-cycloalkyl or C₂₋₉-heterocyclyl;
L² is a covalent bond; and
L³ is a covalent bond or -CH₂-.

5. The compound of formula (Ic) according to any one of claims 1 to 4, or a pharmaceutically acceptable salt thereof, wherein:
L is selected from -CR¹R²-(CH₂)ₚ-, -OCR³R⁴-, -CR³R⁴O- and a covalent bond;
p is 0;
R¹ is selected from 2-methoxyethyl, methyl, 2,2,2-trifluoroethoxy, fluoro, 2-hydroxyethyl, and a group 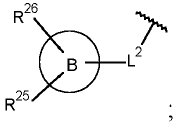
R² is hydrogen or fluoro;
R³ is methyl;
R⁴ is hydrogen;
R²¹ is selected from fluoro, chloro, bromo, methyl, methoxy, tert-butyl, propyl, trifluoromethoxy, 2-fluoroethoxy, 2,2,2-trifluoroethoxy, trifluoromethyl, 2,2,2-trifluoroethyl, 1,1-difluoroethyl, SFs, phenyl, and a group 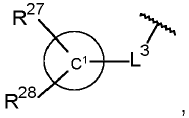 wherein R²⁷, R²⁸, C¹, and L³ are as defined herein;
R²² is selected from hydrogen, fluoro, chloro, methoxy, methyl, and trifluoromethyl;
R²³ is hydrogen or fluoro;
R²⁵ is selected from hydrogen, methoxy, fluoro, and cyclopropyl;
R²⁶ is hydrogen or methoxy;
R²⁷ is selected from methyl, trifluoromethoxy, trifluoromethyl, 2,2,2-trifluoro-1-methyl-ethoxy, 2,2,2-trifluoro-1,1-dimethyl-ethoxy, 2,2,2-trifluoroethoxy, fluoro, and chloro;
R²⁸ is selected from hydrogen, methyl, fluoro, and chloro;
A is selected from phenyl, indol-3-yl, 2-pyridyl, and 3-pyridyl;
B is phenyl or 1,2,4-oxadiazol-5-yl;
C¹ is selected from azetidin-1-yl, pyrrolidin-1-yl, cyclopropyl, and oxetan-3-yl;
L² is a covalent bond; and
L³ is a covalent bond or -CH₂-.

6. The compound of formula (Ic) according to any one of claims 1 to 3, or a pharmaceutically acceptable salt thereof, wherein C² is phenyl.

7. The compound of formula (Ic) according to any one of claims 1 to 3, or a pharmaceutically acceptable salt thereof, wherein L^{3a} is a covalent bond.

8. The compound of formula (Ic) according to claim 1, or a pharmaceutically acceptable salt thereof, wherein:
m is 0, n is 0 or 1 and X is CR²⁴; or
m is 1, n is 1 and X is CR²⁴ or N;
L is selected from -CR¹R²-(CH₂)ₚ-, -OCHR³-, -CHR³O- and a covalent bond;
p is 0 or 1;
R¹ is selected from halogen, C₁₋₆-alkoxy, C₁₋₆-alkyl, halo-C₁₋₆-alkyl, hydroxy-C₁₋₆-alkyl, C₁₋₆-alkoxy-C₁₋₆-alkyl-, halo-C₁₋₆-alkoxy, halo-C₁₋₆-alkoxy-C₁₋₆-alkyl-, cyano, and a group 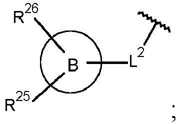 and R² is selected from hydrogen, halogen, and hydroxy; or
R¹ and R², taken together with the carbon atom to which they are attached, form a C₃₋₁₂-cycloalkyl;
R³ is C₁₋₆-alkyl or halo-C₁₋₆-alkyl;
R²⁰ is hydrogen or C₁₋₆-alkyl;
R²¹ is selected from hydrogen, halogen, hydroxy, C₁₋₆-alkoxy, halo-C₁₋₆-alkoxy, C₁₋₆-alkyl, halo-C₁₋₆-alkyl, hydroxy-C₁₋₆-alkyl, (halo-C₁₋₆-alkyl)-hydroxy-C₁₋₆-alkyl, C₁₋₆-alkoxycarbonyl-C₁₋₆-alkyl-, C₁₋₆-alkoxycarbonyl-NH-C₁₋₆-alkoxy-, SF₅, (C₁₋₆-alkyl)₃Si-O-C₁₋₆-alkyl-, a group 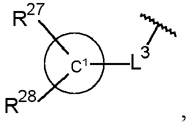 and a group 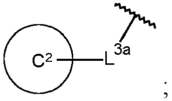
R²² is selected from hydrogen, halogen, C₁₋₆-alkoxy, halo-C₁₋₆-alkoxy, C₁₋₆-alkyl, and cyano;
R²³ is hydrogen or halogen;
R²⁴ is selected from hydrogen, halogen, hydroxy, and C₁₋₆-alkyl;
R²⁵ is selected from hydrogen, halogen, C₁₋₆-alkyl, C₁₋₆-alkoxy, halo-C₁₋₆-alkyl, halo-C₁₋₆-alkoxy, C₁₋₆-alkyl-SO₂-, and C₃₋₁₂-cycloalkyl;
R²⁶ is selected from hydrogen, C₁₋₆-alkyl, and C₁₋₆-alkoxy;
R²⁷ is selected from hydrogen, halogen, C₁₋₆-alkoxy, halo-C₁₋₆-alkoxy, C₁₋₆-alkyl, halo-C₁₋₆-alkyl, hydroxy-C₁₋₆-alkyl, halogen, hydroxy, C₁₋₆-alkylsulfonyl, carbamoyl, cyano, cycloalkyl-C₁₋₆-alkoxy-, and cycloalkyl;
R²⁸ is selected from hydrogen, C₁₋₆-alkyl, and halogen;
A is C₆₋₁₄-aryl or C₁₋₁₃-heteroaryl;
B is C₆₋₁₄-aryl or C₁₋₁₃-heteroaryl;
C¹ is C₃₋₁₂-cycloalkyl or C₂₋₉-heterocyclyl;
C² is C₆₋₁₄-aryl;
L² is selected from a covalent bond, -O-, and -CH₂-;
L³ is selected from a covalent bond, -CH₂O-, and -CH₂-; and
L^{3a} is a covalent bond or -CH₂-.

9. The compound of formula (Ic) according to any one of claims 1 to 8, or a pharmaceutically acceptable salt thereof, selected from:
rac-(4aR,8aS)-6-(4-Benzhydrylpiperidine-1-carbonyl)-4,4a, 5,7,8, 8a-hexahydropyrido[4,3-b][1,4]oxazin-3-one;
rac-(4aR,8aS)-6-(4-(5-Chloro-1-(cyclopropylmethyl)-1H-indol-3-yl)piperidine-1-carbonyl)hexahydro-2H-pyrido[4,3-b][1,4]oxazin-3(4H)-one;
rac-(4aR,8aS)-6-(4-(5-Chloro-1-methyl-1H-indol-3-yl)piperidine-1-carbonyl)hexahydro-2H-pyrido[4,3-b][1,4]oxazin-3(4H)-one;
rac-(4aR,8aS)-6-(4-(9H-Fluoren-9-yl)piperidine-1-carbonyl)hexahydro-2H-pyrido[4,3-b][1,4]oxazin-3 (4H)-one;
(+)-cis-6-[4-(6-Fluoro-1H-indol-3-yl)piperidine-1-carbonyl]-4,4a,5,7,8,8a-hexahydropyrido[4,3-b][1,4]oxazin-3-one;
(4aR,8aS)-6-(4-(1-(4-Fluorophenyl)-3-methoxypropyl)piperidine-1-carbonyl)hexahydro-2H-pyrido[4,3-b][1,4]oxazin-3(4H)-one;
rac-(4aR,8aS)-6-[4-(6-Fluoro-1H-indol-3-yl)piperidine-1-carbonyl]-4,4a,5,7,8,8a-hexahydropyrido[4,3-b][1,4]oxazin-3-one;
(4aR,8aS)-6-(4-((4-Fluorophenyl)(methoxy)methyl)piperidine-1-carbonyl)hexahydro-2H-pyrido[4,3-b][1,4]oxazin-3(4H)-one;
rac-(4aR,8aS)-6-(4-(5-Fluorobenzo[d]isoxazol-3-yl)piperidine-1-carbonyl)hexahydro-2H-pyrido[4,3-b][1,4]oxazin-3(4H)-one;
cis-6-(4-((4-Chlorophenyl)(phenyl)methyl)piperazine-1-carbonyl)hexahydro-2H-pyrido[4,3-b][1,4]oxazin-3(4H)-one;
(+)-cis-6-(4-(bis(4-Fluorophenyl)methyl)piperazine-1-carbonyl)hexahydro-2H-pyrido[4,3-b][1,4]oxazin-3(4H)-one;
rac-(4aR, 8aS)-6-[4-[bis(4-Fluorophenyl)methyl]piperazine-1-carbonyl]-4, 4a, 5,7, 8, 8a-hexahydropyrido[4,3-b][1,4]oxazin-3-one;
(-)-cis-6-(4-(bis(4-Fluorophenyl)methyl)piperazine-1-carbonyl)hexahydro-2H-pyrido[4,3-b][1,4]oxazin-3(4H)-one;
rac-(4aR,8aS)-6-(4-(5-Chloro-1H-indol-3-yl)piperazine-1-carbonyl)hexahydro-2H-pyrido[4,3-b][1,4]oxazin-3(4H)-one;
rac-(4aR,8aS)-6-(4-(1-Phenylethyl)piperazine-1-carbonyl)hexahydro-2H-pyrido[4,3-b][1,4]oxazin-3(4H)-one;
rac-(4aR,8aS)-6-(4-(1-Methyl-1H-indazol-5-yl)piperidine-1-carbonyl)hexahydro-2H-pyrido[4,3-b][1,4]oxazin-3(4H)-one;
rac-(4aR, 8aS)-6-[4-[(4-Fluorophenyl)-phenylmethyl]piperazine-1-carbonyl]-4,4a, 5,7, 8, 8a-hexahydropyrido[4,3-b][1,4]oxazin-3-one ;
(+)- or (-)-cis-6-(4-(5-Chloro-1-cyclopropyl-1H-indol-3-yl)piperidine-1-carbonyl)hexahydro-2H-pyrido[4,3-b][1,4]oxazin-3(4H)-one;
rac-(4aR,8aS)-6-(4-(5-Chloro-1-(1-chloro-3-hydroxypropan-2-yl)-1H-indol-3-yl)piperidine-1-carbonyl)hexahydro-2H-pyrido[4,3-b][1,4]oxazin-3(4H)-one;
(+)- or (-)-cis-6-(4-(bis(4-Fluorophenyl)methyl)piperidine-1-carbonyl)hexahydro-2H-pyrido[4,3-b][1,4]oxazin-3(4H)-one;
(+)-or (-)-cis-6-(4-(5-Chloro-1-(oxetan-3-yl)-1H-indol-3-yl)piperidine-1-carbonyl)hexahydro-2H-pyrido[4,3-b][1,4]oxazin-3(4H)-one;
(+)- or (-)-cis-6-(4-(5-Chloro-1-(oxetan-3-ylmethyl)-1H-indol-3-yl)piperidine-1-carbonyl)hexahydro-2H-pyrido[4,3-b][1,4]oxazin-3(4H)-one;
(+)- or (-)-cis-6-(4-(1-(2-((tert-Butyldimethylsilyl)oxy)ethyl)-5-chloro-1H-indol-3-yl)piperidine-1-carbonyl)hexahydro-2H-pyrido[4,3-b][1,4]oxazin-3(4H)-one;
(+)- or (-)-cis-6-(4-(5-Chloro-1-(2-hydroxyethyl)-1H-indol-3-yl)piperidine-1-carbonyl)hexahydro-2H-pyrido[4,3-b][1,4]oxazin-3(4H)-one;
rac-cis-6-(4-(1-(4-Fluorophenyl)-3-hydroxypropyl)piperidine-1-carbonyl)hexahydro-2H-pyrido[4,3-b][1,4]oxazin-3(4H)-one;
rac-(4aR,8aS)-6-(4-(1-(4-(Trifluoromethyl)phenyl)ethyl)piperidine-1-carbonyl)hexahydro-2H-pyrido[4,3-b][1,4]oxazin-3(4H)-one;
rac-(4aR,8aS)-6-(4-(1-(2-Chloro-4-fluorophenoxy)ethyl)piperidine-1-carbonyl)hexahydro-2H-pyrido[4,3-b][1,4]oxazin-3(4H)-one;
(4aR,8aS)-6-(4-(5-(Trifluoromethyl)pyridin-3-yl)piperidine-1-carbonyl)hexahydro-2H-pyrido[4,3-b][1,4]oxazin-3(4H)-one;
(4aR,8aS)-6-(4-(Difluoro(4-(trifluoromethyl)phenyl)methyl)piperidine-1-carbonyl)hexahydro-2H-pyrido[4,3-b][1,4]oxazin-3(4H)-one;
(+)-(4aR,8aS)-6-(4-((R or S)-1-(4-Fluorophenyl)-3-hydroxypropyl)piperidine-1-carbonyl)hexahydro-2H-pyrido[4,3-b][1,4]oxazin-3(4H)-one;
(-)-(4aR,8aS)--6-(4-((S or R)-1-(4-Fluorophenyl)-3-hydroxypropyl)piperidine-1-carbonyl)hexahydro-2H-pyrido[4,3-b][1,4]oxazin-3(4H)-one;
(+) or (-)-cis-6-(4-((S or R)-1-(2-Chloro-4-fluorophenoxy)ethyl)piperidine-1-carbonyl)hexahydro-2H-pyrido[4,3-b][1,4]oxazin-3(4H)-one;
(+) or (-)-cis-6-(4-((R or S)-1-(2-Chloro-4-fluorophenoxy)ethyl)piperidine-1-carbonyl)hexahydro-2H-pyrido[4,3-b][1,4]oxazin-3(4H)-one;
(+) or (-)-cis-6-(4-(5-Methoxypyridin-3-yl)piperidine-1-carbonyl)hexahydro-2H-pyrido[4,3-b][1,4]oxazin-3(4H)-one;
(+) or (-)-cis-6-(4-(Bis(4-fluorophenyl)methyl)piperidine-1-carbonyl)hexahydro-2H-pyrido[4,3-b][1,4]oxazin-3(4H)-one;
(+) or (-)-cis-6-(4-((4-Chlorophenyl)difluoromethyl)piperidine-1-carbonyl)hexahydro-2H-pyrido[4,3-b][1,4]oxazin-3(4H)-one;
(+) or (-)-cis-6-(4-(5-(Trifluoromethoxy)pyridin-2-yl)piperidine-1-carbonyl)hexahydro-2H-pyrido[4,3-b][1,4]oxazin-3(4H)-one;
(+) or (-)-cis-6-(4-((2-Chloro-4-fluorophenyl)difluoromethyl)piperidine-1-carbonyl)hexahydro-2H-pyrido[4,3-b][1,4]oxazin-3(4H)-one;
(+) or (-)-cis-6-(4-(5-Ethylpyridin-3-yl)piperidine-1-carbonyl)hexahydro-2H-pyrido[4,3-b][1,4]oxazin-3(4H)-one;
(+) or (-)-cis-6-(4-(5-(1,1-Difluoroethyl)pyridin-2-yl)piperidine-1-carbonyl)hexahydro-2H-pyrido[4,3-b][1,4]oxazin-3(4H)-one;
(+) or (-)-cis-6-(4-(6-Chloro-1-methyl-1H-indazol-3-yl)piperidine-1-carbonyl)hexahydro-2H-pyrido[4,3-b][1,4]oxazin-3(4H)-one;
(4aR,8aS)-6-((3R or 3 S)-4-(5-(1, 1-Difluoroethyl)pyridin-2-yl)-3-methylpiperidine-1-carbonyl)hexahydro-2H-pyrido[4,3-b][1,4]oxazin-3(4H)-one;
(4aR,8aS)-6-((3S or 3R)-4-(5-(1,1-Difluoroethyl)pyridin-2-yl)-3-methylpiperidine-1-carbonyl)hexahydro-2H-pyrido[4,3-b][1,4]oxazin-3(4H)-one;
(4aR,8aS)-6-((4R or 4S)-(5-(1,1-Difluoroethyl)pyridin-2-yl)-3-methylpiperidine-1-carbonyl)hexahydro-2H-pyrido[4,3-b][1,4]oxazin-3(4H)-one;
(4aR,8aS)-6-(4-((R or S)-1-(4-(Trifluoromethyl)phenyl)ethyl)piperidine-1-carbonyl)hexahydro-2H-pyrido[4,3-b][1,4]oxazin-3(4H)-one;
(4aR,8aS)-6-(4-((S or R)-1-(4-(Trifluoromethyl)phenyl)ethyl)piperidine-1-carbonyl)hexahydro-2H-pyrido[4,3-b][1,4]oxazin-3(4H)-one;
2-(4-Fluorophenyl)-2-(1-((4aR,8aS)-3-oxooctahydro-2H-pyrido[4,3-b][1,4]oxazine-6-carbonyl)piperidin-4-yl)acetonitrile;
(4aR,8aS)-6-(4-(2,2,2-Trifluoro-1-phenylethyl)piperazine-1-carbonyl)hexahydro-2H-pyrido[4,3-b][1,4]oxazin-3(4H)-one;
(4aR,8aS)-6-(4-((4-Fluorophenyl)(2,2,2-trifluoroethoxy)methyl)piperidine-1-carbonyl)hexahydro-2H-pyrido[4,3-b][1,4]oxazin-3(4H)-one;
(4aR,8aS)-6-(4-(1-(4-Fluorophenyl)-2-(3-methyl-1,2,4-oxadiazol-5-yl)ethyl)piperidine-1-carbonyl)hexahydro-2H-pyrido[4,3-b][1,4]oxazin-3(4H)-one;
(4aR,8aS)-6-(4-(1-(4-(Trifluoromethyl)phenyl)cyclopropyl)piperidine-1-carbonyl)hexahydro-2H-pyrido[4,3-b][1,4]oxazin-3(4H)-one;
(4aR,8aS)-6-(4-(1-(2,4-Difluorophenyl)-2,2,2-trifluoroethyl)piperazine-1-carbonyl)hexahydro-2H-pyrido[4,3-b][1,4]oxazin-3(4H)-one;
(4aR,8aS)-6-(3-(Bis(4-fluorophenyl)(hydroxy)methyl)azetidine-1-carbonyl)hexahydro-2H-pyrido[4,3-b][1,4]oxazin-3(4H)-one;
(4aR,8aS)-6-(3-(Fluorobis(4-fluorophenyl)methyl)azetidine-1-carbonyl)hexahydro-2H-pyrido[4,3-b][1,4]oxazin-3(4H)-one;
(4aR,8aS)-6-(4-(1-(4-Fluorophenyl)-2-(2,2,2-trifluoroethoxy)ethyl)piperidine-1-carbonyl)hexahydro-2H-pyrido[4,3-b][1,4]oxazin-3(4H)-one;
(4aR,8aS)-6-(4-((R or S)-(4-Fluorophenyl)(2,2,2-trifluoroethoxy)methyl)piperidine-1-carbonyl)hexahydro-2H-pyrido[4,3-b][1,4]oxazin-3(4H)-one;
(4aR,8aS)-6-(4-((S or R)-(4-Fluorophenyl)(2,2,2-trifluoroethoxy)methyl)piperidine-1-carbonyl)hexahydro-2H-pyrido[4,3-b][1,4]oxazin-3(4H)-one;
(4aR,8aS)-6-(4-((R or S)-1-(4-Fluorophenyl)-2-(3-methyl-1,2,4-oxadiazol-5-yl)ethyl)piperidine-1-carbonyl)hexahydro-2H-pyrido[4,3-b][1,4]oxazin-3(4H)-one;
(4aR,8aS)-6-(4-((S or R)-1-(4-Fluorophenyl)-2-(3-methyl-1,2,4-oxadiazol-5-yl)ethyl)piperidine-1-carbonyl)hexahydro-2H-pyrido[4,3-b][1,4]oxazin-3(4H)-one;
(4aR,8aS)-6-(4-(2-Cyclopropylpyridin-4-yl)piperidine-1-carbonyl)hexahydro-2H-pyrido[4,3-b][1,4]oxazin-3(4H)-one;
(4aR,8aS)-6-[4-[(S or R)-(4-Fluorophenyl)-(3-methoxyphenyl)methyl]piperidine-1-carbonyl]-4,4a,5,7,8,8a-hexahydropyrido[4,3-b][1,4]oxazin-3-one;
(4aR,8aS)-6-(4-((R or S)-(4-Fluorophenyl)(3-methoxyphenyl)methyl)piperidine-1-carbonyl)hexahydro-2H-pyrido[4,3-b][1,4]oxazin-3(4H)-one;
(4aR,8aS)-6-(4-((S or R)-(3-Methoxyphenyl)(phenyl)methyl)piperidine-1-carbonyl)hexahydro-2H-pyrido[4,3-b][1,4]oxazin-3(4H)-one;
(4aR,8aS)-6-(4-((R or S)-(3-Methoxyphenyl)(phenyl)methyl)piperidine-1-carbonyl)hexahydro-2H-pyrido[4,3-b][1,4]oxazin-3(4H)-one;
(4aR,8aS)-6-(4-((S or R)-(3-(2-Fluoroethoxy)phenyl)(phenyl)methyl)piperidine-1-carbonyl)hexahydro-2H-pyrido[4,3-b][1,4]oxazin-3(4H)-one;
(4aR,8aS)-6-(4-((S)-(4-Fluorophenyl)(phenyl)methyl)piperidine-1-carbonyl)hexahydro-2H-pyrido[4,3-b][1,4]oxazin-3(4H)-one;
(4aR,8aS)-6-(4-((S or R)-(4-Fluorophenyl)(phenyl)methyl)piperidine-1-carbonyl)hexahydro-2H-pyrido[4,3-b][1,4]oxazin-3(4H)-one;
(4aR,8aS)-6-[4-[(S or R)-(4-Fluorophenyl)-(4-methylphenyl)methyl]piperidine-1-carbonyl]-4,4a,5,7,8,8a-hexahydropyrido[4,3-b][1,4]oxazin-3-one;
(4aR,8aS)-6-[4-[(R or S)-[4-(2-Fluoroethoxy)phenyl]-phenylmethyl]piperidine-1-carbonyl]-4,4a,5,7,8,8a-hexahydropyrido[4,3-b][1,4]oxazin-3-one;
(4aR,8aS)-6-(4-((S or R)-(4-Fluorophenyl)(4-methoxyphenyl)methyl)piperidine-1-carbonyl)hexahydro-2H-pyrido[4,3-b][1,4]oxazin-3(4H)-one;
(4aR,8aS)-6-(4-((R or S)-(4-Fluorophenyl)(p-tolyl)methyl)piperidine-1-carbonyl)hexahydro-2H-pyrido[4,3-b][1,4]oxazin-3(4H)-one;
(4aR,8aS)-6-(4-((S or R)-(3,4-Dimethoxyphenyl)(4-fluorophenyl)methyl)piperidine-1-carbonyl)hexahydro-2H-pyrido[4,3-b][1,4]oxazin-3(4H)-one;
(4aR,8aS)-6-(4-((R or S)-(3,4-Dimethoxyphenyl)(phenyl)methyl)piperidine-1-carbonyl)hexahydro-2H-pyrido[4,3-b][1,4]oxazin-3(4H)-one;
(4aR,8aS)-6-(3-(1-(2-Chloro-4-(trifluoromethyl)phenoxy)ethyl)azetidine-1-carbonyl)hexahydro-2H-pyrido[4,3-b][1,4]oxazin-3(4H)-one;
(4aR,8aS)-6-(3-(1-(2-Chloro-4-(trifluoromethyl)phenoxy)ethyl)azetidine-1-carbonyl)hexahydro-2H-pyrido[4,3-b][1,4]oxazin-3(4H)-one;
(4aR,8aS)-6-(4-((R or S)-(4-Fluorophenyl)(phenyl)methyl)piperidine-1-carbonyl)hexahydro-2H-pyrido[4,3-b][1,4]oxazin-3(4H)-one;
(4aR,8aS)-6-(4-((S or R)-Phenyl(m-tolyl)methyl)piperidine-1-carbonyl)hexahydro-2H-pyrido[4,3-b][1,4]oxazin-3(4H)-one;
(4aR,8aS)-6-[4-[(S or R)-[4-(2-Fluoroethoxy)phenyl]-phenylmethyl]piperidine-1-carbonyl]-4,4a,5,7,8,8a-hexahydropyrido[4,3-b][1,4]oxazin-3-one;
(4aR,8aS)-6-(4-((S or R)-((S)-3-Methylcyclohexa-2,4-dien-1-yl)(phenyl)methyl)piperidine-1-carbonyl)hexahydro-2H-pyrido[4,3-b][1,4]oxazin-3(4H)-one;
(4aR,8aS)-6-(4-(1-(2-Chloro-4-fluorophenoxy)-2,2,2-trifluoroethyl)piperidine-1-carbonyl)hexahydro-2H-pyrido[4,3-b][1,4]oxazin-3(4H)-one;
(4aR,8aS)-6-(3-(bis(4-Fluorophenyl)methyl)azetidine-1-carbonyl)hexahydro-2H-pyrido[4,3-b][1,4]oxazin-3(4H)-one;
(4aR,8aS)-6-[3-[1-[4-(Trifluoromethyl)phenyl]ethyl]azetidine-1-carbonyl]-4,4a,5,7,8,8a-hexahydropyrido[4,3-b][1,4]oxazin-3-one;
(4aR,8aS)-6-[4-[(S or R)-Phenyl(p-tolyl)methyl]piperidine-1-carbonyl]-4,4a,5,7,8,8a-hexahydropyrido[4,3-b][1,4]oxazin-3-one;
(4aR,8aS)-6-(4-((R or S)-Phenyl(p-tolyl)methyl)piperidine-1-carbonyl)hexahydro-2H-pyrido[4,3-b][1,4]oxazin-3(4H)-one;
(4aR,8aS)-6-(3-Benzhydrylazetidine-1-carbonyl)hexahydro-2H-pyrido[4,3-b][1,4]oxazin-3 (4H)-one;
(4aR,8aS)-6-(4-((S or R)-(3,4-Dimethoxyphenyl)(phenyl)methyl)piperidine-1-carbonyl)hexahydro-2H-pyrido[4,3-b][1,4]oxazin-3(4H)-one;
(4aR,8aS)-6-(4-((R or S)-(3,4-Dimethoxyphenyl)(4-fluorophenyl)methyl)piperidine-1-carbonyl)hexahydro-2H-pyrido[4,3-b][1,4]oxazin-3(4H)-one;
(4aR,8aS)-6-(4-((R or S)-(4-Fluorophenyl)(4-methoxyphenyl)methyl)piperidine-1-carbonyl)hexahydro-2H-pyrido[4,3-b][1,4]oxazin-3(4H)-one;
(4aR,8aS)-6-[3-[1-[4-(Trifluoromethyl)phenyl]ethoxy]azetidine-1-carbonyl]-4,4a,5,7,8,8a-hexahydropyrido[4,3-b][1,4]oxazin-3-one;
(4aR,8aS)-6-(3-(1-(2-Fluoro-4-(trifluoromethyl)phenoxy)ethyl)azetidine-1-carbonyl)hexahydro-2H-pyrido[4,3-b][1,4]oxazin-3(4H)-one;
(4aR,8aS)-6-(3-(1-(4-(Trifluoromethyl)phenoxy)ethyl)azetidine-1-carbonyl)hexahydro-2H-pyrido[4,3-b][1,4]oxazin-3(4H)-one;
(4aR,8aS)-6-[4-[1-[4-(Trifluoromethyl)phenyl]ethoxy]piperidine-1-carbonyl]-4,4a,5,7,8,8a-hexahydropyrido[4,3-b][1,4]oxazin-3-one;
(4aR,8aS)-6-(4-((R or S)-(3-Cyclopropyl-1,2,4-oxadiazol-5-yl)(4-fluorophenyl)methyl)piperidine-1-carbonyl)hexahydro-2H-pyrido[4,3-b][1,4]oxazin-3(4H)-one;
(4aR,8aS)-6-(4-((S or R)-(3-Cyclopropyl-1,2,4-oxadiazol-5-yl)(4-fluorophenyl)methyl)piperidine-1-carbonyl)hexahydro-2H-pyrido[4,3-b][1,4]oxazin-3(4H)-one;
(4aR,8aS)-6-(3-((R or S)-1-(2-Fluoro-4-(trifluoromethyl)phenoxy)ethyl)azetidine-1-carbonyl)hexahydro-2H-pyrido[4,3-b][1,4]oxazin-3(4H)-one;
(4aR,8aS)-6-(3-((S or R)-1-(2-Fluoro-4-(trifluoromethyl)phenoxy)ethyl)azetidine-1-carbonyl)hexahydro-2H-pyrido[4,3-b][1,4]oxazin-3(4H)-one;
(4aR,8aS)-6-(3-((R or S)-1-(4-(Trifluoromethyl)phenoxy)ethyl)azetidine-1-carbonyl)hexahydro-2H-pyrido[4,3-b][1,4]oxazin-3(4H)-one;
(4aR,8aS)-6-(3-((S or R)-1-(4-(Trifluoromethyl)phenoxy)ethyl)azetidine-1-carbonyl)hexahydro-2H-pyrido[4,3-b][1,4]oxazin-3(4H)-one;
(4aR,8aS)-6-(4-((R or S)-(4-(2-Fluoroethoxy)phenyl)(4-fluorophenyl)methyl)piperidine-1-carbonyl)hexahydro-2H-pyrido[4,3-b][1,4]oxazin-3(4H)-one;
(4aR,8aS)-6-(4-(5-Methyl-6-(trifluoromethyl)pyridin-3-yl)piperidine-1-carbonyl)hexahydro-2H-pyrido[4,3-b][1,4]oxazin-3(4H)-one;
(4aR,8aS)-6-(4-((4-Fluorophenyl)(3-(trifluoromethyl)-1,2,4-oxadiazol-5-yl)methyl)piperidine-1-carbonyl)hexahydro-2H-pyrido[4,3-b][1,4]oxazin-3(4H)-one;
(4aR,8aS)-6-(3-((S or R)-1-(4-(Trifluoromethyl)phenyl)ethyl)azetidine-1-carbonyl)hexahydro-2H-pyrido[4,3-b][1,4]oxazin-3(4H)-one;
(4aR,8aS)-6-(3-((R or S)-1-(4-(Trifluoromethyl)phenyl)ethyl)azetidine-1-carbonyl)hexahydro-2H-pyrido[4,3-b][1,4]oxazin-3(4H)-one;
(4aR,8aS)-6-[3-(S or R)-[(4-Fluorophenyl)-(2,2,2-trifluoroethoxy)methyl]azetidine-1-carbonyl]-4,4a,5,7,8,8a-hexahydropyrido[4,3-b][1,4]oxazin-3-one;
(4aR,8aS)-6-[3-(R or S)-[(4-Fluorophenyl)-(2,2,2-trifluoroethoxy)methyl]azetidine-1-carbonyl]-4,4a,5,7,8,8a-hexahydropyrido[4,3-b][1,4]oxazin-3-one;
(4aR,8aS)-6-(4-(5,6,7,8-Tetrahydroquinolin-4-yl)piperidine-1-carbonyl)hexahydro-2H-pyrido[4,3-b][1,4]oxazin-3(4H)-one;
(4aR,8aS)-6-(4-((R or S)-(3-(2-Fluoroethoxy)phenyl)(phenyl)methyl)piperidine-1-carbonyl)hexahydro-2H-pyrido[4,3-b][1,4]oxazin-3(4H)-one;
(4aR,8aS)-6-(4-((S or R)-(4-(2-Fluoroethoxy)phenyl)(4-fluorophenyl)methyl)piperidine-1-carbonyl)hexahydro-2H-pyrido[4,3-b][1,4]oxazin-3(4H)-one;
(4aR,8aS)-6-(3-(4-Bromophenyl)azetidine-1-carbonyl)hexahydro-2H-pyrido[4,3-b][1,4]oxazin-3(4H)-one;
(4aR,8aS)-6-(3-(4'-Chloro-[1,1'-biphenyl]-4-yl)azetidine-1-carbonyl)hexahydro-2H-pyrido[4,3-b][1,4]oxazin-3(4H)-one;
(4aR,8aS)-6-(3-(2'-Chloro-[1,1'-biphenyl]-4-yl)azetidine-1-carbonyl)hexahydro-2H-pyrido[4,3-b][1,4]oxazin-3(4H)-one;
(4aR,8aS)-6-(3-(2',4'-Dichloro-[1,1'-biphenyl]-4-yl)azetidine-1-carbonyl)hexahydro-2H-pyrido[4,3-b][1,4]oxazin-3(4H)-one;
(4aR,8aS)-6-(3-(1-(R or S)-(4-(T rifluoromethyl)phenyl)ethoxy)azetidine-1-carbonyl)hexahydro-2H-pyrido[4,3-b][1,4]oxazin-3(4H)-one;
(4aR,8aS)-6-(3-(1-(S or R)-(4-(Trifluoromethyl)phenyl)ethoxy)azetidine-1-carbonyl)hexahydro-2H-pyrido[4,3-b][1,4]oxazin-3(4H)-one;
(4aR,8aS)-6-[3-(S or R)-[1-(2-Chloro-4-fluoro-phenyl)ethoxy]azetidine-1-carbonyl]-4,4a,5,7,8,8a-hexahydropyrido[4,3-b][1,4]oxazin-3-one;
(4aR,8aS)-6-[3-(R or S)-[1-(2-Chloro-4-fluoro-phenyl)ethoxy]azetidine-1-carbonyl]-4,4a,5,7,8,8a-hexahydropyrido[4,3-b][1,4]oxazin-3-one;
(4aR,8aS)-6-[4-[(R or S)-(3,4-Dimethoxyphenyl)-(2-pyridyl)methyl]piperidine-1-carbonyl]-4,4a,5,7,8,8a-hexahydropyrido[4,3-b][1,4]oxazin-3-one;
(4aR,8aS)-6-[4-[(S or R)-(3,4-Dimethoxyphenyl)-(2-pyridyl)methyl]piperidine-1-carbonyl]-4,4a,5,7,8,8a-hexahydropyrido[4,3-b][1,4]oxazin-3-one;
(4aR,8aS)-6-[3-[4-(Trifluoromethoxy)phenyl]azetidine-1-carbonyl]-4,4a,5,7,8,8a-hexahydropyrido[4,3-b][1,4]oxazin-3-one;
(4aR,8aS)-6-(3-(2'-(Trifluoromethoxy)-[1,1'-biphenyl]-4-yl)azetidine-1-carbonyl)hexahydro-2H-pyrido[4,3-b][1,4]oxazin-3(4H)-one;
(4aR,8aS)-6-(3-(4-Bromophenyl)-3-fluoroazetidine-1-carbonyl)hexahydro-2H-pyrido[4,3-b][1,4]oxazin-3(4H)-one;
(4aR,8aS)-6-(3-(4-Bromophenyl)-3-hydroxyazetidine-1-carbonyl)hexahydro-2H-pyrido[4,3-b][1,4]oxazin-3(4H)-one;
(4aR,8aS)-6-(3-(4-Bromophenyl)-3-methylazetidine-1-carbonyl)hexahydro-2H-pyrido[4,3-b][1,4]oxazin-3(4H)-one;
(4aR,8aS)-6-(3-(2'-(Trifluoromethyl)-[1,1'-biphenyl]-4-yl)azetidine-1-carbonyl)hexahydro-2H-pyrido[4,3-b][1,4]oxazin-3(4H)-one;
(4aR,8aS)-6-(3-(2',4'-Difluoro-[1,1'-biphenyl]-4-yl)azetidine-1-carbonyl)hexahydro-2H-pyrido[4,3-b][1,4]oxazin-3(4H)-one;
4'-(1-((4aR,8aS)-3-Oxooctahydro-2H-pyrido[4,3-b][1,4]oxazine-6-carbonyl)azetidin-3-yl)-[1,1'-biphenyl]-2-carbonitrile;
(4aR,8aS)-6-(3-(4-(3-Methoxyazetidin-1-yl)phenyl)azetidine-1-carbonyl)hexahydro-2H-pyrido[4,3-b][1,4]oxazin-3(4H)-one;
(4aR,8aS)-6-(3-(4-(3-(Trifluoromethyl)azetidin-1-yl)phenyl)azetidine-1-carbonyl)hexahydro-2H-pyrido[4,3-b][1,4]oxazin-3(4H)-one;
2-[4-[1-[(4aR,8aS)-3-Oxo-4,4a,5,7,8,8a-hexahydropyrido[4,3-b][1,4]oxazine-6-carbonyl]azetidin-3-yl]phenyl]benzamide;
(4aR,8aS)-6-(3-(2-Chloro-[1,1'-biphenyl]-4-yl)azetidine-1-carbonyl)hexahydro-2H-pyrido[4,3-b][1,4]oxazin-3(4H)-one;
(4aR,8aS)-6-(3-(4-Bromo-3-chlorophenyl)azetidine-1-carbonyl)hexahydro-2H-pyrido[4,3-b][1,4]oxazin-3(4H)-one;
(4aR,8aS)-6-[3-[4-(4-Chloro-2-fluoro-phenyl)phenyl]azetidine-1-carbonyl]-4,4a,5,7,8,8a-hexahydropyrido[4,3-b][1,4]oxazin-3-one;
(4aR,8aS)-6-[3-[4-(2-Chloro-4-fluoro-phenyl)phenyl]azetidine-1-carbonyl]-4,4a,5,7,8,8a-hexahydropyrido[4,3-b][1,4]oxazin-3-one;
(4aR,8aS)-6-[4-[(R or S)-(3,4-Dimethoxyphenyl)-(3-pyridyl)methyl]piperidine-1-carbonyl]-4,4a,5,7,8,8a-hexahydropyrido[4,3-b][1,4]oxazin-3-one;
(4aR,8aS)-6-[4-[(S or R)-(3,4-dimethoxyphenyl)-(3-pyridyl)methyl]piperidine-1-carbonyl]-4,4a,5,7,8,8a-hexahydropyrido[4,3-b][1,4]oxazin-3-one;
(4aR,8aS)-6-(3-(2'-Methyl-[1,1'-biphenyl]-4-yl)azetidine-1-carbonyl)hexahydro-2H-pyrido[4,3-b][1,4]oxazin-3(4H)-one;
(4aR,8aS)-6-(3-(4-(3-((1,1,1-Trifluoropropan-2-yl)oxy)azetidin-1-yl)phenyl)azetidine-1-carbonyl)hexahydro-2H-pyrido[4,3-b][1,4]oxazin-3(4H)-one;
(4aR,8aS)-6-(3-(3-Bromophenyl)azetidine-1-carbonyl)hexahydro-2H-pyrido[4,3-b][1,4]oxazin-3(4H)-one;
(4aR,8aS)-6-(3-(4-(tert-Butyl)phenyl)azetidine-1-carbonyl)hexahydro-2H-pyrido[4,3-b][1,4]oxazin-3(4H)-one;
(4aR,8aS)-6-[3-(4-Phenylphenyl)azetidine-1-carbonyl]-4,4a,5,7,8,8a-hexahydropyrido[4,3-b][1,4]oxazin-3-one;
(4aR,8aS)-6-[3-[4-[2-(Difluoromethyl)phenyl]phenyl]azetidine-1-carbonyl]-4,4a,5,7,8,8a-hexahydropyrido[4,3-b][1,4]oxazin-3-one;
(4aR,8aS)-6-(3-(3'-Methyl-[1,1'-biphenyl]-4-yl)azetidine-1-carbonyl)hexahydro-2H-pyrido[4,3-b][1,4]oxazin-3(4H)-one;
(4aR,8aS)-6-(3-(4'-Methyl-[1,1'-biphenyl]-4-yl)azetidine-1-carbonyl)hexahydro-2H-pyrido[4,3-b][1,4]oxazin-3(4H)-one;
(4aR,8aS)-6-(3-(6-Chloropyridin-3-yl)azetidine-1-carbonyl)hexahydro-2H-pyrido[4,3-b][1,4]oxazin-3(4H)-one;
(4aR,8aS)-6-(3-(4-(Trifluoromethyl)phenyl)azetidine-1-carbonyl)hexahydro-2H-pyrido[4,3-b][1,4]oxazin-3(4H)-one;
(4aR,8aS)-6-(3-(4-(3-((1,1,1-Trifluoro-2-methylpropan-2-yl)oxy)azetidin-1-yl)phenyl)azetidine-1-carbonyl)hexahydro-2H-pyrido[4,3-b][1,4]oxazin-3(4H)-one;
(4aR,8aS)-6-(3-(4-(1,1-Difluoroethyl)phenyl)azetidine-1-carbonyl)hexahydro-2H-pyrido[4,3-b][1,4]oxazin-3(4H)-one;
(4aR,8aS)-6-[4-[(R or S)-(3,4-Dimethoxyphenyl)-(4-pyridyl)methyl]piperidine-1-carbonyl]-4,4a,5,7,8,8a-hexahydropyrido[4,3-b][1,4]oxazin-3-one;
(4aR,8aS)-6-[4-[(S or R)-(3,4-Dimethoxyphenyl)-(4-pyridyl)methyl]piperidine-1-carbonyl]-4,4a,5,7,8,8a-hexahydropyrido[4,3-b][1,4]oxazin-3-one;
(4aR,8aS)-6-(3-(4-(3-Methylazetidin-1-yl)phenyl)azetidine-1-carbonyl)hexahydro-2H-pyrido[4,3-b][1,4]oxazin-3(4H)-one;
(4aR,8aS)-6-(3-(4-(3,3-Dimethylpyrrolidin-1-yl)phenyl)azetidine-1-carbonyl)hexahydro-2H-pyrido[4,3-b][1,4]oxazin-3(4H)-one;
(4aR,8aS)-6-(3-(4-(3-(Trifluoromethoxy)azetidin-1-yl)phenyl)azetidine-1-carbonyl)hexahydro-2H-pyrido[4,3-b][1,4]oxazin-3(4H)-one;
(4aR,8aS)-6-(3-(6-(2,4-Dichlorophenyl)pyridin-3-yl)azetidine-1-carbonyl)hexahydro-2H-pyrido[4,3-b][1,4]oxazin-3(4H)-one;
(4aR,8aS)-6-(3-(2'-Chloro-4'-(methylsulfonyl)-[1,1'-biphenyl]-4-yl)azetidine-1-carbonyl)hexahydro-2H-pyrido[4,3-b][1,4]oxazin-3(4H)-one;
(4aR,8aS)-6-(3-(4-(3,3-Difluoroazetidin-1-yl)phenyl)azetidine-1-carbonyl)hexahydro-2H-pyrido[4,3-b][1,4]oxazin-3(4H)-one;
(4aR,8aS)-6-(4-(4-Bromophenyl)piperidine-1-carbonyl)hexahydro-2H-pyrido[4,3-b][1,4]oxazin-3(4H)-one;
(4aR,8aS)-6-[3-[4-(2,2,2-Trifluoroethoxy)phenyl]azetidine-1-carbonyl]-4,4a,5,7,8,8a-hexahydropyrido[4,3-b][1,4]oxazin-3-one;
2-[4-[1-[(4aR,8aS)-3-Oxo-4,4a,5,7,8,8a-hexahydropyrido[4,3-b][1,4]oxazine-6-carbonyl]azetidin-3-yl]phenyl]-5-chloro-benzonitrile;
(4aR,8aS)-6-[3-[4-[3-[(1S or 1R)-2,2,2-Trifluoro-1-methyl-ethoxy]azetidin-1-yl]phenyl]azetidine-1-carbonyl]-4,4a,5,7,8,8a-hexahydropyrido[4,3-b][1,4]oxazin-3-one;
(4aR,8aS)-6-[3-[4-[3-[(1R or 1S)-2,2,2-Trifluoro-1-methyl-ethoxy]azetidin-1-yl]phenyl]azetidine-1-carbonyl]-4,4a,5,7,8,8a-hexahydropyrido[4,3-b][1,4]oxazin-3-one;
(4aR,8aS)-6-(3-(4-(2-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-(3-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(3-Bromophenyl)pyrrolidine-1-carbonyl)hexahydro-2H-pyrido[4,3-b][1,4]oxazin-3(4H)-one;
(4aR,8aS)-6-[3-[4-(2-Ethylpyrrolidin-1-yl)phenyl]azetidine-1-carbonyl]-4,4a,5,7,8,8a-hexahydropyrido[4,3-b][1,4]oxazin-3-one;
2-[4-[1-[(4aR,8aS)-3-Oxo-4,4a,5,7,8,8a-hexahydropyrido[4,3-b][1,4]oxazine-6-carbonyl]azetidin-3-yl]phenyl]-3-chloro-benzonitrile;
(4aR,8aS)-6-(3-(4-(2-Cyclopropylpyrrolidin-1-yl)phenyl)azetidine-1-carbonyl)hexahydro-2H-pyrido[4,3-b][1,4]oxazin-3(4H)-one;
(4aR,8aS)-6-(4-(4-Bromophenyl)piperazine-1-carbonyl)hexahydro-2H-pyrido[4,3-b][1,4]oxazin-3(4H)-one;
(4aR,8aS)-6-(4-(2',4'-Dichloro-[1,1'-biphenyl]-4-yl)piperidine-1-carbonyl)hexahydro-2H-pyrido[4,3-b][1,4]oxazin-3(4H)-one;
(4aR,8aS)-6-(3-(4-(3-Azabicyclo[3.1.0]hexan-3-yl)phenyl)azetidine-1-carbonyl)hexahydro-2H-pyrido[4,3-b][1,4]oxazin-3(4H)-one;
(4aR,8aS)-6-(3-(3-(Trifluoromethoxy)phenyl)azetidine-1-carbonyl)hexahydro-2H-pyrido[4,3-b][1,4]oxazin-3(4H)-one;
(4aR,8aS)-6-(3-(4-(3-(2,2,2-Trifluoroethoxy)azetidin-1-yl)phenyl)azetidine-1-carbonyl)hexahydro-2H-pyrido[4,3-b][1,4]oxazin-3(4H)-one;
(4aR,8aS)-6-(3-(4'-chloro-2'-(methylsulfonyl)-[1,1'-biphenyl]-4-yl)azetidine-1-carbonyl)hexahydro-2H-pyrido[4,3-b][1,4]oxazin-3(4H)-one;
(4aR,8aS)-6-(3-(6-(2-(Trifluoromethyl)pyrrolidin-1-yl)pyridin-3-yl)azetidine-1-carbonyl)hexahydro-2H-pyrido[4,3-b][1,4]oxazin-3(4H)-one;
(4aR,8aS)-6-[4-[(R or S)-(3-methylsulfonylphenyl)-(3-pyridyl)methyl]piperidine-1-carbonyl]-4,4a,5,7,8,8a-hexahydropyrido[4,3-b][1,4]oxazin-3-one;
(4aR,8aS)-6-[4-[(S or R)-(3-methylsulfonylphenyl)-(3-pyridyl)methyl]piperidine-1-carbonyl]-4,4a,5,7,8,8a-hexahydropyrido[4,3-b][1,4]oxazin-3-one;
(4aR,8aS)-6-[4-[(R or S)-(3-methylsulfonylphenyl)-(4-pyridyl)methyl]piperidine-1-carbonyl]-4,4a,5,7,8,8a-hexahydropyrido[4,3-b][1,4]oxazin-3-one;
(4aR,8aS)-6-[4-[(S or R)-(3-methylsulfonylphenyl)-(4-pyridyl)methyl]piperidine-1-carbonyl]-4,4a,5,7,8,8a-hexahydropyrido[4,3-b][1,4]oxazin-3-one;
(4aR,8aS)-6-(3-(4-(5-Azaspiro[2.4]heptan-5-yl)phenyl)azetidine-1-carbonyl)hexahydro-2H-pyrido[4,3-b][1,4]oxazin-3(4H)-one;
(4aR,8aS)-6-(3-(4-(Pentafluoro-l6-sulfaneyl)phenyl)azetidine-1-carbonyl)hexahydro-2H-pyrido[4,3-b][1,4]oxazin-3(4H)-one;
(4aR,8aS)-6-(3-(5-Chloropyridin-2-yl)azetidine-1-carbonyl)hexahydro-2H-pyrido[4,3-b][1,4]oxazin-3(4H)-one;
(4aR,8aS)-6-(3-(2-Fluoro-4-(trifluoromethoxy)phenyl)azetidine-1-carbonyl)hexahydro-2H-pyrido[4,3-b][1,4]oxazin-3(4H)-one;
(4aR,8aS)-6-[3-(6-methoxypyridin-3-yl)azetidine-1-carbonyl]-4,4a,5,7,8,8a-hexahydropyrido[4,3-b][1,4]oxazin-3-one;
(4aR,8aS)-6-(3-(4-Bromophenyl)pyrrolidine-1-carbonyl)hexahydro-2H-pyrido[4,3-b][1,4]oxazin-3(4H)-one;
(4aR,8aS)-6-(3-Phenylazetidine-1-carbonyl)hexahydro-2H-pyrido[4,3-b][1,4]oxazin-3(4H)-one;
(4aR,8aS)-6-(4-Phenylpiperidine-1-carbonyl)hexahydro-2H-pyrido[4,3-b][1,4]oxazin-3(4H)-one;
(4aR,8aS)-6-[3-[4-(2,2,2-Trifluoroethyl)phenyl]azetidine-1-carbonyl]-4,4a,5,7,8,8a-hexahydropyrido[4,3-b][1,4]oxazin-3-one;
(4aR,8aS)-6-[3-[4-[1-(Trifluoromethyl)cyclopropyl]phenyl]azetidine-1-carbonyl]-4,4a,5,7,8,8a-hexahydropyrido[4,3-b][1,4]oxazin-3-one;
(4aR,8aS)-6-[4-[(R or S)-(3-Methylsulfonylphenyl)-(2-pyridyl)methyl]piperidine-1-carbonyl]-4,4a,5,7,8,8a-hexahydropyrido[4,3-b][1,4]oxazin-3-one;
(4aR,8aS)-6-[4-[(S or R)-(3-Methylsulfonylphenyl)-(2-pyridyl)methyl]piperidine-1-carbonyl]-4,4a,5,7,8,8a-hexahydropyrido[4,3-b][1,4]oxazin-3-one;
(4aR,8aS)-6-[3-[4-(6,6-Difluoro-2-azaspiro[3.3]heptan-2-yl)phenyl]azetidine-1-carbonyl]-4,4a,5,7,8,8a-hexahydropyrido[4,3-b][1,4]oxazin-3-one;
(4aR,8aS)-6-(3-(4-(8-Oxa-3-azabicyclo[3.2.1]octan-3-yl)phenyl)azetidine-1-carbonyl)hexahydro-2H-pyrido[4,3-b][1,4]oxazin-3(4H)-one;
(4aR,8aS)-6-(3-(5-(2,4-Dichlorophenyl)pyridin-2-yl)azetidine-1-carbonyl)hexahydro-2H-pyrido[4,3-b][1,4]oxazin-3(4H)-one;
(4aR,8aS)-6-(3-(4-((R or S)-2-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-((S or R)-2-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-[3-[4-(1-Piperidyl)phenyl]azetidine-1-carbonyl]-4,4a,5,7,8,8a-hexahydropyrido[4,3-b][1,4]oxazin-3-one;
(4aR,8aS)-6-(3-(4-((R or S)-3-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-((S or R)-3-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-[3-[4-(3-hydroxy-3-methylazetidin-1-yl)phenyl]azetidine-1-carbonyl]-4,4a,5,7,8,8a-hexahydropyrido[4,3-b][1,4]oxazin-3-one;
(4aR,8aS)-6-[3-[4-(3-Fluoro-3-methyl-azetidin-1-yl)phenyl]azetidine-1-carbonyl]-4,4a,5,7,8,8a-hexahydropyrido[4,3-b][1,4]oxazin-3-one;
(4aR,8aS)-6-(3-(4-(3-Fluoroazetidin-1-yl)phenyl)azetidine-1-carbonyl)hexahydro-2H-pyrido[4,3-b][1,4]oxazin-3(4H)-one;
(4aR,8aS)-6-(3-(3-Fluoro-4-(trifluoromethoxy)phenyl)azetidine-1-carbonyl)hexahydro-2H-pyrido[4,3-b][1,4]oxazin-3(4H)-one;
(4aR,8aS)-6-(3-(3-Methyl-4-(trifluoromethoxy)phenyl)azetidine-1-carbonyl)hexahydro-2H-pyrido[4,3-b][1,4]oxazin-3(4H)-one;
(4aR,8aS)-6-(3-(3,5-Difluoro-4-(trifluoromethoxy)phenyl)azetidine-1-carbonyl)hexahydro-2H-pyrido[4,3-b][1,4]oxazin-3(4H)-one;
(4aR,8aS)-6-(3-(2-Chloro-4-(trifluoromethoxy)phenyl)azetidine-1-carbonyl)hexahydro-2H-pyrido[4,3-b][1,4]oxazin-3(4H)-one;
(4aR,8aS)-6-(3-(4-(Bicyclo[1.1.1]pentan-1-yl)phenyl)azetidine-1-carbonyl)hexahydro-2H-pyrido[4,3-b][1,4]oxazin-3(4H)-one;
(4aR,8aS)-6-(3-(5-(2-(Trifluoromethyl)pyrrolidin-1-yl)pyridin-2-yl)azetidine-1-carbonyl)hexahydro-2H-pyrido[4,3-b][1,4]oxazin-3(4H)-one;
(4aR,8aS)-6-(3-(5-Fluoro-1H-indol-3-yl)pyrrolidine-1-carbonyl)hexahydro-2H-pyrido[4,3-b][1,4]oxazin-3(4H)-one;
(4aR,8aS)-6-(3-(1-Benzyl-5-chloro-1H-indol-3-yl)pyrrolidine-1-carbonyl)hexahydro-2H-pyrido[4,3-b][1,4]oxazin-3(4H)-one;
(4aR,8aS)-6-(3-(2-Fluoro-4-(trifluoromethyl)phenyl)azetidine-1-carbonyl)hexahydro-2H-pyrido[4,3-b][1,4]oxazin-3(4H)-one;
(4aR,8aS)-6-(3-(3-Chloro-4-(trifluoromethoxy)phenyl)azetidine-1-carbonyl)hexahydro-2H-pyrido[4,3-b][1,4]oxazin-3(4H)-one;
(4aR,8aS)-6-[3-[4-[3-(Cyclopropylmethoxy)azetidin-1-yl]phenyl]azetidine-1-carbonyl]-4,4a,5,7,8,8a-hexahydropyrido[4,3-b][1,4]oxazin-3-one;
5-(1-((4aR,8aS)-3-Oxooctahydro-2H-pyrido[4,3-b][1,4]oxazine-6-carbonyl)azetidin-3-yl)-2-(trifluoromethoxy)benzonitrile;
(4aR,8aS)-6-(3-(4-(1-(Hydroxymethyl)cyclopropyl)phenyl)azetidine-1-carbonyl)hexahydro-2H-pyrido[4,3-b][1,4]oxazin-3(4H)-one;
(4aR,8aS)-6-(3-(1-Methyl-1H-indazol-4-yl)azetidine-1-carbonyl)hexahydro-2H-pyrido[4,3-b][1,4]oxazin-3(4H)-one;
(4aR,8aS)-6-(3-(4-(3-Fluoropyrrolidin-1-yl)phenyl)azetidine-1-carbonyl)hexahydro-2H-pyrido[4,3-b][1,4]oxazin-3(4H)-one;
(4aR,8aS)-6-(3-(4-(2-Methylazetidin-1-yl)phenyl)azetidine-1-carbonyl)hexahydro-2H-pyrido[4,3-b][1,4]oxazin-3(4H)-one;
(4aR,8aS)-6-[4-[phenyl(pyridazin-3-yl)methyl]piperidine-1-carbonyl]-4,4a,5,7,8,8a-hexahydropyrido[4,3-b][1,4]oxazin-3-one;
(4aR,8aS)-6-[3-[4-(Trifluoromethoxy)phenyl]pyrrolidine-1-carbonyl]-4,4a,5,7,8,8a-hexahydropyrido[4,3-b][1,4]oxazin-3-one;
(4aR,8aS)-6-(3-(1-Methyl-1H-indazol-6-yl)azetidine-1-carbonyl)hexahydro-2H-pyrido[4,3-b][1,4]oxazin-3(4H)-one;
(4aR,8aS)-6-[3-(S or R)-[3-(Trifluoromethoxy)phenyl]pyrrolidine-1-carbonyl]-4,4a,5,7,8,8a-hexahydropyrido[4,3-b][1,4]oxazin-3-one;
(4aR,8aS)-6-[3-(R or S)-[3-(Trifluoromethoxy)phenyl]pyrrolidine-1-carbonyl]-4,4a,5,7,8,8a-hexahydropyrido[4,3-b][1,4]oxazin-3-one;
(4aR,8aS)-6-(3-(3-Chloro-4-hydroxyphenyl)azetidine-1-carbonyl)hexahydro-2H-pyrido[4,3-b][1,4]oxazin-3(4H)-one;
(4aR,8aS)-6-(3-(3-Chloro-4-(3-methylazetidin-1-yl)phenyl)azetidine-1-carbonyl)hexahydro-2H-pyrido[4,3-b][1,4]oxazin-3(4H)-one;
(4aR,8aS)-6-[3-[4-(Oxetan-3-yl)phenyl]azetidine-1-carbonyl]-4,4a,5,7,8,8a-hexahydropyrido[4,3-b][1,4]oxazin-3-one;
(4aR,8aS)-6-(3-(3-Chloro-4-(3,3-difluoroazetidin-1-yl)phenyl)azetidine-1-carbonyl)hexahydro-2H-pyrido[4,3-b][1,4]oxazin-3(4H)-one;
(4aR,8aS)-6-(3-(3-(3-Methylazetidin-1-yl)-4-(trifluoromethoxy)phenyl)azetidine-1-carbonyl)hexahydro-2H-pyrido[4,3-b][1,4]oxazin-3(4H)-one;
(4aR,8aS)-6-[(3SorR)-3-(3-Bromophenyl)pyrrolidine-1-carbonyl]-4,4a,5,7,8,8a-hexahydropyrido[4,3-b][1,4]oxazin-3-one;
(4aR,8aS)-6-[(3R or S)-3-(3-Bromophenyl)pyrrolidine-1-carbonyl]-4,4a,5,7,8,8a-hexahydropyrido[4,3-b][1,4]oxazin-3-one;
(4aR,8aS)-6-[3-[4-(3-Azabicyclo[3.1.1]heptan-3-yl)phenyl]azetidine-1-carbonyl]-4,4a,5,7,8,8a-hexahydropyrido[4,3-b][1,4]oxazin-3-one;
(4aR,8aS)-6-(2-Methyl-3-(4-(trifluoromethoxy)phenyl)azetidine-1-carbonyl)hexahydro-2H-pyrido[4,3-b][1,4]oxazin-3(4H)-one;
(4aR,8aS)-6-(3-(3,3-Dimethyl-2,3-dihydrobenzofuran-6-yl)azetidine-1-carbonyl)hexahydro-2H-pyrido[4,3-b][1,4]oxazin-3(4H)-one;
(4aR,8aS)-6-(3-(3-Chloro-5-(2,2,2-trifluoroethoxy)phenyl)pyrrolidine-1-carbonyl)hexahydro-2H-pyrido[4,3-b][1,4]oxazin-3(4H)-one;
Methyl 2-methyl-2-(4-(1-((4aR,8aS)-3-Oxooctahydro-2H-pyrido[4,3-b][1,4]oxazine-6-carbonyl)azetidin-3-yl)phenyl)propanoate;
(4aR,8aS)-6-(3-(3,5-Dichlorophenyl)pyrrolidine-1-carbonyl)hexahydro-2H-pyrido[4,3-b][1,4]oxazin-3(4H)-one;
(4aR,8aS)-6-(3-(4-(3-Methoxy-3-methylazetidin-1-yl)phenyl)azetidine-1-carbonyl)hexahydro-2H-pyrido[4,3-b][1,4]oxazin-3(4H)-one;
tert-Butyl (2-(3-((1-((4aR,8aS)-3-oxooctahydro-2H-pyrido[4,3-b][1,4]oxazine-6-carbonyl)piperidin-4-yl)(phenyl)methyl)phenoxy)ethyl)carbamate;
(4aR,8aS)-6-((R or S)-3-(3-Chloro-5-(2,2,2-trifluoroethoxy)phenyl)pyrrolidine-1-carbonyl)hexahydro-2H-pyrido[4,3-b][1,4]oxazin-3(4H)-one;
(4aR,8aS)-6-((S or R)-3-(3-Chloro-5-(2,2,2-trifluoroethoxy)phenyl)pyrrolidine-1-carbonyl)hexahydro-2H-pyrido[4,3-b][1,4]oxazin-3(4H)-one;
(4aR,8aS)-6-(3-(4-(tert-Butyl)-3-methoxyphenyl)azetidine-1-carbonyl)hexahydro-2H-pyrido[4,3-b][1,4]oxazin-3(4H)-one;
(4aR,8aS)-6-(3-(1-Methyl-1H-indazol-5-yl)azetidine-1-carbonyl)hexahydro-2H-pyrido[4,3-b][1,4]oxazin-3(4H)-one;
(4aR,8aS)-6-(3-(4-Propylphenyl)azetidine-1-carbonyl)hexahydro-2H-pyrido[4,3-b][1,4]oxazin-3(4H)-one;
(4aR,8aS)-6-(3-(4-(Trifluoromethoxy)-3-(trifluoromethyl)phenyl)azetidine-1-carbonyl)hexahydro-2H-pyrido[4,3-b][1,4]oxazin-3(4H)-one;
(4aR,8aS)-6-[3-[4-[[1-(Trifluoromethyl)cyclopropyl]methoxy]phenyl]azetidine-1-carbonyl]-4,4a,5,7,8,8a-hexahydropyrido[4,3-b][1,4]oxazin-3-one;
(4aR,8aS)-6-[3-[4-(2,2,2-Trifluoro-1,1-dimethyl-ethyl)phenyl]azetidine-1-carbonyl]-4,4a,5,7,8,8a-hexahydropyrido[4,3-b][1,4]oxazin-3-one;
(4aR,8aS)-6-[3-(R or S)-[4-(2,2,2-Trifluoro-1-methyl-ethoxy)phenyl]azetidine-1-carbonyl]-4,4a,5,7,8,8a-hexahydropyrido[4,3-b][1,4]oxazin-3-one;
(4aR,8aS)-6-[3-[4-(3-Fluoropropyl)phenyl]azetidine-1-carbonyl]-4,4a,5,7,8,8a-hexahydropyrido[4,3-b][1,4]oxazin-3-one;
(4aR,8aS)-6-[3-(SorR)-[4-(2,2,2-Trifluoro-1-methyl-ethoxy)phenyl]azetidine-1-carbonyl]-4,4a,5,7,8,8a-hexahydropyrido[4,3-b][1,4]oxazin-3-one;
(4aR,8aS)-6-[3-(4-Cyclobutylphenyl)azetidine-1-carbonyl]-4,4a,5,7,8,8a-hexahydropyrido[4,3-b][1,4]oxazin-3-one;
(4aR,8aS)-6-[3-(3-Methoxy-4-methyl-phenyl)azetidine-1-carbonyl]-4,4a,5,7,8,8a-hexahydropyrido[4,3-b][1,4]oxazin-3-one;
(4aR,8aS)-6-(4-((4-Fluorophenyl)((1-methyl-5-(trifluoromethyl)-1H-pyrazol-3-yl)oxy)methyl)piperidine-1-carbonyl)hexahydro-2H-pyrido[4,3-b][1,4]oxazin-3(4H)-one;
(4aR,8aS)-6-[4-[[3-(2-Aminoethoxy)phenyl]-phenyl-methyl]piperidine-1-carbonyl]-4,4a,5,7,8,8a-hexahydropyrido[4,3-b][1,4]oxazin-3-one;
(4aR,8aS)-6-[3-[5-(2,4-Dichlorophenyl)-1,3,4-oxadiazol-2-yl]azetidine-1-carbonyl]-4,4a,5,7,8,8a-hexahydropyrido[4,3-b][1,4]oxazin-3-one;
(4aR,8aS)-6-[3-[3-Fluoro-4-(trifluoromethyl)phenyl]azetidine-1-carbonyl]-4,4a,5,7,8,8a-hexahydropyrido[4,3-b][1,4]oxazin-3-one;
tert-ButylN-[2-[3-[2-[3-[[1-[(4aR,8aS)-3-oxo-4,4a,5,7,8,8a-hexahydropyrido[4,3-b][1,4]oxazine-6-carbonyl]-4-piperidyl]-phenyl-methyl]phenoxy]ethylamino]-3-oxo-propoxy]ethyl]carbamate;
tert-ButylN-[2-[2-[3-[2-[3-[[1-[(4aR,8aS)-3-oxo-4,4a,5,7,8,8a-hexahydropyrido[4,3-b][1,4]oxazine-6-carbonyl]-4-piperidyl]-phenyl-methyl]phenoxy]ethylamino]-3-oxo-propoxy]ethoxy]ethyl]carbamate;
(4aR,8aS)-6-[3-[1-(2,4-Dichlorophenyl)pyrazol-3-yl]azetidine-1-carbonyl]-4,4a,5,7,8,8a-hexahydropyrido[4,3-b][1,4]oxazin-3-one;
(4aR,8aS)-6-[3-(4-Propoxyphenyl)azetidine-1-carbonyl]-4,4a,5,7,8,8a-hexahydropyrido[4,3-b][1,4]oxazin-3-one;
(4aR,8aS)-6-[3-(3,4-Pimethylphenyl)azetidine-1-carbonyl]-4,4a,5,7,8,8a-hexahydropyrido[4,3-b][1,4]oxazin-3-one;
2-[4-[1-[(4aR,8aS)-3-Oxo-4,4a,5,7,8,8a-hexahydropyrido[4,3-b][1,4]oxazine-6-carbonyl]azetidin-3-yl]phenyl]-N-ethyl-5-methyl-benzamide;
(4aR,8aS)-6-[3-[4-(2,2-Dimethylpropyl)phenyl]azetidine-1-carbonyl]-4,4a,5,7,8,8a-hexahydropyrido[4,3-b][1,4]oxazin-3-one;
(4aR,8aS)-6-[3-(4-tert-Butoxyphenyl)azetidine-1-carbonyl]-4,4a,5,7,8,8a-hexahydropyrido[4,3-b][1,4]oxazin-3-one;
(4aR,8aS)-6-[4-(5-Chloroindolin-1-yl)piperidine-1-carbonyl]-4,4a,5,7,8,8a-hexahydropyrido[4,3-b][1,4]oxazin-3-one;
(4aR,8aS)-6-[4-(4-Chloroisoindolin-2-yl)piperidine-1-carbonyl]-4,4a,5,7,8,8a-hexahydropyrido[4,3-b][1,4]oxazin-3-one;
(4aR,8aS)-6-[4-(5'-Chlorospiro[cyclopropane-1,3'-indoline]-1'-yl)piperidine-1-carbonyl]-4,4a,5,7,8,8a-hexahydropyrido[4,3-b][1,4]oxazin-3-one;
(4aR,8aS)-6-[3-(4-Chloroisoindolin-2-yl)azetidine-1-carbonyl]-4,4a,5,7,8,8a-hexahydropyrido[4,3-b][1,4]oxazin-3-one;
(4aR,8aS)-6-[4-(5-Chloroisoindolin-2-yl)piperidine-1-carbonyl]-4,4a,5,7,8,8a-hexahydropyrido[4,3-b][1,4]oxazin-3-one;
(4aS,8aS)-6-[4-[1-[2-[tert-Butyl(dimethyl)silyl]oxyethyl]-5-chloro-indol-3-yl]piperidine-1-carbonyl]-4,4a,5,7,8,8a-hexahydropyrido[4,3-b][1,4]oxazin-3-one;
(4aS,8aS)-6-[4-[5-Chloro-1-(2-hydroxyethyl)indol-3-yl]piperidine-1-carbonyl]-4,4a,5,7,8,8a-hexahydropyrido[4,3-b][1,4]oxazin-3-one;
(4aR,8aS)-6-(3-(5-(3-(Trifluoromethyl)pyrrolidin-1-yl)pyridin-2-yl)azetidine-1-carbonyl)hexahydro-2H-pyrido[4,3-b][1,4]oxazin-3(4H)-one;
(4aR,8aS)-6-(3-(5-((R or S)-3-(Trifluoromethyl)pyrrolidin-1-yl)pyridin-2-yl)azetidine-1-carbonyl)hexahydro-2H-pyrido[4,3-b][1,4]oxazin-3(4H)-one;
(4aR,8aS)-6-(3-(5-((S or R)-3-(Trifluoromethyl)pyrrolidin-1-yl)pyridin-2-yl)azetidine-1-carbonyl)hexahydro-2H-pyrido[4,3-b][1,4]oxazin-3(4H)-one;
(4aR,8aS)-6-[3-[6-[3-(Trifluoromethyl)pyrrolidin-1-yl]-3-pyridyl]azetidine-1-carbonyl]-4,4a,5,7,8,8a-hexahydropyrido[4,3-b][1,4]oxazin-3-one;
(4aR,8aS)-6-[3-[6-[3-(R or S)-(Trifluoromethyl)pyrrolidin-1-yl]-3-pyridyl]azetidine-1-carbonyl]-4,4a,5,7,8,8a-hexahydropyrido[4,3-b][1,4]oxazin-3-one;
(4aR,8aS)-6-[3-[6-[3S- or 3R-(trifluoromethyl)pyrrolidin-1-yl]-3-pyridyl]azetidine-1-carbonyl]-4,4a,5,7,8,8a-hexahydropyrido[4,3-b][1,4]oxazin-3-one;
(4aR,8aS)-6-[3-(4-Tetrahydropyran-3-ylphenyl)azetidine-1-carbonyl]-4,4a,5,7,8,8a-hexahydropyrido[4,3-b][1,4]oxazin-3-one;
(4aR,8aS)-6-[3-[2-Methoxy-4-(2,2,2-trifluoroethyl)phenyl]azetidine-1-carbonyl]-4,4a,5,7,8,8a-hexahydropyrido[4,3-b][1,4]oxazin-3-one;
(4aR,8aS)-6-(3-(4-(Neopentyloxy)phenyl)azetidine-1-carbonyl)hexahydro-2H-pyrido[4,3-b][1,4]oxazin-3(4H)-one;
rac-(4aR,8aS)-6-[4-(3-Methoxy-1-phenyl-propyl)piperidine-1-carbonyl]-4,4a,5,7,8,8a-hexahydropyrido[4,3-b][1,4]oxazin-3-one;
rac-(4aR,8aS)-6-[4-[Phenyl(3-pyridyl)methyl]piperidine-1-carbonyl]-4,4a,5,7,8,8a-hexahydropyrido[4,3-b][1,4]oxazin-3-one;
rac-(4aR,8aS)-6-[4-[(5-Methyl-1,2,4-oxadiazol-3-yl)-phenyl-methyl]piperidine-1-carbonyl]-4,4a,5,7,8,8a-hexahydropyrido[4,3-b][1,4]oxazin-3-one;
rac-(4aR,8aS)-6-[4-(5-Fluoro-1-methyl-indol-3-yl)piperidine-1-carbonyl]-4,4a,5,7,8,8a-hexahydropyrido[4,3-b][1,4]oxazin-3-one;
tert-Butyl3-[5-chloro-3-[1-[rac-(4aR,8aS)-3-oxo-4,4a,5,7,8,8a-hexahydropyrido[4,3-b][1,4]oxazine-6-carbonyl]-4-piperidyl]indol-1-yl]azetidine-1-carboxylate;
tert-Butyl3-[5-chloro-3-[1-[rac-(4aR,8aS)-3-oxo-4,4a,5,7,8,8a-hexahydropyrido[4,3-b][1,4]oxazine-6-carbonyl]-4-piperidyl]indol-1-yl]azetidine-1-carboxylate;
rac-(4aR,8aS)-6-[4-[1-(2-Chloro-4-fluoro-phenoxy)propyl]piperidine-1-carbonyl]-4,4a,5,7,8,8a-hexahydropyrido[4,3-b][1,4]oxazin-3-one;
rac-(4aR,8aS)-6-[4-[(2-Chloro-4-fluoro-phenoxy)-cyclopropyl-methyl]piperidine-1-carbonyl]-4,4a,5,7,8,8a-hexahydropyrido[4,3-b][1,4]oxazin-3-one;
rac-(4aR,8aS)-6-[4-[(4-Fluorophenoxy)-phenyl-methyl]piperidine-1-carbonyl]-4,4a,5,7,8,8a-hexahydropyrido[4,3-b][1,4]oxazin-3-one;
rac-(4aR,8aS)-6-[4-[5-(Trifluoromethyl)-2-pyridyl]piperidine-1-carbonyl]-4,4a,5,7,8,8a-hexahydropyrido[4,3-b][1,4]oxazin-3-one;
rac-(4aR,8aS)-6-[4-[Cyclopropylmethoxy-[4-(trifluoromethyl)phenyl]methyl]piperidine-1-carbonyl]-4,4a,5,7,8,8a-hexahydropyrido[4,3-b][1,4]oxazin-3-one;
rac-(4aR,8aS)-6-[4-[1-(4-Fluorophenyl)-2-hydroxy-ethyl]piperazine-1-carbonyl]-4,4a,5,7,8,8a-hexahydropyrido[4,3-b][1,4]oxazin-3-one;
rac-(4aR,8aS)-6-[4-[1-(4-Fluorophenyl)-2-methoxy-ethyl]piperazine-1-carbonyl]-4,4a,5,7,8,8a-hexahydropyrido[4,3-b][1,4]oxazin-3-one;
rac-(4aR,8aS)-6-[4-[1-(3,4-Difluorophenyl)-2-hydroxy-ethyl]piperazine-1-carbonyl]-4,4a,5,7,8,8a-hexahydropyrido[4,3-b][1,4]oxazin-3-one;
rac-(4aR,8aS)-6-[4-[1-(3,4-Difluorophenyl)-2-methoxy-ethyl]piperazine-1-carbonyl]-4,4a,5,7,8,8a-hexahydropyrido[4,3-b][1,4]oxazin-3-one;
rac-(4aR,8aS)-6-[4-[1-(4,4-Difluoro-1-piperidyl)cyclopropyl]piperidine-1-carbonyl]-4,4a,5,7,8,8a-hexahydropyrido[4,3-b][1,4]oxazin-3-one;
rac-(4aR,8aS)-6-[4-[1-[4-(Trifluoromethyl)imidazol-1-yl]ethyl]piperidine-1-carbonyl]-4,4a,5,7,8,8a-hexahydropyrido[4,3-b][1,4]oxazin-3-one;
rac-(4aR,8aS)-6-[4-[1-[4-(Trifluoromethyl)pyrazol-1-yl]cyclopropyl]piperidine-1-carbonyl]-4,4a,5,7,8,8a-hexahydropyrido[4,3-b][1,4]oxazin-3-one;
rac-(4aR,8aS)-6-[4-[(4-tert-Butylpyrazol-1-yl)-(4-fluorophenyl)methyl]piperidine-1-carbonyl]-4,4a,5,7,8,8a-hexahydropyrido[4,3-b][1,4]oxazin-3-one;
rac-(4aR,8aS)-6-[4-[3-[4-(Trifluoromethyl)phenyl]oxetan-3-yl]piperidine-1-carbonyl]-4,4a,5,7,8,8a-hexahydropyrido[4,3-b][1,4]oxazin-3-one;
rac-(4aR,8aS)-6-[4-[4-(Trifluoromethyl)benzoyl]piperidine-1-carbonyl]-4,4a,5,7,8,8a-hexahydropyrido[4,3-b][1,4]oxazin-3-one;
rac-(4aR,8aS)-6-[4-[1-[4-(Trifluoromethyl)pyrazol-1-yl]ethyl]piperidine-1-carbonyl]-4,4a,5,7,8,8a-hexahydropyrido[4,3-b][1,4]oxazin-3-one;
rac-(4aR,8aS)-6-[4-[(4-Fluorophenyl)-morpholino-methyl]piperidine-1-carbonyl]-4,4a,5,7,8,8a-hexahydropyrido[4,3-b][1,4]oxazin-3-one;
rac-(4aR,8aS)-6-[4-[(4-tert-Butyloxazol-2-yl)-difluoro-methyl]piperidine-1-carbonyl]-4,4a,5,7,8,8a-hexahydropyrido[4,3-b][1,4]oxazin-3-one;
rac-(4aR,8aS)-6-[4-[1-(4-Chlorophenyl)-2-hydroxy-ethyl]piperazine-1-carbonyl]-4,4a,5,7,8,8a-hexahydropyrido[4,3-b][1,4]oxazin-3-one;
rac-(4aR,8aS)-6-[4-[1-(4-Chlorophenyl)-2-methoxy-ethyl]piperazine-1-carbonyl]-4,4a,5,7,8,8a-hexahydropyrido[4,3-b][1,4]oxazin-3-one;
rac-(4aR,8aS)-6-[4-[2-Hydroxy-1-[4-(trifluoromethyl)phenyl]ethyl]piperazine-1-carbonyl]-4,4a,5,7,8,8a-hexahydropyrido[4,3-b][1,4]oxazin-3-one;
rac-(4aR,8aS)-6-[4-[2-Methoxy-1-[4-(trifluoromethyl)phenyl]ethyl]piperazine-1-carbonyl]-4,4a,5,7,8,8a-hexahydropyrido[4,3-b][1,4]oxazin-3-one;
rac-(4aR,8aS)-6-[4-(1-Cyclohexyl-2-methoxy-ethyl)piperazine-1-carbonyl]-4,4a,5,7,8,8a-hexahydropyrido[4,3-b][1,4]oxazin-3-one;
rac-(4aR,8aS)-6-[4-(1-Cyclohexyl-2-hydroxy-ethyl)piperazine-1-carbonyl]-4,4a,5,7,8,8a-hexahydropyrido[4,3-b][1,4]oxazin-3-one;
rac-(4aR,8aS)-6-[4-[1-(4-Fluorophenyl)cyclopropyl]piperazine-1-carbonyl]-4,4a,5,7,8,8a-hexahydropyrido[4,3-b][1,4]oxazin-3-one;
rac-(4aR,8aS)-6-[4-[2-(4-Fluorophenyl)cyclopropyl]piperazine-1-carbonyl]-4,4a,5,7,8,8a-hexahydropyrido[4,3-b][1,4]oxazin-3-one;
rac-(4aR,8aS)-6-[4-[1-(4-Fluorophenyl)cyclobutyl]piperazine-1-carbonyl]-4,4a,5,7,8,8a-hexahydropyrido[4,3-b][1,4]oxazin-3-one;
rac-(4aR,8aS)-6-[4-[Cyclopropyl-(4-fluorophenyl)methyl]piperazine-1-carbonyl]-4,4a,5,7,8,8a-hexahydropyrido[4,3-b][1,4]oxazin-3-one;
rac-(4aR,8aS)-6-[4-[Cyclohexyl-(4-fluorophenyl)methyl]piperazine-1-carbonyl]-4,4a,5,7,8,8a-hexahydropyrido[4,3-b][1,4]oxazin-3-one;
rac-(4aR,8aS)-6-[4-[(4-Fluorophenyl)-(4-pyridyl)methyl]piperazine-1-carbonyl]-4,4a,5,7,8,8a-hexahydropyrido[4,3-b][1,4]oxazin-3-one;
rac-(4aR,8aS)-6-[4-[2,2,2-Trifluoro-1-(4-fluorophenyl)ethyl]piperazine-1-carbonyl]-4,4a,5,7,8,8a-hexahydropyrido[4,3-b][1,4]oxazin-3-one;
rac-(4aR,8aS)-6-[4-[(4-Fluorophenyl)-oxazol-5-yl-methyl]piperazine-1-carbonyl]-4,4a,5,7,8,8a-hexahydropyrido[4,3-b][1,4]oxazin-3-one;
rac-(4aR,8aS)-6-[4-[bis(4-Chlorophenyl)methyl]piperazine-1-carbonyl]-4,4a,5,7,8,8a-hexahydropyrido[4,3-b][1,4]oxazin-3-one;
rac-(4aS,8aS)-6-[4-[5-Chloro-1-(cyclopropylmethyl)indol-3-yl]piperidine-1-carbonyl]-4,4a,5,7,8,8a-hexahydropyrido[4,3-b][1,4]oxazin-3-one;
rac-(4aS,8aS)-6-[4-[5-Chloro-1-(cyclopropylmethyl)indol-3-yl]piperidine-1-carbonyl]-4,4a,5,7,8,8a-hexahydropyrido[4,3-b][1,4]oxazin-3-one;
rac-(4aR,8aR)-6-[4-[5-Chloro-1-(cyclopropylmethyl)indol-3-yl]piperidine-1-carbonyl]-4,4a,5,7,8,8a-hexahydropyrido[4,3-b][1,4]oxazin-3-one;
rac-(4aR,8aS)-6-[4-[(4-Chlorophenyl)-(4-fluorophenyl)methyl]piperazine-1-carbonyl]-4,4a,5,7,8,8a-hexahydropyrido[4,3-b][1,4]oxazin-3-one;
rac-(4aR,8aS)-6-(4-Benzhydrylpiperazine-1-carbonyl)-4,4a,5,7,8,8a-hexahydropyrido[4,3-b][1,4]oxazin-3-one;
rac-(4aR,8aS)-6-[4-[4-(2,2,2-Trifluoroethyl)phenyl]piperazine-1-carbonyl]-4,4a,5,7,8,8a-hexahydropyrido[4,3-b][1,4]oxazin-3-one;
rac-(4aR,8aS)-6-[4-[3-(2,2,2-Trifluoroethyl)phenyl]piperazine-1-carbonyl]-4,4a,5,7,8,8a-hexahydropyrido[4,3-b][1,4]oxazin-3-one;
rac-(4aR,8aS)-6-[4-[4-Chloro-3-(trifluoromethyl)phenyl]piperazine-1-carbonyl]-4,4a,5,7,8,8a-hexahydropyrido[4,3-b][1,4]oxazin-3-one;
rac-(4aR,8aS)-6-[4-[1-[4-(Trifluoromethyl)phenyl]ethyl]piperazine-1-carbonyl]-4,4a,5,7,8,8a-hexahydropyrido[4,3-b][1,4]oxazin-3-one;
rac-(4aR,8aS)-6-[3-[1-(2-Chloro-4-fluoro-phenoxy)ethyl]azetidine-1-carbonyl]-4,4a,5,7,8,8a-hexahydropyrido[4,3-b][1,4]oxazin-3-one; and
rac-(4aR,8aS)-6-[3-[5-Chloro-1-(cyclopropylmethyl)indol-3-yl]azetidine-1-carbonyl]-4,4a,5,7,8,8a-hexahydropyrido[4,3-b][1,4]oxazin-3-one.

10. The compound of formula (I) according to any one of claims 1 to 8, or a pharmaceutically acceptable salt there of, selected from:
rac-cis-6-(4-(5-Chloro-1-(cyclopropylmethyl)-1H-indol-3-yl)piperidine-1-carbonyl)hexahydro-2H-pyrido[4,3-b][1,4]oxazin-3(4H)-one;
rac-cis-6-(4-(5-Chloro-1-methyl-1H-indol-3-yl)piperidine-1-carbonyl)hexahydro-2H-pyrido[4,3-b][1,4]oxazin-3(4H)-one;
rac-cis-6-(4-(1-(4-Fluorophenyl)-3-methoxypropyl)piperidine-1-carbonyl)hexahydro-2H-pyrido[4,3-b][1,4]oxazin-3(4H)-one;
rac-cis-6-(4-((4-Chlorophenyl)(phenyl)methyl)piperazine-1-carbonyl)hexahydro-2H-pyrido[4,3-b][1,4]oxazin-3(4H)-one;
(+)-cis-6-(4-(bis(4-Fluorophenyl)methyl)piperazine-1-carbonyl)hexahydro-2H-pyrido[4,3-b][1,4]oxazin-3(4H)-one;
(+)- or (-)-cis-6-(4-(5-Chloro-1-cyclopropyl-1H-indol-3-yl)piperidine-1-carbonyl)hexahydro-2H-pyrido[4,3-b][1,4]oxazin-3(4H)-one;
(+)-or (-)-cis-6-(4-(5-Chloro-1-(oxetan-3-yl)-1H-indol-3-yl)piperidine-1-carbonyl)hexahydro-2H-pyrido[4,3-b][1,4]oxazin-3(4H)-one;
rac-cis-6-(4-(1-(4-Fluorophenyl)-3-hydroxypropyl)piperidine-1-carbonyl)hexahydro-2H-pyrido[4,3-b][1,4]oxazin-3(4H)-one;
(4aR,8aS)-6-(4-(Difluoro(4-(trifluoromethyl)phenyl)methyl)piperidine-1-carbonyl)hexahydro-2H-pyrido[4,3-b][1,4]oxazin-3(4H)-one;
(+) or (-)-cis-6-(4-((R or S)-1-(2-chloro-4-fluorophenoxy)ethyl)piperidine-1-carbonyl)hexahydro-2H-pyrido[4,3-b][1,4]oxazin-3(4H)-one;
(+) or (-)-cis-6-(4-((4-Chlorophenyl)difluoromethyl)piperidine-1-carbonyl)hexahydro-2H-pyrido[4,3-b][1,4]oxazin-3(4H)-one;
(+) or (-)-cis-6-(4-((2-Chloro-4-fluorophenyl)difluoromethyl)piperidine-1-carbonyl)hexahydro-2H-pyrido[4,3-b][1,4]oxazin-3(4H)-one;
(4aR,8aS)-6-(4-((R or S)-1-(4-(Trifluoromethyl)phenyl)ethyl)piperidine-1-carbonyl)hexahydro-2H-pyrido[4,3-b][1,4]oxazin-3(4H)-one;
(4aR,8aS)-6-(4-((S or R)-(4-Fluorophenyl)(2,2,2-trifluoroethoxy)methyl)piperidine-1-carbonyl)hexahydro-2H-pyrido[4,3-b][1,4]oxazin-3(4H)-one;
(4aR,8aS)-6-[4-[(S or R)-(4-Fluorophenyl)-(3-methoxyphenyl)methyl]piperidine-1-carbonyl]-4,4a,5,7,8,8a-hexahydropyrido[4,3-b][1,4]oxazin-3-one;
(4aR,8aS)-6-(4-((R or S)-(4-Fluorophenyl)(3-methoxyphenyl)methyl)piperidine-1-carbonyl)hexahydro-2H-pyrido[4,3-b][1,4]oxazin-3(4H)-one;
(4aR,8aS)-6-(4-((S or R)-(3-(2-Fluoroethoxy)phenyl)(phenyl)methyl)piperidine-1-carbonyl)hexahydro-2H-pyrido[4,3-b][1,4]oxazin-3(4H)-one;
(4aR,8aS)-6-(4-((3-Cyclopropyl-1,2,4-oxadiazol-5-yl)(4-fluorophenyl)methyl)piperidine-1-carbonyl)hexahydro-2H-pyrido[4,3-b][1,4]oxazin-3(4H)-one;
(4aR,8aS)-6-(4-((S or R)-(4-Fluorophenyl)(4-methoxyphenyl)methyl)piperidine-1-carbonyl)hexahydro-2H-pyrido[4,3-b][1,4]oxazin-3(4H)-one;
(4aR,8aS)-6-(4-((S or R)-(3,4-Dimethoxyphenyl)(4-fluorophenyl)methyl)piperidine-1-carbonyl)hexahydro-2H-pyrido[4,3-b][1,4]oxazin-3(4H)-one;
(4aR,8aS)-6-(4-((R or S)-(4-Fluorophenyl)(4-methoxyphenyl)methyl)piperidine-1-carbonyl)hexahydro-2H-pyrido[4,3-b][1,4]oxazin-3(4H)-one;
(4aR,8aS)-6-(4-((S or R)-(4-(2-Fluoroethoxy)phenyl)(4-fluorophenyl)methyl)piperidine-1-carbonyl)hexahydro-2H-pyrido[4,3-b][1,4]oxazin-3(4H)-one;
(4aR,8aS)-6-[3-[4-(Trifluoromethoxy)phenyl]azetidine-1-carbonyl]-4,4a,5,7,8,8a-hexahydropyrido[4,3-b][1,4]oxazin-3-one;
(4aR,8aS)-6-(3-(2'-Trifluoromethoxy)-[1,1'-biphenyl]-4-yl)azetidine-1-carbonyl)hexahydro-2H-pyrido[4,3-b][1,4]oxazin-3(4H)-one;
(4aR,8aS)-6-(3-(2'-(Trifluoromethyl)-[1,1'-biphenyl]-4-yl)azetidine-1-carbonyl)hexahydro-2H-pyrido[4,3-b][1,4]oxazin-3(4H)-one;
(4aR,8aS)-6-(3-(2',4'-Difluoro-[1,1'-biphenyl]-4-yl)azetidine-1-carbonyl)hexahydro-2H-pyrido[4,3-b][1,4]oxazin-3(4H)-one;
(4aR,8aS)-6-(3-(4-(3-(Trifluoromethyl)azetidin-1-yl)phenyl)azetidine-1-carbonyl)hexahydro-2H-pyrido[4,3-b][1,4]oxazin-3(4H)-one;
(4aR,8aS)-6-(3-(4-Bromo-3-chlorophenyl)azetidine-1-carbonyl)hexahydro-2H-pyrido[4,3-b][1,4]oxazin-3(4H)-one;
(4aR,8aS)-6-[3-[4-(4-Chloro-2-fluoro-phenyl)phenyl]azetidine-1-carbonyl]-4,4a,5,7,8,8a-hexahydropyrido[4,3-b][1,4]oxazin-3-one;
(4aR,8aS)-6-[3-[4-(2-Chloro-4-fluoro-phenyl)phenyl]azetidine-1-carbonyl]-4,4a,5,7,8,8a-hexahydropyrido[4,3-b][1,4]oxazin-3-one;
(4aR,8aS)-6-(3-(4-(3-((1,1,1-Trifluoropropan-2-yl)oxy)azetidin-1-yl)phenyl)azetidine-1-carbonyl)hexahydro-2H-pyrido[4,3-b][1,4]oxazin-3(4H)-one;
(4aR,8aS)-6-(3-(4-(tert-Butyl)phenyl)azetidine-1-carbonyl)hexahydro-2H-pyrido[4,3-b][1,4]oxazin-3(4H)-one;
(4aR,8aS)-6-(3-(4-(Trifluoromethyl)phenyl)azetidine-1-carbonyl)hexahydro-2H-pyrido[4,3-b][1,4]oxazin-3(4H)-one;
(4aR,8aS)-6-(3-(4-(3-((1,1,1-Trifluoro-2-methylpropan-2-yl)oxy)azetidin-1-yl)phenyl)azetidine-1-carbonyl)hexahydro-2H-pyrido[4,3-b][1,4]oxazin-3(4H)-one;
(4aR,8aS)-6-(3-(4-(1,1-Difluoroethyl)phenyl)azetidine-1-carbonyl)hexahydro-2H-pyrido[4,3-b][1,4]oxazin-3(4H)-one;
(4aR,8aS)-6-(3-(4-(3,3-Dimethylpyrrolidin-1-yl)phenyl)azetidine-1-carbonyl)hexahydro-2H-pyrido[4,3-b][1,4]oxazin-3(4H)-one;
(4aR,8aS)-6-(3-(4-(3-(Trifluoromethoxy)azetidin-1-yl)phenyl)azetidine-1-carbonyl)hexahydro-2H-pyrido[4,3-b][1,4]oxazin-3(4H)-one;
(4aR,8aS)-6-(3-(6-(2,4-Dichlorophenyl)pyridin-3-yl)azetidine-1-carbonyl)hexahydro-2H-pyrido[4,3-b][1,4]oxazin-3(4H)-one;
(4aR,8aS)-6-[3-[4-(2,2,2-Trifluoroethoxy)phenyl]azetidine-1-carbonyl]-4,4a,5,7,8,8a-hexahydropyrido[4,3-b][1,4]oxazin-3-one;
(4aR,8aS)-6-[3-[4-[3-[(1S or 1R)-2,2,2-Trifluoro-1-methyl-ethoxy]azetidin-1-yl]phenyl]azetidine-1-carbonyl]-4,4a,5,7,8,8a-hexahydropyrido[4,3-b][1,4]oxazin-3-one;
(4aR,8aS)-6-[3-[4-[3-[(1R or 1S)-2,2,2-Trifluoro-1-methyl-ethoxy]azetidin-1-yl]phenyl]azetidine-1-carbonyl]-4,4a,5,7,8,8a-hexahydropyrido[4,3-b][1,4]oxazin-3-one;
(4aR,8aS)-6-(3-(4-(2-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-(3-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-(3-(2,2,2-trifluoroethoxy)azetidin-1-yl)phenyl)azetidine-1-carbonyl)hexahydro-2H-pyrido[4,3-b][1,4]oxazin-3(4H)-one;
(4aR,8aS)-6-(3-(6-(2-(trifluoromethyl)pyrrolidin-1-yl)pyridin-3-yl)azetidine-1-carbonyl)hexahydro-2H-pyrido[4,3-b][1,4]oxazin-3(4H)-one;
(4aR,8aS)-6-(3-(4-(Pentafluoro-l6-sulfaneyl)phenyl)azetidine-1-carbonyl)hexahydro-2H-pyrido[4,3-b][1,4]oxazin-3(4H)-one;
(4aR,8aS)-6-(3-(2-Fluoro-4-(trifluoromethoxy)phenyl)azetidine-1-carbonyl)hexahydro-2H-pyrido[4,3-b][1,4]oxazin-3(4H)-one;
(4aR,8aS)-6-[3-[4-(2,2,2-Trifluoroethyl)phenyl]azetidine-1-carbonyl]-4,4a,5,7,8,8a-hexahydropyrido[4,3-b][1,4]oxazin-3-one;
(4aR,8aS)-6-[3-[4-[1-(Trifluoromethyl)cyclopropyl]phenyl]azetidine-1-carbonyl]-4,4a,5,7,8,8a-hexahydropyrido[4,3-b][1,4]oxazin-3-one;
(4aR,8aS)-6-[3-[4-(6,6-Difluoro-2-azaspiro[3.3]heptan-2-yl)phenyl]azetidine-1-carbonyl]-4,4a,5,7,8,8a-hexahydropyrido[4,3-b][1,4]oxazin-3-one;
(4aR,8aS)-6-(3-(5-(2,4-Dichlorophenyl)pyridin-2-yl)azetidine-1-carbonyl)hexahydro-2H-pyrido[4,3-b][1,4]oxazin-3(4H)-one;
(4aR,8aS)-6-(3-(4-((R or S)-2-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-((S or R)-2-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-((R or S)-3-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(4-((S or R)-3-(Trifluoromethyl)pyrrolidin-1-yl)phenyl)azetidine-1-carbonyl)hexahydro-2H-pyrido[4,3-b][1,4]oxazin-3(4H)-one;
(4aR,8aS)-6-(3-(3-Fluoro-4-(trifluoromethoxy)phenyl)azetidine-1-carbonyl)hexahydro-2H-pyrido[4,3-b][1,4]oxazin-3(4H)-one;
(4aR,8aS)-6-(3-(3-Methyl-4-(trifluoromethoxy)phenyl)azetidine-1-carbonyl)hexahydro-2H-pyrido[4,3-b][1,4]oxazin-3(4H)-one;
(4aR,8aS)-6-(3-(3,5-Difluoro-4-(trifluoromethoxy)phenyl)azetidine-1-carbonyl)hexahydro-2H-pyrido[4,3-b][1,4]oxazin-3(4H)-one;
(4aR,8aS)-6-(3-(3-Chloro-4-(trifluoromethoxy)phenyl)azetidine-1-carbonyl)hexahydro-2H-pyrido[4,3-b][1,4]oxazin-3(4H)-one;
(4aR,8aS)-6-((R or S)-3-(3-Chloro-5-(2,2,2-trifluoroethoxy)phenyl)pyrrolidine-1-carbonyl)hexahydro-2H-pyrido[4,3-b][1,4]oxazin-3(4H)-one;
(4aR,8aS)-6-((S or R)-3-(3-Chloro-5-(2,2,2-trifluoroethoxy)phenyl)pyrrolidine-1-carbonyl)hexahydro-2H-pyrido[4,3-b][1,4]oxazin-3(4H)-one;
(4aR,8aS)-6-(3-(4-(tert-Butyl)-3-methoxyphenyl)azetidine-1-carbonyl)hexahydro-2H-pyrido[4,3-b][1,4]oxazin-3(4H)-one;
(4aR,8aS)-6-(3-(4-Propylphenyl)azetidine-1-carbonyl)hexahydro-2H-pyrido[4,3-b][1,4]oxazin-3(4H)-one;
(4aR,8aS)-6-(3-(4-(Trifluoromethoxy)-3-(trifluoromethyl)phenyl)azetidine-1-carbonyl)hexahydro-2H-pyrido[4,3-b][1,4]oxazin-3(4H)-one;
(4aR,8aS)-6-(3-(5-((R or S)-3-(Trifluoromethyl)pyrrolidin-1-yl)pyridin-2-yl)azetidine-1-carbonyl)hexahydro-2H-pyrido[4,3-b][1,4]oxazin-3(4H)-one;
(4aR,8aS)-6-(3-(5-((S or R)-3-(Trifluoromethyl)pyrrolidin-1-yl)pyridin-2-yl)azetidine-1-carbonyl)hexahydro-2H-pyrido[4,3-b][1,4]oxazin-3(4H)-one;
(4aR,8aS)-6-[3-[6-[3-(R or S)-(trifluoromethyl)pyrrolidin-1-yl]-3-pyridyl]azetidine-1-carbonyl]-4,4a,5,7,8,8a-hexahydropyrido[4,3-b][1,4]oxazin-3-one;
(4aR,8aS)-6-[3-[6-[3-(S or R)-(trifluoromethyl)pyrrolidin-1-yl]-3-pyridyl]azetidine-1-carbonyl]-4,4a,5,7,8,8a-hexahydropyrido[4,3-b][1,4]oxazin-3-one;
(4aR,8aS)-6-[3-(4-Tetrahydropyran-3-ylphenyl)azetidine-1-carbonyl]-4,4a,5,7,8,8a-hexahydropyrido[4,3-b][1,4]oxazin-3-one;
(4aR,8aS)-6-[3-[2-Methoxy-4-(2,2,2-trifluoroethyl)phenyl]azetidine-1-carbonyl]-4,4a,5,7,8,8a-hexahydropyrido[4,3-b][1,4]oxazin-3-one; and
(4aR,8aS)-6-(3-(4-(Neopentyloxy)phenyl)azetidine-1-carbonyl)hexahydro-2H-pyrido[4,3-b][1,4]oxazin-3(4H)-one.

11. A process of manufacturing the compounds of formula (Ic) according to anyone of claims 1 to 10, comprising:
reacting 4a,5,6,7,8,8a-hexahydro-4H-pyrido[4,3-b][1,4]oxazin-3-one (**1**), 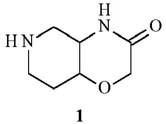
with a heterocyclic amine **2a**, wherein A, L, X, m, n, and R²⁰ to R²³ are as defined in any one of claims 1 to 10 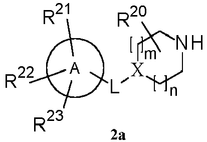
in the presence of a base and a urea forming reagent,
to form said compound of formula (Ic).

12. A compound of formula (Ic) according to any one of claims 1 to 10, or a pharmaceutically acceptable salt thereof, for use as therapeutically active substance.

13. A pharmaceutical composition comprising a compound of formula (Ic) according to any one of claims 1 to 10, or a pharmaceutically acceptable salt thereof, and a therapeutically inert carrier.

14. A compound of formula (Ic) according to any one of claims 1 to 10, or a pharmaceutically acceptable salt thereof, for use in the treatment or prophylaxis of neuroinflammation, neurodegenerative diseases, pain, cancer and/or mental disorders in a mammal.

15. The compound for use according to claim 14, wherein said neuroinflammation, neurodegenerative diseases, pain, cancer and/or mental disorders are selected from multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

## Patentansprüche

1. Verbindung der Formel (Ic) 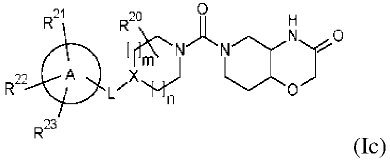 oder ein pharmazeutisch unbedenkliches Salz davon, wobei:
L aus -CR¹R²-(CH₂)ₚ-, -(CH₂)ₚ-CR¹R²-, -OCR³R⁴-, -CR³R⁴O- und einer kovalenten Bindung ausgewählt ist;
m 0 ist, n 0 oder 1 ist und X CR²⁴ ist; oder
m 1 ist, n 1 oder 2 ist und X CR²⁴ oder N ist;
p 0, 1 oder 2 ist;
R¹ aus Halogen, C₁₋₆-Alkoxy, C₁₋₆-Alkyl, Halogen-C₁₋₆-alkyl, Hydroxy-C₁₋₆-alkyl, C₁₋₆-Alkoxy-C₁₋₆-alkyl-, Halogen-C₁₋₆-alkoxy, Halogen-C₁₋₆-alkoxy-C₁₋₆-alkyl-, Cyano und einer Gruppe 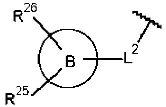 ausgewählt ist; und R² aus Wasserstoff, Halogen und Hydroxy ausgewählt ist; oder
R¹ und R² zusammen mit dem Kohlenstoffatom, an das sie gebunden sind, ein C₃₋₁₂-Cycloalkyl oder ein C₂₋₉-Heterocyclyl bilden;
R³ aus C₁₋₆-Alkyl, Halogen-C₁₋₆-alkyl, C₃₋₁₂-Cycloalkyl und C₆₋₁₄-Aryl ausgewählt ist; und R⁴ Wasserstoff ist; oder
R³ und R⁴ zusammen mit dem Kohlenstoffatom, an das sie gebunden sind, ein C₃₋₁₂-Cycloalkyl oder ein C₂₋₉-Heterocyclyl bilden;
R²⁰ Wasserstoff oder C₁₋₆-Alkyl ist;
R²¹, R²² und R²³ unabhängig voneinander aus Wasserstoff, Halogen, Cyano, Hydroxy, C₁₋₆-Alkoxy, Halogen-C₁₋₆-alkoxy, Amino-C₁₋₆-alkoxy, C₁₋₆-Alkyl, Halogen-C₁₋₆-alkyl, Hydroxy-C₁₋₆-alkyl, (Halogen-C₁₋₆-alkyl)-hydroxy-C₁₋₆-alkyl, C₁₋₆-Alkoxycarbonyl-C₁₋₆-alkyl-, C₁₋₆-Alkoxycarbonyl-NH-C₁₋₆-alkoxy-, C₁₋₆-Alkoxycarbonyl-NH-(C₁₋₆-alkoxy)₂-C₁₋₆-alkyl-C(O)-NH-C₁₋₆-alkoxy-, C₁₋₆-Alkoxycarbonyl-NH-C₁₋₆-alkoxy-C₁₋₆-alkyl-C(O)-NH-C₁₋₆-alkoxy-, SF₅, (C₁₋₆-Alkyl)₃Si-O-C₁₋₆-alkyl-, einer Gruppe 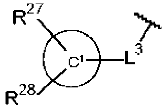 und einer Gruppe 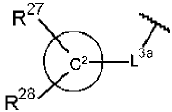 ausgewählt sind;
R²⁴ aus Wasserstoff, Halogen, Hydroxy, Halogen-C₁₋₆-alkyl und C₁₋₆-Alkyl ausgewählt ist;
R²⁵ und R²⁶ unabhängig voneinander aus Wasserstoff, Halogen, C₁₋₆-Alkyl, C₁₋₆-Alkoxy, Halogen-C₁₋₆-alkyl, Halogen-C₁₋₆-alkoxy, C₁₋₆-Alkyl-SO₂- und C₃₋₁₂-Cycloalkyl ausgewählt sind;
R²⁷ und R²⁸ unabhängig voneinander aus Wasserstoff, Halogen, C₁₋₆-Alkoxy, Halogen-C₁₋₆-alkoxy, C₁₋₆-Alkyl, Halogen-C₁₋₆-alkyl, Hydroxy-C₁₋₆-alkyl, Halogen, Hydroxy, C₁₋₆-Alkylsulfonyl, Carbamoyl, Cyano, Cycloalkyl-C₁₋₆-alkoxy-, C₁₋₆-Alkyl-NH-C(O)- und Cycloalkyl ausgewählt sind;
A und B unabhängig voneinander aus C₆₋₁₄-Aryl, C₁₋₁₃-Heteroaryl, C₃₋₁₂-Cycloalkyl und C₂₋₉-Heterocyclyl ausgewählt sind;
C¹ C₃₋₁₂-Cycloalkyl oder C₂₋₉-Heterocyclyl ist;
C² C₆₋₁₄-Aryl ist;
L² aus einer kovalenten Bindung, -O-, -CH₂O-, -CH₂OCH₂- und -CH₂- ausgewählt ist;
L³ aus einer kovalenten Bindung, -O-, -CH₂O-, -OCH₂-, -CH₂OCH₂- und -CH₂-ausgewählt ist; und
L^{3a} aus einer kovalenten Bindung, -CH₂O-, -OCH₂-, -CH₂OCH₂- und -CH₂- ausgewählt ist.

2. Verbindung der Formel (Ic) nach Anspruch 1 oder ein pharmazeutisch unbedenkliches Salz davon, wobei:
m 0 ist, n 0 oder 1 ist und X CR²⁴ ist; oder
m 1 ist, n 1 ist und X CR²⁴ oder N ist; und
R²⁰ und R²⁴ beide Wasserstoff sind.

3. Verbindung der Formel (Ic) nach einem der Ansprüche 1 und 2 oder ein pharmazeutisch unbedenkliches Salz davon, wobei:
L aus -CR¹R²-(CH₂)ₚ-, -OCR³R⁴-, -CR³R⁴O- und einer kovalenten Bindung ausgewählt ist;
p 0 oder 1 ist;
R¹ aus Halogen, C₁₋₆-Alkoxy, C₁₋₆-Alkyl, Halogen-C₁₋₆-alkyl, Hydroxy-C₁₋₆-alkyl, C₁₋₆-Alkoxy-C₁₋₆-alkyl-, Halogen-C₁₋₆-alkoxy, Halogen-C₁₋₆-alkoxy-C₁₋₆-alkyl-, Cyano und einer Gruppe 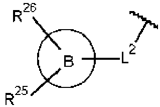 ausgewählt ist; und R² aus Wasserstoff, Halogen und Hydroxy ausgewählt ist; oder
R¹ und R² zusammen mit dem Kohlenstoffatom, an das sie gebunden sind, ein C₃₋₁₂-Cycloalkyl bilden;
R³ C₁₋₆-Alkyl oder Halogen-C₁₋₆-alkyl ist;
R⁴ Wasserstoff ist;
R²¹ aus Wasserstoff, Halogen, Hydroxy, C₁₋₆-Alkoxy, Halogen-C₁₋₆-alkoxy, C₁₋₆-Alkyl, Halogen-C₁₋₆-alkyl, Hydroxy-C₁₋₆-alkyl, (Halogen-C₁₋₆-alkyl)-hydroxy-C₁₋₆-alkyl, C₁₋₆-Alkoxycarbonyl-C₁₋₆-alkyl-, C₁₋₆-Alkoxycarbonyl-NH-C₁₋₆-alkoxy-, SF₅, (C₁₋₆-Alkyl)₃Si-O-C₁₋₆-alkyl-, einer Gruppe 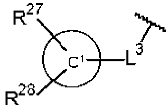 und einer Gruppe 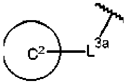 ausgewählt ist;
R²² aus Wasserstoff, Halogen, C₁₋₆-Alkoxy, Halogen-C₁₋₆-alkoxy, C₁₋₆-Alkyl und Cyano ausgewählt ist;
R²³ Wasserstoff oder Halogen ist;
R²⁵ aus Wasserstoff, Halogen, C₁₋₆-Alkyl, C₁₋₆-Alkoxy, Halogen-C₁₋₆-alkyl, Halogen-C₁₋₆-alkoxy, C₁₋₆-Alkyl-SO₂- und C₃₋₁₂-Cycloalkyl ausgewählt ist;
R²⁶ aus Wasserstoff, C₁₋₆-Alkyl und C₁₋₆-Alkoxy ausgewählt ist;
R²⁷ aus Wasserstoff, Halogen, C₁₋₆-Alkoxy, Halogen-C₁₋₆-alkoxy, C₁₋₆-Alkyl, Halogen-C₁₋₆-alkyl, Hydroxy-C₁₋₆-alkyl, Halogen, Hydroxy, C₁₋₆-Alkylsulfonyl, Carbamoyl, Cyano, Cycloalkyl-C₁₋₆-alkoxy- und Cycloalkyl ausgewählt ist;
R²⁸ aus Wasserstoff, C₁₋₆-Akyl und Halogen ausgewählt ist;
A C₆₋₁₄-Aryl oder C₁₋₁₃-Heteroaryl ist;
B C₆₋₁₄-Aryl oder C₁₋₁₃-Heteroaryl ist;
L² aus einer kovalenten Bindung, -O- und -CH₂- ausgewählt ist;
L³ aus einer kovalenten Bindung, -CH₂O- und -CH₂- ausgewählt ist; und
L^{3a} eine kovalente Bindung oder -CH₂- ist.

4. Verbindung der Formel (Ic) nach einem der Ansprüche 1 bis 4 oder ein pharmazeutisch unbedenkliches Salz davon, wobei:
L aus -CR¹R²-(CH₂)ₚ-, -OCR³R⁴-, -CR³R⁴O- und einer kovalenten Bindung ausgewählt ist;
p 0 ist;
R¹ aus Halogen, C₁₋₆-Alkyl, Hydroxy-C₁₋₆-alkyl, C₁₋₆-Alkoxy-C₁₋₆-alkyl-, Halogen-C₁₋₆-alkoxy und einer Gruppe 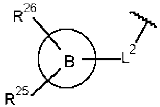 ausgewählt ist;
R² Wasserstoff oder Halogen ist;
R³ C₁₋₆-Alkyl ist;
R⁴ Wasserstoff ist;
R²¹ aus Halogen, C₁₋₆-Alkoxy, Halogen-C₁₋₆-alkoxy, C₁₋₆-Alkyl, Halogen-C₁₋₆-alkyl, SF₅, C₆₋₁₄-Aryl und einer Gruppe 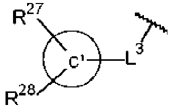 ausgewählt ist;
R²² aus Wasserstoff, Halogen, C₁₋₆-Alkoxy und Halogen-C₁₋₆-alkoxy ausgewählt ist;
R²³ Wasserstoff oder Halogen ist;
R²⁵ aus Wasserstoff, Halogen, C₁₋₆-Alkoxy und C₃₋₁₂-Cycloalkyl ausgewählt ist;
R²⁶ Wasserstoff oder C₁₋₆-Alkoxy ist;
R²⁷ aus Wasserstoff, Halogen-C₁₋₆-alkoxy, C₁₋₆-Alkyl, Halogen-C₁₋₆-alkyl, C₁₋₆-Alkyl-NH-C(O)- und Halogen ausgewählt ist;
R²⁸ aus Wasserstoff, C₁₋₆-Alkyl und Halogen ausgewählt ist;
A C₆₋₁₄-Aryl oder C₁₋₁₃-Heteroaryl ist;
B C₆₋₁₄-Aryl oder C₁₋₁₃-Heteroaryl ist;
C¹ C₃₋₁₂-Cycloalkyl oder C₂₋₉-Heterocyclyl ist;
L² eine kovalente Bindung ist; und
L³ eine kovalente Bindung oder -CH₂- ist.

5. Verbindung der Formel (Ic) nach einem der Ansprüche 1 bis 4 oder ein pharmazeutisch unbedenkliches Salz davon, wobei:
L aus -CR¹R²-(CH₂)ₚ-, -OCR³R⁴-, -CR³R⁴O- und einer kovalenten Bindung ausgewählt ist;
p 0 ist;
R¹ aus 2-Methoxyethyl, Methyl, 2,2,2-Trifluorethoxy, Fluor, 2-Hydroxyethyl und einer Gruppe 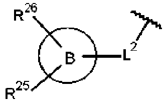 ausgewählt ist;
R² Wasserstoff oder Fluor ist;
R³ Methyl ist;
R⁴ Wasserstoff ist;
R²¹ aus Fluor, Chlor, Brom, Methyl, Methoxy, tert-Butyl, Propyl, Trifluormethoxy, 2-Fluorethoxy, 2,2,2-Trifluorethoxy, Trifluormethyl, 2,2,2-Trifluorethyl, 1,1-Difluorethyl, SF₅, Phenyl und einer Gruppe 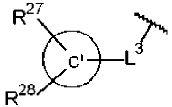 ausgewählt ist, wobei R²⁷, R²⁸, C¹ und L³ wie hierin definiert sind;
R²² aus Wasserstoff, Fluor, Chlor, Methoxy, Methyl und Trifluormethyl ausgewählt ist;
R²³ Wasserstoff oder Fluor ist;
R²⁵ aus Wasserstoff, Methoxy, Fluor und Cyclopropyl ausgewählt ist;
R²⁶ Wasserstoff oder Methoxy ist;
R²⁷ aus Methyl, Trifluormethoxy, Trifluormethyl, 2,2,2-Trifluor-1-methylethoxy, 2,2,2-Trifluor-1,1-dimethylethoxy, 2,2,2-Trifluorethoxy, Fluor und Chlor ausgewählt ist;
R²⁸ aus Wasserstoff, Methyl, Fluor und Chlor ausgewählt ist;
A aus Phenyl, Indol-3-yl, 2-Pyridyl und 3-Pyridyl ausgewählt ist;
B Phenyl oder 1,2,4-Oxadiazol-5-yl ist;
C¹ aus Azetidin-1-yl, Pyrrolidin-1-yl, Cyclopropyl und Oxetan-3-yl ausgewählt ist;
L² eine kovalente Bindung ist; und
L³ eine kovalente Bindung oder -CH₂- ist.

6. Verbindung der Formel (Ic) nach einem der Ansprüche 1 bis 3 oder ein pharmazeutisch unbedenkliches Salz davon, wobei C² Phenyl ist.

7. Verbindung der Formel (Ic) nach einem der Ansprüche 1 bis 3 oder ein pharmazeutisch unbedenkliches Salz davon, wobei L^{3a} eine kovalente Bindung ist.

8. Verbindung der Formel (Ic) nach Anspruch 1 oder ein pharmazeutisch unbedenkliches Salz davon, wobei:
m 0 ist, n 0 oder 1 ist und X CR²⁴ ist; oder
m 1 ist, n 1 ist und X CR²⁴ oder N ist;
L aus -CR¹R²-(CH₂)ₚ-, -OCHR³-, -CHR³O- und einer kovalenten Bindung ausgewählt ist;
p 0 oder 1 ist;
R¹ aus Halogen, C₁₋₆-Alkoxy, C₁₋₆-Alkyl, Halogen-C₁₋₆-alkyl, Hydroxy-C₁₋₆-alkyl, C₁₋₆-Alkoxy-C₁₋₆-alkyl-, Halogen-C₁₋₆-alkoxy, Halogen-C₁₋₆-alkoxy-C₁₋₆-alkyl-, Cyano und einer Gruppe 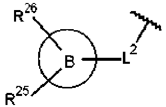 ausgewählt ist; und R² aus Wasserstoff, Halogen und Hydroxy ausgewählt ist; oder
R¹ und R² zusammen mit dem Kohlenstoffatom, an das sie gebunden sind, ein C₃₋₁₂-Cycloalkyl bilden;
R³ C₁₋₆-Alkyl oder Halogen-C₁₋₆-alkyl ist;
R²⁰ Wasserstoff oder C₁₋₆-Alkyl ist;
R²¹ aus Wasserstoff, Halogen, Hydroxy, C₁₋₆-Alkoxy, Halogen-C₁₋₆-alkoxy, C₁₋₆-Alkyl, Halogen-C₁₋₆-alkyl, Hydroxy-C₁₋₆-alkyl, (Halogen-C₁₋₆-alkyl)-hydroxy-C₁₋₆-alkyl, C₁₋₆-Alkoxycarbonyl-C₁₋₆-alkyl-, C₁₋₆-Alkoxycarbonyl-NH-C₁₋₆-alkoxy-, SF₅, (C₁₋₆-Alkyl)₃Si-O-C₁₋₆-alkyl-, einer Gruppe 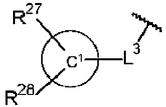 und einer Gruppe 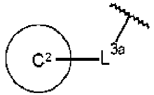 ausgewählt ist;
R²² aus Wasserstoff, Halogen, C₁₋₆-Alkoxy, Halogen-C₁₋₆-alkoxy, C₁₋₆-Alkyl und Cyano ausgewählt ist;
R²³ Wasserstoff oder Halogen ist;
R²⁴ aus Wasserstoff, Halogen, Hydroxy und C₁₋₆-Alkyl ausgewählt ist;
R²⁵ aus Wasserstoff, Halogen, C₁₋₆-Alkyl, C₁₋₆-Alkoxy, Halogen-C₁₋₆-alkyl, Halogen-C₁₋₆-alkoxy, C₁₋₆-Alkyl-SO₂- und C₃₋₁₂-Cycloalkyl ausgewählt ist;
R²⁶ aus Wasserstoff, C₁₋₆-Alkyl und C₁₋₆-Alkoxy ausgewählt ist;
R²⁷ aus Wasserstoff, Halogen, C₁₋₆-Alkoxy, Halogen-C₁₋₆-alkoxy, C₁₋₆-Alkyl, Halogen-C₁₋₆-alkyl, Hydroxy-C₁₋₆-alkyl, Halogen, Hydroxy, C₁₋₆-Alkylsulfonyl, Carbamoyl, Cyano, Cycloalkyl-C₁₋₆-alkoxy- und Cycloalkyl ausgewählt ist;
R²⁸ aus Wasserstoff, C₁₋₆-Alkyl und Halogen ausgewählt ist;
A C₆₋₁₄-Aryl oder C₁₋₁₃-Heteroaryl ist;
B C₆₋₁₄-Aryl oder C₁₋₁₃-Heteroaryl ist;
C¹ C₃₋₁₂-Cycloalkyl oder C₂₋₉-Heterocyclyl ist;
C² C₆₋₁₄-Aryl ist;
L² aus einer kovalenten Bindung, -O- und -CH₂- ausgewählt ist;
L³ aus einer kovalenten Bindung, -CH₂O- und -CH₂- ausgewählt ist; und
L^{3a} eine kovalente Bindung oder -CH₂- ist.

9. Verbindung der Formel (Ic) nach einem der Ansprüche 1 bis 8 oder ein pharmazeutisch unbedenkliches Salz davon, ausgewählt aus:
rac-(4aR,8aS)-6-(4-Benzhydrylpiperidin-1-carbonyl)-4,4a,5,7,8,8a-hexahydropyrido[4,3-b][1,4]oxazin-3-on;
rac-(4aR,8aS)-6-(4-(5-Chlor-1-(cyclopropylmethyl)-1H-indol-3-yl)piperidin-1-carbonyl)hexahydro-2H-pyrido[4,3-b][1,4]oxazin-3(4H)-on;
rac-(4aR,8aS)-6-(4-(5-Chlor-1-methyl-1H-indol-3-yl)piperidin-1-carbonyl)hexahydro-2H-pyrido[4,3-b][1,4]oxazin-3(4H)-on;
rac-(4aR,8aS)-6-(4-(9H-Fluoren-9-yl)piperidin-1-carbonyl)hexahydro-2H-pyrido[4,3-b][1,4]oxazin-3(4H)-on;
(+)-cis-6-[4-(6-Fluor-1H-indol-3-yl)piperidin-1-carbonyl]-4,4a,5,7,8,8a-hexahydropyrido[4,3-b][1,4]oxazin-3-on;
(4aR,8aS)-6-(4-(1-(4-Fluorphenyl)-3-methoxypropyl)piperidin-1-carbonyl)hexahydro-2H-pyrido[4,3-b][1,4]oxazin-3(4H)-on;
rac-(4aR,8aS)-6-[4-(6-Fluor-1H-indol-3-yl)piperidin-1-carbonyl]-4,4a,5,7,8,8a-hexahydropyrido[4,3-b][1,4]oxazin-3-on;
(4aR,8aS)-6-(4-((4-Fluorphenyl)(methoxy)methyl)piperidin-1-carbonyl)hexahydro-2H-pyrido[4,3-b] [1,4]oxazin-3(4H)-on;
rac-(4aR,8aS)-6-(4-(5-Fluorbenzo[d]isoxazol-3-yl)piperidin-1-carbonyl)hexahydro-2H-pyrido[4,3-b] [1,4]oxazin-3(4H)-on;
cis-6-(4-((4-Chlorphenyl)(phenyl)methyl)piperazin-1-carbonyl)hexahydro-2H-pyrido[4,3-b] [1,4]oxazin-3(4H)-on;
(+)-cis-6-(4-(Bis(4-fluorphenyl)methyl)piperazin-1-carbonyl)hexahydro-2H-pyrido[4,3-b][1,4]oxazin-3(4H)-on;
rac-(4aR,8aS)-6-[4-[Bis(4-fluorphenyl)methyl]piperazin-1-carbonyl]-4,4a,5,7,8,8a-hexahydropyrido[4,3-b][1,4]oxazin-3-on;
(-)-cis-6-(4-(Bis(4-fluorphenyl)methyl)piperazin-1-carbonyl)hexahydro-2H-pyrido[4,3-b][1,4]oxazin-3(4H)-on;
rac-(4aR,8aS)-6-(4-(5-Chlor-1H-indol-3-yl)piperazin-1-carbonyl)hexahydro-2H-pyrido[4,3-b] [1,4]oxazin-3(4H)-on;
rac-(4aR,8aS)-6-(4-(1-Phenylethyl)piperazin-1-carbonyl)hexahydro-2H-pyrido[4,3-b][1,4]oxazin-3(4H)-on;
rac-(4aR,8aS)-6-(4-(1-Methyl-1H-indazol-5-yl)piperidin-1-carbonyl)hexahydro-2H-pyrido[4,3-b] [1,4]oxazin-3(4H)-on;
rac-(4aR,8aS)-6-[4-[(4-Fluorphenyl)-phenylmethyl]piperazin-1-carbonyl]-4,4a,5,7,8,8a-hexahydropyrido[4,3-b][1,4]oxazin-3-on;
(+)- oder (-)-cis-6-(4-(5-Chlor-1-cyclopropyl-1H-indol-3-yl)piperidin-1-carbonyl)hexahydro-2H-pyrido[4,3-b][1,4]oxazin-3(4H)-on;
rac-(4aR,8aS)-6-(4-(5-Chlor-1-(1-chlor-3-hydroxypropan-2-yl)-1H-indol-3-yl)piperidin-1-carbonyl)hexahydro-2H-pyrido[4,3-b][1,4]oxazin-3(4H)-on;
(+)- oder (-)-cis-6-(4-(Bis(4-fluorphenyl)methyl)piperidin-1-carbonyl)hexahydro-2H-pyrido[4,3-b][1,4]oxazin-3(4H)-on;
(+)- oder (-)-cis-6-(4-(5-Chlor-1-(oxetan-3-yl)-1H-indol-3-yl)piperidin-1-carbonyl)hexahydro-2H-pyrido[4,3-b][1,4]oxazin-3(4H)-on;
(+)- oder (-)-cis-6-(4-(5-Chlor-1-(oxetan-3-ylmethyl)-1H-indol-3-yl)piperidin-1-carbonyl)hexahydro-2H-pyrido[4,3-b][1,4]oxazin-3(4H)-on;
(+)- oder (-)-cis-6-(4-(1-(2-((tert-Butyldimethylsilyl)oxy)ethyl)-5-chlor-1H-indol-3-yl)piperidin-1-carbonyl)hexahydro-2H-pyrido[4,3-b][1,4]oxazin-3(4H)-on;
(+)- oder (-)-cis-6-(4-(5-Chlor-1-(2-hydroxyethyl)-1H-indol-3-yl)piperidin-1-carbonyl)hexahydro-2H-pyrido[4,3-b][1,4]oxazin-3(4H)-on;
rac-cis-6-(4-(1-(4-Fluorphenyl)-3-hydroxypropyl)piperidin-1-carbonyl)hexahydro-2H-pyrido[4,3-b][1,4]oxazin-3(4H)-on;
rac-(4aR,8aS)-6-(4-(1-(4-(Trifluormethyl)phenyl)ethyl)piperidin-1-carbonyl)hexahydro-2H-pyrido[4,3-b][1,4]oxazin-3(4H)-on;
rac-(4aR,8aS)-6-(4-(1-(2-Chlor-4-fluorphenoxy)ethyl)piperidin-1-carbonyl)hexahydro-2H-pyrido[4,3-b][1,4]oxazin-3(4H)-on;
(4aR,8aS)-6-(4-(5-(Trifluormethyl)pyridin-3-yl)piperidin-1-carbonyl)hexahydro-2H-pyrido[4,3-b][1,4]oxazin-3(4H)-on;
(4aR,8aS)-6-(4-(Difluor(4-(trifluormethyl)phenyl)methyl)piperidin-1-carbonyl)hexahydro-2H-pyrido[4,3-b][1,4]oxazin-3(4H)-on;
(+)-(4aR,8aS)-6-(4-((R oder S)-1-(4-F'luorphenyl)-3-hydroxypropyl)piperidin-1-carbonyl)hexahydro-2H-pyrido[4,3-b][1,4]oxazin-3(4H)-on;
(-)-(4aR,8aS)-6-(4-((S oder R)-1-(4-Fluorphenyl)-3-hydroxypropyl)piperidin-l-carbonyl)hexahydro-2H-pyrido[4,3-b][1,4]oxazin-3(4H)-on;
(+)- oder (-)-cis-6-(4-((S oder R)-1-(2-Chlor-4-fluorphenoxy)ethyl)piperidin-1-carbonyl)hexahydro-2H-pyrido[4,3-b][1,4]oxazin-3(4H)-on;
(+)- oder (-)-cis-6-(4-((R oder S)-1-(2-Chlor-4-fluorphenoxy)ethyl)piperidin-1-carbonyl)hexahydro-2H-pyrido[4,3-b][1,4]oxazin-3(4H)-on;
(+)- oder (-)-cis-6-(4-(5-Methoxypyridin-3-yl)piperidin-1-carbonyl)hexahydro-2H-pyrido[4,3-b] [1,4]oxazin-3(4H)-on;
(+)- oder (-)-cis-6-(4-(Bis(4-fluorphenyl)methyl)piperidin-1-carbonyl)hexahydro-2H-pyrido[4,3-b][1,4]oxazin-3(4H)-on;
(+)- oder (-)-cis-6-(4-((4-Chlorphenyl)difluormethyl)piperidin-1-carbonyl)hexahydro-2H-pyrido[4,3-b][1,4]oxazin-3(4H)-on;
(+)- oder (-)-cis-6-(4-(5-(Trifluormethoxy)pyridin-2-yl)piperidin-1-carbonyl)hexahydro-2H-pyrido[4,3-b][1,4]oxazin-3(4H)-on;
(+)- oder (-)-cis-6-(4-((2-Chlor-4-fluorphenyl)difluormethyl)piperidin-1-carbonyl)hexahydro-2H-pyrido[4,3-b][1,4]oxazin-3(4H)-on;
(+)- oder (-)-cis-6-(4-(5-Ethylpyridin-3-yl)piperidin-1-carbonyl)hexahydro-2H-pyrido[4,3-b][1,4]oxazin-3(4H)-on;
(+)- oder (-)-cis-6-(4-(5-(1,1-Difluorethyl)pyridin-2-yl)piperidin-1-carbonyl)hexahydro-2H-pyrido[4,3-b][1,4]oxazin-3(4H)-on;
(+)- oder (-)-cis-6-(4-(6-Chlor-1-methyl-1H-indazol-3-yl)piperidin-1-carbonyl)hexahydro-2H-pyrido[4,3-b][1,4]oxazin-3(4H)-on;
(4aR,8aS)-6-((3R oder 3S)-4-(5-(1,1-Difluorethyl)pyridin-2-yl)-3-methylpiperidin-1-carbonyl)hexahydro-2H-pyrido[4,3-b][1,4]oxazin-3(4H)-on;
(4aR,8aS)-6-((3S oder 3R)-4-(5-(1,1-Difluorethyl)pyridin-2-yl)-3-methylpiperidin-1-carbonyl)hexahydro-2H-pyrido[4,3-b][1,4]oxazin-3(4H)-on;
(4aR,8aS)-6-((4R oder 4S)-(5-(1,1-Difluorethyl)pyridin-2-yl)-3-methylpiperidin-1-carbonyl)hexahydro-2H-pyrido[4,3-b][1,4]oxazin-3(4H)-on;
(4aR,8aS)-6-(4-((R oder S)-1-(4-(Trifluormethyl)phenyl)ethyl)piperidin-1-carbonyl)hexahydro-2H-pyrido[4,3-b][1,4]oxazin-3(4H)-on;
(4aR,8aS)-6-(4-((S oder R)-1-(4-(Trifluormethyl)phenyl)ethyl)piperidin-1-carbonyl)hexahydro-2H-pyrido[4,3-b][1,4]oxazin-3(4H)-on;
2-(4-Fluorphenyl)-2-(1-((4aR,8aS)-3-oxooctahydro-2H-pyrido[4,3-b][1,4]oxazin-6-carbonyl)piperidin-4-yl)acetonitril;
(4aR,8aS)-6-(4-(2,2,2-Trifluor-1-phenylethyl)piperazin-1-carbonyl)hexahydro-2H-pyrido[4,3-b] [1,4]oxazin-3(4H)-on;
(4aR,8aS)-6-(4-((4-Fluorphenyl)(2,2,2-trifluorethoxy)methyl)piperidin-1-carbonyl)hexahydro-2H-pyrido[4,3-b][1,4]oxazin-3(4H)-on;
(4aR,8aS)-6-(4-(1-(4-Fluorphenyl)-2-(3-methyl-1,2,4-oxadiazol-5-yl)ethyl)piperidin-1-carbonyl)hexahydro-2H-pyrido[4,3-b][1,4]oxazin-3(4H)-on;
(4aR,8aS)-6-(4-(1-(4-(Trifluormethyl)phenyl)cyclopropyl)piperidin-1-carbonyl)hexahydro-2H-pyrido[4,3-b][1,4]oxazin-3(4H)-on;
(4aR,8aS)-6-(4-(1-(2,4-Difluorphenyl)-2,2,2-trifluorethyl)piperazin-1-carbonyl)hexahydro-2H-pyrido[4,3-b][1,4]oxazin-3(4H)-on;
(4aR,8aS)-6-(3-(Bis(4-fluorphenyl)(hydroxy)methyl)azetidin-1-carbonyl)hexahydro-2H-pyrido[4,3-b][1,4]oxazin-3(4H)-on;
(4aR,8aS)-6-(3-(Fluorbis(4-fluorphenyl)methyl)azetidin-1-carbonyl)hexahydro-2H-pyrido[4,3-b] [1,4]oxazin-3(4H)-on;
(4aR,8aS)-6-(4-(1-(4-Fluorphenyl)-2-(2,2,2-trifluorethoxy)ethyl)piperidin-1-carbonyl)hexahydro-2H-pyrido[4,3-b][1,4]oxazin-3(4H)-on;
(4aR,8aS)-6-(4-((R oder S)-(4-Fluorphenyl)(2,2,2-trifluorethoxy)methyl)piperidin-1-carbonyl)hexahydro-2H-pyrido[4,3-b][1,4]oxazin-3(4H)-on;
(4aR,8aS)-6-(4-((S oder R)-(4-Fluorphenyl)(2,2,2-trifluorethoxy)methyl)piperidin-1-carbonyl)hexahydro-2H-pyrido[4,3-b][1,4]oxazin-3(4H)-on;
(4aR,8aS)-6-(4-((R oder S)-1-(4-Fluorphenyl)-2-(3-methyl-1,2,4-oxadiazol-5-yl)ethyl)piperidin-1-carbonyl)hexahydro-2H-pyrido[4,3-b][1,4]oxazin-3(4H)-on;
(4aR,8aS)-6-(4-((S oder R)-1-(4-Fluorphenyl)-2-(3-methyl-1,2,4-oxadiazol-5-yl)ethyl)piperidin-1-carbonyl)hexahydro-2H-pyrido[4,3-b][1,4]oxazin-3(4H)-on;
(4aR,8aS)-6-(4-(2-Cyclopropylpyridin-4-yl)piperidin-1-carbonyl)hexahydro-2H-pyrido[4,3-b] [1,4]oxazin-3(4H)-on;
(4aR,8aS)-6-[4-[(S oder R)-(4-Fluorphenyl)-(3-methoxyphenyl)methyl]piperidin-1-carbonyl]-4,4a,5,7,8,8a-hexahydropyrido[4,3-b][1,4]oxazin-3-on;
(4aR,8aS)-6-(4-((R oder S)-(4-Fluorphenyl)(3-methoxyphenyl)methyl)piperidin-1-carbonyl)hexahydro-2H-pyrido[4,3-b][1,4]oxazin-3(4H)-on;
(4aR,8aS)-6-(4-((S oder R)-(3-Methoxyphenyl)(phenyl)methyl)piperidin-1-carbonyl)hexahydro-2H-pyrido[4,3-b][1,4]oxazin-3(4H)-on;
(4aR,8aS)-6-(4-((R oder S)-(3-Methoxyphenyl)(phenyl)methyl)piperidin-1-carbonyl)hexahydro-2H-pyrido[4,3-b][1,4]oxazin-3(4H)-on;
(4aR,8aS)-6-(4-((S oder R)-(3-(2-Fluorethoxy)phenyl)(phenyl)methyl)piperidin-1-carbonyl)hexahydro-2H-pyrido[4,3-b][1,4]oxazin-3(4H)-on;
(4aR,8aS)-6-(4-((S)-(4-Fluorphenyl)(phenyl)methyl)piperidin-1-carbonyl)hexahydro-2H-pyrido[4,3-b][1,4]oxazin-3(4H)-on;
(4aR,8aS)-6-(4-((S oder R)-(4-Fluorphenyl)(phenyl)methyl)piperidin-1-carbonyl)hexahydro-2H-pyrido[4,3-b][1,4]oxazin-3(4H)-on;
(4aR,8aS)-6-[4-[(S oder R)-(4-Fluorphenyl)-(4-methylphenyl)methyl]piperidin-1-carbonyl]-4,4a,5,7,8,8a-hexahydropyrido[4,3-b][1,4]oxazin-3-on;
(4aR,8aS)-6-[4-[(R oder S)-[4-(2-Fluorethoxy)phenyl]-phenylmethyl]piperidin-1-carbonyl]-4,4a,5,7,8,8a-hexahydropyrido[4,3-b][1,4]oxazin-3-on;
(4aR,8aS)-6-(4-((S oder R)-(4-Fluorphenyl)(4-methoxyphenyl)methyl)piperidin-1-carbonyl)hexahydro-2H-pyrido[4,3-b][1,4]oxazin-3(4H)-on;
(4aR,8aS)-6-(4-((R oder S)-(4-Fluorphenyl)(p-tolyl)methyl)piperidin-1-carbonyl)hexahydro-2H-pyrido[4,3-b][1,4]oxazin-3(4H)-on;
(4aR,8aS)-6-(4-((S oder R)-(3,4-Dimethoxyphenyl)(4-fluorphenyl)methyl)piperidin-1-carbonyl)hexahydro-2H-pyrido[4,3-b][1,4]oxazin-3(4H)-on;
(4aR,8aS)-6-(4-((R oder S)-(3,4-Dimethoxyphenyl)(phenyl)methyl)piperidin-1-carbonyl)hexahydro-2H-pyrido[4,3-b][1,4]oxazin-3 (4H)-on;
(4aR,8aS)-6-(3-(1-(2-Chlor-4-(trifluormethyl)phenoxy)ethyl)azetidin-1-carbonyl)hexahydro-2H-pyrido[4,3-b][1,4]oxazin-3 (4H)-on;
(4aR,8aS)-6-(3-(1-(2-Chlor-4-(trifluormethyl)phenoxy)ethyl)azetidin-1-carbonyl)hexahydro-2H-pyrido[4,3-b][1,4]oxazin-3 (4H)-on;
(4aR,8aS)-6-(4-((R oder S)-(4-Fluorphenyl)(phenyl)methyl)piperidin-1-carbonyl)hexahydro-2H-pyrido[4,3-b][1,4]oxazin-3 (4H)-on;
(4aR,8aS)-6-(4-((S oder R)-Phenyl(m-tolyl)methyl)piperidin-1-carbonyl)hexahydro-2H-pyrido[4,3-b] [1,4]oxazin-3(4H)-on;
(4aR,8aS)-6-[4-[(S oder R)-[4-(2-Fluorethoxy)phenyl]-phenylmethyl]piperidin-1-carbonyl]-4,4a,5,7,8,8a-hexahydropyrido[4,3-b][1,4]oxazin-3-on;
(4aR,8aS)-6-(4-((S oder R)-((S)-3-Methylcyclohexa-2,4-dien-1-yl)(phenyl)methyl)piperidin-1-carbonyl)hexahydro-2H-pyrido[4,3-b][1,4]oxazin-3(4H)-on;
(4aR,8aS)-6-(4-(1-(2-Chlor-4-fluorphenoxy)-2,2,2-trifluorethyl)piperidin-1-carbonyl)hexahydro-2H-pyrido[4,3-b][1,4]oxazin-3(4H)-on;
(4aR,8aS)-6-(3-(Bis(4-fluorphenyl)methyl)azetidin-1-carbonyl)hexahydro-2H-pyrido[4,3-b] [1,4]oxazin-3(4H)-on;
(4aR,8aS)-6-[3-[1-[4-(Trifluormethyl)phenyl]ethyl]azetidin-1-carbonyl]-4,4a,5,7,8,8a-hexahydropyrido[4,3-b][1,4]oxazin-3-on;
(4aR,8aS)-6-[4-[(S oder R)-Phenyl(p-tolyl)methyl]piperidin-1-carbonyl]-4,4a,5,7,8,8a-hexahydropyrido[4,3-b][1,4]oxazin-3-on;
(4aR,8aS)-6-(4-((R oder S)-Phenyl(p-tolyl)methyl)piperidin-1-carbonyl)hexahydro-2H-pyrido[4,3-b][1,4]oxazin-3(4H)-on;
(4aR,8aS)-6-(3-Benzhydrylazetidin-1-carbonyl)hexahydro-2H-pyrido[4,3-b][1,4]oxazin-3(4H)-on;
(4aR,8aS)-6-(4-((S oder R)-(3,4-Dimethoxyphenyl)(phenyl)methyl)piperidin-1-carbonyl)hexahydro-2H-pyrido[4,3-b] [1,4]oxazin-3 (4H)-on;
(4aR,8aS)-6-(4-((R oder S)-(3,4-Dimethoxyphenyl)(4-fluorphenyl)methyl)piperidin-1-carbonyl)hexahydro-2H-pyrido[4,3-b][1,4]oxazin-3(4H)-on;
(4aR,8aS)-6-(4-((R oder S)-(4-Fluorphenyl)(4-methoxyphenyl)methyl)piperidin-1-carbonyl)hexahydro-2H-pyrido[4,3-b][1,4]oxazin-3(4H)-on;
(4aR,8aS)-6-[3-[1-[4-(Trifluormethyl)phenyl]ethoxy]azetidin-1-carbonyl]-4,4a,5,7,8,8a-hexahydropyrido[4,3-b][1,4]oxazin-3-on;
(4aR,8aS)-6-(3-(1-(2-Fluor-4-(trifluormethyl)phenoxy)ethyl)azetidin-1-carbonyl)hexahydro-2H-pyrido[4,3-b][1,4]oxazin-3(4H)-on;
(4aR,8aS)-6-(3-(1-(4-(Trifluormethyl)phenoxy)ethyl)azetidin-1-carbonyl)hexahydro-2H-pyrido[4,3-b][1,4]oxazin-3(4H)-on;
(4aR,8aS)-6-[4-[1-[4-(Trifluormethyl)phenyl]ethoxy]piperidin-1-carbonyl]-4,4a,5,7,8,8a-hexahydropyrido[4,3-b][1,4]oxazin-3-on;
(4aR,8aS)-6-(4-((R oder S)-(3-Cyclopropyl-1,2,4-oxadiazol-5-yl)(4-fluorphenyl)methyl)piperidin-1-carbonyl)hexahydro-2H-pyrido[4,3-b][1,4]oxazin-3 (4H)-on;
(4aR,8aS)-6-(4-((S oder R)-(3-Cyclopropyl-1,2,4-oxadiazol-5-yl)(4-fluorphenyl)methyl)piperidin-1-carbonyl)hexahydro-2H-pyrido[4,3-b][1,4]oxazin-3 (4H)-on;
(4aR,8aS)-6-(3-((R oder S)-1-(2-Fluor-4-(trifluormethyl)phenoxy)ethyl)azetidin-1-carbonyl)hexahydro-2H-pyrido[4,3-b][1,4]oxazin-3(4H)-on;
(4aR,8aS)-6-(3-((S oder R)-1-(2-Fluor-4-(trifluormethyl)phenoxy)ethyl)azetidin-1-carbonyl)hexahydro-2H-pyrido[4,3-b][1,4]oxazin-3(4H)-on;
(4aR,8aS)-6-(3-((R oder S)-1-(4-(Trifluormethyl)phenoxy)ethyl)azetidin-1-carbonyl)hexahydro-2H-pyrido[4,3-b][1,4]oxazin-3(4H)-on;
(4aR,8aS)-6-(3-((S oder R)-1-(4-(Trifluormethyl)phenoxy)ethyl)azetidin-1-carbonyl)hexahydro-2H-pyrido[4,3-b][1,4]oxazin-3(4H)-on;
(4aR,8aS)-6-(4-((R oder S)-(4-(2-Fluorethoxy)phenyl)(4-fluorphenyl)methyl)piperidin-1-carbonyl)hexahydro-2H-pyrido[4,3-b][1,4]oxazin-3(4H)-on;
(4aR,8aS)-6-(4-(5-Methyl-6-(trifluormethyl)pyridin-3-yl)piperidin-1-carbonyl)hexahydro-2H-pyrido[4,3-b][1,4]oxazin-3(4H)-on;
(4aR,8aS)-6-(4-((4-Fluorphenyl)(3-(trifluormethyl)-1,2,4-oxadiazol-5-yl)methyl)piperidin-1-carbonyl)hexahydro-2H-pyrido[4,3-b][1,4]oxazin-3(4H)-on;
(4aR,8aS)-6-(3-((S oder R)-1-(4-(Trifluormethyl)phenyl)ethyl)azetidin-1-carbonyl)hexahydro-2H-pyrido[4,3-b][1,4]oxazin-3(4H)-on;
(4aR,8aS)-6-(3-((R oder S)-1-(4-(Trifluormethyl)phenyl)ethyl)azetidin-1-carbonyl)hexahydro-2H-pyrido[4,3-b][1,4]oxazin-3(4H)-on;
(4aR,8aS)-6-[3-(S oder R)-[(4-Fluorphenyl)-(2,2,2-trifluorethoxy)methyl]azetidin-1-carbonyl]-4,4a,5,7,8,8a-hexahydropyrido[4,3-b][1,4]oxazin-3-on;
(4aR,8aS)-6-[3-(R oder S)-[(4-Fluorphenyl)-(2,2,2-trifluorethoxy)methyl]azetidin-1-carbonyl]-4,4a,5,7,8,8a-hexahydropyrido[4,3-b][1,4]oxazin-3-on;
(4aR,8aS)-6-(4-(5,6,7,8-Tetrahydrochinolin-4-yl)piperidin-1-carbonyl)hexahydro-2H-pyrido[4,3-b][1,4]oxazin-3(4H)-on;
(4aR,8aS)-6-(4-((R oder S)-(3-(2-Fluorethoxy)phenyl)(phenyl)methyl)piperidin-1-carbonyl)hexahydro-2H-pyrido[4,3-b][1,4]oxazin-3(4H)-on;
(4aR,8aS)-6-(4-((S oder R)-(4-(2-Fluorethoxy)phenyl)(4-fluorphenyl)methyl)piperidin-1-carbonyl)hexahydro-2H-pyrido[4,3-b][1,4]oxazin-3(4H)-on;
(4aR,8aS)-6-(3-(4-Bromphenyl)azetidin-1-carbonyl)hexahydro-2H-pyrido[4,3-b][1,4]oxazin-3(4H)-on;
(4aR,8aS)-6-(3-(4'-Chlor-[1,1'-biphenyl]-4-yl)azetidin-1-carbonyl)hexahydro-2H-pyrido[4,3-b][1,4]oxazin-3(4H)-on;
(4aR,8aS)-6-(3-(2'-Chlor-[1,1'-biphenyl]-4-yl)azetidin-1-carbonyl)hexahydro-2H-pyrido[4,3-b] [1,4]oxazin-3(4H)-on;
(4aR,8aS)-6-(3-(2',4'-Dichlor-[1,1'-biphenyl]-4-yl)azetidin-1-carbonyl)hexahydro-2H-pyrido[4,3-b] [1,4]oxazin-3(4H)-on;
(4aR,8aS)-6-(3-(1-(R oder S)-(4-(Trifluormethyl)phenyl)ethoxy)azetidin-1-carbonyl)hexahydro-2H-pyrido[4,3-b][1,4]oxazin-3 (4H)-on;
(4aR,8aS)-6-(3-(1-(S oder R)-(4-(Trifluormethyl)phenyl)ethoxy)azetidin-1-carbonyl)hexahydro-2H-pyrido[4,3-b][1,4]oxazin-3 (4H)-on;
(4aR,8aS)-6-[3-(S oder R)-[1-(2-Chlor-4-fluorphenyl)ethoxy]azetidin-1-carbonyl]-4,4a,5,7,8,8a-hexahydropyrido[4,3-b][1,4]oxazin-3-on;
(4aR,8aS)-6-[3-(R oder S)-[1-(2-Chlor-4-fluorphenyl)ethoxy]azetidin-1-carbonyl]-4,4a,5,7,8,8a-hexahydropyrido[4,3-b][1,4]oxazin-3-on;
(4aR,8aS)-6-[4-[(R oder S)-(3,4-Dimethoxyphenyl)-(2-pyridyl)methyl]piperidin-1-carbonyl]-4,4a,5,7,8,8a-hexahydropyrido[4,3-b][1,4]oxazin-3-on;
(4aR,8aS)-6-[4-[(S oder R)-(3,4-Dimethoxyphenyl)-(2-pyridyl)methyl]piperidin-1-carbonyl]-4,4a,5,7,8,8a-hexahydropyrido[4,3-b][1,4]oxazin-3-on;
(4aR,8aS)-6-[3-[4-(Trifluormethoxy)phenyl]azetidin-1-carbonyl]-4,4a,5,7,8,8a-hexahydropyrido[4,3-b][1,4]oxazin-3-on;
(4aR,8aS)-6-(3-(2'-(Trifluormethoxy)-[1,1'-biphenyl]-4-yl)azetidin-1-carbonyl)hexahydro-2H-pyrido[4,3-b][1,4]oxazin-3 (4H)-on;
(4aR,8aS)-6-(3-(4-Bromphenyl)-3-fluorazetidin-1-carbonyl)hexahydro-2H-pyrido[4,3-b][1,4]oxazin-3 (4H)-on;
(4aR,8aS)-6-(3-(4-Bromphenyl)-3-hydroxyazetidin-1-carbonyl)hexahydro-2H-pyrido[4,3-b] [1,4]oxazin-3(4H)-on;
(4aR,8aS)-6-(3-(4-Bromphenyl)-3-methylazetidin-1-carbonyl)hexahydro-2H-pyrido[4,3-b] [1,4]oxazin-3(4H)-on;
(4aR,8aS)-6-(3-(2'-(Trifluormethyl)-[1,1'-biphenyl]-4-yl)azetidin-1-carbonyl)hexahydro-2H-pyrido[4,3-b][1,4]oxazin-3(4H)-on;
(4aR,8aS)-6-(3-(2',4'-Difluor-[1,1'-biphenyl]-4-yl)azetidin-1-carbonyl)hexahydro-2H-pyrido[4,3-b] [1,4]oxazin-3(4H)-on;
4'-(1-((4aR,8aS)-3-Oxooctahydro-2H-pyrido[4,3-b] [1,4]oxazin-6-carbonyl)azetidin-3-yl)-[1,1'-biphenyl]-2-carbonitril;
(4aR,8aS)-6-(3-(4-(3-Methoxyazetidin-1-yl)phenyl)azetidin-1-carbonyl)hexahydro-2H-pyrido[4,3-b] [1,4]oxazin-3(4H)-on;
(4aR,8aS)-6-(3-(4-(3-(Trifluormethyl)azetidin-1-yl)phenyl)azetidin-1-carbonyl)hexahydro-2H-pyrido[4,3-b][1,4]oxazin-3(4H)-on;
2-[4-[1-[(4aR,8aS)-3-Oxo-4,4a,5,7,8,8a-hexahydropyrido[4,3-b][1,4]oxazin-6-carbonyl]azetidin-3-yl]phenyl]benzamid;
(4aR,8aS)-6-(3-(2-Chlor-[1,1'-biphenyl]-4-yl)azetidin-1-carbonyl)hexahydro-2H-pyrido[4,3-b] [1,4]oxazin-3(4H)-on;
(4aR,8aS)-6-(3-(4-Brom-3-chlorphenyl)azetidin-1-carbonyl)hexahydro-2H-pyrido[4,3-b][1,4]oxazin-3(4H)-on;
(4aR,8aS)-6-[3-[4-(4-Chlor-2-fluorphenyl)phenyl]azetidin-1-carbonyl]-4,4a,5,7,8,8a-hexahydropyrido[4,3-b][1,4]oxazin-3-on;
(4aR,8aS)-6-[3-[4-(2-Chlor-4-fluorphenyl)phenyl]azetidin-1-carbonyl]-4,4a,5,7,8,8a-hexahydropyrido[4,3-b][1,4]oxazin-3-on;
(4aR,8aS)-6-[4-[(R oder S)-(3,4-Dimethoxyphenyl)-(3-pyridyl)methyl]piperidin-1-carbonyl]-4,4a,5,7,8,8a-hexahydropyrido[4,3-b][1,4]oxazin-3-on;
(4aR,8aS)-6-[4-[(S oder R)-(3,4-Dimethoxyphenyl)-(3-pyridyl)methyl]piperidin-1-carbonyl]-4,4a,5,7,8,8a-hexahydropyrido[4,3-b][1,4]oxazin-3-on;
(4aR,8aS)-6-(3-(2'-Methyl-[1,1'-biphenyl]-4-yl)azetidin-1-carbonyl)hexahydro-2H-pyrido[4,3-b] [1,4]oxazin-3(4H)-on;
(4aR,8aS)-6-(3-(4-(3-((1,1,1-Trifluorpropan-2-yl)oxy)azetidin-1-yl)phenyl)azetidin-1-carbonyl)hexahydro-2H-pyrido[4,3-b][1,4]oxazin-3(4H)-on;
(4aR,8aS)-6-(3-(3-Bromphenyl)azetidin-1-carbonyl)hexahydro-2H-pyrido[4,3-b][1,4]oxazin-3 (4H)-on;
(4aR,8aS)-6-(3-(4-(tert-Butyl)phenyl)azetidin-1-carbonyl)hexahydro-2H-pyrido[4,3-b][1,4]oxazin-3(4H)-on;
(4aR,8aS)-6-[3-(4-Phenylphenyl)azetidin-1-carbonyl]-4,4a,5,7,8,8a-hexahydropyrido[4,3-b][1,4]oxazin-3-on;
(4aR,8aS)-6-[3-[4-[2-(Difluormethyl)phenyl]phenyl]azetidin-1-carbonyl]-4,4a,5,7,8,8a-hexahydropyrido[4,3-b][1,4]oxazin-3-on;
(4aR,8aS)-6-(3-(3'-Methyl-[1,1'-biphenyl]-4-yl)azetidin-1-carbonyl)hexahydro-2H-pyrido[4,3-b] [1,4]oxazin-3(4H)-on;
(4aR,8aS)-6-(3-(4' -Methyl-[1,1'-biphenyl]-4-yl)azetidin-1-carbonyl)hexahydro-2H-pyrido[4,3-b] [1,4]oxazin-3(4H)-on;
(4aR,8aS)-6-(3-(6-Chlorpyridin-3-yl)azetidin-1-carbonyl)hexahydro-2H-pyrido[4,3-b][1,4]oxazin-3(4H)-on;
(4aR,8aS)-6-(3-(4-(Trifluormethyl)phenyl)azetidin-1-carbonyl)hexahydro-2H-pyrido[4,3-b] [1,4]oxazin-3(4H)-on;
(4aR,8aS)-6-(3-(4-(3-((1,1,1-Trifluor-2-methylpropan-2-yl)oxy)azetidin-1-yl)phenyl)azetidin-1-carbonyl)hexahydro-2H-pyrido[4,3-b][1,4]oxazin-3(4H)-on;
(4aR,8aS)-6-(3-(4-(1,1-Difluorethyl)phenyl)azetidin-1-carbonyl)hexahydro-2H-pyrido[4,3-b] [1,4]oxazin-3(4H)-on;
(4aR,8aS)-6-[4-[(R oder S)-(3,4-Dimethoxyphenyl)-(4-pyridyl)methyl]piperidin-1-carbonyl]-4,4a,5,7,8,8a-hexahydropyrido[4,3-b][1,4]oxazin-3-on;
(4aR,8aS)-6-[4-[(S oder R)-(3,4-Dimethoxyphenyl)-(4-pyridyl)methyl]piperidin-1-carbonyl]-4,4a,5,7,8,8a-hexahydropyrido[4,3-b][1,4]oxazin-3-on;
(4aR,8aS)-6-(3-(4-(3-Methylazetidin-1-yl)phenyl)azetidin-1-carbonyl)hexahydro-2H-pyrido[4,3-b] [1,4]oxazin-3(4H)-on;
(4aR,8aS)-6-(3-(4-(3,3-Dimethylpyrrolidin-1-yl)phenyl)azetidin-1-carbonyl)hexahydro-2H-pyrido[4,3-b][1,4]oxazin-3(4H)-on;
(4aR,8aS)-6-(3-(4-(3-(Trifluormethoxy)azetidin-1-yl)phenyl)azetidin-1-carbonyl)hexahydro-2H-pyrido[4,3-b][1,4]oxazin-3(4H)-on;
(4aR,8aS)-6-(3-(6-(2,4-Dichlorphenyl)pyridin-3-yl)azetidin-1-carbonyl)hexahydro-2H-pyrido[4,3-b] [1,4]oxazin-3(4H)-on;
(4aR,8aS)-6-(3-(2'-Chlor-4'-(methylsulfonyl)-[1,1'-biphenyl]-4-yl)azetidin-1-carbonyl)hexahydro-2H-pyrido[4,3-b][1,4]oxazin-3(4H)-on;
(4aR,8aS)-6-(3-(4-(3,3-Difluorazetidin-1-yl)phenyl)azetidin-1-carbonyl)hexahydro-2H-pyrido[4,3-b] [1,4]oxazin-3(4H)-on;
(4aR,8aS)-6-(4-(4-Bromphenyl)piperidin-1-carbonyl)hexahydro-2H-pyrido[4,3-b][1,4]oxazin-3(4H)-on;
(4aR,8aS)-6-[3-[4-(2,2,2-Trifluorethoxy)phenyl]azetidin-1-carbonyl]-4,4a,5,7,8,8a-hexahydropyrido[4,3-b][1,4]oxazin-3-on;
2-[4-[1-[(4aR,8aS)-3-Oxo-4,4a,5,7,8,8a-hexahydropyrido[4,3-b][1,4]oxazin-6-carbonyl] azetidin-3-yl]phenyl]-5-chlorbenzonitril;
(4aR,8aS)-6-[3-[4-[3-[(1S oder 1R)-2,2,2-Trifluor-1-methylethoxy]azetidin-1-yl]phenyl]azetidin-1-carbonyl]-4,4a,5,7,8,8a-hexahydropyrido[4,3-b] [1,4]oxazin-3-on;
(4aR,8aS)-6-[3-[4-[3-[(1R oder 1S)-2,2,2-Trifluor-1-methylethoxy]azetidin-1-yl]phenyl]azetidin-1-carbonyl]-4,4a,5,7,8,8a-hexahydropyrido[4,3-b] [1,4]oxazin-3-on;
(4aR,8aS)-6-(3-(4-(2-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(4-(3-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(3-Bromphenyl)pyrrolidin-1-carbonyl)hexahydro-2H-pyrido[4,3-b][1,4]oxazin-3(4H)-on;
(4aR,8aS)-6-[3-[4-(2-Ethylpyrrolidin-1-yl)phenyl]azetidin-1-carbonyl]-4,4a,5,7,8,8a-hexahydropyrido[4,3-b][1,4]oxazin-3-on;
2-[4-[1-[(4aR,8aS)-3-Oxo-4,4a,5,7,8,8a-hexahydropyrido[4,3-b][1,4]oxazin-6-carbonyl] azetidin-3-yl]phenyl]-3-chlorbenzonitril;
(4aR,8aS)-6-(3-(4-(2-Cyclopropylpyrrolidin-1-yl)phenyl)azetidin-1-carbonyl)hexahydro-2H-pyrido[4,3-b][1,4]oxazin-3(4H)-on;
(4aR,8aS)-6-(4-(4-Bromphenyl)piperazin-1-carbonyl)hexahydro-2H-pyrido[4,3-b][1,4]oxazin-3(4H)-on;
(4aR,8aS)-6-(4-(2',4'-Dichlor-[1,1'-biphenyl]-4-yl)piperidin-1-carbonyl)hexahydro-2H-pyrido[4,3-b] [1,4]oxazin-3(4H)-on;
(4aR,8aS)-6-(3-(4-(3-Azabicyclo[3.1.0]hexan-3-yl)phenyl)azetidin-1-carbonyl)hexahydro-2H-pyrido[4,3-b][1,4]oxazin-3(4H)-on;
(4aR,8aS)-6-(3-(3-(Trifluormethoxy)phenyl)azetidin-1-carbonyl)hexahydro-2H-pyrido[4,3-b] [1,4]oxazin-3(4H)-on;
(4aR,8aS)-6-(3-(4-(3-(2,2,2-Trifluorethoxy)azetidin-1-yl)phenyl)azetidin-1-carbonyl)hexahydro-2H-pyrido[4,3-b][1,4]oxazin-3(4H)-on;
(4aR,8aS)-6-(3-(4'-Chlor-2'-(methylsulfonyl)-[1,1'-biphenyl]-4-yl)azetidin-1-carbonyl)hexahydro-2H-pyrido[4,3-b][1,4]oxazin-3(4H)-on;
(4aR,8aS)-6-(3-(6-(2-(Trifluormethyl)pyrrolidin-1-yl)pyridin-3-yl)azetidin-1-carbonyl)hexahydro-2H-pyrido[4,3-b][1,4]oxazin-3(4H)-on;
(4aR,8aS)-6-[4-[(R oder S)-(3-Methylsulfonylphenyl)-(3-pyridyl)methyl]piperidin-1-carbonyl]-4,4a,5,7,8,8a-hexahydropyrido[4,3-b][1,4]oxazin-3-on;
(4aR,8aS)-6-[4-[(S oder R)-(3-Methylsulfonylphenyl)-(3-pyridyl)methyl]piperidin-1-carbonyl]-4,4a,5,7,8,8a-hexahydropyrido[4,3-b][1,4]oxazin-3-on;
(4aR,8aS)-6-[4-[(R oder S)-(3-Methylsulfonylphenyl)-(4-pyridyl)methyl]piperidin-1-carbonyl]-4,4a,5,7,8,8a-hexahydropyrido[4,3-b][1,4]oxazin-3-on;
(4aR,8aS)-6-[4-[(S oder R)-(3-Methylsulfonylphenyl)-(4-pyridyl)methyl]piperidin-1-carbonyl]-4,4a,5,7,8,8a-hexahydropyrido[4,3-b][1,4]oxazin-3-on;
(4aR,8aS)-6-(3-(4-(5-Azaspiro[2.4]heptan-5-yl)phenyl)azetidin-1-carbonyl)hexahydro-2H-pyrido[4,3-b][1,4]oxazin-3(4H)-on;
(4aR,8aS)-6-(3-(4-(Pentafluor-16-sulfaneyl)phenyl)azetidin-1-carbonyl)hexahydro-2H-pyrido[4,3-b][1,4]oxazin-3(4H)-on;
(4aR,8aS)-6-(3-(5-Chlorpyridin-2-yl)azetidin-1-carbonyl)hexahydro-2H-pyrido[4,3-b][1,4]oxazin-3(4H)-on;
(4aR,8aS)-6-(3-(2-Fluor-4-(trifluormethoxy)phenyl)azetidin-1-carbonyl)hexahydro-2H-pyrido[4,3-b][1,4]oxazin-3(4H)-on;
(4aR,8aS)-6-[3-(6-Methoxypyridin-3-yl)azetidin-1-carbonyl]-4,4a,5,7,8,8a-hexahydropyrido[4,3-b][1,4]oxazin-3-on;
(4aR,8aS)-6-(3-(4-Bromphenyl)pyrrolidin-1-carbonyl)hexahydro-2H-pyrido[4,3-b][1,4]oxazin-3(4H)-on;
(4aR,8aS)-6-(3-Phenylazetidin-1-carbonyl)hexahydro-2H-pyrido[4,3-b][1,4]oxazin-3(4H)-on;
(4aR,8aS)-6-(4-Phenylpiperidin-1-carbonyl)hexahydro-2H-pyrido[4,3-b][1,4]oxazin-3(4H)-on;
(4aR,8aS)-6-[3-[4-(2,2,2-Trifluorethyl)phenyl]azetidin-1-carbonyl]-4,4a,5,7,8,8a-hexahydropyrido[4,3-b][1,4]oxazin-3-on;
(4aR,8aS)-6-[3-[4-[1-(Trifluormethyl)cyclopropyl]phenyl]azetidin-1-carbonyl]-4,4a,5,7,8,8a-hexahydropyrido[4,3-b][1,4]oxazin-3-on;
(4aR,8aS)-6-[4-[(R oder S)-(3-Methylsulfonylphenyl)-(2-pyridyl)methyl]piperidin-1-carbonyl]-4,4a,5,7,8,8a-hexahydropyrido[4,3-b][1,4]oxazin-3-on;
(4aR,8aS)-6-[4-[(S oder R)-(3-Methylsulfonylphenyl)-(2-pyridyl)methyl]piperidin-1-carbonyl]-4,4a,5,7,8,8a-hexahydropyrido[4,3-b][1,4]oxazin-3-on;
(4aR,8aS)-6-[3-[4-(6,6-Difluor-2-azaspiro[3.3]heptan-2-yl)phenyl]azetidin-1-carbonyl]-4,4a,5,7,8,8a-hexahydropyrido[4,3-b][1,4]oxazin-3-on;
(4aR,8aS)-6-(3-(4-(8-Oxa-3-azabicyclo[3.2.1]octan-3-yl)phenyl)azetidin-1-carbonyl)hexahydro-2H-pyrido[4,3-b][1,4]oxazin-3(4H)-on;
(4aR,8aS)-6-(3-(5-(2,4-Dichlorphenyl)pyridin-2-yl)azetidin-1-carbonyl)hexahydro-2H-pyrido[4,3-b][1,4]oxazin-3(4H)-on;
(4aR,8aS)-6-(3-(4-((R oder S)-2-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(4-((S oder R)-2-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-[3-[4-(1-Piperidyl)phenyl]azetidin-1-carbonyl]-4,4a,5,7,8,8a-hexahydropyrido[4,3-b][1,4]oxazin-3-on;
(4aR,8aS)-6-(3-(4-((R oder S)-3-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(4-((S oder R)-3-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-[3-[4-(3-Hydroxy-3-methylazetidin-1-yl)phenyl]azetidin-1-carbonyl]-4,4a,5,7,8,8a-hexahydropyrido[4,3-b][1,4]oxazin-3-on;
(4aR,8aS)-6-[3-[4-(3-Fluor-3-methylazetidin-1-yl)phenyl]azetidin-1-carbonyl]-4,4a,5,7,8,8a-hexahydropyrido[4,3-b][1,4]oxazin-3-on;
(4aR,8aS)-6-(3-(4-(3-Fluorazetidin-1-yl)phenyl)azetidin-1-carbonyl)hexahydro-2H-pyrido[4,3-b][1,4]oxazin-3(4H)-on;
(4aR,8aS)-6-(3-(3-Fluor-4-(trifluormethoxy)phenyl)azetidin-1-carbonyl)hexahydro-2H-pyrido[4,3-b] [1,4]oxazin-3(4H)-on;
(4aR,8aS)-6-(3-(3-Methyl-4-(trifluormethoxy)phenyl)azetidin-1-carbonyl)hexahydro-2H-pyrido[4,3-b][1,4]oxazin-3(4H)-on;
(4aR,8aS)-6-(3-(3,5-Difluor-4-(trifluormethoxy)phenyl)azetidin-1-carbonyl)hexahydro-2H-pyrido[4,3-b][1,4]oxazin-3(4H)-on;
(4aR,8aS)-6-(3-(2-Chlor-4-(trifluormethoxy)phenyl)azetidin-1-carbonyl)hexahydro-2H-pyrido[4,3-b] [1,4]oxazin-3(4H)-on;
(4aR,8aS)-6-(3-(4-(Bicyclo[1.1.1]pentan-1-yl)phenyl)azetidin-1-carbonyl)hexahydro-2H-pyrido[4,3-b][1,4]oxazin-3(4H)-on;
(4aR,8aS)-6-(3-(5-(2-(Trifluormethyl)pyrrolidin-1-yl)pyridin-2-yl)azetidin-1-carbonyl)hexahydro-2H-pyrido[4,3-b][1,4]oxazin-3(4H)-on;
(4aR,8aS)-6-(3-(5-F'luor-1H-indol-3-yl)pyrrolidin-1-carbonyl)hexahydro-2H-pyrido[4,3-b] [1,4]oxazin-3(4H)-on;
(4aR,8aS)-6-(3-(1-Benzyl-5-chlor-1H-indol-3-yl)pyrrolidin-1-carbonyl)hexahydro-2H-pyrido[4,3-b] [1,4]oxazin-3(4H)-on;
(4aR,8aS)-6-(3-(2-Fluor-4-(trifluormethyl)phenyl)azetidin-1-carbonyl)hexahydro-2H-pyrido[4,3-b] [1,4]oxazin-3(4H)-on;
(4aR,8aS)-6-(3-(3-Chlor-4-(trifluormethoxy)phenyl)azetidin-1-carbonyl)hexahydro-2H-pyrido[4,3-b] [1,4]oxazin-3(4H)-on;
(4aR,8aS)-6-[3-[4-[3-(Cyclopropylmethoxy)azetidin-1-yl]phenyl]azetidin-1-carbonyl]-4,4a,5,7,8,8a-hexahydropyrido[4,3-b][1,4]oxazin-3-on;
5-(1-((4aR,8aS)-3-Oxooctahydro-2H-pyrido[4,3-b][1,4]oxazin-6-carbonyl)azetidin-3-yl)-2-(trifluormethoxy)benzonitril;
(4aR,8aS)-6-(3-(4-(1-(Hydroxymethyl)cyclopropyl)phenyl)azetidin-1-carbonyl)hexahydro-2H-pyrido[4,3-b][1,4]oxazin-3(4H)-on;
(4aR,8aS)-6-(3-(1-Methyl-1H-indazol-4-yl)azetidin-1-carbonyl)hexahydro-2H-pyrido[4,3-b] [1,4]oxazin-3(4H)-on;
(4aR,8aS)-6-(3-(4-(3-Fluorpyrrolidin-1-yl)phenyl)azetidin-1-carbonyl)hexahydro-2H-pyrido[4,3-b] [1,4]oxazin-3(4H)-on;
(4aR,8aS)-6-(3-(4-(2-Methylazetidin-1-yl)phenyl)azetidin-1-carbonyl)hexahydro-2H-pyrido[4,3-b] [1,4]oxazin-3(4H)-on;
(4aR,8aS)-6-[4-[Phenyl(pyridazin-3-yl)methyl]piperidin-1-carbonyl]-4,4a,5,7,8,8a-hexahydropyrido[4,3-b][1,4]oxazin-3-on;
(4aR,8aS)-6-[3-[4-(Trifluormethoxy)phenyl]pyrrolidin-1-carbonyl]-4,4a,5,7,8,8a-hexahydropyrido[4,3-b][1,4]oxazin-3-on;
(4aR,8aS)-6-(3-(1-Methyl-1H-indazol-6-yl)azetidin-1-carbonyl)hexahydro-2H-pyrido[4,3-b] [1,4]oxazin-3(4H)-on;
(4aR,8aS)-6-[3-(S oder R)-[3-(Trifluormethoxy)phenyl]pyrrolidin-1-carbonyl]-4,4a,5,7,8,8a-hexahydropyrido[4,3-b][1,4]oxazin-3-on;
(4aR,8aS)-6-[3-(R oder S)-[3-(Trifluormethoxy)phenyl]pyrrolidin-1-carbonyl]-4,4a,5,7,8,8a-hexahydropyrido[4,3-b][1,4]oxazin-3-on;
(4aR,8aS)-6-(3-(3-Chlor-4-hydroxyphenyl)azetidin-1-carbonyl)hexahydro-2H-pyrido[4,3-b] [1,4]oxazin-3(4H)-on;
(4aR,8aS)-6-(3-(3-Chlor-4-(3-methylazetidin-1-yl)phenyl)azetidin-1-carbonyl)hexahydro-2H-pyrido[4,3-b][1,4]oxazin-3(4H)-on;
(4aR,8aS)-6-[3-[4-(Oxetan-3-yl)phenyl]azetidin-1-carbonyl]-4,4a,5,7,8,8a-hexahydropyrido[4,3-b][1,4]oxazin-3-on;
(4aR,8aS)-6-(3-(3-Chlor-4-(3,3-difluorazetidin-1-yl)phenyl)azetidin-1-carbonyl)hexahydro-2H-pyrido[4,3-b][1,4]oxazin-3(4H)-on;
(4aR,8aS)-6-(3-(3-(3-Methylazetidin-1-yl)-4-(trifluormethoxy)phenyl)azetidin-1-carbonyl)hexahydro-2H-pyrido[4,3-b][1,4]oxazin-3(4H)-on;
(4aR,8aS)-6-[(3S oder R)-3-(3-Bromphenyl)pyrrolidin-1-carbonyl]-4,4a,5,7,8,8a-hexahydropyrido[4,3-b][1,4]oxazin-3-on;
(4aR,8aS)-6-[(3R oder S)-3-(3-Bromphenyl)pyrrolidin-1-carbonyl]-4,4a,5,7,8,8a-hexahydropyrido[4,3-b][1,4]oxazin-3-on;
(4aR,8aS)-6-[3-[4-(3-Azabicyclo[3.1.1]heptan-3-yl)phenyl]azetidin-1-carbonyl]-4,4a,5,7,8,8a-hexahydropyrido[4,3-b][1,4]oxazin-3-on;
(4aR,8aS)-6-(2-Methyl-3-(4-(trifluormethoxy)phenyl)azetidin-1-carbonyl)hexahydro-2H-pyrido[4,3-b][1,4]oxazin-3(4H)-on;
(4aR,8aS)-6-(3-(3,3-Dimethyl-2,3-dihydrobenzofuran-6-yl)azetidin-1-carbonyl)hexahydro-2H-pyrido[4,3-b][1,4]oxazin-3(4H)-on;
(4aR,8aS)-6-(3-(3-Chlor-5-(2,2,2-trifluorethoxy)phenyl)pyrrolidin-1-carbonyl)hexahydro-2H-pyrido[4,3-b][1,4]oxazin-3(4H)-on;
Methyl-2-methyl-2-(4-(1-((4aR,8aS)-3-oxooctahydro-2H-pyrido[4,3-b] [1,4]oxazin-6-carbonyl)azetidin-3-yl)phenyl)propanoat;
(4aR,8aS)-6-(3-(3,5-Dichlorphenyl)pyrrolidin-1-carbonyl)hexahydro-2H-pyrido[4,3-b][1,4]oxazin-3 (4H)-on;
(4aR,8aS)-6-(3-(4-(3-Methoxy-3-methylazetidin-1-yl)phenyl)azetidin-1-carbonyl)hexahydro-2H-pyrido[4,3-b][1,4]oxazin-3(4H)-on;
tert-Butyl-(2-(3-((1-((4aR,8aS)-3-oxooctahydro-2H-pyrido[4,3-b][1,4]oxazin-6-carbonyl)piperidin-4-yl)(phenyl)methyl)phenoxy)ethyl)carbamat;
(4aR,8aS)-6-((R oder S)-3-(3-Chlor-5-(2,2,2-trifluorethoxy)phenyl)pyrrolidin-1-carbonyl)hexahydro-2H-pyrido[4,3-b][1,4]oxazin-3(4H)-on;
(4aR,8aS)-6-((S oder R)-3-(3-Chlor-5-(2,2,2-trifluorethoxy)phenyl)pyrrolidin-1-carbonyl)hexahydro-2H-pyrido[4,3-b][1,4]oxazin-3(4H)-on;
(4aR,8aS)-6-(3-(4-(tert-Butyl)-3-methoxyphenyl)azetidin-1-carbonyl)hexahydro-2H-pyrido[4,3-b] [1,4]oxazin-3(4H)-on;
(4aR,8aS)-6-(3-(1-Methyl-1H-indazol-5-yl)azetidin-1-carbonyl)hexahydro-2H-pyrido[4,3-b] [1,4]oxazin-3(4H)-on;
(4aR,8aS)-6-(3-(4-Propylphenyl)azetidin-1-carbonyl)hexahydro-2H-pyrido[4,3-b][1,4]oxazin-3(4H)-on;
(4aR,8aS)-6-(3-(4-(Trifluormethoxy)-3-(trifluormethyl)phenyl)azetidin-1-carbonyl)hexahydro-2H-pyrido[4,3-b][1,4]oxazin-3(4H)-on;
(4aR,8aS)-6-[3-[4-[[1-(Trifluormethyl)cyclopropyl]methoxy]phenyl]azetidin-1-carbonyl]-4,4a,5,7,8,8a-hexahydropyrido[4,3-b][1,4]oxazin-3-on;
(4aR,8aS)-6-[3-[4-(2,2,2-Trifluor-1,1-dimethylethyl)phenyl]azetidin-1-carbonyl]-4,4a,5,7,8,8a-hexahydropyrido[4,3-b][1,4]oxazin-3-on;
(4aR,8aS)-6-[3-(R oder S)-[4-(2,2,2-Trifluor-1-methylethoxy)phenyl]azetidin-1-carbonyl]-4,4a,5,7,8,8a-hexahydropyrido[4,3-b][1,4]oxazin-3-on;
(4aR,8aS)-6-[3-[4-(3-Fluorpropyl)phenyl]azetidin-1-carbonyl]-4,4a,5,7,8,8a-hexahydropyrido[4,3-b][1,4]oxazin-3-on;
(4aR,8aS)-6-[3-(S oder R)-[4-(2,2,2-Trifluor-1-methylethoxy)phenyl]azetidin-1-carbonyl]-4,4a,5,7,8,8a-hexahydropyrido[4,3-b][1,4]oxazin-3-on;
(4aR,8aS)-6-[3-(4-Cyclobutylphenyl)azetidin-1-carbonyl]-4,4a,5,7,8,8a-hexahydropyrido[4,3-b][1,4]oxazin-3-on;
(4aR,8aS)-6-[3-(3-Methoxy-4-methylphenyl)azetidin-1-carbonyl]-4,4a,5,7,8,8a-hexahydropyrido[4,3-b][1,4]oxazin-3-on;
(4aR,8aS)-6-(4-((4-Fluorphenyl)((1-methyl-5-(trifluormethyl)-1H-pyrazol-3-yl)oxy)methyl)piperidin-1-carbonyl)hexahydro-2H-pyrido[4,3-b][1,4]oxazin-3(4H)-on;
(4aR,8aS)-6-[4-[[3-(2-Aminoethoxy)phenyl]phenylmethyl]piperidin-1-carbonyl]-4,4a,5,7,8,8a-hexahydropyrido[4,3-b][1,4]oxazin-3-on;
(4aR,8aS)-6-[3-[5-(2,4-Dichlorphenyl)-1,3,4-oxadiazol-2-yl]azetidin-1-carbonyl]-4,4a,5,7,8,8a-hexahydropyrido[4,3-b][1,4]oxazin-3-on;
(4aR,8aS)-6-[3-[3-Fluor-4-(trifluormethyl)phenyl]azetidin-1-carbonyl]-4,4a,5,7,8,8a-hexahydropyrido[4,3-b][1,4]oxazin-3-on;
tert-Butyl-N-[2-[3-[2-[3-[[1-[(4aR,8aS)-3-oxo-4,4a,5,7,8,8a-hexahydropyrido[4,3-b][1,4]oxazin-6-carbonyl]-4-piperidyl]phenylmethyl]phenoxy]ethylamino]-3-oxopropoxy]ethyl]carbamat;
tert-Butyl-N-[2-[2-[3-[2-[3-[[1-[(4aR,8aS)-3-oxo-4,4a,5,7,8,8a-hexahydropyrido[4,3-b][1,4]oxazin-6-carbonyl]-4-piperidyl]phenylmethyl]phenoxy]ethylamino]-3-oxo-propoxy]ethoxy]ethyl]carbamat;
(4aR,8aS)-6-[3-[1-(2,4-Dichlorphenyl)pyrazol-3-yl]azetidin-1-carbonyl]-4,4a,5,7,8,8a-hexahydropyrido[4,3-b][1,4]oxazin-3-on;
(4aR,8aS)-6-[3-(4-Propoxyphenyl)azetidin-1-carbonyl]-4,4a,5,7,8,8a-hexahydropyrido[4,3-b][1,4]oxazin-3-on;
(4aR,8aS)-6-[3-(3,4-Pimethylphenyl)azetidin-1-carbonyl]-4,4a,5,7,8,8a-hexahydropyrido[4,3-b][1,4]oxazin-3-on;
2-[4-[1-[(4aR,8aS)-3-Oxo-4,4a,5,7,8,8a-hexahydropyrido[4,3-b][1,4]oxazin-6-carbonyl]azetidin-3-yl]phenyl]-N-ethyl-5-methylbenzamid;
(4aR,8aS)-6-[3-[4-(2,2-Dimethylpropyl)phenyl]azetidin-1-carbonyl]-4,4a,5,7,8,8a-hexahydropyrido[4,3-b][1,4]oxazin-3-on;
(4aR,8aS)-6-[3-(4-tert-Butoxyphenyl)azetidin-1-carbonyl]-4,4a,5,7,8,8a-hexahydropyrido[4,3-b][1,4]oxazin-3-on;
(4aR,8aS)-6-[4-(5-Chlorindolin-1-yl)piperidin-1-carbonyl]-4,4a,5,7,8,8a-hexahydropyrido[4,3-b][1,4]oxazin-3-on;
(4aR,8aS)-6-[4-(4-Chlorisoindolin-2-yl)piperidin-1-carbonyl]-4,4a,5,7,8,8a-hexahydropyrido[4,3-b][1,4]oxazin-3-on;
(4aR,8aS)-6-[4-(5'-Chlorspiro[cyclopropan-1,3'-indolin]-1'-yl)piperidin-1-carbonyl]-4,4a,5,7,8,8a-hexahydropyrido[4,3-b][1,4]oxazin-3-on;
(4aR,8aS)-6-[3-(4-Chlorisoindolin-2-yl)azetidin-1-carbonyl]-4,4a,5,7,8,8a-hexahydropyrido[4,3-b][1,4]oxazin-3-on;
(4aR,8aS)-6-[4-(5-Chlorisoindolin-2-yl)piperidin-1-carbonyl]-4,4a,5,7,8,8a-hexahydropyrido[4,3-b][1,4]oxazin-3-on;
(4aS,8aS)-6-[4-[1-[2-[tert-Butyl(dimethyl)silyl]oxyethyl]-5-chlorindol-3-yl]piperidin-1-carbonyl]-4,4a,5,7,8,8a-hexahydropyrido[4,3-b][1,4]oxazin-3-on;
(4aS,8aS)-6-[4-[5-Chlor-1-(2-hydroxyethyl)indol-3-yl]piperidin-1-carbonyl]-4,4a,5,7,8,8a-hexahydropyrido[4,3-b][1,4]oxazin-3-on;
(4aR,8aS)-6-(3-(5-(3-(Trifluormethyl)pyrrolidin-1-yl)pyridin-2-yl)azetidin-1-carbonyl)hexahydro-2H-pyrido[4,3-b][1,4]oxazin-3(4H)-on;
(4aR,8aS)-6-(3-(5-((R oder S)-3-(Trifluormethyl)pyrrolidin-1-yl)pyridin-2-yl)azetidin-1-carbonyl)hexahydro-2H-pyrido[4,3-b][1,4]oxazin-3(4H)-on;
(4aR,8aS)-6-(3-(5-((S oder R)-3-(Trifluormethyl)pyrrolidin-1-yl)pyridin-2-yl)azetidin-1-carbonyl)hexahydro-2H-pyrido[4,3-b][1,4]oxazin-3(4H)-on;
(4aR,8aS)-6-[3-[6-[3-(Trifluormethyl)pyrrolidin-1-yl]-3-pyridyl]azetidin-1-carbonyl]-4,4a,5,7,8,8a-hexahydropyrido[4,3-b][1,4]oxazin-3-on;
(4aR,8aS)-6-[3-[6-[3-(R oder S)-(Trifluormethyl)pyrrolidin-1-yl]-3-pyridyl]azetidin-1-carbonyl]-4,4a,5,7,8,8a-hexahydropyrido[4,3-b][1,4]oxazin-3-on;
(4aR,8aS)-6-[3-[6-[3S- oder 3R-(Trifluormethyl)pyrrolidin-1-yl]-3-pyridyl]azetidin-1-carbonyl]-4,4a,5,7,8,8a-hexahydropyrido[4,3-b][1,4]oxazin-3-on;
(4aR,8aS)-6-[3-(4-Tetrahydropyran-3-ylphenyl)azetidin-1-carbonyl]-4,4a,5,7,8,8a-hexahydropyrido[4,3-b][1,4]oxazin-3-on;
(4aR,8aS)-6-[3-[2-Methoxy-4-(2,2,2-trifluorethyl)phenyl]azetidin-1-carbonyl]-4,4a,5,7,8,8a-hexahydropyrido[4,3-b][1,4]oxazin-3-on;
(4aR,8aS)-6-(3-(4-(Neopentyloxy)phenyl)azetidin-1-carbonyl)hexahydro-2H-pyrido[4,3-b][1,4]oxazin-3(4H)-on;
rac-(4aR,8aS)-6-[4-(3-Methoxy-1-phenylpropyl)piperidin-1-carbonyl]-4,4a,5,7,8,8a-hexahydropyrido[4,3-b][1,4]oxazin-3-on;
rac-(4aR,8aS)-6-[4-[Phenyl(3-pyridyl)methyl]piperidin-1-carbonyl]-4,4a,5,7,8,8a-hexahydropyrido[4,3-b][1,4]oxazin-3-on;
rac-(4aR,8aS)-6-[4-[(5-Methyl-1,2,4-oxadiazol-3-yl)phenylmethyl]piperidin-1-carbonyl]-4,4a,5,7,8,8a-hexahydropyrido[4,3-b][1,4]oxazin-3-on;
rac-(4aR,8aS)-6-[4-(5-Fluor-1-methylindol-3-yl)piperidin-1-carbonyl]-4,4a,5,7,8,8a-hexahydropyrido[4,3-b][1,4]oxazin-3-on;
tert-Butyl-3-[5-chlor-3-[1-[rac-(4aR,8aS)-3-oxo-4,4a,5,7,8,8a-hexahydropyrido[4,3-b][1,4]oxazin-6-carbonyl]-4-piperidyl]indol-1-yl]azetidin-1-carboxylat;
tert-Butyl-3-[5-chlor-3-[1-[rac-(4aR,8aS)-3-oxo-4,4a,5,7,8,8a-hexahydropyrido[4,3-b][1,4]oxazin-6-carbonyl]-4-piperidyl]indol-1-yl]azetidin-1-carboxylat;
rac-(4aR,8aS)-6-[4-[1-(2-Chlor-4-fluorphenoxy)propyl]piperidin-1-carbonyl]-4,4a,5,7,8,8a-hexahydropyrido[4,3-b][1,4]oxazin-3-on;
rac-(4aR,8aS)-6-[4-[(2-Chlor-4-fluorphenoxy)cyclopropylmethyl]piperidin-1-carbonyl]-4,4a,5,7,8,8a-hexahydropyrido[4,3-b][1,4]oxazin-3-on;
rac-(4aR,8aS)-6-[4-[(4-Fluorphenoxy)phenylmethyl]piperidin-1-carbonyl]-4,4a,5,7,8,8a-hexahydropyrido[4,3-b][1,4]oxazin-3-on;
rac-(4aR,8aS)-6-[4-[5-(Trifluormethyl)-2-pyridyl]piperidin-1-carbonyl]-4,4a,5,7,8,8a-hexahydropyrido[4,3-b][1,4]oxazin-3-on;
rac-(4aR,8aS)-6-[4-[Cyclopropylmethoxy-[4-(trifluormethyl)phenyl]methyl]piperidin-1-carbonyl]-4,4a,5,7,8,8a-hexahydropyrido[4,3-b][1,4]oxazin-3-on;
rac-(4aR,8aS)-6-[4-[1-(4-Fluorphenyl)-2-hydroxyethyl]piperazin-1-carbonyl]-4,4a,5,7,8,8a-hexahydropyrido[4,3-b][1,4]oxazin-3-on;
rac-(4aR,8aS)-6-[4-[1-(4-Fluorphenyl)-2-methoxyethyl]piperazin-1-carbonyl]-4,4a,5,7,8,8a-hexahydropyrido[4,3-b][1,4]oxazin-3-on;
rac-(4aR,8aS)-6-[4-[1-(3,4-Difluorphenyl)-2-hydroxyethyl]piperazin-1-carbonyl]-4,4a,5,7,8,8a-hexahydropyrido[4,3-b][1,4]oxazin-3-on;
rac-(4aR,8aS)-6-[4-[1-(3,4-Difluorphenyl)-2-methoxyethyl]piperazin-1-carbonyl]-4,4a,5,7,8,8a-hexahydropyrido[4,3-b][1,4]oxazin-3-on;
rac-(4aR,8aS)-6-[4-[1-(4,4-Difluor-1-piperidyl)cyclopropyl]piperidin-1-carbonyl]-4,4a,5,7,8,8a-hexahydropyrido[4,3-b][1,4]oxazin-3-on;
rac-(4aR,8aS)-6-[4-[1-[4-(Trifluormethyl)imidazol-1-yl]ethyl]piperidin-1-carbonyl]-4,4a,5,7,8,8a-hexahydropyrido[4,3-b][1,4]oxazin-3-on;
rac-(4aR,8aS)-6-[4-[1-[4-(Trifluormethyl)pyrazol-1-yl]cyclopropyl]piperidin-1-carbonyl]-4,4a,5,7,8,8a-hexahydropyrido[4,3-b][1,4]oxazin-3-on;
rac-(4aR,8aS)-6-[4-[(4-tert-Butylpyrazol-1-yl)-(4-fluorphenyl)methyl]piperidin-1-carbonyl]-4,4a,5,7,8,8a-hexahydropyrido[4,3-b][1,4]oxazin-3-on;
rac-(4aR,8aS)-6-[4-[3-[4-(Trifluormethyl)phenyl]oxetan-3-yl]piperidin-1-carbonyl]-4,4a,5,7,8,8a-hexahydropyrido[4,3-b][1,4]oxazin-3-on;
rac-(4aR,8aS)-6-[4-[4-(Trifluormethyl)benzoyl]piperidin-1-carbonyl]-4,4a,5,7,8,8a-hexahydropyrido[4,3-b][1,4]oxazin-3-on;
rac-(4aR,8aS)-6-[4-[1-[4-(Trifluormethyl)pyrazol-1-yl]ethyl]piperidin-1-carbonyl]-4,4a,5,7,8,8a-hexahydropyrido[4,3-b][1,4]oxazin-3-on;
rac-(4aR,8aS)-6-[4-[(4-Fluorphenyl)morpholinomethyl]piperidin-1-carbonyl]-4,4a,5,7,8,8a-hexahydropyrido[4,3-b][1,4]oxazin-3-on;
rac-(4aR,8aS)-6-[4-[(4-tert-Butyloxazol-2-yl)difluormethyl]piperidin-1-carbonyl]-4,4a,5,7,8,8a-hexahydropyrido[4,3-b][1,4]oxazin-3-on;
rac-(4aR,8aS)-6-[4-[1-(4-Chlorphenyl)-2-hydroxyethyl]piperazin-1-carbonyl]-4,4a,5,7,8,8a-hexahydropyrido[4,3-b][1,4]oxazin-3-on;
rac-(4aR,8aS)-6-[4-[1-(4-Chlorphenyl)-2-methoxyethyl]piperazin-1-carbonyl]-4,4a,5,7,8,8a-hexahydropyrido[4,3-b][1,4]oxazin-3-on;
rac-(4aR,8aS)-6-[4-[2-Hydroxy-1-[4-(trifluormethyl)phenyl]ethyl]piperazin-1-carbonyl]-4,4a,5,7,8,8a-hexahydropyrido[4,3-b][1,4]oxazin-3-on;
rac-(4aR,8aS)-6-[4-[2-Methoxy-1-[4-(trifluormethyl)phenyl]ethyl]piperazin-1-carbonyl]-4,4a,5,7,8,8a-hexahydropyrido[4,3-b][1,4]oxazin-3-on;
rac-(4aR,8aS)-6-[4-(1-Cyclohexyl-2-methoxyethyl)piperazin-1-carbonyl]-4,4a,5,7,8,8a-hexahydropyrido[4,3-b][1,4]oxazin-3-on;
rac-(4aR,8aS)-6-[4-(1-Cyclohexyl-2-hydroxyethyl)piperazin-1-carbonyl]-4,4a,5,7,8,8a-hexahydropyrido[4,3-b][1,4]oxazin-3-on;
rac-(4aR,8aS)-6-[4-[1-(4-Fluorphenyl)cyclopropyl]piperazin-1-carbonyl]-4,4a,5,7,8,8a-hexahydropyrido[4,3-b][1,4]oxazin-3-on;
rac-(4aR,8aS)-6-[4-[2-(4-Fluorphenyl)cyclopropyl]piperazin-1-carbonyl]-4,4a,5,7,8,8a-hexahydropyrido[4,3-b][1,4]oxazin-3-on;
rac-(4aR,8aS)-6-[4-[1-(4-Fluorphenyl)cyclobutyl]piperazin-1-carbonyl]-4,4a,5,7,8,8a-hexahydropyrido[4,3-b][1,4]oxazin-3-on;
rac-(4aR,8aS)-6-[4-[Cyclopropyl-(4-fluorphenyl)methyl]piperazin-1-carbonyl]-4,4a,5,7,8,8a-hexahydropyrido[4,3-b][1,4]oxazin-3-on;
rac-(4aR,8aS)-6-[4-[Cyclohexyl-(4-fluorphenyl)methyl]piperazin-1-carbonyl]-4,4a,5,7,8,8a-hexahydropyrido[4,3-b][1,4]oxazin-3-on;
rac-(4aR,8aS)-6-[4-[(4-Fluorphenyl)-(4-pyridyl)methyl]piperazin-1-carbonyl]-4,4a,5,7,8,8a-hexahydropyrido[4,3-b][1,4]oxazin-3-on;
rac-(4aR,8aS)-6-[4-[2,2,2-Trifluor-1-(4-fluorphenyl)ethyl]piperazin-1-carbonyl]-4,4a,5,7,8,8a-hexahydropyrido[4,3-b][1,4]oxazin-3-on;
rac-(4aR,8aS)-6-[4-[(4-Fluorphenyl)-oxazol-5-yl-methyl]piperazin-1-carbonyl]-4,4a,5,7,8,8a-hexahydropyrido[4,3-b][1,4]oxazin-3-on;
rac-(4aR,8aS)-6-[4-[Bis(4-chlorphenyl)methyl]piperazin-1-carbonyl]-4,4a,5,7,8,8a-hexahydropyrido[4,3-b][1,4]oxazin-3-on;
rac-(4aS,8aS)-6-[4-[5-Chlor-1-(cyclopropylmethyl)indol-3-yl]piperidin-1-carbonyl]-4,4a,5,7,8,8a-hexahydropyrido[4,3-b][1,4]oxazin-3-on;
rac-(4aS,8aS)-6-[4-[5-Chlor-1-(cyclopropylmethyl)indol-3-yl]piperidin-1-carbonyl]-4,4a,5,7,8,8a-hexahydropyrido[4,3-b][1,4]oxazin-3-on;
rac-(4aR,8aR)-6-[4-[5-Chlor-1-(cyclopropylmethyl)indol-3-yl]piperidin-1-carbonyl]-4,4a,5,7,8,8a-hexahydropyrido[4,3-b][1,4]oxazin-3-on;
rac-(4aR,8aS)-6-[4-[(4-Chlorphenyl)-(4-fluorphenyl)methyl]piperazin-1-carbonyl]-4,4a,5,7,8,8a-hexahydropyrido[4,3-b][1,4]oxazin-3-on;
rac-(4aR,8aS)-6-(4-Benzhydrylpiperazin-1-carbonyl)-4,4a,5,7,8,8a-hexahydropyrido[4,3-b][1,4]oxazin-3-on;
rac-(4aR,8aS)-6-[4-[4-(2,2,2-Trifluorethyl)phenyl]piperazin-1-carbonyl]-4,4a,5,7,8,8a-hexahydropyrido[4,3-b][1,4]oxazin-3-on;
rac-(4aR,8aS)-6-[4-[3-(2,2,2-Trifluorethyl)phenyl]piperazin-1-carbonyl]-4,4a,5,7,8,8a-hexahydropyrido[4,3-b][1,4]oxazin-3-on;
rac-(4aR,8aS)-6-[4-[4-Chlor-3-(trifluormethyl)phenyl]piperazin-1-carbonyl]-4,4a,5,7,8,8a-hexahydropyrido[4,3-b][1,4]oxazin-3-on;
rac-(4aR,8aS)-6-[4-[1-[4-(Trifluormethyl)phenyl]ethyl]piperazin-1-carbonyl]-4,4a,5,7,8,8a-hexahydropyrido[4,3-b][1,4]oxazin-3-on;
rac-(4aR,8aS)-6-[3-[1-(2-Chlor-4-fluorphenoxy)ethyl]azetidin-1-carbonyl]-4,4a,5,7,8,8a-hexahydropyrido[4,3-b][1,4]oxazin-3-on und
rac-(4aR,8aS)-6-[3-[5-Chlor-1-(cyclopropylmethyl)indol-3-yl]azetidin-1-carbonyl]-4,4a,5,7,8,8a-hexahydropyrido[4,3-b][1,4]oxazin-3-on.

10. Verbindung der Formel (I) nach einem der Ansprüche 1 bis 8 oder ein pharmazeutisch unbedenkliches Salz davon, ausgewählt aus:
rac-cis-6-(4-(5 -Chlor-1-(cyclopropylmethyl)-1H-indol-3-yl)piperidin-1-carbonyl)hexahydro-2H-pyrido[4,3-b][1,4]oxazin-3(4H)-on;
rac-cis-6-(4-(5-Chlor-1-methyl-1H-indol-3-yl)piperidin-1-carbonyl)hexahydro-2H-pyrido[4,3-b] [1,4]oxazin-3(4H)-on;
rac-cis-6-(4-(1-(4-Fluorphenyl)-3-methoxypropyl)piperidin-1-carbonyl)hexahydro-2H-pyrido[4,3-b] [1,4]oxazin-3(4H)-on;
rac-cis-6-(4-((4-Chlorphenyl)(phenyl)methyl)piperazin-1-carbonyl)hexahydro-2H-pyrido[4,3-b] [1,4]oxazin-3(4H)-on;
(+)-cis-6-(4-(Bis(4-fluorphenyl)methyl)piperazin-1-carbonyl)hexahydro-2H-pyrido[4,3-b][1,4]oxazin-3(4H)-on;
(+)- oder (-)-cis-6-(4-(5-Chlor-1-cyclopropyl-1H-indol-3-yl)piperidin-1-carbonyl)hexahydro-2H-pyrido[4,3-b][1,4]oxazin-3(4H)-on;
(+)- oder (-)-cis-6-(4-(5-Chlor-1-(oxetan-3-yl)-1H-indol-3-yl)piperidin-1-carbonyl)hexahydro-2H-pyrido[4,3-b][1,4]oxazin-3(4H)-on;
rac-cis-6-(4-(1-(4-Fluorphenyl)-3-hydroxypropyl)piperidin-1-carbonyl)hexahydro-2H-pyrido[4,3-b] [1,4]oxazin-3(4H)-on;
(4aR,8aS)-6-(4-(Difluor(4-(trifluormethyl)phenyl)methyl)piperidin-1-carbonyl)hexahydro-2H-pyrido[4,3-b][1,4]oxazin-3(4H)-on;
(+)- oder (-)-cis-6-(4-((R oder S)-1-(2-Chlor-4-fluorphenoxy)ethyl)piperidin-1-carbonyl)hexahydro-2H-pyrido[4,3-b][1,4]oxazin-3(4H)-on;
(+)- oder (-)-cis-6-(4-((4-Chlorphenyl)difluormethyl)piperidin-1-carbonyl)hexahydro-2H-pyrido[4,3-b][1,4]oxazin-3(4H)-on;
(+)- oder (-)-cis-6-(4-((2-Chlor-4-fluorphenyl)difluormethyl)piperidin-1-carbonyl)hexahydro-2H-pyrido[4,3-b][1,4]oxazin-3(4H)-on;
(4aR,8aS)-6-(4-((R oder S)-1-(4-(Trifluormethyl)phenyl)ethyl)piperidin-1-carbonyl)hexahydro-2H-pyrido[4,3-b][1,4]oxazin-3(4H)-on;
(4aR,8aS)-6-(4-((S oder R)-(4-Fluorphenyl)(2,2,2-trifluorethoxy)methyl)piperidin-1-carbonyl)hexahydro-2H-pyrido[4,3-b][1,4]oxazin-3(4H)-on;
(4aR,8aS)-6-[4-[(S oder R)-(4-Fluorphenyl)-(3-methoxyphenyl)methyl]piperidin-1-carbonyl]-4,4a,5,7,8,8a-hexahydropyrido[4,3-b][1,4]oxazin-3-on;
(4aR,8aS)-6-(4-((R oder S)-(4-Fluorphenyl)(3-methoxyphenyl)methyl)piperidin-1-carbonyl)hexahydro-2H-pyrido[4,3-b][1,4]oxazin-3(4H)-on;
(4aR,8aS)-6-(4-((S oder R)-(3-(2-Fluorethoxy)phenyl)(phenyl)methyl)piperidin-1-carbonyl)hexahydro-2H-pyrido[4,3-b][1,4]oxazin-3(4H)-on;
(4aR,8aS)-6-(4-((3-Cyclopropyl-1,2,4-oxadiazol-5-yl)(4-fluorphenyl)methyl)piperidin-1-carbonyl)hexahydro-2H-pyrido[4,3-b][1,4]oxazin-3(4H)-on;
(4aR,8aS)-6-(4-((S oder R)-(4-Fluorphenyl)(4-methoxyphenyl)methyl)piperidin-1-carbonyl)hexahydro-2H-pyrido[4,3-b][1,4]oxazin-3(4H)-on;
(4aR,8aS)-6-(4-((S oder R)-(3,4-Dimethoxyphenyl)(4-fluorphenyl)methyl)piperidin-1-carbonyl)hexahydro-2H-pyrido[4,3-b][1,4]oxazin-3(4H)-on;
(4aR,8aS)-6-(4-((R oder S)-(4-Fluorphenyl)(4-methoxyphenyl)methyl)piperidin-1-carbonyl)hexahydro-2H-pyrido[4,3-b][1,4]oxazin-3(4H)-on;
(4aR,8aS)-6-(4-((S oder R)-(4-(2-Fluorethoxy)phenyl)(4-fluorphenyl)methyl)piperidin-1-carbonyl)hexahydro-2H-pyrido[4,3-b][1,4]oxazin-3(4H)-on;
(4aR,8aS)-6-[3-[4-(Trifluormethoxy)phenyl]azetidin-1-carbonyl]-4,4a,5,7,8,8a-hexahydropyrido[4,3-b][1,4]oxazin-3-on;
(4aR,8aS)-6-(3-(2'-Trifluormethoxy)-[1,1'-biphenyl]-4-yl)azetidin-1-carbonyl)hexahydro-2H-pyrido[4,3-b][1,4]oxazin-3(4H)-on;
(4aR,8aS)-6-(3-(2'-(Trifluormethyl)-[1,1'-biphenyl]-4-yl)azetidin-1-carbonyl)hexahydro-2H-pyrido[4,3-b][1,4]oxazin-3(4H)-on;
(4aR,8aS)-6-(3-(2',4'-Difluor-[1,1'-biphenyl]-4-yl)azetidin-1-carbonyl)hexahydro-2H-pyrido[4,3-b][1,4]oxazin-3(4H)-on;
(4aR,8aS)-6-(3-(4-(3-(Trifluormethyl)azetidin-1-yl)phenyl)azetidin-1-carbonyl)hexahydro-2H-pyrido[4,3-b][1,4]oxazin-3(4H)-on;
(4aR,8aS)-6-(3-(4-Brom-3-chlorphenyl)azetidin-1-carbonyl)hexahydro-2H-pyrido[4,3-b][1,4]oxazin-3 (4H)-on;
(4aR,8aS)-6-[3-[4-(4-Chlor-2-fluorphenyl)phenyl]azetidin-1-carbonyl]-4,4a,5,7,8,8a-hexahydropyrido[4,3-b][1,4]oxazin-3-on;
(4aR,8aS)-6-[3-[4-(2-Chlor-4-fluorphenyl)phenyl]azetidin-1-carbonyl]-4,4a,5,7,8,8a-hexahydropyrido[4,3-b][1,4]oxazin-3-on;
(4aR,8aS)-6-(3-(4-(3-((1,1,1-Trifluorpropan-2-yl)oxy)azetidin-1-yl)phenyl)azetidin-1-carbonyl)hexahydro-2H-pyrido[4,3-b][1,4]oxazin-3(4H)-on;
(4aR,8aS)-6-(3-(4-(tert-Butyl)phenyl)azetidin-1-carbonyl)hexahydro-2H-pyrido[4,3-b][1,4]oxazin-3 (4H)-on;
(4aR,8aS)-6-(3-(4-(Trifluormethyl)phenyl)azetidin-1-carbonyl)hexahydro-2H-pyrido[4,3-b] [1,4]oxazin-3(4H)-on;
(4aR,8aS)-6-(3-(4-(3-((1,1,1-Trifluor-2-methylpropan-2-yl)oxy)azetidin-1-yl)phenyl)azetidin-1-carbonyl)hexahydro-2H-pyrido[4,3-b][1,4]oxazin-3(4H)-on;
(4aR,8aS)-6-(3-(4-(1,1-Difluorethyl)phenyl)azetidin-1-carbonyl)hexahydro-2H-pyrido[4,3-b] [1,4]oxazin-3(4H)-on;
(4aR,8aS)-6-(3-(4-(3,3-Dimethylpyrrolidin-1-yl)phenyl)azetidin-1-carbonyl)hexahydro-2H-pyrido[4,3-b][1,4]oxazin-3(4H)-on;
(4aR,8aS)-6-(3-(4-(3-(Trifluormethoxy)azetidin-1-yl)phenyl)azetidin-1-carbonyl)hexahydro-2H-pyrido[4,3-b][1,4]oxazin-3(4H)-on;
(4aR,8aS)-6-(3-(6-(2,4-Dichlorphenyl)pyridin-3-yl)azetidin-1-carbonyl)hexahydro-2H-pyrido[4,3-b] [1,4]oxazin-3(4H)-on;
(4aR,8aS)-6-[3-[4-(2,2,2-Trifluorethoxy)phenyl]azetidin-1-carbonyl]-4,4a,5,7,8,8a-hexahydropyrido[4,3-b][1,4]oxazin-3-on;
(4aR,8aS)-6-[3-[4-[3-[(1S oder 1R)-2,2,2-Trifluor-1-methylethoxy]azetidin-1-yl]phenyl]azetidin-1-carbonyl]-4,4a,5,7,8,8a-hexahydropyrido[4,3-b] [1,4]oxazin-3-on;
(4aR,8aS)-6-[3-[4-[3-[(1R oder 1S)-2,2,2-Trifluor-1-methylethoxy]azetidin-1-yl]phenyl]azetidin-1-carbonyl]-4,4a,5,7,8,8a-hexahydropyrido[4,3-b] [1,4]oxazin-3-on;
(4aR,8aS)-6-(3-(4-(2-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(4-(3-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(4-(3-(2,2,2-Trifluorethoxy)azetidin-1-yl)phenyl)azetidin-1-carbonyl)hexahydro-2H-pyrido[4,3-b][1,4]oxazin-3(4H)-on;
(4aR,8aS)-6-(3-(6-(2-(Trifluormethyl)pyrrolidin-1-yl)pyridin-3-yl)azetidin-1-carbonyl)hexahydro-2H-pyrido[4,3-b][1,4]oxazin-3(4H)-on;
(4aR,8aS)-6-(3-(4-(Pentafluor-16-sulfaneyl)phenyl)azetidin-1-carbonyl)hexahydro-2H-pyrido[4,3-b] [1,4]oxazin-3(4H)-on;
(4aR,8aS)-6-(3-(2-Fluor-4-(trifluormethoxy)phenyl)azetidin-1-carbonyl)hexahydro-2H-pyrido[4,3-b] [1,4]oxazin-3(4H)-on;
(4aR,8aS)-6-[3-[4-(2,2,2-Trifluorethyl)phenyl]azetidin-1-carbonyl]-4,4a,5,7,8,8a-hexahydropyrido[4,3-b][1,4]oxazin-3-on;
(4aR,8aS)-6-[3-[4-[1-(Trifluormethyl)cyclopropyl]phenyl]azetidin-1-carbonyl]-4,4a,5,7,8,8a-hexahydropyrido[4,3-b][1,4]oxazin-3-on;
(4aR,8aS)-6-[3-[4-(6,6-Difluor-2-azaspiro[3.3]heptan-2-yl)phenyl]azetidin-1-carbonyl]-4,4a,5,7,8,8a-hexahydropyrido[4,3-b][1,4]oxazin-3-on;
(4aR,8aS)-6-(3-(5-(2,4-Dichlorphenyl)pyridin-2-yl)azetidin-1-carbonyl)hexahydro-2H-pyrido[4,3-b] [1,4]oxazin-3(4H)-on;
(4aR,8aS)-6-(3-(4-((R oder S)-2-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(4-((S oder R)-2-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(4-((R oder S)-3-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(4-((S oder R)-3-(Trifluormethyl)pyrrolidin-1-yl)phenyl)azetidin-1-carbonyl)hexahydro-2H-pyrido[4,3-b][1,4]oxazin-3(4H)-on;
(4aR,8aS)-6-(3-(3-Fluor-4-(trifluormethoxy)phenyl)azetidin-1-carbonyl)hexahydro-2H-pyrido[4,3-b][1,4]oxazin-3(4H)-on;
(4aR,8aS)-6-(3-(3-Methyl-4-(trifluormethoxy)phenyl)azetidin-1-carbonyl)hexahydro-2H-pyrido[4,3-b][1,4]oxazin-3(4H)-on;
(4aR,8aS)-6-(3-(3,5-Difluor-4-(trifluormethoxy)phenyl)azetidin-1-carbonyl)hexahydro-2H-pyrido[4,3-b][1,4]oxazin-3(4H)-on;
(4aR,8aS)-6-(3-(3-Chlor-4-(trifluormethoxy)phenyl)azetidin-1-carbonyl)hexahydro-2H-pyrido[4,3-b][1,4]oxazin-3(4H)-on;
(4aR,8aS)-6-((R oder S)-3-(3-Chlor-5-(2,2,2-trifluorethoxy)phenyl)pyrrolidin-1-carbonyl)hexahydro-2H-pyrido[4,3-b][1,4]oxazin-3(4H)-on;
(4aR,8aS)-6-((S oder R)-3-(3-Chlor-5-(2,2,2-trifluorethoxy)phenyl)pyrrolidin-1-carbonyl)hexahydro-2H-pyrido[4,3-b][1,4]oxazin-3(4H)-on;
(4aR,8aS)-6-(3-(4-(tert-Butyl)-3-methoxyphenyl)azetidin-1-carbonyl)hexahydro-2H-pyrido[4,3-b][1,4]oxazin-3(4H)-on;
(4aR,8aS)-6-(3-(4-Propylphenyl)azetidin-1-carbonyl)hexahydro-2H-pyrido[4,3-b][1,4]oxazin-3(4H)-on;
(4aR,8aS)-6-(3-(4-(Trifluormethoxy)-3-(trifluormethyl)phenyl)azetidin-1-carbonyl)hexahydro-2H-pyrido[4,3-b][1,4]oxazin-3(4H)-on;
(4aR,8aS)-6-(3-(5-((R oder S)-3-(Trifluormethyl)pyrrolidin-1-yl)pyridin-2-yl)azetidin-1-carbonyl)hexahydro-2H-pyrido[4,3-b][1,4]oxazin-3(4H)-on;
(4aR,8aS)-6-(3-(5-((S oder R)-3-(Trifluormethyl)pyrrolidin-1-yl)pyridin-2-yl)azetidin-1-carbonyl)hexahydro-2H-pyrido[4,3-b][1,4]oxazin-3(4H)-on;
(4aR,8aS)-6-[3-[6-[3-(R oder S)-(Trifluormethyl)pyrrolidin-1-yl]-3-pyridyl]azetidin-1-carbonyl]-4,4a,5,7,8,8a-hexahydropyrido[4,3-b][1,4]oxazin-3-on;
(4aR,8aS)-6-[3-[6-[3-(S oder R)-(Trifluormethyl)pyrrolidin-1-yl]-3-pyridyl]azetidin-1-carbonyl]-4,4a,5,7,8,8a-hexahydropyrido[4,3-b][1,4]oxazin-3-on;
(4aR,8aS)-6-[3-(4-Tetrahydropyran-3-ylphenyl)azetidin-1-carbonyl]-4,4a,5,7,8,8a-hexahydropyrido[4,3-b][1,4]oxazin-3-on;
(4aR,8aS)-6-[3-[2-Methoxy-4-(2,2,2-trifluorethyl)phenyl]azetidin-1-carbonyl]-4,4a,5,7,8,8a-hexahydropyrido[4,3-b][1,4]oxazin-3-on und
(4aR,8aS)-6-(3-(4-(Neopentyloxy)phenyl)azetidin-1-carbonyl)hexahydro-2H-pyrido[4,3-b][1,4]oxazin-3(4H)-on.

11. Verfahren zur Herstellung der Verbindungen der Formel (Ic) nach einem der Ansprüche 1 bis 10, umfassend:
Reagieren von 4a,5,6,7,8,8a-Hexahydro-4H-pyrido[4,3-b][1,4]oxazin-3-on (1) 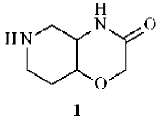
mit einem heterocyclischen Amin 2a, wobei A, L, X, m, n, und R²⁰ bis R²³ wie in einem der Ansprüche 1 bis 10 definiert sind, 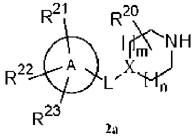
in der Gegenwart einer Base und eines harnstoffbildenden Reagens unter Bildung der Verbindung der Formel (Ic).

12. Verbindung der Formel (Ic) nach einem der Ansprüche 1 bis 10 oder ein pharmazeutisch unbedenkliches Salz davon zur Verwendung als therapeutisch wirksame Substanz.

13. Pharmazeutische Zusammensetzung, umfassend eine Verbindung der Formel (Ic) nach einem der Ansprüche 1 bis 10 oder ein pharmazeutisch unbedenkliches Salz davon und einen therapeutisch inerten Träger.

14. Verbindung der Formel (Ic) nach einem der Ansprüche 1 bis 10 oder ein pharmazeutisch unbedenkliches Salz davon zur Verwendung bei der Behandlung oder Prophylaxe von Neuroinflammation, neurodegenerativen Krankheiten, Schmerz, Krebs und/oder mentalen Störungen bei einem Säuger.

15. Verbindung zur Verwendung nach Anspruch 14, wobei die Neuroinflammation, neurodegenerativen Krankheiten, Schmerz, Krebs und/oder mentalen Störungen aus multipler Sklerose, Alzheimer-Krankheit, Parkinson-Krankheit, amyotropher Lateralsklerose, traumatischer Hirnverletzung, Neurotoxizität, Schlaganfall, Epilepsie, Angst, Migräne, Depression, Leberzellenkarzinom, Kolonkarzinogenese, Eierstockkrebs, neuropathischem Schmerz, durch Chemotherapie induzierter Neuropathie, akutem Schmerz, chronischem Schmerz und/oder Spastizität in Verbindung mit Schmerz bei einem Säuger ausgewählt sind.

## Revendications

1. Composé de formule (Ic) 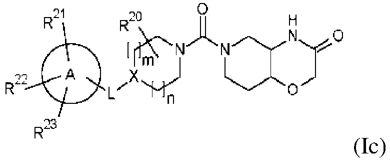 ou un sel pharmaceutiquement acceptable de celui-ci, dans lequel :
L est choisi parmi -CR¹R²-(CH₂)ₚ-, -(CH₂)ₚ-CR¹R²-, -OCR³R⁴-, -CR³R⁴O- et une liaison covalente ;
m représente 0, n représente 0 ou 1 et X représente CR²⁴ ; ou
m représente 1, n représente 1 ou 2 et X représente CR²⁴ ou N ;
p représente 0, 1 ou 2 ;
R¹ est choisi parmi un halogène, un alcoxy en C₁₋₆, un alkyle en C₁₋₆, un halogénoalkyle en C₁₋₆, un hydroxyalkyle en C₁₋₆, un alcoxy en C₁₋₆-alkyle en C₁₋₆-, un halogénoalcoxy en C₁₋₆, un halogénoalcoxy en C₁₋₆-alkyle en C₁₋₆-, un cyano et un groupe 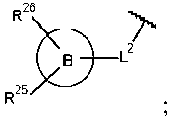 et R² est choisi parmi un hydrogène, un halogène et un hydroxy ; ou
R¹ et R², pris conjointement avec l'atome de carbone auquel ils sont liés, forment un cycloalkyle en C₃₋₁₂ ou un hétérocyclyle en C₂₋₉ ;
R³ est choisi parmi un alkyle en C₁₋₆, un halogénoalkyle en C₁₋₆, un cycloalkyle en C₃₋₁₂ et un aryle en C₆₋₁₄ ; et R⁴ représente un hydrogène ; ou
R³ et R⁴, pris conjointement avec l'atome de carbone auquel ils sont liés, forment un cycloalkyle en C₃₋₁₂ ou un hétérocyclyle en C₂₋₉ ;
R²⁰ représente un hydrogène ou un alkyle en C₁₋₆ ;
R²¹, R²² et R²³ sont indépendamment choisis parmi un hydrogène, un halogène, un cyano, un hydroxy, un alcoxy en C₁₋₆, un halogénoalcoxy en C₁₋₆, un aminoalcoxy en C₁₋₆, un alkyle en C₁₋₆, un halogénoalkyle en C₁₋₆, un hydroxyalkyle en C₁₋₆, un (halogénoalkyle en C₁₋₆)-hydroxyalkyle en C₁₋₆, un alcoxy en C₁₋₆-carbonylalkyle en C₁₋₆-, un alcoxy en C₁₋₆-carbonyl-NH-alcoxy en C₁₋₆-, un alcoxy en C₁₋₆-carbonyl-NH-(alcoxy en C₁₋₆)₂-alkyle C₁₋₆-C(O)-NH-alcoxy en C₁₋₆-, un alcoxy en C₁₋₆-carbonyl-NH-alcoxy C₁₋₆-alkyle en C₁₋₆-C(O)-NH-alcoxy en C₁₋₆-, SF₅, un (alkyle en C₁₋₆)₃Si-O-en en alkyle en C₁₋₆-, un groupe 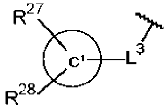 et un groupe 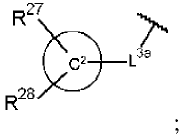
R²⁴ est choisi parmi un hydrogène, un halogène, un hydroxy, un halogénoalkyle en C₁₋₆ et un alkyle en C₁₋₆ ;
R²⁵ et R²⁶ sont indépendamment choisis parmi un hydrogène, un halogène, un alkyle en C₁₋₆, un alcoxy en C₁₋₆, un halogénoalkyle en C₁₋₆, un halogénoalcoxy en C₁₋₆, un alkyle en C₁₋₆-SO₂- et un cycloalkyle en C₃₋₁₂ ;
R²⁷ et R²⁸ sont indépendamment choisis parmi un hydrogène, un halogène, un alcoxy en C₁₋₆, un halogénoalcoxy en C₁₋₆, un alkyle en C₁₋₆, un halogénoalkyle en C₁₋₆, un hydroxyalkyle en C₁₋₆, un halogène, un hydroxy, un alkyle en C₁₋₆-sulfonyle, un carbamoyle, un cyano, un cycloalkylalcoxy en C₁₋₆-, un alkyle en C₁₋₆-NH-C(O)- et un cycloalkyle ;
A et B sont indépendamment choisis parmi un aryle en C₆₋₁₄, un hétéroaryle en C₁₋₁₃, un cycloalkyle en C₃₋₁₂ et un hétérocyclyle en C₂₋₉ ;
C¹ représente un cycloalkyle en C₃₋₁₂ ou un hétérocyclyle en C₂₋₉ ;
C² représente un aryle en C₆₋₁₄ ;
L² est choisi parmi une liaison covalente, -O-, -CH₂O-, -CH₂OCH₂- et -CH₂- ;
L³ est choisi parmi une liaison covalente, -O-, -CH₂O-, -OCH₂-, -CH₂OCH₂- et -CH₂- ; et
L^{3a} est choisi parmi une liaison covalente, -CH₂O-, -OCH₂-, -CH₂OCH₂- et -CH₂-.

2. Composé de formule (Ic) selon la revendication 1, ou un sel pharmaceutiquement acceptable de celui-ci, dans lequel :
m représente 0, n représente 0 ou 1 et X représente CR²⁴ ; ou
m représente 1, n représente 1 et X représente CR²⁴ ou N ; et
R²⁰ et R²⁴ représentent tous deux un hydrogène.

3. Composé de formule (Ic) selon l'une quelconque des revendications 1 et 2, ou un sel pharmaceutiquement acceptable de celui-ci, dans lequel :
L est choisi parmi -CR¹R²-(CH₂)ₚ-, -OCR³R⁴-, -CR³R⁴O- et une liaison covalente ;
p représente 0 ou 1 ;
R¹ est choisi parmi un halogène, un alcoxy en C₁₋₆, un alkyle en C₁₋₆, un halogénoalkyle en C₁₋₆, un hydroxyalkyle en C₁₋₆, un alcoxy en C₁₋₆-alkyle en C₁₋₆-, un halogénoalcoxy en C₁₋₆, un halogénoalcoxy en C₁₋₆-alkyle en C₁₋₆-, un cyano et un groupe 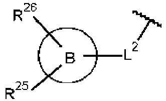 ; et R² est choisi parmi un hydrogène, un halogène et un hydroxy ; ou
R¹ et R², pris conjointement avec l'atome de carbone auquel ils sont liés, forment un cycloalkyle en C₃₋₁₂ ;
R³ représente un alkyle en C₁₋₆ ou un halogénoalkyle en C₁₋₆ ;
R⁴ représente un hydrogène ;
R²¹ est choisi parmi un hydrogène, un halogène, un hydroxy, un alcoxy en C₁₋₆, un halogénoalcoxy en C₁₋₆, un alkyle en C₁₋₆, un halogénoalkyle en C₁₋₆, un hydroxyalkyle en C₁₋₆, un (halogénoalkyle en C₁₋₆)-hydroxyalkyle en C₁₋₆, un alcoxy en C₁₋₆-carbonylalkyle en C₁₋₆-, un alcoxy en C₁₋₆-carbonyl-NH-alcoxy en C₁₋₆-, SF₅, un (alkyle en C₁₋₆)₃Si-O-alkyle en C₁₋₆-, un groupe 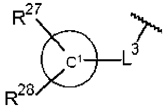 et un groupe 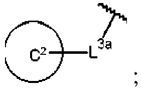
R²² est choisi parmi un hydrogène, un halogène, un alcoxy en C₁₋₆, un halogénoalcoxy en C₁₋₆, un alkyle en C₁₋₆ et un cyano ;
R²³ représente un hydrogène ou un halogène ;
R²⁵ est choisi parmi un hydrogène, un halogène, un alkyle en C₁₋₆, un alcoxy en C₁₋₆, un halogénoalkyle en C₁₋₆, un halogénoalcoxy en C₁₋₆, un alkyle en C₁₋₆-SO₂- et un cycloalkyle en C₃₋₁₂ ;
R²⁶ est choisi parmi un hydrogène, un alkyle en C₁₋₆ et un alcoxy en C₁₋₆ ;
R²⁷ est choisi parmi un hydrogène, un halogène, un alcoxy en C₁₋₆, un halogénoalcoxy en C₁₋₆, un alkyle en C₁₋₆, un halogénoalkyle en C₁₋₆, un hydroxyalkyle en C₁₋₆, un halogène, un hydroxy, un alkyle en C₁₋₆-sulfonyle, un carbamoyle, un cyano, un cycloalkylalcoxy en C₁₋₆- et un cycloalkyle ;
R²⁸ est choisi parmi un hydrogène, un alkyle en C₁₋₆ et un halogène ;
A représente un aryle en C₆₋₁₄ ou un hétéroaryle en C₁₋₁₃ ;
B représente un aryle en C₆₋₁₄ ou un hétéroaryle en C₁₋₁₃ ;
L² est choisi parmi une liaison covalente, -O- et -CH₂- ;
L³ est choisi parmi une liaison covalente, -CH₂O- et -CH₂- ; et
L^{3a} représente une liaison covalente ou -CH₂-.

4. Composé de formule (Ic) selon l'une quelconque des revendications 1 à 4, ou un sel pharmaceutiquement acceptable de celui-ci, dans lequel :
L est choisi parmi -CR¹R²-(CH₂)ₚ-, -OCR³R⁴-, -CR³R⁴O- et une liaison covalente ;
p représente 0 ;
R¹ est choisi parmi un halogène, un alkyle en C₁₋₆, un hydroxyalkyle en C₁₋₆, un alcoxy en C₁₋₆-alkyle en C₁₋₆-, un halogénoalcoxy en C₁₋₆ et un groupe 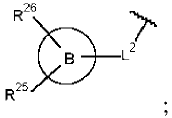
R² représente un hydrogène ou un halogène ;
R³ représente un alkyle en C₁₋₆ ;
R⁴ représente un hydrogène ;
R²¹ est choisi parmi un halogène, un alcoxy en C₁₋₆, un halogénoalcoxy en C₁₋₆, un alkyle en C₁₋₆, un halogénoalkyle en C₁₋₆, SF₅, un aryle en C₆₋₁₄ et un groupe 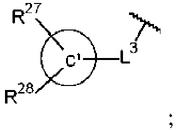
R²² est choisi parmi un hydrogène, un halogène, un alcoxy en C₁₋₆ et un halogénoalcoxy en C₁₋₆ ;
R²³ représente un hydrogène ou un halogène ;
R²⁵ est choisi parmi un hydrogène, un halogène, un alcoxy en C₁₋₆ et un cycloalkyle en C₃₋₁₂ ;
R²⁶ représente un hydrogène ou un alcoxy en C₁₋₆ ;
R²⁷ est choisi parmi un hydrogène, un halogénoalcoxy en C₁₋₆, un alkyle en C₁₋₆, un halogénoalkyle en C₁₋₆, un alkyle en C₁₋₆-NH-C(O)- et un halogène ;
R²⁸ est choisi parmi un hydrogène, un alkyle en C₁₋₆ et un halogène ;
A représente un aryle en C₆₋₁₄ ou un hétéroaryle en C₁₋₁₃ ;
B représente un aryle en C₆₋₁₄ ou un hétéroaryle en C₁₋₁₃ ;
C¹ représente un cycloalkyle en C₃₋₁₂ ou un hétérocyclyle en C₂₋₉ ;
L² représente une liaison covalente ; et
L³ représente une liaison covalente ou -CH₂-.

5. Composé de formule (Ic) selon l'une quelconque des revendications 1 à 4, ou un sel pharmaceutiquement acceptable de celui-ci, dans lequel :
L est choisi parmi -CR¹R²-(CH₂)ₚ-, -OCR³R⁴-, -CR³R⁴O- et une liaison covalente ;
p représente 0 ;
R¹ est choisi parmi un 2-méthoxyéthyle, un méthyle, un 2,2,2-trifluoroéthoxy, un fluor, un 2-hydroxyéthyle et un groupe 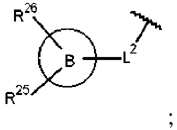
R² représente un hydrogène ou un fluor ;
R³ représente un méthyle ;
R⁴ représente un hydrogène ;
R²¹ est choisi parmi un fluor, un chlore, un brome, un méthyle, un méthoxy, un *tert-*butyle, un propyle, un trifluorométhoxy, un 2-fluoroéthoxy, un 2,2,2-trifluoroéthoxy, un trifluorométhyle, un 2,2,2-trifluoroéthyle, un 1,1-difluoroéthyle, SF₅, un phényle et un groupe 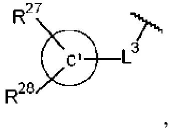 dans lequel R²⁷, R²⁸, C¹ et L³ sont tels que définis ici ;
R²² est choisi parmi un hydrogène, un fluor, un chlore, un méthoxy, un méthyle et un trifluorométhyle ;
R²³ représente un hydrogène ou un fluor ;
R²⁵ est choisi parmi un hydrogène, un méthoxy, un fluor et un cyclopropyle ;
R²⁶ représente un hydrogène ou un méthoxy ;
R²⁷ est choisi parmi un méthyle, un trifluorométhoxy, un trifluorométhyle, un 2,2,2-trifluoro-1-méthyl-éthoxy, un 2,2,2-trifluoro-1,1-diméthyl-éthoxy, un 2,2,2-trifluoroéthoxy, un fluor et un chlore ;
R²⁸ est choisi parmi un hydrogène, un méthyle, un fluor et un chlore ;
A est choisi parmi un phényle, un indol-3-yle, un 2-pyridyle et un 3-pyridyle ;
B représente un phényle ou un 1,2,4-oxadiazol-5-yle ;
C¹ est choisi parmi un azétidin-1-yle, un pyrrolidin-1-yle, un cyclopropyle et un oxétan-3-yle ;
L² représente une liaison covalente ; et
L³ représente une liaison covalente ou -CH₂-.

6. Composé de formule (Ic) selon l'une quelconque des revendications 1 à 3, ou un sel pharmaceutiquement acceptable de celui-ci, dans lequel C² représente un phényle.

7. Composé de formule (Ic) selon l'une quelconque des revendications 1 à 3, ou un sel pharmaceutiquement acceptable de celui-ci, dans lequel L^{3a} représente une liaison covalente.

8. Composé de formule (Ic) selon la revendication 1, ou un sel pharmaceutiquement acceptable de celui-ci, dans lequel :
m représente 0, n représente 0 ou 1 et X représente CR²⁴ ; ou
m représente 1, n représente 1 et X représente CR²⁴ ou N ;
L est choisi parmi -CR¹R²-(CH₂)ₚ-, -OCHR³-, -CHR³O- et une liaison covalente ;
p représente 0 ou 1 ;
R¹ est choisi parmi un halogène, un alcoxy en C₁₋₆, un alkyle en C₁₋₆, un halogénoalkyle en C₁₋₆, un hydroxyalkyle en C₁₋₆, un alcoxy en C₁₋₆-alkyle en C₁₋₆-, un halogénoalcoxy en C₁₋₆, un halogénoalcoxy en C₁₋₆-alkyle en C₁₋₆-, un cyano et un groupe 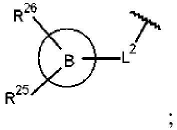 et R² est choisi parmi un hydrogène, un halogène et un hydroxy ; ou
R¹ et R², pris conjointement avec l'atome de carbone auquel ils sont liés, forment un cycloalkyle en C₃₋₁₂ ;
R³ représente un alkyle en C₁₋₆ ou un halogénoalkyle en C₁₋₆ ;
R²⁰ représente un hydrogène ou un alkyle en C₁₋₆ ;
R²¹ est choisi parmi un hydrogène, un halogène, un hydroxy, un alcoxy en C₁₋₆, un halogénoalcoxy en C₁₋₆, un alkyle en C₁₋₆, un halogénoalkyle en C₁₋₆, un hydroxyalkyle en C₁₋₆, un (halogénoalkyle en C₁₋₆)-hydroxyalkyle en C₁₋₆, un alcoxy en C₁₋₆-carbonylalkyle en C₁₋₆-, un alcoxy en C₁₋₆-carbonyl-NH-alcoxy en C₁₋₆-, SF₅, un (alkyle en C₁₋₆)₃Si-O-alkyle en C₁₋₆-, un groupe 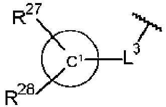 et un groupe 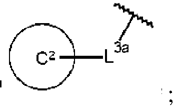
R²² est choisi parmi un hydrogène, un halogène, un alcoxy en C₁₋₆, un halogénoalcoxy en C₁₋₆, un alkyle en C₁₋₆ et un cyano ;
R²³ représente un hydrogène ou un halogène ;
R²⁴ est choisi parmi un hydrogène, un halogène, un hydroxy et un alkyle en C₁₋₆ ;
R²⁵ est choisi parmi un hydrogène, un halogène, un alkyle en C₁₋₆, un alcoxy en C₁₋₆, un halogénoalkyle en C₁₋₆, un halogénoalcoxy en C₁₋₆, un alkyle en C₁₋₆-SO₂- et un cycloalkyle en C₃₋₁₂ ;
R²⁶ est choisi parmi un hydrogène, un alkyle en C₁₋₆ et un alcoxy en C₁₋₆ ;
R²⁷ est choisi parmi un hydrogène, un halogène, un alcoxy en C₁₋₆, un halogénoalcoxy en C₁₋₆, un alkyle en C₁₋₆, un halogénoalkyle en C₁₋₆, un hydroxyalkyle en C₁₋₆, un halogène, un hydroxy, un alkyle en C₁₋₆-sulfonyle, un carbamoyle, un cyano, un cycloalkylalcoxy en C₁₋₆- et un cycloalkyle ;
R²⁸ est choisi parmi un hydrogène, un alkyle en C₁₋₆ et un halogène ;
A représente un aryle en C₆₋₁₄ ou un hétéroaryle en C₁₋₁₃ ;
B représente un aryle en C₆₋₁₄ ou un hétéroaryle en C₁₋₁₃ ;
C¹ représente un cycloalkyle en C₃₋₁₂ ou un hétérocyclyle en C₂₋₉ ;
C² représente un aryle en C₆₋₁₄ ;
L² est choisi parmi une liaison covalente, -O- et -CH₂- ;
L³ est choisi parmi une liaison covalente, -CH₂O- et -CH₂- ; et
L^{3a} représente une liaison covalente ou -CH₂-.

9. Composé de formule (Ic) selon l'une quelconque des revendications 1 à 8, ou un sel pharmaceutiquement acceptable de celui-ci, choisi parmi :
la rac-(4aR,8aS)-6-(4-benzhydrylpipéridine-1-carbonyl)-4,4a,5,7,8,8a-hexahydropyrido[4,3-b][1,4]oxazin-3-one ;
la rac-(4aR,8aS)-6-(4-(5-chloro-1-(cyclopropylméthyl)-1H-indol-3-yl)pipéridine-1-carbonyl)hexahydro-2H-pyrido[4,3-b][1,4]oxazin-3(4H)-one ;
la rac-(4aR,8aS)-6-(4-(5-chloro-1-méthyl-1H-indol-3-yl)pipéridine-1-carbonyl)hexahydro-2H-pyrido[4,3-b][1,4]oxazin-3(4H)-one ;
la rac-(4aR,8aS)-6-(4-(9H-fluorén-9-yl)pipéridine-1-carbonyl)hexahydro-2H-pyrido[4,3-b][1,4]oxazin-3(4H)-one ;
la (+)-cis-6-[4-(6-fluoro-1H-indol-3-yl)pipéridine-1-carbonyl]-4,4a,5,7,8,8a-hexahydropyrido[4,3-b][1,4]oxazin-3-one ;
la (4aR,8aS)-6-(4-(1-(4-fluorophényl)-3-méthoxypropyl)pipéridine-1-carbonyl)hexahydro-2H-pyrido[4,3-b][1,4]oxazin-3(4H)-one ;
la rac-(4aR,8aS)-6-[4-(6-fluoro-1H-indol-3-yl)pipéridine-1-carbonyl]-4,4a,5,7,8,8a-hexahydropyrido[4,3-b][1,4]oxazin-3-one ;
la (4aR,8aS)-6-(4-((4-fluorophényl)(méthoxy)méthyl)pipéridine-1-carbonyl)hexahydro-2H-pyrido[4,3-b][1,4]oxazin-3(4H)-one ;
la rac-(4aR,8aS)-6-(4-(5-fluorobenzo[d]isoxazol-3-yl)pipéridine-1-carbonyl)hexahydro-2H-pyrido[4,3-b][1,4]oxazin-3(4H)-one ;
la cis-6-(4-((4-chlorophényl)(phényl)méthyl)pipérazine-1-carbonyl)hexahydro-2H-pyrido[4,3-b][1,4]oxazin-3(4H)-one ;
la (+)-cis-6-(4-(bis(4-fluorophényl)méthyl)pipérazine-1-carbonyl)hexahydro-2H-pyrido[4,3-b][1,4]oxazin-3(4H)-one ;
la rac-(4aR,8aS)-6-[4-[bis(4-fluorophényl)méthyl]pipérazine-1-carbonyl]-4,4a,5,7,8,8a-hexahydropyrido[4,3-b][1,4]oxazin-3-one ;
la (-)-cis-6-(4-(bis(4-fluorophényl)méthyl)pipérazine-1-carbonyl)hexahydro-2H-pyrido[4,3-b][1,4]oxazin-3(4H)-one ;
la rac-(4aR,8aS)-6-(4-(5-chloro-1H-indol-3-yl)pipérazine-1-carbonyl)hexahydro-2H-pyrido[4,3-b][1,4]oxazin-3(4H)-one ;
la rac-(4aR,8aS)-6-(4-(1-phényléthyl)pipérazine-1-carbonyl)hexahydro-2H-pyrido[4,3-b][1,4]oxazin-3(4H)-one ;
la rac-(4aR,8aS)-6-(4-(1-méthyl-1H-indazol-5-yl)pipéridine-1-carbonyl)hexahydro-2H-pyrido[4,3-b][1,4]oxazin-3(4H)-one ;
la rac-(4aR,8aS)-6-[4-[(4-fluorophényl)-phénylméthyl]pipérazine-1-carbonyl]-4,4a,5,7,8,8a-hexahydropyrido[4,3-b][1,4]oxazin-3-one ;
la (+)- ou (-)-cis-6-(4-(5-chloro-1-cyclopropyl-1H-indol-3-yl)pipéridine-1-carbonyl)hexahydro-2H-pyrido[4,3-b][1,4]oxazin-3(4H)-one ;
la rac-(4aR,8aS)-6-(4-(5-chloro-1-(1-chloro-3-hydroxypropan-2-yl)-1H-indol-3-yl)pipéridine-1-carbonyl)hexahydro-2H-pyrido[4,3-b][1,4]oxazin-3(4H)-one ;
la (+)- ou (-)-cis-6-(4-(bis(4-fluorophényl)méthyl)pipéridine-1-carbonyl)hexahydro-2H-pyrido[4,3-b][1,4]oxazin-3(4H)-one ;
la (+)- ou (-)-cis-6-(4-(5-chloro-1-(oxétan-3-yl)-1H-indol-3-yl)pipéridine-1-carbonyl)hexahydro-2H-pyrido[4,3-b][1,4]oxazin-3(4H)-one ;
la (+)- ou (-)-cis-6-(4-(5-chloro-1-(oxétan-3-ylméthyl)-1H-indol-3-yl)pipéridine-1-carbonyl)hexahydro-2H-pyrido[4,3-b][1,4]oxazin-3(4H)-one ;
la (+)- ou (-)-cis-6-(4-(1-(2-((tert-butyldiméthylsilyl)oxy)éthyl)-5-chloro-1H-indol-3-yl)pipéridine-1-carbonyl)hexahydro-2H-pyrido[4,3-b][1,4]oxazin-3(4H)-one ;
la (+)- ou (-)-cis-6-(4-(5-chloro-1-(2-hydroxyéthyl)-1H-indol-3-yl)pipéridine-1-carbonyl)hexahydro-2H-pyrido[4,3-b][1,4]oxazin-3(4H)-one ;
la rac-cis-6-(4-(1-(4-fluorophényl)-3-hydroxypropyl)pipéridine-1-carbonyl)hexahydro-2H-pyrido[4,3-b][1,4]oxazin-3(4H)-one ;
la rac-(4aR,8aS)-6-(4-(1-(4-(trifluorométhyl)phényl)éthyl)pipéridine-1-carbonyl)hexahydro-2H-pyrido[4,3-b][1,4]oxazin-3(4H)-one ;
la rac-(4aR,8aS)-6-(4-(1-(2-chloro-4-fluorophénoxy)éthyl)pipéridine-1-carbonyl)hexahydro-2H-pyrido[4,3-b][1,4]oxazin-3(4H)-one ;
la (4aR,8aS)-6-(4-(5-(trifluorométhyl)pyridin-3-yl)pipéridine-1-carbonyl)hexahydro-2H-pyrido[4,3-b][1,4]oxazin-3(4H)-one ;
la (4aR,8aS)-6-(4-(difluoro(4-(trifluorométhyl)phényl)méthyl)pipéridine-1-carbonyl)hexahydro-2H-pyrido[4,3-b][1,4]oxazin-3(4H)-one ;
la (+)-(4aR,8aS)-6-(4-((R ou S)-1-(4-fluorophényl)-3-hydroxypropyl)pipéridine-1-carbonyl)hexahydro-2H-pyrido[4,3-b][1,4]oxazin-3(4H)-one ;
la (-)-(4aR,8aS)-6-(4-((S ou R)-1-(4-fluorophényl)-3-hydroxypropyl)pipéridine-1-carbonyl)hexahydro-2H-pyrido[4,3-b][1,4]oxazin-3(4H)-one ;
la (+) ou (-)-cis-6-(4-((S ou R)-1-(2-chloro-4-fluorophénoxy)éthyl)pipéridine-1-carbonyl)hexahydro-2H-pyrido[4,3-b][1,4]oxazin-3(4H)-one ;
la (+) ou (-)-cis-6-(4-((R ou S)-1-(2-chloro-4-fluorophénoxy)éthyl)pipéridine-1-carbonyl)hexahydro-2H-pyrido[4,3-b][1,4]oxazin-3(4H)-one ;
la (+) ou (-)-cis-6-(4-(5-méthoxypyridin-3-yl)pipéridine-1-carbonyl)hexahydro-2H-pyrido[4,3-b][1,4]oxazin-3(4H)-one ;
la (+) ou (-)-cis-6-(4-(bis(4-fluorophényl)méthyl)pipéridine-1-carbonyl)hexahydro-2H-pyrido[4,3-b][1,4]oxazin-3(4H)-one ;
la (+) ou (-)-cis-6-(4-((4-chlorophényl)difluorométhyl)pipéridine-1-carbonyl)hexahydro-2H-pyrido[4,3-b][1,4]oxazin-3(4H)-one ;
la (+) ou (-)-cis-6-(4-(5-(trifluorométhoxy)pyridin-2-yl)pipéridine-1-carbonyl)hexahydro-2H-pyrido[4,3-b][1,4]oxazin-3(4H)-one ;
la (+) ou (-)-cis-6-(4-((2-chloro-4-fluorophényl)difluorométhyl)pipéridine-1-carbonyl)hexahydro-2H-pyrido[4,3-b][1,4]oxazin-3(4H)-one ;
la (+) ou (-)-cis-6-(4-(5-éthylpyridin-3-yl)pipéridine-1-carbonyl)hexahydro-2H-pyrido[4,3-b][1,4]oxazin-3(4H)-one ;
la (+) ou (-)-cis-6-(4-(5-(1,1-difluoroéthyl)pyridin-2-yl)pipéridine-1-carbonyl)hexahydro-2H-pyrido[4,3-b][1,4]oxazin-3(4H)-one ;
la (+) ou (-)-cis-6-(4-(6-chloro-1-méthyl-1H-indazol-3-yl)pipéridine-1-carbonyl)hexahydro-2H-pyrido[4,3-b][1,4]oxazin-3(4H)-one ;
la (4aR,8aS)-6-((3R ou 3S)-4-(5-(1,1-difluoroéthyl)pyridin-2-yl)-3-méthylpipéridine-1-carbonyl)hexahydro-2H-pyrido[4,3-b][1,4]oxazin-3(4H)-one ;
la (4aR,8aS)-6-((3S ou 3R)-4-(5-(1,1-difluoroéthyl)pyridin-2-yl)-3-méthylpipéridine-1-carbonyl)hexahydro-2H-pyrido[4,3-b][1,4]oxazin-3(4H)-one ;
la (4aR,8aS)-6-((4R ou 4S)-(5-(1,1-difluoroéthyl)pyridin-2-yl)-3-méthylpipéridine-1-carbonyl)hexahydro-2H-pyrido[4,3-b][1,4]oxazin-3(4H)-one ;
la (4aR,8aS)-6-(4-((R ou S)-1-(4-(trifluorométhyl)phényl)éthyl)pipéridine-1-carbonyl)hexahydro-2H-pyrido[4,3-b][1,4]oxazin-3(4H)-one ;
la (4aR,8aS)-6-(4-((S ou R)-1-(4-(trifluorométhyl)phényl)éthyl)pipéridine-1-carbonyl)hexahydro-2H-pyrido[4,3-b][1,4]oxazin-3(4H)-one ;
le 2-(4-fluorophényl)-2-(1-((4aR,8aS)-3-oxooctahydro-2H-pyrido[4,3-b][1,4]oxazine-6-carbonyl)pipéridin-4-yl)acétonitrile ;
la (4aR,8aS)-6-(4-(2,2,2-trifluoro-1-phényléthyl)pipérazine-1-carbonyl)hexahydro-2H-pyrido[4,3-b][1,4]oxazin-3(4H)-one ;
la (4aR,8aS)-6-(4-((4-fluorophényl)(2,2,2-trifluoroéthoxy)méthyl)pipéridine-1-carbonyl)hexahydro-2H-pyrido[4,3-b][1,4]oxazin-3(4H)-one ;
la (4aR,8aS)-6-(4-(1-(4-fluorophényl)-2-(3-méthyl-1,2,4-oxadiazol-5-yl)éthyl)pipéridine-1-carbonyl)hexahydro-2H-pyrido[4,3-b][1,4]oxazin-3(4H)-one ;
la (4aR,8aS)-6-(4-(1-(4-(trifluorométhyl)phényl)cyclopropyl)pipéridine-1-carbonyl)hexahydro-2H-pyrido[4,3-b][1,4]oxazin-3(4H)-one ;
la (4aR,8aS)-6-(4-(1-(2,4-difluorophényl)-2,2,2-trifluoroéthyl)pipérazine-1-carbonyl)hexahydro-2H-pyrido[4,3-b][1,4]oxazin-3(4H)-one ;
la (4aR,8aS)-6-(3-(bis(4-fluorophényl)(hydroxy)méthyl)azétidine-1-carbonyl)hexahydro-2H-pyrido[4,3-b][1,4]oxazin-3(4H)-one ;
la (4aR,8aS)-6-(3-(fluorobis(4-fluorophényl)méthyl)azétidine-1-carbonyl)hexahydro-2H-pyrido[4,3-b][1,4]oxazin-3(4H)-one ;
la (4aR,8aS)-6-(4-(1-(4-fluorophényl)-2-(2,2,2-trifluoroéthoxy)éthyl)pipéridine-1-carbonyl)hexahydro-2H-pyrido[4,3-b][1,4]oxazin-3(4H)-one ;
la (4aR,8aS)-6-(4-((R ou S)-(4-fluorophényl)(2,2,2-trifluoroéthoxy)méthyl)pipéridine-1-carbonyl)hexahydro-2H-pyrido[4,3-b][1 ,4]oxazin-3(4H)-one ;
la (4aR,8aS)-6-(4-((S ou R)-(4-fluorophényl)(2,2,2-trifluoroéthoxy)méthyl)pipéridine-1-carbonyl)hexahydro-2H-pyrido[4,3-b][1 ,4]oxazin-3(4H)-one ;
la (4aR,8aS)-6-(4-((R ou S)-1-(4-fluorophényl)-2-(3-méthyl-1,2,4-oxadiazol-5-yl)éthyl)pipéridine-1-carbonyl)hexahydro-2H-pyrido[4,3-b][1,4]oxazin-3(4H)-one ;
la (4aR,8aS)-6-(4-((S ou R)-1-(4-fluorophényl)-2-(3-méthyl-1,2,4-oxadiazol-5-yl)éthyl)pipéridine-1-carbonyl)hexahydro-2H-pyrido[4,3-b][1,4]oxazin-3(4H)-one ;
la (4aR,8aS)-6-(4-(2-cyclopropylpyridin-4-yl)pipéridine-1-carbonyl)hexahydro-2H-pyrido[4,3-b][1,4]oxazin-3(4H)-one ;
la (4aR,8aS)-6-[4-[(S ou R)-(4-fluorophényl)-(3-méthoxyphényl)méthyl]pipéridine-1-carbonyl]-4,4a,5,7,8,8a-hexahydropyrido[4,3-b][1,4]oxazin-3-one ;
la (4aR,8aS)-6-(4-((R ou S)-(4-fluorophényl)(3-méthoxyphényl)méthyl)pipéridine-1-carbonyl)hexahydro-2H-pyrido[4,3-b][1,4]oxazin-3(4H)-one ;
la (4aR,8aS)-6-(4-((S ou R)-(3-méthoxyphényl)(phényl)méthyl)pipéridine-1-carbonyl)hexahydro-2H-pyrido[4,3-b][1,4]oxazin-3(4H)-one ;
la (4aR,8aS)-6-(4-((R ou S)-(3-méthoxyphényl)(phényl)méthyl)pipéridine-1-carbonyl)hexahydro-2H-pyrido[4,3-b][1,4]oxazin-3(4H)-one ;
la (4aR,8aS)-6-(4-((S ou R)-(3-(2-fluoroéthoxy)phényl)(phényl)méthyl)pipéridine-1-carbonyl)hexahydro-2H-pyrido[4,3-b][1,4]oxazin-3(4H)-one ;
la (4aR,8aS)-6-(4-((S)-(4-fluorophényl)(phényl)méthyl)pipéridine-1-carbonyl)hexahydro-2H-pyrido[4,3-b][1,4]oxazin-3(4H)-one ;
la (4aR,8aS)-6-(4-((S ou R)-(4-fluorophényl)(phényl)méthyl)pipéridine-1-carbonyl)hexahydro-2H-pyrido[4,3-b][1,4]oxazin-3(4H)-one ;
la (4aR,8aS)-6-[4-[(S ou R)-(4-fluorophényl)-(4-méthylphényl)méthyl]pipéridine-1-carbonyl]-4,4a,5,7,8,8a-hexahydropyrido[4,3-b][1,4]oxazin-3-one ;
la (4aR,8aS)-6-[4-[(R ou S)-[4-(2-fluoroéthoxy)phényl]-phénylméthyl]pipéridine-1-carbonyl]-4,4a,5,7,8,8a-hexahydropyrido[4,3-b][1,4]oxazin-3-one ;
la (4aR,8aS)-6-(4-((S ou R)-(4-fluorophényl)(4-méthoxyphényl)méthyl)pipéridine-1-carbonyl)hexahydro-2H-pyrido[4,3-b][1,4]oxazin-3(4H)-one ;
la (4aR,8aS)-6-(4-((R ou S)-(4-fluorophényl)(p-tolyl)méthyl)pipéridine-1-carbonyl)hexahydro-2H-pyrido[4,3-b][1,4]oxazin-3(4H)-one ;
la (4aR,8aS)-6-(4-((S ou R)-(3,4-diméthoxyphényl)(4-fluorophényl)méthyl)pipéridine-1-carbonyl)hexahydro-2H-pyrido[4,3-b][1 ,4]oxazin-3(4H)-one ;
la (4aR,8aS)-6-(4-((R ou S)-(3,4-diméthoxyphényl)(phényl)méthyl)pipéridine-1-carbonyl)hexahydro-2H-pyrido[4,3-b][1,4]oxazin-3(4H)-one ;
la (4aR,8aS)-6-(3-(1-(2-chloro-4-(trifluorométhyl)phénoxy)éthyl)azétidine-1-carbonyl)hexahydro-2H-pyrido[4,3-b][1,4]oxazin-3(4H)-one ;
la (4aR,8aS)-6-(3-(1-(2-chloro-4-(trifluorométhyl)phénoxy)éthyl)azétidine-1-carbonyl)hexahydro-2H-pyrido[4,3-b][1,4]oxazin-3(4H)-one ;
la (4aR,8aS)-6-(4-((R ou S)-(4-fluorophényl)(phényl)méthyl)pipéridine-1-carbonyl)hexahydro-2H-pyrido[4,3-b][1,4]oxazin-3(4H)-one ;
la (4aR,8aS)-6-(4-((S ou R)-phényl(m-tolyl)méthyl)pipéridine-1-carbonyl)hexahydro-2H-pyrido[4,3-b][1,4]oxazin-3(4H)-one ;
la (4aR,8aS)-6-[4-[(S ou R)-[4-(2-fluoroéthoxy)phényl]-phénylméthyl]pipéridine-1-carbonyl]-4,4a,5,7,8,8a-hexahydropyrido[4,3-b][1,4]oxazin-3-one ;
la (4aR,8aS)-6-(4-((S ou R)-((S)-3-méthylcyclohexa-2,4-dién-1-yl)(phényl)méthyl)pipéridine-1-carbonyl)hexahydro-2H-pyrido[4,3-b][1,4]oxazin-3(4H)-one ;
la (4aR,8aS)-6-(4-(1-(2-chloro-4-fluorophénoxy)-2,2,2-trifluoroéthyl)pipéridine-1-carbonyl)hexahydro-2H-pyrido[4,3-b][1,4]oxazin-3(4H)-one ;
la (4aR,8aS)-6-(3-(bis(4-fluorophényl)méthyl)azétidine-1-carbonyl)hexahydro-2H-pyrido[4,3-b][1,4]oxazin-3(4H)-one ;
la (4aR,8aS)-6-[3-[1-[4-(trifluorométhyl)phényl]éthyl]azétidine-1-carbonyl]-4,4a,5,7,8,8a-hexahydropyrido[4,3-b][1,4]oxazin-3-one ;
la (4aR,8aS)-6-[4-[(S ou R)-phényl(p-tolyl)méthyl]pipéridine-1-carbonyl]-4,4a,5,7,8,8a-hexahydropyrido[4,3-b][1,4]oxazin-3-one ;
la (4aR,8aS)-6-(4-((R ou S)-phényl(p-tolyl)méthyl)pipéridine-1-carbonyl)hexahydro-2H-pyrido[4,3-b][1,4]oxazin-3(4H)-one ;
la (4aR,8aS)-6-(3-benzhydrylazétidine-1-carbonyl)hexahydro-2H-pyrido[4,3-b][1,4]oxazin-3(4H)-one ;
la (4aR,8aS)-6-(4-((S ou R)-(3,4-diméthoxyphényl)(phényl)méthyl)pipéridine-1-carbonyl)hexahydro-2H-pyrido[4,3-b][1,4]oxazin-3(4H)-one ;
la (4aR,8aS)-6-(4-((R ou S)-(3,4-diméthoxyphényl)(4-fluorophényl)méthyl)pipéridine-1-carbonyl)hexahydro-2H-pyrido[4,3-b][1,4]oxazin-3(4H)-one ;
la (4aR,8aS)-6-(4-((R ou S)-(4-fluorophényl)(4-méthoxyphényl)méthyl)pipéridine-1-carbonyl)hexahydro-2H-pyrido[4,3-b][1,4]oxazin-3(4H)-one ;
la (4aR,8aS)-6-[3-[1-[4-(trifluorométhyl)phényl]éthoxy]azétidine-1-carbonyl]-4,4a,5,7,8,8a-hexahydropyrido[4,3-b][1,4]oxazin-3-one ;
la (4aR,8aS)-6-(3-(1-(2-fluoro-4-(trifluorométhyl)phénoxy)éthyl)azétidine-1-carbonyl)hexahydro-2H-pyrido[4,3-b][1,4]oxazin-3(4H)-one ;
la (4aR,8aS)-6-(3-(1-(4-(trifluorométhyl)phénoxy)éthyl)azétidine-1-carbonyl)hexahydro-2H-pyrido[4,3-b][1,4]oxazin-3(4H)-one ;
la (4aR,8aS)-6-[4-[1-[4-(trifluorométhyl)phényl]éthoxy]pipéridine-1-carbonyl]-4,4a,5,7,8,8a-hexahydropyrido[4,3-b][1,4]oxazin-3-one ;
la (4aR,8aS)-6-(4-((R ou S)-(3-cyclopropyl-1,2,4-oxadiazol-5-yl)(4-fluorophényl)méthyl)pipéridine-1-carbonyl)hexahydro-2H-pyrido[4,3-b][1,4]oxazin-3(4H)-one ;
la (4aR,8aS)-6-(4-((S ou R)-(3-cyclopropyl-1,2,4-oxadiazol-5-yl)(4-fluorophényl)méthyl)pipéridine-1-carbonyl)hexahydro-2H-pyrido[4,3-b][1,4]oxazin-3(4H)-one ;
la (4aR,8aS)-6-(3-((R ou S)-1-(2-fluoro-4-(trifluorométhyl)phénoxy)éthyl)azétidine-1-carbonyl)hexahydro-2H-pyrido[4,3-b][1,4]oxazin-3(4H)-one ;
la (4aR,8aS)-6-(3-((S ou R)-1-(2-fluoro-4-(trifluorométhyl)phénoxy)éthyl)azétidine-1-carbonyl)hexahydro-2H-pyrido[4,3-b][1,4]oxazin-3(4H)-one ;
la (4aR,8aS)-6-(3-((R ou S)-1-(4-(trifluorométhyl)phénoxy)éthyl)azétidine-1-carbonyl)hexahydro-2H-pyrido[4,3-b][1,4]oxazin-3(4H)-one ;
la (4aR,8aS)-6-(3-((S ou R)-1-(4-(trifluorométhyl)phénoxy)éthyl)azétidine-1-carbonyl)hexahydro-2H-pyrido[4,3-b][1,4]oxazin-3(4H)-one ;
la (4aR,8aS)-6-(4-((R ou S)-(4-(2-fluoroéthoxy)phényl)(4-fluorophényl)méthyl)pipéridine-1-carbonyl)hexahydro-2H-pyrido[4,3-b][1,4]oxazin-3(4H)-one ;
la (4aR,8aS)-6-(4-(5-méthyl-6-(trifluorométhyl)pyridin-3-yl)pipéridine-1-carbonyl)hexahydro-2H-pyrido[4,3-b][1,4]oxazin-3(4H)-one ;
la (4aR,8aS)-6-(4-((4-fluorophényl)(3-(trifluorométhyl)-1,2,4-oxadiazol-5-yl)méthyl)pipéridine-1-carbonyl)hexahydro-2H-pyrido[4,3-b][1,4]oxazin-3(4H)-one ;
la (4aR,8aS)-6-(3-((S ou R)-1-(4-(trifluorométhyl)phényl)éthyl)azétidine-1-carbonyl)hexahydro-2H-pyrido[4,3-b][1,4]oxazin-3(4H)-one ;
la (4aR,8aS)-6-(3-((R ou S)-1-(4-(trifluorométhyl)phényl)éthyl)azétidine-1-carbonyl)hexahydro-2H-pyrido[4,3-b][1,4]oxazin-3(4H)-one ;
la (4aR,8aS)-6-[3-(S ou R)-[(4-fluorophényl)-(2,2,2-trifluoroéthoxy)méthyl]azétidine-1-carbonyl]-4,4a,5,7,8,8a-hexahydropyrido[4,3-b][1,4]oxazin-3-one ;
la (4aR,8aS)-6-[3-(R ou S)-[(4-fluorophényl)-(2,2,2-trifluoroéthoxy)méthyl]azétidine-1-carbonyl]-4,4a,5,7,8,8a-hexahydropyrido[4,3-b][1,4]oxazin-3-one ;
la (4aR,8aS)-6-(4-(5,6,7,8-tétrahydroquinolin-4-yl)pipéridine-1-carbonyl)hexahydro-2H-pyrido[4,3-b][1,4]oxazin-3(4H)-one ;
la (4aR,8aS)-6-(4-((R ou S)-(3-(2-fluoroéthoxy)phényl)(phényl)méthyl)pipéridine-1-carbonyl)hexahydro-2H-pyrido[4,3-b][1,4]oxazin-3(4H)-one ;
la (4aR,8aS)-6-(4-((S ou R)-(4-(2-fluoroéthoxy)phényl)(4-fluorophényl)méthyl)pipéridine-1-carbonyl)hexahydro-2H-pyrido[4,3-b][1,4]oxazin-3(4H)-one ;
la (4aR,8aS)-6-(3-(4-bromophényl)azétidine-1-carbonyl)hexahydro-2H-pyrido[4,3-b][1,4]oxazin-3(4H)-one ;
la (4aR,8aS)-6-(3-(4'-chloro-[1,1'-biphényl]-4-yl)azétidine-1-carbonyl)hexahydro-2H-pyrido[4,3-b][1,4]oxazin-3(4H)-one ;
la (4aR,8aS)-6-(3-(2'-chloro-[1,1'-biphényl]-4-yl)azétidine-1-carbonyl)hexahydro-2H-pyrido[4,3-b][1,4]oxazin-3(4H)-one ;
la (4aR,8aS)-6-(3-(2',4'-dichloro-[1,1'-biphényl]-4-yl)azétidine-1-carbonyl)hexahydro-2H-pyrido[4,3-b][1,4]oxazin-3(4H)-one ;
la (4aR,8aS)-6-(3-(1-(R ou S)-(4-(trifluorométhyl)phényl)éthoxy)azétidine-1-carbonyl)hexahydro-2H-pyrido[4,3-b][1,4]oxazin-3(4H)-one ;
la (4aR,8aS)-6-(3-(1-(S ou R)-(4-(trifluorométhyl)phényl)éthoxy)azétidine-1-carbonyl)hexahydro-2H-pyrido[4,3-b][1,4]oxazin-3(4H)-one ;
la (4aR,8aS)-6-[3-(S ou R)-[1-(2-chloro-4-fluoro-phényl)éthoxy]azétidine-1-carbonyl]-4,4a,5,7,8,8a-hexahydropyrido[4,3-b][1,4]oxazin-3-one ;
la (4aR,8aS)-6-[3-(R ou S)-[1-(2-chloro-4-fluoro-phényl)éthoxy]azétidine-1-carbonyl]-4,4a,5,7,8,8a-hexahydropyrido[4,3-b][1,4]oxazin-3-one ;
la (4aR,8aS)-6-[4-[(R ou S)-(3,4-diméthoxyphényl)-(2-pyridyl)méthyl]pipéridine-1-carbonyl]-4,4a,5,7,8,8a-hexahydropyrido[4,3-b][1,4]oxazin-3-one ;
la (4aR,8aS)-6-[4-[(S ou R)-(3,4-diméthoxyphényl)-(2-pyridyl)méthyl]pipéridine-1-carbonyl]-4,4a,5,7,8,8a-hexahydropyrido[4,3-b][1,4]oxazin-3-one ;
la (4aR,8aS)-6-[3-[4-(trifluorométhoxy)phényl]azétidine-1-carbonyl]-4,4a,5,7,8,8a-hexahydropyrido[4,3-b][1,4]oxazin-3-one ;
la (4aR,8aS)-6-(3-(2'-(trifluorométhoxy)-[1,1'-biphényl]-4-yl)azétidine-1-carbonyl)hexahydro-2H-pyrido[4,3-b][1,4]oxazin-3(4H)-one ;
la (4aR,8aS)-6-(3-(4-bromophényl)-3-fluoroazétidine-1-carbonyl)hexahydro-2H-pyrido[4,3-b][1,4]oxazin-3(4H)-one ;
la (4aR,8aS)-6-(3-(4-bromophényl)-3-hydroxyazétidine-1-carbonyl)hexahydro-2H-pyrido[4,3-b][1,4]oxazin-3(4H)-one ;
la (4aR,8aS)-6-(3-(4-bromophényl)-3-méthylazétidine-1-carbonyl)hexahydro-2H-pyrido[4,3-b][1,4]oxazin-3(4H)-one ;
la (4aR,8aS)-6-(3-(2'-(trifluorométhyl)-[1,1'-biphényl]-4-yl)azétidine-1-carbonyl)hexahydro-2H-pyrido[4,3-b][1,4]oxazin-3(4H)-one ;
la (4aR,8aS)-6-(3-(2',4'-difluoro-[1,1'-biphényl]-4-yl)azétidine-1-carbonyl)hexahydro-2H-pyrido[4,3-b][1,4]oxazin-3(4H)-one ;
le 4'-(1-((4aR,8aS)-3-oxooctahydro-2H-pyrido[4,3-b][1,4]oxazine-6-carbonyl)azétidin-3-yl)-[1,1'-biphényl]-2-carbonitrile ;
la (4aR,8aS)-6-(3-(4-(3-méthoxyazétidin-1-yl)phényl)azétidine-1-carbonyl)hexahydro-2H-pyrido[4,3-b][1,4]oxazin-3(4H)-one ;
la (4aR,8aS)-6-(3-(4-(3-(trifluorométhyl)azétidin-1-yl)phényl)azétidine-1-carbonyl)hexahydro-2H-pyrido[4,3-b][1,4]oxazin-3(4H)-one ;
le 2-[4-[1-[(4aR,8aS)-3-oxo-4,4a,5,7,8,8a-hexahydropyrido[4,3-b][1,4]oxazine-6-carbonyl]azétidin-3-yl]phényl]benzamide ;
la (4aR,8aS)-6-(3-(2-chloro-[1,1'-biphényl]-4-yl)azétidine-1-carbonyl)hexahydro-2H-pyrido[4,3-b][1,4]oxazin-3(4H)-one ;
la (4aR,8aS)-6-(3-(4-bromo-3-chlorophényl)azétidine-1-carbonyl)hexahydro-2H-pyrido[4,3-b][1,4]oxazin-3(4H)-one ;
la (4aR,8aS)-6-[3-[4-(4-chloro-2-fluoro-phényl)phényl]azétidine-1-carbonyl]-4,4a,5,7,8,8a-hexahydropyrido[4,3-b][1,4]oxazin-3-one ;
la (4aR,8aS)-6-[3-[4-(2-chloro-4-fluoro-phényl)phényl]azétidine-1-carbonyl]-4,4a,5,7,8,8a-hexahydropyrido[4,3-b][1,4]oxazin-3-one ;
la (4aR,8aS)-6-[4-[(R ou S)-(3,4-diméthoxyphényl)-(3-pyridyl)méthyl]pipéridine-1-carbonyl]-4,4a,5,7,8,8a-hexahydropyrido[4,3-b][1,4]oxazin-3-one ;
la (4aR,8aS)-6-[4-[(S ou R)-(3,4-diméthoxyphényl)-(3-pyridyl)méthyl]pipéridine-1-carbonyl]-4,4a,5,7,8,8a-hexahydropyrido[4,3-b][1,4]oxazin-3-one ;
la (4aR,8aS)-6-(3-(2'-méthyl-[1,1'-biphényl]-4-yl)azétidine-1-carbonyl)hexahydro-2H-pyrido[4,3-b][1,4]oxazin-3(4H)-one ;
la (4aR,8aS)-6-(3-(4-(3-((1,1,1-trifluoropropan-2-yl)oxy)azétidin-1-yl)phényl)azétidine-1-carbonyl)hexahydro-2H-pyrido[4,3-b][1,4]oxazin-3(4H)-one ;
la (4aR,8aS)-6-(3-(3-bromophényl)azétidine-1-carbonyl)hexahydro-2H-pyrido[4,3-b][1,4]oxazin-3(4H)-one ;
la (4aR,8aS)-6-(3-(4-(tert-butyl)phényl)azétidine-1-carbonyl)hexahydro-2H-pyrido[4,3-b][1,4]oxazin-3(4H)-one ;
la (4aR,8aS)-6-[3-(4-phénylphényl)azétidine-1-carbonyl]-4,4a,5,7,8,8a-hexahydropyrido[4,3-b][1,4]oxazin-3-one ;
la (4aR,8aS)-6-[3-[4-[2-(difluorométhyl)phényl]phényl]azétidine-1-carbonyl]-4,4a,5,7,8,8a-hexahydropyrido[4,3-b][1,4]oxazin-3-one ;
la (4aR,8aS)-6-(3-(3'-méthyl-[1,1'-biphényl]-4-yl)azétidine-1-carbonyl)hexahydro-2H-pyrido[4,3-b][1,4]oxazin-3(4H)-one ;
la (4aR,8aS)-6-(3-(4'-méthyl-[1,1'-biphényl]-4-yl)azétidine-1-carbonyl)hexahydro-2H-pyrido[4,3-b][1,4]oxazin-3(4H)-one ;
la (4aR,8aS)-6-(3-(6-chloropyridin-3-yl)azétidine-1-carbonyl)hexahydro-2H-pyrido[4,3-b][1,4]oxazin-3(4H)-one ;
la (4aR,8aS)-6-(3-(4-(trifluorométhyl)phényl)azétidine-1-carbonyl)hexahydro-2H-pyrido[4,3-b][1,4]oxazin-3(4H)-one ;
la (4aR,8aS)-6-(3-(4-(3-((1,1,1-trifluoro-2-méthylpropan-2-yl)oxy)azétidin-1-yl)phényl)azétidine-1-carbonyl)hexahydro-2H-pyrido[4,3-b][1,4]oxazin-3(4H)-one ;
la (4aR,8aS)-6-(3-(4-(1,1-difluoroéthyl)phényl)azétidine-1-carbonyl)hexahydro-2H-pyrido[4,3-b][1,4]oxazin-3(4H)-one ;
la (4aR,8aS)-6-[4-[(R ou S)-(3,4-diméthoxyphényl)-(4-pyridyl)méthyl]pipéridine-1-carbonyl]-4,4a,5,7,8,8a-hexahydropyrido[4,3-b][1,4]oxazin-3-one ;
la (4aR,8aS)-6-[4-[(S ou R)-(3,4-diméthoxyphényl)-(4-pyridyl)méthyl]pipéridine-1-carbonyl]-4,4a,5,7,8,8a-hexahydropyrido[4,3-b][1,4]oxazin-3-one ;
la (4aR,8aS)-6-(3-(4-(3-méthylazétidin-1-yl)phényl)azétidine-1-carbonyl)hexahydro-2H-pyrido[4,3-b][1,4]oxazin-3(4H)-one ;
la (4aR,8aS)-6-(3-(4-(3,3-diméthylpyrrolidin-1-yl)phényl)azétidine-1-carbonyl)hexahydro-2H-pyrido[4,3-b][1,4]oxazin-3(4H)-one ;
la (4aR,8aS)-6-(3-(4-(3-(trifluorométhoxy)azétidin-1-yl)phényl)azétidine-1-carbonyl)hexahydro-2H-pyrido[4,3-b][1,4]oxazin-3(4H)-one ;
la (4aR,8aS)-6-(3-(6-(2,4-dichlorophényl)pyridin-3-yl)azétidine-1-carbonyl)hexahydro-2H-pyrido[4,3-b][1,4]oxazin-3(4H)-one ;
la (4aR,8aS)-6-(3-(2'-chloro-4'-(méthylsulfonyl)-[1,1'-biphényl]-4-yl)azétidine-1-carbonyl)hexahydro-2H-pyrido[4,3-b][1,4]oxazin-3(4H)-one ;
la (4aR,8aS)-6-(3-(4-(3,3-difluoroazétidin-1-yl)phényl)azétidine-1-carbonyl)hexahydro-2H-pyrido[4,3-b][1,4]oxazin-3(4H)-one ;
la (4aR,8aS)-6-(4-(4-bromophényl)pipéridine-1-carbonyl)hexahydro-2H-pyrido[4,3-b][1,4]oxazin-3(4H)-one ;
la (4aR,8aS)-6-[3-[4-(2,2,2-trifluoroéthoxy)phényl]azétidine-1-carbonyl]-4,4a,5,7,8,8a-hexahydropyrido[4,3-b][1,4]oxazin-3-one ;
le 2-[4-[1-[(4aR,8aS)-3-oxo-4,4a,5,7,8,8a-hexahydropyrido[4,3-b][1,4]oxazine-6-carbonyl]azétidin-3-yl]phényl]-5-chloro-benzonitrile ;
la (4aR,8aS)-6-[3-[4-[3-[(1S ou 1R)-2,2,2-trifluoro-1-méthyl-éthoxy]azétidin-1-yl]phényl]azétidine-1-carbonyl]-4,4a,5,7,8,8a-hexahydropyrido[4,3-b][1,4]oxazin-3-one ;
la (4aR,8aS)-6-[3-[4-[3-[(1R ou 1S)-2,2,2-trifluoro-1-méthyl-éthoxy]azétidin-1-yl]phényl]azétidine-1-carbonyl]-4,4a,5,7,8,8a-hexahydropyrido[4,3-b][1,4]oxazin-3-one ;
la (4aR,8aS)-6-(3-(4-(2-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(4-(3-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(3-bromophényl)pyrrolidine-1-carbonyl)hexahydro-2H-pyrido[4,3-b][1,4]oxazin-3(4H)-one ;
la (4aR,8aS)-6-[3-[4-(2-éthylpyrrolidin-1-yl)phényl]azétidine-1-carbonyl]-4,4a,5,7,8,8a-hexahydropyrido[4,3-b][1,4]oxazin-3-one ;
le 2-[4-[1-[(4aR,8aS)-3-oxo-4,4a,5,7,8,8a-hexahydropyrido[4,3-b][1,4]oxazine-6-carbonyl]azétidin-3-yl]phényl]-3-chloro-benzonitrile ;
la (4aR,8aS)-6-(3-(4-(2-cyclopropylpyrrolidin-1-yl)phényl)azétidine-1-carbonyl)hexahydro-2H-pyrido[4,3-b][1,4]oxazin-3(4H)-one ;
la (4aR,8aS)-6-(4-(4-bromophényl)pipérazine-1-carbonyl)hexahydro-2H-pyrido[4,3-b][1,4]oxazin-3(4H)-one ;
la (4aR,8aS)-6-(4-(2',4'-dichloro-[1,1'-biphényl]-4-yl)pipéridine-1-carbonyl)hexahydro-2H-pyrido[4,3-b][1,4]oxazin-3(4H)-one ;
la (4aR,8aS)-6-(3-(4-(3-azabicyclo[3.1.0]hexan-3-yl)phényl)azétidine-1-carbonyl)hexahydro-2H-pyrido[4,3-b][1,4]oxazin-3(4H)-one ;
la (4aR,8aS)-6-(3-(3-(trifluorométhoxy)phényl)azétidine-1-carbonyl)hexahydro-2H-pyrido[4,3-b][1,4]oxazin-3(4H)-one ;
la (4aR,8aS)-6-(3-(4-(3-(2,2,2-trifluoroéthoxy)azétidin-1-yl)phényl)azétidine-1-carbonyl)hexahydro-2H-pyrido[4,3-b][1,4]oxazin-3(4H)-one ;
la (4aR,8aS)-6-(3-(4'-chloro-2'-(méthylsulfonyl)-[1,1'-biphényl]-4-yl)azétidine-1-carbonyl)hexahydro-2H-pyrido[4,3-b][1,4]oxazin-3(4H)-one ;
la (4aR,8aS)-6-(3-(6-(2-(trifluorométhyl)pyrrolidin-1-yl)pyridin-3-yl)azétidine-1-carbonyl)hexahydro-2H-pyrido[4,3-b][1,4]oxazin-3(4H)-one ;
la (4aR,8aS)-6-[4-[(R ou S)-(3-méthylsulfonylphényl)-(3-pyridyl)méthyl]pipéridine-1-carbonyl]-4,4a,5,7,8,8a-hexahydropyrido[4,3-b][1,4]oxazin-3-one ;
la (4aR,8aS)-6-[4-[(S ou R)-(3-méthylsulfonylphényl)-(3-pyridyl)méthyl]pipéridine-1-carbonyl]-4,4a,5,7,8,8a-hexahydropyrido[4,3-b][1,4]oxazin-3-one ;
la (4aR,8aS)-6-[4-[(R ou S)-(3-méthylsulfonylphényl)-(4-pyridyl)méthyl]pipéridine-1-carbonyl]-4,4a,5,7,8,8a-hexahydropyrido[4,3-b][1,4]oxazin-3-one ;
la (4aR,8aS)-6-[4-[(S ou R)-(3-méthylsulfonylphényl)-(4-pyridyl)méthyl]pipéridine-1-carbonyl]-4,4a,5,7,8,8a-hexahydropyrido[4,3-b][1,4]oxazin-3-one ;
la (4aR,8aS)-6-(3-(4-(5-azaspiro[2.4]heptan-5-yl)phényl)azétidine-1-carbonyl)hexahydro-2H-pyrido[4,3-b][1,4]oxazin-3(4H)-one ;
la (4aR,8aS)-6-(3-(4-(pentafluoro-16-sulfaneyl)phényl)azétidine-1-carbonyl)hexahydro-2H-pyrido[4,3-b][1,4]oxazin-3(4H)-one ;
la (4aR,8aS)-6-(3-(5-chloropyridin-2-yl)azétidine-1 -carbonyl)hexahydro-2H-pyrido[4,3-b][1,4]oxazin-3(4H)-one ;
la (4aR,8aS)-6-(3-(2-fluoro-4-(trifluorométhoxy)phényl)azétidine-1-carbonyl)hexahydro-2H-pyrido[4,3-b][1,4]oxazin-3(4H)-one ;
la (4aR,8aS)-6-[3-(6-méthoxypyridin-3-yl)azétidine-1-carbonyl]-4,4a,5,7,8,8a-hexahydropyrido[4,3-b][1,4]oxazin-3-one ;
la (4aR,8aS)-6-(3-(4-bromophényl)pyrrolidine-1-carbonyl)hexahydro-2H-pyrido[4,3-b][1,4]oxazin-3(4H)-one ;
la (4aR,8aS)-6-(3-phénylazétidine-1-carbonyl)hexahydro-2H-pyrido[4,3-b][1,4]oxazin-3(4H)-one ;
la (4aR,8aS)-6-(4-phénylpipéridine-1-carbonyl)hexahydro-2H-pyrido[4,3-b][1,4]oxazin-3(4H)-one ;
la (4aR,8aS)-6-[3-[4-(2,2,2-trifluoroéthyl)phényl]azétidine-1-carbonyl]-4,4a,5,7,8,8a-hexahydropyrido[4,3-b][1,4]oxazin-3-one ;
la (4aR,8aS)-6-[3-[4-[1-(trifluorométhyl)cyclopropyl]phényl]azétidine-1-carbonyl]-4,4a,5,7,8,8a-hexahydropyrido[4,3-b][1,4]oxazin-3-one ;
la (4aR,8aS)-6-[4-[(R ou S)-(3-méthylsulfonylphényl)-(2-pyridyl)méthyl]pipéridine-1-carbonyl]-4,4a,5,7,8,8a-hexahydropyrido[4,3-b][1,4]oxazin-3-one ;
la (4aR,8aS)-6-[4-[(S ou R)-(3-méthylsulfonylphényl)-(2-pyridyl)méthyl]pipéridine-1-carbonyl]-4,4a,5,7,8,8a-hexahydropyrido[4,3-b][1,4]oxazin-3-one ;
la (4aR,8aS)-6-[3-[4-(6,6-difluoro-2-azaspiro[3.3]heptan-2-yl)phényl]azétidine-1-carbonyl]-4,4a,5,7,8,8a-hexahydropyrido[4,3-b][1,4]oxazin-3-one ;
la (4aR,8aS)-6-(3-(4-(8-oxa-3-azabicyclo[3.2.1]octan-3-yl)phényl)azétidine-1-carbonyl)hexahydro-2H-pyrido[4,3-b][1,4]oxazin-3(4H)-one ;
la (4aR,8aS)-6-(3-(5-(2,4-dichlorophényl)pyridin-2-yl)azétidine-1-carbonyl)hexahydro-2H-pyrido[4,3-b][1,4]oxazin-3(4H)-one ;
la (4aR,8aS)-6-(3-(4-((R ou S)-2-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(4-((S ou R)-2-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-[3-[4-(1-pipéridyl)phényl]azétidine-1-carbonyl]-4,4a,5,7,8,8a-hexahydropyrido[4,3-b][1,4]oxazin-3-one ;
la (4aR,8aS)-6-(3-(4-((R ou S)-3-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(4-((S ou R)-3-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-[3-[4-(3-hydroxy-3-méthylazétidin-1-yl)phényl]azétidine-1-carbonyl]-4,4a,5,7,8,8a-hexahydropyrido[4,3-b][1,4]oxazin-3-one ;
la (4aR,8aS)-6-[3-[4-(3-fluoro-3-méthyl-azétidin-1-yl)phényl]azétidine-1-carbonyl]-4,4a,5,7,8,8a-hexahydropyrido[4,3-b][1,4]oxazin-3-one ;
la (4aR,8aS)-6-(3-(4-(3-fluoroazétidin-1-yl)phényl)azétidine-1-carbonyl)hexahydro-2H-pyrido[4,3-b][1,4]oxazin-3(4H)-one ;
la (4aR,8aS)-6-(3-(3-fluoro-4-(trifluorométhoxy)phényl)azétidine-1-carbonyl)hexahydro-2H-pyrido[4,3-b][1,4]oxazin-3(4H)-one ;
la (4aR,8aS)-6-(3-(3-méthyl-4-(trifluorométhoxy)phényl)azétidine-1-carbonyl)hexahydro-2H-pyrido[4,3-b][1,4]oxazin-3(4H)-one ;
la (4aR,8aS)-6-(3-(3,5-difluoro-4-(trifluorométhoxy)phényl)azétidine-1-carbonyl)hexahydro-2H-pyrido[4,3-b][1,4]oxazin-3(4H)-one ;
la (4aR,8aS)-6-(3-(2-chloro-4-(trifluorométhoxy)phényl)azétidine-1-carbonyl)hexahydro-2H-pyrido[4,3-b][1,4]oxazin-3(4H)-one ;
la (4aR,8aS)-6-(3-(4-(bicyclo[1.1.1]pentan-1-yl)phényl)azétidine-1-carbonyl)hexahydro-2H-pyrido[4,3-b][1,4]oxazin-3(4H)-one ;
la (4aR,8aS)-6-(3-(5-(2-(trifluorométhyl)pyrrolidin-1-yl)pyridin-2-yl)azétidine-1-carbonyl)hexahydro-2H-pyrido[4,3-b][1,4]oxazin-3(4H)-one ;
la (4aR,8aS)-6-(3-(5-fluoro-1H-indol-3-yl)pyrrolidine-1-carbonyl)hexahydro-2H-pyrido[4,3-b][1,4]oxazin-3(4H)-one ;
la (4aR,8aS)-6-(3-(1-benzyl-5-chloro-1H-indol-3-yl)pyrrolidine-1-carbonyl)hexahydro-2H-pyrido[4,3-b][1,4]oxazin-3(4H)-one ;
la (4aR,8aS)-6-(3-(2-fluoro-4-(trifluorométhyl)phényl)azétidine-1-carbonyl)hexahydro-2H-pyrido[4,3-b][1,4]oxazin-3(4H)-one ;
la (4aR,8aS)-6-(3-(3-chloro-4-(trifluorométhoxy)phényl)azétidine-1-carbonyl)hexahydro-2H-pyrido[4,3-b][1,4]oxazin-3(4H)-one ;
la (4aR,8aS)-6-[3-[4-[3-(cyclopropylméthoxy)azétidin-1-yl]phényl]azétidine-1-carbonyl]-4,4a,5,7,8,8a-hexahydropyrido[4,3-b][1,4]oxazin-3-one ;
le 5-(1-((4aR,8aS)-3-oxooctahydro-2H-pyrido[4,3-b][1,4]oxazine-6-carbonyl)azétidin-3-yl)-2-(trifluorométhoxy)benzonitrile ;
la (4aR,8aS)-6-(3-(4-(1-(hydroxyméthyl)cyclopropyl)phényl)azétidine-1-carbonyl)hexahydro-2H-pyrido[4,3-b][1,4]oxazin-3(4H)-one ;
la (4aR,8aS)-6-(3-(1-méthyl-1H-indazol-4-yl)azétidine-1-carbonyl)hexahydro-2H-pyrido[4,3-b][1,4]oxazin-3(4H)-one ;
la (4aR,8aS)-6-(3-(4-(3-fluoropyrrolidin-1-yl)phényl)azétidine-1-carbonyl)hexahydro-2H-pyrido[4,3-b][1,4]oxazin-3(4H)-one ;
la (4aR,8aS)-6-(3-(4-(2-méthylazétidin-1-yl)phényl)azétidine-1-carbonyl)hexahydro-2H-pyrido[4,3-b][1,4]oxazin-3(4H)-one ;
la (4aR,8aS)-6-[4-[phényl(pyridazin-3-yl)méthyl]pipéridine-1-carbonyl]-4,4a,5,7,8,8a-hexahydropyrido[4,3-b][1,4]oxazin-3-one ;
la (4aR,8aS)-6-[3-[4-(trifluorométhoxy)phényl]pyrrolidine-1-carbonyl]-4,4a,5,7,8,8a-hexahydropyrido[4,3-b][1,4]oxazin-3-one ;
la (4aR,8aS)-6-(3-(1-méthyl-1H-indazol-6-yl)azétidine-1-carbonyl)hexahydro-2H-pyrido[4,3-b][1,4]oxazin-3(4H)-one ;
la (4aR,8aS)-6-[3-(S ou R)-[3-(trifluorométhoxy)phényl]pyrrolidine-1-carbonyl]-4,4a,5,7,8,8a-hexahydropyrido[4,3-b][1,4]oxazin-3-one ;
la (4aR,8aS)-6-[3-(R ou S)-[3-(trifluorométhoxy)phényl]pyrrolidine-1-carbonyl]-4,4a,5,7,8,8a-hexahydropyrido[4,3-b][1,4]oxazin-3-one ;
la (4aR,8aS)-6-(3-(3-chloro-4-hydroxyphényl)azétidine-1-carbonyl)hexahydro-2H-pyrido[4,3-b][1,4]oxazin-3(4H)-one ;
la (4aR,8aS)-6-(3-(3-chloro-4-(3-méthylazétidin-1-yl)phényl)azétidine-1-carbonyl)hexahydro-2H-pyrido[4,3-b][1,4]oxazin-3(4H)-one ;
la (4aR,8aS)-6-[3-[4-(oxétan-3-yl)phényl]azétidine-1-carbonyl]-4,4a,5,7,8,8a-hexahydropyrido[4,3-b][1,4]oxazin-3-one ;
la (4aR,8aS)-6-(3-(3-chloro-4-(3,3-difluoroazétidin-1-yl)phényl)azétidine-1-carbonyl)hexahydro-2H-pyrido[4,3-b][1,4]oxazin-3(4H)-one ;
la (4aR,8aS)-6-(3-(3-(3-méthylazétidin-1-yl)-4-(trifluorométhoxy)phényl)azétidine-1-carbonyl)hexahydro-2H-pyrido[4,3-b][1,4]oxazin-3(4H)-one ;
la (4aR,8aS)-6-[(3S ou R)-3-(3-bromophényl)pyrrolidine-1-carbonyl]-4,4a,5,7,8,8a-hexahydropyrido[4,3-b][1,4]oxazin-3-one ;
la (4aR,8aS)-6-[(3R ou S)-3-(3-bromophényl)pyrrolidine-1-carbonyl]-4,4a,5,7,8,8a-hexahydropyrido[4,3-b][1,4]oxazin-3-one ;
la (4aR,8aS)-6-[3-[4-(3-azabicyclo[3.1.1]heptan-3-yl)phényl]azétidine-1-carbonyl]-4,4a,5,7,8,8a-hexahydropyrido[4,3-b][1,4]oxazin-3-one ;
la (4aR,8aS)-6-(2-méthyl-3-(4-(trifluorométhoxy)phényl)azétidine-1-carbonyl)hexahydro-2H-pyrido[4,3-b][1,4]oxazin-3(4H)-one ;
la (4aR,8aS)-6-(3-(3,3-diméthyl-2,3-dihydrobenzofuran-6-yl)azétidine-1-carbonyl)hexahydro-2H-pyrido[4,3-b][1,4]oxazin-3(4H)-one ;
la (4aR,8aS)-6-(3-(3-chloro-5-(2,2,2-trifluoroéthoxy)phényl)pyrrolidine-1-carbonyl)hexahydro-2H-pyrido[4,3-b][1,4]oxazin-3(4H)-one ;
le 2-méthyl-2-(4-(1-((4aR,8aS)-3-oxooctahydro-2H-pyrido[4,3-b][1,4]oxazine-6-carbonyl)azétidin-3-yl)phényl)propanoate de méthyle ;
la (4aR,8aS)-6-(3-(3,5-dichlorophényl)pyrrolidine-1-carbonyl)hexahydro-2H-pyrido[4,3-b][1,4]oxazin-3(4H)-one ;
la (4aR,8aS)-6-(3-(4-(3-méthoxy-3-méthylazétidin-1-yl)phényl)azétidine-1-carbonyl)hexahydro-2H-pyrido[4,3-b][1,4]oxazin-3(4H)-one ;
le (2-(3-((1-((4aR,8aS)-3-oxooctahydro-2H-pyrido[4,3-b][1,4]oxazine-6-carbonyl)pipéridin-4-yl)(phényl)méthyl)phénoxy)éthyl)carbamate de tert-butyle ;
la (4aR,8aS)-6-((R ou S)-3-(3-chloro-5-(2,2,2-trifluoroéthoxy)phényl)pyrrolidine-1-carbonyl)hexahydro-2H-pyrido[4,3-b][1,4]oxazin-3(4H)-one ;
la (4aR,8aS)-6-((S ou R)-3-(3-chloro-5-(2,2,2-trifluoroéthoxy)phényl)pyrrolidine-1-carbonyl)hexahydro-2H-pyrido[4,3-b][1,4]oxazin-3(4H)-one ;
la (4aR,8aS)-6-(3-(4-(tert-butyl)-3-méthoxyphényl)azétidine-1-carbonyl)hexahydro-2H-pyrido[4,3-b][1,4]oxazin-3(4H)-one ;
la (4aR,8aS)-6-(3-(1-méthyl-1H-indazol-5-yl)azétidine-1-carbonyl)hexahydro-2H-pyrido[4,3-b][1,4]oxazin-3(4H)-one ;
la (4aR,8aS)-6-(3-(4-propylphényl)azétidine-1-carbonyl)hexahydro-2H-pyrido[4,3-b][1,4]oxazin-3(4H)-one ;
la (4aR,8aS)-6-(3-(4-(trifluorométhoxy)-3-(trifluorométhyl)phényl)azétidine-1-carbonyl)hexahydro-2H-pyrido[4,3-b][1,4]oxazin-3(4H)-one ;
la (4aR, 8aS)-6- [3 - [4- [[1-(trifluorométhyl)cyclopropyl]méthoxy]phényl]azétidine-1-carbonyl]-4,4a,5,7,8,8a-hexahydropyrido[4,3-b][1,4]oxazin-3-one ;
la (4aR,8aS)-6-[3-[4-(2,2,2-trifluoro-1,1-diméthyl-éthyl)phényl]azétidine-1-carbonyl]-4,4a,5,7,8,8a-hexahydropyrido[4,3-b][1,4]oxazin-3-one ;
la (4aR,8aS)-6-[3-(R ou S)-[4-(2,2,2-trifluoro-1-méthyl-éthoxy)phényl]azétidine-1-carbonyl]-4,4a,5,7,8,8a-hexahydropyrido[4,3-b][1,4]oxazin-3-one ;
la (4aR,8aS)-6-[3-[4-(3-fluoropropyl)phényl]azétidine-1-carbonyl]-4,4a,5,7,8,8a-hexahydropyrido[4,3-b][1,4]oxazin-3-one ;
la (4aR,8aS)-6-[3-(S ou R)-[4-(2,2,2-trifluoro-1-méthyl-éthoxy)phényl]azétidine-1-carbonyl]-4,4a,5,7,8,8a-hexahydropyrido[4,3-b][1,4]oxazin-3-one ;
la (4aR,8aS)-6-[3-(4-cyclobutylphényl)azétidine-1-carbonyl]-4,4a,5,7,8,8a-hexahydropyrido[4,3-b][1,4]oxazin-3-one ;
la (4aR,8aS)-6-[3-(3-méthoxy-4-méthyl-phényl)azétidine-1-carbonyl]-4,4a,5,7,8,8a-hexahydropyrido[4,3-b][1,4]oxazin-3-one ;
la (4aR,8aS)-6-(4-((4-fluorophényl)((1-méthyl-5-(trifluorométhyl)-1H-pyrazol-3-yl)oxy)méthyl)pipéridine-1-carbonyl)hexahydro-2H-pyrido[4,3-b][1,4]oxazin-3(4H)-one ;
la (4aR,8aS)-6-[4-[[3-(2-aminoéthoxy)phényl]-phényl-méthyl]pipéridine-1-carbonyl]-4,4a,5,7,8,8a-hexahydropyrido[4,3-b][1,4]oxazin-3-one ;
la (4aR,8aS)-6-[3-[5-(2,4-dichlorophényl)-1,3,4-oxadiazol-2-yl]azétidine-1-carbonyl]-4,4a,5,7,8,8a-hexahydropyrido[4,3-b][1,4]oxazin-3-one ;
la (4aR,8aS)-6-[3-[3-fluoro-4-(trifluorométhyl)phényl]azétidine-1-carbonyl]-4,4a,5,7,8,8a-hexahydropyrido[4,3-b][1,4]oxazin-3-one ;
le N-[2-[3-[2-[3-[[1-[(4aR,8aS)-3-oxo-4,4a,5,7,8,8a-hexahydropyrido[4,3-b][1,4]oxazine-6-carbonyl]-4-pipéridyl]-phényl-méthyl]phénoxy]éthylamino]-3-oxo-propoxy]éthyl]carbamate de tert-butyle ;
le N-[2-[2-[3-[2-[3-[[1-[(4aR,8aS)-3-oxo-4,4a,5,7,8,8a-hexahydropyrido[4,3-b][1,4]oxazine-6-carbonyl]-4-pipéridyl]-phényl-méthyl]phénoxy]éthylamino]-3-oxo-propoxy]éthoxy]éthyl]carbamate de tert-butyle ;
la (4aR,8aS)-6-[3-[1-(2,4-dichlorophényl)pyrazol-3-yl]azétidine-1-carbonyl]-4,4a,5,7,8,8a-hexahydropyrido[4,3-b][1,4]oxazin-3-one ;
la (4aR,8aS)-6-[3-(4-propoxyphényl)azétidine-1-carbonyl]-4,4a,5,7,8,8a-hexahydropyrido[4,3-b][1,4]oxazin-3-one ;
la (4aR,8aS)-6-[3-(3,4-piméthylphényl)azétidine-1-carbonyl]-4,4a,5,7,8,8a-hexahydropyrido[4,3-b][1,4]oxazin-3-one ;
le 2-[4-[1-[(4aR,8aS)-3-oxo-4,4a,5,7,8,8a-hexahydropyrido[4,3-b][1,4]oxazine-6-carbonyl]azétidin-3-yl]phényl]-N-éthyl-5-méthyl-benzamide ;
la (4aR,8aS)-6-[3-[4-(2,2-diméthylpropyl)phényl]azétidine-1-carbonyl]-4,4a,5,7,8,8a-hexahydropyrido[4,3-b][1,4]oxazin-3-one ;
la (4aR,8aS)-6-[3-(4-tert-butoxyphényl)azétidine-1-carbonyl]-4,4a,5,7,8,8a-hexahydropyrido[4,3-b][1,4]oxazin-3-one ;
la (4aR,8aS)-6-[4-(5-chloroindolin-1-yl)pipéridine-1-carbonyl]-4,4a,5,7,8,8a-hexahydropyrido[4,3-b][1,4]oxazin-3-one ;
la (4aR,8aS)-6-[4-(4-chloroisoindolin-2-yl)pipéridine-1-carbonyl]-4,4a,5,7,8,8a-hexahydropyrido[4,3-b][1,4]oxazin-3-one ;
la (4aR,8aS)-6-[4-(5'-chlorospiro[cyclopropane-1,3'-indoline]-1'-yl)pipéridine-1-carbonyl]-4,4a,5,7,8,8a-hexahydropyrido[4,3-b][1,4]oxazin-3-one ;
la (4aR,8aS)-6-[3-(4-chloroisoindolin-2-yl)azétidine-1-carbonyl]-4,4a,5,7,8,8a-hexahydropyrido[4,3-b][1,4]oxazin-3-one ;
la (4aR,8aS)-6-[4-(5-chloroisoindolin-2-yl)pipéridine-1-carbonyl]-4,4a,5,7,8,8a-hexahydropyrido[4,3-b][1,4]oxazin-3-one ;
la (4aS,8aS)-6-[4-[1-[2-[tert-butyl(diméthyl)silyl]oxyéthyl]-5-chloro-indol-3-yl]pipéridine-1-carbonyl]-4,4a,5,7,8,8a-hexahydropyrido[4,3-b][1,4]oxazin-3-one ;
la (4aS,8aS)-6-[4-[5-chloro-1-(2-hydroxyéthyl)indol-3-yl]pipéridine-1-carbonyl]-4,4a,5,7,8,8a-hexahydropyrido[4,3-b][1,4]oxazin-3-one ;
la (4aR,8aS)-6-(3-(5-(3-(trifluorométhyl)pyrrolidin-1-yl)pyridin-2-yl)azétidine-1-carbonyl)hexahydro-2H-pyrido[4,3-b][1,4]oxazin-3(4H)-one ;
la (4aR,8aS)-6-(3-(5-((R ou S)-3-(trifluorométhyl)pyrrolidin-1-yl)pyridin-2-yl)azétidine-1-carbonyl)hexahydro-2H-pyrido[4,3-b][1,4]oxazin-3(4H)-one ;
la (4aR,8aS)-6-(3-(5-((S ou R)-3-(trifluorométhyl)pyrrolidin-1-yl)pyridin-2-yl)azétidine-1-carbonyl)hexahydro-2H-pyrido[4,3-b][1,4]oxazin-3(4H)-one ;
la (4aR,8aS)-6-[3-[6-[3-(trifluorométhyl)pyrrolidin-1-yl]-3-pyridyl]azétidine-1-carbonyl]-4,4a,5,7,8,8a-hexahydropyrido[4,3-b][1,4]oxazin-3-one ;
la (4aR,8aS)-6-[3-[6-[3-(R ou S)-(trifluorométhyl)pyrrolidin-1-yl]-3-pyridyl]azétidine-1-carbonyl]-4,4a,5,7,8,8a-hexahydropyrido[4,3-b][1,4]oxazin-3-one ;
la (4aR,8aS)-6-[3-[6-[3S- ou 3R-(trifluorométhyl)pyrrolidin-1-yl]-3-pyridyl]azétidine-1-carbonyl]-4,4a,5,7,8,8a-hexahydropyrido[4,3-b][1,4]oxazin-3-one ;
la (4aR,8aS)-6-[3-(4-tétrahydropyran-3-ylphényl)azétidine-1-carbonyl]-4,4a,5,7,8,8a-hexahydropyrido[4,3-b][1,4]oxazin-3-one ;
la (4aR,8aS)-6-[3-[2-méthoxy-4-(2,2,2-trifluoroéthyl)phényl]azétidine-1-carbonyl]-4,4a,5,7,8,8a-hexahydropyrido[4,3-b][1,4]oxazin-3-one ;
la (4aR,8aS)-6-(3-(4-(néopentyloxy)phényl)azétidine-1-carbonyl)hexahydro-2H-pyrido[4,3-b][1,4]oxazin-3(4H)-one ;
la rac-(4aR,8aS)-6-[4-(3-méthoxy-1-phényl-propyl)pipéridine-1-carbonyl]-4,4a,5,7,8,8a-hexahydropyrido[4,3-b][1,4]oxazin-3-one ;
la rac-(4aR,8aS)-6-[4-[phényl(3-pyridyl)méthyl]pipéridine-1-carbonyl]-4,4a,5,7,8,8a-hexahydropyrido[4,3-b][1,4]oxazin-3-one ;
la rac-(4aR,8aS)-6-[4-[(5-méthyl-1,2,4-oxadiazol-3-yl)-phényl-méthyl]pipéridine-1-carbonyl]-4,4a,5,7,8,8a-hexahydropyrido[4,3-b][1,4]oxazin-3-one ;
la rac-(4aR,8aS)-6-[4-(5-fluoro-1-méthyl-indol-3-yl)pipéridine-1-carbonyl]-4,4a,5,7,8,8a-hexahydropyrido[4,3-b][1,4]oxazin-3-one ;
le 3-[5-chloro-3-[1-[rac-(4aR,8aS)-3-oxo-4,4a,5,7,8,8a-hexahydropyrido[4,3-b][1,4]oxazine-6-carbonyl]-4-pipéridyl]indol-1-yl]azétidine-1-carboxylate de tert-butyle ;
le 3-[5-chloro-3-[1-[rac-(4aR,8aS)-3-oxo-4,4a,5,7,8,8a-hexahydropyrido[4,3-b][1,4]oxazine-6-carbonyl]-4-pipéridyl]indol-1-yl]azétidine-1-carboxylate de tert-butyle ;
la rac-(4aR,8aS)-6-[4-[1-(2-chloro-4-fluoro-phénoxy)propyl]pipéridine-1-carbonyl]-4,4a,5,7,8,8a-hexahydropyrido[4,3-b][1,4]oxazin-3-one ;
la rac-(4aR,8aS)-6-[4-[(2-chloro-4-fluoro-phénoxy)-cyclopropyl-méthyl]pipéridine-1-carbonyl]-4,4a,5,7,8,8a-hexahydropyrido[4,3-b][1,4]oxazin-3-one ;
la rac-(4aR,8aS)-6-[4-[(4-fluorophénoxy)-phényl-méthyl]pipéridine-1-carbonyl]-4,4a,5,7,8,8a-hexahydropyrido[4,3-b][1,4]oxazin-3-one ;
la rac-(4aR,8aS)-6-[4-[5-(trifluorométhyl)-2-pyridyl]pipéridine-1-carbonyl]-4,4a,5,7,8,8a-hexahydropyrido[4,3-b][1,4]oxazin-3-one ;
la rac-(4aR,8aS)-6-[4-[cyclopropylméthoxy-[4-(trifluorométhyl)phényl]méthyl]pipéridine-1-carbonyl]-4,4a,5,7,8,8a-hexahydropyrido[4,3-b][1,4]oxazin-3-one ;
la rac-(4aR,8aS)-6-[4-[1-(4-fluorophényl)-2-hydroxy-éthyl]pipérazine-1-carbonyl]-4,4a,5,7,8,8a-hexahydropyrido[4,3-b][1,4]oxazin-3-one ;
la rac-(4aR,8aS)-6-[4-[1-(4-fluorophényl)-2-méthoxy-éthyl]pipérazine-1-carbonyl]-4,4a,5,7,8,8a-hexahydropyrido[4,3-b][1,4]oxazin-3-one ;
la rac-(4aR,8aS)-6-[4-[1-(3,4-difluorophényl)-2-hydroxy-éthyl]pipérazine-1-carbonyl]-4,4a,5,7,8,8a-hexahydropyrido[4,3-b][1,4]oxazin-3-one ;
la rac-(4aR,8aS)-6-[4-[1-(3,4-difluorophényl)-2-méthoxy-éthyl]pipérazine-1-carbonyl]-4,4a,5,7,8,8a-hexahydropyrido[4,3-b][1,4]oxazin-3-one ;
la rac-(4aR,8aS)-6-[4-[1-(4,4-difluoro-1-pipéridyl)cyclopropyl]pipéridine-1-carbonyl]-4,4a,5,7,8,8a-hexahydropyrido[4,3-b][1,4]oxazin-3-one ;
la rac-(4aR,8aS)-6-[4-[1-[4-(trifluorométhyl)imidazol-1-yl]éthyl]pipéridine-1-carbonyl]-4,4a,5,7,8,8a-hexahydropyrido[4,3-b][1,4]oxazin-3-one ;
la rac-(4aR,8aS)-6-[4-[1-[4-(trifluorométhyl)pyrazol-1-yl]cyclopropyl]pipéridine-1-carbonyl]-4,4a,5,7,8,8a-hexahydropyrido[4,3-b][1,4]oxazin-3-one ;
la rac-(4aR,8aS)-6-[4-[(4-tert-butylpyrazol-1-yl)-(4-fluorophényl)méthyl]pipéridine-1-carbonyl]-4,4a,5,7,8,8a-hexahydropyrido[4,3-b][1,4]oxazin-3-one ;
la rac-(4aR,8aS)-6-[4-[3-[4-(trifluorométhyl)phényl]oxétan-3-yl]pipéridine-1-carbonyl]-4,4a,5,7,8,8a-hexahydropyrido[4,3-b][1,4]oxazin-3-one ;
la rac-(4aR,8aS)-6-[4-[4-(trifluorométhyl)benzoyl]pipéridine-1-carbonyl]-4,4a,5,7,8,8a-hexahydropyrido[4,3-b][1,4]oxazin-3-one ;
la rac-(4aR,8aS)-6-[4-[1-[4-(trifluorométhyl)pyrazol-1-yl]éthyl]pipéridine-1-carbonyl]-4,4a,5,7,8,8a-hexahydropyrido[4,3-b][1,4]oxazin-3-one ;
la rac-(4aR,8aS)-6-[4-[(4-fluorophényl)-morpholino-méthyl]pipéridine-1-carbonyl]-4,4a,5,7,8,8a-hexahydropyrido[4,3-b][1,4]oxazin-3-one ;
la rac-(4aR,8aS)-6-[4-[(4-tert-butyloxazol-2-yl)-difluoro-méthyl]pipéridine-1-carbonyl]-4,4a,5,7,8,8a-hexahydropyrido[4,3-b][1,4]oxazin-3-one ;
la rac-(4aR,8aS)-6-[4-[1-(4-chlorophényl)-2-hydroxy-éthyl]pipérazine-1-carbonyl]-4,4a,5,7,8,8a-hexahydropyrido[4,3-b][1,4]oxazin-3-one ;
la rac-(4aR,8aS)-6-[4-[1-(4-chlorophényl)-2-méthoxy-éthyl]pipérazine-1-carbonyl]-4,4a,5,7,8,8a-hexahydropyrido[4,3-b][1,4]oxazin-3-one ;
la rac-(4aR,8aS)-6-[4-[2-hydroxy-1-[4-(trifluorométhyl)phényl]éthyl]pipérazine-1-carbonyl]-4,4a,5,7,8,8a-hexahydropyrido[4,3-b][1,4]oxazin-3-one ;
la rac-(4aR,8aS)-6-[4-[2-méthoxy-1-[4-(trifluorométhyl)phényl]éthyl]pipérazine-1-carbonyl]-4,4a,5,7,8,8a-hexahydropyrido[4,3-b][1,4]oxazin-3-one ;
la rac-(4aR,8aS)-6-[4-(1-cyclohexyl-2-méthoxy-éthyl)pipérazine-1-carbonyl]-4,4a,5,7,8,8a-hexahydropyrido[4,3-b][1,4]oxazin-3-one ;
la rac-(4aR,8aS)-6-[4-(1-cyclohexyl-2-hydroxy-éthyl)pipérazine-1-carbonyl]-4,4a,5,7,8,8a-hexahydropyrido[4,3-b][1,4]oxazin-3-one ;
la rac-(4aR,8aS)-6-[4-[1-(4-fluorophényl)cyclopropyl]pipérazine-1-carbonyl]-4,4a,5,7,8,8a-hexahydropyrido[4,3-b][1,4]oxazin-3-one ;
la rac-(4aR,8aS)-6-[4-[2-(4-fluorophényl)cyclopropyl]pipérazine-1-carbonyl]-4,4a,5,7,8,8a-hexahydropyrido[4,3-b][1,4]oxazin-3-one ;
la rac-(4aR,8aS)-6-[4-[1-(4-fluorophényl)cyclobutyl]pipérazine-1-carbonyl]-4,4a,5,7,8,8a-hexahydropyrido[4,3-b][1,4]oxazin-3-one ;
la rac-(4aR,8aS)-6-[4-[cyclopropyl-(4-fluorophényl)méthyl]pipérazine-1-carbonyl]-4,4a,5,7,8,8a-hexahydropyrido[4,3-b][1,4]oxazin-3-one ;
la rac-(4aR,8aS)-6-[4-[cyclohexyl-(4-fluorophényl)méthyl]pipérazine-1-carbonyl]-4,4a,5,7,8,8a-hexahydropyrido[4,3-b][1,4]oxazin-3-one ;
la rac-(4aR,8aS)-6-[4-[(4-fluorophényl)-(4-pyridyl)méthyl]pipérazine-1-carbonyl]-4,4a,5,7,8,8a-hexahydropyrido[4,3-b][1,4]oxazin-3-one ;
la rac-(4aR,8aS)-6-[4-[2,2,2-trifluoro-1-(4-fluorophényl)éthyl]pipérazine-1-carbonyl]-4,4a,5,7,8,8a-hexahydropyrido[4,3-b][1,4]oxazin-3-one ;
la rac-(4aR,8aS)-6-[4-[(4-fluorophényl)-oxazol-5-yl-méthyl]pipérazine-1-carbonyl]-4,4a,5,7,8,8a-hexahydropyrido[4,3-b][1,4]oxazin-3-one ;
la rac-(4aR,8aS)-6-[4-[bis(4-chlorophényl)méthyl]pipérazine-1-carbonyl]-4,4a,5,7,8,8a-hexahydropyrido[4,3-b][1,4]oxazin-3-one ;
la rac-(4aS,8aS)-6-[4-[5-chloro-1-(cyclopropylméthyl)indol-3-yl]pipéridine-1-carbonyl]-4,4a,5,7,8,8a-hexahydropyrido[4,3-b][1,4]oxazin-3-one ;
la rac-(4aS,8aS)-6-[4-[5-chloro-1-(cyclopropylméthyl)indol-3-yl]pipéridine-1-carbonyl]-4,4a,5,7,8,8a-hexahydropyrido[4,3-b][1,4]oxazin-3-one ;
la rac-(4aR,8aR)-6-[4-[5-chloro-1-(cyclopropylméthyl)indol-3-yl]pipéridine-1-carbonyl]-4,4a,5,7,8,8a-hexahydropyrido[4,3-b][1,4]oxazin-3-one ;
la rac-(4aR,8aS)-6-[4-[(4-chlorophényl)-(4-fluorophényl)méthyl]pipérazine-1-carbonyl]-4,4a,5,7,8,8a-hexahydropyrido[4,3-b][1,4]oxazin-3-one ;
la rac-(4aR,8aS)-6-(4-benzhydrylpipérazine-1-carbonyl)-4,4a,5,7,8,8a-hexahydropyrido[4,3-b][1,4]oxazin-3-one ;
la rac-(4aR,8aS)-6-[4-[4-(2,2,2-trifluoroéthyl)phényl]pipérazine-1-carbonyl]-4,4a,5,7,8,8a-hexahydropyrido[4,3-b][1,4]oxazin-3-one ;
la rac-(4aR,8aS)-6-[4-[3-(2,2,2-trifluoroéthyl)phényl]pipérazine-1-carbonyl]-4,4a,5,7,8,8a-hexahydropyrido[4,3-b][1,4]oxazin-3-one ;
la rac-(4aR,8aS)-6-[4-[4-chloro-3-(trifluorométhyl)phényl]pipérazine-1-carbonyl]-4,4a,5,7,8,8a-hexahydropyrido[4,3-b][1,4]oxazin-3-one ;
la rac-(4aR,8aS)-6-[4-[1-[4-(trifluorométhyl)phényl]éthyl]pipérazine-1-carbonyl]-4,4a,5,7,8,8a-hexahydropyrido[4,3-b][1,4]oxazin-3-one ;
la rac-(4aR,8aS)-6-[3-[1-(2-chloro-4-fluoro-phénoxy)éthyl]azétidine-1-carbonyl]-4,4a,5,7,8,8a-hexahydropyrido[4,3-b][1,4]oxazin-3-one ; et
la rac-(4aR,8aS)-6-[3-[5-chloro-1-(cyclopropylméthyl)indol-3-yl]azétidine-1-carbonyl]-4,4a,5,7,8,8a-hexahydropyrido[4,3-b][1,4]oxazin-3-one.

10. Composé de formule (I) selon l'une quelconque des revendications 1 à 8, ou un sel pharmaceutiquement acceptable de celui-ci, choisi parmi :
la rac-cis-6-(4-(5-chloro-1-(cyclopropylméthyl)-1H-indol-3-yl)pipéridine-1-carbonyl)hexahydro-2H-pyrido[4,3-b][1,4]oxazin-3(4H)-one ;
la rac-cis-6-(4-(5-chloro-1-méthyl-1H-indol-3-yl)pipéridine-1-carbonyl)hexahydro-2H-pyrido[4,3-b][1,4]oxazin-3(4H)-one ;
la rac-cis-6-(4-(1-(4-fluorophényl)-3-méthoxypropyl)pipéridine-1 -carbonyl)hexahydro-2H-pyrido[4,3-b][1,4]oxazin-3(4H)-one ;
la rac-cis-6-(4-((4-chlorophényl)(phényl)méthyl)pipérazine-1-carbonyl)hexahydro-2H-pyrido[4,3-b][1,4]oxazin-3(4H)-one ;
la (+)-cis-6-(4-(bis(4-fluorophényl)méthyl)pipérazine-1-carbonyl)hexahydro-2H-pyrido[4,3-b][1,4]oxazin-3(4H)-one ;
la (+)- ou (-)-cis-6-(4-(5-chloro-1-cyclopropyl-1H-indol-3-yl)pipéridine-1-carbonyl)hexahydro-2H-pyrido[4,3-b][1,4]oxazin-3(4H)-one ;
la (+)- ou (-)-cis-6-(4-(5-chloro-1-(oxétan-3-yl)-1H-indol-3-yl)pipéridine-1-carbonyl)hexahydro-2H-pyrido[4,3-b][1,4]oxazin-3(4H)-one ;
la rac-cis-6-(4-(1-(4-fluorophényl)-3-hydroxypropyl)pipéridine-1-carbonyl)hexahydro-2H-pyrido[4,3-b][1,4]oxazin-3(4H)-one ;
la (4aR,8aS)-6-(4-(difluoro(4-(trifluorométhyl)phényl)méthyl)pipéridine-1-carbonyl)hexahydro-2H-pyrido[4,3-b][1,4]oxazin-3(4H)-one ;
la (+) ou (-)-cis-6-(4-((R ou S)-1-(2-chloro-4-fluorophénoxy)éthyl)pipéridine-1-carbonyl)hexahydro-2H-pyrido[4,3-b][1,4]oxazin-3(4H)-one ;
la (+) ou (-)-cis-6-(4-((4-chlorophényl)difluorométhyl)pipéridine-1-carbonyl)hexahydro-2H-pyrido[4,3-b][1,4]oxazin-3(4H)-one ;
la (+) ou (-)-cis-6-(4-((2-chloro-4-fluorophényl)difluorométhyl)pipéridine-1-carbonyl)hexahydro-2H-pyrido[4,3-b][1,4]oxazin-3(4H)-one ;
la (4aR,8aS)-6-(4-((R ou S)-1-(4-(trifluorométhyl)phényl)éthyl)pipéridine-1-carbonyl)hexahydro-2H-pyrido[4,3-b][1,4]oxazin-3(4H)-one ;
la (4aR,8aS)-6-(4-((S ou R)-(4-fluorophényl)(2,2,2-trifluoroéthoxy)méthyl)pipéridine-1-carbonyl)hexahydro-2H-pyrido[4,3-b][1,4]oxazin-3(4H)-one ;
la (4aR,8aS)-6-[4-[(S ou R)-(4-fluorophényl)-(3-méthoxyphényl)méthyl]pipéridine-1-carbonyl]-4,4a,5,7,8,8a-hexahydropyrido[4,3-b][1,4]oxazin-3-one ;
la (4aR,8aS)-6-(4-((R ou S)-(4-fluorophényl)(3-méthoxyphényl)méthyl)pipéridine-1-carbonyl)hexahydro-2H-pyrido[4,3-b][1,4]oxazin-3(4H)-one ;
la (4aR,8aS)-6-(4-((S ou R)-(3-(2-fluoroéthoxy)phényl)(phényl)méthyl)pipéridine-1-carbonyl)hexahydro-2H-pyrido[4,3-b][1,4]oxazin-3(4H)-one ;
la (4aR,8aS)-6-(4-((3-cyclopropyl-1,2,4-oxadiazol-5-yl)(4-fluorophényl)méthyl)pipéridine-1-carbonyl)hexahydro-2H-pyrido[4,3-b][1,4]oxazin-3(4H)-one ;
la (4aR,8aS)-6-(4-((S ou R)-(4-fluorophényl)(4-méthoxyphényl)méthyl)pipéridine-1-carbonyl)hexahydro-2H-pyrido[4,3-b][1,4]oxazin-3(4H)-one ;
la (4aR,8aS)-6-(4-((S ou R)-(3,4-diméthoxyphényl)(4-fluorophényl)méthyl)pipéridine-1-carbonyl)hexahydro-2H-pyrido[4,3-b][1,4]oxazin-3(4H)-one ;
la (4aR,8aS)-6-(4-((R ou S)-(4-fluorophényl)(4-méthoxyphényl)méthyl)pipéridine-1-carbonyl)hexahydro-2H-pyrido[4,3-b][1,4]oxazin-3(4H)-one ;
la (4aR,8aS)-6-(4-((S ou R)-(4-(2-fluoroéthoxy)phényl)(4-fluorophényl)méthyl)pipéridine-1-carbonyl)hexahydro-2H-pyrido[4,3-b][1,4]oxazin-3(4H)-one ;
la (4aR,8aS)-6-[3-[4-(trifluorométhoxy)phényl]azétidine-1-carbonyl]-4,4a,5,7,8,8a-hexahydropyrido[4,3-b][1,4]oxazin-3-one ;
la (4aR,8aS)-6-(3-(2'-trifluorométhoxy)-[1,1'-biphényl]-4-yl)azétidine-1-carbonyl)hexahydro-2H-pyrido[4,3-b][1,4]oxazin-3(4H)-one ;
la (4aR,8aS)-6-(3-(2'-(trifluorométhyl)-[1,1'-biphényl]-4-yl)azétidine-1-carbonyl)hexahydro-2H-pyrido[4,3-b][1,4]oxazin-3(4H)-one ;
la (4aR,8aS)-6-(3-(2',4'-difluoro-[1,1'-biphényl]-4-yl)azétidine-1-carbonyl)hexahydro-2H-pyrido[4,3-b][1,4]oxazin-3(4H)-one ;
la (4aR,8aS)-6-(3-(4-(3-(trifluorométhyl)azétidin-1-yl)phényl)azétidine-1-carbonyl)hexahydro-2H-pyrido[4,3-b][1,4]oxazin-3(4H)-one ;
la (4aR,8aS)-6-(3-(4-bromo-3-chlorophényl)azétidine-1-carbonyl)hexahydro-2H-pyrido[4,3-b][1,4]oxazin-3(4H)-one ;
la (4aR,8aS)-6-[3-[4-(4-chloro-2-fluoro-phényl)phényl]azétidine-1-carbonyl]-4,4a,5,7,8,8a-hexahydropyrido[4,3-b][1,4]oxazin-3-one ;
la (4aR,8aS)-6-[3-[4-(2-chloro-4-fluoro-phényl)phényl]azétidine-1-carbonyl]-4,4a,5,7,8,8a-hexahydropyrido[4,3-b][1,4]oxazin-3-one ;
la (4aR,8aS)-6-(3-(4-(3-((1,1,1-trifluoropropan-2-yl)oxy)azétidin-1-yl)phényl)azétidine-1-carbonyl)hexahydro-2H-pyrido[4,3-b][1,4]oxazin-3(4H)-one ;
la (4aR,8aS)-6-(3-(4-(tert-butyl)phényl)azétidine-1-carbonyl)hexahydro-2H-pyrido[4,3-b][1,4]oxazin-3(4H)-one ;
la (4aR,8aS)-6-(3-(4-(trifluorométhyl)phényl)azétidine-1-carbonyl)hexahydro-2H-pyrido[4,3-b][1,4]oxazin-3(4H)-one ;
la (4aR,8aS)-6-(3-(4-(3-((1,1,1-trifluoro-2-méthylpropan-2-yl)oxy)azétidin-1-yl)phényl)azétidine-1-carbonyl)hexahydro-2H-pyrido[4,3-b][1,4]oxazin-3(4H)-one ;
la (4aR,8aS)-6-(3-(4-(1,1-difluoroéthyl)phényl)azétidine-1-carbonyl)hexahydro-2H-pyrido[4,3-b][1,4]oxazin-3(4H)-one ;
la (4aR,8aS)-6-(3-(4-(3 ,3-diméthylpyrrolidin-1-yl)phényl)azétidine-1-carbonyl)hexahydro-2H-pyrido[4,3-b][1,4]oxazin-3(4H)-one ;
la (4aR,8aS)-6-(3-(4-(3-(trifluorométhoxy)azétidin-1-yl)phényl)azétidine-1-carbonyl)hexahydro-2H-pyrido[4,3-b][1,4]oxazin-3(4H)-one ;
la (4aR,8aS)-6-(3-(6-(2,4-dichlorophényl)pyridin-3-yl)azétidine-1-carbonyl)hexahydro-2H-pyrido[4,3-b][1,4]oxazin-3(4H)-one ;
la (4aR,8aS)-6-[3-[4-(2,2,2-trifluoroéthoxy)phényl]azétidine-1-carbonyl]-4,4a,5,7,8,8a-hexahydropyrido[4,3-b][1,4]oxazin-3-one ;
la (4aR,8aS)-6-[3-[4-[3-[(1S ou 1R)-2,2,2-trifluoro-1-méthyl-éthoxy]azétidin-1-yl]phényl]azétidine-1-carbonyl]-4,4a,5,7,8,8a-hexahydropyrido[4,3-b][1,4]oxazin-3-one ;
la (4aR,8aS)-6-[3-[4-[3-[(1R ou 1S)-2,2,2-trifluoro-1-méthyl-éthoxy]azétidin-1-yl]phényl]azétidine-1-carbonyl]-4,4a,5,7,8,8a-hexahydropyrido[4,3-b][1,4]oxazin-3-one ;
la (4aR,8aS)-6-(3-(4-(2-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(4-(3-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(4-(3-(2,2,2-trifluoroéthoxy)azétidin-1-yl)phényl)azétidine-1-carbonyl)hexahydro-2H-pyrido[4,3-b][1,4]oxazin-3(4H)-one ;
la (4aR,8aS)-6-(3-(6-(2-(trifluorométhyl)pyrrolidin-1-yl)pyridin-3-yl)azétidine-1-carbonyl)hexahydro-2H-pyrido[4,3-b][1,4]oxazin-3(4H)-one ;
la (4aR,8aS)-6-(3-(4-(pentafluoro-16-sulfaneyl)phényl)azétidine-1-carbonyl)hexahydro-2H-pyrido[4,3-b][1,4]oxazin-3(4H)-one ;
la (4aR,8aS)-6-(3-(2-fluoro-4-(trifluorométhoxy)phényl)azétidine-1-carbonyl)hexahydro-2H-pyrido[4,3-b][1,4]oxazin-3(4H)-one ;
la (4aR,8aS)-6-[3-[4-(2,2,2-trifluoroéthyl)phényl]azétidine-1-carbonyl]-4,4a,5,7,8,8a-hexahydropyrido[4,3-b][1,4]oxazin-3-one ;
la (4aR,8aS)-6-[3-[4-[1-(trifluorométhyl)cyclopropyl]phényl]azétidine-1-carbonyl]-4,4a,5,7,8,8a-hexahydropyrido[4,3-b][1,4]oxazin-3-one ;
la (4aR,8aS)-6-[3-[4-(6,6-difluoro-2-azaspiro[3.3]heptan-2-yl)phényl]azétidine-1-carbonyl]-4,4a,5,7,8,8a-hexahydropyrido[4,3-b][1,4]oxazin-3-one ;
la (4aR,8aS)-6-(3-(5-(2,4-dichlorophényl)pyridin-2-yl)azétidine-1-carbonyl)hexahydro-2H-pyrido[4,3-b][1,4]oxazin-3(4H)-one ;
la (4aR,8aS)-6-(3-(4-((R ou S)-2-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(4-((S ou R)-2-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(4-((R ou S)-3-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(4-((S ou R)-3-(trifluorométhyl)pyrrolidin-1-yl)phényl)azétidine-1-carbonyl)hexahydro-2H-pyrido[4,3-b][1,4]oxazin-3(4H)-one ;
la (4aR,8aS)-6-(3-(3-fluoro-4-(trifluorométhoxy)phényl)azétidine-1-carbonyl)hexahydro-2H-pyrido[4,3-b][1,4]oxazin-3(4H)-one ;
la (4aR,8aS)-6-(3-(3-méthyl-4-(trifluorométhoxy)phényl)azétidine-1-carbonyl)hexahydro-2H-pyrido[4,3-b][1,4]oxazin-3(4H)-one ;
la (4aR,8aS)-6-(3-(3,5-difluoro-4-(trifluorométhoxy)phényl)azétidine-1-carbonyl)hexahydro-2H-pyrido[4,3-b][1,4]oxazin-3(4H)-one ;
la (4aR,8aS)-6-(3-(3-chloro-4-(trifluorométhoxy)phényl)azétidine-1-carbonyl)hexahydro-2H-pyrido[4,3-b][1,4]oxazin-3(4H)-one ;
la (4aR,8aS)-6-((R ou S)-3-(3-chloro-5-(2,2,2-trifluoroéthoxy)phényl)pyrrolidine-1-carbonyl)hexahydro-2H-pyrido[4,3-b][1,4]oxazin-3(4H)-one ;
la (4aR,8aS)-6-((S ou R)-3-(3-chloro-5-(2,2,2-trifluoroéthoxy)phényl)pyrrolidine-1-carbonyl)hexahydro-2H-pyrido[4,3-b][1,4]oxazin-3(4H)-one ;
la (4aR,8aS)-6-(3-(4-(tert-butyl)-3-méthoxyphényl)azétidine-1-carbonyl)hexahydro-2H-pyrido[4,3-b][1,4]oxazin-3(4H)-one ;
la (4aR,8aS)-6-(3-(4-propylphényl)azétidine-1-carbonyl)hexahydro-2H-pyrido[4,3-b][1,4]oxazin-3(4H)-one ;
la (4aR,8aS)-6-(3-(4-(trifluorométhoxy)-3-(trifluorométhyl)phényl)azétidine-1-carbonyl)hexahydro-2H-pyrido[4,3-b][1,4]oxazin-3(4H)-one ;
la (4aR,8aS)-6-(3-(5-((R ou S)-3-(trifluorométhyl)pyrrolidin-1-yl)pyridin-2-yl)azétidine-1-carbonyl)hexahydro-2H-pyrido[4,3-b][1,4]oxazin-3(4H)-one ;
la (4aR,8aS)-6-(3-(5-((S ou R)-3-(trifluorométhyl)pyrrolidin-1-yl)pyridin-2-yl)azétidine-1-carbonyl)hexahydro-2H-pyrido[4,3-b][1,4]oxazin-3(4H)-one ;
la (4aR,8aS)-6-[3-[6-[3-(R ou S)-(trifluorométhyl)pyrrolidin-1-yl]-3-pyridyl]azétidine-1-carbonyl]-4,4a,5,7,8,8a-hexahydropyrido[4,3-b][1,4]oxazin-3-one ;
la (4aR,8aS)-6-[3-[6-[3-(S ou R)-(trifluorométhyl)pyrrolidin-1-yl]-3-pyridyl]azétidine-1-carbonyl]-4,4a,5,7,8,8a-hexahydropyrido[4,3-b][1,4]oxazin-3-one ;
la (4aR,8aS)-6-[3-(4-tétrahydropyran-3-ylphényl)azétidine-1-carbonyl]-4,4a,5,7,8,8a-hexahydropyrido[4,3-b][1,4]oxazin-3-one ;
la (4aR,8aS)-6-[3-[2-méthoxy-4-(2,2,2-trifluoroéthyl)phényl]azétidine-1-carbonyl]-4,4a,5,7,8,8a-hexahydropyrido[4,3-b][1,4]oxazin-3-one ; et
la (4aR,8aS)-6-(3-(4-(néopentyloxy)phényl)azétidine-1-carbonyl)hexahydro-2H-pyrido[4,3-b] [1,4]oxazin-3(4H)-one.

11. Procédé de fabrication des composés de formule (Ic) selon l'une quelconque des revendications 1 à 10, comprenant :
la réaction de la 4a,5,6,7,8,8a-hexahydro-4H-pyrido[4,3-b][1,4]oxazin-3-one (1), 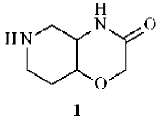
avec une amine hétérocyclique 2a, dans lequel A, L, X, m, n et R²⁰ à R²³ sont tels que définis dans l'une quelconque des revendications 1 à 10 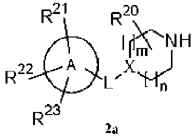
en présence d'une base et d'un réactif de formation d'urée,
pour former ledit composé de formule (Ic).

12. Composé de formule (Ic) selon l'une quelconque des revendications 1 à 10, ou un sel pharmaceutiquement acceptable de celui-ci, pour une utilisation comme substance thérapeutiquement active.

13. Composition pharmaceutique comprenant un composé de formule (Ic) selon l'une quelconque des revendications 1 à 10, ou un sel pharmaceutiquement acceptable de celui-ci, et un véhicule thérapeutiquement inerte.

14. Composé de formule (Ic) selon l'une quelconque des revendications 1 à 10, ou un sel pharmaceutiquement acceptable de celui-ci, pour une utilisation dans le traitement ou la prophylaxie d'une neuroinflammation, de maladies neurodégénératives, d'une douleur, d'un cancer et/ou de troubles mentaux chez un mammifère.

15. Composé pour une utilisation selon la revendication 14, dans lequel lesdits neuroinflammation, maladies neurodégénératives, douleur, cancer et/ou troubles mentaux sont choisis parmi la sclérose en plaques, la maladie d'Alzheimer, la maladie de Parkinson, la sclérose latérale amyotrophique, la lésion cérébrale traumatique, la neurotoxicité, l'accident vasculaire cérébral, l'épilepsie, l'anxiété, la migraine, la dépression, le carcinome hépatocellulaire, la carcinogenèse du côlon, le cancer de l'ovaire, la douleur neuropathique, la neuropathie induite par la chimiothérapie, la douleur aiguë, la douleur chronique et/ou la spasticité associée à la douleur chez un mammifère.
